# EUROPEAN PATENT APPLICATION

(11) **EP 3 686 289 A1**
(43) Date of publication of application: **29.07.2020**
(21) Application number: 19153535.0
(22) Date of filing: 24.01.2019
(51) Int. Cl.: C12Q 1/6883, C12Q 1/70

(54) **CMI SEQUENCES AS AN EARLY MARKER FOR THE FUTURE DEVELOPMENT OF CANCER, ATHEROSCLEROSIS, DIABETES AND DISEASES OF THE CNS AND AS A TARGET FOR THE TREATMENT AND PREVENTION OF THESE DISEASES**

(71) Applicant: Deutsches Krebsforschungszentrum, Stiftung des öffentlichen Rechts, 69120 Heidelberg (DE)
(72) Inventor: de Villiers, Ethel-Michele, 69483 Wald-Michelbach (DE); Gunst, Karin, 69493 Hirschberg (DE); zur Hausen, Harald, 69483 Wald-Michelbach (DE); Bund, Timo, 69221 Dossenheim (DE)
(74) Representative: Schüssler, Andrea

(57) **Abstract**

Described are CMI (Cow Milk Isolate) nucleotide sequences as well as probes and primers comprising part of said nucleotide sequences and antibodies against polypeptides encoded by said nucleotide sequences. Said compounds are useful as early markers for the future development of cancer, diabetes, atherosclerosis and diseases of the CNS (Multiple sclerosis MS, Prion-linked diseases, amyotrophic lateral sclerosis, transmissible spongiforme encephalitis, Parkinson's disease, Alzheimer disease) and should represent targets for treatment and prevention.

SEQUENCE LISTING

<110> Deutsches Krebsforschungszentrum

<120> CMI sequences as an early marker for the future development of cancer,
atherosclerosis, diabetes and diseases of the CNS and as a target for the
treatment and prevention of these diseases

<130> 3653EP

<160> 647

<170> BiSSAP 1.3.6

<210> 1
<211> 18
<212> DNA
<213> Artificial Sequence

<220>
<223> primer

<400> 1
aaggcagatc aacacagg                                                    18

<210> 2
<211> 18
<212> DNA
<213> Artificial Sequence

<220>
<223> primer

<400> 2
agcagattgc aaagcctg                                                    18

<210> 3
<211> 20
<212> DNA
<213> Artificial Sequence

<220>
<223> primer

<400> 3
caacacaggg atagaataac                                                  20

<210> 4
<211> 19
<212> DNA
<213> Artificial Sequence

<220>
<223> primer

<400> 4

atctgcctta gcagattgc                                                    19

<210> 5
<211> 21
<212> DNA
<213> Artificial Sequence

<220>
<223> primer

<400> 5
tgcagaaatt gcccctcgac t                                                 21

<210> 6
<211> 23
<212> DNA
<213> Artificial Sequence

<220>
<223> primer

<400> 6
aacaatgggg aagaagtcaa agg                                               23

<210> 7
<211> 18
<212> DNA
<213> Artificial Sequence

<220>
<223> primer

<400> 7
aaggcagatc aacacagg                                                     18

<210> 8
<211> 18
<212> DNA
<213> Artificial Sequence

<220>
<223> primer

<400> 8
agcagattgc aaagcctg                                                     18

<210> 9
<211> 20
<212> DNA
<213> Artificial Sequence

<220>
<223> primer

<400> 9
caacacaggg atagaataac                                                    20

<210> 10
<211> 19
<212> DNA
<213> Artificial Sequence

<220>
<223> primer

<400> 10
atctgcctta gcagattgc                                                     19

<210> 11
<211> 2296
<212> DNA
<213> Artificial Sequence

<220>
<223> isolate

<400> 11
aaggcagatc aacacaggga tagaataaca cgttttgcga ctttgaaaca tagagctaag      60

aatcaggaaa attatttatt taccctggct aagtttaaag agaactatga aaaagacgtt     120

aaaaacgaag aatctatcaa agctctaaaa tctgctcaga aattgaatga atgcggaaac     180

tatctgctat tcaaaaattt ttacacaata ggcgaagtta aactttccaa gcttcgtacc     240

tgcggacagc atttgctttg tcctttctgt gctgccattc gtgcatctcg tgctattcaa     300

aaatacgttg aacgtattga ccaagtctta aaagaaaatc gcaagctcaa gcctgttcta     360

atcacgctca cagttaaaaa cggctctaac ctagcagaac gctctgaaca tcttatgaag     420

tcatttagaa cgcttctaga gcgtcgtagg gactatgaaa agaaaggacg aggttttaac     480

gagttctgta aggttcaagg tgctatgtat tcatatgaga atacattcaa tgagaaaaca     540

ggcgaatggc atccgcatat tcatatgttt gcactggttg accagtggat tgatcaacaa     600

gaattttcag aatactggca cagtgttact ggcgattcta tggttgttga tgtccgtagg     660

gctaagagag aaaagggaca tggatatagc aaagctgctg ctgaagtctg taaatatgct     720

ttgaagtttt gcgatctgtc tgttgaaaag acttgggagg ctttcaaagt tctcaaaggt     780

aaacgcttaa caggctcttt tgggcttctg tggggtgtca aaattcctga cacaatgaca     840

gacgatatgc cagaagaaga tttgccttat cttgaaatgc tttacaagtt tacatacggc     900

aaaaagtctt actacgactt accgataaca agacacgtag aagcgcaggc gggagagcgc     960

gcggagttag gaggagctac gacgacgccc gacgagagat atagcgcggg tgcttcaagt    1020

gttaaacaag gttcggaatg gagcgtggcg accccgggtc gaacagcgct ctgttctgta    1080

ccgaagtttc aacgtaaaaa agcccactgg agaatcccac cttctacccg tgttcgtgtt 1140

cggcaaagaa tccgaagatg ggacgggatt atgtataaca ttgatctttt cccatatgtc 1200

gaacataggc tattggcttt cattggttag tctgtttctt gagaaacgcg gaagcgtgca 1260

ttctgaaaaa ctcaccactt ttgaaaaaag tgtagtgagt acacttctcc agtagccgaa 1320

cgagtgtcta cgagtgacga ctcctaaata agaatttcaa aaccttcctt tctctgatta 1380

caagctcccc ttagatttct aagcgggagt ttttagccat cccgaacggg tggcgacgca 1440

atactatttg catctttttc gataatgata tataaaagat tcgtctagac tctaagttaa 1500

tttcaaaaat atattaggta tatcactatg tcagagccaa caaacctaca gttattcgtt 1560

gctgaactta aaaaaactgg tcgttcttct tatcacggtg cttatttcca agttccgttc 1620

cgtgtccaaa tgcaccttca cgcaaaagtt gaagccctta ctaaacactt gggtagtaca 1680

cgaaataagg tcttaaacga tcttttatca attgcccttg atcaggttta tcaatctttg 1740

gatctcgatg aagatacctt gcacgaaatc caagtagagg aaggtcgcat tctccatgaa 1800

ttacttgaaa accgtaaaga tattaaatct ggagatatgg cagatgatta atttacaggg 1860

gactttaatc aatgctttcc gtgtggatgg tggtaaaggt aaagacggca aagaatatga 1920

agcgcgtgac aaggtgcaaa ttcttggttc gttggaactc ccaaacgggg aaattaaaca 1980

cgaacttgtt gacctaacag ttgaggattc tcgactattt gaaccattca aaaatcaggt 2040

tattagcatc tcgtgtggtg ctatggctat cggtcgtaat gttgtttttt atgttcgtaa 2100

aggcgcaaaa ccgattttag cagaaagtat ttgatactcg ttaaaaagca gtgagttagc 2160

gaagctactc gctgtttttt cttttacttg atactattga ttaaagtggg gacaaaaatt 2220

gcttaaaaaa gacaataaaa aagcccatat ttcggacact ttggcgggtg atgaaataca 2280

ggctttgcaa tctgct 2296

<210> 12
<211> 89
<212> PRT
<213> Artificial Sequence

<220>
<223> orf 1

<400> 12
Met Lys Arg Lys Asp Glu Val Leu Thr Ser Ser Val Arg Phe Lys Val
1               5                   10                  15
Leu Cys Ile His Met Arg Ile His Ser Met Arg Lys Gln Ala Asn Gly
            20                  25                  30
Ile Arg Ile Phe Ile Cys Leu His Trp Leu Thr Ser Gly Leu Ile Asn
        35                  40                  45
Lys Asn Phe Gln Asn Thr Gly Thr Val Leu Leu Ala Ile Leu Trp Leu
    50                  55                  60
Leu Met Ser Val Gly Leu Arg Glu Lys Arg Asp Met Asp Ile Ala Lys

65                  70                  75                  80
Leu Leu Leu Lys Ser Val Asn Met Leu
                85

<210> 13
<211> 401
<212> PRT
<213> Artificial Sequence

<220>
<223> orf 2

<400> 13
Met Thr Arg Phe Ala Thr Leu Lys His Arg Ala Lys Asn Gln Glu Asn
1               5                   10                  15
Tyr Leu Phe Thr Leu Ala Lys Phe Lys Glu Asn Tyr Glu Lys Asp Val
            20                  25                  30
Lys Asn Glu Glu Ser Ile Lys Ala Leu Lys Ser Ala Gln Lys Leu Asn
        35                  40                  45
Glu Cys Gly Asn Tyr Leu Leu Phe Lys Asn Phe Tyr Thr Ile Gly Glu
    50                  55                  60
Val Lys Leu Ser Lys Leu Arg Thr Cys Gly Gln His Leu Leu Cys Pro
65                  70                  75                  80
Phe Cys Ala Ala Ile Arg Ala Ser Arg Ala Ile Gln Lys Tyr Val Glu
                85                  90                  95
Arg Ile Asp Gln Val Leu Lys Glu Asn Arg Lys Leu Lys Pro Val Leu
            100                 105                 110
Ile Thr Leu Thr Val Lys Asn Gly Ser Asn Leu Ala Glu Arg Ser Glu
        115                 120                 125
His Leu Met Lys Ser Phe Arg Thr Leu Leu Glu Arg Arg Arg Asp Tyr
    130                 135                 140
Glu Lys Lys Gly Arg Gly Phe Asn Glu Phe Cys Lys Val Gln Gly Ala
145                 150                 155                 160
Met Tyr Ser Tyr Glu Asn Thr Phe Asn Glu Lys Thr Gly Glu Trp His
                165                 170                 175
Pro His Ile His Met Phe Ala Leu Val Asp Gln Trp Ile Asp Gln Gln
            180                 185                 190
Glu Phe Ser Glu Tyr Trp His Ser Val Thr Gly Asp Ser Met Val Val
        195                 200                 205
Asp Val Arg Arg Ala Lys Arg Glu Lys Gly His Gly Tyr Ser Lys Ala
    210                 215                 220
Ala Ala Glu Val Cys Lys Tyr Ala Leu Lys Phe Cys Asp Leu Ser Val
225                 230                 235                 240
Glu Lys Thr Trp Glu Ala Phe Lys Val Leu Lys Gly Lys Arg Leu Thr
                245                 250                 255
Gly Ser Phe Gly Leu Leu Trp Gly Val Lys Ile Pro Asp Thr Met Thr
            260                 265                 270
Asp Asp Met Pro Glu Glu Asp Leu Pro Tyr Leu Glu Met Leu Tyr Lys
        275                 280                 285
Phe Thr Tyr Gly Lys Lys Ser Tyr Tyr Asp Leu Pro Ile Thr Arg His
    290                 295                 300
Val Glu Ala Gln Ala Gly Glu Arg Ala Glu Leu Gly Gly Ala Thr Thr
305                 310                 315                 320
Thr Pro Asp Glu Arg Tyr Ser Ala Gly Ala Ser Ser Val Lys Gln Gly
                325                 330                 335
Ser Glu Trp Ser Val Ala Thr Pro Gly Arg Thr Ala Leu Cys Ser Val
            340                 345                 350
Pro Lys Phe Gln Arg Lys Lys Ala His Trp Arg Ile Pro Pro Ser Thr
        355                 360                 365
Arg Val Arg Val Arg Gln Arg Ile Arg Arg Trp Asp Gly Ile Met Tyr
    370                 375                 380
Asn Ile Asp Leu Phe Pro Tyr Val Glu His Arg Leu Leu Ala Phe Ile

385                 390                 395                 400
Gly

<210> 14
<211> 107
<212> PRT
<213> Artificial Sequence

<220>
<223> orf 3

<400> 14
Met Arg Asp Ile Ala Arg Val Leu Gln Val Leu Asn Lys Val Arg Asn
1               5                   10                  15
Gly Ala Trp Arg Pro Arg Val Glu Gln Arg Ser Val Leu Tyr Arg Ser
            20                  25                  30
Phe Asn Val Lys Lys Pro Thr Gly Glu Ser His Leu Leu Pro Val Phe
        35                  40                  45
Val Phe Gly Lys Glu Ser Glu Asp Gly Thr Gly Leu Cys Ile Thr Leu
    50                  55                  60
Ile Phe Ser His Met Ser Asn Ile Gly Tyr Trp Leu Ser Leu Val Ser
65                  70                  75                  80
Leu Phe Leu Glu Lys Arg Gly Ser Val His Ser Glu Lys Leu Thr Thr
                85                  90                  95
Phe Glu Lys Ser Val Val Ser Thr Leu Leu Gln
            100                 105

<210> 15
<211> 61
<212> PRT
<213> Artificial Sequence

<220>
<223> orf 4

<400> 15
Met Leu Gly Ile Ser Leu Cys Gln Ser Gln Gln Thr Tyr Ser Tyr Ser
1               5                   10                  15
Leu Leu Asn Leu Lys Lys Leu Val Val Leu Leu Ile Thr Val Leu Ile
            20                  25                  30
Ser Lys Phe Arg Ser Val Ser Lys Cys Thr Phe Thr Gln Lys Leu Lys
        35                  40                  45
Pro Leu Leu Asn Thr Trp Val Val His Glu Ile Arg Ser
    50                  55                  60

<210> 16
<211> 112
<212> PRT
<213> Artificial Sequence

<220>
<223> orf 5

<400> 16
Met Arg Tyr Ile Thr Met Ser Glu Pro Thr Asn Leu Gln Leu Phe Val
1               5                   10                  15
Ala Glu Leu Lys Lys Thr Gly Arg Ser Ser Tyr His Gly Ala Tyr Phe
            20                  25                  30
Gln Val Pro Phe Arg Val Gln Met His Leu His Ala Lys Val Glu Ala

35                  40                  45
Leu Thr Lys His Leu Gly Ser Thr Arg Asn Lys Val Leu Asn Asp Leu
    50                  55                  60
Leu Ser Ile Ala Leu Asp Gln Val Tyr Gln Ser Leu Asp Leu Asp Glu
65                  70                  75                  80
Asp Thr Leu His Glu Ile Gln Val Glu Glu Gly Arg Ile Leu His Glu
                85                  90                  95
Leu Leu Glu Asn Arg Lys Asp Ile Lys Ser Gly Asp Met Ala Asp Asp
            100                 105                 110

<210> 17
<211> 112
<212> PRT
<213> Artificial Sequence

<220>
<223> orf 6

<400> 17
Met Asn Tyr Leu Lys Thr Val Lys Ile Leu Asn Leu Glu Ile Trp Gln
1               5                   10                  15
Met Ile Asn Leu Gln Gly Thr Leu Ile Asn Ala Phe Arg Val Asp Gly
            20                  25                  30
Gly Lys Gly Lys Asp Gly Lys Glu Tyr Glu Ala Arg Asp Lys Val Gln
        35                  40                  45
Ile Leu Gly Ser Leu Glu Leu Pro Asn Gly Glu Ile Lys His Glu Leu
    50                  55                  60
Val Asp Leu Thr Val Glu Asp Ser Arg Leu Phe Glu Pro Phe Lys Asn
65                  70                  75                  80
Gln Val Ile Ser Ile Ser Cys Gly Ala Met Ala Ile Gly Arg Asn Val
                85                  90                  95
Val Phe Tyr Val Arg Lys Gly Ala Lys Pro Ile Leu Ala Glu Ser Ile
            100                 105                 110

<210> 18
<211> 435
<212> PRT
<213> Artificial Sequence

<220>
<223> orf 7

<400> 18
Met Leu Lys Lys Asp Asn Lys Lys Ala His Ile Ser Asp Thr Leu Ala
1               5                   10                  15
Gly Asp Glu Ile Gln Ala Leu Gln Ser Ala Lys Ala Asp Gln His Arg
            20                  25                  30
Asp Arg Ile Thr Arg Phe Ala Thr Leu Lys His Arg Ala Lys Asn Gln
        35                  40                  45
Glu Asn Tyr Leu Phe Thr Leu Ala Lys Phe Lys Glu Asn Tyr Glu Lys
    50                  55                  60
Asp Val Lys Asn Glu Glu Ser Ile Lys Ala Leu Lys Ser Ala Gln Lys
65                  70                  75                  80
Leu Asn Glu Cys Gly Asn Tyr Leu Leu Phe Lys Asn Phe Tyr Thr Ile
                85                  90                  95
Gly Glu Val Lys Leu Ser Lys Leu Arg Thr Cys Gly Gln His Leu Leu
            100                 105                 110

Cys Pro Phe Cys Ala Ala Ile Arg Ala Ser Arg Ala Ile Gln Lys Tyr
        115                 120                 125
Val Glu Arg Ile Asp Gln Val Leu Lys Glu Asn Arg Lys Leu Lys Pro
    130                 135                 140
Val Leu Ile Thr Leu Thr Val Lys Asn Gly Ser Asn Leu Ala Glu Arg
145                 150                 155                 160
Ser Glu His Leu Met Lys Ser Phe Arg Thr Leu Leu Glu Arg Arg Arg
                165                 170                 175
Asp Tyr Glu Lys Lys Gly Arg Gly Phe Asn Glu Phe Cys Lys Val Gln
            180                 185                 190
Gly Ala Met Tyr Ser Tyr Glu Asn Thr Phe Asn Glu Lys Thr Gly Glu
        195                 200                 205
Trp His Pro His Ile His Met Phe Ala Leu Val Asp Gln Trp Ile Asp
    210                 215                 220
Gln Gln Glu Phe Ser Glu Tyr Trp His Ser Val Thr Gly Asp Ser Met
225                 230                 235                 240
Val Val Asp Val Arg Arg Ala Lys Arg Glu Lys Gly His Gly Tyr Ser
                245                 250                 255
Lys Ala Ala Ala Glu Val Cys Lys Tyr Ala Leu Lys Phe Cys Asp Leu
            260                 265                 270
Ser Val Glu Lys Thr Trp Glu Ala Phe Lys Val Leu Lys Gly Lys Arg
        275                 280                 285
Leu Thr Gly Ser Phe Gly Leu Leu Trp Gly Val Lys Ile Pro Asp Thr
    290                 295                 300
Met Thr Asp Asp Met Pro Glu Glu Asp Leu Pro Tyr Leu Glu Met Leu
305                 310                 315                 320
Tyr Lys Phe Thr Tyr Gly Lys Lys Ser Tyr Tyr Asp Leu Pro Ile Thr
                325                 330                 335
Arg His Val Glu Ala Gln Ala Gly Glu Arg Ala Glu Leu Gly Gly Ala
            340                 345                 350
Thr Thr Thr Pro Asp Glu Arg Tyr Ser Ala Gly Ala Ser Ser Val Lys
        355                 360                 365
Gln Gly Ser Glu Trp Ser Val Ala Thr Pro Gly Arg Thr Ala Leu Cys
    370                 375                 380
Ser Val Pro Lys Phe Gln Arg Lys Lys Ala His Trp Arg Ile Pro Pro
385                 390                 395                 400
Ser Thr Arg Val Arg Val Arg Gln Arg Ile Arg Arg Trp Asp Gly Ile
                405                 410                 415
Met Tyr Asn Ile Asp Leu Phe Pro Tyr Val Glu His Arg Leu Leu Ala
            420                 425                 430
Phe Ile Gly
        435

<210> 19
<211> 76
<212> PRT
<213> Artificial Sequence

<220>
<223> orf 8

<400> 19
Met Asn Gly Ser Asn Ser Arg Glu Ser Ser Thr Val Arg Ser Thr Ser
1               5                   10                  15
Ser Cys Leu Ile Ser Pro Phe Gly Ser Ser Asn Glu Pro Arg Ile Cys
            20                  25                  30
Thr Leu Ser Arg Ala Ser Tyr Ser Leu Pro Ser Leu Pro Leu Pro Pro
        35                  40                  45
Ser Thr Arg Lys Ala Leu Ile Lys Val Pro Cys Lys Leu Ile Ile Cys
    50                  55                  60
His Ile Ser Arg Phe Asn Ile Phe Thr Val Phe Lys
65                  70                  75

<210> 20
<211> 63
<212> PRT
<213> Artificial Sequence

<220>
<223> orf 9

<400> 20
Met Phe Asp Ile Trp Glu Lys Ile Asn Val Ile His Asn Pro Val Pro
1               5                   10                  15
Ser Ser Asp Ser Leu Pro Asn Thr Asn Thr Gly Arg Arg Trp Asp Ser
            20                  25                  30
Pro Val Gly Phe Phe Thr Leu Lys Leu Arg Tyr Arg Thr Glu Arg Cys
        35                  40                  45
Ser Thr Arg Gly Arg His Ala Pro Phe Arg Thr Leu Phe Asn Thr
    50                  55                  60

<210> 21
<211> 84
<212> PRT
<213> Artificial Sequence

<220>
<223> orf

<400> 21
Met Ser Ser Val Ile Val Ser Gly Ile Leu Thr Pro His Arg Ser Pro
1               5                   10                  15
Lys Glu Pro Val Lys Arg Leu Pro Leu Arg Thr Leu Lys Ala Ser Gln
            20                  25                  30
Val Phe Ser Thr Asp Arg Ser Gln Asn Phe Lys Ala Tyr Leu Gln Thr
        35                  40                  45
Ser Ala Ala Ala Leu Leu Tyr Pro Cys Pro Phe Ser Leu Leu Ala Leu
    50                  55                  60
Arg Thr Ser Thr Thr Ile Glu Ser Pro Val Thr Leu Cys Gln Tyr Ser
65                  70                  75                  80
Glu Asn Ser Cys

<210> 22
<211> 56
<212> PRT
<213> Artificial Sequence

<220>
<223> orf

<400> 22
Met Ile Phe Leu Ile Leu Ser Ser Met Phe Gln Ser Arg Lys Thr Cys
1               5                   10                  15
Tyr Ser Ile Pro Val Leu Ile Cys Leu Ser Arg Leu Gln Ser Leu Tyr
            20                  25                  30
Phe Ile Thr Arg Gln Ser Val Arg Asn Met Gly Phe Phe Ile Val Phe
        35                  40                  45
Phe Lys Gln Phe Leu Ser Pro Leu
    50                  55

<210> 23

<211> 267
<212> DNA
<213> Artificial Sequence

<220>
<223> orf sequence

<400> 23
atgaaaagaa aggacgaggt tttaacgagt tctgtaaggt tcaaggtgct atgtattcat 60

atgagaatac attcaatgag aaaacaggcg aatggcatcc gcatattcat atgtttgcac 120

tggttgacca gtggattgat caacaagaat tttcagaata ctggcacagt gttactggcg 180

attctatggt tgttgatgtc cgtagggcta agagagaaaa gggacatgga tatagcaaag 240

ctgctgctga agtctgtaaa tatgctt 267

<210> 24
<211> 1203
<212> DNA
<213> Artificial Sequence

<220>
<223> orf

<400> 24
ataacacgtt ttgcgacttt gaaacataga gctaagaatc aggaaaatta tttatttacc 60

ctggctaagt ttaaagagaa ctatgaaaaa gacgttaaaa acgaagaatc tatcaaagct 120

ctaaaatctg ctcagaaatt gaatgaatgc ggaaactatc tgctattcaa aaatttttac 180

acaataggcg aagttaaact ttccaagctt cgtacctgcg gacagcattt gctttgtcct 240

ttctgtgctg ccattcgtgc atctcgtgct attcaaaaat acgttgaacg tattgaccaa 300

gtcttaaaag aaaatcgcaa gctcaagcct gttctaatca cgctcacagt taaaaacggc 360

tctaacctag cagaacgctc tgaacatctt atgaagtcat ttagaacgct tctagagcgt 420

cgtagggact atgaaaagaa aggacgaggt tttaacgagt tctgtaaggt tcaaggtgct 480

atgtattcat atgagaatac attcaatgag aaaacaggcg aatggcatcc gcatattcat 540

atgtttgcac tggttgacca gtggattgat caacaagaat tttcagaata ctggcacagt 600

gttactggcg attctatggt tgttgatgtc cgtagggcta agagagaaaa gggacatgga 660

tatagcaaag ctgctgctga agtctgtaaa tatgctttga agttttgcga tctgtctgtt 720

gaaaagactt gggaggcttt caaagttctc aaaggtaaac gcttaacagg ctcttttggg 780

cttctgtggg gtgtcaaaat tcctgacaca atgacagacg atatgccaga agaagatttg 840

ccttatcttg aaatgcttta caagtttaca tacggcaaaa agtcttacta cgacttaccg 900

ataacaagac acgtagaagc gcaggcggga gagcgcgcgg agttaggagg agctacgacg 960

acgcccgacg agagatatag cgcgggtgct tcaagtgtta aacaaggttc ggaatggagc 1020

gtggcgaccc cgggtcgaac agcgctctgt tctgtaccga agtttcaacg taaaaaagcc 1080

cactggagaa tcccaccttc tacccgtgtt cgtgttcggc aaagaatccg aagatgggac 1140

gggattatgt ataacattga tcttttccca tatgtcgaac ataggctatt ggctttcatt 1200

ggt 1203

<210> 25
<211> 321
<212> DNA
<213> Artificial Sequence

<220>
<223> orf

<400> 25
acgagagata tagcgcgggt gcttcaagtg ttaaacaagg ttcggaatgg agcgtggcga 60

ccccgggtcg aacagcgctc tgttctgtac cgaagtttca acgtaaaaaa gcccactgga 120

gaatcccacc ttctacccgt gttcgtgttc ggcaaagaat ccgaagatgg gacgggatta 180

tgtataacat tgatcttttc ccatatgtcg aacataggct attggctttc attggttagt 240

ctgtttcttg agaaacgcgg aagcgtgcat tctgaaaaac tcaccacttt tgaaaaaagt 300

gtagtgagta cacttctcca g 321

<210> 26
<211> 183
<212> DNA
<213> Artificial Sequence

<220>
<223> orf

<400> 26
atattaggta tatcactatg tcagagccaa caaacctaca gttattcgtt gctgaactta 60

aaaaaactgg tcgttcttct tatcacggtg cttatttcca agttccgttc cgtgtccaaa 120

tgcaccttca cgcaaaagtt gaagccctta ctaaacactt gggtagtaca cgaaataagg 180

tct 183

<210> 27
<211> 336
<212> DNA
<213> Artificial Sequence

<220>
<223> orf

<400> 27
attaggtata tcactatgtc agagccaaca aacctacagt tattcgttgc tgaacttaaa 60

aaaactggtc gttcttctta tcacggtgct tatttccaag ttccgttccg tgtccaaatg 120

caccttcacg caaaagttga agcccttact aaacacttgg gtagtacacg aaataaggtc 180

ttaaacgatc ttttatcaat tgcccttgat caggtttatc aatctttgga tctcgatgaa 240

gataccttgc acgaaatcca agtagaggaa ggtcgcattc tccatgaatt acttgaaaac 300

cgtaaagata ttaaatctgg agatatggca gatgat 336

<210> 28
<211> 336
<212> DNA
<213> Artificial Sequence

<220>
<223> orf

<400> 28
atgaattact tgaaaaccgt aaagatatta aatctggaga tatggcagat gattaattta 60

caggggactt taatcaatgc tttccgtgtg gatggtggta aaggtaaaga cggcaaagaa 120

tatgaagcgc gtgacaaggt gcaaattctt ggttcgttgg aactcccaaa cggggaaatt 180

aaacacgaac ttgttgacct aacagttgag gattctcgac tatttgaacc attcaaaaat 240

caggttatta gcatctcgtg tggtgctatg gctatcggtc gtaatgttgt tttttatgtt 300

cgtaaaggcg caaaaccgat tttagcagaa agtatt 336

<210> 29
<211> 1305
<212> DNA
<213> Artificial Sequence

<220>
<223> orf

<400> 29
ttgcttaaaa aagacaataa aaaagcccat atttcggaca ctttggcggg tgatgaaata 60

caggctttgc aatctgctaa ggcagatcaa cacagggata gaataacacg ttttgcgact 120

ttgaaacata gagctaagaa tcaggaaaat tatttattta ccctggctaa gtttaaagag 180

aactatgaaa aagacgttaa aaacgaagaa tctatcaaag ctctaaaatc tgctcagaaa 240

ttgaatgaat gcggaaacta tctgctattc aaaaattttt acacaatagg cgaagttaaa 300

ctttccaagc ttcgtacctg cggacagcat ttgctttgtc ctttctgtgc tgccattcgt 360

gcatctcgtg ctattcaaaa atacgttgaa cgtattgacc aagtcttaaa agaaaatcgc 420

aagctcaagc ctgttctaat cacgctcaca gttaaaaacg gctctaacct agcagaacgc 480

tctgaacatc ttatgaagtc atttagaacg cttctagagc gtcgtaggga ctatgaaaag 540

aaaggacgag gttttaacga gttctgtaag gttcaaggtg ctatgtattc atatgagaat 600

acattcaatg agaaaacagg cgaatggcat ccgcatattc atatgtttgc actggttgac 660

cagtggattg atcaacaaga attttcagaa tactggcaca gtgttactgg cgattctatg 720

gttgttgatg tccgtagggc taagagagaa aagggacatg gatatagcaa agctgctgct 780

gaagtctgta aatatgcttt gaagttttgc gatctgtctg ttgaaaagac ttgggaggct 840

ttcaaagttc tcaaaggtaa acgcttaaca ggctcttttg ggcttctgtg gggtgtcaaa 900

attcctgaca caatgacaga cgatatgcca gaagaagatt tgccttatct tgaaatgctt 960

tacaagttta catacggcaa aaagtcttac tacgacttac cgataacaag acacgtagaa 1020

gcgcaggcgg gagagcgcgc ggagttagga ggagctacga cgacgcccga cgagagatat 1080

agcgcgggtg cttcaagtgt taaacaaggt tcggaatgga gcgtggcgac cccgggtcga 1140

acagcgctct gttctgtacc gaagtttcaa cgtaaaaaag cccactggag aatcccacct 1200

tctacccgtg ttcgtgttcg gcaaagaatc cgaagatggg acgggattat gtataacatt 1260

gatcttttcc catatgtcga acataggcta ttggctttca ttggt 1305

<210> 30
<211> 228
<212> DNA
<213> Artificial Sequence

<220>
<223> orf

<400> 30
ttgaatggtt caaatagtcg agaatcctca actgttaggt caacaagttc gtgtttaatt 60

tccccgtttg ggagttccaa cgaaccaaga atttgcacct tgtcacgcgc ttcatattct 120

ttgccgtctt tacctttacc accatccaca cggaaagcat tgattaaagt cccctgtaaa 180

ttaatcatct gccatatctc cagatttaat atctttacgg ttttcaag 228

<210> 31
<211> 189
<212> DNA
<213> Artificial Sequence

<220>
<223> orf

<400> 31
atgttcgaca tatgggaaaa gatcaatgtt atacataatc ccgtcccatc ttcggattct 60

ttgccgaaca cgaacacggg tagaaggtgg gattctccag tgggcttttt tacgttgaaa 120

cttcggtaca gaacagagcg ctgttcgacc cggggtcgcc acgctccatt ccgaaccttg 180

tttaacact 189

<210> 32
<211> 252
<212> DNA
<213> Artificial Sequence

<220>
<223> orf

<400> 32
atatcgtctg tcattgtgtc aggaattttg acaccccaca gaagcccaaa agagcctgtt 60

aagcgtttac ctttgagaac tttgaaagcc tcccaagtct tttcaacaga cagatcgcaa 120

aacttcaaag catatttaca gacttcagca gcagctttgc tatatccatg tcccttttct 180

ctcttagccc tacggacatc aacaaccata gaatcgccag taacactgtg ccagtattct 240

gaaaattctt gt 252

<210> 33
<211> 168
<212> DNA
<213> Artificial Sequence

<220>
<223> orf

<400> 33
ataattttcc tgattcttag ctctatgttt caaagtcgca aaacgtgtta ttctatccct 60

gtgttgatct gccttagcag attgcaaagc ctgtatttca tcacccgcca aagtgtccga 120

aatatgggct tttttattgt cttttttaag caatttttgt ccccactt 168

<210> 34
<211> 2356
<212> DNA
<213> Artificial Sequence

<220>
<223> isolate

<400> 34
aaggcagatc aacacaggga tagaataaca cgttttgcga ctttgaaaca tagggctaag 60

aatcaggaaa attatttatt taccctggct aagtttaaag agaactatga aaaagacgtt 120

aaaaacgaag aatctatcaa agctctaaaa tctgctcaga aattgaatga atgcggaaac 180

tatctgctat tcaaaaattt ttacacaata ggcgaagtta aactttctaa gctccgtacc 240

tgcggacagc atttgctttg tcctttctgc gctgctattc gtgcatctcg tgctattcaa 300

aaatacgttg aacgtattga tcaagtctta caagaaaatc gcaagcttaa gcctgttcta 360

atcacgctca ctgttaaaaa cggttctgat ctagcagaac gctctgaaca tcttatgaag 420

tcctttagaa agctcctaga tcgtcgtagg gactatgaaa agaaagggcg aggttttaac 480

gagttctgta aggttcaagg cgctatgtat tcatatgaaa atacatttaa tgaaaaaaca 540

ggcgaatggc atcctcatat tcatatgttc gctttagttg ataattggat tgatcagcaa 600

gaattttcag aatattggca tagccttact ggcgactcta tggttgtcga tgttcgtagg 660

gcaaaaaaag aaaaaggtca cggttatagc aaagcggctg ctgaagtctg taagtatgct 720

ttgaagtttg gcgatctgtc cgttgaaaag acttgggaag cgtttaaggt tctcaagggc 780

aagcgtttaa caggttcttt tggtctgcta tggggtgtca aaatccctga cacaatgaca 840

gacgatatgc cattagaaga tttgccatat ctcgaaatgc tctacaagtt cgcctacagt 900

aaaaaatcct actatgactt actaatcaca cgacacgtag agccacatag caaggacaac 960

gacgacgagg aggagcttcg acaacgagga gaaggcgtac gaagcgattt ggcgataacg 1020

gacgagggag gagcggagcg gattgagagc gagggcacgc agcgattgac acgcactgca 1080

ccgcaacgag gacgtaaaaa agcccactgg agaatcccac cttctacccg tgttcgtgtt 1140

cggcaacgaa tcagaagatg ggacggatat ttatataata ttgacctatt cccgtatgtt 1200

gagcataggc tattagcctt catagaagac taaagattac ttatataatc tttagttcct 1260

tctctccagt agccgagcga gtgtctacga gcgacgactc ctaaatcaga atttcaaaac 1320

cttcctttcc ctgattacaa agctcccctt agatttctaa gcgggagttt ttagccatcc 1380

cgaaagggtg gcgacgcatt gacaattata atttaatctg caatattcat aagacattca 1440

taaataatct gattggaata ttaaatatgc tttttaaatc tctaggcgtt gcaatcgcta 1500

caaataatgc ttttaactct gacgaagact tgaaaccaat tactattcgt ttagctgata 1560

gtgaaattca ggtgtatgac actgttgcaa aatcaatggg tctaactcgt caagattttc 1620

tatcacacgt tattcgttcc aacttccgac aggctttaaa agagtttgta gtgggttata 1680

caagttcaaa tccttctacg cctttgtctc ttttatttaa ttcaaataca gacaatgacg 1740

aagttaagct caaactttcc aaccttcttt catctatttc tcaggaatta atggacgaag 1800

aagaaaaaga aattcatgaa gcaatcgaaa accatatgaa tacttcctat gcatatgcaa 1860

ctcgtccaaa aggcatcttt gttgagaagg atgattcaaa atgattaagc ttgagggaat 1920

cgttttaaac gtatttaccc agcaaggcgg taaagataaa aaaacaggcg atcaatttga 1980

agaccgtgac aaggtacaaa ttttaggtgc tatggctctg cctaatggtg acgtaaaaaa 2040

tgaacttttt acgttatcag tagaaaatta tcgggacttt aaagactacc taaatcagaa 2100

aatttcgatt gctgttggtg caatggcaag tggtcgtaat gtgatttttt atgttgctaa 2160

aggtgcaaaa cctgttttag cagagacaat gtgaaacgtg aaaaaaaaca gtgagttagc 2220

gaagcttctc gctgtttttt cttttacttg atactattga ttaaagtggg gacaaaaatt 2280

gcttaaaaaa gacaataaaa aagcccatat ttcagaagct ttggcgggcg gagaaataca    2340

ggctttgcaa tctgct                                                    2356

<210> 35
<211> 402
<212> PRT
<213> Artificial Sequence

<220>
<223> orf

<400> 35
Met Thr Arg Phe Ala Thr Leu Lys His Arg Ala Lys Asn Gln Glu Asn
1               5                   10                  15
Tyr Leu Phe Thr Leu Ala Lys Phe Lys Glu Asn Tyr Glu Lys Asp Val
            20                  25                  30
Lys Asn Glu Glu Ser Ile Lys Ala Leu Lys Ser Ala Gln Lys Leu Asn
        35                  40                  45
Glu Cys Gly Asn Tyr Leu Leu Phe Lys Asn Phe Tyr Thr Ile Gly Glu
    50                  55                  60
Val Lys Leu Ser Lys Leu Arg Thr Cys Gly Gln His Leu Leu Cys Pro
65                  70                  75                  80
Phe Cys Ala Ala Ile Arg Ala Ser Arg Ala Ile Gln Lys Tyr Val Glu
                85                  90                  95
Arg Ile Asp Gln Val Leu Gln Glu Asn Arg Lys Leu Lys Pro Val Leu
            100                 105                 110
Ile Thr Leu Thr Val Lys Asn Gly Ser Asp Leu Ala Glu Arg Ser Glu
        115                 120                 125
His Leu Met Lys Ser Phe Arg Lys Leu Leu Asp Arg Arg Arg Asp Tyr
    130                 135                 140
Glu Lys Lys Gly Arg Gly Phe Asn Glu Phe Cys Lys Val Gln Gly Ala
145                 150                 155                 160
Met Tyr Ser Tyr Glu Asn Thr Phe Asn Glu Lys Thr Gly Glu Trp His
                165                 170                 175
Pro His Ile His Met Phe Ala Leu Val Asp Asn Trp Ile Asp Gln Gln
            180                 185                 190
Glu Phe Ser Glu Tyr Trp His Ser Leu Thr Gly Asp Ser Met Val Val
        195                 200                 205
Asp Val Arg Arg Ala Lys Lys Glu Lys Gly His Gly Tyr Ser Lys Ala
    210                 215                 220
Ala Ala Glu Val Cys Lys Tyr Ala Leu Lys Phe Gly Asp Leu Ser Val
225                 230                 235                 240
Glu Lys Thr Trp Glu Ala Phe Lys Val Leu Lys Gly Lys Arg Leu Thr
                245                 250                 255
Gly Ser Phe Gly Leu Leu Trp Gly Val Lys Ile Pro Asp Thr Met Thr
            260                 265                 270
Asp Asp Met Pro Leu Glu Asp Leu Pro Tyr Leu Glu Met Leu Tyr Lys
        275                 280                 285
Phe Ala Tyr Ser Lys Lys Ser Tyr Tyr Asp Leu Leu Ile Thr Arg His
    290                 295                 300
Val Glu Pro His Ser Lys Asp Asn Asp Asp Glu Glu Glu Leu Arg Gln
305                 310                 315                 320
Arg Gly Glu Gly Val Arg Ser Asp Leu Ala Ile Thr Asp Glu Gly Gly
                325                 330                 335
Ala Glu Arg Ile Glu Ser Glu Gly Thr Gln Arg Leu Thr Arg Thr Ala
            340                 345                 350
Pro Gln Arg Gly Arg Lys Lys Ala His Trp Arg Ile Pro Pro Ser Thr
        355                 360                 365
Arg Val Arg Val Arg Gln Arg Ile Arg Arg Trp Asp Gly Tyr Leu Tyr
    370                 375                 380

Asn Ile Asp Leu Phe Pro Tyr Val Glu His Arg Leu Leu Ala Phe Ile
385                 390                 395                 400
Glu Asp

<210> 36
<211> 183
<212> PRT
<213> Artificial Sequence

<220>
<223> orf

<400> 36
Met Ser Lys Arg Glu Phe Leu Ala Ile Pro Lys Gly Trp Arg Arg Ile
1               5                   10                  15
Asp Asn Tyr Asn Leu Ile Cys Asn Ile His Lys Thr Phe Ile Asn Asn
            20                  25                  30
Leu Ile Gly Ile Leu Asn Met Leu Phe Lys Ser Leu Gly Val Ala Ile
        35                  40                  45
Ala Thr Asn Asn Ala Phe Asn Ser Asp Glu Asp Leu Lys Pro Ile Thr
    50                  55                  60
Ile Arg Leu Ala Asp Ser Glu Ile Gln Val Tyr Asp Thr Val Ala Lys
65                  70                  75                  80
Ser Met Gly Leu Thr Arg Gln Asp Phe Leu Ser His Val Ile Arg Ser
                85                  90                  95
Asn Phe Arg Gln Ala Leu Lys Glu Phe Val Val Gly Tyr Thr Ser Ser
            100                 105                 110
Asn Pro Ser Thr Pro Leu Ser Leu Leu Phe Asn Ser Asn Thr Asp Asn
        115                 120                 125
Asp Glu Val Lys Leu Lys Leu Ser Asn Leu Leu Ser Ser Ile Ser Gln
    130                 135                 140
Glu Leu Met Asp Glu Glu Glu Lys Glu Ile His Glu Ala Ile Glu Asn
145                 150                 155                 160
His Met Asn Thr Ser Tyr Ala Tyr Ala Thr Arg Pro Lys Gly Ile Phe
                165                 170                 175
Val Glu Lys Asp Asp Ser Lys
            180

<210> 37
<211> 97
<212> PRT
<213> Artificial Sequence

<220>
<223> orf

<400> 37
Met Ile Lys Leu Glu Gly Ile Val Leu Asn Val Phe Thr Gln Gln Gly
1               5                   10                  15
Gly Lys Asp Lys Lys Thr Gly Asp Gln Phe Glu Asp Arg Asp Lys Val
            20                  25                  30
Gln Ile Leu Gly Ala Met Ala Leu Pro Asn Gly Asp Val Lys Asn Glu
        35                  40                  45
Leu Phe Thr Leu Ser Val Glu Asn Tyr Arg Asp Phe Lys Asp Tyr Leu
    50                  55                  60
Asn Gln Lys Ile Ser Ile Ala Val Gly Ala Met Ala Ser Gly Arg Asn
65                  70                  75                  80
Val Ile Phe Tyr Val Ala Lys Gly Ala Lys Pro Val Leu Ala Glu Thr
                85                  90                  95
Met

<210> 38
<211> 436
<212> PRT
<213> Artificial Sequence

<220>
<223> orf

<400> 38
   Met Leu Lys Lys Asp Asn Lys Lys Ala His Ile Ser Glu Ala Leu Ala
   1               5                   10                  15
   Gly Gly Glu Ile Gln Ala Leu Gln Ser Ala Lys Ala Asp Gln His Arg
               20                  25                  30
   Asp Arg Ile Thr Arg Phe Ala Thr Leu Lys His Arg Ala Lys Asn Gln
           35                  40                  45
   Glu Asn Tyr Leu Phe Thr Leu Ala Lys Phe Lys Glu Asn Tyr Glu Lys
       50                  55                  60
   Asp Val Lys Asn Glu Glu Ser Ile Lys Ala Leu Lys Ser Ala Gln Lys
   65                  70                  75                  80
   Leu Asn Glu Cys Gly Asn Tyr Leu Leu Phe Lys Asn Phe Tyr Thr Ile
                   85                  90                  95
   Gly Glu Val Lys Leu Ser Lys Leu Arg Thr Cys Gly Gln His Leu Leu
               100                 105                 110
   Cys Pro Phe Cys Ala Ala Ile Arg Ala Ser Arg Ala Ile Gln Lys Tyr
           115                 120                 125
   Val Glu Arg Ile Asp Gln Val Leu Gln Glu Asn Arg Lys Leu Lys Pro
       130                 135                 140
   Val Leu Ile Thr Leu Thr Val Lys Asn Gly Ser Asp Leu Ala Glu Arg
   145                 150                 155                 160
   Ser Glu His Leu Met Lys Ser Phe Arg Lys Leu Leu Asp Arg Arg Arg
                   165                 170                 175
   Asp Tyr Glu Lys Lys Gly Arg Gly Phe Asn Glu Phe Cys Lys Val Gln
               180                 185                 190
   Gly Ala Met Tyr Ser Tyr Glu Asn Thr Phe Asn Glu Lys Thr Gly Glu
           195                 200                 205
   Trp His Pro His Ile His Met Phe Ala Leu Val Asp Asn Trp Ile Asp
       210                 215                 220
   Gln Gln Glu Phe Ser Glu Tyr Trp His Ser Leu Thr Gly Asp Ser Met
   225                 230                 235                 240
   Val Val Asp Val Arg Arg Ala Lys Lys Glu Lys Gly His Gly Tyr Ser
                   245                 250                 255
   Lys Ala Ala Ala Glu Val Cys Lys Tyr Ala Leu Lys Phe Gly Asp Leu
               260                 265                 270
   Ser Val Glu Lys Thr Trp Glu Ala Phe Lys Val Leu Lys Gly Lys Arg
           275                 280                 285
   Leu Thr Gly Ser Phe Gly Leu Leu Trp Gly Val Lys Ile Pro Asp Thr
       290                 295                 300
   Met Thr Asp Asp Met Pro Leu Glu Asp Leu Pro Tyr Leu Glu Met Leu
   305                 310                 315                 320
   Tyr Lys Phe Ala Tyr Ser Lys Lys Ser Tyr Tyr Asp Leu Leu Ile Thr
                   325                 330                 335
   Arg His Val Glu Pro His Ser Lys Asp Asn Asp Asp Glu Glu Glu Leu
               340                 345                 350
   Arg Gln Arg Gly Glu Gly Val Arg Ser Asp Leu Ala Ile Thr Asp Glu
           355                 360                 365
   Gly Gly Ala Glu Arg Ile Glu Ser Glu Gly Thr Gln Arg Leu Thr Arg
       370                 375                 380
   Thr Ala Pro Gln Arg Gly Arg Lys Lys Ala His Trp Arg Ile Pro Pro
   385                 390                 395                 400
   Ser Thr Arg Val Arg Val Arg Gln Arg Ile Arg Arg Trp Asp Gly Tyr
   Leu Tyr Asn Ile Asp Leu Phe Pro Tyr Val Glu His Arg Leu Leu Ala
               420                     425                     430
   Phe Ile Glu Asp
           435

<210> 39
<211> 52
<212> PRT
<213> Artificial Sequence

<220>
<223> orf

<400> 39
Met Phe Lys Thr Ile Pro Ser Ser Leu Ile Ile Leu Asn His Pro Ser
1               5                   10                  15
Gln Gln Arg Cys Leu Leu Asp Glu Leu His Met His Arg Lys Tyr Ser
            20                  25                  30
Tyr Gly Phe Arg Leu Leu His Glu Phe Leu Phe Leu Leu Arg Pro Leu
        35                  40                  45
Ile Pro Glu Lys
    50

<210> 40
<211> 64
<212> PRT
<213> Artificial Sequence

<220>
<223> orf

<400> 40
Met Gly Phe Ser Ser Gly Leu Phe Tyr Val Leu Val Ala Val Gln Cys
1               5                   10                  15
Val Ser Ile Ala Ala Cys Pro Arg Ser Gln Ser Ala Pro Leu Leu Pro
            20                  25                  30
Arg Pro Leu Ser Pro Asn Arg Phe Val Arg Leu Leu Leu Val Val Glu
        35                  40                  45
Ala Pro Pro Arg Arg Arg Cys Pro Cys Tyr Val Ala Leu Arg Val Val
    50                  55                  60

<210> 41
<211> 82
<212> PRT
<213> Artificial Sequence

<220>
<223> orf

<400> 41
Met Ser Val Pro Ser Ser Asp Ser Leu Pro Asn Thr Asn Thr Gly Arg
1               5                   10                  15
Arg Trp Asp Ser Pro Val Gly Phe Phe Thr Ser Ser Leu Arg Cys Ser
            20                  25                  30
Ala Cys Gln Ser Leu Arg Ala Leu Ala Leu Asn Pro Leu Arg Ser Ser
        35                  40                  45
Leu Val Arg Tyr Arg Gln Ile Ala Ser Tyr Ala Phe Ser Ser Leu Ser

50                  55                  60
Lys Leu Leu Leu Val Val Val Val Leu Ala Met Trp Leu Tyr Val Ser
65                  70                  75                  80
Cys Asp

<210> 42
<211> 103
<212> PRT
<213> Artificial Sequence

<220>
<223> orf

<400> 42
Met Lys Ala Asn Ser Leu Cys Ser Thr Tyr Gly Asn Arg Ser Ile Leu
1               5                   10                  15
Tyr Lys Tyr Pro Ser His Leu Leu Ile Arg Cys Arg Thr Arg Thr Arg
            20                  25                  30
Val Glu Gly Gly Ile Leu Gln Trp Ala Phe Leu Arg Pro Arg Cys Gly
        35                  40                  45
Ala Val Arg Val Asn Arg Cys Val Pro Ser Leu Ser Ile Arg Ser Ala
    50                  55                  60
Pro Pro Ser Ser Val Ile Ala Lys Ser Leu Arg Thr Pro Ser Pro Arg
65                  70                  75                  80
Cys Arg Ser Ser Ser Ser Ser Ser Leu Ser Leu Leu Cys Gly Ser Thr
                85                  90                  95
Cys Arg Val Ile Ser Lys Ser
            100

<210> 43
<211> 64
<212> PRT
<213> Artificial Sequence

<220>
<223> orf

<400> 43
Met Ser Arg Tyr Gly Lys Ser Ser Asn Gly Ile Ser Ser Val Ile Val
1               5                   10                  15
Ser Gly Ile Leu Thr Pro His Ser Arg Pro Lys Glu Pro Val Lys Arg
            20                  25                  30
Leu Pro Leu Arg Thr Leu Asn Ala Ser Gln Val Phe Ser Thr Asp Arg
        35                  40                  45
Ser Pro Asn Phe Lys Ala Tyr Leu Gln Thr Ser Ala Ala Ala Leu Leu
    50                  55                  60

<210> 44
<211> 59
<212> PRT
<213> Artificial Sequence

<220>
<223> orf

<400> 44
Met Leu Ala Leu Glu Asn Leu Lys Arg Phe Pro Ser Leu Phe Asn Gly

1               5                   10                  15
Gln Ile Ala Lys Leu Gln Ser Ile Leu Thr Asp Phe Ser Ser Arg Phe
            20                  25                  30
Ala Ile Thr Val Thr Phe Phe Phe Phe Cys Pro Thr Asn Ile Asp Asn
        35                  40                  45
His Arg Val Ala Ser Lys Ala Met Pro Ile Phe
    50                  55

<210> 45
<211> 1206
<212> DNA
<213> Artificial Sequence


<220>
<223> orf

<400> 45
ataacacgtt ttgcgacttt gaaacatagg gctaagaatc aggaaaatta tttatttacc      60

ctggctaagt ttaaagagaa ctatgaaaaa gacgttaaaa acgaagaatc tatcaaagct     120

ctaaaatctg ctcagaaatt gaatgaatgc ggaaactatc tgctattcaa aaatttttac     180

acaataggcg aagttaaact ttctaagctc cgtacctgcg gacagcattt gctttgtcct     240

ttctgcgctg ctattcgtgc atctcgtgct attcaaaaat acgttgaacg tattgatcaa     300

gtcttacaag aaaatcgcaa gcttaagcct gttctaatca cgctcactgt taaaaacggt     360

tctgatctag cagaacgctc tgaacatctt atgaagtcct ttagaaagct cctagatcgt     420

cgtagggact atgaaaagaa agggcgaggt tttaacgagt tctgtaaggt tcaaggcgct     480

atgtattcat atgaaaatac atttaatgaa aaaacaggcg aatggcatcc tcatattcat     540

atgttcgctt tagttgataa ttggattgat cagcaagaat tttcagaata ttggcatagc     600

cttactggcg actctatggt tgtcgatgtt cgtagggcaa aaaaagaaaa aggtcacggt     660

tatagcaaag cggctgctga agtctgtaag tatgctttga agtttggcga tctgtccgtt     720

gaaaagactt gggaagcgtt taaggttctc aagggcaagc gtttaacagg ttcttttggt     780

ctgctatggg gtgtcaaaat ccctgacaca atgacagacg atatgccatt agaagatttg     840

ccatatctcg aaatgctcta caagttcgcc tacagtaaaa aatcctacta tgacttacta     900

atcacacgac acgtagagcc acatagcaag gacaacgacg acgaggagga gcttcgacaa     960

cgaggagaag gcgtacgaag cgatttggcg ataacggacg agggaggagc ggagcggatt    1020

gagagcgagg gcacgcagcg attgacacgc actgcaccgc aacgaggacg taaaaaagcc    1080

cactggagaa tcccaccttc tacccgtgtt cgtgttcggc aacgaatcag aagatgggac    1140

ggatatttat ataatattga cctattcccg tatgttgagc ataggctatt agccttcata    1200

gaagac                                                               1206


<210> 46
<211> 549

<212> DNA
<213> Artificial Sequence

<220>
<223> orf

<400> 46
atttctaagc gggagttttt agccatcccg aaagggtggc gacgcattga caattataat 60

ttaatctgca atattcataa gacattcata aataatctga ttggaatatt aaatatgctt 120

tttaaatctc taggcgttgc aatcgctaca aataatgctt ttaactctga cgaagacttg 180

aaaccaatta ctattcgttt agctgatagt gaaattcagg tgtatgacac tgttgcaaaa 240

tcaatgggtc taactcgtca agattttcta tcacacgtta ttcgttccaa cttccgacag 300

gctttaaaag agtttgtagt gggttataca agttcaaatc cttctacgcc tttgtctctt 360

ttatttaatt caaatacaga caatgacgaa gttaagctca aactttccaa ccttctttca 420

tctatttctc aggaattaat ggacgaagaa gaaaaagaaa ttcatgaagc aatcgaaaac 480

catatgaata cttcctatgc atatgcaact cgtccaaaag gcatctttgt tgagaaggat 540

gattcaaaa 549

<210> 47
<211> 291
<212> PRT
<213> Artificial Sequence

<220>
<223> orf

<400> 47
   Ala Thr Gly Ala Thr Thr Ala Ala Gly Cys Thr Thr Gly Ala Gly Gly
   1 5 10 15
   Gly Ala Ala Thr Cys Gly Thr Thr Thr Thr Ala Ala Ala Cys Gly Thr
   20 25 30
   Ala Thr Thr Thr Ala Cys Cys Cys Ala Gly Cys Ala Ala Gly Gly Cys
   35 40 45
   Gly Gly Thr Ala Ala Ala Gly Ala Thr Ala Ala Ala Ala Ala Ala Ala
   50 55 60
   Cys Ala Gly Gly Cys Gly Ala Thr Cys Ala Ala Thr Thr Thr Gly Ala
   65 70 75 80
   Ala Gly Ala Cys Cys Gly Thr Gly Ala Cys Ala Ala Gly Gly Thr Ala
   85 90 95
   Cys Ala Ala Ala Thr Thr Thr Thr Ala Gly Gly Thr Gly Cys Thr Ala
   100 105 110
   Thr Gly Gly Cys Thr Cys Thr Gly Cys Cys Thr Ala Ala Thr Gly Gly
   115 120 125
   Thr Gly Ala Cys Gly Thr Ala Ala Ala Ala Ala Ala Thr Gly Ala Ala
   130 135 140
   Cys Thr Thr Thr Thr Thr Ala Cys Gly Thr Thr Ala Thr Cys Ala Gly
   145 150 155 160
   Thr Ala Gly Ala Ala Ala Ala Thr Thr Ala Thr Cys Gly Gly Gly Ala
   165 170 175
   Cys Thr Thr Thr Ala Ala Ala Gly Ala Cys Thr Ala Cys Cys Thr Ala
   180 185 190
   Ala Ala Thr Cys Ala Gly Ala Ala Ala Ala Thr Thr Thr Cys Gly Ala
           195                 200                 205
   Thr Thr Gly Cys Thr Gly Thr Thr Gly Gly Thr Gly Cys Ala Ala Thr
       210                 215                 220
   Gly Gly Cys Ala Ala Gly Thr Gly Gly Thr Cys Gly Thr Ala Ala Thr
   225                 230                 235                 240
   Gly Thr Gly Ala Thr Thr Thr Thr Thr Thr Ala Thr Gly Thr Thr Gly
                   245                 250                 255
   Cys Thr Ala Ala Ala Gly Gly Thr Gly Cys Ala Ala Ala Ala Cys Cys
               260                 265                 270
   Thr Gly Thr Thr Thr Thr Ala Gly Cys Ala Gly Ala Gly Ala Cys Ala
           275                 280                 285
   Ala Thr Gly
       290

<210> 48
<211> 1308
<212> DNA
<213> Artificial Sequence

<220>
<223> orf

<400> 48
ttgcttaaaa aagacaataa aaaagcccat atttcagaag ctttggcggg cggagaaata      60

caggctttgc aatctgctaa ggcagatcaa cacagggata gaataacacg ttttgcgact     120

ttgaaacata gggctaagaa tcaggaaaat tatttattta ccctggctaa gtttaaagag     180

aactatgaaa aagacgttaa aaacgaagaa tctatcaaag ctctaaaatc tgctcagaaa     240

ttgaatgaat gcggaaacta tctgctattc aaaaattttt acacaatagg cgaagttaaa     300

ctttctaagc tccgtacctg cggacagcat ttgctttgtc ctttctgcgc tgctattcgt     360

gcatctcgtg ctattcaaaa atacgttgaa cgtattgatc aagtcttaca agaaaatcgc     420

aagcttaagc ctgttctaat cacgctcact gttaaaaacg gttctgatct agcagaacgc     480

tctgaacatc ttatgaagtc ctttagaaag ctcctagatc gtcgtaggga ctatgaaaag     540

aaagggcgag gttttaacga gttctgtaag gttcaaggcg ctatgtattc atatgaaaat     600

acatttaatg aaaaaacagg cgaatggcat cctcatattc atatgttcgc tttagttgat     660

aattggattg atcagcaaga attttcagaa tattggcata gccttactgg cgactctatg     720

gttgtcgatg ttcgtagggc aaaaaaagaa aaaggtcacg gttatagcaa agcggctgct     780

gaagtctgta agtatgcttt gaagtttggc gatctgtccg ttgaaaagac ttgggaagcg     840

tttaaggttc tcaagggcaa gcgtttaaca ggttcttttg gtctgctatg gggtgtcaaa     900

atccctgaca caatgacaga cgatatgcca ttagaagatt tgccatatct cgaaatgctc     960

tacaagttcg cctacagtaa aaaatcctac tatgacttac taatcacacg acacgtagag    1020

ccacatagca aggacaacga cgacgaggag gagcttcgac aacgaggaga aggcgtacga    1080

agcgatttgg cgataacgga cgagggagga gcggagcgga ttgagagcga gggcacgcag    1140

cgattgacac gcactgcacc gcaacgagga cgtaaaaaag cccactggag aatcccacct 1200

tctacccgtg ttcgtgttcg gcaacgaatc agaagatggg acggatattt atataatatt 1260

gacctattcc cgtatgttga gcataggcta ttagccttca tagaagac 1308

<210> 49
<211> 156
<212> DNA
<213> Artificial Sequence

<220>
<223> orf

<400> 49
acgtttaaaa cgattccctc aagcttaatc attttgaatc atccttctca acaaagatgc 60

cttttggacg agttgcatat gcataggaag tattcatatg gttttcgatt gcttcatgaa 120

tttctttttc ttcttcgtcc attaattcct gagaaa 156

<210> 50
<211> 192
<212> DNA
<213> Artificial Sequence

<220>
<223> orf

<400> 50
gtgggattct ccagtgggct tttttacgtc ctcgttgcgg tgcagtgcgt gtcaatcgct 60

gcgtgccctc gctctcaatc cgctccgctc ctccctcgtc cgttatcgcc aaatcgcttc 120

gtacgccttc tcctcgttgt cgaagctcct cctcgtcgtc gttgtccttg ctatgtggct 180

ctacgtgtcg tg 192

<210> 51
<211> 309
<212> DNA
<213> Artificial Sequence

<220>
<223> orf

<400> 51
atgaaggcta atagcctatg ctcaacatac gggaataggt caatattata taaatatccg 60

tcccatcttc tgattcgttg ccgaacacga acacgggtag aaggtgggat tctccagtgg 120

gcttttttac gtcctcgttg cggtgcagtg cgtgtcaatc gctgcgtgcc ctcgctctca 180

atccgctccg ctcctccctc gtccgttatc gccaaatcgc ttcgtacgcc ttctcctcgt 240

tgtcgaagct cctcctcgtc gtcgttgtcc ttgctatgtg gctctacgtg tcgtgtgatt 300

agtaagtca                                                          309

<210> 52
<211> 192
<212> DNA
<213> Artificial Sequence

<220>
<223> orf

<400> 52
atttcgagat atggcaaatc ttctaatggc atatcgtctg tcattgtgtc agggattttg      60

acaccccata gcagaccaaa agaacctgtt aaacgcttgc ccttgagaac cttaaacgct     120

tcccaagtct tttcaacgga cagatcgcca aacttcaaag catacttaca gacttcagca     180

gccgctttgc ta                                                         192

<210> 53
<211> 246
<212> DNA
<213> Artificial Sequence

<220>
<223> orf

<400> 53
atatccgtcc catcttctga ttcgttgccg aacacgaaca cgggtagaag gtgggattct      60

ccagtgggct tttttacgtc ctcgttgcgg tgcagtgcgt gtcaatcgct gcgtgccctc     120

gctctcaatc cgctccgctc ctccctcgtc cgttatcgcc aaatcgcttc gtacgccttc     180

tcctcgttgt cgaagctcct cctcgtcgtc gttgtccttg ctatgtggct ctacgtgtcg     240

tgtgat                                                                246

<210> 54
<211> 177
<212> DNA
<213> Artificial Sequence

<220>
<223> orf

<400> 54
acgcttgccc ttgagaacct taaacgcttc ccaagtcttt tcaacggaca gatcgccaaa      60

cttcaaagca tacttacaga cttcagcagc cgctttgcta taaccgtgac ctttttcttt     120

ttttgcccta cgaacatcga caaccataga gtcgccagta aggctatgcc aatattc        177

<210> 55
<211> 2661
<212> DNA

<213> Artificial Sequence

<220>
<223> isolate

<400> 55
caacacaggg atagaataac acgttttgcg actttgaaac atagagctaa gaatcaggaa 60

aattatttat ttaccctggc acaattcaaa gacaactatg aaaaagatgt taaaaacgag 120

gaatctatca aggctctaaa atctgctcag aaattgaatg aatgcggaaa ctatttgcta 180

ttcaaaaatt tttacacaat aggcgaagtt aagctctcca agctccgcac ctgtggacag 240

catttgcttt gtcctttctg cgctgctatc cgtgcatctc gtgcaattca aaaatacgtt 300

gaacgtatca atcaggttct tcaagagaat cgcaagctca agcctgtttt aatcacgctt 360

accgtcaaaa atggttcaga ccttgccgaa aggtcagaac accttatgaa atcgtttaga 420

acgcttctag agcgtcgtag ggactattta aaaaagggtt ggggttatag cgagttctgt 480

aaggttcaag gtgccatgta ctcttacgag aatacataca acgaaaaaac aggtgagtgg 540

catccgcata ttcatatgtt tgcattggtt gatcagtgga ttgatcagca ggaattttca 600

gaatattggc atagccttac tggggactcg atggttgttg acgtccgcag ggcgagaaaa 660

gaaaaaggac acggctatag taaagcggct gccgaagtct gtaagtatgc tttgaagttt 720

ggcgatctat ccgttgaaaa aacttgggaa gcattcaagg tactcaaggg caagcgttta 780

acaggttctt tcggtctttt atggggtgtc aaaattcctg aaactatgac tgatgatatg 840

cctgatgatg aaacactccc ttatatggaa atgctgtaca agttcgttta cggcaaaaag 900

tcacattatg acctagccat tactcgtatg gttgagccaa aggctaagga cgacaacgag 960

gagaaggagg caccgacgtc cgaccgaggc ggagaacgta gccgacaggc tcaaaaggac 1020

gatgcaatcg catccgacat cccgactcgc gggtgcggtg cgaagcgagg acgcaaaaaa 1080

gcccactgga gaatcccacc ttctacccgt gttcgggttc gtcaacgaat ccgaagatgg 1140

gacggatatt tatataatat tgacctattc ccttatgttg agcataggct attagccctc 1200

attggctagt agccgtttcg gagaaacgcg gaagcgtgca ttctgaaaat tctgctaaag 1260

attactcata taatctttag ttccttctcc ccagtagcta ggaatttaat atgactgaaa 1320

ttgaaaaatt gaagaaagag attgaagact taaaactgat tattgaaagt aaaaataatc 1380

aggttgaatc taaaaataag catattaaaa gacttgagag aatcatctcg aatatgaaaa 1440

aagaacaatc tcagcagtag ccgagggggc ggagtgcccc cgaactgaca taataaaaat 1500

ttcaaaacct tccttccctg attacaagct ccccttagat tctaagcggg agcttttagc 1560

catcccgaat gggtggcgac gcaaagaaaa tttgacaaag attctaggtg tattattatt 1620

cgtattacca atagaaaata tgcaccataa tttgaggaat aatctatgtg tcaatcattc 1680

ggtactatgg gacttgcaaa agctgaagct gaaattaacg ctcatgaaca agacagcgca 1740

tctgttgttt cttctgaaat caaacctttt acaactaaag cgcctactca cgttattgaa 1800

ggtttagacc ttcttgctgg tcattttgga atgactcgca atgcattggt tttaaagctc 1860

atcaatcaat atttgggtca ggcattcgct gaatactcta ctggctataa ctctgttttt 1920

cacaatcctg atgtttcaga tgttcagcaa atcattaatg acattgaaaa tctaactgaa 1980

cacagtgacg tttcagaaca agcgaaagaa tacgtaaatc gtttggctgt agatcatttg 2040

gtgaatacct aatgactgag atgcctaaag aacatttaga acaggcgaaa gaccttttcg 2100

cctacatcat tcaagaacat atgaaagacg accgatgtgt cacacacatc aagatattta 2160

aacaagatgg tcgattaaaa atattagagg taacagaaga atgctaaacc ttactggaac 2220

agtgattaac gtctttactc aacaaggcgg aacaaacaaa aaaggcgagt cttttgaaga 2280

ccgtgacaaa attcagctac tcggtgcttt agatctccca aacgggggaa tcaaaaacga 2340

gttagttact cttacagtcg aaaattatcg tgacttcaaa gatcttctaa atcaaaaaat 2400

ttctatccct gttggtgtta tggcaatggg tcgcaacgtg attttttacg ttgcaaaagg 2460

cgcacaacct caaattgtgc aggcataaga attcgttaaa aaaacagtga gttagcgaag 2520

caactcgctg tttttctttt tacttgatac tattgattaa agtggggatg aaaattgctt 2580

aaaaaagaca ataaaaaagc ccatatttcg aacactttgg cgggtggaga aatacaggct 2640

ttgcaatctg ctaaggcaga t 2661

<210> 56
<211> 82
<212> PRT
<213> Artificial Sequence

<220>
<223> orf

<400> 56
Met Ala Ser Ser Val Arg Phe Lys Val Pro Cys Thr Leu Thr Arg Ile
1               5                   10                  15
His Thr Thr Lys Lys Gln Val Ser Gly Ile Arg Ile Phe Ile Cys Leu
            20                  25                  30
His Trp Leu Ile Ser Gly Leu Ile Ser Arg Asn Phe Gln Asn Ile Gly
        35                  40                  45
Ile Ala Leu Leu Gly Thr Arg Trp Leu Leu Thr Ser Ala Gly Arg Glu
    50                  55                  60
Lys Lys Lys Asp Thr Ala Ile Val Lys Arg Leu Pro Lys Ser Val Ser
65                  70                  75                  80
Met Leu

<210> 57
<211> 87
<212> PRT
<213> Artificial Sequence

<220>
<223> orf

<400> 57
Met Ser Gln Arg Leu Arg Thr Thr Thr Arg Arg Arg Arg His Arg Arg
1               5                   10                  15
Pro Thr Glu Ala Glu Asn Val Ala Asp Arg Leu Lys Arg Thr Met Gln
            20                  25                  30
Ser His Pro Thr Ser Arg Leu Ala Gly Ala Val Arg Ser Glu Asp Ala
        35                  40                  45
Lys Lys Pro Thr Gly Glu Ser His Leu Leu Pro Val Phe Gly Phe Val
    50                  55                  60
Asn Glu Ser Glu Asp Gly Thr Asp Ile Tyr Ile Ile Leu Thr Tyr Ser
65                  70                  75                  80
Leu Met Leu Ser Ile Gly Tyr
                85

<210> 58
<211> 397
<212> PRT
<213> Artificial Sequence

<220>
<223> orf

<400> 58
Met Thr Arg Phe Ala Thr Leu Lys His Arg Ala Lys Asn Gln Glu Asn
1               5                   10                  15
Tyr Leu Phe Thr Leu Ala Gln Phe Lys Asp Asn Tyr Glu Lys Asp Val
            20                  25                  30
Lys Asn Glu Glu Ser Ile Lys Ala Leu Lys Ser Ala Gln Lys Leu Asn
        35                  40                  45
Glu Cys Gly Asn Tyr Leu Leu Phe Lys Asn Phe Tyr Thr Ile Gly Glu
    50                  55                  60
Val Lys Leu Ser Lys Leu Arg Thr Cys Gly Gln His Leu Leu Cys Pro
65                  70                  75                  80
Phe Cys Ala Ala Ile Arg Ala Ser Arg Ala Ile Gln Lys Tyr Val Glu
                85                  90                  95
Arg Ile Asn Gln Val Leu Gln Glu Asn Arg Lys Leu Lys Pro Val Leu
            100                 105                 110
Ile Thr Leu Thr Val Lys Asn Gly Ser Asp Leu Ala Glu Arg Ser Glu
        115                 120                 125
His Leu Met Lys Ser Phe Arg Thr Leu Leu Glu Arg Arg Arg Asp Tyr
    130                 135                 140
Leu Lys Lys Gly Trp Gly Tyr Ser Glu Phe Cys Lys Val Gln Gly Ala
145                 150                 155                 160
Met Tyr Ser Tyr Glu Asn Thr Tyr Asn Glu Lys Thr Gly Glu Trp His
                165                 170                 175
Pro His Ile His Met Phe Ala Leu Val Asp Gln Trp Ile Asp Gln Gln
            180                 185                 190
Glu Phe Ser Glu Tyr Trp His Ser Leu Thr Gly Asp Ser Met Val Val
        195                 200                 205
Asp Val Arg Arg Ala Arg Lys Glu Lys Gly His Gly Tyr Ser Lys Ala
    210                 215                 220
Ala Ala Glu Val Cys Lys Tyr Ala Leu Lys Phe Gly Asp Leu Ser Val
225                 230                 235                 240
Glu Lys Thr Trp Glu Ala Phe Lys Val Leu Lys Gly Lys Arg Leu Thr
                245                 250                 255
Gly Ser Phe Gly Leu Leu Trp Gly Val Lys Ile Pro Glu Thr Met Thr
            260                 265                 270
Asp Asp Met Pro Asp Asp Glu Thr Leu Pro Tyr Met Glu Met Leu Tyr

275                 280                 285
Lys Phe Val Tyr Gly Lys Lys Ser His Tyr Asp Leu Ala Ile Thr Arg
    290                 295                 300
Met Val Glu Pro Lys Ala Lys Asp Asp Asn Glu Glu Lys Glu Ala Pro
305                 310                 315                 320
Thr Ser Asp Arg Gly Gly Glu Arg Ser Arg Gln Ala Gln Lys Asp Asp
                325                 330                 335
Ala Ile Ala Ser Asp Ile Pro Thr Arg Gly Cys Gly Ala Lys Arg Gly
            340                 345                 350
Arg Lys Lys Ala His Trp Arg Ile Pro Pro Ser Thr Arg Val Arg Val
        355                 360                 365
Arg Gln Arg Ile Arg Arg Trp Asp Gly Tyr Leu Tyr Asn Ile Asp Leu
    370                 375                 380
Phe Pro Tyr Val Glu His Arg Leu Leu Ala Leu Ile Gly
385                 390                 395

<210> 59
<211> 119
<212> PRT
<213> Artificial Sequence

<220>
<223> orf

<400> 59
Met Ser Ala Val Ala Glu Gly Ala Glu Cys Pro Arg Thr Asp Ile Ile
1               5                   10                  15
Lys Ile Ser Lys Pro Ser Phe Pro Asp Tyr Lys Leu Pro Leu Asp Ser
            20                  25                  30
Lys Arg Glu Leu Leu Ala Ile Pro Asn Gly Trp Arg Arg Lys Glu Asn
        35                  40                  45
Leu Thr Lys Ile Leu Gly Val Leu Leu Phe Val Leu Pro Ile Glu Asn
    50                  55                  60
Met His His Asn Leu Arg Asn Asn Leu Cys Val Asn His Ser Val Leu
65                  70                  75                  80
Trp Asp Leu Gln Lys Leu Lys Leu Lys Leu Thr Leu Met Asn Lys Thr
                85                  90                  95
Ala His Leu Leu Phe Leu Leu Lys Ser Asn Leu Leu Gln Leu Lys Arg
            100                 105                 110
Leu Leu Thr Leu Leu Lys Val
        115

<210> 60
<211> 128
<212> PRT
<213> Artificial Sequence

<220>
<223> orf

<400> 60
Met Cys Gln Ser Phe Gly Thr Met Gly Leu Ala Lys Ala Glu Ala Glu
1               5                   10                  15
Ile Asn Ala His Glu Gln Asp Ser Ala Ser Val Val Ser Ser Glu Ile
            20                  25                  30
Lys Pro Phe Thr Thr Lys Ala Pro Thr His Val Ile Glu Gly Leu Asp
        35                  40                  45
Leu Leu Ala Gly His Phe Gly Met Thr Arg Asn Ala Leu Val Leu Lys
    50                  55                  60
Leu Ile Asn Gln Tyr Leu Gly Gln Ala Phe Ala Glu Tyr Ser Thr Gly
65                  70                  75                  80

Tyr Asn Ser Val Phe His Asn Pro Asp Val Ser Asp Val Gln Gln Ile
                85                  90                  95
Ile Asn Asp Ile Glu Asn Leu Thr Glu His Ser Asp Val Ser Glu Gln
            100                 105                 110
Ala Lys Glu Tyr Val Asn Arg Leu Ala Val Asp His Leu Val Asn Thr
        115                 120                 125

<210> 61
<211> 65
<212> PRT
<213> Artificial Sequence

<220>
<223> orf

<400> 61
Met Arg Lys Ser Phe Gly Cys Arg Ser Phe Gly Glu Tyr Leu Met Thr
1               5                   10                  15
Glu Met Pro Lys Glu His Leu Glu Gln Ala Lys Asp Leu Phe Ala Tyr
            20                  25                  30
Ile Ile Gln Glu His Met Lys Asp Asp Arg Cys Val Thr His Ile Lys
        35                  40                  45
Ile Phe Lys Gln Asp Gly Arg Leu Lys Ile Leu Glu Val Thr Glu Glu
    50                  55                  60
Cys
65

<210> 62
<211> 104
<212> PRT
<213> Artificial Sequence

<220>
<223> orf

<400> 62
Met Lys Asn Ile Arg Gly Asn Arg Arg Met Leu Asn Leu Thr Gly Thr
1               5                   10                  15
Val Ile Asn Val Phe Thr Gln Gln Gly Gly Thr Asn Lys Lys Gly Glu
            20                  25                  30
Ser Phe Glu Asp Arg Asp Lys Ile Gln Leu Leu Gly Ala Leu Asp Leu
        35                  40                  45
Pro Asn Gly Gly Ile Lys Asn Glu Leu Val Thr Leu Thr Val Glu Asn
    50                  55                  60
Tyr Arg Asp Phe Lys Asp Leu Leu Asn Gln Lys Ile Ser Ile Pro Val
65                  70                  75                  80
Gly Val Met Ala Met Gly Arg Asn Val Ile Phe Tyr Val Ala Lys Gly
                85                  90                  95
Ala Gln Pro Gln Ile Val Gln Ala
            100

<210> 63
<211> 431
<212> PRT
<213> Artificial Sequence

<220>
<223> orf

<400> 63
Met Leu Lys Lys Asp Asn Lys Lys Ala His Ile Ser Asn Thr Leu Ala
1               5                   10                  15
Gly Gly Glu Ile Gln Ala Leu Gln Ser Ala Lys Ala Asp Gln His Arg
            20                  25                  30
Asp Arg Ile Thr Arg Phe Ala Thr Leu Lys His Arg Ala Lys Asn Gln
        35                  40                  45
Glu Asn Tyr Leu Phe Thr Leu Ala Gln Phe Lys Asp Asn Tyr Glu Lys
    50                  55                  60
Asp Val Lys Asn Glu Glu Ser Ile Lys Ala Leu Lys Ser Ala Gln Lys
65                  70                  75                  80
Leu Asn Glu Cys Gly Asn Tyr Leu Leu Phe Lys Asn Phe Tyr Thr Ile
                85                  90                  95
Gly Glu Val Lys Leu Ser Lys Leu Arg Thr Cys Gly Gln His Leu Leu
            100                 105                 110
Cys Pro Phe Cys Ala Ala Ile Arg Ala Ser Arg Ala Ile Gln Lys Tyr
        115                 120                 125
Val Glu Arg Ile Asn Gln Val Leu Gln Glu Asn Arg Lys Leu Lys Pro
    130                 135                 140
Val Leu Ile Thr Leu Thr Val Lys Asn Gly Ser Asp Leu Ala Glu Arg
145                 150                 155                 160
Ser Glu His Leu Met Lys Ser Phe Arg Thr Leu Leu Glu Arg Arg Arg
                165                 170                 175
Asp Tyr Leu Lys Lys Gly Trp Gly Tyr Ser Glu Phe Cys Lys Val Gln
            180                 185                 190
Gly Ala Met Tyr Ser Tyr Glu Asn Thr Tyr Asn Glu Lys Thr Gly Glu
        195                 200                 205
Trp His Pro His Ile His Met Phe Ala Leu Val Asp Gln Trp Ile Asp
    210                 215                 220
Gln Gln Glu Phe Ser Glu Tyr Trp His Ser Leu Thr Gly Asp Ser Met
225                 230                 235                 240
Val Val Asp Val Arg Arg Ala Arg Lys Glu Lys Gly His Gly Tyr Ser
                245                 250                 255
Lys Ala Ala Ala Glu Val Cys Lys Tyr Ala Leu Lys Phe Gly Asp Leu
            260                 265                 270
Ser Val Glu Lys Thr Trp Glu Ala Phe Lys Val Leu Lys Gly Lys Arg
        275                 280                 285
Leu Thr Gly Ser Phe Gly Leu Leu Trp Gly Val Lys Ile Pro Glu Thr
    290                 295                 300
Met Thr Asp Asp Met Pro Asp Asp Glu Thr Leu Pro Tyr Met Glu Met
305                 310                 315                 320
Leu Tyr Lys Phe Val Tyr Gly Lys Lys Ser His Tyr Asp Leu Ala Ile
                325                 330                 335
Thr Arg Met Val Glu Pro Lys Ala Lys Asp Asp Asn Glu Glu Lys Glu
            340                 345                 350
Ala Pro Thr Ser Asp Arg Gly Gly Glu Arg Ser Arg Gln Ala Gln Lys
        355                 360                 365
Asp Asp Ala Ile Ala Ser Asp Ile Pro Thr Arg Gly Cys Gly Ala Lys
    370                 375                 380
Arg Gly Arg Lys Lys Ala His Trp Arg Ile Pro Pro Ser Thr Arg Val
385                 390                 395                 400
Arg Val Arg Gln Arg Ile Arg Arg Trp Asp Gly Tyr Leu Tyr Asn Ile
                405                 410                 415
Asp Leu Phe Pro Tyr Val Glu His Arg Leu Leu Ala Leu Ile Gly
            420                 425                 430

<210> 64
<211> 50
<212> PRT
<213> Artificial Sequence

<220>
<223> orf

<400> 64
Met Thr His Arg Leu Phe Leu Lys Leu Trp Cys Ile Phe Ser Ile Gly
1               5                   10                  15
Asn Thr Asn Asn Asn Thr Pro Arg Ile Phe Val Lys Phe Ser Leu Arg
            20                  25                  30
Arg His Pro Phe Gly Met Ala Lys Ser Ser Arg Leu Glu Ser Lys Gly
        35                  40                  45
Ser Leu
    50

<210> 65
<211> 51
<212> PRT
<213> Artificial Sequence

<220>
<223> orf

<400> 65
Met Lys Phe Leu Leu Cys Gln Phe Gly Gly Thr Pro Pro Pro Arg Leu
1               5                   10                  15
Leu Leu Arg Leu Phe Phe Phe His Ile Arg Asp Asp Ser Leu Lys Ser
            20                  25                  30
Phe Asn Met Leu Ile Phe Arg Phe Asn Leu Ile Ile Phe Thr Phe Asn
        35                  40                  45
Asn Gln Phe
    50

<210> 66
<211> 51
<212> PRT
<213> Artificial Sequence

<220>
<223> orf

<400> 66
Met Gly Phe Ser Ser Gly Leu Phe Cys Val Leu Ala Ser His Arg Thr
1               5                   10                  15
Arg Glu Ser Gly Cys Arg Met Arg Leu His Arg Pro Phe Glu Pro Val
            20                  25                  30
Gly Tyr Val Leu Arg Leu Gly Arg Thr Ser Val Pro Pro Ser Pro Arg
        35                  40                  45
Cys Arg Pro
    50

<210> 67
<211> 78
<212> PRT
<213> Artificial Sequence

<220>
<223> orf

<400> 67
Met Ser Val Pro Ser Ser Asp Ser Leu Thr Asn Pro Asn Thr Gly Arg
1               5                   10                  15

Arg Trp Asp Ser Pro Val Gly Phe Phe Ala Ser Ser Leu Arg Thr Ala
                20                  25                  30
Pro Ala Ser Arg Asp Val Gly Cys Asp Cys Ile Val Leu Leu Ser Leu
            35                  40                  45
Ser Ala Thr Phe Ser Ala Ser Val Gly Arg Arg Cys Leu Leu Leu Leu
    50                  55                  60
Val Val Val Leu Ser Leu Trp Leu Asn His Thr Ser Asn Gly
65                  70                  75

<210> 68
<211> 54
<212> PRT
<213> Artificial Sequence

<220>
<223> orf

<400> 68
Met Ser Ser Val Ile Val Ser Gly Ile Leu Thr Pro His Lys Arg Pro
1               5                   10                  15
Lys Glu Pro Val Lys Arg Leu Pro Leu Ser Thr Leu Asn Ala Ser Gln
                20                  25                  30
Val Phe Ser Thr Asp Arg Ser Pro Asn Phe Lys Ala Tyr Leu Gln Thr
            35                  40                  45
Ser Ala Ala Ala Leu Leu
    50

<210> 69
<211> 62
<212> PRT
<213> Artificial Sequence

<220>
<223> orf

<400> 69
Met Leu Lys Ile Pro Ala Asp Gln Ser Thr Asp Gln Pro Met Gln Thr
1               5                   10                  15
Tyr Glu Tyr Ala Asp Ala Thr His Leu Phe Phe Arg Cys Met Tyr Ser
                20                  25                  30
Arg Lys Ser Thr Trp His Leu Glu Pro Tyr Arg Thr Arg Tyr Asn Pro
            35                  40                  45
Asn Pro Phe Leu Asn Ser Pro Tyr Asp Ala Leu Glu Ala Phe
    50                  55                  60

<210> 70
<211> 50
<212> PRT
<213> Artificial Sequence

<220>
<223> orf

<400> 70
Met Leu Leu Lys Asn Leu Ile Asp Thr Phe Asn Val Phe Leu Asn Cys
1               5                   10                  15
Thr Arg Cys Thr Asp Ser Ser Ala Glu Arg Thr Lys Gln Met Leu Ser
                20                  25                  30
Thr Gly Ala Glu Leu Gly Glu Leu Asn Phe Ala Tyr Cys Val Lys Ile
            35                  40                  45

Phe Glu
    50

<210> 71
<211> 56
<212> PRT
<213> Artificial Sequence


<220>
<223> orf

<400> 71
Met Ile Phe Leu Ile Leu Ser Ser Met Phe Gln Ser Arg Lys Thr Cys
1               5                   10                  15
Tyr Ser Ile Pro Val Leu Ile Cys Leu Ser Arg Leu Gln Ser Leu Tyr
            20                  25                  30
Phe Ser Thr Arg Gln Ser Val Arg Asn Met Gly Phe Phe Ile Val Phe
        35                  40                  45
Phe Lys Gln Phe Ser Ser Pro Leu
    50                  55

<210> 72
<211> 246
<212> DNA
<213> Artificial Sequence


<220>
<223> orf

<400> 72
atagcgagtt ctgtaaggtt caaggtgcca tgtactctta cgagaataca tacaacgaaa      60

aaacaggtga gtggcatccg catattcata tgtttgcatt ggttgatcag tggattgatc     120

agcaggaatt ttcagaatat tggcatagcc ttactgggga ctcgatggtt gttgacgtcc     180

gcagggcgag aaaagaaaaa ggacacggct atagtaaagc ggctgccgaa gtctgtaagt     240

atgctt                                                                246


<210> 73
<211> 261
<212> DNA
<213> Artificial Sequence


<220>
<223> orf

<400> 73
ttgagccaaa ggctaaggac gacaacgagg agaaggaggc accgacgtcc gaccgaggcg      60

gagaacgtag ccgacaggct caaaaggacg atgcaatcgc atccgacatc ccgactcgcg     120

ggtgcggtgc gaagcgagga cgcaaaaaag cccactggag aatcccacct tctacccgtg     180

ttcgggttcg tcaacgaatc cgaagatggg acggatattt atataatatt gacctattcc     240

cttatgttga gcataggcta t                                               261

<210> 74
<211> 1191
<212> DNA
<213> Artificial Sequence

<220>
<223> orf

<400> 74
ataacacgtt ttgcgacttt gaaacataga gctaagaatc aggaaaatta tttatttacc 60

ctggcacaat tcaaagacaa ctatgaaaaa gatgttaaaa acgaggaatc tatcaaggct 120

ctaaaatctg ctcagaaatt gaatgaatgc ggaaactatt tgctattcaa aaatttttac 180

acaataggcg aagttaagct ctccaagctc cgcacctgtg gacagcattt gctttgtcct 240

ttctgcgctg ctatccgtgc atctcgtgca attcaaaaat acgttgaacg tatcaatcag 300

gttcttcaag agaatcgcaa gctcaagcct gttttaatca cgcttaccgt caaaaatggt 360

tcagaccttg ccgaaaggtc agaacacctt atgaaatcgt ttagaacgct tctagagcgt 420

cgtagggact atttaaaaaa gggttggggt tatagcgagt tctgtaaggt tcaaggtgcc 480

atgtactctt acgagaatac atacaacgaa aaaacaggtg agtggcatcc gcatattcat 540

atgtttgcat tggttgatca gtggattgat cagcaggaat tttcagaata ttggcatagc 600

cttactgggg actcgatggt tgttgacgtc cgcagggcga gaaaagaaaa aggacacggc 660

tatagtaaag cggctgccga agtctgtaag tatgctttga agtttggcga tctatccgtt 720

gaaaaaactt gggaagcatt caaggtactc aagggcaagc gtttaacagg ttctttcggt 780

cttttatggg gtgtcaaaat tcctgaaact atgactgatg atatgcctga tgatgaaaca 840

ctcccttata tggaaatgct gtacaagttc gtttacggca aaaagtcaca ttatgaccta 900

gccattactc gtatggttga gccaaaggct aaggacgaca acgaggagaa ggaggcaccg 960

acgtccgacc gaggcggaga acgtagccga caggctcaaa aggacgatgc aatcgcatcc 1020

gacatcccga ctcgcgggtg cggtgcgaag cgaggacgca aaaaagccca ctggagaatc 1080

ccaccttcta cccgtgttcg ggttcgtcaa cgaatccgaa gatgggacgg atatttatat 1140

aatattgacc tattccctta tgttgagcat aggctattag ccctcattgg c 1191

<210> 75
<211> 357
<212> DNA
<213> Artificial Sequence

<220>
<223> orf

<400> 75
atctcagcag tagccgaggg ggcggagtgc ccccgaactg acataataaa aatttcaaaa 60

ccttccttcc ctgattacaa gctcccctta gattctaagc gggagctttt agccatcccg 120

aatgggtggc gacgcaaaga aaatttgaca aagattctag gtgtattatt attcgtatta 180

ccaatagaaa atatgcacca taatttgagg aataatctat gtgtcaatca ttcggtacta 240

tgggacttgc aaaagctgaa gctgaaatta acgctcatga acaagacagc gcatctgttg 300

tttcttctga aatcaaacct tttacaacta aagcgcctac tcacgttatt gaaggtt 357

<210> 76
<211> 384
<212> DNA
<213> Artificial Sequence

<220>
<223> orf

<400> 76
atgtgtcaat cattcggtac tatgggactt gcaaaagctg aagctgaaat taacgctcat 60

gaacaagaca gcgcatctgt tgtttcttct gaaatcaaac cttttacaac taaagcgcct 120

actcacgtta ttgaaggttt agaccttctt gctggtcatt ttggaatgac tcgcaatgca 180

ttggttttaa agctcatcaa tcaatatttg ggtcaggcat tcgctgaata ctctactggc 240

tataactctg tttttcacaa tcctgatgtt tcagatgttc agcaaatcat taatgacatt 300

gaaaatctaa ctgaacacag tgacgtttca gaacaagcga aagaatacgt aaatcgtttg 360

gctgtagatc atttggtgaa tacc 384

<210> 77
<211> 195
<212> DNA
<213> Artificial Sequence

<220>
<223> orf

<400> 77
atacgtaaat cgtttggctg tagatcattt ggtgaatacc taatgactga gatgcctaaa 60

gaacatttag aacaggcgaa agaccttttc gcctacatca ttcaagaaca tatgaaagac 120

gaccgatgtg tcacacacat caagatattt aaacaagatg gtcgattaaa aatattagag 180

gtaacagaag aatgc 195

<210> 78
<211> 312
<212> DNA
<213> Artificial Sequence

<220>

<223> orf

<400> 78
attaaaaata ttagaggtaa cagaagaatg ctaaacctta ctggaacagt gattaacgtc 60

tttactcaac aaggcggaac aaacaaaaaa ggcgagtctt ttgaagaccg tgacaaaatt 120

cagctactcg gtgctttaga tctcccaaac gggggaatca aaaacgagtt agttactctt 180

acagtcgaaa attatcgtga cttcaaagat cttctaaatc aaaaaatttc tatccctgtt 240

ggtgttatgg caatgggtcg caacgtgatt ttttacgttg caaaaggcgc acaacctcaa 300

attgtgcagg ca 312

<210> 79
<211> 1293
<212> DNA
<213> Artificial Sequence

<220>
<223> orf

<400> 79
ttgcttaaaa aagacaataa aaaagcccat atttcgaaca ctttggcggg tggagaaata 60

caggctttgc aatctgctaa ggcagatcaa cacagggata gaataacacg ttttgcgact 120

ttgaaacata gagctaagaa tcaggaaaat tatttattta ccctggcaca attcaaagac 180

aactatgaaa aagatgttaa aaacgaggaa tctatcaagg ctctaaaatc tgctcagaaa 240

ttgaatgaat gcggaaacta tttgctattc aaaaattttt acacaatagg cgaagttaag 300

ctctccaagc tccgcacctg tggacagcat ttgctttgtc ctttctgcgc tgctatccgt 360

gcatctcgtg caattcaaaa atacgttgaa cgtatcaatc aggttcttca agagaatcgc 420

aagctcaagc ctgttttaat cacgcttacc gtcaaaaatg gttcagacct tgccgaaagg 480

tcagaacacc ttatgaaatc gtttagaacg cttctagagc gtcgtaggga ctatttaaaa 540

aagggttggg gttatagcga gttctgtaag gttcaaggtg ccatgtactc ttacgagaat 600

acatacaacg aaaaaacagg tgagtggcat ccgcatattc atatgtttgc attggttgat 660

cagtggattg atcagcagga attttcagaa tattggcata gccttactgg ggactcgatg 720

gttgttgacg tccgcagggc gagaaaagaa aaaggacacg gctatagtaa agcggctgcc 780

gaagtctgta agtatgcttt gaagtttggc gatctatccg ttgaaaaaac ttgggaagca 840

ttcaaggtac tcaagggcaa gcgtttaaca ggttctttcg gtcttttatg gggtgtcaaa 900

attcctgaaa ctatgactga tgatatgcct gatgatgaaa cactccctta tatggaaatg 960

ctgtacaagt tcgtttacgg caaaaagtca cattatgacc tagccattac tcgtatggtt 1020

gagccaaagg ctaaggacga caacgaggag aaggaggcac cgacgtccga ccgaggcgga 1080

gaacgtagcc gacaggctca aaaggacgat gcaatcgcat ccgacatccc gactcgcggg 1140

tgcggtgcga agcgaggacg caaaaaagcc cactggagaa tcccaccttc tacccgtgtt 1200

cgggttcgtc aacgaatccg aagatgggac ggatatttat ataatattga cctattccct 1260

tatgttgagc ataggctatt agccctcatt ggc 1293

<210> 80
<211> 150
<212> DNA
<213> Artificial Sequence

<220>
<223> orf

<400> 80
ttgacacata gattattcct caaattatgg tgcatatttt ctattggtaa tacgaataat 60

aatacaccta gaatctttgt caaattttct ttgcgtcgcc acccattcgg gatggctaaa 120

agctcccgct tagaatctaa ggggagcttg 150

<210> 81
<211> 153
<212> DNA
<213> Artificial Sequence

<220>
<223> orf

<400> 81
ttgaaatttt tattatgtca gttcgggggc actccgcccc ctcggctact gctgagattg 60

ttcttttttc atattcgaga tgattctctc aagtctttta atatgcttat ttttagattc 120

aacctgatta tttttacttt caataatcag ttt 153

<210> 82
<211> 152
<212> DNA
<213> Artificial Sequence

<220>
<223> orf

<400> 82
gtgggattct ccagtgggct tttttgcgtc ctcgcttcgc accgcacccg cgagtcggga 60

tgtcggatgc gattgcatcg tccttttgag cctgtcggct acgttctccg cctcggtcgg 120

acgtcggtgc ctccttctcc tcgttgtcgt cc 152

<210> 83
<211> 234
<212> DNA
<213> Artificial Sequence

<220>
<223> orf

<400> 83
atatccgtcc catcttcgga ttcgttgacg aacccgaaca cgggtagaag gtgggattct      60

ccagtgggct tttttgcgtc ctcgcttcgc accgcacccg cgagtcggga tgtcggatgc     120

gattgcatcg tccttttgag cctgtcggct acgttctccg cctcggtcgg acgtcggtgc     180

ctccttctcc tcgttgtcgt ccttagcctt tggctcaacc atacgagtaa tggc           234


<210> 84
<211> 162
<212> DNA
<213> Artificial Sequence


<220>
<223> orf

<400> 84
atatcatcag tcatagtttc aggaattttg acaccccata aaagaccgaa agaacctgtt      60

aaacgcttgc ccttgagtac cttgaatgct tcccaagttt tttcaacgga tagatcgcca     120

aacttcaaag catacttaca gacttcggca gccgctttac ta                        162


<210> 85
<211> 186
<212> DNA
<213> Artificial Sequence


<220>
<223> orf

<400> 85
attctgaaaa ttcctgctga tcaatccact gatcaaccaa tgcaaacata tgaatatgcg      60

gatgccactc acctgttttt tcgttgtatg tattctcgta agagtacatg gcaccttgaa     120

ccttacagaa ctcgctataa ccccaaccct tttttaaata gtccctacga cgctctagaa     180

gcgttc                                                                186


<210> 86
<211> 150
<212> DNA
<213> Artificial Sequence


<220>
<223> orf

<400> 86
attctcttga agaacctgat tgatacgttc aacgtatttt tgaattgcac gagatgcacg      60

gatagcagcg cagaaaggac aaagcaaatg ctgtccacag gtgcggagct tggagagctt 120

aacttcgcct attgtgtaaa aatttttgaa 150

<210> 87
<211> 168
<212> DNA
<213> Artificial Sequence

<220>
<223> orf

<400> 87
ataattttcc tgattcttag ctctatgttt caaagtcgca aaacgtgtta ttctatccct 60

gtgttgatct gccttagcag attgcaaagc ctgtatttct ccacccgcca aagtgttcga 120

aatatgggct tttttattgt cttttttaag caattttcat ccccactt 168

<210> 88
<211> 2362
<212> DNA
<213> Artificial Sequence

<220>
<223> isolate

<400> 88
aaggcagatc aacacaggga tagaataaca cgttttggga ttttgaaaca tagagcgaag 60

ctacaagaac aatatttgtg gacacaagtt gatttcaaat ctgaaggcga agacgatcta 120

tccaataagg ctcttaaggc tgcaacaaaa ttaaaaggtt gtggccagtt tcttatattc 180

agaaattact acaccatcga ccaagttaag ctcgaaaaat tccacgtatg tggacagcat 240

ttgctatgcc caatgtgtgc aggtattcgc gctgcacgtt caatgaagcg ctatttagac 300

cgtatccatg aattaatgcg tcagaaacgc ggtttaaagc ccgttcttat cactttaacg 360

gtcaagaatg gagaagacct agaagaacgc tataatcacc ttacaagctc atttagaacg 420

cttctatccc gttataggga ttataagaaa aaaggttggg gatttaatca attctgtaag 480

attgatggcg ctttctatac tactgaatac acttacaacg ataaaaccaa gcaatggcat 540

ccgcatattc atattttcgc tttactcaat gaatggatag accaggaaga acttgccgag 600

acttggcacg acattaccct ggattcttat atcgttgata ttcgcagagt taaaaaaacc 660

aaagaacacg gttatagcaa agctgttgca gaggtttgta aatacgctct taagtttagc 720

gatctgtcac tggagaacac atgggaagcc tttttgactc ttaaaggtaa acgccttaca 780

ggttctttcg gttctatgca tggagttaaa attccagata aggccacgga tgacatgcca 840

ctggaggagc tgccgtactt agaaatgttc tatcgttttg tatttgataa aaaatcctac 900

tacaacctag aaataacgaa agacgtaaag ccacaaacta aggaatgagg agtgaggagg 960

agctgcgacg accgacacga ccgaatgact ttgtgagtgg cgataaggac gaagcaatcg 1020

catccgacat cccggcttgc gggtgcggtg cgaagcgagg acgttaagca agcctacctg 1080

tatatacaga cttgtaaggg gttcgacccc ttagacccca aagggcgcac ttagccattg 1140

agcaaagctc aacggcagtg ctcccccaat caacagaggg ggtgtctacc cccgaactga 1200

ctcactcgaa tttcaaaact tccttccctg attacaagct ccccttagat ttctaagcgg 1260

gagtttttag caatcccgca gggttgcgac gcaaagataa tttgacaaag attctagatg 1320

tattattatt cgtattacga atagaaaata tgcaccataa tttgaggaat aatctatgtg 1380

tcaatcattc ggtactatgg gacttgcaaa agctgaagct gaaattaacg ctcatgaaca 1440

agacagcgca tctgctgttt cttctgaaat caaacctttt acaactaaag cgcctactca 1500

cgttattgaa ggtttagacc ttcttgctgg tcattttgga atgactcgca atgcattggt 1560

tttaaagctc atcaatcaat atttgggtca ggcattcgct gaatactcta ctggctataa 1620

ctctgttttt cacaatcccg atgtttcaga tgttcagcaa atcattaatg acattgaaaa 1680

tctaactgaa cacagtgacg tttcagaaca agcgaaagaa tacgtaaatc gtttggttgt 1740

agatcatttg gtgaatacct aatgactgag atgcctaaag aacatttgga acaagcgaaa 1800

gaccttttcg ctcggattat ccaagaacac atgaaagaca accgcacact tacagccata 1860

aaaatcttta aagaagatgg tcgtttaaaa attttggagt tatcagatga ttaaattaga 1920

aggcacagta ttaaacgttt ttactcaaca aggcggtaaa gacaaaaaga caggtgatca 1980

atttgctgac cgtgacaagg tgcaaatttt gggtgctatg gatctgccta acggtgacat 2040

taaaaatgag ttatttacgt tatctgtaga aaactatcgg gactttaaag acttcttaaa 2100

tcagaaaatc tatattgctg ttggtgcaat ggcaagtggt cgaaatgtta ttttttacgt 2160

tgctaaaggc gcacagcctg ttttagcaga aacaatgtga aacatgaaaa aaaacagtga 2220

gttagccttg gcttctcgct gttttttctt tctcttgata ctatcaacta aagtggggat 2280

gaaattgact aaaaaagaca ataaaaaggc ccataattct gacactttgg cgggtgatga 2340

attacaggct ttgcaatctg ct 2362

<210> 89
<211> 62
<212> PRT
<213> Artificial Sequence

<220>
<223> orf

<400> 89
Met Ile Thr Leu Gln Ala His Leu Glu Arg Phe Tyr Pro Val Ile Gly
1               5                   10                  15
Ile Ile Arg Lys Lys Val Gly Asp Leu Ile Asn Ser Val Arg Leu Met

                    20                  25                  30
Ala Leu Ser Ile Leu Leu Asn Thr Leu Thr Thr Ile Lys Pro Ser Asn
            35                  40                  45
Gly Ile Arg Ile Phe Ile Phe Ser Leu Tyr Ser Met Asn Gly
    50                  55                  60

<210> 90
<211> 54
<212> PRT
<213> Artificial Sequence

<220>
<223> orf

<400> 90
Met Pro Arg Leu Gly Thr Thr Leu Pro Trp Ile Leu Ile Ser Leu Ile
1               5                   10                  15
Phe Ala Glu Leu Lys Lys Pro Lys Asn Thr Val Ile Ala Lys Leu Leu
                20                  25                  30
Gln Arg Phe Val Asn Thr Leu Leu Ser Leu Ala Ile Cys His Trp Arg
            35                  40                  45
Thr His Gly Lys Pro Phe
    50

<210> 91
<211> 307
<212> PRT
<213> Artificial Sequence

<220>
<223> orf

<400> 91
Met Thr Arg Phe Gly Ile Leu Lys His Arg Ala Lys Leu Gln Glu Gln
1               5                   10                  15
Tyr Leu Trp Thr Gln Val Asp Phe Lys Ser Glu Gly Glu Asp Asp Leu
                20                  25                  30
Ser Asn Lys Ala Leu Lys Ala Ala Thr Lys Leu Lys Gly Cys Gly Gln
            35                  40                  45
Phe Leu Ile Phe Arg Asn Tyr Tyr Thr Ile Asp Gln Val Lys Leu Glu
    50                  55                  60
Lys Phe His Val Cys Gly Gln His Leu Leu Cys Pro Met Cys Ala Gly
65                  70                  75                  80
Ile Arg Ala Ala Arg Ser Met Lys Arg Tyr Leu Asp Arg Ile His Glu
                85                  90                  95
Leu Met Arg Gln Lys Arg Gly Leu Lys Pro Val Leu Ile Thr Leu Thr
            100                 105                 110
Val Lys Asn Gly Glu Asp Leu Glu Glu Arg Tyr Asn His Leu Thr Ser
        115                 120                 125
Ser Phe Arg Thr Leu Leu Ser Arg Tyr Arg Asp Tyr Lys Lys Lys Gly
    130                 135                 140
Trp Gly Phe Asn Gln Phe Cys Lys Ile Asp Gly Ala Phe Tyr Thr Thr
145                 150                 155                 160
Glu Tyr Thr Tyr Asn Asp Lys Thr Lys Gln Trp His Pro His Ile His
                165                 170                 175
Ile Phe Ala Leu Leu Asn Glu Trp Ile Asp Gln Glu Glu Leu Ala Glu
            180                 185                 190
Thr Trp His Asp Ile Thr Leu Asp Ser Tyr Ile Val Asp Ile Arg Arg
        195                 200                 205
Val Lys Lys Thr Lys Glu His Gly Tyr Ser Lys Ala Val Ala Glu Val
    210                 215                 220

Cys Lys Tyr Ala Leu Lys Phe Ser Asp Leu Ser Leu Glu Asn Thr Trp
225                 230                 235                 240
Glu Ala Phe Leu Thr Leu Lys Gly Lys Arg Leu Thr Gly Ser Phe Gly
                245                 250                 255
Ser Met His Gly Val Lys Ile Pro Asp Lys Ala Thr Asp Asp Met Pro
            260                 265                 270
Leu Glu Glu Leu Pro Tyr Leu Glu Met Phe Tyr Arg Phe Val Phe Asp
        275                 280                 285
Lys Lys Ser Tyr Tyr Asn Leu Glu Ile Thr Lys Asp Val Lys Pro Gln
    290                 295                 300
Thr Lys Glu
305

<210> 92
<211> 77
<212> PRT
<213> Artificial Sequence

<220>
<223> orf 4

<400> 92
Met Arg Arg Lys Asp Asn Leu Thr Lys Ile Leu Asp Val Leu Leu Phe
1               5                   10                  15
Val Leu Arg Ile Glu Asn Met His His Asn Leu Arg Asn Asn Leu Cys
            20                  25                  30
Val Asn His Ser Val Leu Trp Asp Leu Gln Lys Leu Lys Leu Lys Leu
        35                  40                  45
Thr Leu Met Asn Lys Thr Ala His Leu Leu Phe Leu Leu Lys Ser Asn
    50                  55                  60
Leu Leu Gln Leu Lys Arg Leu Leu Thr Leu Leu Lys Val
65                  70                  75

<210> 93
<211> 128
<212> PRT
<213> Artificial Sequence

<220>
<223> orf

<400> 93
Met Cys Gln Ser Phe Gly Thr Met Gly Leu Ala Lys Ala Glu Ala Glu
1               5                   10                  15
Ile Asn Ala His Glu Gln Asp Ser Ala Ser Ala Val Ser Ser Glu Ile
            20                  25                  30
Lys Pro Phe Thr Thr Lys Ala Pro Thr His Val Ile Glu Gly Leu Asp
        35                  40                  45
Leu Leu Ala Gly His Phe Gly Met Thr Arg Asn Ala Leu Val Leu Lys
    50                  55                  60
Leu Ile Asn Gln Tyr Leu Gly Gln Ala Phe Ala Glu Tyr Ser Thr Gly
65                  70                  75                  80
Tyr Asn Ser Val Phe His Asn Pro Asp Val Ser Asp Val Gln Gln Ile
                85                  90                  95
Ile Asn Asp Ile Glu Asn Leu Thr Glu His Ser Asp Val Ser Glu Gln
            100                 105                 110
Ala Lys Glu Tyr Val Asn Arg Leu Val Val Asp His Leu Val Asn Thr
        115                 120                 125

<210> 94
<211> 64
<212> PRT
<213> Artificial Sequence

<220>
<223> orf

<400> 94
Met Arg Lys Ser Phe Gly Cys Arg Ser Phe Gly Glu Tyr Leu Met Thr
1               5                   10                  15
Glu Met Pro Lys Glu His Leu Glu Gln Ala Lys Asp Leu Phe Ala Arg
            20                  25                  30
Ile Ile Gln Glu His Met Lys Asp Asn Arg Thr Leu Thr Ala Ile Lys
        35                  40                  45
Ile Phe Lys Glu Asp Gly Arg Leu Lys Ile Leu Glu Leu Ser Asp Asp
    50                  55                  60

<210> 95
<211> 97
<212> PRT
<213> Artificial Sequence

<220>
<223> orf

<400> 95
Met Ile Lys Leu Glu Gly Thr Val Leu Asn Val Phe Thr Gln Gln Gly
1               5                   10                  15
Gly Lys Asp Lys Lys Thr Gly Asp Gln Phe Ala Asp Arg Asp Lys Val
            20                  25                  30
Gln Ile Leu Gly Ala Met Asp Leu Pro Asn Gly Asp Ile Lys Asn Glu
        35                  40                  45
Leu Phe Thr Leu Ser Val Glu Asn Tyr Arg Asp Phe Lys Asp Phe Leu
    50                  55                  60
Asn Gln Lys Ile Tyr Ile Ala Val Gly Ala Met Ala Ser Gly Arg Asn
65                  70                  75                  80
Val Ile Phe Tyr Val Ala Lys Gly Ala Gln Pro Val Leu Ala Glu Thr
                85                  90                  95
Met

<210> 96
<211> 368
<212> PRT
<213> Artificial Sequence

<220>
<223> orf

<400> 96
Met Lys Lys Asn Ser Glu Leu Ala Leu Ala Ser Arg Cys Phe Phe Phe
1               5                   10                  15
Leu Leu Ile Leu Ser Thr Lys Val Gly Met Lys Leu Thr Lys Lys Asp
            20                  25                  30
Asn Lys Lys Ala His Asn Ser Asp Thr Leu Ala Gly Asp Glu Leu Gln
        35                  40                  45
Ala Leu Gln Ser Ala Lys Ala Asp Gln His Arg Asp Arg Ile Thr Arg

50                  55                  60
Phe Gly Ile Leu Lys His Arg Ala Lys Leu Gln Glu Gln Tyr Leu Trp
65                  70                  75                  80
Thr Gln Val Asp Phe Lys Ser Glu Gly Glu Asp Asp Leu Ser Asn Lys
                85                  90                  95
Ala Leu Lys Ala Ala Thr Lys Leu Lys Gly Cys Gly Gln Phe Leu Ile
            100                 105                 110
Phe Arg Asn Tyr Tyr Thr Ile Asp Gln Val Lys Leu Glu Lys Phe His
        115                 120                 125
Val Cys Gly Gln His Leu Leu Cys Pro Met Cys Ala Gly Ile Arg Ala
    130                 135                 140
Ala Arg Ser Met Lys Arg Tyr Leu Asp Arg Ile His Glu Leu Met Arg
145                 150                 155                 160
Gln Lys Arg Gly Leu Lys Pro Val Leu Ile Thr Leu Thr Val Lys Asn
                165                 170                 175
Gly Glu Asp Leu Glu Glu Arg Tyr Asn His Leu Thr Ser Ser Phe Arg
            180                 185                 190
Thr Leu Leu Ser Arg Tyr Arg Asp Tyr Lys Lys Lys Gly Trp Gly Phe
        195                 200                 205
Asn Gln Phe Cys Lys Ile Asp Gly Ala Phe Tyr Thr Thr Glu Tyr Thr
    210                 215                 220
Tyr Asn Asp Lys Thr Lys Gln Trp His Pro His Ile His Ile Phe Ala
225                 230                 235                 240
Leu Leu Asn Glu Trp Ile Asp Gln Glu Glu Leu Ala Glu Thr Trp His
                245                 250                 255
Asp Ile Thr Leu Asp Ser Tyr Ile Val Asp Ile Arg Arg Val Lys Lys
            260                 265                 270
Thr Lys Glu His Gly Tyr Ser Lys Ala Val Ala Glu Val Cys Lys Tyr
        275                 280                 285
Ala Leu Lys Phe Ser Asp Leu Ser Leu Glu Asn Thr Trp Glu Ala Phe
    290                 295                 300
Leu Thr Leu Lys Gly Lys Arg Leu Thr Gly Ser Phe Gly Ser Met His
305                 310                 315                 320
Gly Val Lys Ile Pro Asp Lys Ala Thr Asp Asp Met Pro Leu Glu Glu
                325                 330                 335
Leu Pro Tyr Leu Glu Met Phe Tyr Arg Phe Val Phe Asp Lys Lys Ser
            340                 345                 350
Tyr Tyr Asn Leu Glu Ile Thr Lys Asp Val Lys Pro Gln Thr Lys Glu
        355                 360                 365

<210> 97
<211> 56
<212> PRT
<213> Artificial Sequence

<220>
<223> orf

<400> 97
Met Ser Glu Leu Trp Ala Phe Leu Leu Ser Phe Leu Val Asn Phe Ile
1               5                   10                  15
Pro Thr Leu Val Asp Ser Ile Lys Arg Lys Lys Lys Gln Arg Glu Ala
            20                  25                  30
Lys Ala Asn Ser Leu Phe Phe Phe Met Phe His Ile Val Ser Ala Lys
        35                  40                  45
Thr Gly Cys Ala Pro Leu Ala Thr
    50                  55

<210> 98
<211> 75

<212> PRT
<213> Artificial Sequence

<220>
<223> orf

<400> 98
Met Phe Lys Arg Pro Ser Ser Leu Lys Ile Phe Met Ala Val Ser Val
1               5                   10                  15
Arg Leu Ser Phe Met Cys Ser Trp Ile Ile Arg Ala Lys Arg Ser Phe
            20                  25                  30
Ala Cys Ser Lys Cys Ser Leu Gly Ile Ser Val Ile Arg Tyr Ser Pro
        35                  40                  45
Asn Asp Leu Gln Pro Asn Asp Leu Arg Ile Leu Ser Leu Val Leu Lys
    50                  55                  60
Arg His Cys Val Gln Leu Asp Phe Gln Cys His
65                  70                  75

<210> 99
<211> 60
<212> PRT
<213> Artificial Sequence

<220>
<223> orf

<400> 99
Met Tyr Ile Gln Val Gly Leu Leu Asn Val Leu Ala Ser His Arg Thr
1               5                   10                  15
Arg Lys Pro Gly Cys Arg Met Arg Leu Leu Arg Pro Tyr Arg His Ser
            20                  25                  30
Gln Ser His Ser Val Val Ser Val Val Ala Ala Pro Pro His Ser Ser
        35                  40                  45
Phe Leu Ser Leu Trp Leu Tyr Val Phe Arg Tyr Phe
    50                  55                  60

<210> 100
<211> 54
<212> PRT
<213> Artificial Sequence

<220>
<223> orf

<400> 100
Met Ser Ser Leu Arg Thr Ala Pro Ala Ser Arg Asp Val Gly Cys Asp
1               5                   10                  15
Cys Phe Val Leu Ile Ala Thr His Lys Val Ile Arg Ser Cys Arg Ser
            20                  25                  30
Ser Gln Leu Leu Leu Thr Pro His Ser Leu Val Cys Gly Phe Thr Ser
        35                  40                  45
Phe Val Ile Ser Arg Leu
    50

<210> 101
<211> 64
<212> PRT
<213> Artificial Sequence

<220>
<223> orf

<400> 101
Met Ser Lys Tyr Gly Ser Ser Ser Ser Gly Met Ser Ser Val Ala Leu
1               5                   10                  15
Ser Gly Ile Leu Thr Pro Cys Ile Glu Pro Lys Glu Pro Val Arg Arg
            20                  25                  30
Leu Pro Leu Arg Val Lys Lys Ala Ser His Val Phe Ser Ser Asp Arg
        35                  40                  45
Ser Leu Asn Leu Arg Ala Tyr Leu Gln Thr Ser Ala Thr Ala Leu Leu
    50                  55                  60

<210> 102
<211> 53
<212> PRT
<213> Artificial Sequence

<220>
<223> orf

<400> 102
Met Ala Lys Leu Lys Ser Val Phe Thr Asn Leu Cys Asn Ser Phe Ala
1               5                   10                  15
Ile Thr Val Phe Phe Gly Phe Phe Asn Ser Ala Asn Ile Asn Asp Ile
            20                  25                  30
Arg Ile Gln Gly Asn Val Val Pro Ser Leu Gly Lys Phe Phe Leu Val
        35                  40                  45
Tyr Pro Phe Ile Glu
    50

<210> 103
<211> 57
<212> PRT
<213> Artificial Sequence

<220>
<223> orf

<400> 103
Met Asn Ile Arg Asn Trp Pro Gln Pro Phe Asn Phe Val Ala Ala Leu
1               5                   10                  15
Arg Ala Leu Leu Asp Arg Ser Ser Ser Pro Ser Asp Leu Lys Ser Thr
            20                  25                  30
Cys Val His Lys Tyr Cys Ser Cys Ser Phe Ala Leu Cys Phe Lys Ile
        35                  40                  45
Pro Lys Arg Val Ile Leu Ser Leu Cys
    50                  55

<210> 104
<211> 186
<212> DNA
<213> Artificial Sequence

<220>
<223> orf

<400> 104

ataatcacct tacaagctca tttagaacgc ttctatcccg ttatagggat tataagaaaa 60

aaggttgggg atttaatcaa ttctgtaaga ttgatggcgc tttctatact actgaataca 120

cttacaacga taaaaccaag caatggcatc cgcatattca tattttcgct ttactcaatg 180

aatgga 186

<210> 105
<211> 162
<212> DNA
<213> Artificial Sequence

<220>
<223> orf

<400> 105
ttgccgagac ttggcacgac attaccctgg attcttatat cgttgatatt cgcagagtta 60

aaaaaaccaa agaacacggt tatagcaaag ctgttgcaga ggtttgtaaa tacgctctta 120

agtttagcga tctgtcactg gagaacacat gggaagcctt tt 162

<210> 106
<211> 921
<212> DNA
<213> Artificial Sequence

<220>
<223> orf

<400> 106
ataacacgtt ttgggatttt gaaacataga gcgaagctac aagaacaata tttgtggaca 60

caagttgatt tcaaatctga aggcgaagac gatctatcca ataaggctct taaggctgca 120

acaaaattaa aaggttgtgg ccagtttctt atattcagaa attactacac catcgaccaa 180

gttaagctcg aaaaattcca cgtatgtgga cagcatttgc tatgcccaat gtgtgcaggt 240

attcgcgctg cacgttcaat gaagcgctat ttagaccgta tccatgaatt aatgcgtcag 300

aaacgcggtt taaagcccgt tcttatcact ttaacggtca agaatggaga agacctagaa 360

gaacgctata atcaccttac aagctcattt agaacgcttc tatcccgtta tagggattat 420

aagaaaaaag gttggggatt taatcaattc tgtaagattg atggcgcttt ctatactact 480

gaatacactt acaacgataa aaccaagcaa tggcatccgc atattcatat tttcgcttta 540

ctcaatgaat ggatagacca ggaagaactt gccgagactt ggcacgacat taccctggat 600

tcttatatcg ttgatattcg cagagttaaa aaaaccaaag aacacggtta tagcaaagct 660

gttgcagagg tttgtaaata cgctcttaag tttagcgatc tgtcactgga gaacacatgg 720

gaagcctttt tgactcttaa aggtaaacgc cttacaggtt ctttcggttc tatgcatgga 780

gttaaaattc cagataaggc cacggatgac atgccactgg aggagctgcc gtacttagaa 840

atgttctatc gttttgtatt tgataaaaaa tcctactaca acctagaaat aacgaaagac 900

gtaaagccac aaactaagga a 921

<210> 107
<211> 231
<212> DNA
<213> Artificial Sequence

<220>
<223> orf

<400> 107
ttgcgacgca aagataattt gacaaagatt ctagatgtat tattattcgt attacgaata 60

gaaaatatgc accataattt gaggaataat ctatgtgtca atcattcggt actatgggac 120

ttgcaaaagc tgaagctgaa attaacgctc atgaacaaga cagcgcatct gctgtttctt 180

ctgaaatcaa accttttaca actaaagcgc ctactcacgt tattgaaggt t 231

<210> 108
<211> 384
<212> DNA
<213> Artificial Sequence

<220>
<223> orf

<400> 108
atgtgtcaat cattcggtac tatgggactt gcaaaagctg aagctgaaat taacgctcat 60

gaacaagaca gcgcatctgc tgtttcttct gaaatcaaac cttttacaac taaagcgcct 120

actcacgtta ttgaaggttt agaccttctt gctggtcatt ttggaatgac tcgcaatgca 180

ttggttttaa agctcatcaa tcaatatttg ggtcaggcat tcgctgaata ctctactggc 240

tataactctg tttttcacaa tcccgatgtt tcagatgttc agcaaatcat taatgacatt 300

gaaaatctaa ctgaacacag tgacgtttca gaacaagcga aagaatacgt aaatcgtttg 360

gttgtagatc atttggtgaa tacc 384

<210> 109
<211> 192
<212> DNA
<213> Artificial Sequence

<220>
<223> orf

<400> 109
atacgtaaat cgtttggttg tagatcattt ggtgaatacc taatgactga gatgcctaaa 60

gaacatttgg aacaagcgaa agaccttttc gctcggatta tccaagaaca catgaaagac 120

aaccgcacac ttacagccat aaaaatcttt aaagaagatg gtcgtttaaa aattttggag     180

ttatcagatg at                                                         192

<210> 110
<211> 291
<212> DNA
<213> Artificial Sequence

<220>
<223> orf

<400> 110
atgattaaat tagaaggcac agtattaaac gtttttactc aacaaggcgg taaagacaaa      60

aagacaggtg atcaatttgc tgaccgtgac aaggtgcaaa ttttgggtgc tatggatctg     120

cctaacggtg acattaaaaa tgagttattt acgttatctg tagaaaacta tcgggacttt     180

aaagacttct taaatcagaa aatctatatt gctgttggtg caatggcaag tggtcgaaat     240

gttatttttt acgttgctaa aggcgcacag cctgttttag cagaaacaat g              291

<210> 111
<211> 1104
<212> DNA
<213> Artificial Sequence

<220>
<223> orf

<400> 111
atgaaaaaaa acagtgagtt agccttggct tctcgctgtt ttttctttct cttgatacta      60

tcaactaaag tggggatgaa attgactaaa aaagacaata aaaaggccca taattctgac     120

actttggcgg gtgatgaatt acaggctttg caatctgcta aggcagatca acacagggat     180

agaataacac gttttgggat tttgaaacat agagcgaagc tacaagaaca atatttgtgg     240

acacaagttg atttcaaatc tgaaggcgaa gacgatctat ccaataaggc tcttaaggct     300

gcaacaaaat taaaaggttg tggccagttt cttatattca gaaattacta caccatcgac     360

caagttaagc tcgaaaaatt ccacgtatgt ggacagcatt tgctatgccc aatgtgtgca     420

ggtattcgcg ctgcacgttc aatgaagcgc tatttagacc gtatccatga attaatgcgt     480

cagaaacgcg gtttaaagcc cgttcttatc actttaacgg tcaagaatgg agaagaccta     540

gaagaacgct ataatcacct tacaagctca tttagaacgc ttctatcccg ttatagggat     600

tataagaaaa aaggttgggg atttaatcaa ttctgtaaga ttgatggcgc tttctatact     660

actgaataca cttacaacga taaaaccaag caatggcatc cgcatattca tattttcgct     720

ttactcaatg aatggataga ccaggaagaa cttgccgaga cttggcacga cattaccctg     780

gattcttata tcgttgatat tcgcagagtt aaaaaaacca aagaacacgg ttatagcaaa 840

gctgttgcag aggtttgtaa atacgctctt aagtttagcg atctgtcact ggagaacaca 900

tgggaagcct ttttgactct taaaggtaaa cgccttacag gttctttcgg ttctatgcat 960

ggagttaaaa ttccagataa ggccacggat gacatgccac tggaggagct gccgtactta 1020

gaaatgttct atcgttttgt atttgataaa aaatcctact acaacctaga aataacgaaa 1080

gacgtaaagc cacaaactaa ggaa 1104

<210> 112
<211> 168
<212> DNA
<213> Artificial Sequence

<220>
<223> orf

<400> 112
gtgtcagaat tatgggcctt tttattgtct tttttagtca atttcatccc cactttagtt 60

gatagtatca agagaaagaa aaaacagcga gaagccaagg ctaactcact gttttttttc 120

atgtttcaca ttgtttctgc taaaacaggc tgtgcgcctt tagcaacg 168

<210> 113
<211> 225
<212> DNA
<213> Artificial Sequence

<220>
<223> orf

<400> 113
atttttaaac gaccatcttc tttaaagatt tttatggctg taagtgtgcg gttgtctttc 60

atgtgttctt ggataatccg agcgaaaagg tctttcgctt gttccaaatg ttctttaggc 120

atctcagtca ttaggtattc accaaatgat ctacaaccaa acgatttacg tattctttcg 180

cttgttctga aacgtcactg tgttcagtta gattttcaat gtcat 225

<210> 114
<211> 180
<212> DNA
<213> Artificial Sequence

<220>
<223> orf

<400> 114
ctgtatatac aggtaggctt gcttaacgtc ctcgcttcgc accgcacccg caagccggga 60

tgtcggatgc gattgcttcg tccttatcgc cactcacaaa gtcattcggt cgtgtcggtc 120

gtcgcagctc ctcctcactc ctcattcctt agtttgtggc tttacgtctt tcgttatttc     180

<210> 115
<211> 162
<212> DNA
<213> Artificial Sequence

<220>
<223> orf

<400> 115
acgtcctcgc ttcgcaccgc acccgcaagc cgggatgtcg gatgcgattg cttcgtcctt      60

atcgccactc acaaagtcat tcggtcgtgt cggtcgtcgc agctcctcct cactcctcat     120

tccttagttt gtggctttac gtctttcgtt atttctaggt tg                        162

<210> 116
<211> 192
<212> DNA
<213> Artificial Sequence

<220>
<223> orf

<400> 116
atttctaagt acggcagctc ctccagtggc atgtcatccg tggccttatc tggaatttta      60

actccatgca tagaaccgaa agaacctgta aggcgtttac ctttaagagt caaaaaggct     120

tcccatgtgt tctccagtga cagatcgcta aacttaagag cgtatttaca aacctctgca     180

acagctttgc ta                                                         192

<210> 117
<211> 159
<212> DNA
<213> Artificial Sequence

<220>
<223> orf

<400> 117
atcgctaaac ttaagagcgt atttacaaac ctctgcaaca gctttgctat aaccgtgttc      60

tttggttttt ttaactctgc gaatatcaac gatataagaa tccagggtaa tgtcgtgcca     120

agtctcggca agttcttcct ggtctatcca ttcattgag                            159

<210> 118
<211> 171
<212> DNA
<213> Artificial Sequence

<220>

<223> orf

<400> 118
ctgaatataa gaaactggcc acaacctttt aattttgttg cagccttaag agccttattg      60

gatagatcgt cttcgccttc agatttgaaa tcaacttgtg tccacaaata ttgttcttgt     120

agcttcgctc tatgtttcaa aatcccaaaa cgtgttattc tatccctgtg t              171


<210> 119
<211> 2376
<212> DNA
<213> Artificial Sequence


<220>
<223> isolate

<400> 119
caacacaggg atagaataac acgttttggt attttgaaac acagatcgaa gcaacaagaa      60

aactttttgt ggactcttgc aaaattcaaa gaaaactacc ctcaagacct gccaagtgaa     120

gaaacaacaa aagctttaaa gtctgctcaa aagttgcaag gctgtggcaa ttttttgctc     180

ttcaaaaatt tttacacaat agatcaaact aagttagcca aatttcatgt ctgtggtcag     240

catttgttat gtcctttttg tgctggtatc cgtgcttcaa agtctattca gaaatattca     300

gaacgtattg atgaagttct caagaaaaac cgtaagctaa aagtcgctct tttgacgctg     360

acggttaaaa acggttctga cctaggggaa cgtgctaatc atctcttaac gtcgtttaga     420

acgcttatca agcgcagaca ggactattta aaaaagggtc gtgggtttaa tgagttctgt     480

aaaattgatg gtgcgatgta ttcctatgaa aatacctaca atgaaaaaac aggtgaatgg     540

catcctcatg ttcacatgct tgtacttctc aatgattgga tagatcagga agccttgggc     600

gaaacttggc atgaaattac tggtgattct tatatcgttg atgttcgaaa agttaaaaaa     660

tctaaagaat tagggtatgc caaagcagca gcagaggttt gtaaatatgc tcttaagttt     720

agtgatctga ctgttgaaaa aacttgggaa gcatttaaaa cccttaaggg taaaaacctt     780

agtggctcat ttggttccct gcgtggtgtc aaaatcccta aaaaccttgc tgacgatatg     840

cctgaagatc aggatttacc ttatctcgaa atgctgtaca agttcgtatt tggcaagcat     900

tcgcactatg acttagttat gactagacac gtagaagcgc aggcgggaga gcgctcggag     960

ttaggaggag cttcgacgac gcccgacgag agatatagcg cgggtgcttc aagtgttaaa    1020

caaggttcgg aatggagcgt ggcgacccct ggtcgaacag cgctctgttc tgtaccgagg    1080

tttcaacgta aaaaatccca ctggagaatc ccacctaaaa cacgtgttag ggttctgcaa    1140

agaattcgaa gatgggacgg cattatgtac aacattgatt tattcccata tgtcgaacat    1200

aggctattag ctttcatcgg ttagtacctg tttcggagaa acgagtaagc gtgcattctg    1260

aaaaactcac cacttttgaa aatagtgtag tgagtacact ttctccagta gccgaacgag    1320

tgtctacgag tgacgactcc gaaacaagaa tttcaaaacc ttcctttccc tgattacaaa 1380

gctcccctta gatttctaag cgggagtttt tagcaatccc gcagggttgc gacacaatga 1440

tattgacaaa aaaatgatcg gtattattat gggtattgcc tagacactaa atacaaattt 1500

taaagggtaa tactatgtgt gtttcattct ctgatatggg tcattctaaa gctgaacgag 1560

aaatcgaaag tcacgaaagt ggcaagactg aaatcgaaac tcgtttaaaa ccgttcacta 1620

tcaaaactca agctcatcaa attgaagcgc tggacattgt ttccgaaatg atggaaatga 1680

gtcgaagtgc tttagttgta aagctcatta atcattatct tggtcaggct gtatatgact 1740

tcctttctag ttacaactct gtcttctcac acgataagcc tgaagaagtt gttgtcttag 1800

atcatatcga aggacttctc gctactaagt atcccgatct ttctccagaa gctaaaagct 1860

tcattatcaa ctctgtggca aaaattgctt ttgaaatgta gggaatcgaa aaatgattaa 1920

gttagaagga acagtattaa acgtcttcac tcaacaaggt ggtaaagaca aaaaaacagg 1980

cgatcaattt gctgatcgtg acaaggtaca aattttaggt gctatggatc tgcctaatgg 2040

tgacgtaaaa aatgaacttt ttacgttatc agtagaaaat tatcgggact ttaaagactt 2100

cttgaatcag aaaatctgta ttgctgttgg tgcaatggca agtggtcgta atgtgatttt 2160

ttacgttgct aaaggcgcaa agcctgtttt agcagaacaa atgtgattta tataaaaaac 2220

agtgggttag cgaagctact cgctgttttt tctttctctt gatactatta attaattcgg 2280

ggacaaaatt tcatgaaaaa tgacaataaa aaacccctgt tatcagaagc tttggcgggc 2340

gtagataaca agggttcgca atctgctaag gcagat 2376

<210> 120
<211> 61
<212> PRT
<213> Artificial Sequence

<220>
<223> orf

<400> 120
Met Leu Lys Lys Leu Gly Lys His Leu Lys Pro Leu Arg Val Lys Thr
1               5                   10                  15
Leu Val Ala His Leu Val Pro Cys Val Val Ser Lys Ser Leu Lys Thr
            20                  25                  30
Leu Leu Thr Ile Cys Leu Lys Ile Arg Ile Tyr Leu Ile Ser Lys Cys
        35                  40                  45
Cys Thr Ser Ser Tyr Leu Ala Ser Ile Arg Thr Met Thr
    50                  55                  60

<210> 121
<211> 74
<212> PRT
<213> Artificial Sequence

<220>
<223> orf

<400> 121
Met Arg Asp Ile Ala Arg Val Leu Gln Val Leu Asn Lys Val Arg Asn
1               5                   10                  15
Gly Ala Trp Arg Pro Leu Val Glu Gln Arg Ser Val Leu Tyr Arg Gly
            20                  25                  30
Phe Asn Val Lys Asn Pro Thr Gly Glu Ser His Leu Lys His Val Leu
        35                  40                  45
Gly Phe Cys Lys Glu Phe Glu Asp Gly Thr Ala Leu Cys Thr Thr Leu
    50                  55                  60
Ile Tyr Ser His Met Ser Asn Ile Gly Tyr
65                  70

<210> 122
<211> 402
<212> PRT
<213> Artificial Sequence

<220>
<223> orf

<400> 122
Met Thr Arg Phe Gly Ile Leu Lys His Arg Ser Lys Gln Gln Glu Asn
1               5                   10                  15
Phe Leu Trp Thr Leu Ala Lys Phe Lys Glu Asn Tyr Pro Gln Asp Leu
            20                  25                  30
Pro Ser Glu Glu Thr Thr Lys Ala Leu Lys Ser Ala Gln Lys Leu Gln
        35                  40                  45
Gly Cys Gly Asn Phe Leu Leu Phe Lys Asn Phe Tyr Thr Ile Asp Gln
    50                  55                  60
Thr Lys Leu Ala Lys Phe His Val Cys Gly Gln His Leu Leu Cys Pro
65                  70                  75                  80
Phe Cys Ala Gly Ile Arg Ala Ser Lys Ser Ile Gln Lys Tyr Ser Glu
                85                  90                  95
Arg Ile Asp Glu Val Leu Lys Lys Asn Arg Lys Leu Lys Val Ala Leu
            100                 105                 110
Leu Thr Leu Thr Val Lys Asn Gly Ser Asp Leu Gly Glu Arg Ala Asn
        115                 120                 125
His Leu Leu Thr Ser Phe Arg Thr Leu Ile Lys Arg Arg Gln Asp Tyr
    130                 135                 140
Leu Lys Lys Gly Arg Gly Phe Asn Glu Phe Cys Lys Ile Asp Gly Ala
145                 150                 155                 160
Met Tyr Ser Tyr Glu Asn Thr Tyr Asn Glu Lys Thr Gly Glu Trp His
                165                 170                 175
Pro His Val His Met Leu Val Leu Leu Asn Asp Trp Ile Asp Gln Glu
            180                 185                 190
Ala Leu Gly Glu Thr Trp His Glu Ile Thr Gly Asp Ser Tyr Ile Val
        195                 200                 205
Asp Val Arg Lys Val Lys Lys Ser Lys Glu Leu Gly Tyr Ala Lys Ala
    210                 215                 220
Ala Ala Glu Val Cys Lys Tyr Ala Leu Lys Phe Ser Asp Leu Thr Val
225                 230                 235                 240
Glu Lys Thr Trp Glu Ala Phe Lys Thr Leu Lys Gly Lys Asn Leu Ser
                245                 250                 255
Gly Ser Phe Gly Ser Leu Arg Gly Val Lys Ile Pro Lys Asn Leu Ala
            260                 265                 270
Asp Asp Met Pro Glu Asp Gln Asp Leu Pro Tyr Leu Glu Met Leu Tyr
        275                 280                 285
Lys Phe Val Phe Gly Lys His Ser His Tyr Asp Leu Val Met Thr Arg
    290                 295                 300

His Val Glu Ala Gln Ala Gly Glu Arg Ser Glu Leu Gly Gly Ala Ser
305                 310                 315                 320
Thr Thr Pro Asp Glu Arg Tyr Ser Ala Gly Ala Ser Ser Val Lys Gln
                325                 330                 335
Gly Ser Glu Trp Ser Val Ala Thr Pro Gly Arg Thr Ala Leu Cys Ser
            340                 345                 350
Val Pro Arg Phe Gln Arg Lys Lys Ser His Trp Arg Ile Pro Pro Lys
        355                 360                 365
Thr Arg Val Arg Val Leu Gln Arg Ile Arg Arg Trp Asp Gly Ile Met
    370                 375                 380
Tyr Asn Ile Asp Leu Phe Pro Tyr Val Glu His Arg Leu Leu Ala Phe
385                 390                 395                 400
Ile Gly

<210> 123
<211> 55
<212> PRT
<213> Artificial Sequence

<220>
<223> orf

<400> 123
Met Ser Phe His Arg Leu Val Pro Val Ser Glu Lys Arg Val Ser Val
1               5                   10                  15
His Ser Glu Lys Leu Thr Thr Phe Glu Asn Ser Val Val Ser Thr Leu
            20                  25                  30
Ser Pro Val Ala Glu Arg Val Ser Thr Ser Asp Asp Ser Glu Thr Arg
        35                  40                  45
Ile Ser Lys Pro Ser Phe Pro
    50                  55

<210> 124
<211> 136
<212> PRT
<213> Artificial Sequence

<220>
<223> orf

<400> 124
Met Gln Ile Leu Lys Gly Asn Thr Met Cys Val Ser Phe Ser Asp Met
1               5                   10                  15
Gly His Ser Lys Ala Glu Arg Glu Ile Glu Ser His Glu Ser Gly Lys
            20                  25                  30
Thr Glu Ile Glu Thr Arg Leu Lys Pro Phe Thr Ile Lys Thr Gln Ala
        35                  40                  45
His Gln Ile Glu Ala Leu Asp Ile Val Ser Glu Met Met Glu Met Ser
    50                  55                  60
Arg Ser Ala Leu Val Val Lys Leu Ile Asn His Tyr Leu Gly Gln Ala
65                  70                  75                  80
Val Tyr Asp Phe Leu Ser Ser Tyr Asn Ser Val Phe Ser His Asp Lys
                85                  90                  95
Pro Glu Glu Val Val Val Leu Asp His Ile Glu Gly Leu Leu Ala Thr
            100                 105                 110
Lys Tyr Pro Asp Leu Ser Pro Glu Ala Lys Ser Phe Ile Ile Asn Ser
        115                 120                 125
Val Ala Lys Ile Ala Phe Glu Met
    130                 135

<210> 125
<211> 97
<212> PRT
<213> Artificial Sequence

<220>
<223> orf

<400> 125
Met Ile Lys Leu Glu Gly Thr Val Leu Asn Val Phe Thr Gln Gln Gly
1               5                   10                  15
Gly Lys Asp Lys Lys Thr Gly Asp Gln Phe Ala Asp Arg Asp Lys Val
            20                  25                  30
Gln Ile Leu Gly Ala Met Asp Leu Pro Asn Gly Asp Val Lys Asn Glu
        35                  40                  45
Leu Phe Thr Leu Ser Val Glu Asn Tyr Arg Asp Phe Lys Asp Phe Leu
    50                  55                  60
Asn Gln Lys Ile Cys Ile Ala Val Gly Ala Met Ala Ser Gly Arg Asn
65                  70                  75                  80
Val Ile Phe Tyr Val Ala Lys Gly Ala Lys Pro Val Leu Ala Glu Gln
                85                  90                  95
Met

<210> 126
<211> 441
<212> PRT
<213> Artificial Sequence

<220>
<223> orf

<400> 126
Met Arg Gly Gln Asn Phe Met Lys Asn Asp Asn Lys Lys Pro Leu Leu
1               5                   10                  15
Ser Glu Ala Leu Ala Gly Val Asp Asn Lys Gly Ser Gln Ser Ala Lys
            20                  25                  30
Ala Asp Gln His Arg Asp Arg Ile Thr Arg Phe Gly Ile Leu Lys His
        35                  40                  45
Arg Ser Lys Gln Gln Glu Asn Phe Leu Trp Thr Leu Ala Lys Phe Lys
    50                  55                  60
Glu Asn Tyr Pro Gln Asp Leu Pro Ser Glu Glu Thr Thr Lys Ala Leu
65                  70                  75                  80
Lys Ser Ala Gln Lys Leu Gln Gly Cys Gly Asn Phe Leu Leu Phe Lys
                85                  90                  95
Asn Phe Tyr Thr Ile Asp Gln Thr Lys Leu Ala Lys Phe His Val Cys
            100                 105                 110
Gly Gln His Leu Leu Cys Pro Phe Cys Ala Gly Ile Arg Ala Ser Lys
        115                 120                 125
Ser Ile Gln Lys Tyr Ser Glu Arg Ile Asp Glu Val Leu Lys Lys Asn
    130                 135                 140
Arg Lys Leu Lys Val Ala Leu Leu Thr Leu Thr Val Lys Asn Gly Ser
145                 150                 155                 160
Asp Leu Gly Glu Arg Ala Asn His Leu Leu Thr Ser Phe Arg Thr Leu
                165                 170                 175
Ile Lys Arg Arg Gln Asp Tyr Leu Lys Lys Gly Arg Gly Phe Asn Glu
            180                 185                 190
Phe Cys Lys Ile Asp Gly Ala Met Tyr Ser Tyr Glu Asn Thr Tyr Asn
        195                 200                 205
Glu Lys Thr Gly Glu Trp His Pro His Val His Met Leu Val Leu Leu
    210                 215                 220

Asn Asp Trp Ile Asp Gln Glu Ala Leu Gly Glu Thr Trp His Glu Ile
225                 230                 235                 240
Thr Gly Asp Ser Tyr Ile Val Asp Val Arg Lys Val Lys Lys Ser Lys
                245                 250                 255
Glu Leu Gly Tyr Ala Lys Ala Ala Ala Glu Val Cys Lys Tyr Ala Leu
            260                 265                 270
Lys Phe Ser Asp Leu Thr Val Glu Lys Thr Trp Glu Ala Phe Lys Thr
        275                 280                 285
Leu Lys Gly Lys Asn Leu Ser Gly Ser Phe Gly Ser Leu Arg Gly Val
    290                 295                 300
Lys Ile Pro Lys Asn Leu Ala Asp Asp Met Pro Glu Asp Gln Asp Leu
305                 310                 315                 320
Pro Tyr Leu Glu Met Leu Tyr Lys Phe Val Phe Gly Lys His Ser His
                325                 330                 335
Tyr Asp Leu Val Met Thr Arg His Val Glu Ala Gln Ala Gly Glu Arg
            340                 345                 350
Ser Glu Leu Gly Gly Ala Ser Thr Thr Pro Asp Glu Arg Tyr Ser Ala
        355                 360                 365
Gly Ala Ser Ser Val Lys Gln Gly Ser Glu Trp Ser Val Ala Thr Pro
    370                 375                 380
Gly Arg Thr Ala Leu Cys Ser Val Pro Arg Phe Gln Arg Lys Lys Ser
385                 390                 395                 400
His Trp Arg Ile Pro Pro Lys Thr Arg Val Arg Val Leu Gln Arg Ile
                405                 410                 415
Arg Arg Trp Asp Gly Ile Met Tyr Asn Ile Asp Leu Phe Pro Tyr Val
            420                 425                 430
Glu His Arg Leu Leu Ala Phe Ile Gly
        435                 440

<210> 127
<211> 56
<212> PRT
<213> Artificial Sequence

<220>
<223> orf

<400> 127
Met Asn Val Val His Asn Ala Val Pro Ser Ser Asn Ser Leu Gln Asn
1               5                   10                  15
Pro Asn Thr Cys Phe Arg Trp Asp Ser Pro Val Gly Phe Phe Thr Leu
            20                  25                  30
Lys Pro Arg Tyr Arg Thr Glu Arg Cys Ser Thr Arg Gly Arg His Ala
        35                  40                  45
Pro Phe Arg Thr Leu Phe Asn Thr
    50                  55

<210> 128
<211> 69
<212> PRT
<213> Artificial Sequence

<220>
<223> orf

<400> 128
Met Ser Ser Ala Arg Phe Leu Gly Ile Leu Thr Pro Arg Arg Glu Pro
1               5                   10                  15
Asn Glu Pro Leu Arg Phe Leu Pro Leu Arg Val Leu Asn Ala Ser Gln
            20                  25                  30
Val Phe Ser Thr Val Arg Ser Leu Asn Leu Arg Ala Tyr Leu Gln Thr

35                  40                  45
Ser Ala Ala Ala Leu Ala Tyr Pro Asn Ser Leu Asp Phe Leu Thr Phe
    50                  55                  60
Arg Thr Ser Thr Ile
65

<210> 129
<211> 64
<212> PRT
<213> Artificial Sequence

<220>
<223> orf

<400> 129
Met Asn Asp Ile Arg Ile Thr Ser Asn Phe Met Pro Ser Phe Ala Gln
1               5                   10                  15
Gly Phe Leu Ile Tyr Pro Ile Ile Glu Lys Tyr Lys His Val Asn Met
            20                  25                  30
Arg Met Pro Phe Thr Cys Phe Phe Ile Val Gly Ile Phe Ile Gly Ile
        35                  40                  45
His Arg Thr Ile Asn Phe Thr Glu Leu Ile Lys Pro Thr Thr Leu Phe
    50                  55                  60

<210> 130
<211> 52
<212> PRT
<213> Artificial Sequence

<220>
<223> orf

<400> 130
Met Arg Val Val Phe Phe Glu Phe Cys Lys Ser Pro Gln Lys Val Phe
1               5                   10                  15
Leu Leu Leu Arg Ser Val Phe Gln Asn Thr Lys Thr Cys Tyr Ser Ile
            20                  25                  30
Pro Val Leu Ile Cys Leu Ser Arg Leu Arg Thr Leu Val Ile Tyr Ala
        35                  40                  45
Arg Gln Ser Phe
    50

<210> 131
<211> 183
<212> DNA
<213> Artificial Sequence

<220>
<223> orf

<400> 131
ctgttgaaaa aacttgggaa gcatttaaaa cccttaaggg taaaaacctt agtggctcat      60

ttggttccct gcgtggtgtc aaaatcccta aaaaccttgc tgacgatatg cctgaagatc     120

aggatttacc ttatctcgaa atgctgtaca agttcgtatt tggcaagcat tcgcactatg     180

act                                                                   183

<210> 132
<211> 222
<212> DNA
<213> Artificial Sequence

<220>
<223> orf

<400> 132
acgagagata tagcgcgggt gcttcaagtg ttaaacaagg ttcggaatgg agcgtggcga 60

cccctggtcg aacagcgctc tgttctgtac cgaggtttca acgtaaaaaa tcccactgga 120

gaatcccacc taaaacacgt gttagggttc tgcaaagaat tcgaagatgg gacggcatta 180

tgtacaacat tgatttattc ccatatgtcg aacataggct at 222

<210> 133
<211> 1206
<212> DNA
<213> Artificial Sequence

<220>
<223> orf

<400> 133
ataacacgtt ttggtatttt gaaacacaga tcgaagcaac aagaaaactt tttgtggact 60

cttgcaaaat tcaaagaaaa ctaccctcaa gacctgccaa gtgaagaaac aacaaaagct 120

ttaaagtctg ctcaaaagtt gcaaggctgt ggcaattttt tgctcttcaa aaatttttac 180

acaatagatc aaactaagtt agccaaattt catgtctgtg gtcagcattt gttatgtcct 240

ttttgtgctg gtatccgtgc ttcaaagtct attcagaaat attcagaacg tattgatgaa 300

gttctcaaga aaaaccgtaa gctaaaagtc gctcttttga cgctgacggt taaaaacggt 360

tctgacctag gggaacgtgc taatcatctc ttaacgtcgt ttagaacgct tatcaagcgc 420

agacaggact atttaaaaaa gggtcgtggg tttaatgagt tctgtaaaat tgatggtgcg 480

atgtattcct atgaaaatac ctacaatgaa aaaacaggtg aatggcatcc tcatgttcac 540

atgcttgtac ttctcaatga ttggatagat caggaagcct tgggcgaaac ttggcatgaa 600

attactggtg attcttatat cgttgatgtt cgaaaagtta aaaaatctaa agaattaggg 660

tatgccaaag cagcagcaga ggtttgtaaa tatgctctta agtttagtga tctgactgtt 720

gaaaaaactt gggaagcatt taaaaccctt aagggtaaaa accttagtgg ctcatttggt 780

tccctgcgtg gtgtcaaaat ccctaaaaac cttgctgacg atatgcctga agatcaggat 840

ttaccttatc tcgaaatgct gtacaagttc gtatttggca agcattcgca ctatgactta 900

gttatgacta gacacgtaga agcgcaggcg ggagagcgct cggagttagg aggagcttcg 960

acgacgcccg acgagagata tagcgcgggt gcttcaagtg ttaaacaagg ttcggaatgg 1020

agcgtggcga cccctggtcg aacagcgctc tgttctgtac cgaggtttca acgtaaaaaa 1080

tcccactgga gaatcccacc taaaacacgt gttagggttc tgcaaagaat tcgaagatgg 1140

gacggcatta tgtacaacat tgatttattc ccatatgtcg aacataggct attagctttc 1200

atcggt 1206

<210> 134
<211> 165
<212> DNA
<213> Artificial Sequence

<220>
<223> orf

<400> 134
attagctttc atcggttagt acctgtttcg gagaaacgag taagcgtgca ttctgaaaaa 60

ctcaccactt ttgaaaatag tgtagtgagt acactttctc cagtagccga acgagtgtct 120

acgagtgacg actccgaaac aagaatttca aaaccttcct ttccc 165

<210> 135
<211> 408
<212> DNA
<213> Artificial Sequence

<220>
<223> orf

<400> 135
atacaaattt taaagggtaa tactatgtgt gtttcattct ctgatatggg tcattctaaa 60

gctgaacgag aaatcgaaag tcacgaaagt ggcaagactg aaatcgaaac tcgtttaaaa 120

ccgttcacta tcaaaactca agctcatcaa attgaagcgc tggacattgt ttccgaaatg 180

atggaaatga gtcgaagtgc tttagttgta aagctcatta atcattatct tggtcaggct 240

gtatatgact tcctttctag ttacaactct gtcttctcac acgataagcc tgaagaagtt 300

gttgtcttag atcatatcga aggacttctc gctactaagt atcccgatct ttctccagaa 360

gctaaaagct tcattatcaa ctctgtggca aaaattgctt ttgaaatg 408

<210> 136
<211> 291
<212> DNA
<213> Artificial Sequence

<220>
<223> orf

<400> 136

atgattaagt tagaaggaac agtattaaac gtcttcactc aacaaggtgg taaagacaaa 60

aaaacaggcg atcaatttgc tgatcgtgac aaggtacaaa ttttaggtgc tatggatctg 120

cctaatggtg acgtaaaaaa tgaacttttt acgttatcag tagaaaatta tcgggacttt 180

aaagacttct tgaatcagaa aatctgtatt gctgttggtg caatggcaag tggtcgtaat 240

gtgatttttt acgttgctaa aggcgcaaag cctgttttag cagaacaaat g 291

<210> 137
<211> 1323
<212> DNA
<213> Artificial Sequence

<220>
<223> orf

<400> 137
attcggggac aaaatttcat gaaaaatgac aataaaaaac ccctgttatc agaagctttg 60

gcgggcgtag ataacaaggg ttcgcaatct gctaaggcag atcaacacag ggatagaata 120

acacgttttg gtattttgaa acacagatcg aagcaacaag aaaacttttt gtggactctt 180

gcaaaattca aagaaaacta ccctcaagac ctgccaagtg aagaaacaac aaaagcttta 240

aagtctgctc aaaagttgca aggctgtggc aattttttgc tcttcaaaaa tttttacaca 300

atagatcaaa ctaagttagc caaatttcat gtctgtggtc agcatttgtt atgtcctttt 360

tgtgctggta tccgtgcttc aaagtctatt cagaaatatt cagaacgtat tgatgaagtt 420

ctcaagaaaa accgtaagct aaaagtcgct cttttgacgc tgacggttaa aaacggttct 480

gacctagggg aacgtgctaa tcatctctta acgtcgttta gaacgcttat caagcgcaga 540

caggactatt taaaaaaggg tcgtgggttt aatgagttct gtaaaattga tggtgcgatg 600

tattcctatg aaaataccta caatgaaaaa acaggtgaat ggcatcctca tgttcacatg 660

cttgtacttc tcaatgattg gatagatcag gaagccttgg gcgaaacttg gcatgaaatt 720

actggtgatt cttatatcgt tgatgttcga aaagttaaaa aatctaaaga attagggtat 780

gccaaagcag cagcagaggt ttgtaaatat gctcttaagt ttagtgatct gactgttgaa 840

aaaacttggg aagcatttaa aacccttaag ggtaaaaacc ttagtggctc atttggttcc 900

ctgcgtggtg tcaaaatccc taaaaacctt gctgacgata tgcctgaaga tcaggattta 960

ccttatctcg aaatgctgta caagttcgta tttggcaagc attcgcacta tgacttagtt 1020

atgactagac acgtagaagc gcaggcggga gagcgctcgg agttaggagg agcttcgacg 1080

acgcccgacg agagatatag cgcgggtgct tcaagtgtta aacaaggttc ggaatggagc 1140

gtggcgaccc ctggtcgaac agcgctctgt tctgtaccga ggtttcaacg taaaaaatcc 1200

cactggagaa tcccacctaa aacacgtgtt agggttctgc aaagaattcg aagatgggac 1260

ggcattatgt acaacattga tttattccca tatgtcgaac ataggctatt agctttcatc 1320

ggt 1323

<210> 138
<211> 168
<212> DNA
<213> Artificial Sequence

<220>
<223> orf

<400> 138
atcaatgttg tacataatgc cgtcccatct tcgaattctt tgcagaaccc taacacgtgt 60

tttaggtggg attctccagt gggatttttt acgttgaaac ctcggtacag aacagagcgc 120

tgttcgacca ggggtcgcca cgctccattc cgaaccttgt ttaacact 168

<210> 139
<211> 207
<212> DNA
<213> Artificial Sequence

<220>
<223> orf

<400> 139
atatcgtcag caaggttttt agggattttg acaccacgca gggaaccaaa tgagccacta 60

aggtttttac ccttaagggt tttaaatgct tcccaagttt tttcaacagt cagatcacta 120

aacttaagag catatttaca aacctctgct gctgctttgg cataccctaa ttctttagat 180

tttttaactt ttcgaacatc aacgata 207

<210> 140
<211> 192
<212> DNA
<213> Artificial Sequence

<220>
<223> orf

<400> 140
atcaacgata taagaatcac cagtaatttc atgccaagtt tcgcccaagg cttcctgatc 60

tatccaatca ttgagaagta caagcatgtg aacatgagga tgccattcac ctgttttttc 120

attgtaggta ttttcatagg aatacatcgc accatcaatt ttacagaact cattaaaccc 180

acgacccttt tt 192

<210> 141
<211> 156
<212> DNA

<213> Artificial Sequence

<220>
<223> orf

<400> 141
ttgagggtag ttttctttga attttgcaag agtccacaaa aagttttctt gttgcttcga      60

tctgtgtttc aaaataccaa aacgtgttat tctatccctg tgttgatctg ccttagcaga     120

ttgcgaaccc ttgttatcta cgcccgccaa agcttc                               156

<210> 142
<211> 2392
<212> DNA
<213> Artificial Sequence

<220>
<223> isolate

<400> 142
caacacaggg atagaataac acgttttgga cttttgaaac atagagcgaa gctacaagaa      60

caatatttgt ggacgcaggt tgatttcaaa tccgaaggtg aaaatgagac atccaataag     120

gctcttaagg ctgcaaccaa attaaaggga tgcggtcaat ttctgctatt ccataactac     180

tacacaattg accaagttaa acttgctaag gcccattatt gttctcagca tttgctttgc     240

cctatgtgtg ctggtgtaag ggctgctaag tcaatgagta gctatgttca acgtattgaa     300

gaattgatgc gtcagaatcg caaattaaag cccgtattga tcactttaac ggttaagaat     360

ggggaagacc tagaagaacg ctttaaacac cttagacgct catttaggac gcttttagat     420

cgttataacg attacaaaaa gaaaggtcgt ggttttaatc aattctgcaa gattgatggt     480

gctttttatt ccactgaata tacctacaat tcaaaaacaa aagagtggca tccccatatc     540

catattttcg ctttactcaa tgaatggata gaccaggaag aattggccga gacctggcat     600

gacattaccc tggattctta tattgttgat attcgcagag ttaaaaggac caaagaacac     660

ggctatagca aagctgttgc agaggtttgt aaatatgctc ttaagtttag tgatttgtca     720

cttgagaata cgtgggaagc ttatctttct ttaaaaggta ataggcttac tggctgtttt     780

ggttctatgt atggtgtcaa gttgcctgaa aaactcacag atgatttacc ccttgatgat     840

cttccatata tggagctgct ataccgtttt gtctttggta aaaaatcata ttacgactta     900

gaaataacaa aagacgttaa gccaaacaaa aggaatgagg aaggatgaac gaggagcttc     960

gacgaggccc gacgcccgaa tgacttgtga aggcgatacg tcaatcggtt gtctgtgatg    1020

tgacgttccg tgaacggcca tcatcatcag acaatcgaat aagcaagcct accctgtagc    1080

cacaggactt gtaaggggtt cgacccctta gaccccaaag ggcgcacttt accattgagc    1140

aaagctcaac ggaaattgct ccctaaaaat aacggagaat aaaatgcttt ttgaaggaaa    1200

aattaaagac ttagcagatg ataatcatct ttcttggatt gctgatactt accacaatat 1260

gatttttgaa atcttattca gtgaatacca agatggtttt aattctttgt ctgaattact 1320

tagatcaaac gatattaaac caatggattt tgatttaaac gttgctcgtt taaatcgctc 1380

ttatgtttac gccttgcagt atttcggaat ttaacagagg gggtgtctac ccccgaactg 1440

acatcatcta aatttcaaaa cctttcttcc ccgattacaa gctccccttg gacttccaag 1500

cgggagtttt tagccatccc gaatgggtgg cgacacaata ctatttgatt ttttcatgat 1560

tcaaggtatg attcattgtg aatcacattc ctatatatga aaaaatcagg tgagaatatg 1620

caaattgatg aaccaacaaa cttccaagta ttcatggcag aagtaaacaa gacagcaaag 1680

acagaaagta ttggtgttta ccatcaagta cctttccgta tggctcgatg gaactttgta 1740

cgactggaag gcctccgtaa tcacatggga gaacctcgaa acaaagttct taactcacta 1800

atcgaaattg ctttagatca ggtatttgaa caacttgagc atggcagtaa agagatccgt 1860

cgttctgttc ttgaggaagt ttccaaagtt ctcgaatcaa ttgaacatga tggttctggg 1920

agcttagaca atgattaatc tacaaggaac tttgataaat gctttccgtg tggatggtgg 1980

taaaggtaaa gacggcaaag aatatgaagc gcgtgacaag gtgcaaattc ttggttcgtt 2040

ggaactccca aacggggaaa ttaaacacga acttgttgac ctaacagttg aggattctcg 2100

actgtttgaa ccattcaaaa atcaggttat tagcatctct tgcggtgcta tggctatcgg 2160

tcgtaatgtt gttttttatg ttcgtaaagg cgcaaagcct gttttagcag aaataatgtg 2220

aaacacgaaa aaaaacagtg agttagcctt ggcttctcgc tgttttttct ttctcttgat 2280

actactgatt aactagggga caaaattgac taaaaaagac aataaaaaag cccataattc 2340

ggacactttg gcgggtgatg aattacaggc tttgcaatct gctaaggcag at 2392

<210> 143
<211> 50
<212> PRT
<213> Artificial Sequence

<220>
<223> orf

<400> 143
Met Val Ile Thr Ile Thr Lys Arg Lys Val Val Val Leu Ile Asn Ser
1               5                   10                  15
Ala Arg Leu Met Val Leu Phe Ile Pro Leu Asn Ile Pro Thr Ile Gln
            20                  25                  30
Lys Gln Lys Ser Gly Ile Pro Ile Ser Ile Phe Ser Leu Tyr Ser Met
        35                  40                  45
Asn Gly
    50

<210> 144
<211> 51

<212> PRT
<213> Artificial Sequence

<220>
<223> orf

<400> 144
Met Thr Leu Pro Trp Ile Leu Ile Leu Leu Ile Phe Ala Glu Leu Lys
1               5                   10                  15
Gly Pro Lys Asn Thr Ala Ile Ala Lys Leu Leu Gln Arg Phe Val Asn
            20                  25                  30
Met Leu Leu Ser Leu Val Ile Cys His Leu Arg Ile Arg Gly Lys Leu
        35                  40                  45
Ile Phe Leu
    50

<210> 145
<211> 310
<212> PRT
<213> Artificial Sequence

<220>
<223> orf

<400> 145
Met Thr Arg Phe Gly Leu Leu Lys His Arg Ala Lys Leu Gln Glu Gln
1               5                   10                  15
Tyr Leu Trp Thr Gln Val Asp Phe Lys Ser Glu Gly Glu Asn Glu Thr
            20                  25                  30
Ser Asn Lys Ala Leu Lys Ala Ala Thr Lys Leu Lys Gly Cys Gly Gln
        35                  40                  45
Phe Leu Leu Phe His Asn Tyr Tyr Thr Ile Asp Gln Val Lys Leu Ala
    50                  55                  60
Lys Ala His Tyr Cys Ser Gln His Leu Leu Cys Pro Met Cys Ala Gly
65                  70                  75                  80
Val Arg Ala Ala Lys Ser Met Ser Ser Tyr Val Gln Arg Ile Glu Glu
                85                  90                  95
Leu Met Arg Gln Asn Arg Lys Leu Lys Pro Val Leu Ile Thr Leu Thr
            100                 105                 110
Val Lys Asn Gly Glu Asp Leu Glu Glu Arg Phe Lys His Leu Arg Arg
        115                 120                 125
Ser Phe Arg Thr Leu Leu Asp Arg Tyr Asn Asp Tyr Lys Lys Lys Gly
    130                 135                 140
Arg Gly Phe Asn Gln Phe Cys Lys Ile Asp Gly Ala Phe Tyr Ser Thr
145                 150                 155                 160
Glu Tyr Thr Tyr Asn Ser Lys Thr Lys Glu Trp His Pro His Ile His
                165                 170                 175
Ile Phe Ala Leu Leu Asn Glu Trp Ile Asp Gln Glu Glu Leu Ala Glu
            180                 185                 190
Thr Trp His Asp Ile Thr Leu Asp Ser Tyr Ile Val Asp Ile Arg Arg
        195                 200                 205
Val Lys Arg Thr Lys Glu His Gly Tyr Ser Lys Ala Val Ala Glu Val
    210                 215                 220
Cys Lys Tyr Ala Leu Lys Phe Ser Asp Leu Ser Leu Glu Asn Thr Trp
225                 230                 235                 240
Glu Ala Tyr Leu Ser Leu Lys Gly Asn Arg Leu Thr Gly Cys Phe Gly
                245                 250                 255
Ser Met Tyr Gly Val Lys Leu Pro Glu Lys Leu Thr Asp Asp Leu Pro
            260                 265                 270
Leu Asp Asp Leu Pro Tyr Met Glu Leu Leu Tyr Arg Phe Val Phe Gly
        275                 280                 285

Lys Lys Ser Tyr Tyr Asp Leu Glu Ile Thr Lys Asp Val Lys Pro Asn
    290                 295                 300
Lys Arg Asn Glu Glu Gly
305                 310

<210> 146
<211> 81
<212> PRT
<213> Artificial Sequence

<220>
<223> orf

<400> 146
Met Thr Glu Asn Lys Met Leu Phe Glu Gly Lys Ile Lys Asp Leu Ala
1               5                   10                  15
Asp Asp Asn His Leu Ser Trp Ile Ala Asp Thr Tyr His Asn Met Ile
            20                  25                  30
Phe Glu Ile Leu Phe Ser Glu Tyr Gln Asp Gly Phe Asn Ser Leu Ser
        35                  40                  45
Glu Leu Leu Arg Ser Asn Asp Ile Lys Pro Met Asp Phe Asp Leu Asn
    50                  55                  60
Val Ala Arg Leu Asn Arg Ser Tyr Val Tyr Ala Leu Gln Tyr Phe Gly
65                  70                  75                  80
Ile

<210> 147
<211> 118
<212> PRT
<213> Artificial Sequence

<220>
<223> orf

<400> 147
Met Thr Phe Leu Tyr Met Lys Lys Ser Gly Glu Asn Met Gln Ile Asp
1               5                   10                  15
Glu Pro Thr Asn Phe Gln Val Phe Met Ala Glu Val Asn Lys Thr Ala
            20                  25                  30
Lys Thr Glu Ser Ile Gly Val Tyr His Gln Val Pro Phe Arg Met Ala
        35                  40                  45
Arg Trp Asn Phe Val Arg Leu Glu Gly Leu Arg Asn His Met Gly Glu
    50                  55                  60
Pro Arg Asn Lys Val Leu Asn Ser Leu Ile Glu Ile Ala Leu Asp Gln
65                  70                  75                  80
Val Phe Glu Gln Leu Glu His Gly Ser Lys Glu Ile Arg Arg Ser Val
                85                  90                  95
Leu Glu Glu Val Ser Lys Val Leu Glu Ser Ile Glu His Asp Gly Ser
            100                 105                 110
Gly Ser Leu Asp Asn Asp
        115

<210> 148
<211> 96
<212> PRT
<213> Artificial Sequence

<220>
<223> orf

<400> 148
Met Ile Asn Leu Gln Gly Thr Leu Ile Asn Ala Phe Arg Val Asp Gly
1               5                   10                  15
Gly Lys Gly Lys Asp Gly Lys Glu Tyr Glu Ala Arg Asp Lys Val Gln
            20                  25                  30
Ile Leu Gly Ser Leu Glu Leu Pro Asn Gly Glu Ile Lys His Glu Leu
        35                  40                  45
Val Asp Leu Thr Val Glu Asp Ser Arg Leu Phe Glu Pro Phe Lys Asn
    50                  55                  60
Gln Val Ile Ser Ile Ser Cys Gly Ala Met Ala Ile Gly Arg Asn Val
65                  70                  75                  80
Val Phe Tyr Val Arg Lys Gly Ala Lys Pro Val Leu Ala Glu Ile Met
                85                  90                  95

<210> 149
<211> 344
<212> PRT
<213> Artificial Sequence

<220>
<223> orf

<400> 149
Met Thr Lys Lys Asp Asn Lys Lys Ala His Asn Ser Asp Thr Leu Ala
1               5                   10                  15
Gly Asp Glu Leu Gln Ala Leu Gln Ser Ala Lys Ala Asp Gln His Arg
            20                  25                  30
Asp Arg Ile Thr Arg Phe Gly Leu Leu Lys His Arg Ala Lys Leu Gln
        35                  40                  45
Glu Gln Tyr Leu Trp Thr Gln Val Asp Phe Lys Ser Glu Gly Glu Asn
    50                  55                  60
Glu Thr Ser Asn Lys Ala Leu Lys Ala Ala Thr Lys Leu Lys Gly Cys
65                  70                  75                  80
Gly Gln Phe Leu Leu Phe His Asn Tyr Tyr Thr Ile Asp Gln Val Lys
                85                  90                  95
Leu Ala Lys Ala His Tyr Cys Ser Gln His Leu Leu Cys Pro Met Cys
            100                 105                 110
Ala Gly Val Arg Ala Ala Lys Ser Met Ser Ser Tyr Val Gln Arg Ile
        115                 120                 125
Glu Glu Leu Met Arg Gln Asn Arg Lys Leu Lys Pro Val Leu Ile Thr
    130                 135                 140
Leu Thr Val Lys Asn Gly Glu Asp Leu Glu Glu Arg Phe Lys His Leu
145                 150                 155                 160
Arg Arg Ser Phe Arg Thr Leu Leu Asp Arg Tyr Asn Asp Tyr Lys Lys
                165                 170                 175
Lys Gly Arg Gly Phe Asn Gln Phe Cys Lys Ile Asp Gly Ala Phe Tyr
            180                 185                 190
Ser Thr Glu Tyr Thr Tyr Asn Ser Lys Thr Lys Glu Trp His Pro His
        195                 200                 205
Ile His Ile Phe Ala Leu Leu Asn Glu Trp Ile Asp Gln Glu Glu Leu
    210                 215                 220
Ala Glu Thr Trp His Asp Ile Thr Leu Asp Ser Tyr Ile Val Asp Ile
225                 230                 235                 240
Arg Arg Val Lys Arg Thr Lys Glu His Gly Tyr Ser Lys Ala Val Ala
                245                 250                 255
Glu Val Cys Lys Tyr Ala Leu Lys Phe Ser Asp Leu Ser Leu Glu Asn
            260                 265                 270
Thr Trp Glu Ala Tyr Leu Ser Leu Lys Gly Asn Arg Leu Thr Gly Cys
        275                 280                 285

Phe Gly Ser Met Tyr Gly Val Lys Leu Pro Glu Lys Leu Thr Asp Asp
    290                 295                 300
Leu Pro Leu Asp Asp Leu Pro Tyr Met Glu Leu Leu Tyr Arg Phe Val
305                 310                 315                 320
Phe Gly Lys Lys Ser Tyr Tyr Asp Leu Glu Ile Thr Lys Asp Val Lys
                325                 330                 335
Pro Asn Lys Arg Asn Glu Glu Gly
            340

<210> 150
<211> 64
<212> PRT
<213> Artificial Sequence

<220>
<223> orf

<400> 150
Met Asn Gly Ser Asn Ser Arg Glu Ser Ser Thr Val Arg Ser Thr Ser
1               5                   10                  15
Ser Cys Leu Ile Ser Pro Phe Gly Ser Ser Asn Glu Pro Arg Ile Cys
            20                  25                  30
Thr Leu Ser Arg Ala Ser Tyr Ser Leu Pro Ser Leu Pro Leu Pro Pro
        35                  40                  45
Ser Thr Arg Lys Ala Phe Ile Lys Val Pro Cys Arg Leu Ile Ile Val
    50                  55                  60

<210> 151
<211> 52
<212> PRT
<213> Artificial Sequence

<220>
<223> orf

<400> 151
Met Ile Thr Glu Ala Phe Gln Ser Tyr Lys Val Pro Ser Ser His Thr
1               5                   10                  15
Glu Arg Tyr Leu Met Val Asn Thr Asn Thr Phe Cys Leu Cys Cys Leu
            20                  25                  30
Val Tyr Phe Cys His Glu Tyr Leu Glu Val Cys Trp Phe Ile Asn Leu
        35                  40                  45
His Ile Leu Thr
    50

<210> 152
<211> 51
<212> PRT
<213> Artificial Sequence

<220>
<223> orf

<400> 152
Met Ser Val Glu Leu Cys Ser Met Val Lys Cys Ala Leu Trp Gly Leu
1               5                   10                  15
Arg Gly Arg Thr Pro Tyr Lys Ser Cys Gly Tyr Arg Val Gly Leu Leu
            20                  25                  30

Ile Arg Leu Ser Asp Asp Asp Ala Arg Ser Arg Asn Val Thr Ser Gln
        35                  40                  45
Thr Thr Asp
    50

<210> 153
<211> 64
<212> PRT
<213> Artificial Sequence

<220>
<223> orf

<400> 153
Met Asn Gly Ser Asn Ser Arg Glu Ser Ser Thr Val Arg Ser Thr Ser
1               5                   10                  15
Ser Cys Leu Ile Ser Pro Phe Gly Ser Ser Asn Glu Pro Arg Ile Cys
            20                  25                  30
Thr Leu Ser Arg Ala Ser Tyr Ser Leu Pro Ser Leu Pro Leu Pro Pro
        35                  40                  45
Ser Thr Arg Lys Ala Phe Ile Lys Val Pro Cys Arg Leu Ile Ile Val
    50                  55                  60

<210> 154
<211> 50
<212> PRT
<213> Artificial Sequence

<220>
<223> orf

<400> 154
Met Met Met Met Pro Val His Gly Thr Ser His His Arg Gln Pro Ile
1               5                   10                  15
Asp Val Ser Pro Ser Gln Val Ile Arg Ala Ser Gly Leu Val Glu Ala
            20                  25                  30
Pro Arg Ser Ser Phe Leu Ile Pro Phe Val Trp Leu Asn Val Phe Cys
        35                  40                  45
Tyr Phe
    50

<210> 155
<211> 53
<212> PRT
<213> Artificial Sequence

<220>
<223> orf

<400> 155
Met Thr Lys Leu Lys Ser Ile Phe Thr Asn Leu Cys Asn Ser Phe Ala
1               5                   10                  15
Ile Ala Val Phe Phe Gly Pro Phe Asn Ser Ala Asn Ile Asn Asn Ile
            20                  25                  30
Arg Ile Gln Gly Asn Val Met Pro Gly Leu Gly Gln Phe Phe Leu Val
        35                  40                  45
Tyr Pro Phe Ile Glu
    50

<210> 156
<211> 150
<212> DNA
<213> Artificial Sequence

<220>
<223> orf

<400> 156
atcgttataa cgattacaaa aagaaaggtc gtggttttaa tcaattctgc aagattgatg 60

gtgcttttta ttccactgaa tatacctaca attcaaaaac aaaagagtgg catccccata 120

tccatatttt cgctttactc aatgaatgga 150

<210> 157
<211> 153
<212> DNA
<213> Artificial Sequence

<220>
<223> orf

<400> 157
atgacattac cctggattct tatattgttg atattcgcag agttaaaagg accaaagaac 60

acggctatag caaagctgtt gcagaggttt gtaaatatgc tcttaagttt agtgatttgt 120

cacttgagaa tacgtgggaa gcttatcttt ctt 153

<210> 158
<211> 930
<212> DNA
<213> Artificial Sequence

<220>
<223> orf

<400> 158
ataacacgtt ttggactttt gaaacataga gcgaagctac aagaacaata tttgtggacg 60

caggttgatt tcaaatccga aggtgaaaat gagacatcca ataaggctct taaggctgca 120

accaaattaa agggatgcgg tcaatttctg ctattccata actactacac aattgaccaa 180

gttaaacttg ctaaggccca ttattgttct cagcatttgc tttgccctat gtgtgctggt 240

gtaagggctg ctaagtcaat gagtagctat gttcaacgta ttgaagaatt gatgcgtcag 300

aatcgcaaat taaagcccgt attgatcact ttaacggtta agaatgggga agacctagaa 360

gaacgcttta aacaccttag acgctcattt aggacgcttt tagatcgtta taacgattac 420

aaaaagaaag gtcgtggttt taatcaattc tgcaagattg atggtgcttt ttattccact 480

gaatatacct acaattcaaa aacaaaagag tggcatcccc atatccatat tttcgcttta 540

ctcaatgaat ggatagacca ggaagaattg gccgagacct ggcatgacat taccctggat 600

tcttatattg ttgatattcg cagagttaaa aggaccaaag aacacggcta tagcaaagct 660

gttgcagagg tttgtaaata tgctcttaag tttagtgatt tgtcacttga gaatacgtgg 720

gaagcttatc tttctttaaa aggtaatagg cttactggct gttttggttc tatgtatggt 780

gtcaagttgc ctgaaaaact cacagatgat ttaccccttg atgatcttcc atatatggag 840

ctgctatacc gttttgtctt tggtaaaaaa tcatattacg acttagaaat aacaaaagac 900

gttaagccaa acaaaaggaa tgaggaagga 930

<210> 159
<211> 243
<212> DNA
<213> Artificial Sequence

<220>
<223> orf

<400> 159
ataacggaga ataaaatgct ttttgaagga aaaattaaag acttagcaga tgataatcat 60

ctttcttgga ttgctgatac ttaccacaat atgatttttg aaatcttatt cagtgaatac 120

caagatggtt ttaattcttt gtctgaatta cttagatcaa acgatattaa accaatggat 180

tttgatttaa acgttgctcg tttaaatcgc tcttatgttt acgccttgca gtatttcgga 240

att 243

<210> 160
<211> 354
<212> DNA
<213> Artificial Sequence

<220>
<223> orf

<400> 160
atcacattcc tatatatgaa aaaatcaggt gagaatatgc aaattgatga accaacaaac 60

ttccaagtat tcatggcaga agtaaacaag acagcaaaga cagaaagtat tggtgtttac 120

catcaagtac ctttccgtat ggctcgatgg aactttgtac gactggaagg cctccgtaat 180

cacatgggag aacctcgaaa caaagttctt aactcactaa tcgaaattgc tttagatcag 240

gtatttgaac aacttgagca tggcagtaaa gagatccgtc gttctgttct tgaggaagtt 300

tccaaagttc tcgaatcaat tgaacatgat ggttctggga gcttagacaa tgat 354

<210> 161
<211> 288
<212> DNA
<213> Artificial Sequence

<220>
<223> orf

<400> 161
atgattaatc tacaaggaac tttgataaat gctttccgtg tggatggtgg taaaggtaaa      60

gacggcaaag aatatgaagc gcgtgacaag gtgcaaattc ttggttcgtt ggaactccca     120

aacggggaaa ttaaacacga acttgttgac ctaacagttg aggattctcg actgtttgaa     180

ccattcaaaa atcaggttat tagcatctct tgcggtgcta tggctatcgg tcgtaatgtt     240

gttttttatg ttcgtaaagg cgcaaagcct gttttagcag aaataatg                   288

<210> 162
<211> 1032
<212> DNA
<213> Artificial Sequence

<220>
<223> orf

<400> 162
ttgactaaaa aagacaataa aaaagcccat aattcggaca ctttggcggg tgatgaatta      60

caggctttgc aatctgctaa ggcagatcaa cacagggata gaataacacg ttttggactt     120

ttgaaacata gagcgaagct acaagaacaa tatttgtgga cgcaggttga tttcaaatcc     180

gaaggtgaaa atgagacatc caataaggct cttaaggctg caaccaaatt aaagggatgc      240

ggtcaatttc tgctattcca taactactac acaattgacc aagttaaact tgctaaggcc     300

cattattgtt ctcagcattt gctttgccct atgtgtgctg gtgtaagggc tgctaagtca     360

atgagtagct atgttcaacg tattgaagaa ttgatgcgtc agaatcgcaa attaaagccc     420

gtattgatca ctttaacggt taagaatggg gaagacctag aagaacgctt taaacacctt     480

agacgctcat ttaggacgct tttagatcgt tataacgatt acaaaaagaa aggtcgtggt     540

tttaatcaat tctgcaagat tgatggtgct ttttattcca ctgaatatac ctacaattca      600

aaaacaaaag agtggcatcc ccatatccat attttcgctt tactcaatga atggatagac     660

caggaagaat tggccgagac ctggcatgac attaccctgg attcttatat tgttgatatt     720

cgcagagtta aaaggaccaa agaacacggc tatagcaaag ctgttgcaga ggtttgtaaa     780

tatgctctta agtttagtga tttgtcactt gagaatacgt gggaagctta tctttcttta     840

aaaggtaata ggcttactgg ctgttttggt tctatgtatg gtgtcaagtt gcctgaaaaa     900

ctcacagatg atttacccct tgatgatctt ccatatatgg agctgctata ccgttttgtc     960

tttggtaaaa aatcatatta cgacttagaa ataacaaaag acgttaagcc aaacaaaagg    1020

aatgaggaag ga                                                        1032

<210> 163
<211> 192
<212> DNA
<213> Artificial Sequence

<220>
<223> orf

<400> 163
ttgaatggtt caaacagtcg agaatcctca actgttaggt caacaagttc gtgtttaatt 60

tccccgtttg ggagttccaa cgaaccaaga atttgcacct tgtcacgcgc ttcatattct 120

ttgccgtctt tacctttacc accatccaca cggaaagcat ttatcaaagt tccttgtaga 180

ttaatcattg tc 192

<210> 164
<211> 156
<212> DNA
<213> Artificial Sequence

<220>
<223> orf

<400> 164
gtgattacgg aggccttcca gtcgtacaaa gttccatcga gccatacgga aaggtacttg 60

atggtaaaca ccaatacttt ctgtctttgc tgtcttgttt acttctgcca tgaatacttg 120

gaagtttgtt ggttcatcaa tttgcatatt ctcacc 156

<210> 165
<211> 153
<212> DNA
<213> Artificial Sequence

<220>
<223> orf

<400> 165
atttccgttg agctttgctc aatggtaaag tgcgcccttt ggggtctaag gggtcgaacc 60

ccttacaagt cctgtggcta cagggtaggc ttgcttattc gattgtctga tgatgatgcc 120

cgttcacgga acgtcacatc acagacaacc gat 153

<210> 166
<211> 150
<212> DNA
<213> Artificial Sequence

<220>
<223> orf

<400> 166
ctgatgatga tgcccgttca cggaacgtca catcacagac aaccgattga cgtatcgcct      60

tcacaagtca ttcgggcgtc gggcctcgtc gaagctcctc gttcatcctt cctcattcct     120

tttgtttggc ttaacgtctt ttgttatttc                                      150


<210> 167
<211> 159
<212> DNA
<213> Artificial Sequence


<220>
<223> orf

<400> 167
atcactaaac ttaagagcat atttacaaac ctctgcaaca gctttgctat agccgtgttc      60

tttggtcctt ttaactctgc gaatatcaac aatataagaa tccagggtaa tgtcatgcca     120

ggtctcggcc aattcttcct ggtctatcca ttcattgag                            159


<210> 168
<211> 2315
<212> DNA
<213> Artificial Sequence


<220>
<223> isolate

<400> 168
aaggcagatc aacacaggga tagaatatca cgttttggac ttttgaaaca tagagcgaag      60

ctacaagaac aatatttgtg gacgcaggtt gattttaaat ccgaaggtga aaatgagata     120

tccaataagg ctctataggc tgcaaccaaa ttaaagggat gcggtcgatt tctgctattc     180

cataattact acacaataga tcaagttaaa cttgctaagg ctcattattg ttctcagcat     240

ttactttgcc ctatgtgtgc tggtgtaagg gctgctaagt caatgagtaa atacgttcaa     300

cgtattgaag aattgatgcg tcagaatcgc aaattaaagc ccgtattgat cactttaacg     360

gttaagaatg gggaagacct agaagaacgc tttaagcacc ttagacgctc atttaggacg     420

cttctcgatc gttataacga ttataagaaa aaaggtcgtg gttttaatca attctgtaaa     480

attgatggtg ctttctattc cactgaatat acctataact caaaaacaaa agaatggcat     540

ccgcacattc atattttcgc tttactcaat gaatggatag accaggaaga actttctgag     600

acctggcatg acattaccct ggactcgtac atcgtagata ttcgtagagt taaaaaaacc     660

aaagaacacg gctatagcaa agctgttgca gaggtttgta aatacgccct caaatttagc     720

gatctgtcac tggagaacac ttgggaggct tatctttctt tgaaaggtaa tagactgact     780

ggctgttttg gttctatgta tggcgttaaa ttacctgaaa aattaacaga tgatttgccc     840

cttgatgatc ttccatatat ggaacttctt taccgatttg tctttggtaa aaaatcatat 900

tacgacttag aaataacaaa agacgtgaag ccaaacaaaa ggaatgagga aggatgaacg 960

aggagcttcg acgaggcccg acgcccgaat gacttgtgaa ggcggtacgt caatcggttg 1020

tctgtgatgt gacgttccgt gaacgtgcat catcatcaga caatcgaata agcaagccta 1080

cctgtatata cagacttgta aggggttcga ccccttagac cccaaagggc gcacttagcc 1140

attgagcaaa gctcaacggc agtgctccca ccagtagcga ggaattgaat atgactgaaa 1200

ttgaaaaatt gaagaaagag attgaagact taaagctgat tattgaaagt aaaaataatc 1260

aggttgaatc taaaaataag catattaaaa gacttgagag aatcatctcg aatataaaaa 1320

aagaacaatc ccagtagtaa ccgagggggc ggagtgcccc cgaactgaca tcatctaaat 1380

ttcaaaacct tcccttcctg attacaagct ccccttagat ttctaagcgg gagtttttag 1440

ccatcccgaa tgggtggcgt cgcaaatcat aatttgcttt taaattaata gtggtttata 1500

agatggttta catagtgaat tatttaaaac tcacccttaa ttaggaatta tttatgacaa 1560

attatgatga cgaaccaacc aaagtagaaa tgcttctttc tgaaattaac aatactggaa 1620

agtctgctta tagcggtgtt cttaaacccc tgtctattcg tttacctatt caaacttatg 1680

caaaagttgt tgctattgag aattttattg gtgctgaaaa aactagtaaa aataaaatca 1740

ttaatgattt gttagaaatt gcttttgatc agatttatcc atccttaagt gaatctcaaa 1800

aacaagcgtt cgattctttc tcacaatctc ttttagatgg ttcggagtca ggcaaactat 1860

gatcaaatta gaaggaatcg ttttaaatat ctttacccag caaggcggta aagataaaaa 1920

aacaggtgat caatttgctg atcgtgacaa ggtgcaaatt ttaagtgcta tggatctgcc 1980

taacggtgac gtaaaaaatg aattatttac gttatctgta gaaaactatc gggactttag 2040

cgacttctta aatcaaaaaa tttctattcc tgttggggca atggcaagcg gtcgtaatgt 2100

gattttttat gtcgcaaaag gcgcacagcc tgaattagca aaaaccgtgt gaaacacgaa 2160

aaaaaaacag tgagttagcc ttggcttctc gctgtttttt ctttctcttg atactattaa 2220

ctaaagtggg gatgaaattg actaaaaaag acaataaaaa agcccataat tctgacactt 2280

tggcgggtga tgaattacag gctttgcaat ctgct 2315

<210> 169
<211> 50
<212> PRT
<213> Artificial Sequence

<220>
<223> orf

<400> 169
Met Val Ile Thr Ile Ile Arg Lys Lys Val Val Val Leu Ile Asn Ser
1               5                   10                  15

Val Lys Leu Met Val Leu Ser Ile Pro Leu Asn Ile Pro Ile Thr Gln
            20                  25                  30
Lys Gln Lys Asn Gly Ile Arg Thr Phe Ile Phe Ser Leu Tyr Ser Met
        35                  40                  45
Asn Gly
    50

<210> 170
<211> 261
<212> PRT
<213> Artificial Sequence

<220>
<223> orf

<400> 170
Met Leu Phe His Asn Tyr Tyr Thr Ile Asp Gln Val Lys Leu Ala Lys
1               5                   10                  15
Ala His Tyr Cys Ser Gln His Leu Leu Cys Pro Met Cys Ala Gly Val
            20                  25                  30
Arg Ala Ala Lys Ser Met Ser Lys Tyr Val Gln Arg Ile Glu Glu Leu
        35                  40                  45
Met Arg Gln Asn Arg Lys Leu Lys Pro Val Leu Ile Thr Leu Thr Val
    50                  55                  60
Lys Asn Gly Glu Asp Leu Glu Glu Arg Phe Lys His Leu Arg Arg Ser
65                  70                  75                  80
Phe Arg Thr Leu Leu Asp Arg Tyr Asn Asp Tyr Lys Lys Lys Gly Arg
                85                  90                  95
Gly Phe Asn Gln Phe Cys Lys Ile Asp Gly Ala Phe Tyr Ser Thr Glu
            100                 105                 110
Tyr Thr Tyr Asn Ser Lys Thr Lys Glu Trp His Pro His Ile His Ile
        115                 120                 125
Phe Ala Leu Leu Asn Glu Trp Ile Asp Gln Glu Glu Leu Ser Glu Thr
    130                 135                 140
Trp His Asp Ile Thr Leu Asp Ser Tyr Ile Val Asp Ile Arg Arg Val
145                 150                 155                 160
Lys Lys Thr Lys Glu His Gly Tyr Ser Lys Ala Val Ala Glu Val Cys
                165                 170                 175
Lys Tyr Ala Leu Lys Phe Ser Asp Leu Ser Leu Glu Asn Thr Trp Glu
            180                 185                 190
Ala Tyr Leu Ser Leu Lys Gly Asn Arg Leu Thr Gly Cys Phe Gly Ser
        195                 200                 205
Met Tyr Gly Val Lys Leu Pro Glu Lys Leu Thr Asp Asp Leu Pro Leu
    210                 215                 220
Asp Asp Leu Pro Tyr Met Glu Leu Leu Tyr Arg Phe Val Phe Gly Lys
225                 230                 235                 240
Lys Ser Tyr Tyr Asp Leu Glu Ile Thr Lys Asp Val Lys Pro Asn Lys
                245                 250                 255
Arg Asn Glu Glu Gly
            260

<210> 171
<211> 50
<212> PRT
<213> Artificial Sequence

<220>
<223> orf

<400> 171
Met Asn Met Thr Glu Ile Glu Lys Leu Lys Lys Glu Ile Glu Asp Leu

1               5                   10                  15
Lys Leu Ile Ile Glu Ser Lys Asn Asn Gln Val Glu Ser Lys Asn Lys
            20                  25                  30
His Ile Lys Arg Leu Glu Arg Ile Ile Ser Asn Ile Lys Lys Glu Gln
        35                  40                  45
Ser Gln
    50

<210> 172
<211> 51
<212> PRT
<213> Artificial Sequence

<220>
<223> orf

<400> 172
Met Lys Leu Thr Ile Leu Glu Ser Leu Leu Ile Ala Val Phe Leu Asn
1               5                   10                  15
Pro Cys Leu Phe Val Tyr Leu Phe Lys Leu Met Gln Lys Leu Leu Leu
            20                  25                  30
Leu Arg Ile Leu Leu Val Leu Lys Lys Leu Val Lys Ile Lys Ser Leu
        35                  40                  45
Met Ile Cys
    50

<210> 173
<211> 102
<212> PRT
<213> Artificial Sequence

<220>
<223> orf

<400> 173
Met Thr Asn Tyr Asp Asp Glu Pro Thr Lys Val Glu Met Leu Leu Ser
1               5                   10                  15
Glu Ile Asn Asn Thr Gly Lys Ser Ala Tyr Ser Gly Val Leu Lys Pro
            20                  25                  30
Leu Ser Ile Arg Leu Pro Ile Gln Thr Tyr Ala Lys Val Val Ala Ile
        35                  40                  45
Glu Asn Phe Ile Gly Ala Glu Lys Thr Ser Lys Asn Lys Ile Ile Asn
    50                  55                  60
Asp Leu Leu Glu Ile Ala Phe Asp Gln Ile Tyr Pro Ser Leu Ser Glu
65                  70                  75                  80
Ser Gln Lys Gln Ala Phe Asp Ser Phe Ser Gln Ser Leu Leu Asp Gly
                85                  90                  95
Ser Glu Ser Gly Lys Leu
            100

<210> 174
<211> 119
<212> PRT
<213> Artificial Sequence

<220>
<223> orf

<400> 174
Met Ser Lys Thr Ser Val Arg Phe Phe Leu Thr Ile Ser Phe Arg Trp

1                   5                   10                  15
Phe Gly Val Arg Gln Thr Met Ile Lys Leu Glu Gly Ile Val Leu Asn
                20                  25                  30
Ile Phe Thr Gln Gln Gly Gly Lys Asp Lys Lys Thr Gly Asp Gln Phe
            35                  40                  45
Ala Asp Arg Asp Lys Val Gln Ile Leu Ser Ala Met Asp Leu Pro Asn
    50                  55                  60
Gly Asp Val Lys Asn Glu Leu Phe Thr Leu Ser Val Glu Asn Tyr Arg
65                  70                  75                  80
Asp Phe Ser Asp Phe Leu Asn Gln Lys Ile Ser Ile Pro Val Gly Ala
                85                  90                  95
Met Ala Ser Gly Arg Asn Val Ile Phe Tyr Val Ala Lys Gly Ala Gln
            100                 105                 110
Pro Glu Leu Ala Lys Thr Val
        115

<210> 175
<211> 90
<212> PRT
<213> Artificial Sequence

<220>
<223> orf

<400> 175
Met Ala Ser Arg Cys Phe Phe Phe Leu Leu Ile Leu Leu Thr Lys Val
1               5                   10                  15
Gly Met Lys Leu Thr Lys Lys Asp Asn Lys Lys Ala His Asn Ser Asp
            20                  25                  30
Thr Leu Ala Gly Asp Glu Leu Gln Ala Leu Gln Ser Ala Lys Ala Asp
        35                  40                  45
Gln His Arg Asp Arg Ile Ser Arg Phe Gly Leu Leu Lys His Arg Ala
    50                  55                  60
Lys Leu Gln Glu Gln Tyr Leu Trp Thr Gln Val Asp Phe Lys Ser Glu
65                  70                  75                  80
Gly Glu Asn Glu Ile Ser Asn Lys Ala Leu
                85                  90

<210> 176
<211> 50
<212> PRT
<213> Artificial Sequence

<220>
<223> orf

<400> 176
Met Met Met Met His Val His Gly Thr Ser His His Arg Gln Pro Ile
1               5                   10                  15
Asp Val Pro Pro Ser Gln Val Ile Arg Ala Ser Gly Leu Val Glu Ala
            20                  25                  30
Pro Arg Ser Ser Phe Leu Ile Pro Phe Val Trp Leu His Val Phe Cys
        35                  40                  45
Tyr Phe
    50

<210> 177
<211> 53
<212> PRT
<213> Artificial Sequence

<220>
<223> orf

<400> 177
Met Ala Lys Phe Glu Gly Val Phe Thr Asn Leu Cys Asn Ser Phe Ala
1               5                   10                  15
Ile Ala Val Phe Phe Gly Phe Phe Asn Ser Thr Asn Ile Tyr Asp Val
            20                  25                  30
Arg Val Gln Gly Asn Val Met Pro Gly Leu Arg Lys Phe Phe Leu Val
        35                  40                  45
Tyr Pro Phe Ile Glu
    50

<210> 178
<211> 150
<212> DNA
<213> Artificial Sequence

<220>
<223> orf

<400> 178
atcgttataa cgattataag aaaaaaggtc gtggttttaa tcaattctgt aaaattgatg      60

gtgctttcta ttccactgaa tatacctata actcaaaaac aaaagaatgg catccgcaca     120

ttcatatttt cgctttactc aatgaatgga                                      150

<210> 179
<211> 783
<212> DNA
<213> Artificial Sequence

<220>
<223> orf

<400> 179
ctgctattcc ataattacta cacaatagat caagttaaac ttgctaaggc tcattattgt      60

tctcagcatt tactttgccc tatgtgtgct ggtgtaaggg ctgctaagtc aatgagtaaa     120

tacgttcaac gtattgaaga attgatgcgt cagaatcgca aattaaagcc cgtattgatc     180

actttaacgg ttaagaatgg ggaagaccta gaagaacgct ttaagcacct tagacgctca     240

tttaggacgc ttctcgatcg ttataacgat tataagaaaa aaggtcgtgg ttttaatcaa     300

ttctgtaaaa ttgatggtgc tttctattcc actgaatata cctataactc aaaaacaaaa     360

gaatggcatc cgcacattca tattttcgct ttactcaatg aatggataga ccaggaagaa     420

ctttctgaga cctggcatga cattaccctg gactcgtaca tcgtagatat tcgtagagtt     480

aaaaaaacca aagaacacgg ctatagcaaa gctgttgcag aggtttgtaa atacgccctc     540

aaatttagcg atctgtcact ggagaacact tgggaggctt atctttcttt gaaaggtaat     600

agactgactg gctgttttgg ttctatgtat ggcgttaaat tacctgaaaa attaacagat     660

gatttgcccc ttgatgatct tccatatatg gaacttcttt accgatttgt ctttggtaaa 720

aaatcatatt acgacttaga aataacaaaa gacgtgaagc caaacaaaag gaatgaggaa 780

gga 783

<210> 180
<211> 150
<212> DNA
<213> Artificial Sequence

<220>
<223> orf

<400> 180
ttgaatatga ctgaaattga aaaattgaag aaagagattg aagacttaaa gctgattatt 60

gaaagtaaaa ataatcaggt tgaatctaaa aataagcata ttaaaagact tgagagaatc 120

atctcgaata taaaaaaaga acaatcccag 150

<210> 181
<211> 153
<212> DNA
<213> Artificial Sequence

<220>
<223> orf

<400> 181
ctgaaattaa caatactgga aagtctgctt atagcggtgt tcttaaaccc ctgtctattc 60

gtttacctat tcaaacttat gcaaaagttg ttgctattga gaattttatt ggtgctgaaa 120

aaactagtaa aaataaaatc attaatgatt tgt 153

<210> 182
<211> 306
<212> DNA
<213> Artificial Sequence

<220>
<223> orf

<400> 182
atgacaaatt atgatgacga accaaccaaa gtagaaatgc ttctttctga aattaacaat 60

actggaaagt ctgcttatag cggtgttctt aaacccctgt ctattcgttt acctattcaa 120

acttatgcaa aagttgttgc tattgagaat tttattggtg ctgaaaaaac tagtaaaaat 180

aaaatcatta atgatttgtt agaaattgct tttgatcaga tttatccatc cttaagtgaa 240

tctcaaaaac aagcgttcga ttctttctca caatctcttt tagatggttc ggagtcaggc 300

aaacta 306

<210> 183
<211> 357
<212> DNA
<213> Artificial Sequence

<220>
<223> orf

<400> 183
atctcaaaaa caagcgttcg attctttctc acaatctctt ttagatggtt cggagtcagg 60

caaactatga tcaaattaga aggaatcgtt ttaaatatct ttacccagca aggcggtaaa 120

gataaaaaaa caggtgatca atttgctgat cgtgacaagg tgcaaatttt aagtgctatg 180

gatctgccta acggtgacgt aaaaaatgaa ttatttacgt tatctgtaga aaactatcgg 240

gactttagcg acttcttaaa tcaaaaaatt tctattcctg ttggggcaat ggcaagcggt 300

cgtaatgtga ttttttatgt cgcaaaaggc gcacagcctg aattagcaaa aaccgtg 357

<210> 184
<211> 270
<212> DNA
<213> Artificial Sequence

<220>
<223> orf

<400> 184
ttggcttctc gctgtttttt ctttctcttg atactattaa ctaaagtggg gatgaaattg 60

actaaaaaag acaataaaaa agcccataat tctgacactt tggcgggtga tgaattacag 120

gctttgcaat ctgctaaggc agatcaacac agggatagaa tatcacgttt tggacttttg 180

aaacatagag cgaagctaca agaacaatat ttgtggacgc aggttgattt taaatccgaa 240

ggtgaaaatg agatatccaa taaggctcta 270

<210> 185
<211> 150
<212> DNA
<213> Artificial Sequence

<220>
<223> orf

<400> 185
ctgatgatga tgcacgttca cggaacgtca catcacagac aaccgattga cgtaccgcct 60

tcacaagtca ttcgggcgtc gggcctcgtc gaagctcctc gttcatcctt cctcattcct 120

tttgtttggc ttcacgtctt ttgttatttc 150

<210> 186

<211> 159
<212> DNA
<213> Artificial Sequence

<220>
<223> orf

<400> 186
atcgctaaat ttgagggcgt atttacaaac ctctgcaaca gctttgctat agccgtgttc 60

tttggttttt ttaactctac gaatatctac gatgtacgag tccagggtaa tgtcatgcca 120

ggtctcagaa agttcttcct ggtctatcca ttcattgag 159

<210> 187
<211> 2460
<212> DNA
<213> Artificial Sequence

<220>
<223> isolate

<400> 187
aaggcagatc aacacaggga tagaataaca cgttttgcga ctttgaaaca tagggctaag 60

aatcaggaaa attatttatt tacactggct cagtttaaag agaactatga aaaagacgtt 120

aaaaacgagg aatctatcaa cgccctaaaa tctgctcaga agttgaatga atgcggaaac 180

tatctgctat tcaaaaattt ttacacaata ggcgaagtta aactctccaa gctccacacc 240

tgcggacagc atttgctttg tcctttctgt gctgccattc gtgcatctcg tgctatccaa 300

aaatacgttg aacgtattga tcaagtcctg caagaaaatc gcaagcttaa acctgtttta 360

attacgctta ccgttaaaaa cggttctgac ctagcggaac gctctgaaca tcttatgaag 420

tcctttagaa agctcctaga tcgtcgtagg gactatgaaa agaaagggcg aggttttaac 480

gagttctgta aggttcaagg tgctatgtat tcatatgaga atacattcaa tgagaaaaca 540

ggcgaatggc atccgcatat tcatatgttt gcactggttg accagtggat tgatcatcag 600

gaattttcag aatattggca tagccttact ggtgactcga tggttgtcga tgttcgcagg 660

gcgagaaaag aaaaaggaca cggctatagc aaagctgctg ccgaagtctg taagtatgct 720

ttgaagtttg gcgatctatc cgttgaaaaa acttgggaag ctttcaaagt tctcaaaggt 780

aaacgcttaa caggctcttt tgggcttctg tggggtgtca aaattcctga cacaatgaca 840

gacgatatgc cagaagaaga tttaccttat cttgaaatgc tttacaagtt tacatacggc 900

aaaaagtctt actacgactt actgataaca agacacgtag aagcgcaggc gggagagcgc 960

gcggagttag gaggagctac gacgacgccc gacgagagat atagcgcggg tgcttcaagt 1020

gttaaacaag gttcggaatg gagcgtggcg acccctggtc gaacagcgct ctgttctgta 1080

ccgaagtttc aacgtaaaaa agcccactgg agaatcccac cttctacccg tgttcgtgtt 1140

cggcaaagaa tccgaagatg ggacgggatt atgtataaca ttgatctttt cccatatgtc 1200

gaacataggc tattagcttt tattagttag cctgtttctt gagaaacgcg gaagcgtgca 1260

ttctgaaaat tccactaaag attactgaca taatctttag ttccttctct ccagtagcta 1320

ggaattgaat atgactgaaa ttgaaaaatt gaagaaagag attgaagact taaagctgat 1380

tattgaaagt aaaaataatc aggttgaatc taaaaataag catattaaaa gacttgagag 1440

aatcatctcg aatatgaaaa aagaacaatc tcagcagtag ccgaacgagt gtctacgagt 1500

gacgactccg aaataagaat ttcaaaacct tcctttccct gattacaaag ctccccttag 1560

atttctaagc gggagttttt agccatcccg aatgggtggc gtcacgactt aaatttgcct 1620

taatatttaa tttgatatat aaatagattt ctaaaaagat tttaatatat atcgagtaaa 1680

gattatggaa ccaactaaat tacaaatgtt cgttgctgag gttcagggta ctgctgaatc 1740

tgaatatatg ggattgtcaa aacaaatccc gctacgtctt cggattgatc tttttgctga 1800

ggttatggct cttcatgaaa ttctttcagc taatcaaaag acaccacgaa ataaggtttt 1860

aaatgacctt ttagatattg ctattgatca agttaaaaaa gaactagatc aagattctta 1920

tgaaaaattg cagactttgt ctttccatca ttacaaagaa ctatcttcct ttgattcagg 1980

tgatttatct gatgacgagg tttcaaaatg actaatctac aaggaacttt gataaatgct 2040

ttccgtgtgg atggtggtaa aggtaaagac ggcaaagaat atgaagcgcg tgacaaggtg 2100

caaattcttg gttcgttgga actcccaaac ggggaaatta aacacgaact tgttgaccta 2160

acagttgagg attctcgact atttgaacca ttcaaaaatc aggttattag tatctcgtgt 2220

ggtgctatgg ctatcggtcg taatgttgtt ttttatgttc gtaaaggcgc aaaaccgatt 2280

ttagcagaaa gtatttgata ctcgttaaaa aacagtgagt tagcgaagct actcgctgtt 2340

ttttctttta cttgatacta ttgattaaag tggggacaaa aattgcttaa aaaagacaat 2400

aaaaaagccc atatttcgga cactttggcg ggtggagaaa tacaggcttt gcaatctgct 2460

<210> 188
<211> 94
<212> PRT
<213> Artificial Sequence

<220>
<223> orf

<400> 188
Met Val Val Gly Thr Met Lys Arg Lys Gly Glu Val Leu Thr Ser Ser
1               5                   10                  15
Val Arg Phe Lys Val Leu Cys Ile His Met Arg Ile His Ser Met Arg
            20                  25                  30
Lys Gln Ala Asn Gly Ile Arg Ile Phe Ile Cys Leu His Trp Leu Thr
        35                  40                  45
Ser Gly Leu Ile Ile Arg Asn Phe Gln Asn Ile Gly Ile Ala Leu Leu

50                  55                  60
Val Thr Arg Trp Leu Ser Met Phe Ala Gly Arg Glu Lys Lys Lys Asp
65                  70                  75                  80
Thr Ala Ile Ala Lys Leu Leu Pro Lys Ser Val Ser Met Leu
                85                  90

<210> 189
<211> 74
<212> PRT
<213> Artificial Sequence

<220>
<223> orf

<400> 189
Met Arg Asp Ile Ala Arg Val Leu Gln Val Leu Asn Lys Val Arg Asn
1               5                   10                  15
Gly Ala Trp Arg Pro Leu Val Glu Gln Arg Ser Val Leu Tyr Arg Ser
            20                  25                  30
Phe Asn Val Lys Lys Pro Thr Gly Glu Ser His Leu Leu Pro Val Phe
        35                  40                  45
Val Phe Gly Lys Glu Ser Glu Asp Gly Thr Gly Leu Cys Ile Thr Leu
    50                  55                  60
Ile Phe Ser His Met Ser Asn Ile Gly Tyr
65                  70

<210> 190
<211> 401
<212> PRT
<213> Artificial Sequence

<220>
<223> orf

<400> 190
Met Thr Arg Phe Ala Thr Leu Lys His Arg Ala Lys Asn Gln Glu Asn
1               5                   10                  15
Tyr Leu Phe Thr Leu Ala Gln Phe Lys Glu Asn Tyr Glu Lys Asp Val
            20                  25                  30
Lys Asn Glu Glu Ser Ile Asn Ala Leu Lys Ser Ala Gln Lys Leu Asn
        35                  40                  45
Glu Cys Gly Asn Tyr Leu Leu Phe Lys Asn Phe Tyr Thr Ile Gly Glu
    50                  55                  60
Val Lys Leu Ser Lys Leu His Thr Cys Gly Gln His Leu Leu Cys Pro
65                  70                  75                  80
Phe Cys Ala Ala Ile Arg Ala Ser Arg Ala Ile Gln Lys Tyr Val Glu
                85                  90                  95
Arg Ile Asp Gln Val Leu Gln Glu Asn Arg Lys Leu Lys Pro Val Leu
            100                 105                 110
Ile Thr Leu Thr Val Lys Asn Gly Ser Asp Leu Ala Glu Arg Ser Glu
        115                 120                 125
His Leu Met Lys Ser Phe Arg Lys Leu Leu Asp Arg Arg Arg Asp Tyr
    130                 135                 140
Glu Lys Lys Gly Arg Gly Phe Asn Glu Phe Cys Lys Val Gln Gly Ala
145                 150                 155                 160
Met Tyr Ser Tyr Glu Asn Thr Phe Asn Glu Lys Thr Gly Glu Trp His
                165                 170                 175
Pro His Ile His Met Phe Ala Leu Val Asp Gln Trp Ile Asp His Gln
            180                 185                 190
Glu Phe Ser Glu Tyr Trp His Ser Leu Thr Gly Asp Ser Met Val Val
        195                 200                 205

Asp Val Arg Arg Ala Arg Lys Glu Lys Gly His Gly Tyr Ser Lys Ala
    210                 215                 220
Ala Ala Glu Val Cys Lys Tyr Ala Leu Lys Phe Gly Asp Leu Ser Val
225                 230                 235                 240
Glu Lys Thr Trp Glu Ala Phe Lys Val Leu Lys Gly Lys Arg Leu Thr
                245                 250                 255
Gly Ser Phe Gly Leu Leu Trp Gly Val Lys Ile Pro Asp Thr Met Thr
            260                 265                 270
Asp Asp Met Pro Glu Glu Asp Leu Pro Tyr Leu Glu Met Leu Tyr Lys
        275                 280                 285
Phe Thr Tyr Gly Lys Lys Ser Tyr Tyr Asp Leu Leu Ile Thr Arg His
    290                 295                 300
Val Glu Ala Gln Ala Gly Glu Arg Ala Glu Leu Gly Gly Ala Thr Thr
305                 310                 315                 320
Thr Pro Asp Glu Arg Tyr Ser Ala Gly Ala Ser Ser Val Lys Gln Gly
                325                 330                 335
Ser Glu Trp Ser Val Ala Thr Pro Gly Arg Thr Ala Leu Cys Ser Val
            340                 345                 350
Pro Lys Phe Gln Arg Lys Lys Ala His Trp Arg Ile Pro Pro Ser Thr
        355                 360                 365
Arg Val Arg Val Arg Gln Arg Ile Arg Arg Trp Asp Gly Ile Met Tyr
    370                 375                 380
Asn Ile Asp Leu Phe Pro Tyr Val Glu His Arg Leu Leu Ala Phe Ile
385                 390                 395                 400
Ser

<210> 191
<211> 153
<212> PRT
<213> Artificial Sequence

<220>
<223> orf

<400> 191
Met Asn Met Thr Glu Ile Glu Lys Leu Lys Lys Glu Ile Glu Asp Leu
1               5                   10                  15
Lys Leu Ile Ile Glu Ser Lys Asn Asn Gln Met Asn Met Thr Glu Ile
            20                  25                  30
Glu Lys Leu Lys Lys Glu Ile Glu Asp Leu Lys Leu Ile Ile Glu Ser
        35                  40                  45
Lys Asn Asn Gln Val Glu Ser Lys Asn Lys His Ile Lys Arg Leu Glu
    50                  55                  60
Arg Ile Ile Ser Asn Met Lys Lys Glu Gln Ser Gln Gln Met Asn Met
65                  70                  75                  80
Thr Glu Ile Glu Lys Leu Lys Lys Glu Ile Glu Asp Leu Lys Leu Ile
                85                  90                  95
Ile Glu Ser Lys Asn Asn Gln Val Glu Ser Lys Asn Lys His Ile Lys
            100                 105                 110
Arg Leu Glu Arg Ile Ile Ser Asn Met Lys Lys Glu Gln Ser Gln Gln
        115                 120                 125
Val Glu Ser Lys Asn Lys His Ile Lys Arg Leu Glu Arg Ile Ile Ser
    130                 135                 140
Asn Met Lys Lys Glu Gln Ser Gln Gln
145                 150

<210> 192
<211> 114
<212> PRT
<213> Artificial Sequence

<220>
<223> orf

<400> 192
Met Tyr Arg Val Lys Ile Met Glu Pro Thr Lys Leu Gln Met Phe Val
1               5                   10                  15
Ala Glu Val Gln Gly Thr Ala Glu Ser Glu Tyr Met Gly Leu Ser Lys
            20                  25                  30
Gln Ile Pro Leu Arg Leu Arg Ile Asp Leu Phe Ala Glu Val Met Ala
        35                  40                  45
Leu His Glu Ile Leu Ser Ala Asn Gln Lys Thr Pro Arg Asn Lys Val
    50                  55                  60
Leu Asn Asp Leu Leu Asp Ile Ala Ile Asp Gln Val Lys Lys Glu Leu
65                  70                  75                  80
Asp Gln Asp Ser Tyr Glu Lys Leu Gln Thr Leu Ser Phe His His Tyr
                85                  90                  95
Lys Glu Leu Ser Ser Phe Asp Ser Gly Asp Leu Ser Asp Asp Glu Val
            100                 105                 110
Ser Lys


<210> 193
<211> 96
<212> PRT
<213> Artificial Sequence


<220>
<223> orf

<400> 193
Met Thr Asn Leu Gln Gly Thr Leu Ile Asn Ala Phe Arg Val Asp Gly
1               5                   10                  15
Gly Lys Gly Lys Asp Gly Lys Glu Tyr Glu Ala Arg Asp Lys Val Gln
            20                  25                  30
Ile Leu Gly Ser Leu Glu Leu Pro Asn Gly Glu Ile Lys His Glu Leu
        35                  40                  45
Val Asp Leu Thr Val Glu Asp Ser Arg Leu Phe Glu Pro Phe Lys Asn
    50                  55                  60
Gln Val Ile Ser Ile Ser Cys Gly Ala Met Ala Ile Gly Arg Asn Val
65                  70                  75                  80
Val Phe Tyr Val Arg Lys Gly Ala Lys Pro Ile Leu Ala Glu Ser Ile
                85                  90                  95


<210> 194
<211> 435
<212> PRT
<213> Artificial Sequence


<220>
<223> orf

<400> 194
Met Leu Lys Lys Asp Asn Lys Lys Ala His Ile Ser Asp Thr Leu Ala
1               5                   10                  15
Gly Gly Glu Ile Gln Ala Leu Gln Ser Ala Lys Ala Asp Gln His Arg
            20                  25                  30
Asp Arg Ile Thr Arg Phe Ala Thr Leu Lys His Arg Ala Lys Asn Gln
        35                  40                  45

Glu Asn Tyr Leu Phe Thr Leu Ala Gln Phe Lys Glu Asn Tyr Glu Lys
    50                  55                  60
Asp Val Lys Asn Glu Glu Ser Ile Asn Ala Leu Lys Ser Ala Gln Lys
65                  70                  75                  80
Leu Asn Glu Cys Gly Asn Tyr Leu Leu Phe Lys Asn Phe Tyr Thr Ile
                85                  90                  95
Gly Glu Val Lys Leu Ser Lys Leu His Thr Cys Gly Gln His Leu Leu
            100                 105                 110
Cys Pro Phe Cys Ala Ala Ile Arg Ala Ser Arg Ala Ile Gln Lys Tyr
        115                 120                 125
Val Glu Arg Ile Asp Gln Val Leu Gln Glu Asn Arg Lys Leu Lys Pro
    130                 135                 140
Val Leu Ile Thr Leu Thr Val Lys Asn Gly Ser Asp Leu Ala Glu Arg
145                 150                 155                 160
Ser Glu His Leu Met Lys Ser Phe Arg Lys Leu Leu Asp Arg Arg Arg
                165                 170                 175
Asp Tyr Glu Lys Lys Gly Arg Gly Phe Asn Glu Phe Cys Lys Val Gln
            180                 185                 190
Gly Ala Met Tyr Ser Tyr Glu Asn Thr Phe Asn Glu Lys Thr Gly Glu
        195                 200                 205
Trp His Pro His Ile His Met Phe Ala Leu Val Asp Gln Trp Ile Asp
    210                 215                 220
His Gln Glu Phe Ser Glu Tyr Trp His Ser Leu Thr Gly Asp Ser Met
225                 230                 235                 240
Val Val Asp Val Arg Arg Ala Arg Lys Glu Lys Gly His Gly Tyr Ser
                245                 250                 255
Lys Ala Ala Ala Glu Val Cys Lys Tyr Ala Leu Lys Phe Gly Asp Leu
            260                 265                 270
Ser Val Glu Lys Thr Trp Glu Ala Phe Lys Val Leu Lys Gly Lys Arg
        275                 280                 285
Leu Thr Gly Ser Phe Gly Leu Leu Trp Gly Val Lys Ile Pro Asp Thr
    290                 295                 300
Met Thr Asp Asp Met Pro Glu Glu Asp Leu Pro Tyr Leu Glu Met Leu
305                 310                 315                 320
Tyr Lys Phe Thr Tyr Gly Lys Lys Ser Tyr Tyr Asp Leu Leu Ile Thr
                325                 330                 335
Arg His Val Glu Ala Gln Ala Gly Glu Arg Ala Glu Leu Gly Gly Ala
            340                 345                 350
Thr Thr Thr Pro Asp Glu Arg Tyr Ser Ala Gly Ala Ser Ser Val Lys
        355                 360                 365
Gln Gly Ser Glu Trp Ser Val Ala Thr Pro Gly Arg Thr Ala Leu Cys
    370                 375                 380
Ser Val Pro Lys Phe Gln Arg Lys Lys Ala His Trp Arg Ile Pro Pro
385                 390                 395                 400
Ser Thr Arg Val Arg Val Arg Gln Arg Ile Arg Arg Trp Asp Gly Ile
                405                 410                 415
Met Tyr Asn Ile Asp Leu Phe Pro Tyr Val Glu His Arg Leu Leu Ala
            420                 425                 430
Phe Ile Ser
        435

<210> 195
<211> 100
<212> PRT
<213> Artificial Sequence

<220>
<223> orf

<400> 195
Met Asn Gly Ser Asn Ser Arg Glu Ser Ser Thr Val Arg Ser Thr Ser
1               5                   10                  15

Ser Cys Leu Ile Ser Pro Phe Gly Ser Ser Asn Glu Pro Arg Ile Cys
            20                  25                  30
Thr Leu Ser Arg Ala Ser Tyr Ser Leu Pro Ser Leu Pro Leu Pro Pro
        35                  40                  45
Ser Thr Arg Lys Ala Phe Ile Lys Val Pro Cys Arg Leu Val Ile Leu
    50                  55                  60
Lys Pro Arg His Gln Ile Asn His Leu Asn Gln Arg Lys Ile Val Leu
65                  70                  75                  80
Cys Asn Asp Gly Lys Thr Lys Ser Ala Ile Phe His Lys Asn Leu Asp
                85                  90                  95
Leu Val Leu Phe
            100

<210> 196
<211> 63
<212> PRT
<213> Artificial Sequence

<220>
<223> orf

<400> 196
Met Phe Asp Ile Trp Glu Lys Ile Asn Val Ile His Asn Pro Val Pro
1               5                   10                  15
Ser Ser Asp Ser Leu Pro Asn Thr Asn Thr Gly Arg Arg Trp Asp Ser
            20                  25                  30
Pro Val Gly Phe Phe Thr Leu Lys Leu Arg Tyr Arg Thr Glu Arg Cys
        35                  40                  45
Ser Thr Arg Gly Arg His Ala Pro Phe Arg Thr Leu Phe Asn Thr
    50                  55                  60

<210> 197
<211> 54
<212> PRT
<213> Artificial Sequence

<220>
<223> orf

<400> 197
Met Ser Ser Val Ile Val Ser Gly Ile Leu Thr Pro His Arg Ser Pro
1               5                   10                  15
Lys Glu Pro Val Lys Arg Leu Pro Leu Arg Thr Leu Lys Ala Ser Gln
            20                  25                  30
Val Phe Ser Thr Asp Arg Ser Pro Asn Phe Lys Ala Tyr Leu Gln Thr
        35                  40                  45
Ser Ala Ala Ala Leu Leu
    50

<210> 198
<211> 50
<212> PRT
<213> Artificial Sequence

<220>
<223> orf

<400> 198
Met Phe Leu Gln Asp Leu Ile Asn Thr Phe Asn Val Phe Leu Asp Ser
1               5                   10                  15

Thr Arg Cys Thr Asn Gly Ser Thr Glu Arg Thr Lys Gln Met Leu Ser
            20                  25                  30
Ala Gly Val Glu Leu Gly Glu Phe Asn Phe Ala Tyr Cys Val Lys Ile
        35                  40                  45
Phe Glu
    50

<210> 199
<211> 56
<212> PRT
<213> Artificial Sequence

<220>
<223> orf

<400> 199
Met Ile Phe Leu Ile Leu Ser Pro Met Phe Gln Ser Arg Lys Thr Cys
1               5                   10                  15
Tyr Ser Ile Pro Val Leu Ile Cys Leu Ser Arg Leu Gln Ser Leu Tyr
            20                  25                  30
Phe Ser Thr Arg Gln Ser Val Arg Asn Met Gly Phe Phe Ile Val Phe
        35                  40                  45
Phe Lys Gln Phe Leu Ser Pro Leu
    50                  55

<210> 200
<211> 282
<212> DNA
<213> Artificial Sequence

<220>
<223> orf

<400> 200
atcgtcgtag ggactatgaa aagaaagggc gaggttttaa cgagttctgt aaggttcaag      60

gtgctatgta ttcatatgag aatacattca atgagaaaac aggcgaatgg catccgcata     120

ttcatatgtt tgcactggtt gaccagtgga ttgatcatca ggaattttca gaatattggc     180

atagccttac tggtgactcg atggttgtcg atgttcgcag ggcgagaaaa gaaaaaggac     240

acggctatag caaagctgct gccgaagtct gtaagtatgc tt                        282

<210> 201
<211> 222
<212> DNA
<213> Artificial Sequence

<220>
<223> orf

<400> 201
acgagagata tagcgcgggt gcttcaagtg ttaaacaagg ttcggaatgg agcgtggcga      60

cccctggtcg aacagcgctc tgttctgtac cgaagtttca acgtaaaaaa gcccactgga     120

gaatcccacc ttctacccgt gttcgtgttc ggcaaagaat ccgaagatgg gacgggatta     180

tgtataacat tgatcttttc ccatatgtcg aacataggct at 222

<210> 202
<211> 1203
<212> DNA
<213> Artificial Sequence

<220>
<223> orf

<400> 202
ataacacgtt ttgcgacttt gaaacatagg gctaagaatc aggaaaatta tttatttaca 60

ctggctcagt ttaaagagaa ctatgaaaaa gacgttaaaa acgaggaatc tatcaacgcc 120

ctaaaatctg ctcagaagtt gaatgaatgc ggaaactatc tgctattcaa aaatttttac 180

acaataggcg aagttaaact ctccaagctc cacacctgcg gacagcattt gctttgtcct 240

ttctgtgctg ccattcgtgc atctcgtgct atccaaaaat acgttgaacg tattgatcaa 300

gtcctgcaag aaaatcgcaa gcttaaacct gttttaatta cgcttaccgt taaaaacggt 360

tctgacctag cggaacgctc tgaacatctt atgaagtcct ttagaaagct cctagatcgt 420

cgtagggact atgaaaagaa agggcgaggt tttaacgagt tctgtaaggt tcaaggtgct 480

atgtattcat atgagaatac attcaatgag aaaacaggcg aatggcatcc gcatattcat 540

atgtttgcac tggttgacca gtggattgat catcaggaat tttcagaata ttggcatagc 600

cttactggtg actcgatggt tgtcgatgtt cgcagggcga gaaaagaaaa aggacacggc 660

tatagcaaag ctgctgccga agtctgtaag tatgctttga agtttggcga tctatccgtt 720

gaaaaaactt gggaagcttt caaagttctc aaaggtaaac gcttaacagg ctcttttggg 780

cttctgtggg gtgtcaaaat tcctgacaca atgacagacg atatgccaga agaagattta 840

ccttatcttg aaatgcttta caagtttaca tacggcaaaa agtcttacta cgacttactg 900

ataacaagac acgtagaagc gcaggcggga gagcgcgcgg agttaggagg agctacgacg 960

acgcccgacg agagatatag cgcgggtgct tcaagtgtta aacaaggttc ggaatggagc 1020

gtggcgaccc ctggtcgaac agcgctctgt tctgtaccga agtttcaacg taaaaaagcc 1080

cactggagaa tcccaccttc tacccgtgtt cgtgttcggc aaagaatccg aagatgggac 1140

gggattatgt ataacattga tcttttccca tatgtcgaac ataggctatt agcttttatt 1200

agt 1203

<210> 203
<211> 153
<212> DNA
<213> Artificial Sequence

<220>

<223> orf

<400> 203
ttgaatatga ctgaaattga aaaattgaag aaagagattg aagacttaaa gctgattatt      60

gaaagtaaaa ataatcaggt tgaatctaaa aataagcata ttaaaagact tgagagaatc     120

atctcgaata tgaaaaaaga acaatctcag cag                                  153


<210> 204
<211> 342
<212> DNA
<213> Artificial Sequence


<220>
<223> orf

<400> 204
atatatcgag taaagattat ggaaccaact aaattacaaa tgttcgttgc tgaggttcag      60

ggtactgctg aatctgaata tatgggattg tcaaaacaaa tcccgctacg tcttcggatt     120

gatctttttg ctgaggttat ggctcttcat gaaattcttt cagctaatca aaagacacca     180

cgaaataagg ttttaaatga ccttttagat attgctattg atcaagttaa aaaagaacta     240

gatcaagatt cttatgaaaa attgcagact ttgtctttcc atcattacaa agaactatct     300

tcctttgatt caggtgattt atctgatgac gaggtttcaa aa                        342


<210> 205
<211> 288
<212> DNA
<213> Artificial Sequence


<220>
<223> orf

<400> 205
atgactaatc tacaaggaac tttgataaat gctttccgtg tggatggtgg taaaggtaaa      60

gacggcaaag aatatgaagc gcgtgacaag gtgcaaattc ttggttcgtt ggaactccca     120

aacggggaaa ttaaacacga acttgttgac ctaacagttg aggattctcg actatttgaa     180

ccattcaaaa atcaggttat tagtatctcg tgtggtgcta tggctatcgg tcgtaatgtt     240

gttttttatg ttcgtaaagg cgcaaaaccg attttagcag aaagtatt                   288


<210> 206
<211> 1305
<212> DNA
<213> Artificial Sequence


<220>
<223> orf

<400> 206
ttgcttaaaa aagacaataa aaaagcccat atttcggaca ctttggcggg tggagaaata 60

caggctttgc aatctgctaa ggcagatcaa cacagggata gaataacacg ttttgcgact 120

ttgaaacata gggctaagaa tcaggaaaat tatttattta cactggctca gtttaaagag 180

aactatgaaa aagacgttaa aaacgaggaa tctatcaacg ccctaaaatc tgctcagaag 240

ttgaatgaat gcggaaacta tctgctattc aaaaattttt acacaatagg cgaagttaaa 300

ctctccaagc tccacacctg cggacagcat ttgctttgtc ctttctgtgc tgccattcgt 360

gcatctcgtg ctatccaaaa atacgttgaa cgtattgatc aagtcctgca agaaaatcgc 420

aagcttaaac ctgttttaat tacgcttacc gttaaaaacg gttctgacct agcggaacgc 480

tctgaacatc ttatgaagtc ctttagaaag ctcctagatc gtcgtaggga ctatgaaaag 540

aaagggcgag gttttaacga gttctgtaag gttcaaggtg ctatgtattc atatgagaat 600

acattcaatg agaaaacagg cgaatggcat ccgcatattc atatgtttgc actggttgac 660

cagtggattg atcatcagga attttcagaa tattggcata gccttactgg tgactcgatg 720

gttgtcgatg ttcgcagggc gagaaaagaa aaaggacacg gctatagcaa agctgctgcc 780

gaagtctgta agtatgcttt gaagtttggc gatctatccg ttgaaaaaac ttgggaagct 840

ttcaaagttc tcaaaggtaa acgcttaaca ggctcttttg ggcttctgtg gggtgtcaaa 900

attcctgaca caatgacaga cgatatgcca gaagaagatt taccttatct tgaaatgctt 960

tacaagttta catacggcaa aaagtcttac tacgacttac tgataacaag acacgtagaa 1020

gcgcaggcgg gagagcgcgc ggagttagga ggagctacga cgacgcccga cgagagatat 1080

agcgcgggtg cttcaagtgt taaacaaggt tcggaatgga gcgtggcgac ccctggtcga 1140

acagcgctct gttctgtacc gaagtttcaa cgtaaaaaag cccactggag aatcccacct 1200

tctacccgtg ttcgtgttcg gcaaagaatc cgaagatggg acgggattat gtataacatt 1260

gatcttttcc catatgtcga acataggcta ttagctttta ttagt 1305

<210> 207
<211> 300
<212> DNA
<213> Artificial Sequence

<220>
<223> orf

<400> 207
ttgaatggtt caaatagtcg agaatcctca actgttaggt caacaagttc gtgtttaatt 60

tccccgtttg ggagttccaa cgaaccaaga atttgcacct tgtcacgcgc ttcatattct 120

ttgccgtctt tacctttacc accatccaca cggaaagcat ttatcaaagt tccttgtaga 180

ttagtcattt tgaaacctcg tcatcagata aatcacctga atcaaaggaa gatagttctt 240

tgtaatgatg gaaagacaaa gtctgcaatt tttcataaga atcttgatct agttcttttt 300

<210> 208
<211> 189
<212> DNA
<213> Artificial Sequence

<220>
<223> orf

<400> 208
atgttcgaca tatgggaaaa gatcaatgtt atacataatc ccgtcccatc ttcggattct 60

ttgccgaaca cgaacacggg tagaaggtgg gattctccag tgggcttttt tacgttgaaa 120

cttcggtaca gaacagagcg ctgttcgacc aggggtcgcc acgctccatt ccgaaccttg 180

tttaacact 189

<210> 209
<211> 162
<212> DNA
<213> Artificial Sequence

<220>
<223> orf

<400> 209
atatcgtctg tcattgtgtc aggaattttg acaccccaca gaagcccaaa agagcctgtt 60

aagcgtttac ctttgagaac tttgaaagct tcccaagttt tttcaacgga tagatcgcca 120

aacttcaaag catacttaca gacttcggca gcagctttgc ta 162

<210> 210
<211> 150
<212> DNA
<213> Artificial Sequence

<220>
<223> orf

<400> 210
attttcttgc aggacttgat caatacgttc aacgtatttt tggatagcac gagatgcacg 60

aatggcagca cagaaaggac aaagcaaatg ctgtccgcag gtgtggagct tggagagttt 120

aacttcgcct attgtgtaaa aatttttgaa 150

<210> 211
<211> 168
<212> DNA
<213> Artificial Sequence

<220>
<223> orf

<400> 211
ataattttcc tgattcttag ccctatgttt caaagtcgca aaacgtgtta ttctatccct      60

gtgttgatct gccttagcag attgcaaagc ctgtatttct ccacccgcca aagtgtccga     120

aatatgggct tttttattgt cttttttaag caatttttgt ccccactt                 168


<210> 212
<211> 2564
<212> DNA
<213> Artificial Sequence


<220>
<223> isolate

<400> 212
caacacaggg atagaataac acgttttggt attttgaagc acagatcgaa gcaacaagaa      60

aaccttttgt ggactcttgc aaaattcaaa gaaaactacc caatagacac gccaaatgaa     120

gaaactgtaa aggctctaaa atctgcacag aagttgaacg gctgtggcaa ttttttgttg     180

ttcaaaaatt tttacacaat agatcaaacc aagctcgcaa aatttcatgt ttgtaatcag     240

catttgttgt gtccattttg tgcaggtatt cgtgcatcta aagcaattca aaaatataca     300

gaacgtgttg atgaagtttt aaagaaaaat cgcaagctca agcctgttct aattaccctc     360

acagtgaaaa acggtactga cctaagtgaa cggtctgcac atctcataaa atcgcttaga     420

acgcttatta agcgtcgtca ggactataat aaaaaaggtc gtggctttaa cgaattttgc     480

aaaatcaatg gtgcaatgta ttcctatgaa aatacctaca atgaacaaac caaagaatgg     540

catccgcatg ttcacatgtt tgctcttttg gatgattgga tagaccagga acaaatgtcg     600

gaaacttggc atgaaatcac tggtgattcg tttattgtag atattcgaaa agttaaaaaa     660

gaaaaagact taggctatgc aaaggctgct gctgaagtat gtaaatacgc tcttaaattt     720

ggtgatctaa ctgttgaaaa tacttgggaa gcattcaaag ttctaaaggg taaacgctta     780

agcggtgcat ttggatcact ttatggtgtc aagattcctg aaaatcttgc tgacgatatg     840

cccgatgaaa aagaccttcc gtatttagag atgctctata aattcgtgtt cgggaaacag     900

tcttactatg acctacaaat gacacgtcat gtagagccac atagcaagga caacgacgac     960

gaggcggagt ttcgacaacg agaagaaggc ggacgtagcg atttggcgat aacggagaat    1020

gctcaccgta tcgcgtgtga cgtgaaaacg gggagagttc tccgtaaaaa gcaacactgg    1080

caagtaccac caacaacgag ggtacgagtg aagaaacgaa tccgaagatg ggatggttat    1140

ttgtgtgtta tgcccgtgta aaaacataaa cttatgataa aattctagtc gctgaatagt    1200

cggacggcaa tccaactacc cagctaacca atcagatcta ttaaggagat catcatggct    1260

gttgcacata ctactgtttt gtctttgctt ggcaaagatg tttcattttc tgttttattg 1320

gatgagcaaa taaaatcctt ttttcctgaa ggaaatataa ctggcttagt cgaagaagtt 1380

attatcgctc taaatggtaa tcaccaaatc ctagttggtg atgagtatta ccaaatgtcc 1440

aaaatccatt ttatataaaa atctctgttt aattgaatgc gcctattagg gcgcattttt 1500

gtttttgata gcatttcggc gacttttgcc tatcctgcgc tagtctgttt cttgagaaac 1560

gcgtaagcgt gcattctgaa aaaatcaacc gaacgagtgt ctacgagtga cgcatccgga 1620

ttgaaaatcc taaaaccacc ttttccctga ttacaagctc ccctttgccg ttctccagta 1680

agaaccgagt catcccgaat gggtggcgga atagtgatat tgatttaata tcatcaatga 1740

tatataaata taaaaccaaa tatgtacaag acatatcaat ttaatatcag gtgaaaaaat 1800

ggaaccaact aatttacaaa tcttcgttgc tgaagttaag ggtactggcg aatcggaata 1860

catgggtatt tataaacaag tgcctttacg tcttcgtgca ggtgtttttg cagaagttga 1920

agcgcttcaa gaaatgatgg ctcgaactca aaaggtatca agaaataagg ttattaatga 1980

cttgcttgaa attgctattg atcaggttaa aggctcatta gatgagaaat ctttagaaca 2040

atttaatatg ttcgcctcta gtcactataa cgatttcact ggttctggag atttatcaga 2100

tgattaaatt agaaggaaca attattaacg ttttcaccca acagggtggc aaagataaaa 2160

aaacaggcga tgcttttgaa gatcgcgaca aggttcagct catgggtgag ttagagcttc 2220

caaatggtga tattaagcat gaattaatca atctaagtgt tgatgatggc aagcactatg 2280

aaacactaaa aaacaagcgt gtcagcattc catgcggtgc gcttgcttct ggtcgtaatg 2340

tgatttttta cgttacaaaa ggcggtacgc ctaagattct tccaagtata tgaaaaatgg 2400

aaaaaaacag tgagttagcg aagcggctcg ctgttttttt atttacttga tactattaat 2460

taagtcgggg acaaaatttc atgagaaatg acaataaaaa acccctgtta tcggacagtt 2520

tggcgaccgg tgataacaag ggtttgcaat ctgctaaggc agat 2564

<210> 213
<211> 381
<212> PRT
<213> Artificial Sequence

<220>
<223> orf

<400> 213
Met Thr Arg Phe Gly Ile Leu Lys His Arg Ser Lys Gln Gln Glu Asn
1               5                   10                  15
Leu Leu Trp Thr Leu Ala Lys Phe Lys Glu Asn Tyr Pro Ile Asp Thr
            20                  25                  30
Pro Asn Glu Glu Thr Val Lys Ala Leu Lys Ser Ala Gln Lys Leu Asn
        35                  40                  45
Gly Cys Gly Asn Phe Leu Leu Phe Lys Asn Phe Tyr Thr Ile Asp Gln
    50                  55                  60

Thr Lys Leu Ala Lys Phe His Val Cys Asn Gln His Leu Leu Cys Pro
65                  70                  75                  80
Phe Cys Ala Gly Ile Arg Ala Ser Lys Ala Ile Gln Lys Tyr Thr Glu
                85                  90                  95
Arg Val Asp Glu Val Leu Lys Lys Asn Arg Lys Leu Lys Pro Val Leu
            100                 105                 110
Ile Thr Leu Thr Val Lys Asn Gly Thr Asp Leu Ser Glu Arg Ser Ala
        115                 120                 125
His Leu Ile Lys Ser Leu Arg Thr Leu Ile Lys Arg Arg Gln Asp Tyr
    130                 135                 140
Asn Lys Lys Gly Arg Gly Phe Asn Glu Phe Cys Lys Ile Asn Gly Ala
145                 150                 155                 160
Met Tyr Ser Tyr Glu Asn Thr Tyr Asn Glu Gln Thr Lys Glu Trp His
                165                 170                 175
Pro His Val His Met Phe Ala Leu Leu Asp Asp Trp Ile Asp Gln Glu
            180                 185                 190
Gln Met Ser Glu Thr Trp His Glu Ile Thr Gly Asp Ser Phe Ile Val
        195                 200                 205
Asp Ile Arg Lys Val Lys Lys Glu Lys Asp Leu Gly Tyr Ala Lys Ala
    210                 215                 220
Ala Ala Glu Val Cys Lys Tyr Ala Leu Lys Phe Gly Asp Leu Thr Val
225                 230                 235                 240
Glu Asn Thr Trp Glu Ala Phe Lys Val Leu Lys Gly Lys Arg Leu Ser
                245                 250                 255
Gly Ala Phe Gly Ser Leu Tyr Gly Val Lys Ile Pro Glu Asn Leu Ala
            260                 265                 270
Asp Asp Met Pro Asp Glu Lys Asp Leu Pro Tyr Leu Glu Met Leu Tyr
        275                 280                 285
Lys Phe Val Phe Gly Lys Gln Ser Tyr Tyr Asp Leu Gln Met Thr Arg
    290                 295                 300
His Val Glu Pro His Ser Lys Asp Asn Asp Asp Glu Ala Glu Phe Arg
305                 310                 315                 320
Gln Arg Glu Glu Gly Gly Arg Ser Asp Leu Ala Ile Thr Glu Asn Ala
                325                 330                 335
His Arg Ile Ala Cys Asp Val Lys Thr Gly Arg Val Leu Arg Lys Lys
            340                 345                 350
Gln His Trp Gln Val Pro Pro Thr Thr Arg Val Arg Val Lys Lys Arg
        355                 360                 365
Ile Arg Arg Trp Asp Gly Tyr Leu Cys Val Met Pro Val
    370                 375                 380

<210> 214
<211> 75
<212> PRT
<213> Artificial Sequence

<220>
<223> orf

<400> 214
Met Arg Ser Ile Lys Glu Ile Ile Met Ala Val Ala His Thr Thr Val
1               5                   10                  15
Leu Ser Leu Leu Gly Lys Asp Val Ser Phe Ser Val Leu Leu Asp Glu
            20                  25                  30
Gln Ile Lys Ser Phe Phe Pro Glu Gly Asn Ile Thr Gly Leu Val Glu
        35                  40                  45
Glu Val Ile Ile Ala Leu Asn Gly Asn His Gln Ile Leu Val Gly Asp
    50                  55                  60
Glu Tyr Tyr Gln Met Ser Lys Ile His Phe Ile
65                  70                  75

<210> 215

<211> 56
<212> PRT
<213> Artificial Sequence

<220>
<223> orf

<400> 215
Met Arg Ile Glu Asn Pro Lys Thr Thr Phe Ser Leu Ile Thr Ser Ser
1               5                   10                  15
Pro Leu Pro Phe Ser Ser Lys Asn Arg Val Ile Pro Asn Gly Trp Arg
            20                  25                  30
Asn Ser Asp Ile Asp Leu Ile Ser Ser Met Ile Tyr Lys Tyr Lys Thr
        35                  40                  45
Lys Tyr Val Gln Asp Ile Ser Ile
    50                  55

<210> 216
<211> 115
<212> PRT
<213> Artificial Sequence

<220>
<223> orf

<400> 216
Met Cys Thr Arg His Ile Asn Leu Ile Ser Gly Glu Lys Met Glu Pro
1               5                   10                  15
Thr Asn Leu Gln Ile Phe Val Ala Glu Val Lys Gly Thr Gly Glu Ser
            20                  25                  30
Glu Tyr Met Gly Ile Tyr Lys Gln Val Pro Leu Arg Leu Arg Ala Gly
        35                  40                  45
Val Phe Ala Glu Val Glu Ala Leu Gln Glu Met Met Ala Arg Thr Gln
    50                  55                  60
Lys Val Ser Arg Asn Lys Val Ile Asn Asp Leu Leu Glu Ile Ala Ile
65                  70                  75                  80
Asp Gln Val Lys Gly Ser Leu Asp Glu Lys Ser Leu Glu Gln Phe Asn
                85                  90                  95
Met Phe Ala Ser Ser His Tyr Asn Asp Phe Thr Gly Ser Gly Asp Leu
            100                 105                 110
Ser Asp Asp
        115

<210> 217
<211> 115
<212> PRT
<213> Artificial Sequence

<220>
<223> orf

<400> 217
Met Cys Ser Pro Leu Val Thr Ile Thr Ile Ser Leu Val Leu Glu Ile
1               5                   10                  15
Tyr Gln Met Ile Lys Leu Glu Gly Thr Ile Ile Asn Val Phe Thr Gln
            20                  25                  30
Gln Gly Gly Lys Asp Lys Lys Thr Gly Asp Ala Phe Glu Asp Arg Asp
        35                  40                  45
Lys Val Gln Leu Met Gly Glu Leu Glu Leu Pro Asn Gly Asp Ile Lys
    50                  55                  60

His Glu Leu Ile Asn Leu Ser Val Asp Asp Gly Lys His Tyr Glu Thr
65                  70                  75                  80
Leu Lys Asn Lys Arg Val Ser Ile Pro Cys Gly Ala Leu Ala Ser Gly
                85                  90                  95
Arg Asn Val Ile Phe Tyr Val Thr Lys Gly Gly Thr Pro Lys Ile Leu
            100                 105                 110
Pro Ser Ile
        115

<210> 218
<211> 414
<212> PRT
<213> Artificial Sequence

<220>
<223> orf

<400> 218
Met Arg Asn Asp Asn Lys Lys Pro Leu Leu Ser Asp Ser Leu Ala Thr
1               5                   10                  15
Gly Asp Asn Lys Gly Leu Gln Ser Ala Lys Ala Asp Gln His Arg Asp
            20                  25                  30
Arg Ile Thr Arg Phe Gly Ile Leu Lys His Arg Ser Lys Gln Gln Glu
        35                  40                  45
Asn Leu Leu Trp Thr Leu Ala Lys Phe Lys Glu Asn Tyr Pro Ile Asp
    50                  55                  60
Thr Pro Asn Glu Glu Thr Val Lys Ala Leu Lys Ser Ala Gln Lys Leu
65                  70                  75                  80
Asn Gly Cys Gly Asn Phe Leu Leu Phe Lys Asn Phe Tyr Thr Ile Asp
                85                  90                  95
Gln Thr Lys Leu Ala Lys Phe His Val Cys Asn Gln His Leu Leu Cys
            100                 105                 110
Pro Phe Cys Ala Gly Ile Arg Ala Ser Lys Ala Ile Gln Lys Tyr Thr
        115                 120                 125
Glu Arg Val Asp Glu Val Leu Lys Lys Asn Arg Lys Leu Lys Pro Val
    130                 135                 140
Leu Ile Thr Leu Thr Val Lys Asn Gly Thr Asp Leu Ser Glu Arg Ser
145                 150                 155                 160
Ala His Leu Ile Lys Ser Leu Arg Thr Leu Ile Lys Arg Arg Gln Asp
                165                 170                 175
Tyr Asn Lys Lys Gly Arg Gly Phe Asn Glu Phe Cys Lys Ile Asn Gly
            180                 185                 190
Ala Met Tyr Ser Tyr Glu Asn Thr Tyr Asn Glu Gln Thr Lys Glu Trp
        195                 200                 205
His Pro His Val His Met Phe Ala Leu Leu Asp Asp Trp Ile Asp Gln
    210                 215                 220
Glu Gln Met Ser Glu Thr Trp His Glu Ile Thr Gly Asp Ser Phe Ile
225                 230                 235                 240
Val Asp Ile Arg Lys Val Lys Lys Glu Lys Asp Leu Gly Tyr Ala Lys
                245                 250                 255
Ala Ala Ala Glu Val Cys Lys Tyr Ala Leu Lys Phe Gly Asp Leu Thr
            260                 265                 270
Val Glu Asn Thr Trp Glu Ala Phe Lys Val Leu Lys Gly Lys Arg Leu
        275                 280                 285
Ser Gly Ala Phe Gly Ser Leu Tyr Gly Val Lys Ile Pro Glu Asn Leu
    290                 295                 300
Ala Asp Asp Met Pro Asp Glu Lys Asp Leu Pro Tyr Leu Glu Met Leu
305                 310                 315                 320
Tyr Lys Phe Val Phe Gly Lys Gln Ser Tyr Tyr Asp Leu Gln Met Thr
                325                 330                 335
Arg His Val Glu Pro His Ser Lys Asp Asn Asp Asp Glu Ala Glu Phe
            340                 345                 350

Arg Gln Arg Glu Glu Gly Gly Arg Ser Asp Leu Ala Ile Thr Glu Asn
        355                 360                 365
Ala His Arg Ile Ala Cys Asp Val Lys Thr Gly Arg Val Leu Arg Lys
    370                 375                 380
Lys Gln His Trp Gln Val Pro Pro Thr Thr Arg Val Arg Val Lys Lys
385                 390                 395                 400
Arg Ile Arg Arg Trp Asp Gly Tyr Leu Cys Val Met Pro Val
                405                 410

<210> 219
<211> 59
<212> PRT
<213> Artificial Sequence

<220>
<223> orf

<400> 219
Met Pro Met Tyr Ser Asp Ser Pro Val Pro Leu Thr Ser Ala Thr Lys
1               5                   10                  15
Ile Cys Lys Leu Val Gly Ser Ile Phe Ser Pro Asp Ile Lys Leu Ile
            20                  25                  30
Cys Leu Val His Ile Trp Phe Tyr Ile Tyr Ile Ser Leu Met Ile Leu
        35                  40                  45
Asn Gln Tyr His Tyr Ser Ala Thr His Ser Gly
    50                  55

<210> 220
<211> 59
<212> PRT
<213> Artificial Sequence

<220>
<223> orf

<400> 220
Met Asp Met Ser Cys Thr Tyr Leu Val Leu Tyr Leu Tyr Ile Ile Asp
1               5                   10                  15
Asp Ile Lys Ser Ile Ser Leu Phe Arg His Pro Phe Gly Met Thr Arg
            20                  25                  30
Phe Leu Leu Glu Asn Gly Lys Gly Glu Leu Val Ile Arg Glu Lys Val
        35                  40                  45
Val Leu Gly Phe Ser Ile Arg Met Arg His Ser
    50                  55

<210> 221
<211> 64
<212> PRT
<213> Artificial Sequence

<220>
<223> orf

<400> 221
Met Asn Ile Thr Ile Pro Pro Pro Ile Arg Asp Asp Ser Val Leu Thr
1               5                   10                  15
Gly Glu Arg Gln Arg Gly Ala Cys Asn Gln Gly Lys Gly Gly Phe Arg
            20                  25                  30
Ile Phe Asn Pro Asp Ala Ser Leu Val Asp Thr Arg Ser Val Asp Phe
        35                  40                  45

Phe Arg Met His Ala Tyr Ala Phe Leu Lys Lys Gln Thr Ser Ala Gly
    50                  55                  60

<210> 222
<211> 53
<212> PRT
<213> Artificial Sequence

<220>
<223> orf

<400> 222
Met Ile Thr Ser Ser Thr Lys Pro Val Ile Phe Pro Ser Gly Lys Lys
1               5                   10                  15
Asp Phe Ile Cys Ser Ser Asn Lys Thr Glu Asn Glu Thr Ser Leu Pro
            20                  25                  30
Ser Lys Asp Lys Thr Val Val Cys Ala Thr Ala Met Met Ile Ser Leu
        35                  40                  45
Ile Asp Leu Ile Gly
    50

<210> 223
<211> 100
<212> PRT
<213> Artificial Sequence

<220>
<223> orf

<400> 223
Met Asp Cys Arg Pro Thr Ile Gln Arg Leu Glu Phe Tyr His Lys Phe
1               5                   10                  15
Met Phe Leu His Gly His Asn Thr Gln Ile Thr Ile Pro Ser Ser Asp
            20                  25                  30
Ser Phe Leu His Ser Tyr Pro Arg Cys Trp Trp Tyr Leu Pro Val Leu
        35                  40                  45
Leu Phe Thr Glu Asn Ser Pro Arg Phe His Val Thr Arg Asp Thr Val
    50                  55                  60
Ser Ile Leu Arg Tyr Arg Gln Ile Ala Thr Ser Ala Phe Phe Ser Leu
65                  70                  75                  80
Ser Lys Leu Arg Leu Val Val Val Val Leu Ala Met Trp Leu Tyr Met
                85                  90                  95
Thr Cys His Leu
            100

<210> 224
<211> 106
<212> PRT
<213> Artificial Sequence

<220>
<223> orf

<400> 224
Met Phe Gly Phe Val Ser Ser Leu Val Pro Ser Leu Leu Val Val Leu
1               5                   10                  15
Ala Ser Val Ala Phe Tyr Gly Glu Leu Ser Pro Phe Ser Arg His Thr
            20                  25                  30

Arg Tyr Gly Glu His Ser Pro Leu Ser Pro Asn Arg Tyr Val Arg Leu
        35                  40                  45
Leu Leu Val Val Glu Thr Pro Pro Arg Arg Arg Cys Pro Cys Tyr Val
    50                  55                  60
Ala Leu His Asp Val Ser Phe Val Gly His Ser Lys Thr Val Ser Arg
65                  70                  75                  80
Thr Arg Ile Tyr Arg Ala Ser Leu Asn Thr Glu Gly Leu Phe His Arg
                85                  90                  95
Ala Tyr Arg Gln Gln Asp Phe Gln Glu Ser
            100                 105

<210> 225
<211> 86
<212> PRT
<213> Artificial Sequence

<220>
<223> orf

<400> 225
Met Tyr Asn Lys Arg Ile Thr Ser Asp Phe Met Pro Ser Phe Arg His
1               5                   10                  15
Leu Phe Leu Val Tyr Pro Ile Ile Gln Lys Ser Lys His Val Asn Met
            20                  25                  30
Arg Met Pro Phe Phe Gly Leu Phe Ile Val Gly Ile Phe Ile Gly Ile
        35                  40                  45
His Cys Thr Ile Asp Phe Ala Lys Phe Val Lys Ala Thr Thr Phe Phe
    50                  55                  60
Ile Ile Val Leu Thr Thr Leu Asn Lys Arg Ser Lys Arg Phe Tyr Glu
65                  70                  75                  80
Met Cys Arg Pro Phe Thr
                85

<210> 226
<211> 58
<212> PRT
<213> Artificial Sequence

<220>
<223> orf

<400> 226
Met Asn Thr Phe Cys Ile Phe Leu Asn Cys Phe Arg Cys Thr Asn Thr
1               5                   10                  15
Cys Thr Lys Trp Thr Gln Gln Met Leu Ile Thr Asn Met Lys Phe Cys
            20                  25                  30
Glu Leu Gly Leu Ile Tyr Cys Val Lys Ile Phe Glu Gln Gln Lys Ile
        35                  40                  45
Ala Thr Ala Val Gln Leu Leu Cys Arg Phe
    50                  55

<210> 227
<211> 57
<212> PRT
<213> Artificial Sequence

<220>
<223> orf

<400> 227

Met Trp Arg Val Tyr Trp Val Val Phe Phe Glu Phe Cys Lys Ser Pro
1               5                   10                  15
Gln Lys Val Phe Leu Leu Leu Arg Ser Val Leu Gln Asn Thr Lys Thr
            20                  25                  30
Cys Tyr Ser Ile Pro Val Leu Ile Cys Leu Ser Arg Leu Gln Thr Leu
        35                  40                  45
Val Ile Thr Gly Arg Gln Thr Val Arg
    50                  55

<210> 228
<211> 1143
<212> DNA
<213> Artificial Sequence

<220>
<223> orf

<400> 228
ataacacgtt ttggtatttt gaagcacaga tcgaagcaac aagaaaacct tttgtggact      60

cttgcaaaat tcaaagaaaa ctacccaata gacacgccaa atgaagaaac tgtaaaggct     120

ctaaaatctg cacagaagtt gaacggctgt ggcaattttt tgttgttcaa aaatttttac     180

acaatagatc aaaccaagct cgcaaaattt catgtttgta atcagcattt gttgtgtcca     240

ttttgtgcag gtattcgtgc atctaaagca attcaaaaat atacagaacg tgttgatgaa     300

gttttaaaga aaaatcgcaa gctcaagcct gttctaatta ccctcacagt gaaaaacggt     360

actgacctaa gtgaacggtc tgcacatctc ataaaatcgc ttagaacgct tattaagcgt     420

cgtcaggact ataataaaaa aggtcgtggc tttaacgaat tttgcaaaat caatggtgca     480

atgtattcct atgaaaatac ctacaatgaa caaaccaaag aatggcatcc gcatgttcac     540

atgtttgctc ttttggatga ttggatagac caggaacaaa tgtcggaaac ttggcatgaa     600

atcactggtg attcgtttat tgtagatatt cgaaaagtta aaaaagaaaa agacttaggc     660

tatgcaaagg ctgctgctga agtatgtaaa tacgctctta aatttggtga tctaactgtt     720

gaaaatactt gggaagcatt caaagttcta aagggtaaac gcttaagcgg tgcatttgga     780

tcactttatg gtgtcaagat tcctgaaaat cttgctgacg atatgcccga tgaaaaagac     840

cttccgtatt tagagatgct ctataaattc gtgttcggga aacagtctta ctatgaccta     900

caaatgacac gtcatgtaga gccacatagc aaggacaacg acgacgaggc ggagtttcga     960

caacgagaag aaggcggacg tagcgatttg gcgataacgg agaatgctca ccgtatcgcg    1020

tgtgacgtga aaacggggag agttctccgt aaaaagcaac actggcaagt accaccaaca    1080

acgagggtac gagtgaagaa acgaatccga agatgggatg gttatttgtg tgttatgccc    1140

gtg                                                                  1143

<210> 229
<211> 225
<212> DNA

<213> Artificial Sequence

<220>
<223> orf

<400> 229
atcagatcta ttaaggagat catcatggct gttgcacata ctactgtttt gtctttgctt      60

ggcaaagatg tttcattttc tgttttattg gatgagcaaa taaaatcctt ttttcctgaa     120

ggaaatataa ctggcttagt cgaagaagtt attatcgctc taaatggtaa tcaccaaatc     180

ctagttggtg atgagtatta ccaaatgtcc aaaatccatt ttata                     225

<210> 230
<211> 168
<212> DNA
<213> Artificial Sequence

<220>
<223> orf

<400> 230
atccggattg aaaatcctaa aaccaccttt tccctgatta caagctcccc tttgccgttc      60

tccagtaaga accgagtcat cccgaatggg tggcggaata gtgatattga tttaatatca     120

tcaatgatat ataaatataa aaccaaatat gtacaagaca tatcaatt                  168

<210> 231
<211> 345
<212> DNA
<213> Artificial Sequence

<220>
<223> orf

<400> 231
atatgtacaa gacatatcaa tttaatatca ggtgaaaaaa tggaaccaac taatttacaa      60

atcttcgttg ctgaagttaa gggtactggc gaatcggaat acatgggtat ttataaacaa     120

gtgcctttac gtcttcgtgc aggtgttttt gcagaagttg aagcgcttca agaaatgatg     180

gctcgaactc aaaaggtatc aagaaataag gttattaatg acttgcttga aattgctatt     240

gatcaggtta aaggctcatt agatgagaaa tctttagaac aatttaatat gttcgcctct     300

agtcactata acgatttcac tggttctgga gatttatcag atgat                     345

<210> 232
<211> 345
<212> DNA
<213> Artificial Sequence

<220>

<223> orf

<400> 232
atatgttcgc ctctagtcac tataacgatt tcactggttc tggagattta tcagatgatt      60

aaattagaag gaacaattat taacgttttc acccaacagg gtggcaaaga taaaaaaaca     120

ggcgatgctt ttgaagatcg cgacaaggtt cagctcatgg gtgagttaga gcttccaaat     180

ggtgatatta agcatgaatt aatcaatcta agtgttgatg atggcaagca ctatgaaaca     240

ctaaaaaaca agcgtgtcag cattccatgc ggtgcgcttg cttctggtcg taatgtgatt     300

ttttacgtta caaaaggcgg tacgcctaag attcttccaa gtata                     345

<210> 233
<211> 1242
<212> DNA
<213> Artificial Sequence

<220>
<223> orf

<400> 233
atgagaaatg acaataaaaa acccctgtta tcggacagtt tggcgaccgg tgataacaag      60

ggtttgcaat ctgctaaggc agatcaacac agggatagaa taacacgttt tggtattttg     120

aagcacagat cgaagcaaca agaaaacctt ttgtggactc ttgcaaaatt caaagaaaac     180

tacccaatag acacgccaaa tgaagaaact gtaaaggctc taaaatctgc acagaagttg     240

aacggctgtg gcaatttttt gttgttcaaa aatttttaca caatagatca aaccaagctc     300

gcaaaatttc atgtttgtaa tcagcatttg ttgtgtccat tttgtgcagg tattcgtgca     360

tctaaagcaa ttcaaaaata tacagaacgt gttgatgaag ttttaaagaa aaatcgcaag     420

ctcaagcctg ttctaattac cctcacagtg aaaaacggta ctgacctaag tgaacggtct     480

gcacatctca taaaatcgct tagaacgctt attaagcgtc gtcaggacta taataaaaaa     540

ggtcgtggct ttaacgaatt ttgcaaaatc aatggtgcaa tgtattccta tgaaaatacc     600

tacaatgaac aaaccaaaga atggcatccg catgttcaca tgtttgctct tttggatgat     660

tggatagacc aggaacaaat gtcggaaact tggcatgaaa tcactggtga ttcgtttatt     720

gtagatattc gaaaagttaa aaaagaaaaa gacttaggct atgcaaaggc tgctgctgaa     780

gtatgtaaat acgctcttaa atttggtgat ctaactgttg aaaatacttg ggaagcattc     840

aaagttctaa agggtaaacg cttaagcggt gcatttggat cactttatgg tgtcaagatt     900

cctgaaaatc ttgctgacga tatgcccgat gaaaaagacc ttccgtattt agagatgctc     960

tataaattcg tgttcgggaa acagtcttac tatgacctac aaatgacacg tcatgtagag    1020

ccacatagca aggacaacga cgacgaggcg gagtttcgac aacgagaaga aggcggacgt    1080

agcgatttgg cgataacgga gaatgctcac cgtatcgcgt gtgacgtgaa aacggggaga    1140

gttctccgta aaaagcaaca ctggcaagta ccaccaacaa cgagggtacg agtgaagaaa 1200

cgaatccgaa gatgggatgg ttatttgtgt gttatgcccg tg 1242

<210> 234
<211> 177
<212> DNA
<213> Artificial Sequence

<220>
<223> orf

<400> 234
atacccatgt attccgattc gccagtaccc ttaacttcag caacgaagat ttgtaaatta 60

gttggttcca ttttttcacc tgatattaaa ttgatatgtc ttgtacatat ttggttttat 120

atttatatat cattgatgat attaaatcaa tatcactatt ccgccaccca ttcggga 177

<210> 235
<211> 177
<212> DNA
<213> Artificial Sequence

<220>
<223> orf

<400> 235
attgatatgt cttgtacata tttggtttta tatttatata tcattgatga tattaaatca 60

atatcactat tccgccaccc attcgggatg actcggttct tactggagaa cggcaaaggg 120

gagcttgtaa tcagggaaaa ggtggtttta ggattttcaa tccggatgcg tcactcg 177

<210> 236
<211> 192
<212> DNA
<213> Artificial Sequence

<220>
<223> orf

<400> 236
atcaatatca ctattccgcc acccattcgg gatgactcgg ttcttactgg agaacggcaa 60

aggggagctt gtaatcaggg aaaaggtggt tttaggattt tcaatccgga tgcgtcactc 120

gtagacactc gttcggttga ttttttcaga atgcacgctt acgcgtttct caagaaacag 180

actagcgcag ga 192

<210> 237
<211> 154
<212> DNA
<213> Artificial Sequence

<220>
<223> orf

<400> 237
ataataactt cttcgactaa gccagttata tttccttcag gaaaaaagga ttttatttgc      60

tcatccaata aaacagaaaa tgaaacatct ttgccaagca aagacaaaac agtagtatgt     120

gcaacagcca tgatgatctc cttaatagat ctga                                 154


<210> 238
<211> 300
<212> DNA
<213> Artificial Sequence


<220>
<223> orf

<400> 238
ttggattgcc gtccgactat tcagcgacta gaattttatc ataagtttat gtttttacac      60

gggcataaca cacaaataac catcccatct tcggattcgt ttcttcactc gtaccctcgt     120

tgttggtggt acttgccagt gttgcttttt acggagaact ctccccgttt tcacgtcaca     180

cgcgatacgg tgagcattct ccgttatcgc caaatcgcta cgtccgcctt cttctcgttg     240

tcgaaactcc gcctcgtcgt cgttgtcctt gctatgtggc tctacatgac gtgtcatttg     300


<210> 239
<211> 318
<212> DNA
<213> Artificial Sequence


<220>
<223> orf

<400> 239
atcttcggat tcgtttcttc actcgtaccc tcgttgttgg tggtacttgc cagtgttgct      60

ttttacggag aactctcccc gttttcacgt cacacgcgat acggtgagca ttctccgtta     120

tcgccaaatc gctacgtccg ccttcttctc gttgtcgaaa ctccgcctcg tcgtcgttgt     180

ccttgctatg tggctctaca tgacgtgtca tttgtaggtc atagtaagac tgtttcccga     240

acacgaattt atagagcatc tctaaatacg gaaggtcttt ttcatcgggc atatcgtcag     300

caagattttc aggaatct                                                   318


<210> 240
<211> 258
<212> DNA
<213> Artificial Sequence

<220>
<223> orf

<400> 240
atctacaata aacgaatcac cagtgatttc atgccaagtt tccgacattt gttcctggtc      60

tatccaatca tccaaaagag caaacatgtg aacatgcgga tgccattctt tggtttgttc     120

attgtaggta ttttcatagg aatacattgc accattgatt ttgcaaaatt cgttaaagcc     180

acgacctttt ttattatagt cctgacgacg cttaataagc gttctaagcg attttatgag     240

atgtgcagac cgttcact                                                   258


<210> 241
<211> 174
<212> DNA
<213> Artificial Sequence


<220>
<223> orf

<400> 241
atcaacacgt tctgtatatt tttgaattgc tttagatgca cgaatacctg cacaaaatgg      60

acacaacaaa tgctgattac aaacatgaaa ttttgcgagc ttggtttgat ctattgtgta     120

aaaatttttg aacaacaaaa aattgccaca gccgttcaac ttctgtgcag attt           174


<210> 242
<211> 170
<212> DNA
<213> Artificial Sequence


<220>
<223> orf

<400> 242
atttggcgtg tctattgggt agttttcttt gaattttgca agagtccaca aaaggttttc      60

ttgttgcttc gatctgtgct tcaaaatacc aaaacgtgtt attctatccc tgtgttgatc     120

tgccttagca gattgcaaac ccttgttatc accggtcgcc aaactgtccg                170


<210> 243
<211> 2850
<212> DNA
<213> Artificial Sequence


<220>
<223> isolate

<400> 243
caacacaggg atagaataac acgttttggg attttgaaac atagagcgaa gcaacaagaa      60

aattttttgt ggactctggc aaaattcaaa gaaaactatc ctaaagatgc tccaagtgaa     120

gaaagcacaa gagcaattaa atctgcacag aaattaaaag agtgcggaaa tttcttacta 180

ttcaaaaatt tttacacaat agaccaaact aagctctcaa aatttcatgt atgtgggcag 240

catttgttat gtcctttctg tgctggtatt cgtgcatcta aagcaattca aaaatatact 300

gaacgtgttg atgaagtgct taagaaaaat cgcaagctta agcctgtttt aatcacgttt 360

acggtgaaga atggaatcga cttaggggaa agatcatccc acctcataaa atcgtttaga 420

acgcttctga agcgtcgtag ggactatatt aaaaaaggtc gtgggtttaa tgagttctgt 480

aagatcaatg gggctgtgta ttcttatgaa aacacgtata acgagcaaac aaaggaatgg 540

catccgcact tacatatgtt tgcacttttg gatgattgga tagaccagga agagctttca 600

cagtattggc attccatcac tggtgattca atgattgtag atattcgcag ggtcaaaaaa 660

gaaaaaggct taggctattc aaaggctgct gcggaagtat gtaaatatgc tcttaaattc 720

ggtgatttgt ctgtccaaaa tacttgggaa gctttcaaag tcttaaaagg taagcgttta 780

agtggtgcat ttggttcttt atggggtgtc aaaattcctg aaaatcttgc tgatgatatg 840

cctgatgaaa aagatcttcc ttacttagaa atgctctata aattcgtatt tggaaaacag 900

tcttactatg acttaactat gacacgacac gtagagccac aatgcaagga cagggacaac 960

gacgaagagg aggtagcgac gactgacgga ggcggaaatg gacgcagcag tgaggcgatc 1020

aaggacgggc gaggacgggc ggagcgcacc gagggcacgc agggtgcacg gcaccgcccg 1080

cagcacggac gtaaaaaaca acactggcaa gtacctccag taacgagggt acgagtgaaa 1140

aaacggatta aaagatggga tggttatttg tgtgttatgc acatgtaaaa tcataaaccc 1200

atgataaaat tctagtcgct gaatagtcgg attgcagtcc aactacccag ctaaccacaa 1260

cgatctaaga aggagatcat catggctgtt gcacatacta ctgttttgtc tttgcttggc 1320

aaagaagttt cattttctgt tttattagat gaccaaaata aaatcttttc ccctggcggt 1380

attcatataa ccggattagt tgaaaaagtt gtaattgatt taactggtga tcatgcaatt 1440

ttagttggtg atgagtttta tcaactttcc gaaatagact tacctgttta gttaacgatt 1500

ttaccaatgc ccgaaaatac atatttttgg gcattttttt gtccgtttct tgagaaatgc 1560

gtaagcgtgc attctgaaaa aataaacatt gagattggcg aagagattcg gggcgctggg 1620

cgtggggtcg ggatgtcact cgatgtcccg ccctgtgact ggcgacacga gatattcgac 1680

aatctcactt tagaaacaat atcagagggc acggagtgtg cccgaactga agaaataaaa 1740

attaaaaact acctttccct gattacaagc tccctttgga ttttctccgg gataaacaga 1800

gccatccgga tggtggcgac tgactattac ttttgctttt ttacttaata tggtttataa 1860

gttggtttac aggttaaacc atataaaatc taaccttaat ttaggaatta tttatgacaa 1920

attatcatga tgaaccaact aaagtagaaa tgcttctttc tgaaattaac cgtactggta 1980

aatcttctta ttctggtgct cttaaaccat tatctattcg tttacctatt caaacttacg 2040

caaaagtcgt tgctattgaa aactttattg gtgctgaaaa aactagtaaa aataaagtta 2100

tcaatgattt gttagaaatt gcttttgatc agatttatcc atctttaaat gaatctcaaa 2160

aacatgcatt tgatcatttt tcacagtctc ttttagatgg tttggagtca ggaaaattat 2220

gattggacct caagttcttt caaagggaac aatggattcc ctggaagaag catctgctta 2280

tttcaagcaa gttttacaag aacatcttga taacaaacgt gacatcacat tatttgcgat 2340

tgctgttgta ggcggtcgtt tgaaagtcgt agagggatta ccaaaatgat taaattagaa 2400

ggcacaatta ttaatgtttt tacccagcaa ggcggtaaag acaaaaaaac tggtgaagca 2460

tttgccgatc gtgacaagat tcaattgtta ggtgagtttg aactccctaa cggtgataaa 2520

aaaaatgaat taattgatct tactgttgaa aacggtaagg aatatgaagg tatgaaaaat 2580

aagcgtgtga gcattccatg tggtgctctt gcttcgggtc gaaatgtgat tttttatgtc 2640

gcaaaaggcg caacacccaa acttttaaac acagtttgat actcgttaaa aaacagcaac 2700

ttacgaagta agttcgctgt tttttctttt acttgatact attaattaaa gtggggacaa 2760

aaattgactg taaaagacaa taaaaaagcc catatttctg atgctttggc gggcgacgaa 2820

atacaggctt tgcaatctgc taaggcagat 2850

<210> 244
<211> 390
<212> PRT
<213> Artificial Sequence

<220>
<223> orf

<400> 244
Met Thr Arg Phe Gly Ile Leu Lys His Arg Ala Lys Gln Gln Glu Asn
1               5                   10                  15
Phe Leu Trp Thr Leu Ala Lys Phe Lys Glu Asn Tyr Pro Lys Asp Ala
            20                  25                  30
Pro Ser Glu Glu Ser Thr Arg Ala Ile Lys Ser Ala Gln Lys Leu Lys
        35                  40                  45
Glu Cys Gly Asn Phe Leu Leu Phe Lys Asn Phe Tyr Thr Ile Asp Gln
    50                  55                  60
Thr Lys Leu Ser Lys Phe His Val Cys Gly Gln His Leu Leu Cys Pro
65                  70                  75                  80
Phe Cys Ala Gly Ile Arg Ala Ser Lys Ala Ile Gln Lys Tyr Thr Glu
                85                  90                  95
Arg Val Asp Glu Val Leu Lys Lys Asn Arg Lys Leu Lys Pro Val Leu
            100                 105                 110
Ile Thr Phe Thr Val Lys Asn Gly Ile Asp Leu Gly Glu Arg Ser Ser
        115                 120                 125
His Leu Ile Lys Ser Phe Arg Thr Leu Leu Lys Arg Arg Arg Asp Tyr
    130                 135                 140
Ile Lys Lys Gly Arg Gly Phe Asn Glu Phe Cys Lys Ile Asn Gly Ala
145                 150                 155                 160
Val Tyr Ser Tyr Glu Asn Thr Tyr Asn Glu Gln Thr Lys Glu Trp His
                165                 170                 175
Pro His Leu His Met Phe Ala Leu Leu Asp Asp Trp Ile Asp Gln Glu

180                 185                 190
Glu Leu Ser Gln Tyr Trp His Ser Ile Thr Gly Asp Ser Met Ile Val
        195                 200                 205
Asp Ile Arg Arg Val Lys Lys Glu Lys Gly Leu Gly Tyr Ser Lys Ala
    210                 215                 220
Ala Ala Glu Val Cys Lys Tyr Ala Leu Lys Phe Gly Asp Leu Ser Val
225                 230                 235                 240
Gln Asn Thr Trp Glu Ala Phe Lys Val Leu Lys Gly Lys Arg Leu Ser
                245                 250                 255
Gly Ala Phe Gly Ser Leu Trp Gly Val Lys Ile Pro Glu Asn Leu Ala
            260                 265                 270
Asp Asp Met Pro Asp Glu Lys Asp Leu Pro Tyr Leu Glu Met Leu Tyr
        275                 280                 285
Lys Phe Val Phe Gly Lys Gln Ser Tyr Tyr Asp Leu Thr Met Thr Arg
    290                 295                 300
His Val Glu Pro Gln Cys Lys Asp Arg Asp Asn Asp Glu Glu Glu Val
305                 310                 315                 320
Ala Thr Thr Asp Gly Gly Gly Asn Gly Arg Ser Ser Glu Ala Ile Lys
                325                 330                 335
Asp Gly Arg Gly Arg Ala Glu Arg Thr Glu Gly Thr Gln Gly Ala Arg
            340                 345                 350
His Arg Pro Gln His Gly Arg Lys Lys Gln His Trp Gln Val Pro Pro
        355                 360                 365
Val Thr Arg Val Arg Val Lys Lys Arg Ile Lys Arg Trp Asp Gly Tyr
    370                 375                 380
Leu Cys Val Met His Met
385                 390

<210> 245
<211> 71
<212> PRT
<213> Artificial Sequence

<220>
<223> orf

<400> 245
Met Ile Met Ala Val Ala His Thr Thr Val Leu Ser Leu Leu Gly Lys
1               5                   10                  15
Glu Val Ser Phe Ser Val Leu Leu Asp Asp Gln Asn Lys Ile Phe Ser
            20                  25                  30
Pro Gly Gly Ile His Ile Thr Gly Leu Val Glu Lys Val Val Ile Asp
        35                  40                  45
Leu Thr Gly Asp His Ala Ile Leu Val Gly Asp Glu Phe Tyr Gln Leu
    50                  55                  60
Ser Glu Ile Asp Leu Pro Val
65                  70

<210> 246
<211> 117
<212> PRT
<213> Artificial Sequence

<220>
<223> orf

<400> 246
Met Ile Leu Pro Met Pro Glu Asn Thr Tyr Phe Trp Ala Phe Phe Cys
1               5                   10                  15
Pro Phe Leu Glu Lys Cys Val Ser Val His Ser Glu Lys Ile Asn Ile
            20                  25                  30

Glu Ile Gly Glu Glu Ile Arg Gly Ala Gly Arg Gly Val Gly Met Ser
        35                  40                  45
Leu Asp Val Pro Pro Cys Asp Trp Arg His Glu Ile Phe Asp Asn Leu
    50                  55                  60
Thr Leu Glu Thr Ile Ser Glu Gly Thr Glu Cys Ala Arg Thr Glu Glu
65                  70                  75                  80
Ile Lys Ile Lys Asn Tyr Leu Ser Leu Ile Thr Ser Ser Leu Leu Ile
                85                  90                  95
Phe Ser Gly Ile Asn Arg Ala Ile Arg Met Val Ala Thr Asp Tyr Tyr
            100                 105                 110
Phe Cys Phe Phe Thr
        115

<210> 247
<211> 51
<212> PRT
<213> Artificial Sequence

<220>
<223> orf

<400> 247
Met Lys Leu Thr Val Leu Val Asn Leu Leu Ile Leu Val Leu Leu Asn
1               5                   10                  15
His Tyr Leu Phe Val Tyr Leu Phe Lys Leu Thr Gln Lys Ser Leu Leu
            20                  25                  30
Leu Lys Thr Leu Leu Val Leu Lys Lys Leu Val Lys Ile Lys Leu Ser
        35                  40                  45
Met Ile Cys
    50

<210> 248
<211> 102
<212> PRT
<213> Artificial Sequence

<220>
<223> orf

<400> 248
Met Thr Asn Tyr His Asp Glu Pro Thr Lys Val Glu Met Leu Leu Ser
1               5                   10                  15
Glu Ile Asn Arg Thr Gly Lys Ser Ser Tyr Ser Gly Ala Leu Lys Pro
            20                  25                  30
Leu Ser Ile Arg Leu Pro Ile Gln Thr Tyr Ala Lys Val Val Ala Ile
        35                  40                  45
Glu Asn Phe Ile Gly Ala Glu Lys Thr Ser Lys Asn Lys Val Ile Asn
    50                  55                  60
Asp Leu Leu Glu Ile Ala Phe Asp Gln Ile Tyr Pro Ser Leu Asn Glu
65                  70                  75                  80
Ser Gln Lys His Ala Phe Asp His Phe Ser Gln Ser Leu Leu Asp Gly
                85                  90                  95
Leu Glu Ser Gly Lys Leu
            100

<210> 249
<211> 57
<212> PRT
<213> Artificial Sequence

<220>
<223> orf

<400> 249
Met Met Ile Gly Pro Gln Val Leu Ser Lys Gly Thr Met Asp Ser Leu
1               5                   10                  15
Glu Glu Ala Ser Ala Tyr Phe Lys Gln Val Leu Gln Glu His Leu Asp
            20                  25                  30
Asn Lys Arg Asp Ile Thr Leu Phe Ala Ile Ala Val Val Gly Gly Arg
        35                  40                  45
Leu Lys Val Val Glu Gly Leu Pro Lys
    50                  55

<210> 250
<211> 116
<212> PRT
<213> Artificial Sequence

<220>
<223> orf

<400> 250
Met Ile Cys Asp Cys Cys Cys Arg Arg Ser Phe Glu Ser Arg Arg Gly
1               5                   10                  15
Ile Thr Lys Met Ile Lys Leu Glu Gly Thr Ile Ile Asn Val Phe Thr
            20                  25                  30
Gln Gln Gly Gly Lys Asp Lys Lys Thr Gly Glu Ala Phe Ala Asp Arg
        35                  40                  45
Asp Lys Ile Gln Leu Leu Gly Glu Phe Glu Leu Pro Asn Gly Asp Lys
    50                  55                  60
Lys Asn Glu Leu Ile Asp Leu Thr Val Glu Asn Gly Lys Glu Tyr Glu
65                  70                  75                  80
Gly Met Lys Asn Lys Arg Val Ser Ile Pro Cys Gly Ala Leu Ala Ser
                85                  90                  95
Gly Arg Asn Val Ile Phe Tyr Val Ala Lys Gly Ala Thr Pro Lys Leu
            100                 105                 110
Leu Asn Thr Val
        115

<210> 251
<211> 424
<212> PRT
<213> Artificial Sequence

<220>
<223> orf

<400> 251
Met Thr Val Lys Asp Asn Lys Lys Ala His Ile Ser Asp Ala Leu Ala
1               5                   10                  15
Gly Asp Glu Ile Gln Ala Leu Gln Ser Ala Lys Ala Asp Gln His Arg
            20                  25                  30
Asp Arg Ile Thr Arg Phe Gly Ile Leu Lys His Arg Ala Lys Gln Gln
        35                  40                  45
Glu Asn Phe Leu Trp Thr Leu Ala Lys Phe Lys Glu Asn Tyr Pro Lys
    50                  55                  60
Asp Ala Pro Ser Glu Glu Ser Thr Arg Ala Ile Lys Ser Ala Gln Lys
65                  70                  75                  80
Leu Lys Glu Cys Gly Asn Phe Leu Leu Phe Lys Asn Phe Tyr Thr Ile
                85                  90                  95
Asp Gln Thr Lys Leu Ser Lys Phe His Val Cys Gly Gln His Leu Leu

            100                 105                 110
Cys Pro Phe Cys Ala Gly Ile Arg Ala Ser Lys Ala Ile Gln Lys Tyr
        115                 120                 125
Thr Glu Arg Val Asp Glu Val Leu Lys Lys Asn Arg Lys Leu Lys Pro
    130                 135                 140
Val Leu Ile Thr Phe Thr Val Lys Asn Gly Ile Asp Leu Gly Glu Arg
145                 150                 155                 160
Ser Ser His Leu Ile Lys Ser Phe Arg Thr Leu Leu Lys Arg Arg Arg
                165                 170                 175
Asp Tyr Ile Lys Lys Gly Arg Gly Phe Asn Glu Phe Cys Lys Ile Asn
            180                 185                 190
Gly Ala Val Tyr Ser Tyr Glu Asn Thr Tyr Asn Glu Gln Thr Lys Glu
        195                 200                 205
Trp His Pro His Leu His Met Phe Ala Leu Leu Asp Asp Trp Ile Asp
    210                 215                 220
Gln Glu Glu Leu Ser Gln Tyr Trp His Ser Ile Thr Gly Asp Ser Met
225                 230                 235                 240
Ile Val Asp Ile Arg Arg Val Lys Lys Glu Lys Gly Leu Gly Tyr Ser
                245                 250                 255
Lys Ala Ala Ala Glu Val Cys Lys Tyr Ala Leu Lys Phe Gly Asp Leu
            260                 265                 270
Ser Val Gln Asn Thr Trp Glu Ala Phe Lys Val Leu Lys Gly Lys Arg
        275                 280                 285
Leu Ser Gly Ala Phe Gly Ser Leu Trp Gly Val Lys Ile Pro Glu Asn
    290                 295                 300
Leu Ala Asp Asp Met Pro Asp Glu Lys Asp Leu Pro Tyr Leu Glu Met
305                 310                 315                 320
Leu Tyr Lys Phe Val Phe Gly Lys Gln Ser Tyr Tyr Asp Leu Thr Met
                325                 330                 335
Thr Arg His Val Glu Pro Gln Cys Lys Asp Arg Asp Asn Asp Glu Glu
            340                 345                 350
Glu Val Ala Thr Thr Asp Gly Gly Gly Asn Gly Arg Ser Ser Glu Ala
        355                 360                 365
Ile Lys Asp Gly Arg Gly Arg Ala Glu Arg Thr Glu Gly Thr Gln Gly
    370                 375                 380
Ala Arg His Arg Pro Gln His Gly Arg Lys Lys Gln His Trp Gln Val
385                 390                 395                 400
Pro Pro Val Thr Arg Val Arg Val Lys Lys Arg Ile Lys Arg Trp Asp
                405                 410                 415
Gly Tyr Leu Cys Val Met His Met
            420

<210> 252
<211> 51
<212> PRT
<213> Artificial Sequence

<220>
<223> orf

<400> 252
Met Leu Ser Arg Ser Ala Asn Ala Ser Pro Val Phe Leu Ser Leu Pro
1               5                   10                  15
Pro Cys Trp Val Lys Thr Leu Ile Ile Val Pro Ser Asn Leu Ile Ile
            20                  25                  30
Leu Val Ile Pro Leu Arg Leu Ser Asn Asp Arg Leu Gln Gln Gln Ser
        35                  40                  45
Gln Ile Met
    50

<210> 253
<211> 84

<212> PRT
<213> Artificial Sequence

<220>
<223> orf

<400> 253
Met Phe Ile Ser Ser Val Arg Ala His Ser Val Pro Ser Asp Ile Val
1               5                   10                  15
Ser Lys Val Arg Leu Ser Asn Ile Ser Cys Arg Gln Ser Gln Gly Gly
            20                  25                  30
Thr Ser Ser Asp Ile Pro Thr Pro Arg Pro Ala Pro Arg Ile Ser Ser
        35                  40                  45
Pro Ile Ser Met Phe Ile Phe Ser Glu Cys Thr Leu Thr His Phe Ser
    50                  55                  60
Arg Asn Gly Gln Lys Asn Ala Gln Lys Tyr Val Phe Ser Gly Ile Gly
65                  70                  75                  80
Lys Ile Val Asn

<210> 254
<211> 125
<212> PRT
<213> Artificial Sequence

<220>
<223> orf

<400> 254
Met Ser Arg Val Ala Ser His Arg Ala Gly His Arg Val Thr Ser Arg
1               5                   10                  15
Pro His Ala Gln Arg Pro Glu Ser Leu Arg Gln Ser Gln Cys Leu Phe
            20                  25                  30
Phe Gln Asn Ala Arg Leu Arg Ile Ser Gln Glu Thr Asp Lys Lys Met
        35                  40                  45
Pro Lys Asn Met Tyr Phe Arg Ala Leu Val Lys Ser Leu Thr Lys Gln
    50                  55                  60
Val Ser Leu Phe Arg Lys Val Asp Lys Thr His His Gln Leu Lys Leu
65                  70                  75                  80
His Asp His Gln Leu Asn Gln Leu Gln Leu Phe Gln Leu Ile Arg Leu
                85                  90                  95
Tyr Glu Tyr Arg Gln Gly Lys Arg Phe Tyr Phe Gly His Leu Ile Lys
            100                 105                 110
Gln Lys Met Lys Leu Leu Cys Gln Ala Lys Thr Lys Gln
        115                 120                 125

<210> 255
<211> 109
<212> PRT
<213> Artificial Sequence

<220>
<223> orf

<400> 255
Met Asp Cys Asn Pro Thr Ile Gln Arg Leu Glu Phe Tyr His Gly Phe
1               5                   10                  15
Met Ile Leu His Val His Asn Thr Gln Ile Thr Ile Pro Ser Phe Asn
            20                  25                  30
Pro Phe Phe His Ser Tyr Pro Arg Tyr Trp Arg Tyr Leu Pro Val Leu

35                  40                  45
Phe Phe Thr Ser Val Leu Arg Ala Val Pro Cys Thr Leu Arg Ala Leu
    50                  55                  60
Gly Ala Leu Arg Pro Ser Ser Pro Val Leu Asp Arg Leu Thr Ala Ala
65                  70                  75                  80
Ser Ile Ser Ala Ser Val Ser Arg Arg Tyr Leu Leu Phe Val Val Val
                85                  90                  95
Pro Val Leu Ala Leu Trp Leu Tyr Val Ser Cys His Ser
            100                 105

<210> 256
<211> 80
<212> PRT
<213> Artificial Sequence

<220>
<223> orf

<400> 256
Met Arg Phe Phe Thr Arg Thr Leu Val Thr Gly Gly Thr Cys Gln Cys
1               5                   10                  15
Cys Phe Leu Arg Pro Cys Cys Gly Arg Cys Arg Ala Pro Cys Val Pro
            20                  25                  30
Ser Val Arg Ser Ala Arg Pro Arg Pro Ser Leu Ile Ala Ser Leu Leu
        35                  40                  45
Arg Pro Phe Pro Pro Pro Ser Val Val Ala Thr Ser Ser Ser Ser Leu
    50                  55                  60
Ser Leu Ser Leu His Cys Gly Ser Thr Cys Arg Val Ile Val Lys Ser
65                  70                  75                  80

<210> 257
<211> 54
<212> PRT
<213> Artificial Sequence

<220>
<223> orf

<400> 257
Met Ser Ser Ala Arg Phe Ser Gly Ile Leu Thr Pro His Lys Glu Pro
1               5                   10                  15
Asn Ala Pro Leu Lys Arg Leu Pro Phe Lys Thr Leu Lys Ala Ser Gln
            20                  25                  30
Val Phe Trp Thr Asp Lys Ser Pro Asn Leu Arg Ala Tyr Leu His Thr
        35                  40                  45
Ser Ala Ala Ala Phe Glu
    50

<210> 258
<211> 50
<212> PRT
<213> Artificial Sequence

<220>
<223> orf

<400> 258
Met Thr Leu Arg Ile Ser Thr Ile Ile Glu Ser Pro Val Met Glu Cys

1               5                   10                  15
Gln Tyr Cys Glu Ser Ser Ser Trp Ser Ile Gln Ser Ser Lys Ser Ala
            20                  25                  30
Asn Ile Cys Lys Cys Gly Cys His Ser Phe Val Cys Ser Leu Tyr Val
        35                  40                  45
Phe Ser
    50

<210> 259
<211> 51
<212> PRT
<213> Artificial Sequence

<220>
<223> orf

<400> 259
Met Arg Met Pro Phe Leu Cys Leu Leu Val Ile Arg Val Phe Ile Arg
1               5                   10                  15
Ile His Ser Pro Ile Asp Leu Thr Glu Leu Ile Lys Pro Thr Thr Phe
            20                  25                  30
Phe Asn Ile Val Pro Thr Thr Leu Gln Lys Arg Ser Lys Arg Phe Tyr
        35                  40                  45
Glu Val Gly
    50

<210> 260
<211> 84
<212> PRT
<213> Artificial Sequence

<220>
<223> orf

<400> 260
Met Cys Arg Phe Asn Cys Ser Cys Ala Phe Phe Thr Trp Ser Ile Phe
1               5                   10                  15
Arg Ile Val Phe Phe Glu Phe Cys Gln Ser Pro Gln Lys Ile Phe Leu
            20                  25                  30
Leu Leu Arg Ser Met Phe Gln Asn Pro Lys Thr Cys Tyr Ser Ile Pro
        35                  40                  45
Val Leu Ile Cys Leu Ser Arg Leu Gln Ser Leu Tyr Phe Val Ala Arg
    50                  55                  60
Gln Ser Ile Arg Asn Met Gly Phe Phe Ile Val Phe Tyr Ser Gln Phe
65                  70                  75                  80
Leu Ser Pro Leu

<210> 261
<211> 1170
<212> DNA
<213> Artificial Sequence

<220>
<223> orf

<400> 261
ataacacgtt ttgggatttt gaaacataga gcgaagcaac aagaaaattt tttgtggact      60

ctggcaaaat tcaaagaaaa ctatcctaaa gatgctccaa gtgaagaaag cacaagagca     120

attaaatctg cacagaaatt aaaagagtgc ggaaatttct tactattcaa aaatttttac 180

acaatagacc aaactaagct ctcaaaattt catgtatgtg ggcagcattt gttatgtcct 240

ttctgtgctg gtattcgtgc atctaaagca attcaaaaat atactgaacg tgttgatgaa 300

gtgcttaaga aaaatcgcaa gcttaagcct gttttaatca cgtttacggt gaagaatgga 360

atcgacttag gggaaagatc atcccacctc ataaaatcgt ttagaacgct tctgaagcgt 420

cgtagggact atattaaaaa aggtcgtggg tttaatgagt tctgtaagat caatggggct 480

gtgtattctt atgaaaacac gtataacgag caaacaaagg aatggcatcc gcacttacat 540

atgtttgcac ttttggatga ttggatagac caggaagagc tttcacagta ttggcattcc 600

atcactggtg attcaatgat tgtagatatt cgcagggtca aaaaagaaaa aggcttaggc 660

tattcaaagg ctgctgcgga agtatgtaaa tatgctctta aattcggtga tttgtctgtc 720

caaaatactt gggaagcttt caaagtctta aaaggtaagc gtttaagtgg tgcatttggt 780

tctttatggg gtgtcaaaat tcctgaaaat cttgctgatg atatgcctga tgaaaaagat 840

cttccttact tagaaatgct ctataaattc gtatttggaa aacagtctta ctatgactta 900

actatgacac gacacgtaga gccacaatgc aaggacaggg acaacgacga agaggaggta 960

gcgacgactg acggaggcgg aaatggacgc agcagtgagg cgatcaagga cgggcgagga 1020

cgggcggagc gcaccgaggg cacgcagggt gcacggcacc gcccgcagca cggacgtaaa 1080

aaacaacact ggcaagtacc tccagtaacg agggtacgag tgaaaaaacg gattaaaaga 1140

tgggatggtt atttgtgtgt tatgcacatg 1170

<210> 262
<211> 213
<212> DNA
<213> Artificial Sequence

<220>
<223> orf

<400> 262
atcatcatgg ctgttgcaca tactactgtt ttgtctttgc ttggcaaaga agtttcattt 60

tctgttttat tagatgacca aaataaaatc ttttcccctg gcggtattca tataaccgga 120

ttagttgaaa aagttgtaat tgatttaact ggtgatcatg caattttagt tggtgatgag 180

ttttatcaac tttccgaaat agacttacct gtt 213

<210> 263
<211> 351
<212> DNA
<213> Artificial Sequence

<220>
<223> orf

<400> 263
acgattttac caatgcccga aaatacatat ttttgggcat ttttttgtcc gtttcttgag 60

aaatgcgtaa gcgtgcattc tgaaaaaata aacattgaga ttggcgaaga gattcggggc 120

gctgggcgtg gggtcgggat gtcactcgat gtcccgccct gtgactggcg acacgagata 180

ttcgacaatc tcactttaga aacaatatca gagggcacgg agtgtgcccg aactgaagaa 240

ataaaaatta aaaactacct ttccctgatt acaagctccc ttttgatttt ctccgggata 300

aacagagcca tccggatggt ggcgactgac tattactttt gcttttttac t 351


<210> 264
<211> 153
<212> DNA
<213> Artificial Sequence


<220>
<223> orf

<400> 264
ctgaaattaa ccgtactggt aaatcttctt attctggtgc tcttaaacca ttatctattc 60

gtttacctat tcaaacttac gcaaaagtcg ttgctattga aaactttatt ggtgctgaaa 120

aaactagtaa aaataaagtt atcaatgatt tgt 153


<210> 265
<211> 306
<212> DNA
<213> Artificial Sequence


<220>
<223> orf

<400> 265
atgacaaatt atcatgatga accaactaaa gtagaaatgc ttctttctga aattaaccgt 60

actggtaaat cttcttattc tggtgctctt aaaccattat ctattcgttt acctattcaa 120

acttacgcaa aagtcgttgc tattgaaaac tttattggtg ctgaaaaaac tagtaaaaat 180

aaagttatca atgatttgtt agaaattgct tttgatcaga tttatccatc tttaaatgaa 240

tctcaaaaac atgcatttga tcatttttca cagtctcttt tagatggttt ggagtcagga 300

aaatta 306


<210> 266
<211> 171
<212> DNA
<213> Artificial Sequence

<220>
<223> orf

<400> 266
attatgattg gacctcaagt tctttcaaag ggaacaatgg attccctgga agaagcatct      60

gcttatttca agcaagtttt acaagaacat cttgataaca aacgtgacat cacattattt     120

gcgattgctg ttgtaggcgg tcgtttgaaa gtcgtagagg gattaccaaa a              171


<210> 267
<211> 348
<212> DNA
<213> Artificial Sequence


<220>
<223> orf

<400> 267
attatttgcg attgctgttg taggcggtcg tttgaaagtc gtagagggat taccaaaatg      60

attaaattag aaggcacaat tattaatgtt tttacccagc aaggcggtaa agacaaaaaa     120

actggtgaag catttgccga tcgtgacaag attcaattgt taggtgagtt tgaactccct     180

aacggtgata aaaaaaatga attaattgat cttactgttg aaaacggtaa ggaatatgaa     240

ggtatgaaaa ataagcgtgt gagcattcca tgtggtgctc ttgcttcggg tcgaaatgtg     300

attttttatg tcgcaaaagg cgcaacaccc aaacttttaa acacagtt                 348


<210> 268
<211> 1272
<212> DNA
<213> Artificial Sequence


<220>
<223> orf

<400> 268
ttgactgtaa aagacaataa aaaagcccat atttctgatg ctttggcggg cgacgaaata      60

caggctttgc aatctgctaa ggcagatcaa cacagggata gaataacacg ttttgggatt     120

ttgaaacata gagcgaagca acaagaaaat tttttgtgga ctctggcaaa attcaaagaa     180

aactatccta aagatgctcc aagtgaagaa agcacaagag caattaaatc tgcacagaaa     240

ttaaaagagt gcggaaattt cttactattc aaaaattttt acacaataga ccaaactaag     300

ctctcaaaat ttcatgtatg tgggcagcat ttgttatgtc ctttctgtgc tggtattcgt     360

gcatctaaag caattcaaaa atatactgaa cgtgttgatg aagtgcttaa gaaaaatcgc     420

aagcttaagc ctgttttaat cacgtttacg gtgaagaatg gaatcgactt aggggaaaga     480

tcatcccacc tcataaaatc gtttagaacg cttctgaagc gtcgtaggga ctatattaaa     540

aaaggtcgtg ggtttaatga gttctgtaag atcaatgggg ctgtgtattc ttatgaaaac     600

acgtataacg agcaaacaaa ggaatggcat ccgcacttac atatgtttgc acttttggat 660

gattggatag accaggaaga gctttcacag tattggcatt ccatcactgg tgattcaatg 720

attgtagata ttcgcagggt caaaaaagaa aaaggcttag gctattcaaa ggctgctgcg 780

gaagtatgta aatatgctct taaattcggt gatttgtctg tccaaaatac ttgggaagct 840

ttcaaagtct taaaaggtaa gcgtttaagt ggtgcatttg gttctttatg gggtgtcaaa 900

attcctgaaa atcttgctga tgatatgcct gatgaaaaag atcttcctta cttagaaatg 960

ctctataaat tcgtatttgg aaaacagtct tactatgact taactatgac acgacacgta 1020

gagccacaat gcaaggacag ggacaacgac gaagaggagg tagcgacgac tgacggaggc 1080

ggaaatggac gcagcagtga ggcgatcaag gacgggcgag gacgggcgga gcgcaccgag 1140

ggcacgcagg gtgcacggca ccgcccgcag cacggacgta aaaaacaaca ctggcaagta 1200

cctccagtaa cgagggtacg agtgaaaaaa cggattaaaa gatgggatgg ttatttgtgt 1260

gttatgcaca tg 1272

<210> 269
<211> 152
<212> DNA
<213> Artificial Sequence

<220>
<223> orf

<400> 269
tcttgtcacg atcggcaaat gcttcaccag tttttttgtc tttaccgcct tgctgggtaa 60

aaacattaat aattgtgcct tctaatttaa tcattttggt aatccctcta cgactttcaa 120

acgaccgcct acaacagcaa tcgcaaataa tg 152

<210> 270
<211> 252
<212> DNA
<213> Artificial Sequence

<220>
<223> orf

<400> 270
atttttattt cttcagttcg ggcacactcc gtgccctctg atattgtttc taaagtgaga 60

ttgtcgaata tctcgtgtcg ccagtcacag ggcgggacat cgagtgacat cccgacccca 120

cgcccagcgc cccgaatctc ttcgccaatc tcaatgttta ttttttcaga atgcacgctt 180

acgcatttct caagaaacgg acaaaaaaat gcccaaaaat atgtattttc gggcattggt 240

aaaatcgtta ac 252

<210> 271
<211> 375
<212> DNA
<213> Artificial Sequence

<220>
<223> orf

<400> 271
atatctcgtg tcgccagtca cagggcggga catcgagtga catcccgacc ccacgcccag 60

cgccccgaat ctcttcgcca atctcaatgt ttattttttc agaatgcacg cttacgcatt 120

tctcaagaaa cggacaaaaa aatgcccaaa aatatgtatt ttcgggcatt ggtaaaatcg 180

ttaactaaac aggtaagtct atttcggaaa gttgataaaa ctcatcacca actaaaattg 240

catgatcacc agttaaatca attacaactt tttcaactaa tccggttata tgaataccgc 300

caggggaaaa gattttattt tggtcatcta ataaaacaga aaatgaaact tctttgccaa 360

gcaaagacaa aacag 375

<210> 272
<211> 327
<212> DNA
<213> Artificial Sequence

<220>
<223> orf

<400> 272
ttggactgca atccgactat tcagcgacta gaattttatc atgggtttat gattttacat 60

gtgcataaca cacaaataac catcccatct tttaatccgt tttttcactc gtaccctcgt 120

tactggaggt acttgccagt gttgtttttt acgtccgtgc tgcgggcggt gccgtgcacc 180

ctgcgtgccc tcggtgcgct ccgcccgtcc tcgcccgtcc ttgatcgcct cactgctgcg 240

tccatttccg cctccgtcag tcgtcgctac ctcctcttcg tcgttgtccc tgtccttgca 300

ttgtggctct acgtgtcgtg tcatagt 327

<210> 273
<211> 240
<212> DNA
<213> Artificial Sequence

<220>
<223> orf

<400> 273
atccgttttt tcactcgtac cctcgttact ggaggtactt gccagtgttg ttttttacgt 60

ccgtgctgcg ggcggtgccg tgcaccctgc gtgccctcgg tgcgctccgc ccgtcctcgc 120

ccgtccttga tcgcctcact gctgcgtcca tttccgcctc cgtcagtcgt cgctacctcc 180

tcttcgtcgt tgtccctgtc cttgcattgt ggctctacgt gtcgtgtcat agttaagtca 240

<210> 274
<211> 162
<212> DNA
<213> Artificial Sequence

<220>
<223> orf

<400> 274
atatcatcag caagattttc aggaattttg acaccccata aagaaccaaa tgcaccactt 60

aaacgcttac cttttaagac tttgaaagct tcccaagtat tttggacaga caaatcaccg 120

aatttaagag catatttaca tacttccgca gcagcctttg aa 162

<210> 275
<211> 150
<212> DNA
<213> Artificial Sequence

<220>
<223> orf

<400> 275
ttgaccctgc gaatatctac aatcattgaa tcaccagtga tggaatgcca atactgtgaa 60

agctcttcct ggtctatcca atcatccaaa agtgcaaaca tatgtaagtg cggatgccat 120

tcctttgttt gctcgttata cgtgttttca 150

<210> 276
<211> 153
<212> DNA
<213> Artificial Sequence

<220>
<223> orf

<400> 276
gtgcggatgc cattcctttg tttgctcgtt atacgtgttt tcataagaat acacagcccc 60

attgatctta cagaactcat taaacccacg acctttttta atatagtccc tacgacgctt 120

cagaagcgtt ctaaacgatt ttatgaggtg gga 153

<210> 277
<211> 252
<212> DNA
<213> Artificial Sequence

<220>

<223> orf

<400> 277
ctgtgcagat ttaattgctc ttgtgctttc ttcacttgga gcatctttag gatagttttc 60

tttgaatttt gccagagtcc acaaaaaatt ttcttgttgc ttcgctctat gtttcaaaat 120

cccaaaacgt gttattctat ccctgtgttg atctgcctta gcagattgca aagcctgtat 180

ttcgtcgccc gccaaagcat cagaaatatg ggctttttta ttgtctttta cagtcaattt 240

ttgtccccac tt 252

<210> 278
<211> 3090
<212> DNA
<213> Artificial Sequence

<220>
<223> isolate

<400> 278
caacacaggg atagaataac acgttttgcg actttgaaac atagggctaa gaatcaggaa 60

aattatttat ttaccctggc taagtttaaa gagaactatg aaaaagacgt taaaaacgaa 120

gaatctatca aggctttaaa atctgctcat aaattgaatg aatgcggaaa ctatctgcta 180

ttcaaaaatt tttacacaat aggtgaagtt aaactctcca agctccgcac ctgcggacag 240

catttgctct gccctttctg tgctgctatt cgtgcttctc gtgctatcca aaaatacgtt 300

gaacgtattg atcaggtgtt gcaagaaaat cgcaagctca agcccgttct aatcactctt 360

accgttaaaa acggttctga cctagcggaa cgctctgaac atcttatgaa gtcctttagg 420

acgcttttag atcgtcgtag ggactatgaa aagaaagggc gaggttttaa cgagttctgt 480

aaggttcaag gggctatgta ctcttacgag agtacataca acgaaaaaac agttgaatgg 540

catccgcata tccatatgtt tgctttggtt gatcagtgga ttgatcagca agaattttca 600

gaatattggc atagccttac tggggactcg atggttgtcg atgttcgcag ggcaagaaaa 660

gaaaaaggac acggctatag caaagcggct gccgaagttt gtaagtatgc tttaaagttt 720

ggtgatctat ccgttgaaaa cacttgggaa gcctttaaag ttctcaaggg caagcgttta 780

acaggttctt ttggtctgtt atggggtgtc aaaattcctg aaaacttagc agatgaaatt 840

ccagatgatc aggacttgcc atatcttgaa atgctgtaca agttcgttta cggcaaaaat 900

tcacattatg acttagctat gactcgtatg gttgagccac aagcgaagat tgagcgaatg 960

agtgaggagg aggcaacgac cgaccgacac gacatgagag aaatagagcg agtggcgata 1020

aaggacgatg tgaggacggt ggcaggtggg gcaccactgc gctgcccgca acgaggacgc 1080

aaaaagcaac attggcaagt atcaccttac gtccgtgtta ggacacggac tagaatccgt 1140

caatggacgg ggttcttata caacatagat ttattccctt atgtagaaaa acgattatat 1200

cgctatttag ttgttgatta attgccattt agttgttata atggttttat caactggagt 1260

tcatgctatg actattcaat caagattaaa caaagatact gttcagctaa ttaagattgc 1320

tcaacgtata gaatttatta gaaaacaaga tgaaatgggc aaccaaaaag ttatagctat 1380

gatttatata tcagaagatt ctaaccctat tcgagcgatg gctggtgatg ttacttggga 1440

cgctcatgac attgataagg ctaaacgctc tattcgtcgt cataataaaa cttgtttaat 1500

tattgataga cgagatcaag ttatttaatc gctggatgat tgagttacga cctaaaccat 1560

ccagctaacc acaacgatct atttcggaga tcatcatggc tgtgccacat actactgttt 1620

tgtctttgct tggcaaagaa gtttcatttt ctgtattact agatgaagaa ataaaaatct 1680

tttttcctca aggcattcac ataaatggat gcgtccaaga agtaatcata gctctcaatg 1740

gtaatcacca aattttggtt gatgacgaat tttatgaatt atcacgtatt gacttatttt 1800

cttaaattta cccgataggg gagaccctga atcctttatt cataaaggtt tcagggtttt 1860

tttatccttt ttacataatc tgtcttatag gaactctcct actaaacatt acttatataa 1920

tctttagtta ctggagacat tctctccagg aacagagggc acggagtgtg cccgaactga 1980

cgcaataaaa atctaaaaac ctccctctct gattacaagc tcccctctgc attctcacag 2040

taagtgccga gccatcccga atgggtggcg actcactatt gatttgatat aaaatcggtg 2100

tataatttat tcttaaatta tgagtaaatc atttattgaa aggtacatag tatgccaaat 2160

gatagaattt cacctttaga ggttgtttta ggaaatattc ttggtgagga atcaccaagt 2220

agaccagacc aaaaagcagt ttctttccga attgattttg ataactacgt caatctttgc 2280

gctctgcaaa gcattactcc gaaaggagtt agtcggaatc agcttttaaa tgatcttcta 2340

gctgttgcta ttgatcaggt ttcatctgct ttacctgatg gtgtttctga tgcttatgaa 2400

gaatctgtcg ttcaccatga acaagggttg aacgaacttt tagcaattga gggccattta 2460

tgattggccc tcaagttatt agcaagggaa ccatggattc ccttgaagaa gcatcagctt 2520

atttcaaaca ggtcttacaa gtgcacttgg ataacaagcg cgatatcaca ttgtttgcga 2580

ttaccgttgt tggcggtcgc cttaaggtag tagagggttt accaaaatga tcaaactaga 2640

aggcacaatt atcaatgtat tcacccagca gggtggcacg agcaagaaaa caggcgaagc 2700

gtttgcggat cgtgacaaga ttcagctaat gggagaactg gaactcccaa acggttccat 2760

aaaacatgaa ttgattgacc ttaccgttga aaatggcaag gaatatgaaa ccctaaaagg 2820

caagcgtgtc agcatcccat gtggtgctct cgcttcaggt cgtaaagtga ttttttatgt 2880

tgcgaaaggt ggaacaccta aacttttgac agcagtatga tactcgttaa aaaacagtga 2940

gttagcgaag cggctcgctg tttttctttt tacttgatac tattgattaa agtggggaca 3000

aaattgctta aaaaagacaa taaaaaagcc catatttcga acactttggc gggtggagaa 3060

atacaggctt tgcaatctgc taaggcagat                                    3090

<210> 279
<211> 94
<212> PRT
<213> Artificial Sequence

<220>
<223> orf

<400> 279
Met Val Val Gly Thr Met Lys Arg Lys Gly Glu Val Leu Thr Ser Ser
1               5                   10                  15
Val Arg Phe Lys Gly Leu Cys Thr Leu Thr Arg Val His Thr Thr Lys
            20                  25                  30
Lys Gln Leu Asn Gly Ile Arg Ile Ser Ile Cys Leu Leu Trp Leu Ile
        35                  40                  45
Ser Gly Leu Ile Ser Lys Asn Phe Gln Asn Ile Gly Ile Ala Leu Leu
    50                  55                  60
Gly Thr Arg Trp Leu Ser Met Phe Ala Gly Gln Glu Lys Lys Lys Asp
65                  70                  75                  80
Thr Ala Ile Ala Lys Arg Leu Pro Lys Phe Val Ser Met Leu
                85                  90

<210> 280
<211> 401
<212> PRT
<213> Artificial Sequence

<220>
<223> orf

<400> 280
Met Thr Arg Phe Ala Thr Leu Lys His Arg Ala Lys Asn Gln Glu Asn
1               5                   10                  15
Tyr Leu Phe Thr Leu Ala Lys Phe Lys Glu Asn Tyr Glu Lys Asp Val
            20                  25                  30
Lys Asn Glu Glu Ser Ile Lys Ala Leu Lys Ser Ala His Lys Leu Asn
        35                  40                  45
Glu Cys Gly Asn Tyr Leu Leu Phe Lys Asn Phe Tyr Thr Ile Gly Glu
    50                  55                  60
Val Lys Leu Ser Lys Leu Arg Thr Cys Gly Gln His Leu Leu Cys Pro
65                  70                  75                  80
Phe Cys Ala Ala Ile Arg Ala Ser Arg Ala Ile Gln Lys Tyr Val Glu
                85                  90                  95
Arg Ile Asp Gln Val Leu Gln Glu Asn Arg Lys Leu Lys Pro Val Leu
            100                 105                 110
Ile Thr Leu Thr Val Lys Asn Gly Ser Asp Leu Ala Glu Arg Ser Glu
        115                 120                 125
His Leu Met Lys Ser Phe Arg Thr Leu Leu Asp Arg Arg Arg Asp Tyr
    130                 135                 140
Glu Lys Lys Gly Arg Gly Phe Asn Glu Phe Cys Lys Val Gln Gly Ala
145                 150                 155                 160
Met Tyr Ser Tyr Glu Ser Thr Tyr Asn Glu Lys Thr Val Glu Trp His
                165                 170                 175
Pro His Ile His Met Phe Ala Leu Val Asp Gln Trp Ile Asp Gln Gln
            180                 185                 190
Glu Phe Ser Glu Tyr Trp His Ser Leu Thr Gly Asp Ser Met Val Val
        195                 200                 205
Asp Val Arg Arg Ala Arg Lys Glu Lys Gly His Gly Tyr Ser Lys Ala

210                 215                 220
Ala Ala Glu Val Cys Lys Tyr Ala Leu Lys Phe Gly Asp Leu Ser Val
225                 230                 235                 240
Glu Asn Thr Trp Glu Ala Phe Lys Val Leu Lys Gly Lys Arg Leu Thr
                245                 250                 255
Gly Ser Phe Gly Leu Leu Trp Gly Val Lys Ile Pro Glu Asn Leu Ala
            260                 265                 270
Asp Glu Ile Pro Asp Asp Gln Asp Leu Pro Tyr Leu Glu Met Leu Tyr
        275                 280                 285
Lys Phe Val Tyr Gly Lys Asn Ser His Tyr Asp Leu Ala Met Thr Arg
    290                 295                 300
Met Val Glu Pro Gln Ala Lys Ile Glu Arg Met Ser Glu Glu Glu Ala
305                 310                 315                 320
Thr Thr Asp Arg His Asp Met Arg Glu Ile Glu Arg Val Ala Ile Lys
                325                 330                 335
Asp Asp Val Arg Thr Val Ala Gly Gly Ala Pro Leu Arg Cys Pro Gln
            340                 345                 350
Arg Gly Arg Lys Lys Gln His Trp Gln Val Ser Pro Tyr Val Arg Val
        355                 360                 365
Arg Thr Arg Thr Arg Ile Arg Gln Trp Asp Gly Phe Leu Tyr Asn Ile
    370                 375                 380
Asp Leu Phe Pro Tyr Val Glu Lys Arg Leu Tyr Arg Tyr Leu Val Val
385                 390                 395                 400
Asp

<210> 281
<211> 105
<212> PRT
<213> Artificial Sequence

<220>
<223> orf

<400> 281
Met Leu Ile Asn Cys His Leu Val Val Ile Met Val Leu Ser Thr Gly
1               5                   10                  15
Val His Ala Met Thr Ile Gln Ser Arg Leu Asn Lys Asp Thr Val Gln
            20                  25                  30
Leu Ile Lys Ile Ala Gln Arg Ile Glu Phe Ile Arg Lys Gln Asp Glu
        35                  40                  45
Met Gly Asn Gln Lys Val Ile Ala Met Ile Tyr Ile Ser Glu Asp Ser
    50                  55                  60
Asn Pro Ile Arg Ala Met Ala Gly Asp Val Thr Trp Asp Ala His Asp
65                  70                  75                  80
Ile Asp Lys Ala Lys Arg Ser Ile Arg Arg His Asn Lys Thr Cys Leu
                85                  90                  95
Ile Ile Asp Arg Arg Asp Gln Val Ile
            100                 105

<210> 282
<211> 74
<212> PRT
<213> Artificial Sequence

<220>
<223> orf

<400> 282
Met Ser Glu Ile Ile Met Ala Val Pro His Thr Thr Val Leu Ser Leu
1               5                   10                  15

Leu Gly Lys Glu Val Ser Phe Ser Val Leu Leu Asp Glu Glu Ile Lys
            20                  25                  30
Ile Phe Phe Pro Gln Gly Ile His Ile Asn Gly Cys Val Gln Glu Val
        35                  40                  45
Ile Ile Ala Leu Asn Gly Asn His Gln Ile Leu Val Asp Asp Glu Phe
    50                  55                  60
Tyr Glu Leu Ser Arg Ile Asp Leu Phe Ser
65                  70

<210> 283
<211> 60
<212> PRT
<213> Artificial Sequence

<220>
<223> orf

<400> 283
Met Tyr Pro Ile Gly Glu Thr Leu Asn Pro Leu Phe Ile Lys Val Ser
1               5                   10                  15
Gly Phe Phe Tyr Pro Phe Tyr Ile Ile Cys Leu Ile Gly Thr Leu Leu
            20                  25                  30
Leu Asn Ile Thr Tyr Ile Ile Phe Ser Tyr Trp Arg His Ser Leu Gln
        35                  40                  45
Glu Gln Arg Ala Arg Ser Val Pro Glu Leu Thr Gln
    50                  55                  60

<210> 284
<211> 103
<212> PRT
<213> Artificial Sequence

<220>
<223> orf

<400> 284
Met Pro Asn Asp Arg Ile Ser Pro Leu Glu Val Val Leu Gly Asn Ile
1               5                   10                  15
Leu Gly Glu Glu Ser Pro Ser Arg Pro Asp Gln Lys Ala Val Ser Phe
            20                  25                  30
Arg Ile Asp Phe Asp Asn Tyr Val Asn Leu Cys Ala Leu Gln Ser Ile
        35                  40                  45
Thr Pro Lys Gly Val Ser Arg Asn Gln Leu Leu Asn Asp Leu Leu Ala
    50                  55                  60
Val Ala Ile Asp Gln Val Ser Ser Ala Leu Pro Asp Gly Val Ser Asp
65                  70                  75                  80
Ala Tyr Glu Glu Ser Val Val His His Glu Gln Gly Leu Asn Glu Leu
                85                  90                  95
Leu Ala Ile Glu Gly His Leu
            100

<210> 285
<211> 56
<212> PRT
<213> Artificial Sequence

<220>
<223> orf

<400> 285

Met Ile Gly Pro Gln Val Ile Ser Lys Gly Thr Met Asp Ser Leu Glu
1               5                   10                  15
Glu Ala Ser Ala Tyr Phe Lys Gln Val Leu Gln Val His Leu Asp Asn
            20                  25                  30
Lys Arg Asp Ile Thr Leu Phe Ala Ile Thr Val Val Gly Gly Arg Leu
        35                  40                  45
Lys Val Val Glu Gly Leu Pro Lys
    50                  55

<210> 286
<211> 97
<212> PRT
<213> Artificial Sequence

<220>
<223> orf

<400> 286
Met Ile Lys Leu Glu Gly Thr Ile Ile Asn Val Phe Thr Gln Gln Gly
1               5                   10                  15
Gly Thr Ser Lys Lys Thr Gly Glu Ala Phe Ala Asp Arg Asp Lys Ile
            20                  25                  30
Gln Leu Met Gly Glu Leu Glu Leu Pro Asn Gly Ser Ile Lys His Glu
        35                  40                  45
Leu Ile Asp Leu Thr Val Glu Asn Gly Lys Glu Tyr Glu Thr Leu Lys
    50                  55                  60
Gly Lys Arg Val Ser Ile Pro Cys Gly Ala Leu Ala Ser Gly Arg Lys
65                  70                  75                  80
Val Ile Phe Tyr Val Ala Lys Gly Gly Thr Pro Lys Leu Leu Thr Ala
                85                  90                  95
Val

<210> 287
<211> 60
<212> PRT
<213> Artificial Sequence

<220>
<223> orf

<400> 287
Met Asn Tyr Thr Pro Ile Leu Tyr Gln Ile Asn Ser Glu Ser Pro Pro
1               5                   10                  15
Ile Arg Asp Gly Ser Ala Leu Thr Val Arg Met Gln Arg Gly Ala Cys
            20                  25                  30
Asn Gln Arg Gly Arg Phe Leu Asp Phe Tyr Cys Val Ser Ser Gly Thr
        35                  40                  45
Leu Arg Ala Leu Cys Ser Trp Arg Glu Cys Leu Gln
    50                  55                  60

<210> 288
<211> 62
<212> PRT
<213> Artificial Sequence

<220>
<223> orf

<400> 288

Met Ile Leu Ala Asn Val Ala Phe Cys Val Leu Val Ala Gly Ser Ala
1               5                   10                  15
Val Val Pro His Leu Pro Pro Ser Ser His Arg Pro Leu Ser Pro Leu
            20                  25                  30
Ala Leu Phe Leu Ser Cys Arg Val Gly Arg Ser Leu Pro Pro Pro His
        35                  40                  45
Ser Phe Ala Gln Ser Ser Leu Val Ala Gln Pro Tyr Glu Ser
    50                  55                  60

<210> 289
<211> 61
<212> PRT
<213> Artificial Sequence

<220>
<223> orf

<400> 289
Met Pro Met Leu Leu Phe Ala Ser Ser Leu Arg Ala Ala Gln Trp Cys
1               5                   10                  15
Pro Thr Cys His Arg Pro His Ile Val Leu Tyr Arg His Ser Leu Tyr
            20                  25                  30
Phe Ser His Val Val Ser Val Gly Arg Cys Leu Leu Leu Thr His Ser
        35                  40                  45
Leu Asn Leu Arg Leu Trp Leu Asn His Thr Ser His Ser
    50                  55                  60

<210> 290
<211> 58
<212> PRT
<213> Artificial Sequence

<220>
<223> orf

<400> 290
Met Arg Pro Arg Cys Gly Gln Arg Ser Gly Ala Pro Pro Ala Thr Val
1               5                   10                  15
Leu Thr Ser Ser Phe Ile Ala Thr Arg Ser Ile Ser Leu Met Ser Cys
            20                  25                  30
Arg Ser Val Val Ala Ser Ser Ser Leu Ile Arg Ser Ile Phe Ala Cys
        35                  40                  45
Gly Ser Thr Ile Arg Val Ile Ala Lys Ser
    50                  55

<210> 291
<211> 54
<212> PRT
<213> Artificial Sequence

<220>
<223> orf

<400> 291
Met Ser Ser Ala Lys Phe Ser Gly Ile Leu Thr Pro His Asn Arg Pro
1               5                   10                  15
Lys Glu Pro Val Lys Arg Leu Pro Leu Arg Thr Leu Lys Ala Ser Gln
            20                  25                  30
Val Phe Ser Thr Asp Arg Ser Pro Asn Phe Lys Ala Tyr Leu Gln Thr
        35                  40                  45

Ser Ala Ala Ala Leu Leu
    50

<210> 292
<211> 54
<212> PRT
<213> Artificial Sequence

<220>
<223> orf

<400> 292
Met Leu Ile Asn Pro Leu Ile Asn Gln Ser Lys His Met Asp Met Arg
1               5                   10                  15
Met Pro Phe Asn Cys Phe Phe Val Val Cys Thr Leu Val Arg Val His
            20                  25                  30
Ser Pro Leu Asn Leu Thr Glu Leu Val Lys Thr Ser Pro Phe Leu Phe
        35                  40                  45
Ile Val Pro Thr Thr Ile
    50

<210> 293
<211> 50
<212> PRT
<213> Artificial Sequence

<220>
<223> orf

<400> 293
Met Phe Leu Gln His Leu Ile Asn Thr Phe Asn Val Phe Leu Asp Ser
1               5                   10                  15
Thr Arg Ser Thr Asn Ser Ser Thr Glu Arg Ala Glu Gln Met Leu Ser
            20                  25                  30
Ala Gly Ala Glu Leu Gly Glu Phe Asn Phe Thr Tyr Cys Val Lys Ile
        35                  40                  45
Phe Glu
    50

<210> 294
<211> 58
<212> PRT
<213> Artificial Sequence

<220>
<223> orf

<400> 294
Met Ile Phe Leu Ile Leu Ser Pro Met Phe Gln Ser Arg Lys Thr Cys
1               5                   10                  15
Tyr Ser Ile Pro Val Leu Ile Cys Leu Ser Arg Leu Gln Ser Leu Tyr
            20                  25                  30
Phe Ser Thr Arg Gln Ser Val Arg Asn Met Gly Phe Phe Ile Val Phe
        35                  40                  45
Phe Lys Gln Phe Cys Pro His Phe Asn Gln
    50                  55

<210> 295
<211> 282
<212> DNA

<213> Artificial Sequence

<220>
<223> orf

<400> 295
atcgtcgtag ggactatgaa aagaaagggc gaggttttaa cgagttctgt aaggttcaag      60

gggctatgta ctcttacgag agtacataca acgaaaaaac agttgaatgg catccgcata     120

tccatatgtt tgctttggtt gatcagtgga ttgatcagca agaattttca gaatattggc     180

atagccttac tggggactcg atggttgtcg atgttcgcag ggcaagaaaa gaaaaaggac     240

acggctatag caaagcggct gccgaagttt gtaagtatgc tt                        282

<210> 296
<211> 1203
<212> DNA
<213> Artificial Sequence

<220>
<223> orf

<400> 296
ataacacgtt ttgcgacttt gaaacatagg gctaagaatc aggaaaatta tttatttacc      60

ctggctaagt ttaaagagaa ctatgaaaaa gacgttaaaa acgaagaatc tatcaaggct     120

ttaaaatctg ctcataaatt gaatgaatgc ggaaactatc tgctattcaa aaatttttac     180

acaataggtg aagttaaact ctccaagctc cgcacctgcg gacagcattt gctctgccct     240

ttctgtgctg ctattcgtgc ttctcgtgct atccaaaaat acgttgaacg tattgatcag     300

gtgttgcaag aaaatcgcaa gctcaagccc gttctaatca ctcttaccgt taaaaacggt     360

tctgacctag cggaacgctc tgaacatctt atgaagtcct ttaggacgct tttagatcgt     420

cgtagggact atgaaaagaa agggcgaggt tttaacgagt tctgtaaggt tcaaggggct     480

atgtactctt acgagagtac atacaacgaa aaaacagttg aatggcatcc gcatatccat     540

atgtttgctt tggttgatca gtggattgat cagcaagaat tttcagaata ttggcatagc     600

cttactgggg actcgatggt tgtcgatgtt cgcagggcaa gaaaagaaaa aggacacggc     660

tatagcaaag cggctgccga agtttgtaag tatgctttaa agtttggtga tctatccgtt     720

gaaaacactt gggaagcctt taaagttctc aagggcaagc gtttaacagg ttcttttggt     780

ctgttatggg gtgtcaaaat tcctgaaaac ttagcagatg aaattccaga tgatcaggac     840

ttgccatatc ttgaaatgct gtacaagttc gtttacggca aaaattcaca ttatgactta     900

gctatgactc gtatggttga gccacaagcg aagattgagc gaatgagtga ggaggaggca     960

acgaccgacc gacacgacat gagagaaata gagcgagtgg cgataaagga cgatgtgagg    1020

acggtggcag gtggggcacc actgcgctgc ccgcaacgag gacgcaaaaa gcaacattgg    1080

caagtatcac cttacgtccg tgttaggaca cggactagaa tccgtcaatg ggacgggttc 1140

ttatacaaca tagatttatt cccttatgta gaaaaacgat tatatcgcta tttagttgtt 1200

gat 1203

<210> 297
<211> 315
<212> DNA
<213> Artificial Sequence

<220>
<223> orf

<400> 297
ttgttgatta attgccattt agttgttata atggttttat caactggagt tcatgctatg 60

actattcaat caagattaaa caaagatact gttcagctaa ttaagattgc tcaacgtata 120

gaatttatta gaaaacaaga tgaaatgggc aaccaaaaag ttatagctat gatttatata 180

tcagaagatt ctaaccctat tcgagcgatg gctggtgatg ttacttggga cgctcatgac 240

attgataagg ctaaacgctc tattcgtcgt cataataaaa cttgtttaat tattgataga 300

cgagatcaag ttatt 315

<210> 298
<211> 222
<212> DNA
<213> Artificial Sequence

<220>
<223> orf

<400> 298
atttcggaga tcatcatggc tgtgccacat actactgttt tgtctttgct tggcaaagaa 60

gtttcatttt ctgtattact agatgaagaa ataaaaatct tttttcctca aggcattcac 120

ataaatggat gcgtccaaga agtaatcata gctctcaatg gtaatcacca aattttggtt 180

gatgacgaat tttatgaatt atcacgtatt gacttatttt ct 222

<210> 299
<211> 180
<212> DNA
<213> Artificial Sequence

<220>
<223> orf

<400> 299
atttacccga taggggagac cctgaatcct ttattcataa aggtttcagg gtttttttat 60

cctttttaca taatctgtct tataggaact ctcctactaa acattactta tataatcttt 120

agttactgga gacattctct ccaggaacag agggcacgga gtgtgcccga actgacgcaa 180

<210> 300
<211> 309
<212> DNA
<213> Artificial Sequence

<220>
<223> orf

<400> 300
atgccaaatg atagaatttc acctttagag gttgttttag gaaatattct tggtgaggaa 60

tcaccaagta gaccagacca aaaagcagtt tctttccgaa ttgattttga taactacgtc 120

aatctttgcg ctctgcaaag cattactccg aaaggagtta gtcggaatca gcttttaaat 180

gatcttctag ctgttgctat tgatcaggtt tcatctgctt tacctgatgg tgtttctgat 240

gcttatgaag aatctgtcgt tcaccatgaa caaggttgaa acgaactttt agcaattgag 300

ggccattta 309

<210> 301
<211> 168
<212> DNA
<213> Artificial Sequence

<220>
<223> orf

<400> 301
atgattggcc ctcaagttat tagcaaggga accatggatt cccttgaaga agcatcagct 60

tatttcaaac aggtcttaca agtgcacttg gataacaagc gcgatatcac attgtttgcg 120

attaccgttg ttggcggtcg ccttaaggta gtagagggtt taccaaaa 168

<210> 302
<211> 291
<212> DNA
<213> Artificial Sequence

<220>
<223> orf

<400> 302
atgatcaaac tagaaggcac aattatcaat gtattcaccc agcagggtgg cacgagcaag 60

aaaacaggcg aagcgtttgc ggatcgtgac aagattcagc taatgggaga actggaactc 120

ccaaacggtt ccataaaaca tgaattgatt gaccttaccg ttgaaaatgg caaggaatat 180

gaaaccctaa aaggcaagcg tgtcagcatc ccatgtggtg ctctcgcttc aggtcgtaaa 240

gtgatttttt atgttgcgaa aggtggaaca cctaaacttt tgacagcagt a 291

<210> 303
<211> 1335
<212> DNA
<213> Artificial Sequence

<220>
<223> orf

<400> 303
ttgatactat tgattaaagt ggggacaaaa ttgcttaaaa aagacaataa aaaagcccat      60

atttcgaaca ctttggcggg tggagaaata caggctttgc aatctgctaa ggcagatcaa     120

cacagggata gaataacacg ttttgcgact ttgaaacata gggctaagaa tcaggaaaat     180

tatttattta ccctggctaa gtttaaagag aactatgaaa aagacgttaa aaacgaagaa     240

tctatcaagg ctttaaaatc tgctcataaa ttgaatgaat gcggaaacta tctgctattc     300

aaaaattttt acacaatagg tgaagttaaa ctctccaagc tccgcacctg cggacagcat     360

ttgctctgcc ctttctgtgc tgctattcgt gcttctcgtg ctatccaaaa atacgttgaa     420

cgtattgatc aggtgttgca agaaaatcgc aagctcaagc ccgttctaat cactcttacc     480

gttaaaaacg gttctgacct agcggaacgc tctgaacatc ttatgaagtc ctttaggacg     540

cttttagatc gtcgtaggga ctatgaaaag aaagggcgag gttttaacga gttctgtaag     600

gttcaagggg ctatgtactc ttacgagagt acatacaacg aaaaaacagt tgaatggcat     660

ccgcatatcc atatgtttgc tttggttgat cagtggattg atcagcaaga attttcagaa     720

tattggcata gccttactgg ggactcgatg gttgtcgatg ttcgcagggc aagaaaagaa     780

aaaggacacg gctatagcaa agcggctgcc gaagtttgta agtatgcttt aaagtttggt     840

gatctatccg ttgaaaacac ttgggaagcc tttaaagttc tcaagggcaa gcgtttaaca     900

ggttcttttg gtctgttatg gggtgtcaaa attcctgaaa acttagcaga tgaaattcca     960

gatgatcagg acttgccata tcttgaaatg ctgtacaagt tcgtttacgg caaaaattca    1020

cattatgact tagctatgac tcgtatggtt gagccacaag cgaagattga gcgaatgagt    1080

gaggaggagg caacgaccga ccgacacgac atgagagaaa tagagcgagt ggcgataaag    1140

gacgatgtga ggacggtggc aggtggggca ccactgcgct gcccgcaacg aggacgcaaa    1200

aagcaacatt ggcaagtatc accttacgtc cgtgttagga cacggactag aatccgtcaa    1260

tgggacgggt tcttatacaa catagattta ttcccttatg tagaaaaacg attatatcgc    1320

tatttagttg ttgat                                                    1335

<210> 304
<211> 180
<212> DNA
<213> Artificial Sequence

<220>
<223> orf

<400> 304
ataaattata caccgatttt atatcaaatc aatagtgagt cgccacccat tcgggatggc 60

tcggcactta ctgtgagaat gcagagggga gcttgtaatc agagagggag gtttttagat 120

ttttattgcg tcagttcggg cacactccgt gccctctgtt cctggagaga atgtctccag 180


<210> 305
<211> 186
<212> DNA
<213> Artificial Sequence


<220>
<223> orf

<400> 305
gtgatacttg ccaatgttgc tttttgcgtc ctcgttgcgg gcagcgcagt ggtgccccac 60

ctgccaccgt cctcacatcg tcctttatcg ccactcgctc tatttctctc atgtcgtgtc 120

ggtcggtcgt tgcctcctcc tcactcattc gctcaatctt cgcttgtggc tcaaccatac 180

gagtca 186


<210> 306
<211> 183
<212> DNA
<213> Artificial Sequence


<220>
<223> orf

<400> 306
ttgccaatgt tgctttttgc gtcctcgttg cgggcagcgc agtggtgccc cacctgccac 60

cgtcctcaca tcgtccttta tcgccactcg ctctatttct ctcatgtcgt gtcggtcggt 120

cgttgcctcc tcctcactca ttcgctcaat cttcgcttgt ggctcaacca tacgagtcat 180

agc 183


<210> 307
<211> 174
<212> DNA
<213> Artificial Sequence


<220>
<223> orf

<400> 307
ttgcgtcctc gttgcgggca gcgcagtggt gccccacctg ccaccgtcct cacatcgtcc 60

tttatcgcca ctcgctctat ttctctcatg tcgtgtcggt cggtcgttgc ctcctcctca 120

ctcattcgct caatcttcgc ttgtggctca accatacgag tcatagctaa gtca 174

<210> 308
<211> 162
<212> DNA
<213> Artificial Sequence

<220>
<223> orf

<400> 308
atttcatctg ctaagttttc aggaattttg acaccccata acagaccaaa agaacctgtt 60

aaacgcttgc ccttgagaac tttaaaggct tcccaagtgt tttcaacgga tagatcacca 120

aactttaaag catacttaca aacttcggca gccgctttgc ta 162

<210> 309
<211> 162
<212> DNA
<213> Artificial Sequence

<220>
<223> orf

<400> 309
ttgctgatca atccactgat caaccaaagc aaacatatgg atatgcggat gccattcaac 60

tgttttttcg ttgtatgtac tctcgtaaga gtacatagcc ccttgaacct tacagaactc 120

gttaaaacct cgccctttct tttcatagtc cctacgacga tc 162

<210> 310
<211> 150
<212> DNA
<213> Artificial Sequence

<220>
<223> orf

<400> 310
attttcttgc aacacctgat caatacgttc aacgtatttt tggatagcac gagaagcacg 60

aatagcagca cagaaagggc agagcaaatg ctgtccgcag gtgcggagct tggagagttt 120

aacttcacct attgtgtaaa aatttttgaa 150

<210> 311
<211> 174
<212> DNA
<213> Artificial Sequence

<220>

<223> orf

<400> 311
ataattttcc tgattcttag ccctatgttt caaagtcgca aaacgtgtta ttctatccct 60

gtgttgatct gccttagcag attgcaaagc ctgtatttct ccacccgcca aagtgttcga 120

aatatgggct tttttattgt cttttttaag caattttgtc cccactttaa tcaa 174

<210> 312
<211> 2736
<212> DNA
<213> Artificial Sequence

<220>
<223> isolate

<400> 312
caacacaggg atagaataac acgttttggt attttgaaac acagatcgaa gcaacaagaa 60

aactttttgt ggactcttgc aaaattcaaa gaaaactacc caatagacac gccaaatgaa 120

gaaactgtaa aggctctaaa atctgcacag aagttgaacg gctgtggcaa ttttttgttg 180

ttcaaaaatt tttacacaat agatcaaacc aagctcgcaa aatttcatgt ttgtaatcag 240

catttgttgt gtccattttg tgcaggtatt cgtgcatcta aagcaattca aaaatataca 300

gaacgtgttg atgaggtctt aaagaaaaat cgcaagctca agcctgttct aatcacgctc 360

acagtaaaaa acggtactga cctaagtgaa cggtctgcac atctcataaa atcgcttaga 420

acgcttatta agcgtcgtca ggactataat aaaaaaggtc gtggattcaa cgagttttgt 480

aaaatcaacg gtgcaatgta ctcctatgaa aatacttata acgaacaaac cgaagaatgg 540

catccgcatg ttcacatgtt tgcccttttg gatgattgga tagaccagga acaaatgtct 600

gaaacttggc atgaaatcac tggtgattca ttcattgtag atattcgaaa agttaaaaaa 660

gaaaaagact taggctatgc aaaggctgct gctgaggtat gcaaatacgc tcttaaattt 720

ggtgatctaa ctgttgaaaa tacttgggaa gcattcaagg ttctaaaggg caaacgctta 780

agcggtgcat ttggatcact ttatggtgtc aaaattcctg aaaatcttgc tgatgatatg 840

ccagatgaaa aagaccttcc gtatttagaa atgctctata aattcgtgtt tagaaaacag 900

tcttactacg atctacaaat gacacgtcat gtagagccac aggcgaatga tcgacagagg 960

gagaaggagg aacgaccgtc cgacccgact ggagagaatg agaaggtggt gattggggac 1020

gaggcgagga cggtggcagg tggagggctc gcaccactgc accgcccgca gcaaggacgc 1080

aaaaaacaac actggcaagt accaccagta acgagggtac gagtgaaaaa acgtatcaaa 1140

agatgggatg gttatttgtg tgttatacac atgtaaaatc ataaacctat gttaaaattc 1200

tagtcgctga atagtcggac ggcaattcaa ctacccagct agccaatcag atctatcgag 1260

gagatcatca tggctgttgc acatactact gttttgtctt tgcttggcaa agatgtttca 1320

ttttctgttt tattggatga gcaaataaaa tccttttttc ctgaaggaat aaatataact 1380

ggcttattcg aagaagttat tatcgctcta aatggtaatc accaaatcct agttggtgat 1440

gagttttacc aactttcaaa aattgaccta aatctttaaa taaccccgag aggggatacc 1500

tcgaaccctt ttaatataaa ggtttcgggg ttttttttat ctattaacat aatgatgact 1560

atgggaaatt agctactaaa gattactgct ataatcttta gcgagtgtct acgagtgacg 1620

ctcccacaat aaaaatttca aagcctttcc cctctgatta caagctccct tttgcctttc 1680

tccggtaaga accgagccat cccgaatggg tggcgactcc cctattgatt tgtttaagat 1740

tcggtgtatg atttattgat aaattacgaa cgaatcatta atgattaggt aaaaatatgc 1800

caaatgatag aatttcccct ctagatgttg ttctaggaaa tattcttggt gcagatgcat 1860

caagtagacc cgaccaaaaa gcagtttcct ttcgaattga ttttaataat tacgtgaacc 1920

tttgcgcctt gcagagcatc acacctaagg gagttagccg aaatcaactt ttgaatgatc 1980

ttttagctgt tgctattgat cgggttgtat cgtccttacc tgatggcgtt tcagacgctt 2040

atgaagaagc tgttgcccat aatgaagaag gtttaacaat gatcttagaa agtgagggtc 2100

atttatgatt aggcccgaag ttattagtag taccccaatg gaatctcttg aagaagcatg 2160

tgcttatttc aagcaagtat tacaagaaca tttagataaa aaacgtgaca tcacattatt 2220

tgcgattgct gttgtaggcg gtcgtttgaa agtagtagag ggattaccaa aatgattaaa 2280

ttagaaggca caattattaa tgtttttacc caacaaggtg gtaaagataa aaaaactggt 2340

gatgcttttg aagatcgtga caaggttcaa cttatgggtg agttagaact cccaaacggt 2400

gatattaagc atgaattaat caacctaagt gttgatgatg gtaagcatta cgaaacacta 2460

aaaaacaagc gtgtgagcat tccttgcggt gctctagctt ctggtcgaaa tgtgattttt 2520

tatgtcacaa aaggcggtac acctaaaatt cttccaagta tctaaaattt gaaaaaaaac 2580

agtgagttag cgaagcggct cgctgttttt ttatttactt gatactattc attaagtcgg 2640

ggacaaaatt tcatgagaaa tgacaataaa aaacccctgt tatcagaagc tttggcgggc 2700

gtagataaca agggttcgca atctgctaag gcagat 2736

<210> 313
<211> 386
<212> PRT
<213> Artificial Sequence

<220>
<223> orf

<400> 313
Met Thr Arg Phe Gly Ile Leu Lys His Arg Ser Lys Gln Gln Glu Asn
1               5                   10                  15
Phe Leu Trp Thr Leu Ala Lys Phe Lys Glu Asn Tyr Pro Ile Asp Thr

                20                  25                  30
Pro Asn Glu Glu Thr Val Lys Ala Leu Lys Ser Ala Gln Lys Leu Asn
        35                  40                  45
Gly Cys Gly Asn Phe Leu Leu Phe Lys Asn Phe Tyr Thr Ile Asp Gln
    50                  55                  60
Thr Lys Leu Ala Lys Phe His Val Cys Asn Gln His Leu Leu Cys Pro
65                  70                  75                  80
Phe Cys Ala Gly Ile Arg Ala Ser Lys Ala Ile Gln Lys Tyr Thr Glu
                85                  90                  95
Arg Val Asp Glu Val Leu Lys Lys Asn Arg Lys Leu Lys Pro Val Leu
            100                 105                 110
Ile Thr Leu Thr Val Lys Asn Gly Thr Asp Leu Ser Glu Arg Ser Ala
        115                 120                 125
His Leu Ile Lys Ser Leu Arg Thr Leu Ile Lys Arg Arg Gln Asp Tyr
    130                 135                 140
Asn Lys Lys Gly Arg Gly Phe Asn Glu Phe Cys Lys Ile Asn Gly Ala
145                 150                 155                 160
Met Tyr Ser Tyr Glu Asn Thr Tyr Asn Glu Gln Thr Glu Glu Trp His
                165                 170                 175
Pro His Val His Met Phe Ala Leu Leu Asp Asp Trp Ile Asp Gln Glu
            180                 185                 190
Gln Met Ser Glu Thr Trp His Glu Ile Thr Gly Asp Ser Phe Ile Val
        195                 200                 205
Asp Ile Arg Lys Val Lys Lys Glu Lys Asp Leu Gly Tyr Ala Lys Ala
    210                 215                 220
Ala Ala Glu Val Cys Lys Tyr Ala Leu Lys Phe Gly Asp Leu Thr Val
225                 230                 235                 240
Glu Asn Thr Trp Glu Ala Phe Lys Val Leu Lys Gly Lys Arg Leu Ser
                245                 250                 255
Gly Ala Phe Gly Ser Leu Tyr Gly Val Lys Ile Pro Glu Asn Leu Ala
            260                 265                 270
Asp Asp Met Pro Asp Glu Lys Asp Leu Pro Tyr Leu Glu Met Leu Tyr
        275                 280                 285
Lys Phe Val Phe Arg Lys Gln Ser Tyr Tyr Asp Leu Gln Met Thr Arg
    290                 295                 300
His Val Glu Pro Gln Ala Asn Asp Arg Gln Arg Glu Lys Glu Glu Arg
305                 310                 315                 320
Pro Ser Asp Pro Thr Gly Glu Asn Glu Lys Val Val Ile Gly Asp Glu
                325                 330                 335
Ala Arg Thr Val Ala Gly Gly Gly Leu Ala Pro Leu His Arg Pro Gln
            340                 345                 350
Gln Gly Arg Lys Lys Gln His Trp Gln Val Pro Pro Val Thr Arg Val
        355                 360                 365
Arg Val Lys Lys Arg Ile Lys Arg Trp Asp Gly Tyr Leu Cys Val Ile
    370                 375                 380
His Met
385

<210> 314
<211> 77
<212> PRT
<213> Artificial Sequence

<220>
<223> orf

<400> 314
Met Arg Ser Ile Glu Glu Ile Ile Met Ala Val Ala His Thr Thr Val
1               5                   10                  15
Leu Ser Leu Leu Gly Lys Asp Val Ser Phe Ser Val Leu Leu Asp Glu
            20                  25                  30
Gln Ile Lys Ser Phe Phe Pro Glu Gly Ile Asn Ile Thr Gly Leu Phe

35                  40                  45
Glu Glu Val Ile Ile Ala Leu Asn Gly Asn His Gln Ile Leu Val Gly
    50                  55                  60
Asp Glu Phe Tyr Gln Leu Ser Lys Ile Asp Leu Asn Leu
65                  70                  75

<210> 315
<211> 103
<212> PRT
<213> Artificial Sequence

<220>
<223> orf

<400> 315
Met Pro Asn Asp Arg Ile Ser Pro Leu Asp Val Val Leu Gly Asn Ile
1               5                   10                  15
Leu Gly Ala Asp Ala Ser Ser Arg Pro Asp Gln Lys Ala Val Ser Phe
            20                  25                  30
Arg Ile Asp Phe Asn Asn Tyr Val Asn Leu Cys Ala Leu Gln Ser Ile
        35                  40                  45
Thr Pro Lys Gly Val Ser Arg Asn Gln Leu Leu Asn Asp Leu Leu Ala
    50                  55                  60
Val Ala Ile Asp Arg Val Val Ser Ser Leu Pro Asp Gly Val Ser Asp
65                  70                  75                  80
Ala Tyr Glu Glu Ala Val Ala His Asn Glu Glu Gly Leu Thr Met Ile
                85                  90                  95
Leu Glu Ser Glu Gly His Leu
            100

<210> 316
<211> 56
<212> PRT
<213> Artificial Sequence

<220>
<223> orf

<400> 316
Met Ile Arg Pro Glu Val Ile Ser Ser Thr Pro Met Glu Ser Leu Glu
1               5                   10                  15
Glu Ala Cys Ala Tyr Phe Lys Gln Val Leu Gln Glu His Leu Asp Lys
            20                  25                  30
Lys Arg Asp Ile Thr Leu Phe Ala Ile Ala Val Val Gly Gly Arg Leu
        35                  40                  45
Lys Val Val Glu Gly Leu Pro Lys
    50                  55

<210> 317
<211> 116
<212> PRT
<213> Artificial Sequence

<220>
<223> orf

<400> 317
Met Ile Cys Asp Cys Cys Cys Arg Arg Ser Phe Glu Ser Ser Arg Gly
1               5                   10                  15
Ile Thr Lys Met Ile Lys Leu Glu Gly Thr Ile Ile Asn Val Phe Thr

                20                  25                  30
Gln Gln Gly Gly Lys Asp Lys Lys Thr Gly Asp Ala Phe Glu Asp Arg
            35                  40                  45
Asp Lys Val Gln Leu Met Gly Glu Leu Glu Leu Pro Asn Gly Asp Ile
    50                  55                  60
Lys His Glu Leu Ile Asn Leu Ser Val Asp Asp Gly Lys His Tyr Glu
65                  70                  75                  80
Thr Leu Lys Asn Lys Arg Val Ser Ile Pro Cys Gly Ala Leu Ala Ser
                85                  90                  95
Gly Arg Asn Val Ile Phe Tyr Val Thr Lys Gly Gly Thr Pro Lys Ile
            100                 105                 110
Leu Pro Ser Ile
        115

<210> 318
<211> 419
<212> PRT
<213> Artificial Sequence

<220>
<223> orf

<400> 318
Met Arg Asn Asp Asn Lys Lys Pro Leu Leu Ser Glu Ala Leu Ala Gly
1               5                   10                  15
Val Asp Asn Lys Gly Ser Gln Ser Ala Lys Ala Asp Gln His Arg Asp
            20                  25                  30
Arg Ile Thr Arg Phe Gly Ile Leu Lys His Arg Ser Lys Gln Gln Glu
        35                  40                  45
Asn Phe Leu Trp Thr Leu Ala Lys Phe Lys Glu Asn Tyr Pro Ile Asp
    50                  55                  60
Thr Pro Asn Glu Glu Thr Val Lys Ala Leu Lys Ser Ala Gln Lys Leu
65                  70                  75                  80
Asn Gly Cys Gly Asn Phe Leu Leu Phe Lys Asn Phe Tyr Thr Ile Asp
                85                  90                  95
Gln Thr Lys Leu Ala Lys Phe His Val Cys Asn Gln His Leu Leu Cys
            100                 105                 110
Pro Phe Cys Ala Gly Ile Arg Ala Ser Lys Ala Ile Gln Lys Tyr Thr
        115                 120                 125
Glu Arg Val Asp Glu Val Leu Lys Lys Asn Arg Lys Leu Lys Pro Val
    130                 135                 140
Leu Ile Thr Leu Thr Val Lys Asn Gly Thr Asp Leu Ser Glu Arg Ser
145                 150                 155                 160
Ala His Leu Ile Lys Ser Leu Arg Thr Leu Ile Lys Arg Arg Gln Asp
                165                 170                 175
Tyr Asn Lys Lys Gly Arg Gly Phe Asn Glu Phe Cys Lys Ile Asn Gly
            180                 185                 190
Ala Met Tyr Ser Tyr Glu Asn Thr Tyr Asn Glu Gln Thr Glu Glu Trp
        195                 200                 205
His Pro His Val His Met Phe Ala Leu Leu Asp Asp Trp Ile Asp Gln
    210                 215                 220
Glu Gln Met Ser Glu Thr Trp His Glu Ile Thr Gly Asp Ser Phe Ile
225                 230                 235                 240
Val Asp Ile Arg Lys Val Lys Lys Glu Lys Asp Leu Gly Tyr Ala Lys
                245                 250                 255
Ala Ala Ala Glu Val Cys Lys Tyr Ala Leu Lys Phe Gly Asp Leu Thr
            260                 265                 270
Val Glu Asn Thr Trp Glu Ala Phe Lys Val Leu Lys Gly Lys Arg Leu
        275                 280                 285
Ser Gly Ala Phe Gly Ser Leu Tyr Gly Val Lys Ile Pro Glu Asn Leu
    290                 295                 300
Ala Asp Asp Met Pro Asp Glu Lys Asp Leu Pro Tyr Leu Glu Met Leu

305                 310                 315                 320
Tyr Lys Phe Val Phe Arg Lys Gln Ser Tyr Tyr Asp Leu Gln Met Thr
                325                 330                 335
Arg His Val Glu Pro Gln Ala Asn Asp Arg Gln Arg Glu Lys Glu Glu
            340                 345                 350
Arg Pro Ser Asp Pro Thr Gly Glu Asn Glu Lys Val Val Ile Gly Asp
        355                 360                 365
Glu Ala Arg Thr Val Ala Gly Gly Gly Leu Ala Pro Leu His Arg Pro
    370                 375                 380
Gln Gln Gly Arg Lys Lys Gln His Trp Gln Val Pro Pro Val Thr Arg
385                 390                 395                 400
Val Arg Val Lys Lys Arg Ile Lys Arg Trp Asp Gly Tyr Leu Cys Val
                405                 410                 415
Ile His Met

<210> 319
<211> 50
<212> PRT
<213> Artificial Sequence

<220>
<223> orf

<400> 319
Met Ser Arg Ser Ser Lys Ala Ser Pro Val Phe Leu Ser Leu Pro Pro
1               5                   10                  15
Cys Trp Val Lys Thr Leu Ile Ile Val Pro Ser Asn Leu Ile Ile Leu
            20                  25                  30
Val Ile Pro Leu Leu Leu Ser Asn Asp Arg Leu Gln Gln Gln Ser Gln
        35                  40                  45
Ile Met
    50

<210> 320
<211> 54
<212> PRT
<213> Artificial Sequence

<220>
<223> orf

<400> 320
Met Ile Thr Ser Ser Asn Lys Pro Val Ile Phe Ile Pro Ser Gly Lys
1               5                   10                  15
Lys Asp Phe Ile Cys Ser Ser Asn Lys Thr Glu Asn Glu Thr Ser Leu
            20                  25                  30
Pro Ser Lys Asp Lys Thr Val Val Cys Ala Thr Ala Met Met Ile Ser
        35                  40                  45
Ser Ile Asp Leu Ile Gly
    50

<210> 321
<211> 70
<212> PRT
<213> Artificial Sequence

<220>
<223> orf

<400> 321
Met Ile Arg Phe Phe Thr Arg Thr Leu Val Thr Gly Gly Thr Cys Gln
1               5                   10                  15
Cys Cys Phe Leu Arg Pro Cys Cys Gly Arg Cys Ser Gly Ala Ser Pro
            20                  25                  30
Pro Pro Ala Thr Val Leu Ala Ser Ser Pro Ile Thr Thr Phe Ser Phe
        35                  40                  45
Ser Pro Val Gly Ser Asp Gly Arg Ser Ser Phe Ser Leu Cys Arg Ser
    50                  55                  60
Phe Ala Cys Gly Ser Thr
65                  70

<210> 322
<211> 89
<212> PRT
<213> Artificial Sequence

<220>
<223> orf

<400> 322
Met Ile Leu His Val Tyr Asn Thr Gln Ile Thr Ile Pro Ser Phe Asp
1               5                   10                  15
Thr Phe Phe His Ser Tyr Pro Arg Tyr Trp Trp Tyr Leu Pro Val Leu
            20                  25                  30
Phe Phe Ala Ser Leu Leu Arg Ala Val Gln Trp Cys Glu Pro Ser Thr
        35                  40                  45
Cys His Arg Pro Arg Leu Val Pro Asn His His Leu Leu Ile Leu Ser
    50                  55                  60
Ser Arg Val Gly Arg Ser Phe Leu Leu Leu Pro Leu Ser Ile Ile Arg
65                  70                  75                  80
Leu Trp Leu Tyr Met Thr Cys His Leu
                85

<210> 323
<211> 72
<212> PRT
<213> Artificial Sequence

<220>
<223> orf

<400> 323
Met Val Val Leu Ala Ser Val Val Phe Cys Val Leu Ala Ala Gly Gly
1               5                   10                  15
Ala Val Val Arg Ala Leu His Leu Pro Pro Ser Ser Pro Arg Pro Gln
            20                  25                  30
Ser Pro Pro Ser His Ser Leu Gln Ser Gly Arg Thr Val Val Pro Pro
        35                  40                  45
Ser Pro Ser Val Asp His Ser Pro Val Ala Leu His Asp Val Ser Phe
    50                  55                  60
Val Asp Arg Ser Lys Thr Val Phe
65                  70

<210> 324
<211> 81
<212> PRT
<213> Artificial Sequence

<220>

<223> orf

<400> 324
Met Thr Ser Asp Phe Met Pro Ser Phe Arg His Leu Phe Leu Val Tyr
1               5                   10                  15
Pro Ile Ile Gln Lys Gly Lys His Val Asn Met Arg Met Pro Phe Phe
            20                  25                  30
Gly Leu Phe Val Ile Ser Ile Phe Ile Gly Val His Cys Thr Val Asp
        35                  40                  45
Phe Thr Lys Leu Val Glu Ser Thr Thr Phe Phe Ile Ile Val Leu Thr
    50                  55                  60
Thr Leu Asn Lys Arg Ser Lys Arg Phe Tyr Glu Met Cys Arg Pro Phe
65                  70                  75                  80
Thr

<210> 325
<211> 58
<212> PRT
<213> Artificial Sequence

<220>
<223> orf

<400> 325
Met Asn Thr Phe Cys Ile Phe Leu Asn Cys Phe Arg Cys Thr Asn Thr
1               5                   10                  15
Cys Thr Lys Trp Thr Gln Gln Met Leu Ile Thr Asn Met Lys Phe Cys
            20                  25                  30
Glu Leu Gly Leu Ile Tyr Cys Val Lys Ile Phe Glu Gln Gln Lys Ile
        35                  40                  45
Ala Thr Ala Val Gln Leu Leu Cys Arg Phe
    50                  55

<210> 326
<211> 56
<212> PRT
<213> Artificial Sequence

<220>
<223> orf

<400> 326
Met Trp Arg Val Tyr Trp Val Val Phe Phe Glu Phe Cys Lys Ser Pro
1               5                   10                  15
Gln Lys Val Phe Leu Leu Leu Arg Ser Val Phe Gln Asn Thr Lys Thr
            20                  25                  30
Cys Tyr Ser Ile Pro Val Leu Ile Cys Leu Ser Arg Leu Arg Thr Leu
        35                  40                  45
Val Ile Tyr Ala Arg Gln Ser Phe
    50                  55

<210> 327
<211> 1158
<212> DNA
<213> Artificial Sequence

<220>
<223> orf

<400> 327
ataacacgtt ttggtatttt gaaacacaga tcgaagcaac aagaaaactt tttgtggact      60

cttgcaaaat tcaaagaaaa ctacccaata gacacgccaa atgaagaaac tgtaaaggct     120

ctaaaatctg cacagaagtt gaacggctgt ggcaattttt tgttgttcaa aaatttttac     180

acaatagatc aaaccaagct cgcaaaattt catgtttgta atcagcattt gttgtgtcca     240

ttttgtgcag gtattcgtgc atctaaagca attcaaaaat atacagaacg tgttgatgag     300

gtcttaaaga aaaatcgcaa gctcaagcct gttctaatca cgctcacagt aaaaaacggt     360

actgacctaa gtgaacggtc tgcacatctc ataaaatcgc ttagaacgct tattaagcgt     420

cgtcaggact ataataaaaa aggtcgtgga ttcaacgagt tttgtaaaat caacggtgca     480

atgtactcct atgaaaatac ttataacgaa caaaccgaag aatggcatcc gcatgttcac     540

atgtttgccc ttttggatga ttggatagac caggaacaaa tgtctgaaac ttggcatgaa     600

atcactggtg attcattcat tgtagatatt cgaaaagtta aaaaagaaaa agacttaggc     660

tatgcaaagg ctgctgctga ggtatgcaaa tacgctctta aatttggtga tctaactgtt     720

gaaaatactt gggaagcatt caaggttcta aagggcaaac gcttaagcgg tgcatttgga     780

tcactttatg gtgtcaaaat tcctgaaaat cttgctgatg atatgccaga tgaaaaagac     840

cttccgtatt tagaaatgct ctataaattc gtgtttagaa aacagtctta ctacgatcta     900

caaatgacac gtcatgtaga gccacaggcg aatgatcgac agagggagaa ggaggaacga     960

ccgtccgacc cgactggaga gaatgagaag gtggtgattg gggacgaggc gaggacggtg    1020

gcaggtggag ggctcgcacc actgcaccgc ccgcagcaag gacgcaaaaa acaacactgg    1080

caagtaccac cagtaacgag ggtacgagtg aaaaaacgta tcaaaagatg ggatggttat    1140

ttgtgtgtta tacacatg                                                  1158

<210> 328
<211> 231
<212> DNA
<213> Artificial Sequence

<220>
<223> orf

<400> 328
atcagatcta tcgaggagat catcatggct gttgcacata ctactgtttt gtctttgctt      60

ggcaaagatg tttcattttc tgttttattg gatgagcaaa taaaatcctt ttttcctgaa     120

ggaataaata taactggctt attcgaagaa gttattatcg ctctaaatgg taatcaccaa     180

atcctagttg gtgatgagtt ttaccaactt tcaaaaattg acctaaatct t              231

<210> 329
<211> 309

<212> DNA
<213> Artificial Sequence

<220>
<223> orf

<400> 329
atgccaaatg atagaatttc ccctctagat gttgttctag gaaatattct tggtgcagat      60

gcatcaagta gacccgacca aaaagcagtt tcctttcgaa ttgattttaa taattacgtg     120

aacctttgcg ccttgcagag catcacacct aagggagtta gccgaaatca acttttgaat     180

gatcttttag ctgttgctat tgatcgggtt gtatcgtcct tacctgatgg cgtttcagac     240

gcttatgaag aagctgttgc ccataatgaa gaaggtttaa caatgatctt agaaagtgag     300

ggtcattta                                                             309

<210> 330
<211> 168
<212> DNA
<213> Artificial Sequence

<220>
<223> orf

<400> 330
atgattaggc ccgaagttat tagtagtacc ccaatggaat ctcttgaaga agcatgtgct      60

tatttcaagc aagtattaca agaacattta gataaaaaac gtgacatcac attatttgcg     120

attgctgttg taggcggtcg tttgaaagta gtagagggat taccaaaa                  168

<210> 331
<211> 348
<212> DNA
<213> Artificial Sequence

<220>
<223> orf

<400> 331
attatttgcg attgctgttg taggcggtcg tttgaaagta gtagagggat taccaaaatg      60

attaaattag aaggcacaat tattaatgtt tttacccaac aaggtggtaa agataaaaaa     120

actggtgatg cttttgaaga tcgtgacaag gttcaactta tgggtgagtt agaactccca     180

aacggtgata ttaagcatga attaatcaac ctaagtgttg atgatggtaa gcattacgaa     240

acactaaaaa acaagcgtgt gagcattcct tgcggtgctc tagcttctgg tcgaaatgtg     300

attttttatg tcacaaaagg cggtacacct aaaattcttc caagtatc                   348

<210> 332
<211> 1257

<212> DNA
<213> Artificial Sequence

<220>
<223> orf

<400> 332
atgagaaatg acaataaaaa acccctgtta tcagaagctt tggcgggcgt agataacaag 60

ggttcgcaat ctgctaaggc agatcaacac agggatagaa taacacgttt tggtattttg 120

aaacacagat cgaagcaaca agaaaacttt ttgtggactc ttgcaaaatt caaagaaaac 180

tacccaatag acacgccaaa tgaagaaact gtaaaggctc taaaatctgc acagaagttg 240

aacggctgtg gcaatttttt gttgttcaaa aatttttaca caatagatca aaccaagctc 300

gcaaaatttc atgtttgtaa tcagcatttg ttgtgtccat tttgtgcagg tattcgtgca 360

tctaaagcaa ttcaaaaata tacagaacgt gttgatgagg tcttaaagaa aaatcgcaag 420

ctcaagcctg ttctaatcac gctcacagta aaaaacggta ctgacctaag tgaacggtct 480

gcacatctca taaaatcgct tagaacgctt attaagcgtc gtcaggacta taataaaaaa 540

ggtcgtggat tcaacgagtt ttgtaaaatc aacggtgcaa tgtactccta tgaaaatact 600

tataacgaac aaaccgaaga atggcatccg catgttcaca tgtttgccct tttggatgat 660

tggatagacc aggaacaaat gtctgaaact tggcatgaaa tcactggtga ttcattcatt 720

gtagatattc gaaaagttaa aaaagaaaaa gacttaggct atgcaaaggc tgctgctgag 780

gtatgcaaat acgctcttaa atttggtgat ctaactgttg aaaatacttg ggaagcattc 840

aaggttctaa agggcaaacg cttaagcggt gcatttggat cactttatgg tgtcaaaatt 900

cctgaaaatc ttgctgatga tatgccagat gaaaaagacc ttccgtattt agaaatgctc 960

tataaattcg tgtttagaaa acagtcttac tacgatctac aaatgacacg tcatgtagag 1020

ccacaggcga atgatcgaca gagggagaag gaggaacgac cgtccgaccc gactggagag 1080

aatgagaagg tggtgattgg ggacgaggcg aggacggtgg caggtggagg gctcgcacca 1140

ctgcaccgcc cgcagcaagg acgcaaaaaa caacactggc aagtaccacc agtaacgagg 1200

gtacgagtga aaaaacgtat caaaagatgg gatggttatt tgtgtgttat acacatg 1257

<210> 333
<211> 150
<212> DNA
<213> Artificial Sequence

<220>
<223> orf

<400> 333
ttgtcacgat cttcaaaagc atcaccagtt tttttatctt taccaccttg ttgggtaaaa 60

acattaataa ttgtgccttc taatttaatc attttggtaa tccctctact actttcaaac 120

gaccgcctac aacagcaatc gcaaataatg 150

<210> 334
<211> 162
<212> DNA
<213> Artificial Sequence

<220>
<223> orf

<400> 334
ataataactt cttcgaataa gccagttata tttattcctt caggaaaaaa ggattttatt 60

tgctcatcca ataaaacaga aaatgaaaca tctttgccaa gcaaagacaa aacagtagta 120

tgtgcaacag ccatgatgat ctcctcgata gatctgattg gc 162

<210> 335
<211> 210
<212> DNA
<213> Artificial Sequence

<220>
<223> orf

<400> 335
ttgatacgtt ttttcactcg taccctcgtt actggtggta cttgccagtg ttgttttttg 60

cgtccttgct gcgggcggtg cagtggtgcg agccctccac ctgccaccgt cctcgcctcg 120

tccccaatca ccaccttctc attctctcca gtcgggtcgg acggtcgttc ctccttctcc 180

ctctgtcgat cattcgcctg tggctctaca 210

<210> 336
<211> 267
<212> DNA
<213> Artificial Sequence

<220>
<223> orf

<400> 336
atgattttac atgtgtataa cacacaaata accatcccat cttttgatac gttttttcac 60

tcgtaccctc gttactggtg gtacttgcca gtgttgtttt ttgcgtcctt gctgcgggcg 120

gtgcagtggt gcgagccctc cacctgccac cgtcctcgcc tcgtccccaa tcaccacctt 180

ctcattctct ccagtcgggt cggacggtcg ttcctccttc tccctctgtc gatcattcgc 240

ctgtggctct acatgacgtg tcatttg 267

<210> 337

<211> 216
<212> DNA
<213> Artificial Sequence

<220>
<223> orf

<400> 337
ctggtggtac ttgccagtgt tgttttttgc gtccttgctg cgggcggtgc agtggtgcga 60

gccctccacc tgccaccgtc ctcgcctcgt ccccaatcac caccttctca ttctctccag 120

tcgggtcgga cggtcgttcc tccttctccc tctgtcgatc attcgcctgt ggctctacat 180

gacgtgtcat ttgtagatcg tagtaagact gttttc 216

<210> 338
<211> 243
<212> DNA
<213> Artificial Sequence

<220>
<223> orf

<400> 338
atcaccagtg atttcatgcc aagtttcaga catttgttcc tggtctatcc aatcatccaa 60

aagggcaaac atgtgaacat gcggatgcca ttcttcggtt tgttcgttat aagtattttc 120

ataggagtac attgcaccgt tgattttaca aaactcgttg aatccacgac cttttttatt 180

atagtcctga cgacgcttaa taagcgttct aagcgatttt atgagatgtg cagaccgttc 240

act 243

<210> 339
<211> 174
<212> DNA
<213> Artificial Sequence

<220>
<223> orf

<400> 339
atcaacacgt tctgtatatt tttgaattgc tttagatgca cgaatacctg cacaaaatgg 60

acacaacaaa tgctgattac aaacatgaaa ttttgcgagc ttggtttgat ctattgtgta 120

aaaatttttg aacaacaaaa aattgccaca gccgttcaac ttctgtgcag attt 174

<210> 340
<211> 168
<212> DNA
<213> Artificial Sequence

<220>

<223> orf

<400> 340
atttggcgtg tctattgggt agttttcttt gaattttgca agagtccaca aaaagttttc      60

ttgttgcttc gatctgtgtt tcaaaatacc aaaacgtgtt attctatccc tgtgttgatc     120

tgccttagca gattgcgaac ccttgttatc tacgcccgcc aaagcttc                  168


<210> 341
<211> 2405
<212> DNA
<213> Artificial Sequence


<220>
<223> isolate

<400> 341
aaggcagatc aacacaggga tagaatatca cgttttggac ttttgaaaca tagagcgaaa      60

ctacaagaac aatatttgtg gacgcaagtt gatttcaaat ctgaaggcga agacgatcta     120

tcctataagg ctcttaaggc tgcaacaaaa ttaaaaggtt gtggtcagtt tcttatattc     180

agaaattact acaccatcga ccaagttaag ctcgaaaaat tccacgtatg cggacagcat     240

ttgctatgcc ccatgtgtgc aggtattcgc gctgcacgtt caatgaaacg ctatttagac     300

cgtatccatg aattaatgcg tcagaatccg cgtttaaagc ccgtcctgat cactttaacg     360

gtcaagaatg gggaagacct agaagaacgc tataatcacc ttacaagctc atttagaacg     420

cttctatcgc gttataggga ttacaagaaa aagggttggg ggtttaatca attctgtaag     480

attgatggcg ctttctatac cactgaatac acttacaacg ataaaactaa gcaatggcat     540

ccgcatattc atattttcgc tttactcaat gaatggatag accaagaaga acttgcagaa     600

acttggcacg atatcaccct tgattcttat attgtcgata ttcgtagagt taaaaaaacc     660

aaagaacacg gctatagcaa agctgttgct gaggtatgta aatacgctct taagtttggc     720

gatctatcag ttgaaaacac ttgggacgca tttttaacac ttaagggtaa acgccttaca     780

ggttctttcg gttctatgca cggtgtcaaa attcctgaaa aattcactga tgatatgcca     840

ctggaggagc tgccttactt agagatgttc taccgttttg tatttggtcc gaaatcatat     900

tacaacttag aaataacaaa agacgttaag ccgaacaaaa ggaatgagga gtgaggagga     960

ggcaccgacc gaccgacacg accgaatgac ttgtgaaggc ggtacgtcaa tcggttgtct    1020

gtgatgtgac gttccgtgaa cgtgcatcat catcagacaa tcgaataagc aagcctacct    1080

gtatacacag gacttgtagg gggttcgacc ccttagaccc caaagggcgc actttaccaa     1140

acctttcagg ttcggcagtg ctccccataa taggattata gatatgaacg aattactaga    1200

attgaaacac cgtattgata actgtactga tgatctccaa tctttcgaac ttcgaaagat    1260

ttatgacaag ctcattattg atctatggca atctgaaaaa attactttgt cagaaatgca    1320

gtctatggct ctccctctcc agtagcagag ggcgcggagt gcgcccgaac tgacatcatc 1380

taaatttcaa aaccttcctt ccctgattac aagctcccct tggatttcca agcgggagct 1440

tttagcaatc ccgctgggtt gcgtcacatt gaaacattga aacattgaaa cattgaaaca 1500

ttgaaacatt gaaacattga aacattgaaa cattgaaaca ttgaaacatt gaaacattgt 1560

ttcaatgaca catttatttg catatttttc aataatgatt tataagtggt tcacagatga 1620

attttttata aaactatcca tgaatagggg ctgatatgcc aaatccactt gatgtagtcc 1680

ttggaaatat ccttggttca gaacaaaccc gacctgatca aaaggctgtt tcctttcgta 1740

ttgattttaa aaactatgtg aagctgtctg ctttacaaag cattacacct aaaggtgtta 1800

gccgtaatca gcttttaaat gatcttttag ctgttgctct tgatcaagtt gaatcgtcct 1860

tgcctgatgg tgcttcggat gcttatgaac atgcacttgt agaaaatgaa gaaggtttaa 1920

ctatgctttt agaaacggaa ggtcatttat gattaagtta gaaggaattg ttttaaacgt 1980

atttacccag caaggcggta aagataaaaa aacaggtgat caatttgctg accgtgacaa 2040

ggtgcaaatt ttaggtgcta tggatctgcc taatggtgac gtaaaaaacg aactatttac 2100

gttatctgta gaaaactatc gggactttaa agacttccta aataagaaaa tctgtattgc 2160

tgttggtgca atggcaagcg gtcgaaatgt tattttttac gttgctaaag gcgcacagcc 2220

tgttttagca gaaacaatgt gaaacatgaa aaaaaacagt gagttagcct tggcttctcg 2280

ctgttttttt ctttctcttg atactattaa ctagagtggg gatgaaattg actaaaaaag 2340

acaataaaaa agcccataat tctgacactt tggcgggtga tgaattacag gctttgcaat 2400

ctgct 2405

<210> 342
<211> 62
<212> PRT
<213> Artificial Sequence

<220>
<223> orf

<400> 342
Met Ile Thr Leu Gln Ala His Leu Glu Arg Phe Tyr Arg Val Ile Gly
1               5                   10                  15
Ile Thr Arg Lys Arg Val Gly Gly Leu Ile Asn Ser Val Arg Leu Met
            20                  25                  30
Ala Leu Ser Ile Pro Leu Asn Thr Leu Thr Thr Ile Lys Leu Ser Asn
        35                  40                  45
Gly Ile Arg Ile Phe Ile Phe Ser Leu Tyr Ser Met Asn Gly
    50                  55                  60

<210> 343
<211> 54
<212> PRT
<213> Artificial Sequence

<220>
<223> orf

<400> 343
Met Gln Lys Leu Gly Thr Ile Ser Pro Leu Ile Leu Ile Leu Ser Ile
1               5                   10                  15
Phe Val Glu Leu Lys Lys Pro Lys Asn Thr Ala Ile Ala Lys Leu Leu
            20                  25                  30
Leu Arg Tyr Val Asn Thr Leu Leu Ser Leu Ala Ile Tyr Gln Leu Lys
        35                  40                  45
Thr Leu Gly Thr His Phe
    50

<210> 344
<211> 309
<212> PRT
<213> Artificial Sequence

<220>
<223> orf

<400> 344
Met Ser Arg Phe Gly Leu Leu Lys His Arg Ala Lys Leu Gln Glu Gln
1               5                   10                  15
Tyr Leu Trp Thr Gln Val Asp Phe Lys Ser Glu Gly Glu Asp Asp Leu
            20                  25                  30
Ser Tyr Lys Ala Leu Lys Ala Ala Thr Lys Leu Lys Gly Cys Gly Gln
        35                  40                  45
Phe Leu Ile Phe Arg Asn Tyr Tyr Thr Ile Asp Gln Val Lys Leu Glu
    50                  55                  60
Lys Phe His Val Cys Gly Gln His Leu Leu Cys Pro Met Cys Ala Gly
65                  70                  75                  80
Ile Arg Ala Ala Arg Ser Met Lys Arg Tyr Leu Asp Arg Ile His Glu
                85                  90                  95
Leu Met Arg Gln Asn Pro Arg Leu Lys Pro Val Leu Ile Thr Leu Thr
            100                 105                 110
Val Lys Asn Gly Glu Asp Leu Glu Glu Arg Tyr Asn His Leu Thr Ser
        115                 120                 125
Ser Phe Arg Thr Leu Leu Ser Arg Tyr Arg Asp Tyr Lys Lys Lys Gly
    130                 135                 140
Trp Gly Phe Asn Gln Phe Cys Lys Ile Asp Gly Ala Phe Tyr Thr Thr
145                 150                 155                 160
Glu Tyr Thr Tyr Asn Asp Lys Thr Lys Gln Trp His Pro His Ile His
                165                 170                 175
Ile Phe Ala Leu Leu Asn Glu Trp Ile Asp Gln Glu Glu Leu Ala Glu
            180                 185                 190
Thr Trp His Asp Ile Thr Leu Asp Ser Tyr Ile Val Asp Ile Arg Arg
        195                 200                 205
Val Lys Lys Thr Lys Glu His Gly Tyr Ser Lys Ala Val Ala Glu Val
    210                 215                 220
Cys Lys Tyr Ala Leu Lys Phe Gly Asp Leu Ser Val Glu Asn Thr Trp
225                 230                 235                 240
Asp Ala Phe Leu Thr Leu Lys Gly Lys Arg Leu Thr Gly Ser Phe Gly
                245                 250                 255
Ser Met His Gly Val Lys Ile Pro Glu Lys Phe Thr Asp Asp Met Pro
            260                 265                 270
Leu Glu Glu Leu Pro Tyr Leu Glu Met Phe Tyr Arg Phe Val Phe Gly
        275                 280                 285
Pro Lys Ser Tyr Tyr Asn Leu Glu Ile Thr Lys Asp Val Lys Pro Asn
    290                 295                 300

Lys Arg Asn Glu Glu
305

<210> 345
<211> 62
<212> PRT
<213> Artificial Sequence

<220>
<223> orf

<400> 345
Met Lys Ala Val Arg Gln Ser Val Val Cys Asp Val Thr Phe Arg Glu
1               5                   10                  15
Arg Ala Ser Ser Ser Asp Asn Arg Ile Ser Lys Pro Thr Cys Ile His
            20                  25                  30
Arg Thr Cys Arg Gly Phe Asp Pro Leu Asp Pro Lys Gly Arg Thr Leu
        35                  40                  45
Pro Asn Leu Ser Gly Ser Ala Val Leu Pro Ile Ile Gly Leu
    50                  55                  60

<210> 346
<211> 56
<212> PRT
<213> Artificial Sequence

<220>
<223> orf

<400> 346
Met Ile Asp Met Asn Glu Leu Leu Glu Leu Lys His Arg Ile Asp Asn
1               5                   10                  15
Cys Thr Asp Asp Leu Gln Ser Phe Glu Leu Arg Lys Ile Tyr Asp Lys
            20                  25                  30
Leu Ile Ile Asp Leu Trp Gln Ser Glu Lys Ile Thr Leu Ser Glu Met
        35                  40                  45
Gln Ser Met Ala Leu Pro Leu Gln
    50                  55

<210> 347
<211> 52
<212> PRT
<213> Artificial Sequence

<220>
<223> orf

<400> 347
Met Ile Thr Val Leu Met Ile Ser Asn Leu Ser Asn Phe Glu Arg Phe
1               5                   10                  15
Met Thr Ser Ser Leu Leu Ile Tyr Gly Asn Leu Lys Lys Leu Leu Cys
            20                  25                  30
Gln Lys Cys Ser Leu Trp Leu Ser Leu Ser Ser Ser Arg Gly Arg Gly
        35                  40                  45
Val Arg Pro Asn
    50

<210> 348
<211> 102
<212> PRT

<213> Artificial Sequence

<220>
<223> orf

<400> 348
Met Gly Ala Asp Met Pro Asn Pro Leu Asp Val Val Leu Gly Asn Ile
1               5                   10                  15
Leu Gly Ser Glu Gln Thr Arg Pro Asp Gln Lys Ala Val Ser Phe Arg
            20                  25                  30
Ile Asp Phe Lys Asn Tyr Val Lys Leu Ser Ala Leu Gln Ser Ile Thr
        35                  40                  45
Pro Lys Gly Val Ser Arg Asn Gln Leu Leu Asn Asp Leu Leu Ala Val
    50                  55                  60
Ala Leu Asp Gln Val Glu Ser Ser Leu Pro Asp Gly Ala Ser Asp Ala
65                  70                  75                  80
Tyr Glu His Ala Leu Val Glu Asn Glu Glu Gly Leu Thr Met Leu Leu
                85                  90                  95
Glu Thr Glu Gly His Leu
            100

<210> 349
<211> 102
<212> PRT
<213> Artificial Sequence

<220>
<223> orf

<400> 349
Met Gly Ala Asp Met Pro Asn Pro Leu Asp Val Val Leu Gly Asn Ile
1               5                   10                  15
Leu Gly Ser Glu Gln Thr Arg Pro Asp Gln Lys Ala Val Ser Phe Arg
            20                  25                  30
Ile Asp Phe Lys Asn Tyr Val Lys Leu Ser Ala Leu Gln Ser Ile Thr
        35                  40                  45
Pro Lys Gly Val Ser Arg Asn Gln Leu Leu Asn Asp Leu Leu Ala Val
    50                  55                  60
Ala Leu Asp Gln Val Glu Ser Ser Leu Pro Asp Gly Ala Ser Asp Ala
65                  70                  75                  80
Tyr Glu His Ala Leu Val Glu Asn Glu Glu Gly Leu Thr Met Leu Leu
                85                  90                  95
Glu Thr Glu Gly His Leu
            100

<210> 350
<211> 97
<212> PRT
<213> Artificial Sequence

<220>
<223> orf

<400> 350
Met Ile Lys Leu Glu Gly Ile Val Leu Asn Val Phe Thr Gln Gln Gly
1               5                   10                  15
Gly Lys Asp Lys Lys Thr Gly Asp Gln Phe Ala Asp Arg Asp Lys Val
            20                  25                  30
Gln Ile Leu Gly Ala Met Asp Leu Pro Asn Gly Asp Val Lys Asn Glu
        35                  40                  45

Leu Phe Thr Leu Ser Val Glu Asn Tyr Arg Asp Phe Lys Asp Phe Leu
    50                  55                  60
Asn Lys Lys Ile Cys Ile Ala Val Gly Ala Met Ala Ser Gly Arg Asn
65                  70                  75                  80
Val Ile Phe Tyr Val Ala Lys Gly Ala Gln Pro Val Leu Ala Glu Thr
                85                  90                  95
Met

<210> 351
<211> 353
<212> PRT
<213> Artificial Sequence

<220>
<223> orf

<400> 351
Met Ile Leu Leu Thr Arg Val Gly Met Lys Leu Thr Lys Lys Asp Asn
1               5                   10                  15
Lys Lys Ala His Asn Ser Asp Thr Leu Ala Gly Asp Glu Leu Gln Ala
            20                  25                  30
Leu Gln Ser Ala Lys Ala Asp Gln His Arg Asp Arg Ile Ser Arg Phe
        35                  40                  45
Gly Leu Leu Lys His Arg Ala Lys Leu Gln Glu Gln Tyr Leu Trp Thr
    50                  55                  60
Gln Val Asp Phe Lys Ser Glu Gly Glu Asp Asp Leu Ser Tyr Lys Ala
65                  70                  75                  80
Leu Lys Ala Ala Thr Lys Leu Lys Gly Cys Gly Gln Phe Leu Ile Phe
                85                  90                  95
Arg Asn Tyr Tyr Thr Ile Asp Gln Val Lys Leu Glu Lys Phe His Val
            100                 105                 110
Cys Gly Gln His Leu Leu Cys Pro Met Cys Ala Gly Ile Arg Ala Ala
        115                 120                 125
Arg Ser Met Lys Arg Tyr Leu Asp Arg Ile His Glu Leu Met Arg Gln
    130                 135                 140
Asn Pro Arg Leu Lys Pro Val Leu Ile Thr Leu Thr Val Lys Asn Gly
145                 150                 155                 160
Glu Asp Leu Glu Glu Arg Tyr Asn His Leu Thr Ser Ser Phe Arg Thr
                165                 170                 175
Leu Leu Ser Arg Tyr Arg Asp Tyr Lys Lys Lys Gly Trp Gly Phe Asn
            180                 185                 190
Gln Phe Cys Lys Ile Asp Gly Ala Phe Tyr Thr Thr Glu Tyr Thr Tyr
        195                 200                 205
Asn Asp Lys Thr Lys Gln Trp His Pro His Ile His Ile Phe Ala Leu
    210                 215                 220
Leu Asn Glu Trp Ile Asp Gln Glu Glu Leu Ala Glu Thr Trp His Asp
225                 230                 235                 240
Ile Thr Leu Asp Ser Tyr Ile Val Asp Ile Arg Arg Val Lys Lys Thr
                245                 250                 255
Lys Glu His Gly Tyr Ser Lys Ala Val Ala Glu Val Cys Lys Tyr Ala
            260                 265                 270
Leu Lys Phe Gly Asp Leu Ser Val Glu Asn Thr Trp Asp Ala Phe Leu
        275                 280                 285
Thr Leu Lys Gly Lys Arg Leu Thr Gly Ser Phe Gly Ser Met His Gly
    290                 295                 300
Val Lys Ile Pro Glu Lys Phe Thr Asp Asp Met Pro Leu Glu Glu Leu
305                 310                 315                 320
Pro Tyr Leu Glu Met Phe Tyr Arg Phe Val Phe Gly Pro Lys Ser Tyr
                325                 330                 335
Tyr Asn Leu Glu Ile Thr Lys Asp Val Lys Pro Asn Lys Arg Asn Glu
            340                 345                 350

Glu

<210> 352
<211> 70
<212> PRT
<213> Artificial Sequence

<220>
<223> orf

<400> 352
Met Ile Ile Glu Lys Tyr Ala Asn Lys Cys Val Ile Glu Thr Met Phe
1               5                   10                  15
Gln Cys Phe Asn Val Ser Met Phe Gln Cys Phe Asn Val Ser Met Phe
            20                  25                  30
Gln Cys Phe Asn Val Ser Met Phe Gln Cys Phe Asn Val Thr Gln Pro
        35                  40                  45
Ser Gly Ile Ala Lys Ser Ser Arg Leu Glu Ile Gln Gly Glu Leu Val
    50                  55                  60
Ile Arg Glu Gly Arg Phe
65                  70

<210> 353
<211> 61
<212> PRT
<213> Artificial Sequence

<220>
<223> orf

<400> 353
Met Phe Arg Ser Ser Lys Asp Trp Arg Ser Ser Val Gln Leu Ser Ile
1               5                   10                  15
Arg Cys Phe Asn Ser Ser Asn Ser Phe Ile Ser Ile Ile Leu Leu Trp
            20                  25                  30
Gly Ala Leu Pro Asn Leu Lys Gly Leu Val Lys Cys Ala Leu Trp Gly
        35                  40                  45
Leu Arg Gly Arg Thr Pro Tyr Lys Ser Cys Val Tyr Arg
    50                  55                  60

<210> 354
<211> 58
<212> PRT
<213> Artificial Sequence

<220>
<223> orf

<400> 354
Met Tyr Asn Pro Ile Met Gly Ser Thr Ala Glu Pro Glu Arg Phe Gly
1               5                   10                  15
Lys Val Arg Pro Leu Gly Ser Lys Gly Ser Asn Pro Leu Gln Val Leu
            20                  25                  30
Cys Ile Gln Val Gly Leu Leu Ile Arg Leu Ser Asp Asp Asp Ala Arg
        35                  40                  45
Ser Arg Asn Val Thr Ser Gln Thr Thr Asp
    50                  55

<210> 355

<211> 54
<212> PRT
<213> Artificial Sequence

<220>
<223> orf

<400> 355
Met Ser Ser Val Asn Phe Ser Gly Ile Leu Thr Pro Cys Ile Glu Pro
1               5                   10                  15
Lys Glu Pro Val Arg Arg Leu Pro Leu Ser Val Lys Asn Ala Ser Gln
            20                  25                  30
Val Phe Ser Thr Asp Arg Ser Pro Asn Leu Arg Ala Tyr Leu His Thr
        35                  40                  45
Ser Ala Thr Ala Leu Leu
    50

<210> 356
<211> 53
<212> PRT
<213> Artificial Sequence

<220>
<223> orf

<400> 356
Met Ala Lys Leu Lys Ser Val Phe Thr Tyr Leu Ser Asn Ser Phe Ala
1               5                   10                  15
Ile Ala Val Phe Phe Gly Phe Phe Asn Ser Thr Asn Ile Asp Asn Ile
            20                  25                  30
Arg Ile Lys Gly Asp Ile Val Pro Ser Phe Cys Lys Phe Phe Leu Val
        35                  40                  45
Tyr Pro Phe Ile Glu
    50

<210> 357
<211> 186
<212> DNA
<213> Artificial Sequence

<220>
<223> orf

<400> 357
ataatcacct tacaagctca tttagaacgc ttctatcgcg ttatagggat tacaagaaaa      60

agggttgggg gtttaatcaa ttctgtaaga ttgatggcgc tttctatacc actgaataca     120

cttacaacga taaaactaag caatggcatc cgcatattca tattttcgct ttactcaatg     180

aatgga                                                                186

<210> 358
<211> 162
<212> DNA
<213> Artificial Sequence

<220>

<223> orf

<400> 358
ttgcagaaac ttggcacgat atcacccttg attcttatat tgtcgatatt cgtagagtta      60

aaaaaaccaa agaacacggc tatagcaaag ctgttgctga ggtatgtaaa tacgctctta     120

agtttggcga tctatcagtt gaaaacactt gggacgcatt tt                        162


<210> 359
<211> 927
<212> DNA
<213> Artificial Sequence


<220>
<223> orf

<400> 359
atatcacgtt ttggactttt gaaacataga gcgaaactac aagaacaata tttgtggacg      60

caagttgatt tcaaatctga aggcgaagac gatctatcct ataaggctct taaggctgca     120

acaaaattaa aaggttgtgg tcagtttctt atattcagaa attactacac catcgaccaa     180

gttaagctcg aaaaattcca cgtatgcgga cagcatttgc tatgccccat gtgtgcaggt     240

attcgcgctg cacgttcaat gaaacgctat ttagaccgta tccatgaatt aatgcgtcag     300

aatccgcgtt taaagcccgt cctgatcact ttaacggtca agaatgggga agacctagaa     360

gaacgctata atcaccttac aagctcattt agaacgcttc tatcgcgtta tagggattac     420

aagaaaaagg gttgggggtt taatcaattc tgtaagattg atggcgcttt ctataccact     480

gaatacactt acaacgataa aactaagcaa tggcatccgc atattcatat tttcgcttta     540

ctcaatgaat ggatagacca agaagaactt gcagaaactt ggcacgatat cacccttgat     600

tcttatattg tcgatattcg tagagttaaa aaaaccaaag aacacggcta tagcaaagct     660

gttgctgagg tatgtaaata cgctcttaag tttggcgatc tatcagttga aaacacttgg     720

gacgcatttt taacacttaa gggtaaacgc cttacaggtt ctttcggttc tatgcacggt     780

gtcaaaattc ctgaaaaatt cactgatgat atgccactgg aggagctgcc ttacttagag     840

atgttctacc gttttgtatt tggtccgaaa tcatattaca acttagaaat aacaaaagac     900

gttaagccga acaaaaggaa tgaggag                                         927


<210> 360
<400> 360
000

<210> 361
<211> 168
<212> DNA
<213> Artificial Sequence

<220>
<223> orf

<400> 361
attatagata tgaacgaatt actagaattg aaacaccgta ttgataactg tactgatgat      60

ctccaatctt tcgaacttcg aaagatttat gacaagctca ttattgatct atggcaatct     120

gaaaaaatta ctttgtcaga aatgcagtct atggctctcc ctctccag                  168


<210> 362
<211> 168
<212> DNA
<213> Artificial Sequence


<220>
<223> orf

<400> 362
attatagata tgaacgaatt actagaattg aaacaccgta ttgataactg tactgatgat      60

ctccaatctt tcgaacttcg aaagatttat gacaagctca ttattgatct atggcaatct     120

gaaaaaatta ctttgtcaga aatgcagtct atggctctcc ctctccag                  168


<210> 363
<211> 156
<212> DNA
<213> Artificial Sequence


<220>
<223> orf

<400> 363
ttgataactg tactgatgat ctccaatctt tcgaacttcg aaagatttat gacaagctca      60

ttattgatct atggcaatct gaaaaaatta ctttgtcaga aatgcagtct atggctctcc     120

ctctccagta gcagagggcg cggagtgcgc ccgaac                               156


<210> 364
<211> 306
<212> DNA
<213> Artificial Sequence


<220>
<223> orf

<400> 364
ataggggctg atatgccaaa tccacttgat gtagtccttg gaaatatcct tggttcagaa      60

caaacccgac ctgatcaaaa ggctgtttcc tttcgtattg attttaaaaa ctatgtgaag     120

ctgtctgctt tacaaagcat tacacctaaa ggtgttagcc gtaatcagct tttaaatgat     180

cttttagctg ttgctcttga tcaagttgaa tcgtccttgc ctgatggtgc ttcggatgct     240

tatgaacatg cacttgtaga aaatgaagaa ggtttaacta tgcttttaga aacggaaggt 300

cattta 306

<210> 365
<211> 291
<212> DNA
<213> Artificial Sequence

<220>
<223> orf

<400> 365
atgattaagt tagaaggaat tgttttaaac gtatttaccc agcaaggcgg taaagataaa 60

aaaacaggtg atcaatttgc tgaccgtgac aaggtgcaaa ttttaggtgc tatggatctg 120

cctaatggtg acgtaaaaaa cgaactattt acgttatctg tagaaaacta tcgggacttt 180

aaagacttcc taaataagaa aatctgtatt gctgttggtg caatggcaag cggtcgaaat 240

gttatttttt acgttgctaa aggcgcacag cctgttttag cagaaacaat g 291

<210> 366
<211> 1059
<212> DNA
<213> Artificial Sequence

<220>
<223> orf

<400> 366
ttgatactat taactagagt ggggatgaaa ttgactaaaa aagacaataa aaaagcccat 60

aattctgaca ctttggcggg tgatgaatta caggctttgc aatctgctaa ggcagatcaa 120

cacagggata gaatatcacg ttttggactt ttgaaacata gagcgaaact acaagaacaa 180

tatttgtgga cgcaagttga tttcaaatct gaaggcgaag acgatctatc ctataaggct 240

cttaaggctg caacaaaatt aaaaggttgt ggtcagtttc ttatattcag aaattactac 300

accatcgacc aagttaagct cgaaaaattc cacgtatgcg gacagcattt gctatgcccc 360

atgtgtgcag gtattcgcgc tgcacgttca atgaaacgct atttagaccg tatccatgaa 420

ttaatgcgtc agaatccgcg tttaaagccc gtcctgatca ctttaacggt caagaatggg 480

gaagacctag aagaacgcta taatcacctt acaagctcat ttagaacgct tctatcgcgt 540

tatagggatt acaagaaaaa gggttggggg tttaatcaat tctgtaagat tgatggcgct 600

ttctatacca ctgaatacac ttacaacgat aaaactaagc aatggcatcc gcatattcat 660

attttcgctt tactcaatga atggatagac caagaagaac ttgcagaaac ttggcacgat 720

atcacccttg attcttatat tgtcgatatt cgtagagtta aaaaaaccaa agaacacggc 780

tatagcaaag ctgttgctga ggtatgtaaa tacgctctta agtttggcga tctatcagtt 840

gaaaacactt gggacgcatt tttaacactt aagggtaaac gccttacagg ttctttcggt 900

tctatgcacg gtgtcaaaat tcctgaaaaa ttcactgatg atatgccact ggaggagctg 960

ccttacttag agatgttcta ccgttttgta tttggtccga aatcatatta caacttagaa 1020

ataacaaaag acgttaagcc gaacaaaagg aatgaggag 1059

<210> 367
<211> 210
<212> DNA
<213> Artificial Sequence

<220>
<223> orf

<400> 367
atcattattg aaaaatatgc aaataaatgt gtcattgaaa caatgtttca atgtttcaat 60

gtttcaatgt ttcaatgttt caatgtttca atgtttcaat gtttcaatgt ttcaatgttt 120

caatgtttca atgtgacgca acccagcggg attgctaaaa gctcccgctt ggaaatccaa 180

ggggagcttg taatcaggga aggaaggttt 210

<210> 368
<211> 183
<212> DNA
<213> Artificial Sequence

<220>
<223> orf

<400> 368
atctttcgaa gttcgaaaga ttggagatca tcagtacagt tatcaatacg gtgtttcaat 60

tctagtaatt cgttcatatc tataatccta ttatggggag cactgccgaa cctgaaaggt 120

ttggtaaagt gcgccctttg gggtctaagg ggtcgaaccc cctacaagtc ctgtgtatac 180

agg 183

<210> 369
<211> 174
<212> DNA
<213> Artificial Sequence

<220>
<223> orf

<400> 369
atctataatc ctattatggg gagcactgcc gaacctgaaa ggtttggtaa agtgcgccct 60

ttggggtcta aggggtcgaa ccccctacaa gtcctgtgta tacaggtagg cttgcttatt 120

cgattgtctg atgatgatgc acgttcacgg aacgtcacat cacagacaac cgat 174

<210> 370
<211> 162
<212> DNA
<213> Artificial Sequence

<220>
<223> orf

<400> 370
atatcatcag tgaatttttc aggaattttg acaccgtgca tagaaccgaa agaacctgta      60

aggcgtttac ccttaagtgt taaaaatgcg tcccaagtgt tttcaactga tagatcgcca     120

aacttaagag cgtatttaca tacctcagca acagctttgc ta                        162

<210> 371
<211> 159
<212> DNA
<213> Artificial Sequence

<220>
<223> orf

<400> 371
atcgccaaac ttaagagcgt atttacatac ctcagcaaca gctttgctat agccgtgttc      60

tttggttttt ttaactctac gaatatcgac aatataagaa tcaagggtga tatcgtgcca     120

agtttctgca agttcttctt ggtctatcca ttcattgag                            159

<210> 372
<211> 2365
<212> DNA
<213> Artificial Sequence

<220>
<223> isolate

<400> 372
caacacaggg atagaataac acgttttgcg actttgaaac atagggctaa gaatcaggaa      60

aattatttat ttaccctggc taagtttaaa gagaactatg agaaagacgt taaaaacgaa     120

gaatctatca aggctctaaa atctgctcag aaattgaatg aatgcggaaa ctatctgcta     180

ttcaaaaatt tttacacaat aggcgaagtt aaactctcca agctccgcac ctgcggacag     240

catttgcttt gccctttctg tgctgccatt cgtgcttctc gtgctattca aaaatacgtt     300

gaacgtattg atcaagtcct gcaagaaaat cgcaagctca agcccgttct aatcacgctc     360

accgttaaaa acggttctaa cctagcggaa cgctctgaac accttatgaa gtcctttagg     420

acgcttctag agcgtcgtag ggactatgaa aagaaaggtc gaggttttaa tgagttctgt     480

aaggttcaag gggctatgta ctcctatgaa aatacattca atgaaaaaac aggcgaatgg      540

catccgcata ttcatatgtt cgctttggtt ggtcaatgga ttgatcagca agagttttcg 600

gaatattggc atagccttac tggggactcg atggttgtcg atgtccgcag ggcaagaaaa 660

gaaaaaggtc acggttatag caaagcggct gccgaagtct gtaagtatgc tttgaagttt 720

ggtgatctgt ccgttgaaaa gacttgggaa gcatttaagg ttcttaaggg aaagcgttta 780

acaggttctt ttggtctgct atggggtgtc aaaatccctg acacaatgac agacgatatg 840

ccatcagaag acttgccata tctcgaaatg ctgtacaagt ttgcctacag taaaaagtct 900

tactacgact tactaatcac aaggcacgta gagccaaaag aaactgaccg aatgaggagt 960

gaggaggagg gaccgaccga ccgacacgac cgaatgaggg atgttgattt ggcgatgacg 1020

gacgaagcga ggacgttgcc ggtgcgaggg cacgcagtca cggaacgacc acagcgagga 1080

cgtaaaaaag cccactggag aatcccgcct aaaacccgtg taagggttcg ggaacgaatc 1140

cgaagatggg acggaattat gtataacatt gatcttttcc catatgttga acgtaggcta 1200

ttggctttca tcgggtaact tacgaacctt gagtgaaacg aaaggtgtag taagtacacc 1260

ttctccagta gccgagcgag tgtctacgag cgacgactcc gaaattagaa tttcaaaacc 1320

ttcctttccc tgattacaag ctcccatctg attttagatc gggagctttt agccatcccg 1380

aatgggtggc gacgcattga cattctcata agaatctata atattctaat aacaatctaa 1440

tagaatctaa atggaatcta aaaatgttat tcaaatctct tggtattgct accgctacaa 1500

atacagcttc taattctgag gactctaggc ctgtgactat tcgtttaact gatagtgaag 1560

ttcaacttta cgatgctgtt gctaagtcta tgggcttaac tcgacaagac ttcttagctc 1620

acttaatccg caataacttc aagcaggctt taactgattt cttaaccggt tatacccaat 1680

catctccgtc agtttctttg tgtgaattaa ttaattcaaa tactgacagt gacgaagtta 1740

aacagaaggt ttcttggtta ctacgttcta tctcacaaga attaatggaa gaagacgaga 1800

aagaaattca cgaacacatt gagaacgagc gtttctatca attcgctact cctcgtaaag 1860

gcatttttgc tgaaacgcct aagccagagg agaaataaca atgattaagc tggaaggaac 1920

ggtattgaat gtctttactc agcaaggcgg taaagacaaa aaaacaggtg atcaattttc 1980

tgatcgtgac aaggtacaaa ttttaggtgc tatggatctg ccaaatggtg acgtaaaaaa 2040

tgaacttttt acgttatcgg tagagaatta tcgggacttt aaagactacc taaatcagaa 2100

aatttcaatt gctgttggtg caatggcaag tggtcgtaat gtgatttttt atgttgctaa 2160

aggtgcaaaa cctgttttag cggaaacaat gtgaaacgtg aaaaaaaaca gtgagttagc 2220

gaagctactc gctgtttttt cttttacttg atactattga ttaaagtggg gacaaaaatt 2280

gcttaaaaaa gacaataaaa aagcccatat ttcggacact ttggctggtg atgaaataca 2340

ggctttgcaa tctgctaagg cagat 2365

<210> 373
<211> 89
<212> PRT
<213> Artificial Sequence

<220>
<223> orf

<400> 373
Met Lys Arg Lys Val Glu Val Leu Met Ser Ser Val Arg Phe Lys Gly
1               5                   10                  15
Leu Cys Thr Pro Met Lys Ile His Ser Met Lys Lys Gln Ala Asn Gly
            20                  25                  30
Ile Arg Ile Phe Ile Cys Ser Leu Trp Leu Val Asn Gly Leu Ile Ser
        35                  40                  45
Lys Ser Phe Arg Asn Ile Gly Ile Ala Leu Leu Gly Thr Arg Trp Leu
    50                  55                  60
Ser Met Ser Ala Gly Gln Glu Lys Lys Lys Val Thr Val Ile Ala Lys
65                  70                  75                  80
Arg Leu Pro Lys Ser Val Ser Met Leu
                85

<210> 374
<211> 400
<212> PRT
<213> Artificial Sequence

<220>
<223> orf

<400> 374
Met Thr Arg Phe Ala Thr Leu Lys His Arg Ala Lys Asn Gln Glu Asn
1               5                   10                  15
Tyr Leu Phe Thr Leu Ala Lys Phe Lys Glu Asn Tyr Glu Lys Asp Val
            20                  25                  30
Lys Asn Glu Glu Ser Ile Lys Ala Leu Lys Ser Ala Gln Lys Leu Asn
        35                  40                  45
Glu Cys Gly Asn Tyr Leu Leu Phe Lys Asn Phe Tyr Thr Ile Gly Glu
    50                  55                  60
Val Lys Leu Ser Lys Leu Arg Thr Cys Gly Gln His Leu Leu Cys Pro
65                  70                  75                  80
Phe Cys Ala Ala Ile Arg Ala Ser Arg Ala Ile Gln Lys Tyr Val Glu
                85                  90                  95
Arg Ile Asp Gln Val Leu Gln Glu Asn Arg Lys Leu Lys Pro Val Leu
            100                 105                 110
Ile Thr Leu Thr Val Lys Asn Gly Ser Asn Leu Ala Glu Arg Ser Glu
        115                 120                 125
His Leu Met Lys Ser Phe Arg Thr Leu Leu Glu Arg Arg Arg Asp Tyr
    130                 135                 140
Glu Lys Lys Gly Arg Gly Phe Asn Glu Phe Cys Lys Val Gln Gly Ala
145                 150                 155                 160
Met Tyr Ser Tyr Glu Asn Thr Phe Asn Glu Lys Thr Gly Glu Trp His
                165                 170                 175
Pro His Ile His Met Phe Ala Leu Val Gly Gln Trp Ile Asp Gln Gln
            180                 185                 190
Glu Phe Ser Glu Tyr Trp His Ser Leu Thr Gly Asp Ser Met Val Val
        195                 200                 205
Asp Val Arg Arg Ala Arg Lys Glu Lys Gly His Gly Tyr Ser Lys Ala
    210                 215                 220
Ala Ala Glu Val Cys Lys Tyr Ala Leu Lys Phe Gly Asp Leu Ser Val

225                 230                 235                 240
Glu Lys Thr Trp Glu Ala Phe Lys Val Leu Lys Gly Lys Arg Leu Thr
                245                 250                 255
Gly Ser Phe Gly Leu Leu Trp Gly Val Lys Ile Pro Asp Thr Met Thr
            260                 265                 270
Asp Asp Met Pro Ser Glu Asp Leu Pro Tyr Leu Glu Met Leu Tyr Lys
        275                 280                 285
Phe Ala Tyr Ser Lys Lys Ser Tyr Tyr Asp Leu Leu Ile Thr Arg His
    290                 295                 300
Val Glu Pro Lys Glu Thr Asp Arg Met Arg Ser Glu Glu Glu Gly Pro
305                 310                 315                 320
Thr Asp Arg His Asp Arg Met Arg Asp Val Asp Leu Ala Met Thr Asp
                325                 330                 335
Glu Ala Arg Thr Leu Pro Val Arg Gly His Ala Val Thr Glu Arg Pro
            340                 345                 350
Gln Arg Gly Arg Lys Lys Ala His Trp Arg Ile Pro Pro Lys Thr Arg
        355                 360                 365
Val Arg Val Arg Glu Arg Ile Arg Arg Trp Asp Gly Ile Met Tyr Asn
    370                 375                 380
Ile Asp Leu Phe Pro Tyr Val Glu Arg Arg Leu Leu Ala Phe Ile Gly
385                 390                 395                 400

<210> 375
<211> 60
<212> PRT
<213> Artificial Sequence

<220>
<223> orf

<400> 375
Met Glu Leu Cys Ile Thr Leu Ile Phe Ser His Met Leu Asn Val Gly
1               5                   10                  15
Tyr Trp Leu Ser Ser Gly Asn Leu Arg Thr Leu Ser Glu Thr Lys Gly
            20                  25                  30
Val Val Ser Thr Pro Ser Pro Val Ala Glu Arg Val Ser Thr Ser Asp
        35                  40                  45
Asp Ser Glu Ile Arg Ile Ser Lys Pro Ser Phe Pro
    50                  55                  60

<210> 376
<211> 152
<212> PRT
<213> Artificial Sequence

<220>
<223> orf

<400> 376
Met Glu Ser Lys Trp Asn Leu Lys Met Leu Phe Lys Ser Leu Gly Ile
1               5                   10                  15
Ala Thr Ala Thr Asn Thr Ala Ser Asn Ser Glu Asp Ser Arg Pro Val
            20                  25                  30
Thr Ile Arg Leu Thr Asp Ser Glu Val Gln Leu Tyr Asp Ala Val Ala
        35                  40                  45
Lys Ser Met Gly Leu Thr Arg Gln Asp Phe Leu Ala His Leu Ile Arg
    50                  55                  60
Asn Asn Phe Lys Gln Ala Leu Thr Asp Phe Leu Thr Gly Tyr Thr Gln
65                  70                  75                  80

Ser Ser Pro Ser Val Ser Leu Cys Glu Leu Ile Asn Ser Asn Thr Asp
                85                  90                  95
Ser Asp Glu Val Lys Gln Lys Val Ser Trp Leu Leu Arg Ser Ile Ser
            100                 105                 110
Gln Glu Leu Met Glu Glu Asp Glu Lys Glu Ile His Glu His Ile Glu
        115                 120                 125
Asn Glu Arg Phe Tyr Gln Phe Ala Thr Pro Arg Lys Gly Ile Phe Ala
    130                 135                 140
Glu Thr Pro Lys Pro Glu Glu Lys
145                 150

<210> 377
<211> 99
<212> PRT
<213> Artificial Sequence

<220>
<223> orf

<400> 377
Met Thr Met Ile Lys Leu Glu Gly Thr Val Leu Asn Val Phe Thr Gln
1               5                   10                  15
Gln Gly Gly Lys Asp Lys Lys Thr Gly Asp Gln Phe Ser Asp Arg Asp
            20                  25                  30
Lys Val Gln Ile Leu Gly Ala Met Asp Leu Pro Asn Gly Asp Val Lys
        35                  40                  45
Asn Glu Leu Phe Thr Leu Ser Val Glu Asn Tyr Arg Asp Phe Lys Asp
    50                  55                  60
Tyr Leu Asn Gln Lys Ile Ser Ile Ala Val Gly Ala Met Ala Ser Gly
65                  70                  75                  80
Arg Asn Val Ile Phe Tyr Val Ala Lys Gly Ala Lys Pro Val Leu Ala
                85                  90                  95
Glu Thr Met

<210> 378
<211> 434
<212> PRT
<213> Artificial Sequence

<220>
<223> orf

<400> 378
Met Leu Lys Lys Asp Asn Lys Lys Ala His Ile Ser Asp Thr Leu Ala
1               5                   10                  15
Gly Asp Glu Ile Gln Ala Leu Gln Ser Ala Lys Ala Asp Gln His Arg
            20                  25                  30
Asp Arg Ile Thr Arg Phe Ala Thr Leu Lys His Arg Ala Lys Asn Gln
        35                  40                  45
Glu Asn Tyr Leu Phe Thr Leu Ala Lys Phe Lys Glu Asn Tyr Glu Lys
    50                  55                  60
Asp Val Lys Asn Glu Glu Ser Ile Lys Ala Leu Lys Ser Ala Gln Lys
65                  70                  75                  80
Leu Asn Glu Cys Gly Asn Tyr Leu Leu Phe Lys Asn Phe Tyr Thr Ile
                85                  90                  95
Gly Glu Val Lys Leu Ser Lys Leu Arg Thr Cys Gly Gln His Leu Leu
            100                 105                 110
Cys Pro Phe Cys Ala Ala Ile Arg Ala Ser Arg Ala Ile Gln Lys Tyr
        115                 120                 125
Val Glu Arg Ile Asp Gln Val Leu Gln Glu Asn Arg Lys Leu Lys Pro

130                 135                 140
Val Leu Ile Thr Leu Thr Val Lys Asn Gly Ser Asn Leu Ala Glu Arg
145                 150                 155                 160
Ser Glu His Leu Met Lys Ser Phe Arg Thr Leu Leu Glu Arg Arg Arg
                165                 170                 175
Asp Tyr Glu Lys Lys Gly Arg Gly Phe Asn Glu Phe Cys Lys Val Gln
            180                 185                 190
Gly Ala Met Tyr Ser Tyr Glu Asn Thr Phe Asn Glu Lys Thr Gly Glu
        195                 200                 205
Trp His Pro His Ile His Met Phe Ala Leu Val Gly Gln Trp Ile Asp
    210                 215                 220
Gln Gln Glu Phe Ser Glu Tyr Trp His Ser Leu Thr Gly Asp Ser Met
225                 230                 235                 240
Val Val Asp Val Arg Arg Ala Arg Lys Glu Lys Gly His Gly Tyr Ser
                245                 250                 255
Lys Ala Ala Ala Glu Val Cys Lys Tyr Ala Leu Lys Phe Gly Asp Leu
            260                 265                 270
Ser Val Glu Lys Thr Trp Glu Ala Phe Lys Val Leu Lys Gly Lys Arg
        275                 280                 285
Leu Thr Gly Ser Phe Gly Leu Leu Trp Gly Val Lys Ile Pro Asp Thr
    290                 295                 300
Met Thr Asp Asp Met Pro Ser Glu Asp Leu Pro Tyr Leu Glu Met Leu
305                 310                 315                 320
Tyr Lys Phe Ala Tyr Ser Lys Lys Ser Tyr Tyr Asp Leu Leu Ile Thr
                325                 330                 335
Arg His Val Glu Pro Lys Glu Thr Asp Arg Met Arg Ser Glu Glu Glu
            340                 345                 350
Gly Pro Thr Asp Arg His Asp Arg Met Arg Asp Val Asp Leu Ala Met
        355                 360                 365
Thr Asp Glu Ala Arg Thr Leu Pro Val Arg Gly His Ala Val Thr Glu
    370                 375                 380
Arg Pro Gln Arg Gly Arg Lys Lys Ala His Trp Arg Ile Pro Pro Lys
385                 390                 395                 400
Thr Arg Val Arg Val Arg Glu Arg Ile Arg Arg Trp Asp Gly Ile Met
                405                 410                 415
Tyr Asn Ile Asp Leu Phe Pro Tyr Val Glu Arg Arg Leu Leu Ala Phe
            420                 425                 430
Ile Gly


<210> 379
<211> 53
<212> PRT
<213> Artificial Sequence


<220>
<223> orf

<400> 379
Met Tyr Leu Val Thr Ile Arg Lys Leu Ile Thr Cys Phe Phe Val Phe
1               5                   10                  15
Thr Ala Leu Leu Ser Lys Asp Ile Gln Tyr Arg Ser Phe Gln Leu Asn
            20                  25                  30
His Cys Tyr Phe Ser Ser Gly Leu Gly Val Ser Ala Lys Met Pro Leu
        35                  40                  45
Arg Gly Val Ala Asn
    50

<210> 380
<211> 93
<212> PRT
<213> Artificial Sequence

<220>
<223> orf

<400> 380
Met Phe Asn Ile Trp Glu Lys Ile Asn Val Ile His Asn Ser Val Pro
1               5                   10                  15
Ser Ser Asp Ser Phe Pro Asn Pro Tyr Thr Gly Phe Arg Arg Asp Ser
            20                  25                  30
Pro Val Gly Phe Phe Thr Ser Ser Leu Trp Ser Phe Arg Asp Cys Val
        35                  40                  45
Pro Ser His Arg Gln Arg Pro Arg Phe Val Arg His Arg Gln Ile Asn
    50                  55                  60
Ile Pro His Ser Val Val Ser Val Gly Arg Ser Leu Leu Leu Thr Pro
65                  70                  75                  80
His Ser Val Ser Phe Phe Trp Leu Tyr Val Pro Cys Asp
                85                  90

<210> 381
<211> 121
<212> PRT
<213> Artificial Sequence

<220>
<223> orf

<400> 381
Met Glu Lys Val Tyr Leu Leu His Leu Ser Phe His Ser Arg Phe Val
1               5                   10                  15
Ser Tyr Pro Met Lys Ala Asn Ser Leu Arg Ser Thr Tyr Gly Lys Arg
            20                  25                  30
Ser Met Leu Tyr Ile Ile Pro Ser His Leu Arg Ile Arg Ser Arg Thr
        35                  40                  45
Leu Thr Arg Val Leu Gly Gly Ile Leu Gln Trp Ala Phe Leu Arg Pro
    50                  55                  60
Arg Cys Gly Arg Ser Val Thr Ala Cys Pro Arg Thr Gly Asn Val Leu
65                  70                  75                  80
Ala Ser Ser Val Ile Ala Lys Ser Thr Ser Leu Ile Arg Ser Cys Arg
                85                  90                  95
Ser Val Gly Pro Ser Ser Ser Leu Leu Ile Arg Ser Val Ser Phe Gly
            100                 105                 110
Ser Thr Cys Leu Val Ile Ser Lys Ser
        115                 120

<210> 382
<211> 67
<212> PRT
<213> Artificial Sequence

<220>
<223> orf

<400> 382
Met Tyr Ser Ile Ser Arg Tyr Gly Lys Ser Ser Asp Gly Ile Ser Ser
1               5                   10                  15
Val Ile Val Ser Gly Ile Leu Thr Pro His Ser Arg Pro Lys Glu Pro
            20                  25                  30
Val Lys Arg Phe Pro Leu Arg Thr Leu Asn Ala Ser Gln Val Phe Ser
        35                  40                  45
Thr Asp Arg Ser Pro Asn Phe Lys Ala Tyr Leu Gln Thr Ser Ala Ala

50                  55                  60
Ala Leu Leu
65

<210> 383
<211> 116
<212> PRT
<213> Artificial Sequence

<220>
<223> orf

<400> 383
Met Leu Ser Leu Lys Asn Leu Lys Cys Phe Pro Ser Leu Phe Asn Gly
1               5                   10                  15
Gln Ile Thr Lys Leu Gln Ser Ile Leu Thr Asp Phe Gly Ser Arg Phe
            20                  25                  30
Ala Ile Thr Val Thr Phe Phe Phe Ser Cys Pro Ala Asp Ile Asp Asn
        35                  40                  45
His Arg Val Pro Ser Lys Ala Met Pro Ile Phe Arg Lys Leu Leu Leu
    50                  55                  60
Ile Asn Pro Leu Thr Asn Gln Ser Glu His Met Asn Met Arg Met Pro
65                  70                  75                  80
Phe Ala Cys Phe Phe Ile Glu Cys Ile Phe Ile Gly Val His Ser Pro
                85                  90                  95
Leu Asn Leu Thr Glu Leu Ile Lys Thr Ser Thr Phe Leu Phe Ile Val
            100                 105                 110
Pro Thr Thr Leu
        115

<210> 384
<211> 50
<212> PRT
<213> Artificial Sequence

<220>
<223> orf

<400> 384
Met Phe Leu Gln Asp Leu Ile Asn Thr Phe Asn Val Phe Leu Asn Ser
1               5                   10                  15
Thr Arg Ser Thr Asn Gly Ser Thr Glu Arg Ala Lys Gln Met Leu Ser
            20                  25                  30
Ala Gly Ala Glu Leu Gly Glu Phe Asn Phe Ala Tyr Cys Val Lys Ile
        35                  40                  45
Phe Glu
    50

<210> 385
<211> 56
<212> PRT
<213> Artificial Sequence

<220>
<223> orf

<400> 385
Met Ile Phe Leu Ile Leu Ser Pro Met Phe Gln Ser Arg Lys Thr Cys
1               5                   10                  15
Tyr Ser Ile Pro Val Leu Ile Cys Leu Ser Arg Leu Gln Ser Leu Tyr

                20                  25                  30
Phe Ile Thr Ser Gln Ser Val Arg Asn Met Gly Phe Phe Ile Val Phe
        35                  40                  45
Phe Lys Gln Phe Leu Ser Pro Leu
    50                  55

<210> 386
<211> 267
<212> DNA
<213> Artificial Sequence

<220>
<223> orf

<400> 386
atgaaaagaa aggtcgaggt tttaatgagt tctgtaaggt tcaaggggct atgtactcct      60

atgaaaatac attcaatgaa aaaacaggcg aatggcatcc gcatattcat atgttcgctt     120

tggttggtca atggattgat cagcaagagt tttcggaata ttggcatagc cttactgggg     180

actcgatggt tgtcgatgtc cgcagggcaa gaaaagaaaa aggtcacggt tatagcaaag     240

cggctgccga agtctgtaag tatgctt                                         267

<210> 387
<211> 1200
<212> DNA
<213> Artificial Sequence

<220>
<223> orf

<400> 387
ataacacgtt ttgcgacttt gaaacatagg gctaagaatc aggaaaatta tttatttacc      60

ctggctaagt ttaaagagaa ctatgagaaa gacgttaaaa acgaagaatc tatcaaggct     120

ctaaaatctg ctcagaaatt gaatgaatgc ggaaactatc tgctattcaa aaatttttac     180

acaataggcg aagttaaact ctccaagctc cgcacctgcg gacagcattt gctttgccct     240

ttctgtgctg ccattcgtgc ttctcgtgct attcaaaaat acgttgaacg tattgatcaa     300

gtcctgcaag aaaatcgcaa gctcaagccc gttctaatca cgctcaccgt taaaaacggt     360

tctaacctag cggaacgctc tgaacacctt atgaagtcct ttaggacgct tctagagcgt     420

cgtagggact atgaaaagaa aggtcgaggt tttaatgagt tctgtaaggt tcaaggggct     480

atgtactcct atgaaaatac attcaatgaa aaaacaggcg aatggcatcc gcatattcat     540

atgttcgctt tggttggtca atggattgat cagcaagagt tttcggaata ttggcatagc     600

cttactgggg actcgatggt tgtcgatgtc cgcagggcaa gaaaagaaaa aggtcacggt     660

tatagcaaag cggctgccga agtctgtaag tatgctttga agtttggtga tctgtccgtt     720

gaaaagactt gggaagcatt taaggttctt aagggaaagc gtttaacagg ttcttttggt     780

ctgctatggg gtgtcaaaat ccctgacaca atgacagacg atatgccatc agaagacttg 840

ccatatctcg aaatgctgta caagtttgcc tacagtaaaa agtcttacta cgacttacta 900

atcacaaggc acgtagagcc aaaagaaact gaccgaatga ggagtgagga ggagggaccg 960

accgaccgac acgaccgaat gagggatgtt gatttggcga tgacggacga agcgaggacg 1020

ttgccggtgc gagggcacgc agtcacggaa cgaccacagc gaggacgtaa aaaagcccac 1080

tggagaatcc cgcctaaaac ccgtgtaagg gttcgggaac gaatccgaag atgggacgga 1140

attatgtata acattgatct tttcccatat gttgaacgta ggctattggc tttcatcggg 1200

<210> 388
<211> 180
<212> DNA
<213> Artificial Sequence

<220>
<223> orf

<400> 388
acggaattat gtataacatt gatcttttcc catatgttga acgtaggcta ttggctttca 60

tcgggtaact tacgaacctt gagtgaaacg aaaggtgtag taagtacacc ttctccagta 120

gccgagcgag tgtctacgag cgacgactcc gaaattagaa tttcaaaacc ttcctttccc 180

<210> 389
<211> 456
<212> DNA
<213> Artificial Sequence

<220>
<223> orf

<400> 389
atagaatcta aatggaatct aaaaatgtta ttcaaatctc ttggtattgc taccgctaca 60

aatacagctt ctaattctga ggactctagg cctgtgacta ttcgtttaac tgatagtgaa 120

gttcaacttt acgatgctgt tgctaagtct atgggcttaa ctcgacaaga cttcttagct 180

cacttaatcc gcaataactt caagcaggct ttaactgatt tcttaaccgg ttatacccaa 240

tcatctccgt cagtttcttt gtgtgaatta attaattcaa atactgacag tgacgaagtt 300

aaacagaagg tttcttggtt actacgttct atctcacaag aattaatgga agaagacgag 360

aaagaaattc acgaacacat tgagaacgag cgtttctatc aattcgctac tcctcgtaaa 420

ggcatttttg ctgaaacgcc taagccagag gagaaa 456

<210> 390
<211> 297
<212> DNA
<213> Artificial Sequence

<220>
<223> orf

<400> 390
ataacaatga ttaagctgga aggaacggta ttgaatgtct ttactcagca aggcggtaaa      60

gacaaaaaaa caggtgatca attttctgat cgtgacaagg tacaaatttt aggtgctatg     120

gatctgccaa atggtgacgt aaaaaatgaa ctttttacgt tatcggtaga gaattatcgg     180

gactttaaag actacctaaa tcagaaaatt tcaattgctg ttggtgcaat ggcaagtggt     240

cgtaatgtga ttttttatgt tgctaaaggt gcaaaacctg ttttagcgga aacaatg        297

<210> 391
<211> 1302
<212> DNA
<213> Artificial Sequence

<220>
<223> orf

<400> 391
ttgcttaaaa aagacaataa aaaagcccat atttcggaca ctttggctgg tgatgaaata      60

caggctttgc aatctgctaa ggcagatcaa cacagggata gaataacacg ttttgcgact     120

ttgaaacata gggctaagaa tcaggaaaat tatttattta ccctggctaa gtttaaagag     180

aactatgaga aagacgttaa aaacgaagaa tctatcaagg ctctaaaatc tgctcagaaa     240

ttgaatgaat gcggaaacta tctgctattc aaaaattttt acacaatagg cgaagttaaa     300

ctctccaagc tccgcacctg cggacagcat ttgctttgcc ctttctgtgc tgccattcgt     360

gcttctcgtg ctattcaaaa atacgttgaa cgtattgatc aagtcctgca agaaaatcgc     420

aagctcaagc ccgttctaat cacgctcacc gttaaaaacg gttctaacct agcggaacgc     480

tctgaacacc ttatgaagtc ctttaggacg cttctagagc gtcgtaggga ctatgaaaag     540

aaaggtcgag gttttaatga gttctgtaag gttcaagggg ctatgtactc ctatgaaaat     600

acattcaatg aaaaaacagg cgaatggcat ccgcatattc atatgttcgc tttggttggt     660

caatggattg atcagcaaga gttttcggaa tattggcata gccttactgg ggactcgatg     720

gttgtcgatg tccgcagggc aagaaaagaa aaaggtcacg gttatagcaa agcggctgcc     780

gaagtctgta agtatgcttt gaagtttggt gatctgtccg ttgaaaagac ttgggaagca     840

tttaaggttc ttaagggaaa gcgtttaaca ggttcttttg gtctgctatg gggtgtcaaa     900

atccctgaca caatgacaga cgatatgcca tcagaagact tgccatatct cgaaatgctg     960

tacaagtttg cctacagtaa aaagtcttac tacgacttac taatcacaag gcacgtagag    1020

ccaaaagaaa ctgaccgaat gaggagtgag gaggagggac cgaccgaccg acacgaccga    1080

atgagggatg ttgatttggc gatgacggac gaagcgagga cgttgccggt gcgagggcac 1140

gcagtcacgg aacgaccaca gcgaggacgt aaaaaagccc actggagaat cccgcctaaa 1200

acccgtgtaa gggttcggga acgaatccga agatgggacg gaattatgta taacattgat 1260

cttttcccat atgttgaacg taggctattg gctttcatcg gg 1302

<210> 392
<211> 159
<212> DNA
<213> Artificial Sequence

<220>
<223> orf

<400> 392
ttgtaccttg tcacgatcag aaaattgatc acctgttttt ttgtctttac cgccttgctg 60

agtaaagaca ttcaataccg ttccttccag cttaatcatt gttatttctc ctctggctta 120

ggcgtttcag caaaaatgcc tttacgagga gtagcgaat 159

<210> 393
<211> 279
<212> DNA
<213> Artificial Sequence

<220>
<223> orf

<400> 393
acgttcaaca tatgggaaaa gatcaatgtt atacataatt ccgtcccatc ttcggattcg 60

ttcccgaacc cttacacggg ttttaggcgg gattctccag tgggcttttt tacgtcctcg 120

ctgtggtcgt tccgtgactg cgtgccctcg caccggcaac gtcctcgctt cgtccgtcat 180

cgccaaatca acatccctca ttcggtcgtg tcggtcggtc ggtccctcct cctcactcct 240

cattcggtca gtttcttttg gctctacgtg ccttgtgat 279

<210> 394
<211> 363
<212> DNA
<213> Artificial Sequence

<220>
<223> orf

<400> 394
ctggagaagg tgtacttact acacctttcg tttcactcaa ggttcgtaag ttacccgatg 60

aaagccaata gcctacgttc aacatatggg aaaagatcaa tgttatacat aattccgtcc 120

catcttcgga ttcgttcccg aacccttaca cgggttttag gcgggattct ccagtgggct 180

tttttacgtc ctcgctgtgg tcgttccgtg actgcgtgcc ctcgcaccgg caacgtcctc 240

gcttcgtccg tcatcgccaa atcaacatcc ctcattcggt cgtgtcggtc ggtcggtccc 300

tcctcctcac tcctcattcg gtcagtttct tttggctcta cgtgccttgt gattagtaag 360

tcg 363

<210> 395
<211> 201
<212> DNA
<213> Artificial Sequence

<220>
<223> orf

<400> 395
ttgtacagca tttcgagata tggcaagtct tctgatggca tatcgtctgt cattgtgtca 60

gggattttga caccccatag cagaccaaaa gaacctgtta aacgctttcc cttaagaacc 120

ttaaatgctt cccaagtctt ttcaacggac agatcaccaa acttcaaagc atacttacag 180

acttcggcag ccgctttgct a 201

<210> 396
<211> 348
<212> DNA
<213> Artificial Sequence

<220>
<223> orf

<400> 396
acgctttccc ttaagaacct taaatgcttc ccaagtcttt tcaacggaca gatcaccaaa 60

cttcaaagca tacttacaga cttcggcagc cgctttgcta taaccgtgac ctttttcttt 120

tcttgccctg cggacatcga caaccatcga gtccccagta aggctatgcc aatattccga 180

aaactcttgc tgatcaatcc attgaccaac caaagcgaac atatgaatat gcggatgcca 240

ttcgcctgtt ttttcattga atgtattttc ataggagtac atagcccctt gaaccttaca 300

gaactcatta aaacctcgac ctttcttttc atagtcccta cgacgctc 348

<210> 397
<211> 150
<212> DNA
<213> Artificial Sequence

<220>
<223> orf

<400> 397
attttcttgc aggacttgat caatacgttc aacgtatttt tgaatagcac gagaagcacg 60

aatggcagca cagaaagggc aaagcaaatg ctgtccgcag gtgcggagct tggagagttt 120

aacttcgcct attgtgtaaa aatttttgaa 150

<210> 398
<211> 168
<212> DNA
<213> Artificial Sequence

<220>
<223> orf

<400> 398
ataattttcc tgattcttag ccctatgttt caaagtcgca aaacgtgtta ttctatccct 60

gtgttgatct gccttagcag attgcaaagc ctgtatttca tcaccagcca aagtgtccga 120

aatatgggct tttttattgt cttttttaag caatttttgt ccccactt 168

<210> 399
<211> 2278
<212> DNA
<213> Artificial Sequence

<220>
<223> isolate

<400> 399
caacacaggg atagaataac acgttttggt attttgaaac acagatcgaa gcaacaagaa 60

aactttttgt ggactcttgc aaaattcaaa gaaaactacc ctcaagacct gccaagtgag 120

gaaacaacaa aagctttaaa gtctgctcaa aagttgcaag gctgtggcaa ttttttgctc 180

ttcaaaaatt tttacacaat agatcaaact aagttagcca aatttcatgt ctgtggtcag 240

catttgttat gtcctttttg tgctggtatc cgtgcttcaa agtctattca gaaatattca 300

gaacgtattg atgaagttct caagaaaaac cgtaagctaa aagtcgctct tttgacgctg 360

acggttaaaa acggttctga cctaggggaa cgtgctaatc atctcttaac gtcgtttaga 420

acgcttatca agcgcagaca ggactattta aaaagggtcg tgggtttaat gagttctgta 480

aaattgatgg tgcgatgtat tcctatgaaa atacctacaa tgaaaaaaca ggtgaatggc 540

atcctcatgt tcacatgctt gtacttctca atgattggat agatcaggaa gccttgggcg 600

aaacttggca tgaaattact ggtgattctt atatcgttga tgttcgaaaa gttaaaaaat 660

ctaaagaatt agggtatgcc aaagcagcag cagaggtttg taaatatgct cttaagttta 720

gtgatctgac tgttgaaaaa acttgggagg catttaaaac cctgaagggt aaaaacctta 780

gtggttcatt tggttccctg cgtggtgtca aaatccctaa aaaccttgct gacgatatgc 840

ctgaagatca ggatttacct tatctcgaaa tgctgtacaa gttcgtattt ggcaagcatt 900

cgcactatga cttagttatg actagacacg tagaagcgca ggcgggagag cgcgcggagt 960

taggaggagc tgcgacgacg cccgacgaga gatatagcgc gggtgcttca agtgtagacg 1020

atttgagtac ggaggcaggt ggagggctcg caccactgcg acgtacgaaa agaggacgca 1080

aaaaacagca ctggagaatc ccaccttcta cccgtgttcg tgttcggcaa agaatccgaa 1140

gatgggacgg gattatgtat aacattgatc ttttcccata tgtcgaacat aggctattgg 1200

ctttcattgg ttagtctgtt tcttgagaaa cgcggaagcg tgcattctga aaaactcacc 1260

acttttgaaa aaagtgtagt gagtacactt ctccagtagc cgaacgagtg tctacgagtg 1320

acgactccta aataagaatt tcaaaacctt ccttccttga ttacaagctc cccttggatt 1380

tccaagcggg agtttttagt catcccgaat gggtgacgac gcaatactat ttgatttttt 1440

catgattcaa ggtatgattc attgtgaatc acattcctat atatgaaaaa atcaggtgag 1500

aatatgcaaa ttgatgaacc aacaaacttc caagtattca tggcagaagt aaacaagaca 1560

gcaaagacag aaagtattgg tgtttaccat caagtacctt tccgtatggc tcgatggaac 1620

tttgcaagat tagaagggct tcgtaaccac atgggagaac ctcgaaacaa agttcttaat 1680

tcactaatcg aaattgcttt agatcaggta ttcgaacaac ttgagcatgg cagtaaagag 1740

atccgtcgct ctgttcttga ggaagtttcc aaagttctcg aatcaattga acatgatggt 1800

tctgggagct tagacaatga ttaatctaca aggaactcta atcaatgctt tccgtgtgga 1860

tggtggtaaa ggtaaagacg gcaaagaata tgaagcgcgt gacaaggtgc aaattcttgg 1920

ttcgttggaa ctcccaaacg gggaaattaa acacgaactt gttgacctaa cagttgagga 1980

ttctcgactg tttgaaccat tcaaaaatca ggttattagc atctcttgcg gtgctatggc 2040

tatcggtcgt aatgttgttt tttatgttcg taaaagcgca aaaccgatct tagcacagag 2100

catttgatat tcgttaaaaa acagtgagtt agcgaagcta ctcgctgttt tttctttctc 2160

ttgatactat taattaattc ggggacaaaa tttcatgaaa aatgacaata aaaaacccct 2220

gttatcggaa gctttggcgg gcgtagataa caagggtttg caatctgcta aggcagat 2278

<210> 400
<211> 188
<212> PRT
<213> Artificial Sequence

<220>
<223> orf

<400> 400
Met Thr Arg Phe Gly Ile Leu Lys His Arg Ser Lys Gln Gln Glu Asn
1               5                   10                  15
Phe Leu Trp Thr Leu Ala Lys Phe Lys Glu Asn Tyr Pro Gln Asp Leu
            20                  25                  30
Pro Ser Glu Glu Thr Thr Lys Ala Leu Lys Ser Ala Gln Lys Leu Gln
        35                  40                  45
Gly Cys Gly Asn Phe Leu Leu Phe Lys Asn Phe Tyr Thr Ile Asp Gln

50                  55                  60
Thr Lys Leu Ala Lys Phe His Val Cys Gly Gln His Leu Leu Cys Pro
65                  70                  75                  80
Phe Cys Ala Gly Ile Arg Ala Ser Lys Ser Ile Gln Lys Tyr Ser Glu
                85                  90                  95
Arg Ile Asp Glu Val Leu Lys Lys Asn Arg Lys Leu Lys Val Ala Leu
            100                 105                 110
Leu Thr Leu Thr Val Lys Asn Gly Ser Asp Leu Gly Glu Arg Ala Asn
        115                 120                 125
His Leu Leu Thr Ser Phe Arg Thr Leu Ile Lys Arg Arg Gln Asp Tyr
    130                 135                 140
Leu Lys Arg Val Val Gly Leu Met Ser Ser Val Lys Leu Met Val Arg
145                 150                 155                 160
Cys Ile Pro Met Lys Ile Pro Thr Met Lys Lys Gln Val Asn Gly Ile
                165                 170                 175
Leu Met Phe Thr Cys Leu Tyr Phe Ser Met Ile Gly
            180                 185

<210> 401
<211> 243
<212> PRT
<213> Artificial Sequence

<220>
<223> orf

<400> 401
Met Asp Gly Ala Met Tyr Ser Tyr Glu Asn Thr Tyr Asn Glu Lys Thr
1               5                   10                  15
Gly Glu Trp His Pro His Val His Met Leu Val Leu Leu Asn Asp Trp
            20                  25                  30
Ile Asp Gln Glu Ala Leu Gly Glu Thr Trp His Glu Ile Thr Gly Asp
        35                  40                  45
Ser Tyr Ile Val Asp Val Arg Lys Val Lys Lys Ser Lys Glu Leu Gly
    50                  55                  60
Tyr Ala Lys Ala Ala Ala Glu Val Cys Lys Tyr Ala Leu Lys Phe Ser
65                  70                  75                  80
Asp Leu Thr Val Glu Lys Thr Trp Glu Ala Phe Lys Thr Leu Lys Gly
                85                  90                  95
Lys Asn Leu Ser Gly Ser Phe Gly Ser Leu Arg Gly Val Lys Ile Pro
            100                 105                 110
Lys Asn Leu Ala Asp Asp Met Pro Glu Asp Gln Asp Leu Pro Tyr Leu
        115                 120                 125
Glu Met Leu Tyr Lys Phe Val Phe Gly Lys His Ser His Tyr Asp Leu
    130                 135                 140
Val Met Thr Arg His Val Glu Ala Gln Ala Gly Glu Arg Ala Glu Leu
145                 150                 155                 160
Gly Gly Ala Ala Thr Thr Pro Asp Glu Arg Tyr Ser Ala Gly Ala Ser
                165                 170                 175
Ser Val Asp Asp Leu Ser Thr Glu Ala Gly Gly Gly Leu Ala Pro Leu
            180                 185                 190
Arg Arg Thr Lys Arg Gly Arg Lys Lys Gln His Trp Arg Ile Pro Pro
        195                 200                 205
Ser Thr Arg Val Arg Val Arg Gln Arg Ile Arg Arg Trp Asp Gly Ile
    210                 215                 220
Met Tyr Asn Ile Asp Leu Phe Pro Tyr Val Glu His Arg Leu Leu Ala
225                 230                 235                 240
Phe Ile Gly

<210> 402
<211> 86

<212> PRT
<213> Artificial Sequence

<220>
<223> orf

<400> 402
Met Glu Gly Ser His His Cys Asp Val Arg Lys Glu Asp Ala Lys Asn
1               5                   10                  15
Ser Thr Gly Glu Ser His Leu Leu Pro Val Phe Val Phe Gly Lys Glu
            20                  25                  30
Ser Glu Asp Gly Thr Gly Leu Cys Ile Thr Leu Ile Phe Ser His Met
        35                  40                  45
Ser Asn Ile Gly Tyr Trp Leu Ser Leu Val Ser Leu Phe Leu Glu Lys
    50                  55                  60
Arg Gly Ser Val His Ser Glu Lys Leu Thr Thr Phe Glu Lys Ser Val
65                  70                  75                  80
Val Ser Thr Leu Leu Gln
                85

<210> 403
<211> 118
<212> PRT
<213> Artificial Sequence

<220>
<223> orf

<400> 403
Met Thr Phe Leu Tyr Met Lys Lys Ser Gly Glu Asn Met Gln Ile Asp
1               5                   10                  15
Glu Pro Thr Asn Phe Gln Val Phe Met Ala Glu Val Asn Lys Thr Ala
            20                  25                  30
Lys Thr Glu Ser Ile Gly Val Tyr His Gln Val Pro Phe Arg Met Ala
        35                  40                  45
Arg Trp Asn Phe Ala Arg Leu Glu Gly Leu Arg Asn His Met Gly Glu
    50                  55                  60
Pro Arg Asn Lys Val Leu Asn Ser Leu Ile Glu Ile Ala Leu Asp Gln
65                  70                  75                  80
Val Phe Glu Gln Leu Glu His Gly Ser Lys Glu Ile Arg Arg Ser Val
                85                  90                  95
Leu Glu Glu Val Ser Lys Val Leu Glu Ser Ile Glu His Asp Gly Ser
            100                 105                 110
Gly Ser Leu Asp Asn Asp
        115

<210> 404
<211> 96
<212> PRT
<213> Artificial Sequence

<220>
<223> orf

<400> 404
Met Ile Asn Leu Gln Gly Thr Leu Ile Asn Ala Phe Arg Val Asp Gly
1               5                   10                  15
Gly Lys Gly Lys Asp Gly Lys Glu Tyr Glu Ala Arg Asp Lys Val Gln
            20                  25                  30
Ile Leu Gly Ser Leu Glu Leu Pro Asn Gly Glu Ile Lys His Glu Leu

35                  40                  45
Val Asp Leu Thr Val Glu Asp Ser Arg Leu Phe Glu Pro Phe Lys Asn
    50                  55                  60
Gln Val Ile Ser Ile Ser Cys Gly Ala Met Ala Ile Gly Arg Asn Val
65                  70                  75                  80
Val Phe Tyr Val Arg Lys Ser Ala Lys Pro Ile Leu Ala Gln Ser Ile
                85                  90                  95

<210> 405
<211> 227
<212> PRT
<213> Artificial Sequence

<220>
<223> orf

<400> 405
Met Arg Gly Gln Asn Phe Met Lys Asn Asp Asn Lys Lys Pro Leu Leu
1               5                   10                  15
Ser Glu Ala Leu Ala Gly Val Asp Asn Lys Gly Leu Gln Ser Ala Lys
            20                  25                  30
Ala Asp Gln His Arg Asp Arg Ile Thr Arg Phe Gly Ile Leu Lys His
        35                  40                  45
Arg Ser Lys Gln Gln Glu Asn Phe Leu Trp Thr Leu Ala Lys Phe Lys
    50                  55                  60
Glu Asn Tyr Pro Gln Asp Leu Pro Ser Glu Glu Thr Thr Lys Ala Leu
65                  70                  75                  80
Lys Ser Ala Gln Lys Leu Gln Gly Cys Gly Asn Phe Leu Leu Phe Lys
                85                  90                  95
Asn Phe Tyr Thr Ile Asp Gln Thr Lys Leu Ala Lys Phe His Val Cys
            100                 105                 110
Gly Gln His Leu Leu Cys Pro Phe Cys Ala Gly Ile Arg Ala Ser Lys
        115                 120                 125
Ser Ile Gln Lys Tyr Ser Glu Arg Ile Asp Glu Val Leu Lys Lys Asn
    130                 135                 140
Arg Lys Leu Lys Val Ala Leu Leu Thr Leu Thr Val Lys Asn Gly Ser
145                 150                 155                 160
Asp Leu Gly Glu Arg Ala Asn His Leu Leu Thr Ser Phe Arg Thr Leu
                165                 170                 175
Ile Lys Arg Arg Gln Asp Tyr Leu Lys Arg Val Val Gly Leu Met Ser
            180                 185                 190
Ser Val Lys Leu Met Val Arg Cys Ile Pro Met Lys Ile Pro Thr Met
        195                 200                 205
Lys Lys Gln Val Asn Gly Ile Leu Met Phe Thr Cys Leu Tyr Phe Ser
    210                 215                 220
Met Ile Gly
225

<210> 406
<211> 64
<212> PRT
<213> Artificial Sequence

<220>
<223> orf

<400> 406
Met Asn Gly Ser Asn Ser Arg Glu Ser Ser Thr Val Arg Ser Thr Ser
1               5                   10                  15

Ser Cys Leu Ile Ser Pro Phe Gly Ser Ser Asn Glu Pro Arg Ile Cys
                20                  25                  30
Thr Leu Ser Arg Ala Ser Tyr Ser Leu Pro Ser Leu Pro Leu Pro Pro
            35                  40                  45
Ser Thr Arg Lys Ala Leu Ile Arg Val Pro Cys Arg Leu Ile Ile Val
    50                  55                  60

<210> 407
<211> 60
<212> PRT
<213> Artificial Sequence

<220>
<223> orf

<400> 407
Met Phe Asp Ile Trp Glu Lys Ile Asn Val Ile His Asn Pro Val Pro
1               5                   10                  15
Ser Ser Asp Ser Leu Pro Asn Thr Asn Thr Gly Arg Arg Trp Asp Ser
                20                  25                  30
Pro Val Leu Phe Phe Ala Ser Ser Phe Arg Thr Ser Gln Trp Cys Glu
            35                  40                  45
Pro Ser Thr Cys Leu Arg Thr Gln Ile Val Tyr Thr
    50                  55                  60

<210> 408
<211> 60
<212> PRT
<213> Artificial Sequence

<220>
<223> orf

<400> 408
Met Gly Phe Ser Ser Ala Val Phe Cys Val Leu Phe Ser Tyr Val Ala
1               5                   10                  15
Val Val Arg Ala Leu His Leu Pro Pro Tyr Ser Asn Arg Leu His Leu
                20                  25                  30
Lys His Pro Arg Tyr Ile Ser Arg Arg Ala Ser Ser Gln Leu Leu Leu
            35                  40                  45
Thr Pro Arg Ala Leu Pro Pro Ala Leu Leu Arg Val
    50                  55                  60

<210> 409
<211> 100
<212> PRT
<213> Artificial Sequence

<220>
<223> orf

<400> 409
Met Lys Ala Asn Ser Leu Cys Ser Thr Tyr Gly Lys Arg Ser Met Leu
1               5                   10                  15
Tyr Ile Ile Pro Ser His Leu Arg Ile Leu Cys Arg Thr Arg Thr Arg
                20                  25                  30
Val Glu Gly Gly Ile Leu Gln Cys Cys Phe Leu Arg Pro Leu Phe Val
            35                  40                  45

Arg Arg Ser Gly Ala Ser Pro Pro Pro Ala Ser Val Leu Lys Ser Ser
    50                  55                  60
Thr Leu Glu Ala Pro Ala Leu Tyr Leu Ser Ser Gly Val Val Ala Ala
65                  70                  75                  80
Pro Pro Asn Ser Ala Arg Ser Pro Ala Cys Ala Ser Thr Cys Leu Val
                85                  90                  95
Ile Thr Lys Ser
            100

<210> 410
<211> 69
<212> PRT
<213> Artificial Sequence

<220>
<223> orf

<400> 410
Met Ser Ser Ala Arg Phe Leu Gly Ile Leu Thr Pro Arg Arg Glu Pro
1               5                   10                  15
Asn Glu Pro Leu Arg Phe Leu Pro Phe Arg Val Leu Asn Ala Ser Gln
            20                  25                  30
Val Phe Ser Thr Val Arg Ser Leu Asn Leu Arg Ala Tyr Leu Gln Thr
        35                  40                  45
Ser Ala Ala Ala Leu Ala Tyr Pro Asn Ser Leu Asp Phe Leu Thr Phe
    50                  55                  60
Arg Thr Ser Thr Ile
65

<210> 411
<211> 65
<212> PRT
<213> Artificial Sequence

<220>
<223> orf

<400> 411
Met Asn Asp Ile Arg Ile Thr Ser Asn Phe Met Pro Ser Phe Ala Gln
1               5                   10                  15
Gly Phe Leu Ile Tyr Pro Ile Ile Glu Lys Tyr Lys His Val Asn Met
            20                  25                  30
Arg Met Pro Phe Thr Cys Phe Phe Ile Val Gly Ile Phe Ile Gly Ile
        35                  40                  45
His Arg Thr Ile Asn Phe Thr Glu Leu Ile Lys Pro Thr Thr Leu Phe
    50                  55                  60
Lys
65

<210> 412
<211> 53
<212> PRT
<213> Artificial Sequence

<220>
<223> orf

<400> 412
Met Arg Val Val Phe Phe Glu Phe Cys Lys Ser Pro Gln Lys Val Phe
1               5                   10                  15

Leu Leu Leu Arg Ser Val Phe Gln Asn Thr Lys Thr Cys Tyr Ser Ile
            20                  25                  30
Pro Val Leu Ile Cys Leu Ser Arg Leu Gln Thr Leu Val Ile Tyr Ala
        35                  40                  45
Arg Gln Ser Phe Arg
    50

<210> 413
<211> 564
<212> DNA
<213> Artificial Sequence


<220>
<223> orf

<400> 413
ataacacgtt ttggtatttt gaaacacaga tcgaagcaac aagaaaactt tttgtggact      60

cttgcaaaat tcaaagaaaa ctaccctcaa gacctgccaa gtgaggaaac aacaaaagct     120

ttaaagtctg ctcaaaagtt gcaaggctgt ggcaattttt tgctcttcaa aaatttttac     180

acaatagatc aaactaagtt agccaaattt catgtctgtg gtcagcattt gttatgtcct     240

ttttgtgctg gtatccgtgc ttcaaagtct attcagaaat attcagaacg tattgatgaa     300

gttctcaaga aaaaccgtaa gctaaaagtc gctcttttga cgctgacggt taaaaacggt     360

tctgacctag gggaacgtgc taatcatctc ttaacgtcgt ttagaacgct tatcaagcgc     420

agacaggact atttaaaaag ggtcgtgggt ttaatgagtt ctgtaaaatt gatggtgcga     480

tgtattccta tgaaaatacc tacaatgaaa aaacaggtga atggcatcct catgttcaca     540

tgcttgtact tctcaatgat tgga                                            564


<210> 414
<211> 729
<212> DNA
<213> Artificial Sequence


<220>
<223> orf

<400> 414
attgatggtg cgatgtattc ctatgaaaat acctacaatg aaaaaacagg tgaatggcat      60

cctcatgttc acatgcttgt acttctcaat gattggatag atcaggaagc cttgggcgaa     120

acttggcatg aaattactgg tgattcttat atcgttgatg ttcgaaaagt taaaaaatct     180

aaagaattag ggtatgccaa agcagcagca gaggtttgta aatatgctct taagtttagt     240

gatctgactg ttgaaaaaac ttgggaggca tttaaaaccc tgaagggtaa aaaccttagt     300

ggttcatttg gttccctgcg tggtgtcaaa atccctaaaa accttgctga cgatatgcct     360

gaagatcagg atttacctta tctcgaaatg ctgtacaagt tcgtatttgg caagcattcg     420

cactatgact tagttatgac tagacacgta gaagcgcagg cgggagagcg cgcggagtta     480

ggaggagctg cgacgacgcc cgacgagaga tatagcgcgg gtgcttcaag tgtagacgat 540

ttgagtacgg aggcaggtgg agggctcgca ccactgcgac gtacgaaaag aggacgcaaa 600

aaacagcact ggagaatccc accttctacc cgtgttcgtg ttcggcaaag aatccgaaga 660

tgggacggga ttatgtataa cattgatctt ttcccatatg tcgaacatag gctattggct 720

ttcattggt 729

<210> 415
<211> 258
<212> DNA
<213> Artificial Sequence

<220>
<223> orf

<400> 415
gtggagggct cgcaccactg cgacgtacga aaagaggacg caaaaaacag cactggagaa 60

tcccaccttc tacccgtgtt cgtgttcggc aaagaatccg aagatgggac gggattatgt 120

ataacattga tcttttccca tatgtcgaac ataggctatt ggctttcatt ggttagtctg 180

tttcttgaga aacgcggaag cgtgcattct gaaaaactca ccacttttga aaaaagtgta 240

gtgagtacac ttctccag 258

<210> 416
<211> 354
<212> DNA
<213> Artificial Sequence

<220>
<223> orf

<400> 416
atcacattcc tatatatgaa aaaatcaggt gagaatatgc aaattgatga accaacaaac 60

ttccaagtat tcatggcaga agtaaacaag acagcaaaga cagaaagtat tggtgtttac 120

catcaagtac ctttccgtat ggctcgatgg aactttgcaa gattagaagg gcttcgtaac 180

cacatgggag aacctcgaaa caaagttctt aattcactaa tcgaaattgc tttagatcag 240

gtattcgaac aacttgagca tggcagtaaa gagatccgtc gctctgttct tgaggaagtt 300

tccaaagttc tcgaatcaat tgaacatgat ggttctggga gcttagacaa tgat 354

<210> 417
<211> 288
<212> DNA
<213> Artificial Sequence

<220>

<223> orf

<400> 417
atgattaatc tacaaggaac tctaatcaat gctttccgtg tggatggtgg taaaggtaaa      60

gacggcaaag aatatgaagc gcgtgacaag gtgcaaattc ttggttcgtt ggaactccca     120

aacggggaaa ttaaacacga acttgttgac ctaacagttg aggattctcg actgtttgaa     180

ccattcaaaa atcaggttat tagcatctct tgcggtgcta tggctatcgg tcgtaatgtt     240

gttttttatg ttcgtaaaag cgcaaaaccg atcttagcac agagcatt                   288


<210> 418
<211> 681
<212> DNA
<213> Artificial Sequence


<220>
<223> orf

<400> 418
attcggggac aaaatttcat gaaaaatgac aataaaaaac ccctgttatc ggaagctttg      60

gcgggcgtag ataacaaggg tttgcaatct gctaaggcag atcaacacag ggatagaata     120

acacgttttg gtattttgaa acacagatcg aagcaacaag aaaacttttt gtggactctt     180

gcaaaattca aagaaaacta ccctcaagac ctgccaagtg aggaaacaac aaaagcttta     240

aagtctgctc aaaagttgca aggctgtggc aattttttgc tcttcaaaaa tttttacaca     300

atagatcaaa ctaagttagc caaatttcat gtctgtggtc agcatttgtt atgtcctttt     360

tgtgctggta tccgtgcttc aaagtctatt cagaaatatt cagaacgtat tgatgaagtt     420

ctcaagaaaa accgtaagct aaaagtcgct cttttgacgc tgacggttaa aaacggttct     480

gacctagggg aacgtgctaa tcatctctta acgtcgttta gaacgcttat caagcgcaga     540

caggactatt taaaaagggt cgtgggttta atgagttctg taaaattgat ggtgcgatgt     600

attcctatga aaatacctac aatgaaaaaa caggtgaatg gcatcctcat gttcacatgc     660

ttgtacttct caatgattgg a                                               681


<210> 419
<211> 192
<212> DNA
<213> Artificial Sequence


<220>
<223> orf

<400> 419
ttgaatggtt caaacagtcg agaatcctca actgttaggt caacaagttc gtgtttaatt      60

tccccgtttg ggagttccaa cgaaccaaga atttgcacct tgtcacgcgc ttcatattct     120

ttgccgtctt tacctttacc accatccaca cggaaagcat tgattagagt tccttgtaga 180

ttaatcattg tc 192

<210> 420
<211> 180
<212> DNA
<213> Artificial Sequence

<220>
<223> orf

<400> 420
atgttcgaca tatgggaaaa gatcaatgtt atacataatc ccgtcccatc ttcggattct 60

ttgccgaaca cgaacacggg tagaaggtgg gattctccag tgctgttttt tgcgtcctct 120

tttcgtacgt cgcagtggtg cgagccctcc acctgcctcc gtactcaaat cgtctacact 180

<210> 421
<211> 180
<212> DNA
<213> Artificial Sequence

<220>
<223> orf

<400> 421
gtgggattct ccagtgctgt tttttgcgtc ctcttttcgt acgtcgcagt ggtgcgagcc 60

ctccacctgc ctccgtactc aaatcgtcta cacttgaagc acccgcgcta tatctctcgt 120

cgggcgtcgt cgcagctcct cctaactccg cgcgctctcc cgcctgcgct tctacgtgtc 180

<210> 422
<211> 300
<212> DNA
<213> Artificial Sequence

<220>
<223> orf

<400> 422
atgaaagcca atagcctatg ttcgacatat gggaaaagat caatgttata cataatcccg 60

tcccatcttc ggattctttg ccgaacacga acacgggtag aaggtgggat tctccagtgc 120

tgttttttgc gtcctctttt cgtacgtcgc agtggtgcga gccctccacc tgcctccgta 180

ctcaaatcgt ctacacttga agcacccgcg ctatatctct cgtcgggcgt cgtcgcagct 240

cctcctaact ccgcgcgctc tcccgcctgc gcttctacgt gtctagtcat aactaagtca 300

<210> 423
<211> 207
<212> DNA

<213> Artificial Sequence

<220>
<223> orf

<400> 423
atatcgtcag caaggttttt agggattttg acaccacgca gggaaccaaa tgaaccacta      60

aggtttttac ccttcagggt tttaaatgcc tcccaagttt tttcaacagt cagatcacta     120

aacttaagag catatttaca aacctctgct gctgctttgg cataccctaa ttctttagat     180

tttttaactt ttcgaacatc aacgata                                          207

<210> 424
<211> 195
<212> DNA
<213> Artificial Sequence

<220>
<223> orf

<400> 424
atcaacgata taagaatcac cagtaatttc atgccaagtt tcgcccaagg cttcctgatc      60

tatccaatca ttgagaagta caagcatgtg aacatgagga tgccattcac ctgttttttc     120

attgtaggta ttttcatagg aatacatcgc accatcaatt ttacagaact cattaaaccc     180

acgacccttt ttaaa                                                       195

<210> 425
<211> 159
<212> DNA
<213> Artificial Sequence

<220>
<223> orf

<400> 425
ttgagggtag ttttctttga attttgcaag agtccacaaa aagttttctt gttgcttcga      60

tctgtgtttc aaaataccaa aacgtgttat tctatccctg tgttgatctg ccttagcaga     120

ttgcaaaccc ttgttatcta cgcccgccaa agcttccga                             159

<210> 426
<211> 2832
<212> DNA
<213> Artificial Sequence

<220>
<223> isolate

<400> 426
caacacaggg atagaataac acgttttggt attttgaaac atagatcgaa gcaacaagaa      60

aactttttgt ggactctagc aaaattcaaa gaaaactatc caaatgacct accaagcgaa 120

gagactataa aggctctgaa atctgcacag aaattgcaag ggtgcggaaa cttttgcttt 180

ttcaaaaatt tttacacaat agatcaaact aagctctcaa aatttcatgt atgtaatcaa 240

catttgttat gtccattttg tgcaggtatc cgtgcatcta aggcaattca aaaatatact 300

gaacgtgttg atgaggtctt aaagaaaaat cgtaagctta agcccgtttt aatcactctc 360

acggtaaaaa acggtactga cctaagtgaa cggtctgcgc atcttataaa atcgcttaga 420

acgcttatta agcgtcgtca ggactataat aaaaaaggtc gtggatttaa tgagttttgt 480

aagatcaatg gggctatgta ttcttatgaa aacacgtaca acgaacaaac taaagagtgg 540

catccccatg ttcacatgtt tgcccttttg gatgattgga tagaccaaga acacatgtca 600

gaaacctggc atgaagtcac tggtgactcg ttcattgtag atattcgaaa agtaaaaaaa 660

agaaaaagac ttaggctatt caaaagctgc tgcggaagtt tgtaaatatg cacttaaatt 720

tggcgatctt tcagtagaaa acacatggga agcattcaag attttgaaag gtaagcgttt 780

aagcggtgca tttggctctc tttatggtgt aaaaattcct gaaaatctta ctgatgaaat 840

gcccgatgaa aacgatcttc cttatttaga aatgctctat aaatttgtct tttcgaagca 900

gtcttactat gacttaacaa tgacacgaca cgtagagcca caatgcaagg acagggacaa 960

cgacgaagag gaggtagcga cgactgacgg aggcggaaat ggacgcagca gtgaggcgat 1020

taaggacggg cgaggacggg cggagcgcac cgagggcacg cagggtgcac ggcaccgccc 1080

acagcacgga cgtaaaaaac aacactggca agtacctcca gtaacgaggg tacgagtgaa 1140

aaaacgcatc aaaagatggg atggttattt gtgtgttatg cacatgtaaa atcataaacc 1200

tatgataaaa ttctagtcgc tgaatagtcg gattgcagtc caactaccca gctaaccaaa 1260

acgatctaat aaggagatca tcatggctgt tgcacatact actgttttgt ctttgcttgg 1320

caaagaagtt tcattttctg ttttattgga tgaccaaata aaaccctttt ttcctgacgg 1380

tgttcatgta tctggctctg ttgaagctgt atatattgct ttaaatggca atcatcatat 1440

tttggtttct gataatttat atgaactctc tgaaatagat ttaattattt gatatctgat 1500

tttaccaatg cccgaaaata catatttttg ggcttttttt tgtctgtttc ttgagaaacg 1560

cggaagcgtg cattctgaaa aaaatataca ttgagattgg cggagagatc ggagcgctgg 1620

gcgtgggatt gggatgtcgc tcgatgtcct agcctgcgac tggcgatacg agatatacgc 1680

caatttaact tcataaaata atatcagagg gcacggagtg tgcccgaact gaagaaataa 1740

aaaatcaaaa actcctttcc ctgattacaa gctcccattt gtttctactc gataagaaca 1800

gagccatccg gatggtggcg actgactatt gatttgatat taaatttgat atataaatat 1860

ctaaccataa atatcaaatc caatatgaga accaatatca tggaattatc aaatttatct 1920

caatttatga tcgaacttaa acactctggt aaatctgaat atcacggtgc ttataagcaa 1980

attcctttgc gtcttcgttt tgtttcaaat gctcgtgtaa ctgcattaac ttcaatgttt 2040

ggaggtgcaa gtaaaaataa aattttgaat gacttgatcg aaattgcttt agatcaagtc 2100

tatgaaaaaa tgactgatga acaaaaagct gtatatgaag aaattcagtc tttcactttg 2160

gctggcacaa ttaaaagcgt tgaatcgggt gacgaaaaag atgattaaac ctgaagtcat 2220

cagcaacact ccaatggaat cgcttgaaga agcatgttct tacttcaagc aagtgttgca 2280

agaacactta gacaaaaaac gtgacatcac attatttgcc attgccgttg taggtgggcg 2340

cttaaaagtt gtagaaggat taccaaaatg attaatttag aaggcacaat tatcaatgtt 2400

tttacccaac agggtggtaa agacaaaaaa acaggtgaag cttttgcaga tcgggacaaa 2460

atacaattat tgggtgagtt agaactcccc aatggtgaca aaaagaatga attaattgat 2520

cttaccgttg aaaacggaaa ggcttatgaa aacctaaaaa acaggcgtgt gagcattcct 2580

tgtggtgctc ttgcttcagg tagaaatgtg attttttatg tcgcaaaagg tgcaacacct 2640

aaacttttga gcacagtatg acactcgtta aaaaacagca acttacgaag taagttcgct 2700

gtttttcttt ttacttgata ctattcatta agtcggggac aaaatttcat gagaaatgac 2760

ataaaaaaac ccctgttatc tagcactttg gttggtggcg ataacaaggg tttgcaatct 2820

gctaaggcag at 2832

<210> 427
<211> 229
<212> PRT
<213> Artificial Sequence

<220>
<223> orf

<400> 427
Met Thr Arg Phe Gly Ile Leu Lys His Arg Ser Lys Gln Gln Glu Asn
1               5                   10                  15
Phe Leu Trp Thr Leu Ala Lys Phe Lys Glu Asn Tyr Pro Asn Asp Leu
            20                  25                  30
Pro Ser Glu Glu Thr Ile Lys Ala Leu Lys Ser Ala Gln Lys Leu Gln
        35                  40                  45
Gly Cys Gly Asn Phe Leu Leu Phe Lys Asn Phe Tyr Thr Ile Asp Gln
    50                  55                  60
Thr Lys Leu Ser Lys Phe His Val Cys Asn Gln His Leu Leu Cys Pro
65                  70                  75                  80
Phe Cys Ala Gly Ile Arg Ala Ser Lys Ala Ile Gln Lys Tyr Thr Glu
                85                  90                  95
Arg Val Asp Glu Val Leu Lys Lys Asn Arg Lys Leu Lys Pro Val Leu
            100                 105                 110
Ile Thr Leu Thr Val Lys Asn Gly Thr Asp Leu Ser Glu Arg Ser Ala
        115                 120                 125
His Leu Ile Lys Ser Leu Arg Thr Leu Ile Lys Arg Arg Gln Asp Tyr
    130                 135                 140
Asn Lys Lys Gly Arg Gly Phe Asn Glu Phe Cys Lys Ile Asn Gly Ala
145                 150                 155                 160

Met Tyr Ser Tyr Glu Asn Thr Tyr Asn Glu Gln Thr Lys Glu Trp His
                165                 170                 175
Pro His Val His Met Phe Ala Leu Leu Asp Asp Trp Ile Asp Gln Glu
            180                 185                 190
His Met Ser Glu Thr Trp His Glu Val Thr Gly Asp Ser Phe Ile Val
        195                 200                 205
Asp Ile Arg Lys Val Lys Lys Arg Lys Arg Leu Arg Leu Phe Lys Ser
    210                 215                 220
Cys Cys Gly Ser Leu
225

<210> 428
<211> 142
<212> PRT
<213> Artificial Sequence

<220>
<223> orf

<400> 428
Met Leu Lys Gly Lys Arg Leu Ser Gly Ala Phe Gly Ser Leu Tyr Gly
1               5                   10                  15
Val Lys Ile Pro Glu Asn Leu Thr Asp Glu Met Pro Asp Glu Asn Asp
            20                  25                  30
Leu Pro Tyr Leu Glu Met Leu Tyr Lys Phe Val Phe Ser Lys Gln Ser
        35                  40                  45
Tyr Tyr Asp Leu Thr Met Thr Arg His Val Glu Pro Gln Cys Lys Asp
    50                  55                  60
Arg Asp Asn Asp Glu Glu Glu Val Ala Thr Thr Asp Gly Gly Gly Asn
65                  70                  75                  80
Gly Arg Ser Ser Glu Ala Ile Lys Asp Gly Arg Gly Arg Ala Glu Arg
                85                  90                  95
Thr Glu Gly Thr Gln Gly Ala Arg His Arg Pro Gln His Gly Arg Lys
            100                 105                 110
Lys Gln His Trp Gln Val Pro Pro Val Thr Arg Val Arg Val Lys Lys
        115                 120                 125
Arg Ile Lys Arg Trp Asp Gly Tyr Leu Cys Val Met His Met
    130                 135                 140

<210> 429
<211> 71
<212> PRT
<213> Artificial Sequence

<220>
<223> orf

<400> 429
Met Ile Met Ala Val Ala His Thr Thr Val Leu Ser Leu Leu Gly Lys
1               5                   10                  15
Glu Val Ser Phe Ser Val Leu Leu Asp Asp Gln Ile Lys Pro Phe Phe
            20                  25                  30
Pro Asp Gly Val His Val Ser Gly Ser Val Glu Ala Val Tyr Ile Ala
        35                  40                  45
Leu Asn Gly Asn His His Ile Leu Val Ser Asp Asn Leu Tyr Glu Leu
    50                  55                  60
Ser Glu Ile Asp Leu Ile Ile
65                  70

<210> 430
<211> 143

<212> PRT
<213> Artificial Sequence

<220>
<223> orf

<400> 430
Met Cys Phe Tyr Ser Ile Arg Thr Glu Pro Ser Gly Trp Trp Arg Leu
1               5                   10                  15
Thr Ile Asp Leu Ile Leu Asn Leu Ile Tyr Lys Tyr Leu Thr Ile Asn
            20                  25                  30
Ile Lys Ser Asn Met Arg Thr Asn Ile Met Glu Leu Ser Asn Leu Ser
        35                  40                  45
Gln Phe Met Ile Glu Leu Lys His Ser Gly Lys Ser Glu Tyr His Gly
    50                  55                  60
Ala Tyr Lys Gln Ile Pro Leu Arg Leu Arg Phe Val Ser Asn Ala Arg
65                  70                  75                  80
Val Thr Ala Leu Thr Ser Met Phe Gly Gly Ala Ser Lys Asn Lys Ile
                85                  90                  95
Leu Asn Asp Leu Ile Glu Ile Ala Leu Asp Gln Val Tyr Glu Lys Met
            100                 105                 110
Thr Asp Glu Gln Lys Ala Val Tyr Glu Glu Ile Gln Ser Phe Thr Leu
        115                 120                 125
Ala Gly Thr Ile Lys Ser Val Glu Ser Gly Asp Glu Lys Asp Asp
    130                 135                 140

<210> 431
<211> 85
<212> PRT
<213> Artificial Sequence

<220>
<223> orf

<400> 431
Met Met Asn Lys Lys Leu Tyr Met Lys Lys Phe Ser Leu Ser Leu Trp
1               5                   10                  15
Leu Ala Gln Leu Lys Ala Leu Asn Arg Val Thr Lys Lys Met Ile Lys
            20                  25                  30
Pro Glu Val Ile Ser Asn Thr Pro Met Glu Ser Leu Glu Glu Ala Cys
        35                  40                  45
Ser Tyr Phe Lys Gln Val Leu Gln Glu His Leu Asp Lys Lys Arg Asp
    50                  55                  60
Ile Thr Leu Phe Ala Ile Ala Val Val Gly Gly Arg Leu Lys Val Val
65                  70                  75                  80
Glu Gly Leu Pro Lys
                85

<210> 432
<211> 116
<212> PRT
<213> Artificial Sequence

<220>
<223> orf

<400> 432
Met Ile Cys His Cys Arg Cys Arg Trp Ala Leu Lys Ser Cys Arg Arg
1               5                   10                  15
Ile Thr Lys Met Ile Asn Leu Glu Gly Thr Ile Ile Asn Val Phe Thr

20                  25                  30
Gln Gln Gly Gly Lys Asp Lys Lys Thr Gly Glu Ala Phe Ala Asp Arg
        35                  40                  45
Asp Lys Ile Gln Leu Leu Gly Glu Leu Glu Leu Pro Asn Gly Asp Lys
    50                  55                  60
Lys Asn Glu Leu Ile Asp Leu Thr Val Glu Asn Gly Lys Ala Tyr Glu
65                  70                  75                  80
Asn Leu Lys Asn Arg Arg Val Ser Ile Pro Cys Gly Ala Leu Ala Ser
                85                  90                  95
Gly Arg Asn Val Ile Phe Tyr Val Ala Lys Gly Ala Thr Pro Lys Leu
            100                 105                 110
Leu Ser Thr Val
        115

<210> 433
<211> 262
<212> PRT
<213> Artificial Sequence

<220>
<223> orf

<400> 433
Met Arg Asn Asp Ile Lys Lys Pro Leu Leu Ser Ser Thr Leu Val Gly
1               5                   10                  15
Gly Asp Asn Lys Gly Leu Gln Ser Ala Lys Ala Asp Gln His Arg Asp
            20                  25                  30
Arg Ile Thr Arg Phe Gly Ile Leu Lys His Arg Ser Lys Gln Gln Glu
        35                  40                  45
Asn Phe Leu Trp Thr Leu Ala Lys Phe Lys Glu Asn Tyr Pro Asn Asp
    50                  55                  60
Leu Pro Ser Glu Glu Thr Ile Lys Ala Leu Lys Ser Ala Gln Lys Leu
65                  70                  75                  80
Gln Gly Cys Gly Asn Phe Leu Leu Phe Lys Asn Phe Tyr Thr Ile Asp
                85                  90                  95
Gln Thr Lys Leu Ser Lys Phe His Val Cys Asn Gln His Leu Leu Cys
            100                 105                 110
Pro Phe Cys Ala Gly Ile Arg Ala Ser Lys Ala Ile Gln Lys Tyr Thr
        115                 120                 125
Glu Arg Val Asp Glu Val Leu Lys Lys Asn Arg Lys Leu Lys Pro Val
    130                 135                 140
Leu Ile Thr Leu Thr Val Lys Asn Gly Thr Asp Leu Ser Glu Arg Ser
145                 150                 155                 160
Ala His Leu Ile Lys Ser Leu Arg Thr Leu Ile Lys Arg Arg Gln Asp
                165                 170                 175
Tyr Asn Lys Lys Gly Arg Gly Phe Asn Glu Phe Cys Lys Ile Asn Gly
            180                 185                 190
Ala Met Tyr Ser Tyr Glu Asn Thr Tyr Asn Glu Gln Thr Lys Glu Trp
        195                 200                 205
His Pro His Val His Met Phe Ala Leu Leu Asp Asp Trp Ile Asp Gln
    210                 215                 220
Glu His Met Ser Glu Thr Trp His Glu Val Thr Gly Asp Ser Phe Ile
225                 230                 235                 240
Val Asp Ile Arg Lys Val Lys Lys Arg Lys Arg Leu Arg Leu Phe Lys
                245                 250                 255
Ser Cys Cys Gly Ser Leu
            260

<210> 434
<211> 73
<212> PRT
<213> Artificial Sequence

<220>
<223> orf

<400> 434
Met Val Arg Ser Ile Asn Ser Phe Phe Leu Ser Pro Leu Gly Ser Ser
1               5                   10                  15
Asn Ser Pro Asn Asn Cys Ile Leu Ser Arg Ser Ala Lys Ala Ser Pro
            20                  25                  30
Val Phe Leu Ser Leu Pro Pro Cys Trp Val Lys Thr Leu Ile Ile Val
        35                  40                  45
Pro Ser Lys Leu Ile Ile Leu Val Ile Leu Leu Gln Leu Leu Ser Ala
    50                  55                  60
His Leu Gln Arg Gln Trp Gln Ile Met
65                  70

<210> 435
<211> 65
<212> PRT
<213> Artificial Sequence


<220>
<223> orf

<400> 435
Met Gln Leu Phe Val His Gln Ser Phe Phe His Arg Leu Asp Leu Lys
1               5                   10                  15
Gln Phe Arg Ser Ser His Ser Lys Phe Tyr Phe Tyr Leu His Leu Gln
            20                  25                  30
Thr Leu Lys Leu Met Gln Leu His Glu His Leu Lys Gln Asn Glu Asp
        35                  40                  45
Ala Lys Glu Phe Ala Tyr Lys His Arg Asp Ile Gln Ile Tyr Gln Ser
    50                  55                  60
Val
65

<210> 436
<211> 69
<212> PRT
<213> Artificial Sequence


<220>
<223> orf

<400> 436
Met Lys Leu Asn Trp Arg Ile Ser Arg Ile Ala Ser Arg Arg Leu Gly
1               5                   10                  15
His Arg Ala Thr Ser Gln Ser His Ala Gln Arg Ser Asp Leu Ser Ala
            20                  25                  30
Asn Leu Asn Val Tyr Phe Phe Gln Asn Ala Arg Phe Arg Val Ser Gln
        35                  40                  45
Glu Thr Asp Lys Lys Lys Pro Lys Asn Met Tyr Phe Arg Ala Leu Val
    50                  55                  60
Lys Ser Asp Ile Lys
65

<210> 437
<211> 90
<212> PRT
<213> Artificial Sequence

<220>
<223> orf

<400> 437
Met Ile Leu Phe Tyr Glu Val Lys Leu Ala Tyr Ile Ser Tyr Arg Gln
1               5                   10                  15
Ser Gln Ala Arg Thr Ser Ser Asp Ile Pro Ile Pro Arg Pro Ala Leu
            20                  25                  30
Arg Ser Leu Arg Gln Ser Gln Cys Ile Phe Phe Ser Glu Cys Thr Leu
        35                  40                  45
Pro Arg Phe Ser Arg Asn Arg Gln Lys Lys Ala Gln Lys Tyr Val Phe
    50                  55                  60
Ser Gly Ile Gly Lys Ile Arg Tyr Gln Ile Ile Lys Ser Ile Ser Glu
65                  70                  75                  80
Ser Ser Tyr Lys Leu Ser Glu Thr Lys Ile
                85                  90

<210> 438
<211> 53
<212> PRT
<213> Artificial Sequence

<220>
<223> orf

<400> 438
Met Asn Tyr Gln Lys Pro Lys Tyr Asp Asp Cys His Leu Lys Gln Tyr
1               5                   10                  15
Ile Gln Leu Gln Gln Ser Gln Ile His Glu His Arg Gln Glu Lys Arg
            20                  25                  30
Val Leu Phe Gly His Pro Ile Lys Gln Lys Met Lys Leu Leu Cys Gln
        35                  40                  45
Ala Lys Thr Lys Gln
    50

<210> 439
<211> 109
<212> PRT
<213> Artificial Sequence

<220>
<223> orf

<400> 439
Met Asp Cys Asn Pro Thr Ile Gln Arg Leu Glu Phe Tyr His Arg Phe
1               5                   10                  15
Met Ile Leu His Val His Asn Thr Gln Ile Thr Ile Pro Ser Phe Asp
            20                  25                  30
Ala Phe Phe His Ser Tyr Pro Arg Tyr Trp Arg Tyr Leu Pro Val Leu
        35                  40                  45
Phe Phe Thr Ser Val Leu Trp Ala Val Pro Cys Thr Leu Arg Ala Leu
    50                  55                  60
Gly Ala Leu Arg Pro Ser Ser Pro Val Leu Asn Arg Leu Thr Ala Ala
65                  70                  75                  80
Ser Ile Ser Ala Ser Val Ser Arg Arg Tyr Leu Leu Phe Val Val Val
                85                  90                  95
Pro Val Leu Ala Leu Trp Leu Tyr Val Ser Cys His Cys
            100                 105

<210> 440
<211> 81
<212> PRT
<213> Artificial Sequence

<220>
<223> orf

<400> 440
Met Met Arg Phe Phe Thr Arg Thr Leu Val Thr Gly Gly Thr Cys Gln
1               5                   10                  15
Cys Cys Phe Leu Arg Pro Cys Cys Gly Arg Cys Arg Ala Pro Cys Val
            20                  25                  30
Pro Ser Val Arg Ser Ala Arg Pro Arg Pro Ser Leu Ile Ala Ser Leu
        35                  40                  45
Leu Arg Pro Phe Pro Pro Pro Ser Val Val Ala Thr Ser Ser Ser Ser
    50                  55                  60
Leu Ser Leu Ser Leu His Cys Gly Ser Thr Cys Arg Val Ile Val Lys
65                  70                  75                  80
Ser

<210> 441
<211> 53
<212> PRT
<213> Artificial Sequence

<220>
<223> orf

<400> 441
Met His Ile Tyr Lys Leu Pro Gln Gln Leu Leu Asn Ser Leu Ser Leu
1               5                   10                  15
Phe Leu Phe Phe Thr Phe Arg Ile Ser Thr Met Asn Glu Ser Pro Val
            20                  25                  30
Thr Ser Cys Gln Val Ser Asp Met Cys Ser Trp Ser Ile Gln Ser Ser
        35                  40                  45
Lys Arg Ala Asn Met
    50

<210> 442
<211> 70
<212> PRT
<213> Artificial Sequence

<220>
<223> orf

<400> 442
Met Phe Leu Val Tyr Pro Ile Ile Gln Lys Gly Lys His Val Asn Met
1               5                   10                  15
Gly Met Pro Leu Phe Ser Leu Phe Val Val Arg Val Phe Ile Arg Ile
            20                  25                  30
His Ser Pro Ile Asp Leu Thr Lys Leu Ile Lys Ser Thr Thr Phe Phe
        35                  40                  45
Ile Ile Val Leu Thr Thr Leu Asn Lys Arg Ser Lys Arg Phe Tyr Lys
    50                  55                  60
Met Arg Arg Pro Phe Thr
65                  70

<210> 443
<211> 687
<212> DNA
<213> Artificial Sequence

<220>
<223> orf

<400> 443
ataacacgtt ttggtatttt gaaacataga tcgaagcaac aagaaaactt tttgtggact 60

ctagcaaaat tcaaagaaaa ctatccaaat gacctaccaa gcgaagagac tataaaggct 120

ctgaaatctg cacagaaatt gcaagggtgc ggaaactttt tgcttttcaa aaatttttac 180

acaatagatc aaactaagct ctcaaaattt catgtatgta atcaacattt gttatgtcca 240

ttttgtgcag gtatccgtgc atctaaggca attcaaaaat atactgaacg tgttgatgag 300

gtcttaaaga aaaatcgtaa gcttaagccc gttttaatca ctctcacggt aaaaaacggt 360

actgacctaa gtgaacggtc tgcgcatctt ataaaatcgc ttagaacgct tattaagcgt 420

cgtcaggact ataataaaaa aggtcgtgga tttaatgagt tttgtaagat caatggggct 480

atgtattctt atgaaaacac gtacaacgaa caaactaaag agtggcatcc ccatgttcac 540

atgtttgccc ttttggatga ttggatagac caagaacaca tgtcagaaac ctggcatgaa 600

gtcactggtg actcgttcat tgtagatatt cgaaaagtaa aaaaaagaaa aagacttagg 660

ctattcaaaa gctgctgcgg aagtttg 687

<210> 444
<211> 426
<212> DNA
<213> Artificial Sequence

<220>
<223> orf

<400> 444
attttgaaag gtaagcgttt aagcggtgca tttggctctc tttatggtgt aaaaattcct 60

gaaaatctta ctgatgaaat gcccgatgaa aacgatcttc cttatttaga aatgctctat 120

aaatttgtct tttcgaagca gtcttactat gacttaacaa tgacacgaca cgtagagcca 180

caatgcaagg acagggacaa cgacgaagag gaggtagcga cgactgacgg aggcggaaat 240

ggacgcagca gtgaggcgat taaggacggg cgaggacggg cggagcgcac cgagggcacg 300

cagggtgcac ggcaccgccc acagcacgga cgtaaaaaac aacactggca agtacctcca 360

gtaacgaggg tacgagtgaa aaaacgcatc aaaagatggg atggttattt gtgtgttatg 420

cacatg 426

<210> 445

<211> 213
<212> DNA
<213> Artificial Sequence

<220>
<223> orf

<400> 445
atcatcatgg ctgttgcaca tactactgtt ttgtctttgc ttggcaaaga agtttcattt 60

tctgttttat tggatgacca aataaaaccc ttttttcctg acggtgttca tgtatctggc 120

tctgttgaag ctgtatatat tgctttaaat ggcaatcatc atattttggt ttctgataat 180

ttatatgaac tctctgaaat agatttaatt att 213

<210> 446
<211> 429
<212> DNA
<213> Artificial Sequence

<220>
<223> orf

<400> 446
atttgtttct actcgataag aacagagcca tccggatggt ggcgactgac tattgatttg 60

atattaaatt tgatatataa atatctaacc ataaatatca aatccaatat gagaaccaat 120

atcatggaat tatcaaattt atctcaattt atgatcgaac ttaaacactc tggtaaatct 180

gaatatcacg gtgcttataa gcaaattcct ttgcgtcttc gttttgtttc aaatgctcgt 240

gtaactgcat taacttcaat gtttggaggt gcaagtaaaa ataaaatttt gaatgacttg 300

atcgaaattg ctttagatca agtctatgaa aaaatgactg atgaacaaaa agctgtatat 360

gaagaaattc agtctttcac tttggctggc acaattaaaa gcgttgaatc gggtgacgaa 420

aaagatgat 429

<210> 447
<211> 255
<212> DNA
<213> Artificial Sequence

<220>
<223> orf

<400> 447
ctgatgaaca aaaagctgta tatgaagaaa ttcagtcttt cactttggct ggcacaatta 60

aaagcgttga atcgggtgac gaaaaagatg attaaacctg aagtcatcag caacactcca 120

atggaatcgc ttgaagaagc atgttcttac ttcaagcaag tgttgcaaga acacttagac 180

aaaaaacgtg acatcacatt atttgccatt gccgttgtag gtgggcgctt aaaagttgta 240

gaaggattac caaaa 255

<210> 448
<211> 348
<212> DNA
<213> Artificial Sequence

<220>
<223> orf

<400> 448
attatttgcc attgccgttg taggtgggcg cttaaaagtt gtagaaggat taccaaaatg 60

attaatttag aaggcacaat tatcaatgtt tttacccaac agggtggtaa agacaaaaaa 120

acaggtgaag cttttgcaga tcgggacaaa atacaattat tgggtgagtt agaactcccc 180

aatggtgaca aaaagaatga attaattgat cttaccgttg aaaacggaaa ggcttatgaa 240

aacctaaaaa acaggcgtgt gagcattcct tgtggtgctc ttgcttcagg tagaaatgtg 300

attttttatg tcgcaaaagg tgcaacacct aaacttttga gcacagta 348

<210> 449
<211> 786
<212> DNA
<213> Artificial Sequence

<220>
<223> orf

<400> 449
atgagaaatg acataaaaaa acccctgtta tctagcactt tggttggtgg cgataacaag 60

ggtttgcaat ctgctaaggc agatcaacac agggatagaa taacacgttt tggtattttg 120

aaacatagat cgaagcaaca agaaaacttt ttgtggactc tagcaaaatt caaagaaaac 180

tatccaaatg acctaccaag cgaagagact ataaaggctc tgaaatctgc acagaaattg 240

caagggtgcg gaaacttttt gcttttcaaa aatttttaca caatagatca aactaagctc 300

tcaaaatttc atgtatgtaa tcaacatttg ttatgtccat tttgtgcagg tatccgtgca 360

tctaaggcaa ttcaaaaata tactgaacgt gttgatgagg tcttaaagaa aaatcgtaag 420

cttaagcccg ttttaatcac tctcacggta aaaaacggta ctgacctaag tgaacggtct 480

gcgcatctta taaaatcgct tagaacgctt attaagcgtc gtcaggacta taataaaaaa 540

ggtcgtggat ttaatgagtt ttgtaagatc aatggggcta tgtattctta tgaaaacacg 600

tacaacgaac aaactaaaga gtggcatccc catgttcaca tgtttgccct tttggatgat 660

tggatagacc aagaacacat gtcagaaacc tggcatgaag tcactggtga ctcgttcatt 720

gtagatattc gaaaagtaaa aaaaagaaaa agacttaggc tattcaaaag ctgctgcgga 780

agtttg 786

<210> 450
<211> 219
<212> DNA
<213> Artificial Sequence

<220>
<223> orf

<400> 450
acggtaagat caattaattc attctttttg tcaccattgg ggagttctaa ctcacccaat 60

aattgtattt tgtcccgatc tgcaaaagct tcacctgttt ttttgtcttt accaccctgt 120

tgggtaaaaa cattgataat tgtgccttct aaattaatca ttttggtaat ccttctacaa 180

cttttaagcg cccacctaca acggcaatgg caaataatg 219

<210> 451
<211> 195
<212> DNA
<213> Artificial Sequence

<220>
<223> orf

<400> 451
atacagcttt ttgttcatca gtcatttttt catagacttg atctaaagca atttcgatca 60

agtcattcaa aattttattt ttacttgcac ctccaaacat tgaagttaat gcagttacac 120

gagcatttga aacaaaacga agacgcaaag gaatttgctt ataagcaccg tgatattcag 180

atttaccaga gtgtt 195

<210> 452
<211> 207
<212> DNA
<213> Artificial Sequence

<220>
<223> orf

<400> 452
atgaagttaa attggcgtat atctcgtatc gccagtcgca ggctaggaca tcgagcgaca 60

tcccaatccc acgcccagcg ctccgatctc tccgccaatc tcaatgtata tttttttcag 120

aatgcacgct tccgcgtttc tcaagaaaca gacaaaaaaa agcccaaaaa tatgtatttt 180

cgggcattgg taaaatcaga tatcaaa 207

<210> 453
<211> 270
<212> DNA
<213> Artificial Sequence

<220>
<223> orf

<400> 453
ctgatattat tttatgaagt taaattggcg tatatctcgt atcgccagtc gcaggctagg      60

acatcgagcg acatcccaat cccacgccca gcgctccgat ctctccgcca atctcaatgt     120

atattttttt cagaatgcac gcttccgcgt ttctcaagaa acagacaaaa aaaagcccaa     180

aaatatgtat tttcgggcat tggtaaaatc agatatcaaa taattaaatc tatttcagag     240

agttcatata aattatcaga aaccaaaata                                      270


<210> 454
<211> 159
<212> DNA
<213> Artificial Sequence


<220>
<223> orf

<400> 454
   ataaattatc agaaaccaaa atatgatgat tgccatttaa agcaatatat acagcttcaa      60
   cagagccaga tacatgaaca ccgtcaggaa aaaagggttt tatttggtca tccaataaaa     120
   cagaaaatga aacttctttg ccaagcaaag acaaaacag                           159


<210> 455
<211> 327
<212> DNA
<213> Artificial Sequence


<220>
<223> orf

<400> 455
ttggactgca atccgactat tcagcgacta gaattttatc ataggtttat gattttacat      60

gtgcataaca cacaaataac catcccatct tttgatgcgt tttttcactc gtaccctcgt     120

tactggaggt acttgccagt gttgtttttt acgtccgtgc tgtgggcggt gccgtgcacc     180

ctgcgtgccc tcggtgcgct ccgcccgtcc tcgcccgtcc ttaatcgcct cactgctgcg     240

tccatttccg cctccgtcag tcgtcgctac ctcctcttcg tcgttgtccc tgtccttgca     300

ttgtggctct acgtgtcgtg tcattgt                                         327


<210> 456
<211> 243
<212> DNA
<213> Artificial Sequence

<220>
<223> orf

<400> 456
ttgatgcgtt ttttcactcg taccctcgtt actggaggta cttgccagtg ttgtttttta      60

cgtccgtgct gtgggcggtg ccgtgcaccc tgcgtgccct cggtgcgctc cgcccgtcct     120

cgcccgtcct taatcgcctc actgctgcgt ccatttccgc ctccgtcagt cgtcgctacc     180

tcctcttcgt cgttgtccct gtccttgcat tgtggctcta cgtgtcgtgt cattgttaag     240

tca                                                                   243


<210> 457
<211> 159
<212> DNA
<213> Artificial Sequence


<220>
<223> orf

<400> 457
gtgcatattt acaaacttcc gcagcagctt ttgaatagcc taagtctttt tctttttttt      60

acttttcgaa tatctacaat gaacgagtca ccagtgactt catgccaggt ttctgacatg     120

tgttcttggt ctatccaatc atccaaaagg gcaaacatg                            159


<210> 458
<211> 210
<212> DNA
<213> Artificial Sequence


<220>
<223> orf

<400> 458
gtgttcttgg tctatccaat catccaaaag ggcaaacatg tgaacatggg gatgccactc      60

tttagtttgt tcgttgtacg tgttttcata agaatacata gccccattga tcttacaaaa     120

ctcattaaat ccacgacctt ttttattata gtcctgacga cgcttaataa gcgttctaag     180

cgattttata agatgcgcag accgttcact                                      210


<210> 459
<211> 2537
<212> DNA
<213> Artificial Sequence


<220>
<223> isolate

<400> 459
caacacaggg atagaataac acgttttggg attttgaaac atagagcgaa gcaacaagaa      60

aattttttgt ggactctggc aaaattcaaa gaaaactatc ctaaagatgc tccaagtgaa 120

gaaagcacaa gagcaattaa atctgcacag aaattaaaag agtgcggaaa tttcttacta 180

ttcaaaaatt tttacacaat agaccaaact aagctctcaa aatttcatgt atgtgggcag 240

catttgttat gtcctttctg tgctggtatt cgtgcatcta aagcaattca aaaatatact 300

gaacgtgttg atgaagtgct taagaaaaat cgcaagctta agcctgtttt aatcacgttt 360

acggtgaaga atggaatcga cttaggggaa agatcatccc acctcataaa atcgtttaga 420

acgcttctga agcgtcgtag ggactatatt aaaaaaggtc gtgggtttaa tgagttctgt 480

aagatcaatg gggctatgta ttcttatgaa aacacgtata acgagcaaac aaaggaatgg 540

catccgcact tacatatgtt tgcacttttg gatgattgga tagaccagga agagctttca 600

cagtattggc attccatcac tggtgattca atgattgtag atattcgcag ggtcaaaaaa 660

gaaaaaggct taggctattc aaaggctgct gcggaagtat gtaaatatgc tcttaaattc 720

ggtgttttgt ctgtccaaaa tacttgggaa gctttcaaag tcttaaaagg taagcgttta 780

agtggtgcat ttggttcttt atggggtgtc aaaattcctg aagatcttgc tgatgatatg 840

cctgatgaaa aagatcttcc ttacttagaa atgctctata aattcgtatt tggaaaacag 900

tcttactatg acttaactat gacacgacat gtagagccac aaaataagga cgacgccgac 960

gacgaggaga aggaacgtcg acgagaagga gaaggcggac gcaagttagt ggcgaatgag 1020

gacgatgtga ggagtgacga ggagcgtgcc gaccgacgga cacgaacgac acgaggacga 1080

aaaaaacaac actggcaagt acctccagta acgagggtac gagtgaaaaa acggattaaa 1140

agatgggatg gttatttgtg tgttatgcac atgtaaaatc ataaacctat gataaaattc 1200

tagtcgctga atagtcggat tgcagtccaa ctacccagct aaccacaacg atctaagaag 1260

gagatcataa tggctgttgc acatactact gttttgtctt tgcttggcaa agaagtttca 1320

ttttctgttt tattagatga ccaaataaaa cccttttttc ctgacggtgt tcatgtatct 1380

ggctctgttg aagctgtata tattgcttta aatggcaatc atcatatttt ggtttctgat 1440

aatttatatg aactctctga aatagattta gttatttgat atctgatttt accaatgccc 1500

gaaaatacat atttttgggc ttttttttgt ccgtttctta agaaacgcgt aagcgtacat 1560

tctgaaaaaa tcaaccgaac gagtgtctac gagtgacgac tccggtataa aaatttcaaa 1620

acctcttttc cctgattaca agctcccctt tgccgttctc cagtaagaac cgagccatcc 1680

cgaatgggtg gcgactctcc tattgatttg ttatggattc agtgtatgat tcttttgtga 1740

atccaataat ctctttaatg aatcaggtat gaatatggaa cctacaaatt tagatatttt 1800

cttaagtgaa gttaaaaaaa caggtgaaac gaaattaact ggtgttttcc accaagttcc 1860

atttcgtatg ttctctccac actatcaacg tgtcgaaggt cttcgaaatt atatgaatga 1920

acctcgtaat aaggttctta actctctaat agaaattgct ttagatcagg tttttgaaaa 1980

cctatctaaa gatcctgaaa tctataaagc tgttatggct gaggtttcaa aaattgacat 2040

tggtggcttt gatggttctg gagacttaga tcgtgattaa ccttgaagga acaattatta 2100

atgtttttac tcagcaaggc ggtaaagaca aaaaaacagg tgaagctttt gctgatcgtg 2160

acaaaattca gctattggga gaattagaac ttcccaatgg tgataaaaaa aacgaattaa 2220

ttgatcttac cgttgaaaac ggaaaggcat atgaaaacct aaaaaataag cgtgtgagca 2280

tcccttgcgg tgctctagct tctggtcgaa atgttatttt ttatgttgca aaaggcgcaa 2340

ctccaaaact cttaaaccaa gtatgatact cgttaaaaaa cagcaactta cgaagtaagt 2400

tcgctgtttt ttcttttact tgatactatt aattaaagtg gggacaaaaa ttgactgtaa 2460

aagacaataa aaaagcccat atttctgatg ctttggcggg cgacgaaata caggctttgc 2520

aatctgctaa ggcagat 2537

<210> 460
<211> 386
<212> PRT
<213> Artificial Sequence

<220>
<223> orf

<400> 460
Met Thr Arg Phe Gly Ile Leu Lys His Arg Ala Lys Gln Gln Glu Asn
1               5                   10                  15
Phe Leu Trp Thr Leu Ala Lys Phe Lys Glu Asn Tyr Pro Lys Asp Ala
            20                  25                  30
Pro Ser Glu Glu Ser Thr Arg Ala Ile Lys Ser Ala Gln Lys Leu Lys
        35                  40                  45
Glu Cys Gly Asn Phe Leu Leu Phe Lys Asn Phe Tyr Thr Ile Asp Gln
    50                  55                  60
Thr Lys Leu Ser Lys Phe His Val Cys Gly Gln His Leu Leu Cys Pro
65                  70                  75                  80
Phe Cys Ala Gly Ile Arg Ala Ser Lys Ala Ile Gln Lys Tyr Thr Glu
                85                  90                  95
Arg Val Asp Glu Val Leu Lys Lys Asn Arg Lys Leu Lys Pro Val Leu
            100                 105                 110
Ile Thr Phe Thr Val Lys Asn Gly Ile Asp Leu Gly Glu Arg Ser Ser
        115                 120                 125
His Leu Ile Lys Ser Phe Arg Thr Leu Leu Lys Arg Arg Arg Asp Tyr
    130                 135                 140
Ile Lys Lys Gly Arg Gly Phe Asn Glu Phe Cys Lys Ile Asn Gly Ala
145                 150                 155                 160
Met Tyr Ser Tyr Glu Asn Thr Tyr Asn Glu Gln Thr Lys Glu Trp His
                165                 170                 175
Pro His Leu His Met Phe Ala Leu Leu Asp Asp Trp Ile Asp Gln Glu
            180                 185                 190
Glu Leu Ser Gln Tyr Trp His Ser Ile Thr Gly Asp Ser Met Ile Val
        195                 200                 205
Asp Ile Arg Arg Val Lys Lys Glu Lys Gly Leu Gly Tyr Ser Lys Ala
    210                 215                 220
Ala Ala Glu Val Cys Lys Tyr Ala Leu Lys Phe Gly Val Leu Ser Val
225                 230                 235                 240
Gln Asn Thr Trp Glu Ala Phe Lys Val Leu Lys Gly Lys Arg Leu Ser

245                         250                         255
Gly Ala Phe Gly Ser Leu Trp Gly Val Lys Ile Pro Glu Asp Leu Ala
                260                         265                         270
Asp Asp Met Pro Asp Glu Lys Asp Leu Pro Tyr Leu Glu Met Leu Tyr
        275                         280                         285
Lys Phe Val Phe Gly Lys Gln Ser Tyr Tyr Asp Leu Thr Met Thr Arg
    290                         295                         300
His Val Glu Pro Gln Asn Lys Asp Asp Ala Asp Asp Glu Glu Lys Glu
305                         310                         315                         320
Arg Arg Arg Glu Gly Glu Gly Gly Arg Lys Leu Val Ala Asn Glu Asp
                325                         330                         335
Asp Val Arg Ser Asp Glu Glu Arg Ala Asp Arg Arg Thr Arg Thr Thr
                340                         345                         350
Arg Gly Arg Lys Lys Gln His Trp Gln Val Pro Pro Val Thr Arg Val
        355                         360                         365
Arg Val Lys Lys Arg Ile Lys Arg Trp Asp Gly Tyr Leu Cys Val Met
    370                         375                         380
His Met
385

<210> 461
<211> 71
<212> PRT
<213> Artificial Sequence

<220>
<223> orf

<400> 461
Met Ile Met Ala Val Ala His Thr Thr Val Leu Ser Leu Leu Gly Lys
1               5                   10                  15
Glu Val Ser Phe Ser Val Leu Leu Asp Asp Gln Ile Lys Pro Phe Phe
            20                  25                  30
Pro Asp Gly Val His Val Ser Gly Ser Val Glu Ala Val Tyr Ile Ala
        35                  40                  45
Leu Asn Gly Asn His His Ile Leu Val Ser Asp Asn Leu Tyr Glu Leu
    50                  55                  60
Ser Glu Ile Asp Leu Val Ile
65                  70

<210> 462
<211> 103
<212> PRT
<213> Artificial Sequence

<220>
<223> orf

<400> 462
Met Asn Met Glu Pro Thr Asn Leu Asp Ile Phe Leu Ser Glu Val Lys
1               5                   10                  15
Lys Thr Gly Glu Thr Lys Leu Thr Gly Val Phe His Gln Val Pro Phe
            20                  25                  30
Arg Met Phe Ser Pro His Tyr Gln Arg Val Glu Gly Leu Arg Asn Tyr
        35                  40                  45
Met Asn Glu Pro Arg Asn Lys Val Leu Asn Ser Leu Ile Glu Ile Ala
    50                  55                  60
Leu Asp Gln Val Phe Glu Asn Leu Ser Lys Asp Pro Glu Ile Tyr Lys
65                  70                  75                  80
Ala Val Met Ala Glu Val Ser Lys Ile Asp Ile Gly Gly Phe Asp Gly
                85                  90                  95

Ser Gly Asp Leu Asp Arg Asp
            100

<210> 463
<211> 99
<212> PRT
<213> Artificial Sequence

<220>
<223> orf

<400> 463
Val Met Val Ile Asn Leu Glu Gly Thr Ile Ile Asn Val Phe Thr Gln
1               5                   10                  15
Gln Gly Gly Lys Asp Lys Lys Thr Gly Glu Ala Phe Ala Asp Arg Asp
            20                  25                  30
Lys Ile Gln Leu Leu Gly Glu Leu Glu Leu Pro Asn Gly Asp Lys Lys
        35                  40                  45
Asn Glu Leu Ile Asp Leu Thr Val Glu Asn Gly Lys Ala Tyr Glu Asn
    50                  55                  60
Leu Lys Asn Lys Arg Val Ser Ile Pro Cys Gly Ala Leu Ala Ser Gly
65                  70                  75                  80
Arg Asn Val Ile Phe Tyr Val Ala Lys Gly Ala Thr Pro Lys Leu Leu
                85                  90                  95
Asn Gln Val

<210> 464
<211> 420
<212> PRT
<213> Artificial Sequence

<220>
<223> orf

<400> 464
Met Thr Val Lys Asp Asn Lys Lys Ala His Ile Ser Asp Ala Leu Ala
1               5                   10                  15
Gly Asp Glu Ile Gln Ala Leu Gln Ser Ala Lys Ala Asp Gln His Arg
            20                  25                  30
Asp Arg Ile Thr Arg Phe Gly Ile Leu Lys His Arg Ala Lys Gln Gln
        35                  40                  45
Glu Asn Phe Leu Trp Thr Leu Ala Lys Phe Lys Glu Asn Tyr Pro Lys
    50                  55                  60
Asp Ala Pro Ser Glu Glu Ser Thr Arg Ala Ile Lys Ser Ala Gln Lys
65                  70                  75                  80
Leu Lys Glu Cys Gly Asn Phe Leu Leu Phe Lys Asn Phe Tyr Thr Ile
                85                  90                  95
Asp Gln Thr Lys Leu Ser Lys Phe His Val Cys Gly Gln His Leu Leu
            100                 105                 110
Cys Pro Phe Cys Ala Gly Ile Arg Ala Ser Lys Ala Ile Gln Lys Tyr
        115                 120                 125
Thr Glu Arg Val Asp Glu Val Leu Lys Lys Asn Arg Lys Leu Lys Pro
    130                 135                 140
Val Leu Ile Thr Phe Thr Val Lys Asn Gly Ile Asp Leu Gly Glu Arg
145                 150                 155                 160
Ser Ser His Leu Ile Lys Ser Phe Arg Thr Leu Leu Lys Arg Arg Arg
                165                 170                 175
Asp Tyr Ile Lys Lys Gly Arg Gly Phe Asn Glu Phe Cys Lys Ile Asn
            180                 185                 190
Gly Ala Met Tyr Ser Tyr Glu Asn Thr Tyr Asn Glu Gln Thr Lys Glu

195                 200                 205
Trp His Pro His Leu His Met Phe Ala Leu Leu Asp Asp Trp Ile Asp
    210                 215                 220
Gln Glu Glu Leu Ser Gln Tyr Trp His Ser Ile Thr Gly Asp Ser Met
225                 230                 235                 240
Ile Val Asp Ile Arg Arg Val Lys Lys Glu Lys Gly Leu Gly Tyr Ser
                245                 250                 255
Lys Ala Ala Ala Glu Val Cys Lys Tyr Ala Leu Lys Phe Gly Val Leu
            260                 265                 270
Ser Val Gln Asn Thr Trp Glu Ala Phe Lys Val Leu Lys Gly Lys Arg
        275                 280                 285
Leu Ser Gly Ala Phe Gly Ser Leu Trp Gly Val Lys Ile Pro Glu Asp
    290                 295                 300
Leu Ala Asp Asp Met Pro Asp Glu Lys Asp Leu Pro Tyr Leu Glu Met
305                 310                 315                 320
Leu Tyr Lys Phe Val Phe Gly Lys Gln Ser Tyr Tyr Asp Leu Thr Met
                325                 330                 335
Thr Arg His Val Glu Pro Gln Asn Lys Asp Asp Ala Asp Asp Glu Glu
            340                 345                 350
Lys Glu Arg Arg Arg Glu Gly Glu Gly Gly Arg Lys Leu Val Ala Asn
        355                 360                 365
Glu Asp Asp Val Arg Ser Asp Glu Glu Arg Ala Asp Arg Arg Thr Arg
    370                 375                 380
Thr Thr Arg Gly Arg Lys Lys Gln His Trp Gln Val Pro Pro Val Thr
385                 390                 395                 400
Arg Val Arg Val Lys Lys Arg Ile Lys Arg Trp Asp Gly Tyr Leu Cys
                405                 410                 415
Val Met His Met
            420

<210> 465
<211> 52
<212> PRT
<213> Artificial Sequence

<220>
<223> orf

<400> 465
Met Asn Phe Val Thr Ile Ser Lys Ser Phe Thr Cys Phe Phe Val Phe
1               5                   10                  15
Thr Ala Leu Leu Ser Lys Asn Ile Asn Asn Cys Ser Phe Lys Val Asn
            20                  25                  30
His Asp Leu Ser Leu Gln Asn His Gln Ser His Gln Cys Gln Phe Leu
        35                  40                  45
Lys Pro Gln Pro
    50

<210> 466
<211> 52
<212> PRT
<213> Artificial Sequence

<220>
<223> orf

<400> 466
Met Arg Asn Gly Thr Trp Trp Lys Thr Pro Val Asn Phe Val Ser Pro
1               5                   10                  15
Val Phe Leu Thr Ser Leu Lys Lys Ile Ser Lys Phe Val Gly Ser Ile
            20                  25                  30

Phe Ile Pro Asp Ser Leu Lys Arg Leu Leu Asp Ser Gln Lys Asn His
        35                  40                  45
Thr Leu Asn Pro
    50

<210> 467
<211> 50
<212> PRT
<213> Artificial Sequence

<220>
<223> orf

<400> 467
Met Ile Phe Ser Glu Cys Thr Leu Thr Arg Phe Leu Arg Asn Gly Gln
1               5                   10                  15
Lys Lys Ala Gln Lys Tyr Val Phe Ser Gly Ile Gly Lys Ile Arg Tyr
            20                  25                  30
Gln Ile Thr Lys Ser Ile Ser Glu Ser Ser Tyr Lys Leu Ser Glu Thr
        35                  40                  45
Lys Ile
    50

<210> 468
<211> 53
<212> PRT
<213> Artificial Sequence

<220>
<223> orf

<400> 468
Met Asn Tyr Gln Lys Pro Lys Tyr Asp Asp Cys His Leu Lys Gln Tyr
1               5                   10                  15
Ile Gln Leu Gln Gln Ser Gln Ile His Glu His Arg Gln Glu Lys Arg
            20                  25                  30
Val Leu Phe Gly His Leu Ile Lys Gln Lys Met Lys Leu Leu Cys Gln
        35                  40                  45
Ala Lys Thr Lys Gln
    50

<210> 469
<211> 74
<212> PRT
<213> Artificial Sequence

<220>
<223> orf

<400> 469
Met Asp Cys Asn Pro Thr Ile Gln Arg Leu Glu Phe Tyr His Arg Phe
1               5                   10                  15
Met Ile Leu His Val His Asn Thr Gln Ile Thr Ile Pro Ser Phe Asn
            20                  25                  30
Pro Phe Phe His Ser Tyr Pro Arg Tyr Trp Arg Tyr Leu Pro Val Leu
        35                  40                  45
Phe Phe Ser Ser Ser Cys Arg Ser Cys Pro Ser Val Gly Thr Leu Leu
    50                  55                  60
Val Thr Pro His Ile Val Leu Ile Arg His
65                  70

<210> 470
<211> 63
<212> PRT
<213> Artificial Sequence

<220>
<223> orf

<400> 470
Met Glu Val Leu Ala Ser Val Val Phe Phe Val Leu Val Ser Phe Val
1               5                   10                  15
Ser Val Gly Arg His Ala Pro Arg His Ser Ser His Arg Pro His Ser
            20                  25                  30
Pro Leu Thr Cys Val Arg Leu Leu Leu Leu Val Asp Val Pro Ser Pro
        35                  40                  45
Arg Arg Arg Arg Arg Pro Tyr Phe Val Ala Leu His Val Val Ser
    50                  55                  60

<210> 471
<211> 76
<212> PRT
<213> Artificial Sequence

<220>
<223> orf

<400> 471
Met Arg Phe Phe Thr Arg Thr Leu Val Thr Gly Gly Thr Cys Gln Cys
1               5                   10                  15
Cys Phe Phe Arg Pro Arg Val Val Arg Val Arg Arg Ser Ala Arg Ser
            20                  25                  30
Ser Ser Leu Leu Thr Ser Ser Ser Phe Ala Thr Asn Leu Arg Pro Pro
        35                  40                  45
Ser Pro Ser Arg Arg Arg Ser Phe Ser Ser Ser Ser Ala Ser Ser Leu
    50                  55                  60
Phe Cys Gly Ser Thr Cys Arg Val Ile Val Lys Ser
65                  70                  75

<210> 472
<211> 54
<212> PRT
<213> Artificial Sequence

<220>
<223> orf

<400> 472
Met Ser Ser Ala Arg Ser Ser Gly Ile Leu Thr Pro His Lys Glu Pro
1               5                   10                  15
Asn Ala Pro Leu Lys Arg Leu Pro Phe Lys Thr Leu Lys Ala Ser Gln
            20                  25                  30
Val Phe Trp Thr Asp Lys Thr Pro Asn Leu Arg Ala Tyr Leu His Thr
        35                  40                  45
Ser Ala Ala Ala Phe Glu
    50

<210> 473
<211> 50
<212> PRT

<213> Artificial Sequence

<220>
<223> orf

<400> 473
Met Thr Leu Arg Ile Ser Thr Ile Ile Glu Ser Pro Val Met Glu Cys
1               5                   10                  15
Gln Tyr Cys Glu Ser Ser Ser Trp Ser Ile Gln Ser Ser Lys Ser Ala
            20                  25                  30
Asn Ile Cys Lys Cys Gly Cys His Ser Phe Val Cys Ser Leu Tyr Val
        35                  40                  45
Phe Ser
    50

<210> 474
<211> 51
<212> PRT
<213> Artificial Sequence

<220>
<223> orf

<400> 474
Met Arg Met Pro Phe Leu Cys Leu Leu Val Ile Arg Val Phe Ile Arg
1               5                   10                  15
Ile His Ser Pro Ile Asp Leu Thr Glu Leu Ile Lys Pro Thr Thr Phe
            20                  25                  30
Phe Asn Ile Val Pro Thr Thr Leu Gln Lys Arg Ser Lys Arg Phe Tyr
        35                  40                  45
Glu Val Gly
    50

<210> 475
<211> 84
<212> PRT
<213> Artificial Sequence

<220>
<223> orf

<400> 475
Met Cys Arg Phe Asn Cys Ser Cys Ala Phe Phe Thr Trp Ser Ile Phe
1               5                   10                  15
Arg Ile Val Phe Phe Glu Phe Cys Gln Ser Pro Gln Lys Ile Phe Leu
            20                  25                  30
Leu Leu Arg Ser Met Phe Gln Asn Pro Lys Thr Cys Tyr Ser Ile Pro
        35                  40                  45
Val Leu Ile Cys Leu Ser Arg Leu Gln Ser Leu Tyr Phe Val Ala Arg
    50                  55                  60
Gln Ser Ile Arg Asn Met Gly Phe Phe Ile Val Phe Tyr Ser Gln Phe
65                  70                  75                  80
Leu Ser Pro Leu

<210> 476
<211> 1158
<212> DNA
<213> Artificial Sequence

<220>
<223> orf

<400> 476
ataacacgtt ttgggatttt gaaacataga gcgaagcaac aagaaaattt tttgtggact      60

ctggcaaaat tcaaagaaaa ctatcctaaa gatgctccaa gtgaagaaag cacaagagca     120

attaaatctg cacagaaatt aaaagagtgc ggaaatttct tactattcaa aaatttttac     180

acaatagacc aaactaagct ctcaaaattt catgtatgtg ggcagcattt gttatgtcct     240

ttctgtgctg gtattcgtgc atctaaagca attcaaaaat atactgaacg tgttgatgaa     300

gtgcttaaga aaaatcgcaa gcttaagcct gttttaatca cgtttacggt gaagaatgga     360

atcgacttag gggaaagatc atcccacctc ataaaatcgt ttagaacgct tctgaagcgt     420

cgtagggact atattaaaaa aggtcgtggg tttaatgagt tctgtaagat caatggggct     480

atgtattctt atgaaaacac gtataacgag caaacaaagg aatggcatcc gcacttacat     540

atgtttgcac ttttggatga ttggatagac caggaagagc tttcacagta ttggcattcc     600

atcactggtg attcaatgat tgtagatatt cgcagggtca aaaaagaaaa aggcttaggc     660

tattcaaagg ctgctgcgga agtatgtaaa tatgctctta aattcggtgt tttgtctgtc     720

caaaatactt gggaagcttt caaagtctta aaaggtaagc gtttaagtgg tgcatttggt     780

tctttatggg gtgtcaaaat tcctgaagat cttgctgatg atatgcctga tgaaaaagat     840

cttccttact tagaaatgct ctataaattc gtatttggaa aacagtctta ctatgactta     900

actatgacac gacatgtaga gccacaaaat aaggacgacg ccgacgacga ggagaaggaa     960

cgtcgacgag aaggagaagg cggacgcaag ttagtggcga atgaggacga tgtgaggagt    1020

gacgaggagc gtgccgaccg acggacacga acgacacgag gacgaaaaaa acaacactgg    1080

caagtacctc cagtaacgag ggtacgagtg aaaaaacgga ttaaaagatg ggatggttat    1140

ttgtgtgtta tgcacatg                                                  1158

<210> 477
<211> 213
<212> DNA
<213> Artificial Sequence

<220>
<223> orf

<400> 477
atcataatgg ctgttgcaca tactactgtt ttgtctttgc ttggcaaaga agtttcattt      60

tctgttttat tagatgacca aataaaaccc ttttttcctg acggtgttca tgtatctggc     120

tctgttgaag ctgtatatat tgctttaaat ggcaatcatc atattttggt ttctgataat     180

ttatatgaac tctctgaaat agatttagtt att                                  213

<210> 478
<211> 309
<212> DNA
<213> Artificial Sequence

<220>
<223> orf

<400> 478
atgaatatgg aacctacaaa tttagatatt ttcttaagtg aagttaaaaa aacaggtgaa      60

acgaaattaa ctggtgtttt ccaccaagtt ccatttcgta tgttctctcc acactatcaa     120

cgtgtcgaag gtcttcgaaa ttatatgaat gaacctcgta ataaggttct taactctcta     180

atagaaattg ctttagatca ggtttttgaa aacctatcta aagatcctga aatctataaa     240

gctgttatgg ctgaggtttc aaaaattgac attggtggct ttgatggttc tggagactta     300

gatcgtgat                                                             309

<210> 479
<211> 294
<212> DNA
<213> Artificial Sequence

<220>
<223> orf

<400> 479
atcgtgatta accttgaagg aacaattatt aatgttttta ctcagcaagg cggtaaagac      60

aaaaaaacag gtgaagcttt tgctgatcgt gacaaaattc agctattggg agaattagaa     120

cttcccaatg gtgataaaaa aaacgaatta attgatctta ccgttgaaaa cggaaaggca     180

tatgaaaacc taaaaaataa gcgtgtgagc atcccttgcg gtgctctagc ttctggtcga     240

aatgttattt tttatgttgc aaaaggcgca actccaaaac tcttaaacca agta           294

<210> 480
<211> 1260
<212> DNA
<213> Artificial Sequence

<220>
<223> orf

<400> 480
ttgactgtaa aagacaataa aaaagcccat atttctgatg ctttggcggg cgacgaaata      60

caggctttgc aatctgctaa ggcagatcaa cacagggata gaataacacg ttttgggatt     120

ttgaaacata gagcgaagca acaagaaaat tttttgtgga ctctggcaaa attcaaagaa     180

aactatccta aagatgctcc aagtgaagaa agcacaagag caattaaatc tgcacagaaa     240

ttaaaagagt gcggaaattt cttactattc aaaaattttt acacaataga ccaaactaag 300

ctctcaaaat ttcatgtatg tgggcagcat ttgttatgtc ctttctgtgc tggtattcgt 360

gcatctaaag caattcaaaa atatactgaa cgtgttgatg aagtgcttaa gaaaaatcgc 420

aagcttaagc ctgttttaat cacgtttacg gtgaagaatg gaatcgactt aggggaaaga 480

tcatcccacc tcataaaatc gtttagaacg cttctgaagc gtcgtaggga ctatattaaa 540

aaaggtcgtg ggtttaatga gttctgtaag atcaatgggg ctatgtattc ttatgaaaac 600

acgtataacg agcaaacaaa ggaatggcat ccgcacttac atatgtttgc acttttggat 660

gattggatag accaggaaga gctttcacag tattggcatt ccatcactgg tgattcaatg 720

attgtagata ttcgcagggt caaaaaagaa aaaggcttag gctattcaaa ggctgctgcg 780

gaagtatgta aatatgctct taaattcggt gttttgtctg tccaaaatac ttgggaagct 840

ttcaaagtct taaaaggtaa gcgtttaagt ggtgcatttg gttctttatg gggtgtcaaa 900

attcctgaag atcttgctga tgatatgcct gatgaaaaag atcttcctta cttagaaatg 960

ctctataaat tcgtatttgg aaaacagtct tactatgact taactatgac acgacatgta 1020

gagccacaaa ataaggacga cgccgacgac gaggagaagg aacgtcgacg agaaggagaa 1080

ggcggacgca agttagtggc gaatgaggac gatgtgagga gtgacgagga gcgtgccgac 1140

cgacggacac gaacgacacg aggacgaaaa aaacaacact ggcaagtacc tccagtaacg 1200

agggtacgag tgaaaaaacg gattaaaaga tgggatggtt atttgtgtgt tatgcacatg 1260

<210> 481
<211> 156
<212> DNA
<213> Artificial Sequence

<220>
<223> orf

<400> 481
ctgaattttg tcacgatcag caaaagcttc acctgttttt ttgtctttac cgccttgctg 60

agtaaaaaca ttaataattg ttccttcaag gttaatcacg atctaagtct ccagaaccat 120

caaagccacc aatgtcaatt tttgaaacct cagcca 156

<210> 482
<211> 156
<212> DNA
<213> Artificial Sequence

<220>
<223> orf

<400> 482

atacgaaatg gaacttggtg gaaaacacca gttaatttcg tttcacctgt ttttttaact 60

tcacttaaga aaatatctaa atttgtaggt tccatattca tacctgattc attaaagaga 120

ttattggatt cacaaaagaa tcatacactg aatcca 156

<210> 483
<211> 150
<212> DNA
<213> Artificial Sequence

<220>
<223> orf

<400> 483
ttgatttttt cagaatgtac gcttacgcgt ttcttaagaa acggacaaaa aaaagcccaa 60

aaatatgtat tttcgggcat tggtaaaatc agatatcaaa taactaaatc tatttcagag 120

agttcatata aattatcaga aaccaaaata 150

<210> 484
<211> 159
<212> DNA
<213> Artificial Sequence

<220>
<223> orf

<400> 484
ataaattatc agaaaccaaa atatgatgat tgccatttaa agcaatatat acagcttcaa 60

cagagccaga tacatgaaca ccgtcaggaa aaaagggttt tatttggtca tctaataaaa 120

cagaaaatga aacttctttg ccaagcaaag acaaaacag 159

<210> 485
<211> 222
<212> DNA
<213> Artificial Sequence

<220>
<223> orf

<400> 485
ttggactgca atccgactat tcagcgacta gaattttatc ataggtttat gattttacat 60

gtgcataaca cacaaataac catcccatct tttaatccgt tttttcactc gtaccctcgt 120

tactggaggt acttgccagt gttgtttttt tcgtcctcgt gtcgttcgtg tccgtcggtc 180

ggcacgctcc tcgtcactcc tcacatcgtc ctcattcgcc ac 222

<210> 486
<211> 189
<212> DNA

<213> Artificial Sequence

<220>
<223> orf

<400> 486
ctggaggtac ttgccagtgt tgtttttttc gtcctcgtgt cgttcgtgtc cgtcggtcgg      60

cacgctcctc gtcactcctc acatcgtcct cattcgccac taacttgcgt ccgccttctc     120

cttctcgtcg acgttccttc tcctcgtcgt cggcgtcgtc cttattttgt ggctctacat     180

gtcgtgtca                                                             189

<210> 487
<211> 228
<212> DNA
<213> Artificial Sequence

<220>
<223> orf

<400> 487
atccgttttt tcactcgtac cctcgttact ggaggtactt gccagtgttg tttttttcgt      60

cctcgtgtcg ttcgtgtccg tcggtcggca cgctcctcgt cactcctcac atcgtcctca     120

ttcgccacta acttgcgtcc gccttctcct tctcgtcgac gttccttctc ctcgtcgtcg     180

gcgtcgtcct tattttgtgg ctctacatgt cgtgtcatag ttaagtca                  228

<210> 488
<211> 162
<212> DNA
<213> Artificial Sequence

<220>
<223> orf

<400> 488
atatcatcag caagatcttc aggaattttg acaccccata aagaaccaaa tgcaccactt      60

aaacgcttac cttttaagac tttgaaagct tcccaagtat tttggacaga caaaacaccg     120

aatttaagag catatttaca tacttccgca gcagcctttg aa                        162

<210> 489
<211> 150
<212> DNA
<213> Artificial Sequence

<220>
<223> orf

<400> 489
ttgaccctgc gaatatctac aatcattgaa tcaccagtga tggaatgcca atactgtgaa      60

agctcttcct ggtctatcca atcatccaaa agtgcaaaca tatgtaagtg cggatgccat 120

tcctttgttt gctcgttata cgtgttttca 150

<210> 490
<211> 153
<212> DNA
<213> Artificial Sequence

<220>
<223> orf

<400> 490
gtgcggatgc cattcctttg tttgctcgtt atacgtgttt tcataagaat acatagcccc 60

attgatctta cagaactcat taaacccacg acctttttta atatagtccc tacgacgctt 120

cagaagcgtt ctaaacgatt ttatgaggtg gga 153

<210> 491
<211> 252
<212> DNA
<213> Artificial Sequence

<220>
<223> orf

<400> 491
ctgtgcagat ttaattgctc ttgtgctttc ttcacttgga gcatctttag gatagttttc 60

tttgaatttt gccagagtcc acaaaaaatt ttcttgttgc ttcgctctat gtttcaaaat 120

cccaaaacgt gttattctat ccctgtgttg atctgcctta gcagattgca aagcctgtat 180

ttcgtcgccc gccaaagcat cagaaatatg ggctttttta ttgtctttta cagtcaattt 240

ttgtccccac tt 252

<210> 492
<211> 2593
<212> DNA
<213> Artificial Sequence

<220>
<223> isolate

<400> 492
caacacaggg atagaataac acgttttggt attttgaaac acaggtcgaa gcaacaagaa 60

aactttttgt ggacgctagc aaaattcaaa gaaaactacc caatagactc gccaaatgaa 120

gaaactgtaa aggccctaaa atctgcacag aagttaaacg gctgcggtaa ttttttgctg 180

ttcaaaaatt tttacacaat agatcaaact aagctcacaa agttccatgt ctgtaatcaa 240

catttgcttt gcccattttg tgcaggtatt cgtgcatcta aagcaattca aaaatataca 300

gagcgtgttg atgaagtatt aaagaaaaat cgtaagctca agcccgttct aatcacgctc 360

actgtgaaaa acggtactga cctaagtgaa cggtctgcac acctcataaa atcgcttaga 420

acgcttatta agcgccgtca ggactataat aaaaaaggtc gtggttttaa cgaattttgt 480

aaaattaatg gtgcgatgta ttcgtacgaa aatacttaca atgaacaaac caaagaatgg 540

catccgcacg ttcatatgtt tgctcttttg gatgattgga tagaccagga actactttct 600

gaaacatggc atgaaatcac tggtgattca tttgttgtgg atattcgaaa agttaaaaaa 660

caaaaagact taggctatgc aaaagctgct gctgaggtgt gtaaatacgc tcttaagttt 720

ggggatctaa cggtcgaaaa tacatgggaa gctttcaaag tcctaaaagg taagcgctta 780

agcggtgcat ttggctctct ttatggtgtc aaaattccgg aaaaccttgc tgacgatatg 840

ccagaagaaa aagatcttcc gtatttagag atgctctata agttcgtctt tggaaaacac 900

tcttactaca atcttgaaat tactagacac gtagaaccgc aggcgggaga gcgcgcggag 960

ttaggaggag ttgcgacgac gcccgacgag agatatagcg cgggcggttc tagtgtagac 1020

gatttgagta cggaggcagg tggagggctc gcaccactgc gacgtacgaa aagaggacgc 1080

aaaaaacagc actggcaact accaccagta acgagagtta gagtcaaaaa acgaatcaaa 1140

agatgggatg gttatttgtg tgttatacac atgtaaaatc atgaacctat gataaaattc 1200

tagccgctga atagtaggac ggcaatccta ctacccagct aaccaaaacg atctaagaag 1260

gagatcatca tggctgtttc gaatattact gttttgtctt tgcttggcaa agaagtttca 1320

ttttctgttt cgttagatga ccaaataaaa tccgttttcc ctaatggtgt tcagattgct 1380

ggagctgtcg aacaggtcat tattgcatta aatggaaatc accaaattct tgttggtgat 1440

gagttttatc cagtttctga tattcaaaaa atttatgtca aaacttgtat agaattttcc 1500

taattacccc gagaggggag acctcgaagc ctttattcat aaaggtttcg gggttttttt 1560

atgtctttaa cataatgcgt actattgaaa ataaggcact aaagagtact tatataatct 1620

ttagttcccg aacgagtgtc tacgagtgac gactccagaa taaaaatcac aaattcccct 1680

ttctctgatt acaagctccc ctttgatttt ctccggtaga aacagagcca tccggatggt 1740

ggcgactccc ctgttgaatt atttgcgatt cagtgtatga ttcataatgt gaatcacaaa 1800

aacttttatc aaaattcagg tgagaatatg gaacctacaa atttacaagt attcatgggt 1860

gaagtgaata aaacagcaaa aaccgaaact attggtgttt atcaccaagt accatttcga 1920

atgtctacat ggaattttga gcgccttgag gggttgcgaa attacatgtc agagcctcgt 1980

aacaaggtct taaattctct tattgaaatt gctcttgatc aggttttttc agaactcgaa 2040

aagggtgatg aaaacattaa aaatgccatt ctttcagaat gtgcaaaggt taatgcaatt 2100

caaaaacatg atggttctgg agacttagac aatgattaag ttagagggaa ccgtgattaa 2160

tacatttcat cttgaaggtg gaaaaaataa aaagggggag gaatacgaag ctagtgacaa 2220

ggtgcagtta cttggttcgt tggagctacc caatgggcaa actaaaaacg aatttattga 2280

tcttaaagtc gaagatgcaa gcatctacga tgcattcaaa aataagctta ttagcattag 2340

ttgtggcgca tttcccgcag ggaaaaacgt agtgttttat gttcgaaaag gtgcaaagcc 2400

tgttttagcc gacggtcttt gatttatgtt aaaaaacagt gagttagcga agctactcgc 2460

tgttttttat tttacttgat actattcatt aagtcgggga caaaatttca tgataaatga 2520

caataaaaaa cccctgttat cggacagttt ggcgaccggt gataacaagg gtttgcaatc 2580

tgctaaggca gat 2593

<210> 493
<211> 386
<212> PRT
<213> Artificial Sequence

<220>
<223> orf

<400> 493
Met Thr Arg Phe Gly Ile Leu Lys His Arg Ser Lys Gln Gln Glu Asn
1               5                   10                  15
Phe Leu Trp Thr Leu Ala Lys Phe Lys Glu Asn Tyr Pro Ile Asp Ser
            20                  25                  30
Pro Asn Glu Glu Thr Val Lys Ala Leu Lys Ser Ala Gln Lys Leu Asn
        35                  40                  45
Gly Cys Gly Asn Phe Leu Leu Phe Lys Asn Phe Tyr Thr Ile Asp Gln
    50                  55                  60
Thr Lys Leu Thr Lys Phe His Val Cys Asn Gln His Leu Leu Cys Pro
65                  70                  75                  80
Phe Cys Ala Gly Ile Arg Ala Ser Lys Ala Ile Gln Lys Tyr Thr Glu
                85                  90                  95
Arg Val Asp Glu Val Leu Lys Lys Asn Arg Lys Leu Lys Pro Val Leu
            100                 105                 110
Ile Thr Leu Thr Val Lys Asn Gly Thr Asp Leu Ser Glu Arg Ser Ala
        115                 120                 125
His Leu Ile Lys Ser Leu Arg Thr Leu Ile Lys Arg Arg Gln Asp Tyr
    130                 135                 140
Asn Lys Lys Gly Arg Gly Phe Asn Glu Phe Cys Lys Ile Asn Gly Ala
145                 150                 155                 160
Met Tyr Ser Tyr Glu Asn Thr Tyr Asn Glu Gln Thr Lys Glu Trp His
                165                 170                 175
Pro His Val His Met Phe Ala Leu Leu Asp Asp Trp Ile Asp Gln Glu
            180                 185                 190
Leu Leu Ser Glu Thr Trp His Glu Ile Thr Gly Asp Ser Phe Val Val
        195                 200                 205
Asp Ile Arg Lys Val Lys Lys Gln Lys Asp Leu Gly Tyr Ala Lys Ala
    210                 215                 220
Ala Ala Glu Val Cys Lys Tyr Ala Leu Lys Phe Gly Asp Leu Thr Val
225                 230                 235                 240
Glu Asn Thr Trp Glu Ala Phe Lys Val Leu Lys Gly Lys Arg Leu Ser
                245                 250                 255
Gly Ala Phe Gly Ser Leu Tyr Gly Val Lys Ile Pro Glu Asn Leu Ala
            260                 265                 270
Asp Asp Met Pro Glu Glu Lys Asp Leu Pro Tyr Leu Glu Met Leu Tyr
        275                 280                 285

Lys Phe Val Phe Gly Lys His Ser Tyr Tyr Asn Leu Glu Ile Thr Arg
    290                 295                 300
His Val Glu Pro Gln Ala Gly Glu Arg Ala Glu Leu Gly Gly Val Ala
305                 310                 315                 320
Thr Thr Pro Asp Glu Arg Tyr Ser Ala Gly Gly Ser Ser Val Asp Asp
                325                 330                 335
Leu Ser Thr Glu Ala Gly Gly Gly Leu Ala Pro Leu Arg Arg Thr Lys
            340                 345                 350
Arg Gly Arg Lys Lys Gln His Trp Gln Leu Pro Pro Val Thr Arg Val
        355                 360                 365
Arg Val Lys Lys Arg Ile Lys Arg Trp Asp Gly Tyr Leu Cys Val Ile
    370                 375                 380
His Met
385

<210> 494
<211> 79
<212> PRT
<213> Artificial Sequence

<220>
<223> orf

<400> 494
Met Ile Met Ala Val Ser Asn Ile Thr Val Leu Ser Leu Leu Gly Lys
1               5                   10                  15
Glu Val Ser Phe Ser Val Ser Leu Asp Asp Gln Ile Lys Ser Val Phe
            20                  25                  30
Pro Asn Gly Val Gln Ile Ala Gly Ala Val Glu Gln Val Ile Ile Ala
        35                  40                  45
Leu Asn Gly Asn His Gln Ile Leu Val Gly Asp Glu Phe Tyr Pro Val
    50                  55                  60
Ser Asp Ile Gln Lys Ile Tyr Val Lys Thr Cys Ile Glu Phe Ser
65                  70                  75

<210> 495
<211> 54
<212> PRT
<213> Artificial Sequence

<220>
<223> orf

<400> 495
Met Tyr Arg Ile Phe Leu Ile Thr Pro Arg Gly Glu Thr Ser Lys Pro
1               5                   10                  15
Leu Phe Ile Lys Val Ser Gly Phe Phe Tyr Val Phe Asn Ile Met Arg
            20                  25                  30
Thr Ile Glu Asn Lys Ala Leu Lys Ser Thr Tyr Ile Ile Phe Ser Ser
        35                  40                  45
Arg Thr Ser Val Tyr Glu
    50

<210> 496
<211> 123
<212> PRT
<213> Artificial Sequence

<220>
<223> orf

<400> 496
Met Gln Cys Met Ile His Asn Val Asn His Lys Asn Phe Tyr Gln Asn
1               5                   10                  15
Ser Gly Glu Asn Met Glu Pro Thr Asn Leu Gln Val Phe Met Gly Glu
            20                  25                  30
Val Asn Lys Thr Ala Lys Thr Glu Thr Ile Gly Val Tyr His Gln Val
        35                  40                  45
Pro Phe Arg Met Ser Thr Trp Asn Phe Glu Arg Leu Glu Gly Leu Arg
    50                  55                  60
Asn Tyr Met Ser Glu Pro Arg Asn Lys Val Leu Asn Ser Leu Ile Glu
65                  70                  75                  80
Ile Ala Leu Asp Gln Val Phe Ser Glu Leu Glu Lys Gly Asp Glu Asn
                85                  90                  95
Ile Lys Asn Ala Ile Leu Ser Glu Cys Ala Lys Val Asn Ala Ile Gln
            100                 105                 110
Lys His Asp Gly Ser Gly Asp Leu Asp Asn Asp
        115                 120

<210> 497
<211> 70
<212> PRT
<213> Artificial Sequence

<220>
<223> orf

<400> 497
Met His Phe Ile Leu Lys Val Glu Lys Ile Lys Arg Gly Arg Asn Thr
1               5                   10                  15
Lys Leu Val Thr Arg Cys Ser Tyr Leu Val Arg Trp Ser Tyr Pro Met
            20                  25                  30
Gly Lys Leu Lys Thr Asn Leu Leu Ile Leu Lys Ser Lys Met Gln Ala
        35                  40                  45
Ser Thr Met His Ser Lys Ile Ser Leu Leu Ala Leu Val Val Ala His
    50                  55                  60
Phe Pro Gln Gly Lys Thr
65                  70

<210> 498
<211> 96
<212> PRT
<213> Artificial Sequence

<220>
<223> orf

<400> 498
Met Ile Lys Leu Glu Gly Thr Val Ile Asn Thr Phe His Leu Glu Gly
1               5                   10                  15
Gly Lys Asn Lys Lys Gly Glu Glu Tyr Glu Ala Ser Asp Lys Val Gln
            20                  25                  30
Leu Leu Gly Ser Leu Glu Leu Pro Asn Gly Gln Thr Lys Asn Glu Phe
        35                  40                  45
Ile Asp Leu Lys Val Glu Asp Ala Ser Ile Tyr Asp Ala Phe Lys Asn
    50                  55                  60
Lys Leu Ile Ser Ile Ser Cys Gly Ala Phe Pro Ala Gly Lys Asn Val
65                  70                  75                  80
Val Phe Tyr Val Arg Lys Gly Ala Lys Pro Val Leu Ala Asp Gly Leu
                85                  90                  95

<210> 499
<211> 419
<212> PRT
<213> Artificial Sequence

<220>
<223> orf

<400> 499
Met Ile Asn Asp Asn Lys Lys Pro Leu Leu Ser Asp Ser Leu Ala Thr
1               5                   10                  15
Gly Asp Asn Lys Gly Leu Gln Ser Ala Lys Ala Asp Gln His Arg Asp
            20                  25                  30
Arg Ile Thr Arg Phe Gly Ile Leu Lys His Arg Ser Lys Gln Gln Glu
        35                  40                  45
Asn Phe Leu Trp Thr Leu Ala Lys Phe Lys Glu Asn Tyr Pro Ile Asp
    50                  55                  60
Ser Pro Asn Glu Glu Thr Val Lys Ala Leu Lys Ser Ala Gln Lys Leu
65                  70                  75                  80
Asn Gly Cys Gly Asn Phe Leu Leu Phe Lys Asn Phe Tyr Thr Ile Asp
                85                  90                  95
Gln Thr Lys Leu Thr Lys Phe His Val Cys Asn Gln His Leu Leu Cys
            100                 105                 110
Pro Phe Cys Ala Gly Ile Arg Ala Ser Lys Ala Ile Gln Lys Tyr Thr
        115                 120                 125
Glu Arg Val Asp Glu Val Leu Lys Lys Asn Arg Lys Leu Lys Pro Val
    130                 135                 140
Leu Ile Thr Leu Thr Val Lys Asn Gly Thr Asp Leu Ser Glu Arg Ser
145                 150                 155                 160
Ala His Leu Ile Lys Ser Leu Arg Thr Leu Ile Lys Arg Arg Gln Asp
                165                 170                 175
Tyr Asn Lys Lys Gly Arg Gly Phe Asn Glu Phe Cys Lys Ile Asn Gly
            180                 185                 190
Ala Met Tyr Ser Tyr Glu Asn Thr Tyr Asn Glu Gln Thr Lys Glu Trp
        195                 200                 205
His Pro His Val His Met Phe Ala Leu Leu Asp Asp Trp Ile Asp Gln
    210                 215                 220
Glu Leu Leu Ser Glu Thr Trp His Glu Ile Thr Gly Asp Ser Phe Val
225                 230                 235                 240
Val Asp Ile Arg Lys Val Lys Lys Gln Lys Asp Leu Gly Tyr Ala Lys
                245                 250                 255
Ala Ala Ala Glu Val Cys Lys Tyr Ala Leu Lys Phe Gly Asp Leu Thr
            260                 265                 270
Val Glu Asn Thr Trp Glu Ala Phe Lys Val Leu Lys Gly Lys Arg Leu
        275                 280                 285
Ser Gly Ala Phe Gly Ser Leu Tyr Gly Val Lys Ile Pro Glu Asn Leu
    290                 295                 300
Ala Asp Asp Met Pro Glu Glu Lys Asp Leu Pro Tyr Leu Glu Met Leu
305                 310                 315                 320
Tyr Lys Phe Val Phe Gly Lys His Ser Tyr Tyr Asn Leu Glu Ile Thr
                325                 330                 335
Arg His Val Glu Pro Gln Ala Gly Glu Arg Ala Glu Leu Gly Gly Val
            340                 345                 350
Ala Thr Thr Pro Asp Glu Arg Tyr Ser Ala Gly Gly Ser Ser Val Asp
        355                 360                 365
Asp Leu Ser Thr Glu Ala Gly Gly Gly Leu Ala Pro Leu Arg Arg Thr
    370                 375                 380
Lys Arg Gly Arg Lys Lys Gln His Trp Gln Leu Pro Pro Val Thr Arg
385                 390                 395                 400
Val Arg Val Lys Lys Arg Ile Lys Arg Trp Asp Gly Tyr Leu Cys Val

405                 410                 415
Ile His Met

<210> 500
<211> 50
<212> PRT
<213> Artificial Sequence

<220>
<223> orf

<400> 500
Met Asn Phe Asp Lys Ser Phe Cys Asp Ser His Tyr Glu Ser Tyr Thr
1               5                   10                  15
Glu Ser Gln Ile Ile Gln Gln Gly Ser Arg His His Pro Asp Gly Ser
            20                  25                  30
Val Ser Thr Gly Glu Asn Gln Arg Gly Ala Cys Asn Gln Arg Lys Gly
        35                  40                  45
Asn Leu
    50

<210> 501
<211> 77
<212> PRT
<213> Artificial Sequence

<220>
<223> orf

<400> 501
Met Ile Arg Phe Leu Thr Leu Thr Leu Val Thr Gly Gly Ser Cys Gln
1               5                   10                  15
Cys Cys Phe Leu Arg Pro Leu Phe Val Arg Arg Ser Gly Ala Ser Pro
            20                  25                  30
Pro Pro Ala Ser Val Leu Lys Ser Ser Thr Leu Glu Pro Pro Ala Leu
        35                  40                  45
Tyr Leu Ser Ser Gly Val Val Ala Thr Pro Pro Asn Ser Ala Arg Ser
    50                  55                  60
Pro Ala Cys Gly Ser Thr Cys Leu Val Ile Ser Arg Leu
65                  70                  75

<210> 502
<211> 68
<212> PRT
<213> Artificial Sequence

<220>
<223> orf

<400> 502
Met Val Tyr Pro Ile Ile Gln Lys Ser Lys His Met Asn Val Arg Met
1               5                   10                  15
Pro Phe Phe Gly Leu Phe Ile Val Ser Ile Phe Val Arg Ile His Arg
            20                  25                  30
Thr Ile Asn Phe Thr Lys Phe Val Lys Thr Thr Thr Phe Phe Ile Ile
        35                  40                  45
Val Leu Thr Ala Leu Asn Lys Arg Ser Lys Arg Phe Tyr Glu Val Cys
    50                  55                  60
Arg Pro Phe Thr

65

<210> 503
<211> 52
<212> PRT
<213> Artificial Sequence

<220>
<223> orf

<400> 503
Met Asn Thr Leu Cys Ile Phe Leu Asn Cys Phe Arg Cys Thr Asn Thr
1               5                   10                  15
Cys Thr Lys Trp Ala Lys Gln Met Leu Ile Thr Asp Met Glu Leu Cys
            20                  25                  30
Glu Leu Ser Leu Ile Tyr Cys Val Lys Ile Phe Glu Gln Gln Lys Ile
        35                  40                  45
Thr Ala Ala Val
    50

<210> 504
<211> 1158
<212> DNA
<213> Artificial Sequence

<220>
<223> orf

<400> 504
ataacacgtt ttggtatttt gaaacacagg tcgaagcaac aagaaaactt tttgtggacg      60
ctagcaaaat tcaaagaaaa ctacccaata gactcgccaa atgaagaaac tgtaaaggcc     120
ctaaaatctg cacagaagtt aaacggctgc ggtaattttt tgctgttcaa aaatttttac     180
acaatagatc aaactaagct cacaaagttc catgtctgta atcaacattt gctttgccca     240
ttttgtgcag gtattcgtgc atctaaagca attcaaaaat atacagagcg tgttgatgaa     300
gtattaaaga aaaatcgtaa gctcaagccc gttctaatca cgctcactgt gaaaaacggt     360
actgacctaa gtgaacggtc tgcacacctc ataaaatcgc ttagaacgct tattaagcgc     420
cgtcaggact ataataaaaa aggtcgtggt tttaacgaat tttgtaaaat taatggtgcg     480
atgtattcgt acgaaaatac ttacaatgaa caaaccaaag aatggcatcc gcacgttcat     540
atgtttgctc ttttggatga ttggatagac caggaactac tttctgaaac atggcatgaa     600
atcactggtg attcatttgt tgtggatatt cgaaaagtta aaaaacaaaa agacttaggc     660
tatgcaaaag ctgctgctga ggtgtgtaaa tacgctctta agtttgggga tctaacggtc     720
gaaaatacat gggaagcttt caaagtccta aaaggtaagc gcttaagcgg tgcatttggc     780
tctctttatg gtgtcaaaat tccggaaaac cttgctgacg atatgccaga agaaaaagat     840
cttccgtatt tagagatgct ctataagttc gtctttggaa aacactctta ctacaatctt     900
gaaattacta gacacgtaga accgcaggcg ggagagcgcg cggagttagg aggagttgcg     960

acgacgcccg acgagagata tagcgcgggc ggttctagtg tagacgattt gagtacggag 1020

gcaggtggag ggctcgcacc actgcgacgt acgaaaagag gacgcaaaaa acagcactgg 1080

caactaccac cagtaacgag agttagagtc aaaaaacgaa tcaaaagatg ggatggttat 1140

ttgtgtgtta tacacatg 1158

<210> 505
<211> 237
<212> DNA
<213> Artificial Sequence

<220>
<223> orf

<400> 505
atcatcatgg ctgtttcgaa tattactgtt ttgtctttgc ttggcaaaga agtttcattt 60

tctgtttcgt tagatgacca aataaaatcc gttttcccta atggtgttca gattgctgga 120

gctgtcgaac aggtcattat tgcattaaat ggaaatcacc aaattcttgt tggtgatgag 180

ttttatccag tttctgatat tcaaaaaatt tatgtcaaaa cttgtataga attttcc 237

<210> 506
<211> 162
<212> DNA
<213> Artificial Sequence

<220>
<223> orf

<400> 506
ttgtatagaa ttttcctaat taccccgaga ggggagacct cgaagccttt attcataaag 60

gtttcggggt ttttttatgt ctttaacata atgcgtacta ttgaaaataa ggcactaaag 120

agtacttata taatctttag ttcccgaacg agtgtctacg ag 162

<210> 507
<211> 369
<212> DNA
<213> Artificial Sequence

<220>
<223> orf

<400> 507
attcagtgta tgattcataa tgtgaatcac aaaaactttt atcaaaattc aggtgagaat 60

atggaaccta caaatttaca agtattcatg ggtgaagtga ataaaacagc aaaaaccgaa 120

actattggtg tttatcacca agtaccattt cgaatgtcta catggaattt tgagcgcctt 180

gaggggttgc gaaattacat gtcagagcct cgtaacaagg tcttaaattc tcttattgaa 240

attgctcttg atcaggtttt ttcagaactc gaaaagggtg atgaaaacat taaaaatgcc 300

attctttcag aatgtgcaaa ggttaatgca attcaaaaac atgatggttc tggagactta 360

gacaatgat 369

<210> 508
<211> 210
<212> DNA
<213> Artificial Sequence

<220>
<223> orf

<400> 508
atacatttca tcttgaaggt ggaaaaaata aaaaggggga ggaatacgaa gctagtgaca 60

aggtgcagtt acttggttcg ttggagctac ccaatgggca aactaaaaac gaatttattg 120

atcttaaagt cgaagatgca agcatctacg atgcattcaa aaataagctt attagcatta 180

gttgtggcgc atttcccgca gggaaaaacg 210

<210> 509
<211> 288
<212> DNA
<213> Artificial Sequence

<220>
<223> orf

<400> 509
atgattaagt tagagggaac cgtgattaat acatttcatc ttgaaggtgg aaaaaataaa 60

aagggggagg aatacgaagc tagtgacaag gtgcagttac ttggttcgtt ggagctaccc 120

aatgggcaaa ctaaaaacga atttattgat cttaaagtcg aagatgcaag catctacgat 180

gcattcaaaa ataagcttat tagcattagt tgtggcgcat ttcccgcagg gaaaaacgta 240

gtgttttatg ttcgaaaagg tgcaaagcct gttttagccg acggtctt 288

<210> 510
<211> 1257
<212> DNA
<213> Artificial Sequence

<220>
<223> orf

<400> 510
atgataaatg acaataaaaa acccctgtta tcggacagtt tggcgaccgg tgataacaag 60

ggtttgcaat ctgctaaggc agatcaacac agggatagaa taacacgttt tggtattttg 120

aaacacaggt cgaagcaaca agaaaacttt ttgtggacgc tagcaaaatt caaagaaaac 180

tacccaatag actcgccaaa tgaagaaact gtaaaggccc taaaatctgc acagaagtta 240

aacggctgcg gtaatttttt gctgttcaaa aatttttaca caatagatca aactaagctc 300

acaaagttcc atgtctgtaa tcaacatttg ctttgcccat tttgtgcagg tattcgtgca 360

tctaaagcaa ttcaaaaata tacagagcgt gttgatgaag tattaaagaa aaatcgtaag 420

ctcaagcccg ttctaatcac gctcactgtg aaaaacggta ctgacctaag tgaacggtct 480

gcacacctca taaaatcgct tagaacgctt attaagcgcc gtcaggacta taataaaaaa 540

ggtcgtggtt ttaacgaatt ttgtaaaatt aatggtgcga tgtattcgta cgaaaatact 600

tacaatgaac aaaccaaaga atggcatccg cacgttcata tgtttgctct tttggatgat 660

tggatagacc aggaactact ttctgaaaca tggcatgaaa tcactggtga ttcatttgtt 720

gtggatattc gaaaagttaa aaaacaaaaa gacttaggct atgcaaaagc tgctgctgag 780

gtgtgtaaat acgctcttaa gtttggggat ctaacggtcg aaaatacatg ggaagctttc 840

aaagtcctaa aaggtaagcg cttaagcggt gcatttggct ctctttatgg tgtcaaaatt 900

ccggaaaacc ttgctgacga tatgccagaa gaaaaagatc ttccgtattt agagatgctc 960

tataagttcg tctttggaaa acactcttac tacaatcttg aaattactag acacgtagaa 1020

ccgcaggcgg gagagcgcgc ggagttagga ggagttgcga cgacgcccga cgagagatat 1080

agcgcgggcg gttctagtgt agacgatttg agtacggagg caggtggagg gctcgcacca 1140

ctgcgacgta cgaaaagagg acgcaaaaaa cagcactggc aactaccacc agtaacgaga 1200

gttagagtca aaaaacgaat caaaagatgg gatggttatt tgtgtgttat acacatg 1257

<210> 511
<211> 150
<212> DNA
<213> Artificial Sequence

<220>
<223> orf

<400> 511
ctgaattttg ataaaagttt ttgtgattca cattatgaat catacactga atcgcaaata 60

attcaacagg ggagtcgcca ccatccggat ggctctgttt ctaccggaga aaatcaaagg 120

ggagcttgta atcagagaaa ggggaatttg 150

<210> 512
<211> 231
<212> DNA
<213> Artificial Sequence

<220>
<223> orf

<400> 512
ttgattcgtt ttttgactct aactctcgtt actggtggta gttgccagtg ctgttttttg 60

cgtcctcttt tcgtacgtcg cagtggtgcg agccctccac ctgcctccgt actcaaatcg 120

tctacactag aaccgcccgc gctatatctc tcgtcgggcg tcgtcgcaac tcctcctaac 180

tccgcgcgct ctcccgcctg cggttctacg tgtctagtaa tttcaagatt g 231

<210> 513
<211> 204
<212> DNA
<213> Artificial Sequence

<220>
<223> orf

<400> 513
ctggtctatc caatcatcca aaagagcaaa catatgaacg tgcggatgcc attctttggt 60

ttgttcattg taagtatttt cgtacgaata catcgcacca ttaattttac aaaattcgtt 120

aaaaccacga ccttttttat tatagtcctg acggcgctta ataagcgttc taagcgattt 180

tatgaggtgt gcagaccgtt cact 204

<210> 514
<211> 156
<212> DNA
<213> Artificial Sequence

<220>
<223> orf

<400> 514
atcaacacgc tctgtatatt tttgaattgc tttagatgca cgaatacctg cacaaaatgg 60

gcaaagcaaa tgttgattac agacatggaa ctttgtgagc ttagtttgat ctattgtgta 120

aaaatttttg aacagcaaaa aattaccgca gccgtt 156

<210> 515
<211> 2554
<212> DNA
<213> Artificial Sequence

<220>
<223> isolate

<400> 515
caacacaggg atagaataac acgttttggt attttgaaac acagatcgaa gcaacaagaa 60

aactttttgt ggactcttgc aaaattcaaa gaaaactacc caatagactc gccaaatgaa 120

gaaactgtaa aggctctaaa atctgcacag aagttgagcg gctgtggcaa ttttttgttg 180

ttcaaaaatt tttacacaat agatcaaacc aagctctcaa aatttcatgt ttgtaatcag 240

catttgttgt gtccgttttg tacaggtatt cgtgcttcta aagcaattca aaaatattct 300
gaacgtgttg atgaagtact taaacaaaat cgcaagctca agcctgtttt aatcaccctg 360
acagtgaaaa tggtactgac ctaagggaaa ggtctgaaca tctcataaag tcgcttagaa 420
cgctaattaa gcgtcgtcag gactacaata aaaagaatcg tggttttaac gagttttgca 480
aaatcaacgg tgcaatgtat tcatacgaaa acacctacaa tgaaaaaaca ggcgaatggc 540
atccgcacgt tcatatgttt gcactgctcg atacttggat cgatcaagaa caactttctg 600
agacttggca cgagatcaca ggtgattctt ttgttgttga tattcgaaag gttaaaaaag 660
acaaagactt tggttattca aaggcagcag ctgaagtatg caaatatgct ttaaagtttg 720
gtgatctgtc agttgaacat acctgggaag cgttcaaggt tctcaaaggc aaacgcctaa 780
gcggtgcatt cggttgtctt tatggggtta aaattcctga aaatcttgca gacgacatgc 840
ccgatgaaaa agaccttccg tatttagaaa tgctttatag attcgtgttt ggaaaacagc 900
cttactatga tctacaaatg acacgtcatg tagagccaca ggcgaatgaa cctgagaacg 960
tgaggagctt tgacgaatgt gatcgggttt gtgagccatt ggcgatgacg gacgagacga 1020
ggacgttgcc ggtgcgaggg cacgcagtca cggaacgacc gcagcgagta cgtaaaaagc 1080
aacactggca agtaccacca gtaacgaggg tacgagtgaa aaaacgtatt aaaagatggg 1140
atggttattt gtgtgttatg cacatgtaaa atcataaaca tatgttaaaa ttctagtcgc 1200
tgaatagtcg gatggccgtc caactaccca gctaaccaat cagatctaag aaggagatca 1260
tcatggctgt tgcacatact actgttttgg aaatgctagg caaagaagtc tcattttctg 1320
ttttgctaga tgaccaaata aaatgctttt tccctgaagg tattcagatt actggagacg 1380
ttcaggaagt gattattgcc cttgatggca atcatcaaat cttagttcta gatgaatatt 1440
atcaactctc aagaattgct ctgaaatctt aattagcccc gataggggag acctcaaacc 1500
cattgtttac aatggttttg gggttttttt atgcccttta cataatgtgt attatcttaa 1560
ccgccttact aaagattact catgtaatct ttagcgagtg tctacgagcg tctctccagt 1620
aataaaaatt tcaaaccttt ttccctctga ttacaagctc ccttttgcct ttctccggga 1680
taacccgagc caacccgaat gggtggcgac tcccctattg atttgttatt tattcagtgt 1740
atgattcttt tgtgaatcat ataaattaat caacgaatca ggtatgaata tggaacctac 1800
aaatttagat atttttttaa gcgaagttaa gaagactggt gaatcaaaac ttgttggtat 1860
ttatcatcaa gtcccattcc gtatgttctc tccacattat caacgtgttg aaggccttcg 1920
aaattatatg aatgagcctc ggaacaaagt tcttaattct ttgatcgaaa ttgctttaga 1980
tcaggttttt gaaaacctgt ctaaggatcc agaagtttat aaagcagtaa tgactgaagt 2040
ttataaaatc aatattggtg aatctttcga tggttctgga gatttatcta atgattaaat 2100

tagaaggcac agtcttgaac gttctcgttc aacaaggcgg aaaagataag aaaacaggcg 2160

atcagtttga ggatcgtgac aaggtacaaa ttttgggtgc tatggagctt ccaaacggtg 2220

gcgttaaaaa tgagcttttt acgttatcag tagaaaatta tcgtgatttt aaagacttca 2280

taaatcaaaa aatcacaatt gctgttggtg caatggccag tggtagaaac gtaatttttt 2340

acgttgcaaa gggtgcttta cctgttttag tacaggattg atatttgtta gaaaaaacag 2400

tgagttagcg aagcaactcg ctgttttttt atttacttga tactattcat taagtcgggg 2460

acaaaatttc atgagaaatg acaataaaaa acccctgtta tcagaagctt tggcgggcgt 2520

agataacaag ggttcgcaat ctgctaaggc agat 2554

<210> 516
<211> 122
<212> PRT
<213> Artificial Sequence

<220>
<223> orf

<400> 516
Met Thr Arg Phe Gly Ile Leu Lys His Arg Ser Lys Gln Gln Glu Asn
1               5                   10                  15
Phe Leu Trp Thr Leu Ala Lys Phe Lys Glu Asn Tyr Pro Ile Asp Ser
            20                  25                  30
Pro Asn Glu Glu Thr Val Lys Ala Leu Lys Ser Ala Gln Lys Leu Ser
        35                  40                  45
Gly Cys Gly Asn Phe Leu Leu Phe Lys Asn Phe Tyr Thr Ile Asp Gln
    50                  55                  60
Thr Lys Leu Ser Lys Phe His Val Cys Asn Gln His Leu Leu Cys Pro
65                  70                  75                  80
Phe Cys Thr Gly Ile Arg Ala Ser Lys Ala Ile Gln Lys Tyr Ser Glu
                85                  90                  95
Arg Val Asp Glu Val Leu Lys Gln Asn Arg Lys Leu Lys Pro Val Leu
            100                 105                 110
Ile Thr Leu Thr Val Lys Met Val Leu Thr
        115                 120

<210> 517
<211> 88
<212> PRT
<213> Artificial Sequence

<220>
<223> orf

<400> 517
Met Lys Arg Ile Val Val Leu Thr Ser Phe Ala Lys Ser Thr Val Gln
1               5                   10                  15
Cys Ile His Thr Lys Thr Pro Thr Met Lys Lys Gln Ala Asn Gly Ile
            20                  25                  30
Arg Thr Phe Ile Cys Leu His Cys Ser Ile Leu Gly Ser Ile Lys Asn
        35                  40                  45
Asn Phe Leu Arg Leu Gly Thr Arg Ser Gln Val Ile Leu Leu Leu Leu
    50                  55                  60
Ile Phe Glu Arg Leu Lys Lys Thr Lys Thr Leu Val Ile Gln Arg Gln

65                  70                  75                  80
Gln Leu Lys Tyr Ala Asn Met Leu
                85

<210> 518
<211> 254
<212> PRT
<213> Artificial Sequence

<220>
<223> orf

<400> 518
Met Lys Ser Leu Arg Thr Leu Ile Lys Arg Arg Gln Asp Tyr Asn Lys
1               5                   10                  15
Lys Asn Arg Gly Phe Asn Glu Phe Cys Lys Ile Asn Gly Ala Met Tyr
            20                  25                  30
Ser Tyr Glu Asn Thr Tyr Asn Glu Lys Thr Gly Glu Trp His Pro His
        35                  40                  45
Val His Met Phe Ala Leu Leu Asp Thr Trp Ile Asp Gln Glu Gln Leu
    50                  55                  60
Ser Glu Thr Trp His Glu Ile Thr Gly Asp Ser Phe Val Val Asp Ile
65                  70                  75                  80
Arg Lys Val Lys Lys Asp Lys Asp Phe Gly Tyr Ser Lys Ala Ala Ala
                85                  90                  95
Glu Val Cys Lys Tyr Ala Leu Lys Phe Gly Asp Leu Ser Val Glu His
            100                 105                 110
Thr Trp Glu Ala Phe Lys Val Leu Lys Gly Lys Arg Leu Ser Gly Ala
        115                 120                 125
Phe Gly Cys Leu Tyr Gly Val Lys Ile Pro Glu Asn Leu Ala Asp Asp
    130                 135                 140
Met Pro Asp Glu Lys Asp Leu Pro Tyr Leu Glu Met Leu Tyr Arg Phe
145                 150                 155                 160
Val Phe Gly Lys Gln Pro Tyr Tyr Asp Leu Gln Met Thr Arg His Val
                165                 170                 175
Glu Pro Gln Ala Asn Glu Pro Glu Asn Val Arg Ser Phe Asp Glu Cys
            180                 185                 190
Asp Arg Val Cys Glu Pro Leu Ala Met Thr Asp Glu Thr Arg Thr Leu
        195                 200                 205
Pro Val Arg Gly His Ala Val Thr Glu Arg Pro Gln Arg Val Arg Lys
    210                 215                 220
Lys Gln His Trp Gln Val Pro Pro Val Thr Arg Val Arg Val Lys Lys
225                 230                 235                 240
Arg Ile Lys Arg Trp Asp Gly Tyr Leu Cys Val Met His Met
                245                 250

<210> 519
<211> 77
<212> PRT
<213> Artificial Sequence

<220>
<223> orf

<400> 519
Met Arg Ser Lys Lys Glu Ile Ile Met Ala Val Ala His Thr Thr Val
1               5                   10                  15
Leu Glu Met Leu Gly Lys Glu Val Ser Phe Ser Val Leu Leu Asp Asp
            20                  25                  30
Gln Ile Lys Cys Phe Phe Pro Glu Gly Ile Gln Ile Thr Gly Asp Val
        35                  40                  45

Gln Glu Val Ile Ile Ala Leu Asp Gly Asn His Gln Ile Leu Val Leu
    50                  55                  60
Asp Glu Tyr Tyr Gln Leu Ser Arg Ile Ala Leu Lys Ser
65                  70                  75

<210> 520
<211> 52
<212> PRT
<213> Artificial Sequence

<220>
<223> orf

<400> 520
Met Leu Ser Thr Leu Lys Asn Cys Ser Glu Ile Leu Ile Ser Pro Asp
1               5                   10                  15
Arg Gly Asp Leu Lys Pro Ile Val Tyr Asn Gly Phe Gly Val Phe Leu
            20                  25                  30
Cys Pro Leu His Asn Val Tyr Tyr Leu Asn Arg Leu Thr Lys Asp Tyr
        35                  40                  45
Ser Cys Asn Leu
    50

<210> 521
<211> 109
<212> PRT
<213> Artificial Sequence

<220>
<223> orf

<400> 521
Met Asn Glu Ser Gly Met Asn Met Glu Pro Thr Asn Leu Asp Ile Phe
1               5                   10                  15
Leu Ser Glu Val Lys Lys Thr Gly Glu Ser Lys Leu Val Gly Ile Tyr
            20                  25                  30
His Gln Val Pro Phe Arg Met Phe Ser Pro His Tyr Gln Arg Val Glu
        35                  40                  45
Gly Leu Arg Asn Tyr Met Asn Glu Pro Arg Asn Lys Val Leu Asn Ser
    50                  55                  60
Leu Ile Glu Ile Ala Leu Asp Gln Val Phe Glu Asn Leu Ser Lys Asp
65                  70                  75                  80
Pro Glu Val Tyr Lys Ala Val Met Thr Glu Val Tyr Lys Ile Asn Ile
                85                  90                  95
Gly Glu Ser Phe Asp Gly Ser Gly Asp Leu Ser Asn Asp
            100                 105

<210> 522
<211> 115
<212> PRT
<213> Artificial Sequence

<220>
<223> orf

<400> 522
Met Lys Phe Ile Lys Ser Ile Leu Val Asn Leu Ser Met Val Leu Glu
1               5                   10                  15
Ile Tyr Leu Met Ile Lys Leu Glu Gly Thr Val Leu Asn Val Leu Val
            20                  25                  30

Gln Gln Gly Gly Lys Asp Lys Lys Thr Gly Asp Gln Phe Glu Asp Arg
        35                  40                  45
Asp Lys Val Gln Ile Leu Gly Ala Met Glu Leu Pro Asn Gly Gly Val
    50                  55                  60
Lys Asn Glu Leu Phe Thr Leu Ser Val Glu Asn Tyr Arg Asp Phe Lys
65                  70                  75                  80
Asp Phe Ile Asn Gln Lys Ile Thr Ile Ala Val Gly Ala Met Ala Ser
                85                  90                  95
Gly Arg Asn Val Ile Phe Tyr Val Ala Lys Gly Ala Leu Pro Val Leu
            100                 105                 110
Val Gln Asp
        115

<210> 523
<211> 155
<212> PRT
<213> Artificial Sequence

<220>
<223> orf

<400> 523
Met Arg Asn Asp Asn Lys Lys Pro Leu Leu Ser Glu Ala Leu Ala Gly
1               5                   10                  15
Val Asp Asn Lys Gly Ser Gln Ser Ala Lys Ala Asp Gln His Arg Asp
            20                  25                  30
Arg Ile Thr Arg Phe Gly Ile Leu Lys His Arg Ser Lys Gln Gln Glu
        35                  40                  45
Asn Phe Leu Trp Thr Leu Ala Lys Phe Lys Glu Asn Tyr Pro Ile Asp
    50                  55                  60
Ser Pro Asn Glu Glu Thr Val Lys Ala Leu Lys Ser Ala Gln Lys Leu
65                  70                  75                  80
Ser Gly Cys Gly Asn Phe Leu Leu Phe Lys Asn Phe Tyr Thr Ile Asp
                85                  90                  95
Gln Thr Lys Leu Ser Lys Phe His Val Cys Asn Gln His Leu Leu Cys
            100                 105                 110
Pro Phe Cys Thr Gly Ile Arg Ala Ser Lys Ala Ile Gln Lys Tyr Ser
        115                 120                 125
Glu Arg Val Asp Glu Val Leu Lys Gln Asn Arg Lys Leu Lys Pro Val
    130                 135                 140
Leu Ile Thr Leu Thr Val Lys Met Val Leu Thr
145                 150                 155

<210> 524
<211> 67
<212> PRT
<213> Artificial Sequence

<220>
<223> orf

<400> 524
Met Arg Phe Phe Thr Arg Thr Leu Val Thr Gly Gly Thr Cys Gln Cys
1               5                   10                  15
Cys Phe Leu Arg Thr Arg Cys Gly Arg Ser Val Thr Ala Cys Pro Arg
            20                  25                  30
Thr Gly Asn Val Leu Val Ser Ser Val Ile Ala Asn Gly Ser Gln Thr
        35                  40                  45
Arg Ser His Ser Ser Lys Leu Leu Thr Phe Ser Gly Ser Phe Ala Cys
    50                  55                  60
Gly Ser Thr

65

<210> 525
<211> 89
<212> PRT
<213> Artificial Sequence

<220>
<223> orf

<400> 525
Met Phe Met Ile Leu His Val His Asn Thr Gln Ile Thr Ile Pro Ser
1               5                   10                  15
Phe Asn Thr Phe Phe His Ser Tyr Pro Arg Tyr Trp Trp Tyr Leu Pro
            20                  25                  30
Val Leu Leu Phe Thr Tyr Ser Leu Arg Ser Phe Arg Asp Cys Val Pro
        35                  40                  45
Ser His Arg Gln Arg Pro Arg Leu Val Arg His Arg Gln Trp Leu Thr
    50                  55                  60
Asn Pro Ile Thr Phe Val Lys Ala Pro His Val Leu Arg Phe Ile Arg
65                  70                  75                  80
Leu Trp Leu Tyr Met Thr Cys His Leu
                85

<210> 526
<211> 79
<212> PRT
<213> Artificial Sequence

<220>
<223> orf

<400> 526
Met Arg Ala Leu Ala Pro Ala Thr Ser Ser Ser Arg Pro Ser Ser Pro
1               5                   10                  15
Met Ala His Lys Pro Asp His Ile Arg Gln Ser Ser Ser Arg Ser Gln
            20                  25                  30
Val His Ser Pro Val Ala Leu His Asp Val Ser Phe Val Asp His Ser
        35                  40                  45
Lys Ala Val Phe Gln Thr Arg Ile Tyr Lys Ala Phe Leu Asn Thr Glu
    50                  55                  60
Gly Leu Phe His Arg Ala Cys Arg Leu Gln Asp Phe Gln Glu Phe
65                  70                  75

<210> 527
<211> 58
<212> PRT
<213> Artificial Sequence

<220>
<223> orf

<400> 527
Met Thr Lys Val Phe Val Phe Phe Asn Leu Ser Asn Ile Asn Asn Lys
1               5                   10                  15
Arg Ile Thr Cys Asp Leu Val Pro Ser Leu Arg Lys Leu Phe Leu Ile
            20                  25                  30
Asp Pro Ser Ile Glu Gln Cys Lys His Met Asn Val Arg Met Pro Phe
        35                  40                  45
Ala Cys Phe Phe Ile Val Gly Val Phe Val

50                  55

<210> 528
<211> 56
<212> PRT
<213> Artificial Sequence


<220>
<223> orf

<400> 528
Met Trp Arg Val Tyr Trp Val Val Phe Phe Glu Phe Cys Lys Ser Pro
1               5                   10                  15
Gln Lys Val Phe Leu Leu Leu Arg Ser Val Phe Gln Asn Thr Lys Thr
            20                  25                  30
Cys Tyr Ser Ile Pro Val Leu Ile Cys Leu Ser Arg Leu Arg Thr Leu
        35                  40                  45
Val Ile Tyr Ala Arg Gln Ser Phe
    50                  55

<210> 529
<211> 366
<212> DNA
<213> Artificial Sequence


<220>
<223> orf

<400> 529
ataacacgtt ttggtatttt gaaacacaga tcgaagcaac aagaaaactt tttgtggact      60

cttgcaaaat tcaaagaaaa ctacccaata gactcgccaa atgaagaaac tgtaaaggct     120

ctaaaatctg cacagaagtt gagcggctgt ggcaattttt tgttgttcaa aaatttttac     180

acaatagatc aaaccaagct ctcaaaattt catgtttgta atcagcattt gttgtgtccg     240

ttttgtacag gtattcgtgc ttctaaagca attcaaaaat attctgaacg tgttgatgaa     300

gtacttaaac aaaatcgcaa gctcaagcct gttttaatca ccctgacagt gaaaatggta     360

ctgacc                                                                366


<210> 530
<211> 264
<212> DNA
<213> Artificial Sequence


<220>
<223> orf

<400> 530
ataaaaagaa tcgtggtttt aacgagtttt gcaaaatcaa cggtgcaatg tattcatacg      60

aaaacaccta caatgaaaaa acaggcgaat ggcatccgca cgttcatatg tttgcactgc     120

tcgatacttg gatcgatcaa gaacaacttt ctgagacttg gcacgagatc acaggtgatt     180

cttttgttgt tgatattcga aaggttaaaa aagacaaaga ctttggttat tcaaaggcag     240

cagctgaagt atgcaaatat gctt                                          264

<210> 531
<211> 762
<212> DNA
<213> Artificial Sequence

<220>
<223> orf

<400> 531
ataaagtcgc ttagaacgct aattaagcgt cgtcaggact acaataaaaa gaatcgtggt      60

tttaacgagt tttgcaaaat caacggtgca atgtattcat acgaaaacac ctacaatgaa     120

aaaacaggcg aatggcatcc gcacgttcat atgtttgcac tgctcgatac ttggatcgat     180

caagaacaac tttctgagac ttggcacgag atcacaggtg attcttttgt tgttgatatt     240

cgaaaggtta aaaaagacaa agactttggt tattcaaagg cagcagctga agtatgcaaa     300

tatgctttaa agtttggtga tctgtcagtt gaacatacct gggaagcgtt caaggttctc     360

aaaggcaaac gcctaagcgg tgcattcggt tgtctttatg gggttaaaat tcctgaaaat     420

cttgcagacg acatgcccga tgaaaaagac cttccgtatt tagaaatgct ttatagattc     480

gtgtttggaa aacagcctta ctatgatcta caaatgacac gtcatgtaga gccacaggcg     540

aatgaacctg agaacgtgag gagctttgac gaatgtgatc gggtttgtga gccattggcg     600

atgacggacg agacgaggac gttgccggtg cgagggcacg cagtcacgga acgaccgcag     660

cgagtacgta aaaagcaaca ctggcaagta ccaccagtaa cgagggtacg agtgaaaaaa     720

cgtattaaaa gatgggatgg ttatttgtgt gttatgcaca tg                        762

<210> 532
<211> 231
<212> DNA
<213> Artificial Sequence

<220>
<223> orf

<400> 532
atcagatcta agaaggagat catcatggct gttgcacata ctactgtttt ggaaatgcta      60

ggcaaagaag tctcattttc tgttttgcta gatgaccaaa taaaatgctt tttccctgaa     120

ggtattcaga ttactggaga cgttcaggaa gtgattattg cccttgatgg caatcatcaa     180

atcttagttc tagatgaata ttatcaactc tcaagaattg ctctgaaatc t              231

<210> 533
<211> 156
<212> DNA

<213> Artificial Sequence

<220>
<223> orf

<400> 533
atattatcaa ctctcaagaa ttgctctgaa atcttaatta gccccgatag gggagacctc 60

aaacccattg tttacaatgg ttttggggtt tttttatgcc ctttacataa tgtgtattat 120

cttaaccgcc ttactaaaga ttactcatgt aatctt 156

<210> 534
<211> 327
<212> DNA
<213> Artificial Sequence

<220>
<223> orf

<400> 534
atcaacgaat caggtatgaa tatggaacct acaaatttag atattttttt aagcgaagtt 60

aagaagactg gtgaatcaaa acttgttggt atttatcatc aagtcccatt ccgtatgttc 120

tctccacatt atcaacgtgt tgaaggcctt cgaaattata tgaatgagcc tcggaacaaa 180

gttcttaatt ctttgatcga aattgcttta gatcaggttt ttgaaaacct gtctaaggat 240

ccagaagttt ataaagcagt aatgactgaa gtttataaaa tcaatattgg tgaatctttc 300

gatggttctg gagatttatc taatgat 327

<210> 535
<211> 345
<212> DNA
<213> Artificial Sequence

<220>
<223> orf

<400> 535
ctgaagttta taaaatcaat attggtgaat ctttcgatgg ttctggagat ttatctaatg 60

attaaattag aaggcacagt cttgaacgtt ctcgttcaac aaggcggaaa agataagaaa 120

acaggcgatc agtttgagga tcgtgacaag gtacaaattt tgggtgctat ggagcttcca 180

aacggtggcg ttaaaaatga gctttttacg ttatcagtag aaaattatcg tgattttaaa 240

gacttcataa atcaaaaaat cacaattgct gttggtgcaa tggccagtgg tagaaacgta 300

attttttacg ttgcaaaggg tgctttacct gttttagtac aggat 345

<210> 536
<211> 465
<212> DNA

<213> Artificial Sequence

<220>
<223> orf

<400> 536
atgagaaatg acaataaaaa acccctgtta tcagaagctt tggcgggcgt agataacaag      60

ggttcgcaat ctgctaaggc agatcaacac agggatagaa taacacgttt tggtattttg     120

aaacacagat cgaagcaaca agaaaacttt ttgtggactc ttgcaaaatt caaagaaaac     180

tacccaatag actcgccaaa tgaagaaact gtaaaggctc taaaatctgc acagaagttg     240

agcggctgtg gcaatttttt gttgttcaaa aatttttaca caatagatca aaccaagctc     300

tcaaaatttc atgtttgtaa tcagcatttg ttgtgtccgt tttgtacagg tattcgtgct     360

tctaaagcaa ttcaaaaata ttctgaacgt gttgatgaag tacttaaaca aaatcgcaag     420

ctcaagcctg ttttaatcac cctgacagtg aaaatggtac tgacc                     465

<210> 537
<211> 201
<212> DNA
<213> Artificial Sequence

<220>
<223> orf

<400> 537
atacgttttt tcactcgtac cctcgttact ggtggtactt gccagtgttg ctttttacgt      60

actcgctgcg gtcgttccgt gactgcgtgc cctcgcaccg gcaacgtcct cgtctcgtcc     120

gtcatcgcca atggctcaca aacccgatca cattcgtcaa agctcctcac gttctcaggt     180

tcattcgcct gtggctctac a                                               201

<210> 538
<211> 267
<212> DNA
<213> Artificial Sequence

<220>
<223> orf

<400> 538
atgtttatga ttttacatgt gcataacaca caaataacca tcccatcttt taatacgttt      60

tttcactcgt accctcgtta ctggtggtac ttgccagtgt tgctttttac gtactcgctg     120

cggtcgttcc gtgactgcgt gccctcgcac cggcaacgtc ctcgtctcgt ccgtcatcgc     180

caatggctca caaacccgat cacattcgtc aaagctcctc acgttctcag gttcattcgc     240

ctgtggctct acatgacgtg tcatttg                                         267

<210> 539
<211> 237
<212> DNA
<213> Artificial Sequence

<220>
<223> orf

<400> 539
ctgcgtgccc tcgcaccggc aacgtcctcg tctcgtccgt catcgccaat ggctcacaaa 60

cccgatcaca ttcgtcaaag ctcctcacgt tctcaggttc attcgcctgt ggctctacat 120

gacgtgtcat ttgtagatca tagtaaggct gttttccaaa cacgaatcta taaagcattt 180

ctaaatacgg aaggtctttt tcatcgggca tgtcgtctgc aagattttca ggaattt 237

<210> 540
<211> 174
<212> DNA
<213> Artificial Sequence

<220>
<223> orf

<400> 540
ataaccaaag tctttgtctt ttttaacctt tcgaatatca acaacaaaag aatcacctgt 60

gatctcgtgc caagtctcag aaagttgttc ttgatcgatc caagtatcga gcagtgcaaa 120

catatgaacg tgcggatgcc attcgcctgt tttttcattg taggtgtttt cgta 174

<210> 541
<211> 168
<212> DNA
<213> Artificial Sequence

<220>
<223> orf

<400> 541
atttggcgag tctattgggt agttttcttt gaattttgca agagtccaca aaaagttttc 60

ttgttgcttc gatctgtgtt tcaaaatacc aaaacgtgtt attctatccc tgtgttgatc 120

tgccttagca gattgcgaac ccttgttatc tacgcccgcc aaagcttc 168

<210> 542
<211> 2505
<212> DNA
<213> Artificial Sequence

<220>
<223> orf

<400> 542
aaggcagatc aacacaggga tagaataaca cgttttggaa ttttgaaaca tagatctaaa 60
ctgcaagaac aatatttgtg gacgcaggtt gatttcaaat ccgaaggcga agacgaccta 120
tctaataagg ctcttaaggc tgcaacaaaa ttaaaaggtt gtggtcaatt tctgctgttc 180
agaaattact acacgataga ccaaatcaag ctcgaaaaat tccatgtatg cggtcaacat 240
ttgctatgcc caatgtgtgc tggtattcgt gcagctcgtt ctatgagtcg ttatattcag 300
cgcattgaag aattgatgcg tcagaatcgc catttaaagc ccgtattgat cactttaacg 360
gtaaagaatg gtgaagacct gcaagaacgc tttgatcacc ttacacgctc atttaagacc 420
cttttagctc gttatcggga ctataagaaa aaaggcaggg gttttaatca attctgtaag 480
attgatggtg cgttttatac cactgaatac acttacaacg ataagactaa gcaatggcat 540
ccgcacattc atatttttgc tttactcaat gaatggattg accaggaaga attggcagaa 600
acttggcacg acattaccct ggattcttat atcgttgata ttcgcagagt taaaaaaaaa 660
ccaaagaaca cggttatagc aaagctgttg cagaggtttg taagtacgct cttaagttta 720
gcgatctgtc actggagaac acatgggaag cctttttgac tcttaaagat aaacgcctta 780
caggttcttt cggttctatg cacggtgtaa aagtccctga aaaagctaca gacgaaatgc 840
caaaagaaga actcccatat ctagagctgc agtaccgttt cgtatttggt ccgaaatctt 900
attacaacct agaaataaca aaagacgtaa ggccacaaac taaggaatga ggggtgagga 960
ggagctgcga cgaccgacac gaccgaatga ctttgtgagt ggcgataagg acgaagcaat 1020
cgcatccgac atcccggctt gcgggtgcgg tgcgaagcga ggacgttaag caagcctacc 1080
tgtatataca gaacttgtag ggggttcgac cccttagacc ccaaagggcg cactttacca 1140
aacccttcag gttcggcagt gctcccaaaa attcccattg gagaacatta tgaaattaat 1200
ctatagaccg tatccattta agcctaacta cgtttttctt gaagatcgct taagttctta 1260
tgttctttgc ccttggtggt ggaattaatt aacaaagcta ctggagaaca ttctctccag 1320
tagtaaccga gggggcggag tgcccccgaa ctgacatcat ctaaattctc aaaccttcct 1380
tctctgatta caagctcccc ttagattcta agcgggagtt tttagccatc ccgaatgggt 1440
ggcggcacaa agaaaattga caaaataata attagtatta ctatggtatt accaaccaca 1500
caaaatatat ggtacatgaa atgggtaata catttaaaaa tcttggtcat ttaaaagctg 1560
tatcagaatt agaagaggaa atggagggta atacctctca aattcattca cctctaaaac 1620
ctttttctct taaagttcca caacacgttc ttgagtcttt ggactttttg gctgaaacaa 1680
tgggtcttag ccgtagtgct ttaatcgtta atatttttga tcaatatctt ggtcaagtta 1740
cttatgaata tttagatggc tataacggtt tagtaactcc agaagcacct cctgaagttg 1800
ttgtttctga agatttagaa caacgtttaa aaaattcaga attatcagaa tcagcaaaac 1860

aatatttaac agatggtgtc tatcacgttg cttttggaat gtgacatgac acaaatgcct 1920

aaagaacatt tagaacaagc gaaagacttt tttgcttgga ttatccaaga acacatgaaa 1980

gacaaccgaa ccttaacaac catcaaaatc tttaaacaag atggccaatt aaaaattttg 2040

gagttatcaa atgattaaat tagaagggac attgttaaac acctttactc aacaaggcgg 2100

acaaaataag aaaactggtg atcagtttga agaccgtgac aaagtacaaa ttctaggcgc 2160

tatggatctg ccaaacggtg acgttaaaaa tgaacttttt acgttatctg tagagaatta 2220

tcgggacttt aaagacttcc taaatcacaa aatttctgtt gcagttggtg caatggcaag 2280

tggacgtaat gtgatttttt atgttgctaa aggcgcaaag cctattttag ccgaacaagc 2340

gtgaaaccta aaaaaaacag tgagttagcg aagcggctcg ctgttttttc tttctcttga 2400

tactattaac taaagtgggg acaagaattg cttaaaaaag acaatgaaaa agcccatatt 2460

tctgatgctt tggcgggcga cgaaatacag gctttgcaat ctgct 2505

<210> 543
<211> 52
<212> PRT
<213> Artificial Sequence

<220>
<223> orf

<400> 543
Met Lys Arg Leu Trp Ser Ile Ser Ala Val Gln Lys Leu Leu His Asp
1               5                   10                  15
Arg Pro Asn Gln Ala Arg Lys Ile Pro Cys Met Arg Ser Thr Phe Ala
            20                  25                  30
Met Pro Asn Val Cys Trp Tyr Ser Cys Ser Ser Phe Tyr Glu Ser Leu
        35                  40                  45
Tyr Ser Ala His
    50

<210> 544
<211> 81
<212> PRT
<213> Artificial Sequence

<220>
<223> orf

<400> 544
Met Gly Thr Ile Arg Lys Lys Ala Gly Val Leu Ile Asn Ser Val Arg
1               5                   10                  15
Leu Met Val Arg Phe Ile Pro Leu Asn Thr Leu Thr Thr Ile Arg Leu
            20                  25                  30
Ser Asn Gly Ile Arg Thr Phe Ile Phe Leu Leu Tyr Ser Met Asn Gly
        35                  40                  45
Leu Thr Arg Lys Asn Trp Gln Lys Leu Gly Thr Thr Leu Pro Trp Ile
    50                  55                  60
Leu Ile Ser Leu Ile Phe Ala Glu Leu Lys Lys Asn Gln Arg Thr Arg
65                  70                  75                  80
Leu

<210> 545
<211> 244
<212> PRT
<213> Artificial Sequence

<220>
<223> orf

<400> 545
Met Thr Arg Phe Gly Ile Leu Lys His Arg Ser Lys Leu Gln Glu Gln
1               5                   10                  15
Tyr Leu Trp Thr Gln Val Asp Phe Lys Ser Glu Gly Glu Asp Asp Leu
            20                  25                  30
Ser Asn Lys Ala Leu Lys Ala Ala Thr Lys Leu Lys Gly Cys Gly Gln
        35                  40                  45
Phe Leu Leu Phe Arg Asn Tyr Tyr Thr Ile Asp Gln Ile Lys Leu Glu
    50                  55                  60
Lys Phe His Val Cys Gly Gln His Leu Leu Cys Pro Met Cys Ala Gly
65                  70                  75                  80
Ile Arg Ala Ala Arg Ser Met Ser Arg Tyr Ile Gln Arg Ile Glu Glu
                85                  90                  95
Leu Met Arg Gln Asn Arg His Leu Lys Pro Val Leu Ile Thr Leu Thr
            100                 105                 110
Val Lys Asn Gly Glu Asp Leu Gln Glu Arg Phe Asp His Leu Thr Arg
        115                 120                 125
Ser Phe Lys Thr Leu Leu Ala Arg Tyr Arg Asp Tyr Lys Lys Lys Gly
    130                 135                 140
Arg Gly Phe Asn Gln Phe Cys Lys Ile Asp Gly Ala Phe Tyr Thr Thr
145                 150                 155                 160
Glu Tyr Thr Tyr Asn Asp Lys Thr Lys Gln Trp His Pro His Ile His
                165                 170                 175
Ile Phe Ala Leu Leu Asn Glu Trp Ile Asp Gln Glu Glu Leu Ala Glu
            180                 185                 190
Thr Trp His Asp Ile Thr Leu Asp Ser Tyr Ile Val Asp Ile Arg Arg
        195                 200                 205
Val Lys Lys Lys Pro Lys Asn Thr Val Ile Ala Lys Leu Leu Gln Arg
    210                 215                 220
Phe Val Ser Thr Leu Leu Ser Leu Ala Ile Cys His Trp Arg Thr His
225                 230                 235                 240
Gly Lys Pro Phe


<210> 546
<211> 74
<212> PRT
<213> Artificial Sequence

<220>
<223> orf

<400> 546
Met Ser Leu Glu Asn Thr Trp Glu Ala Phe Leu Thr Leu Lys Asp Lys
1               5                   10                  15
Arg Leu Thr Gly Ser Phe Gly Ser Met His Gly Val Lys Val Pro Glu
            20                  25                  30
Lys Ala Thr Asp Glu Met Pro Lys Glu Glu Leu Pro Tyr Leu Glu Leu
        35                  40                  45
Gln Tyr Arg Phe Val Phe Gly Pro Lys Ser Tyr Tyr Asn Leu Glu Ile
    50                  55                  60

Thr Lys Asp Val Arg Pro Gln Thr Lys Glu
65                  70

<210> 547
<211> 60
<212> PRT
<213> Artificial Sequence

<220>
<223> orf

<400> 547
Met Arg His Pro Gly Leu Arg Val Arg Cys Glu Ala Arg Thr Leu Ser
1               5                   10                  15
Lys Pro Thr Cys Ile Tyr Arg Thr Cys Arg Gly Phe Asp Pro Leu Asp
            20                  25                  30
Pro Lys Gly Arg Thr Leu Pro Asn Pro Ser Gly Ser Ala Val Leu Pro
        35                  40                  45
Lys Ile Pro Ile Gly Glu His Tyr Glu Ile Asn Leu
    50                  55                  60

<210> 548
<211> 138
<212> PRT
<213> Artificial Sequence

<220>
<223> orf

<400> 548
Met Thr Asn His Thr Lys Tyr Met Val His Glu Met Gly Asn Thr Phe
1               5                   10                  15
Lys Asn Leu Gly His Leu Lys Ala Val Ser Glu Leu Glu Glu Glu Met
            20                  25                  30
Glu Gly Asn Thr Ser Gln Ile His Ser Pro Leu Lys Pro Phe Ser Leu
        35                  40                  45
Lys Val Pro Gln His Val Leu Glu Ser Leu Asp Phe Leu Ala Glu Thr
    50                  55                  60
Met Gly Leu Ser Arg Ser Ala Leu Ile Val Asn Ile Phe Asp Gln Tyr
65                  70                  75                  80
Leu Gly Gln Val Thr Tyr Glu Tyr Leu Asp Gly Tyr Asn Gly Leu Val
                85                  90                  95
Thr Pro Glu Ala Pro Pro Glu Val Val Val Ser Glu Asp Leu Glu Gln
            100                 105                 110
Arg Leu Lys Asn Ser Glu Leu Ser Glu Ser Ala Lys Gln Tyr Leu Thr
        115                 120                 125
Asp Gly Val Tyr His Val Ala Phe Gly Met
    130                 135

<210> 549
<211> 61
<212> PRT
<213> Artificial Sequence

<220>
<223> orf

<400> 549
Met Val Ser Ile Thr Leu Leu Leu Glu Cys Asp Met Thr Gln Met Pro
1               5                   10                  15

Lys Glu His Leu Glu Gln Ala Lys Asp Phe Phe Ala Trp Ile Ile Gln
            20                  25                  30
Glu His Met Lys Asp Asn Arg Thr Leu Thr Thr Ile Lys Ile Phe Lys
        35                  40                  45
Gln Asp Gly Gln Leu Lys Ile Leu Glu Leu Ser Asn Asp
    50                  55                  60

<210> 550
<211> 97
<212> PRT
<213> Artificial Sequence

<220>
<223> orf

<400> 550
Met Ile Lys Leu Glu Gly Thr Leu Leu Asn Thr Phe Thr Gln Gln Gly
1               5                   10                  15
Gly Gln Asn Lys Lys Thr Gly Asp Gln Phe Glu Asp Arg Asp Lys Val
            20                  25                  30
Gln Ile Leu Gly Ala Met Asp Leu Pro Asn Gly Asp Val Lys Asn Glu
        35                  40                  45
Leu Phe Thr Leu Ser Val Glu Asn Tyr Arg Asp Phe Lys Asp Phe Leu
    50                  55                  60
Asn His Lys Ile Ser Val Ala Val Gly Ala Met Ala Ser Gly Arg Asn
65                  70                  75                  80
Val Ile Phe Tyr Val Ala Lys Gly Ala Lys Pro Ile Leu Ala Glu Gln
                85                  90                  95
Ala

<210> 551
<211> 278
<212> PRT
<213> Artificial Sequence

<220>
<223> orf

<400> 551
Met Leu Lys Lys Asp Asn Glu Lys Ala His Ile Ser Asp Ala Leu Ala
1               5                   10                  15
Gly Asp Glu Ile Gln Ala Leu Gln Ser Ala Lys Ala Asp Gln His Arg
            20                  25                  30
Asp Arg Ile Thr Arg Phe Gly Ile Leu Lys His Arg Ser Lys Leu Gln
        35                  40                  45
Glu Gln Tyr Leu Trp Thr Gln Val Asp Phe Lys Ser Glu Gly Glu Asp
    50                  55                  60
Asp Leu Ser Asn Lys Ala Leu Lys Ala Ala Thr Lys Leu Lys Gly Cys
65                  70                  75                  80
Gly Gln Phe Leu Leu Phe Arg Asn Tyr Tyr Thr Ile Asp Gln Ile Lys
                85                  90                  95
Leu Glu Lys Phe His Val Cys Gly Gln His Leu Leu Cys Pro Met Cys
            100                 105                 110
Ala Gly Ile Arg Ala Ala Arg Ser Met Ser Arg Tyr Ile Gln Arg Ile
        115                 120                 125
Glu Glu Leu Met Arg Gln Asn Arg His Leu Lys Pro Val Leu Ile Thr
    130                 135                 140
Leu Thr Val Lys Asn Gly Glu Asp Leu Gln Glu Arg Phe Asp His Leu
145                 150                 155                 160
Thr Arg Ser Phe Lys Thr Leu Leu Ala Arg Tyr Arg Asp Tyr Lys Lys

                    165                         170                         175
Lys Gly Arg Gly Phe Asn Gln Phe Cys Lys Ile Asp Gly Ala Phe Tyr
                180                         185                         190
Thr Thr Glu Tyr Thr Tyr Asn Asp Lys Thr Lys Gln Trp His Pro His
            195                         200                         205
Ile His Ile Phe Ala Leu Leu Asn Glu Trp Ile Asp Gln Glu Glu Leu
    210                         215                         220
Ala Glu Thr Trp His Asp Ile Thr Leu Asp Ser Tyr Ile Val Asp Ile
225                         230                         235                         240
Arg Arg Val Lys Lys Lys Pro Lys Asn Thr Val Ile Ala Lys Leu Leu
                    245                         250                         255
Gln Arg Phe Val Ser Thr Leu Leu Ser Leu Ala Ile Cys His Trp Arg
                260                         265                         270
Thr His Gly Lys Pro Phe
            275

<210> 552
<211> 56
<212> PRT
<213> Artificial Sequence

<220>
<223> orf

<400> 552
Met Phe Asn Trp Pro Ser Cys Leu Lys Ile Leu Met Val Val Lys Val
1               5                   10                  15
Arg Leu Ser Phe Met Cys Ser Trp Ile Ile Gln Ala Lys Lys Ser Phe
                20                  25                  30
Ala Cys Ser Lys Cys Ser Leu Gly Ile Cys Val Met Ser His Ser Lys
            35                  40                  45
Ser Asn Val Ile Asp Thr Ile Cys
    50                  55

<210> 553
<211> 63
<212> PRT
<213> Artificial Sequence

<220>
<223> orf

<400> 553
Met Phe Phe Arg His Leu Cys His Val Thr Phe Gln Lys Gln Arg Asp
1               5                   10                  15
Arg His His Leu Leu Asn Ile Val Leu Leu Ile Leu Ile Ile Leu Asn
                20                  25                  30
Phe Leu Asn Val Val Leu Asn Leu Gln Lys Gln Gln Leu Gln Glu Val
            35                  40                  45
Leu Leu Glu Leu Leu Asn Arg Tyr Ser His Leu Asn Ile His Lys
    50                  55                  60

<210> 554
<211> 54
<212> PRT
<213> Artificial Sequence

<220>
<223> orf

<400> 554
Met Arg Glu Gly Arg Phe Glu Asn Leu Asp Asp Val Ser Ser Gly Ala
1               5                   10                  15
Leu Arg Pro Leu Gly Tyr Tyr Trp Arg Glu Cys Ser Pro Val Ala Leu
            20                  25                  30
Leu Ile Asn Ser Thr Thr Lys Gly Lys Glu His Lys Asn Leu Ser Asp
        35                  40                  45
Leu Gln Glu Lys Arg Ser
    50

<210> 555
<211> 89
<212> PRT
<213> Artificial Sequence

<220>
<223> orf

<400> 555
Met Gly Ile Phe Gly Ser Thr Ala Glu Pro Glu Gly Phe Gly Lys Val
1               5                   10                  15
Arg Pro Leu Gly Ser Lys Gly Ser Asn Pro Leu Gln Val Leu Tyr Ile
            20                  25                  30
Gln Val Gly Leu Leu Asn Val Leu Ala Ser His Arg Thr Arg Lys Pro
        35                  40                  45
Gly Cys Arg Met Arg Leu Leu Arg Pro Tyr Arg His Ser Gln Ser His
    50                  55                  60
Ser Val Val Ser Val Val Ala Ala Pro Pro His Pro Ser Phe Leu Ser
65                  70                  75                  80
Leu Trp Pro Tyr Val Phe Cys Tyr Phe
                85

<210> 556
<211> 54
<212> PRT
<213> Artificial Sequence

<220>
<223> orf

<400> 556
Met Ser Ser Leu Arg Thr Ala Pro Ala Ser Arg Asp Val Gly Cys Asp
1               5                   10                  15
Cys Phe Val Leu Ile Ala Thr His Lys Val Ile Arg Ser Cys Arg Ser
            20                  25                  30
Ser Gln Leu Leu Leu Thr Pro His Ser Leu Val Cys Gly Leu Thr Ser
        35                  40                  45
Phe Val Ile Ser Arg Leu
    50

<210> 557
<211> 69
<212> PRT
<213> Artificial Sequence

<220>
<223> orf

<400> 557
Met Lys Arg Tyr Cys Ser Ser Arg Tyr Gly Ser Ser Ser Phe Gly Ile

1                   5                   10                  15
Ser Ser Val Ala Phe Ser Gly Thr Phe Thr Pro Cys Ile Glu Pro Lys
            20                  25                  30
Glu Pro Val Arg Arg Leu Ser Leu Arg Val Lys Lys Ala Ser His Val
        35                  40                  45
Phe Ser Ser Asp Arg Ser Leu Asn Leu Arg Ala Tyr Leu Gln Thr Ser
    50                  55                  60
Ala Thr Ala Leu Leu
65

<210> 558
<211> 62
<212> PRT
<213> Artificial Sequence


<220>
<223> orf

<400> 558
Met Asn Leu Arg Pro Gln Ile Leu Phe Leu Gln Phe Arg Ser Met Phe
1               5                   10                  15
Gln Asn Ser Lys Thr Cys Tyr Ser Ile Pro Val Leu Ile Cys Leu Ser
            20                  25                  30
Arg Leu Gln Ser Leu Tyr Phe Val Ala Arg Gln Ser Ile Arg Asn Met
        35                  40                  45
Gly Phe Phe Ile Val Phe Phe Lys Gln Phe Leu Ser Pro Leu
    50                  55                  60

<210> 559
<211> 156
<212> DNA
<213> Artificial Sequence


<220>
<223> orf

<400> 559
attaaaaggt tgtggtcaat ttctgctgtt cagaaattac tacacgatag accaaatcaa      60

gctcgaaaaa ttccatgtat gcggtcaaca tttgctatgc ccaatgtgtg ctggtattcg     120

tgcagctcgt tctatgagtc gttatattca gcgcat                               156


<210> 560
<211> 243
<212> DNA
<213> Artificial Sequence


<220>
<223> orf

<400> 560
atcgggacta taagaaaaaa ggcaggggtt ttaatcaatt ctgtaagatt gatggtgcgt      60

tttataccac tgaatacact tacaacgata agactaagca atggcatccg cacattcata     120

tttttgcttt actcaatgaa tggattgacc aggaagaatt ggcagaaact tggcacgaca     180

ttaccctgga ttcttatatc gttgatattc gcagagttaa aaaaaaacca aagaacacgg     240

tta                                                                 243

<210> 561
<211> 732
<212> DNA
<213> Artificial Sequence

<220>
<223> orf

<400> 561
ataacacgtt ttggaatttt gaaacataga tctaaactgc aagaacaata tttgtggacg      60

caggttgatt tcaaatccga aggcgaagac gacctatcta ataaggctct taaggctgca     120

acaaaattaa aaggttgtgg tcaatttctg ctgttcagaa attactacac gatagaccaa     180

atcaagctcg aaaaattcca tgtatgcggt caacatttgc tatgcccaat gtgtgctggt     240

attcgtgcag ctcgttctat gagtcgttat attcagcgca ttgaagaatt gatgcgtcag     300

aatcgccatt taaagcccgt attgatcact ttaacggtaa agaatggtga agacctgcaa     360

gaacgctttg atcaccttac acgctcattt aagacccttt tagctcgtta tcgggactat     420

aagaaaaaag gcaggggttt taatcaattc tgtaagattg atggtgcgtt ttataccact     480

gaatacactt acaacgataa gactaagcaa tggcatccgc acattcatat ttttgcttta     540

ctcaatgaat ggattgacca ggaagaattg gcagaaactt ggcacgacat taccctggat     600

tcttatatcg ttgatattcg cagagttaaa aaaaaaccaa agaacacggt tatagcaaag     660

ctgttgcaga ggtttgtaag tacgctctta agtttagcga tctgtcactg gagaacacat     720

gggaagcctt tt                                                         732

<210> 562
<211> 222
<212> DNA
<213> Artificial Sequence

<220>
<223> orf

<400> 562
ctgtcactgg agaacacatg ggaagccttt ttgactctta aagataaacg ccttacaggt      60

tctttcggtt ctatgcacgg tgtaaaagtc cctgaaaaag ctacagacga aatgccaaaa     120

gaagaactcc catatctaga gctgcagtac cgtttcgtat ttggtccgaa atcttattac     180

aacctagaaa taacaaaaga cgtaaggcca caaactaagg aa                        222

<210> 563
<211> 180
<212> DNA

<213> Artificial Sequence

<220>
<223> orf

<400> 563
atccgacatc ccggcttgcg ggtgcggtgc gaagcgagga cgttaagcaa gcctacctgt 60

atatacagaa cttgtagggg gttcgacccc ttagacccca aagggcgcac tttaccaaac 120

ccttcaggtt cggcagtgct cccaaaaatt cccattggag aacattatga aattaatcta 180

<210> 564
<211> 414
<212> DNA
<213> Artificial Sequence

<220>
<223> orf

<400> 564
attaccaacc acacaaaata tatggtacat gaaatgggta atacatttaa aaatcttggt 60

catttaaaag ctgtatcaga attagaagag gaaatggagg gtaatacctc tcaaattcat 120

tcacctctaa aacctttttc tcttaaagtt ccacaacacg ttcttgagtc tttggacttt 180

ttggctgaaa caatgggtct tagccgtagt gctttaatcg ttaatatttt tgatcaatat 240

cttggtcaag ttacttatga atatttagat ggctataacg gtttagtaac tccagaagca 300

cctcctgaag ttgttgtttc tgaagattta gaacaacgtt taaaaaattc agaattatca 360

gaatcagcaa aacaatattt aacagatggt gtctatcacg ttgcttttgg aatg 414

<210> 565
<211> 183
<212> DNA
<213> Artificial Sequence

<220>
<223> orf

<400> 565
atggtgtcta tcacgttgct tttggaatgt gacatgacac aaatgcctaa agaacattta 60

gaacaagcga aagacttttt tgcttggatt atccaagaac acatgaaaga caaccgaacc 120

ttaacaacca tcaaaatctt taaacaagat ggccaattaa aaattttgga gttatcaaat 180

gat 183

<210> 566
<211> 291
<212> DNA
<213> Artificial Sequence

<220>
<223> orf

<400> 566
atgattaaat tagaagggac attgttaaac acctttactc aacaaggcgg acaaaataag 60

aaaactggtg atcagtttga agaccgtgac aaagtacaaa ttctaggcgc tatggatctg 120

ccaaacggtg acgttaaaaa tgaacttttt acgttatctg tagagaatta tcgggacttt 180

aaagacttcc taaatcacaa aatttctgtt gcagttggtg caatggcaag tggacgtaat 240

gtgatttttt atgttgctaa aggcgcaaag cctattttag ccgaacaagc g 291


<210> 567
<211> 834
<212> DNA
<213> Artificial Sequence


<220>
<223> orf

<400> 567
ttgcttaaaa aagacaatga aaaagcccat atttctgatg ctttggcggg cgacgaaata 60

caggctttgc aatctgctaa ggcagatcaa cacagggata gaataacacg ttttggaatt 120

ttgaaacata gatctaaact gcaagaacaa tatttgtgga cgcaggttga tttcaaatcc 180

gaaggcgaag acgacctatc taataaggct cttaaggctg caacaaaatt aaaaggttgt 240

ggtcaatttc tgctgttcag aaattactac acgatagacc aaatcaagct cgaaaaattc 300

catgtatgcg gtcaacattt gctatgccca atgtgtgctg gtattcgtgc agctcgttct 360

atgagtcgtt atattcagcg cattgaagaa ttgatgcgtc agaatcgcca tttaaagccc 420

gtattgatca ctttaacggt aaagaatggt gaagacctgc aagaacgctt tgatcacctt 480

acacgctcat ttaagaccct tttagctcgt tatcgggact ataagaaaaa aggcaggggt 540

tttaatcaat tctgtaagat tgatggtgcg ttttatacca ctgaatacac ttacaacgat 600

aagactaagc aatggcatcc gcacattcat atttttgctt tactcaatga atggattgac 660

caggaagaat tggcagaaac ttggcacgac attaccctgg attcttatat cgttgatatt 720

cgcagagtta aaaaaaaacc aaagaacacg gttatagcaa agctgttgca gaggtttgta 780

agtacgctct taagtttagc gatctgtcac tggagaacac atgggaagcc tttt 834


<210> 568
<211> 168
<212> DNA
<213> Artificial Sequence


<220>
<223> orf

<400> 568
atttttaatt ggccatcttg tttaaagatt ttgatggttg ttaaggttcg gttgtctttc      60

atgtgttctt ggataatcca agcaaaaaag tctttcgctt gttctaaatg ttctttaggc     120

atttgtgtca tgtcacattc caaaagcaac gtgatagaca ccatctgt                  168


<210> 569
<211> 189
<212> DNA
<213> Artificial Sequence


<220>
<223> orf

<400> 569
atgttcttta ggcatttgtg tcatgtcaca ttccaaaagc aacgtgatag acaccatctg      60

ttaaatattg ttttgctgat tctgataatt ctgaattttt taaacgttgt tctaaatctt     120

cagaaacaac aacttcagga ggtgcttctg gagttactaa accgttatag ccatctaaat     180

attcataag                                                             189


<210> 570
<211> 162
<212> DNA
<213> Artificial Sequence


<220>
<223> orf

<400> 570
atcagagaag gaaggtttga gaatttagat gatgtcagtt cgggggcact ccgccccctc      60

ggttactact ggagagaatg ttctccagta gctttgttaa ttaattccac caccaagggc     120

aaagaacata agaacttaag cgatcttcaa gaaaaacgta gt                        162


<210> 571
<211> 267
<212> DNA
<213> Artificial Sequence


<220>
<223> orf

<400> 571
atgggaattt ttgggagcac tgccgaacct gaagggtttg gtaaagtgcg ccctttgggg      60

tctaaggggt cgaaccccct acaagttctg tatatacagg taggcttgct taacgtcctc     120

gcttcgcacc gcacccgcaa gccgggatgt cggatgcgat tgcttcgtcc ttatcgccac     180

tcacaaagtc attcggtcgt gtcggtcgtc gcagctcctc ctcacccctc attccttagt     240

ttgtggcctt acgtcttttg ttatttc                                          267

<210> 572
<211> 162
<212> DNA
<213> Artificial Sequence

<220>
<223> orf

<400> 572
acgtcctcgc ttcgcaccgc acccgcaagc cgggatgtcg gatgcgattg cttcgtcctt      60

atcgccactc acaaagtcat tcggtcgtgt cggtcgtcgc agctcctcct cacccctcat     120

tccttagttt gtggccttac gtcttttgtt atttctaggt tg                        162

<210> 573
<211> 207
<212> DNA
<213> Artificial Sequence

<220>
<223> orf

<400> 573
acgaaacggt actgcagctc tagatatggg agttcttctt ttggcatttc gtctgtagct      60

ttttcaggga cttttacacc gtgcatagaa ccgaaagaac ctgtaaggcg tttatcttta     120

agagtcaaaa aggcttccca tgtgttctcc agtgacagat cgctaaactt aagagcgtac     180

ttacaaacct ctgcaacagc tttgcta                                         207

<210> 574
<211> 186
<212> DNA
<213> Artificial Sequence

<220>
<223> orf

<400> 574
atcaacctgc gtccacaaat attgttcttg cagtttagat ctatgtttca aaattccaaa      60

acgtgttatt ctatccctgt gttgatctgc cttagcagat tgcaaagcct gtatttcgtc     120

gcccgccaaa gcatcagaaa tatgggcttt ttcattgtct tttttaagca attcttgtcc     180

ccactt                                                                186

<210> 575
<211> 2590
<212> DNA
<213> Artificial Sequence

<220>
<223> isolate

<400> 575
caacacaggg atagaataac acgttttggt attttgaaac acaggtcgaa gcaacaagaa 60

aactttttgt ggacgctagc aaaattcaaa gaaaactacc caatagactc gccaaatgaa 120

gaaactgtaa aggccctaaa atctgcacag aagttaaacg gctgcggtaa ttttttgctg 180

ttcaaaaatc tttacacaat agatcaaact aagctcacaa aatttcatgt ctgtaatcaa 240

catttgcttt gcccattttg tgcaggtatt cgtgcatcta aagcaattca aaaatataca 300

gaacgtgttg atgaagtatt aaagaaaaat cgtaagctca agcccgttct aatcacgctc 360

actgtgaaaa acggtactga cctaagtgaa cggtctgcac acctcataaa atcgcttaga 420

acgcttatta agcgccgtca ggactataat aaaaaaggtc gtggttttaa cgaattttgt 480

aaaattaatg gtgcgatgta ctcgtacgaa aatacttaca atgaacaaac caaagaatgg 540

catccgcacg ttcatatgtt tgctcctttg gatgattgga tagaccagga actactttct 600

gaaacatggc atgaaatcac tggtgattca tttgttgtgg acattcgaaa agttaaaaaa 660

caaaaagact taggctatgc aaaagctgct gctgaggtgt gtaaatacgc tcttaagttt 720

ggggatctaa cggtcgaaaa tacatgggaa gcttttaaag tcctaaaagg taagcgctta 780

agcggtgcat ttggctctct ttatggtgtc aaaattcctg aaaaccttgc tgacgatatg 840

ccagaagaaa aagatcttcc gtatttagag atgctctata agttcgtctt tggaaaacac 900

tcttactaca atctcgaaat tactagacac gtaggaccgc aggcgggaga gcgcgcggag 960

ttaggaggag ttgcgacgac gcccgacgag agatatagcg cgggcggttc tagtgtagac 1020

gatttgagta cggaggcagg tggagggctc gcaccactgc gacgtacgaa aagaggacgc 1080

aaaaaacagc actggcaatt accaccagta acgagagtta gagtcaaaaa acgaatcaaa 1140

agatgggatg gttatttgtg tgttatacac atgtaaaatc atgaacctat gataaaattc 1200

tagtcgctga atagtaggac ggcaatccta ctacccagct aaccaaaacg atctattgag 1260

gagatcatca tggctgtgcc acatactaca ttgcttggct atctaggcaa acacatcaca 1320

ttcaaagttc cttgtccaat tgagttcaat aactcaggtt ttgatcagct ttcagggaaa 1380

gtgatcgctg ttaatattga attaaatgaa aaacattctt tgtgtgttat gcatgacgaa 1440

tcaacagatt cggctgatta ttacgaattt gaaaaaatgc taatacagcc ctaattttta 1500

gatttgtacc cgagagggga gacctcgaag tctttattta taaaggtttc ggggtttttt 1560

tatgtcttta acataatgcg tactattgaa aataaggcac taaagagtac ttatataatc 1620

tttagttccc gaacgagtgt ctacgagtga cgactccgga ataaaaatta caaatccccc 1680

tttctctgat tacaagctcc cctttttttc gttctccggt aagaacagag ccatcccgaa 1740

tgggtggcga cccccctatt gatttgttct tgattcagtg tatgattcat ttgtgaatca 1800

cataatcttg ttaatgaatc aggtatgaat atggaaccta caaatttaga tattttttta 1860

agtgaagtta aaaaaacagg tgaaacgaaa ttaactggtg ttttccacca agttccattt 1920

cgaatgttct ctccacacta tcgacgtgtt gaaggtcttc gcaattatat gaatgagcct 1980

cgtaataaag ttcttaattc tctaattgaa attgctttag atcaggtttt tgaaaatctg 2040

tctaaggatc cagaaattta tgaagctgta atggccgaag tttcaaaaat tgatattggt 2100

acgtttgatg gctctggaga cttagatcgt gattaaacta gagggaaccg tgataaatac 2160

atttcatctt gaaggtggaa aaaataaaaa gggggaagaa tacgaagcca gtgacaaggt 2220

gcagttactt ggttcgttgg agctacccaa tgggcaaatt aaaaacgaat tgattgatct 2280

taaagtcgaa gatgcaagca tctacgatgc attcaaaaat aagcttatta gcattagttg 2340

tggcgcattt cccgcaggga aaaacgtcgt gttttatgtt cgaaaaggtg caaagcctgt 2400

tttagctgac ggtctttgat ttatgttaaa aaacagtgag ttagcgaagc tactcgctgt 2460

tttttatttt acttgatact attcattaag tcggggacaa aatttcatga taaatgacaa 2520

taaaaaaccc ctgttatcgg acagtttggc gaccggtgat aacaagggtt tgcaatctgc 2580

taaggcagat 2590

<210> 576
<211> 386
<212> PRT
<213> Artificial Sequence

<220>
<223> orf

<400> 576
Met Thr Arg Phe Gly Ile Leu Lys His Arg Ser Lys Gln Gln Glu Asn
1               5                   10                  15
Phe Leu Trp Thr Leu Ala Lys Phe Lys Glu Asn Tyr Pro Ile Asp Ser
            20                  25                  30
Pro Asn Glu Glu Thr Val Lys Ala Leu Lys Ser Ala Gln Lys Leu Asn
        35                  40                  45
Gly Cys Gly Asn Phe Leu Leu Phe Lys Asn Leu Tyr Thr Ile Asp Gln
    50                  55                  60
Thr Lys Leu Thr Lys Phe His Val Cys Asn Gln His Leu Leu Cys Pro
65                  70                  75                  80
Phe Cys Ala Gly Ile Arg Ala Ser Lys Ala Ile Gln Lys Tyr Thr Glu
                85                  90                  95
Arg Val Asp Glu Val Leu Lys Lys Asn Arg Lys Leu Lys Pro Val Leu
            100                 105                 110
Ile Thr Leu Thr Val Lys Asn Gly Thr Asp Leu Ser Glu Arg Ser Ala
        115                 120                 125
His Leu Ile Lys Ser Leu Arg Thr Leu Ile Lys Arg Arg Gln Asp Tyr
    130                 135                 140
Asn Lys Lys Gly Arg Gly Phe Asn Glu Phe Cys Lys Ile Asn Gly Ala
145                 150                 155                 160
Met Tyr Ser Tyr Glu Asn Thr Tyr Asn Glu Gln Thr Lys Glu Trp His
                165                 170                 175

Pro His Val His Met Phe Ala Pro Leu Asp Asp Trp Ile Asp Gln Glu
            180                 185                 190
Leu Leu Ser Glu Thr Trp His Glu Ile Thr Gly Asp Ser Phe Val Val
        195                 200                 205
Asp Ile Arg Lys Val Lys Lys Gln Lys Asp Leu Gly Tyr Ala Lys Ala
    210                 215                 220
Ala Ala Glu Val Cys Lys Tyr Ala Leu Lys Phe Gly Asp Leu Thr Val
225                 230                 235                 240
Glu Asn Thr Trp Glu Ala Phe Lys Val Leu Lys Gly Lys Arg Leu Ser
                245                 250                 255
Gly Ala Phe Gly Ser Leu Tyr Gly Val Lys Ile Pro Glu Asn Leu Ala
            260                 265                 270
Asp Asp Met Pro Glu Glu Lys Asp Leu Pro Tyr Leu Glu Met Leu Tyr
        275                 280                 285
Lys Phe Val Phe Gly Lys His Ser Tyr Tyr Asn Leu Glu Ile Thr Arg
    290                 295                 300
His Val Gly Pro Gln Ala Gly Glu Arg Ala Glu Leu Gly Gly Val Ala
305                 310                 315                 320
Thr Thr Pro Asp Glu Arg Tyr Ser Ala Gly Gly Ser Ser Val Asp Asp
                325                 330                 335
Leu Ser Thr Glu Ala Gly Gly Gly Leu Ala Pro Leu Arg Arg Thr Lys
            340                 345                 350
Arg Gly Arg Lys Lys Gln His Trp Gln Leu Pro Pro Val Thr Arg Val
        355                 360                 365
Arg Val Lys Lys Arg Ile Lys Arg Trp Asp Gly Tyr Leu Cys Val Ile
    370                 375                 380
His Met
385

<210> 577
<211> 79
<212> PRT
<213> Artificial Sequence

<220>
<223> orf

<400> 577
Met Glu Glu Ile Ile Met Ala Val Pro His Thr Thr Leu Leu Gly Tyr
1               5                   10                  15
Leu Gly Lys His Ile Thr Phe Lys Val Pro Cys Pro Ile Glu Phe Asn
            20                  25                  30
Asn Ser Gly Phe Asp Gln Leu Ser Gly Lys Val Ile Ala Val Asn Ile
        35                  40                  45
Glu Leu Asn Glu Lys His Ser Leu Cys Val Met His Asp Glu Ser Thr
    50                  55                  60
Asp Ser Ala Asp Tyr Tyr Glu Phe Glu Lys Met Leu Ile Gln Pro
65                  70                  75

<210> 578
<211> 115
<212> PRT
<213> Artificial Sequence

<220>
<223> orf

<400> 578
Met Cys Glu Ser His Asn Leu Val Asn Glu Ser Gly Met Asn Met Glu
1               5                   10                  15
Pro Thr Asn Leu Asp Ile Phe Leu Ser Glu Val Lys Lys Thr Gly Glu

20                  25                  30
Thr Lys Leu Thr Gly Val Phe His Gln Val Pro Phe Arg Met Phe Ser
        35                  40                  45
Pro His Tyr Arg Arg Val Glu Gly Leu Arg Asn Tyr Met Asn Glu Pro
    50                  55                  60
Arg Asn Lys Val Leu Asn Ser Leu Ile Glu Ile Ala Leu Asp Gln Val
65                  70                  75                  80
Phe Glu Asn Leu Ser Lys Asp Pro Glu Ile Tyr Glu Ala Val Met Ala
                85                  90                  95
Glu Val Ser Lys Ile Asp Ile Gly Thr Phe Asp Gly Ser Gly Asp Leu
            100                 105                 110
Asp Arg Asp
        115

<210> 579
<211> 97
<212> PRT
<213> Artificial Sequence

<220>
<223> orf

<400> 579
Met Val Ile Lys Leu Glu Gly Thr Val Ile Asn Thr Phe His Leu Glu
1               5                   10                  15
Gly Gly Lys Asn Lys Lys Gly Glu Glu Tyr Glu Ala Ser Asp Lys Val
            20                  25                  30
Gln Leu Leu Gly Ser Leu Glu Leu Pro Asn Gly Gln Ile Lys Asn Glu
        35                  40                  45
Leu Ile Asp Leu Lys Val Glu Asp Ala Ser Ile Tyr Asp Ala Phe Lys
    50                  55                  60
Asn Lys Leu Ile Ser Ile Ser Cys Gly Ala Phe Pro Ala Gly Lys Asn
65                  70                  75                  80
Val Val Phe Tyr Val Arg Lys Gly Ala Lys Pro Val Leu Ala Asp Gly
                85                  90                  95
Leu

<210> 580
<211> 419
<212> PRT
<213> Artificial Sequence

<220>
<223> orf

<400> 580
Met Ile Asn Asp Asn Lys Lys Pro Leu Leu Ser Asp Ser Leu Ala Thr
1               5                   10                  15
Gly Asp Asn Lys Gly Leu Gln Ser Ala Lys Ala Asp Gln His Arg Asp
            20                  25                  30
Arg Ile Thr Arg Phe Gly Ile Leu Lys His Arg Ser Lys Gln Gln Glu
        35                  40                  45
Asn Phe Leu Trp Thr Leu Ala Lys Phe Lys Glu Asn Tyr Pro Ile Asp
    50                  55                  60
Ser Pro Asn Glu Glu Thr Val Lys Ala Leu Lys Ser Ala Gln Lys Leu
65                  70                  75                  80
Asn Gly Cys Gly Asn Phe Leu Leu Phe Lys Asn Leu Tyr Thr Ile Asp
                85                  90                  95
Gln Thr Lys Leu Thr Lys Phe His Val Cys Asn Gln His Leu Leu Cys
            100                 105                 110

Pro Phe Cys Ala Gly Ile Arg Ala Ser Lys Ala Ile Gln Lys Tyr Thr
        115                 120                 125
Glu Arg Val Asp Glu Val Leu Lys Lys Asn Arg Lys Leu Lys Pro Val
    130                 135                 140
Leu Ile Thr Leu Thr Val Lys Asn Gly Thr Asp Leu Ser Glu Arg Ser
145                 150                 155                 160
Ala His Leu Ile Lys Ser Leu Arg Thr Leu Ile Lys Arg Arg Gln Asp
                165                 170                 175
Tyr Asn Lys Lys Gly Arg Gly Phe Asn Glu Phe Cys Lys Ile Asn Gly
            180                 185                 190
Ala Met Tyr Ser Tyr Glu Asn Thr Tyr Asn Glu Gln Thr Lys Glu Trp
        195                 200                 205
His Pro His Val His Met Phe Ala Pro Leu Asp Asp Trp Ile Asp Gln
    210                 215                 220
Glu Leu Leu Ser Glu Thr Trp His Glu Ile Thr Gly Asp Ser Phe Val
225                 230                 235                 240
Val Asp Ile Arg Lys Val Lys Lys Gln Lys Asp Leu Gly Tyr Ala Lys
                245                 250                 255
Ala Ala Ala Glu Val Cys Lys Tyr Ala Leu Lys Phe Gly Asp Leu Thr
            260                 265                 270
Val Glu Asn Thr Trp Glu Ala Phe Lys Val Leu Lys Gly Lys Arg Leu
        275                 280                 285
Ser Gly Ala Phe Gly Ser Leu Tyr Gly Val Lys Ile Pro Glu Asn Leu
    290                 295                 300
Ala Asp Asp Met Pro Glu Glu Lys Asp Leu Pro Tyr Leu Glu Met Leu
305                 310                 315                 320
Tyr Lys Phe Val Phe Gly Lys His Ser Tyr Tyr Asn Leu Glu Ile Thr
                325                 330                 335
Arg His Val Gly Pro Gln Ala Gly Glu Arg Ala Glu Leu Gly Gly Val
            340                 345                 350
Ala Thr Thr Pro Asp Glu Arg Tyr Ser Ala Gly Gly Ser Ser Val Asp
        355                 360                 365
Asp Leu Ser Thr Glu Ala Gly Gly Gly Leu Ala Pro Leu Arg Arg Thr
    370                 375                 380
Lys Arg Gly Arg Lys Lys Gln His Trp Gln Leu Pro Pro Val Thr Arg
385                 390                 395                 400
Val Arg Val Lys Lys Arg Ile Lys Arg Trp Asp Gly Tyr Leu Cys Val
                405                 410                 415
Ile His Met

<210> 581
<211> 85
<212> PRT
<213> Artificial Sequence

<220>
<223> orf

<400> 581
Met Arg Asn Gly Thr Trp Trp Lys Thr Pro Val Asn Phe Val Ser Pro
1               5                   10                  15
Val Phe Leu Thr Ser Leu Lys Lys Ile Ser Lys Phe Val Gly Ser Ile
            20                  25                  30
Phe Ile Pro Asp Ser Leu Thr Arg Leu Cys Asp Ser Gln Met Asn His
        35                  40                  45
Thr Leu Asn Gln Glu Gln Ile Asn Arg Gly Val Ala Thr His Ser Gly
    50                  55                  60
Trp Leu Cys Ser Tyr Arg Arg Thr Lys Lys Arg Gly Ala Cys Asn Gln
65                  70                  75                  80
Arg Lys Gly Asp Leu
                85

<210> 582
<211> 50
<212> PRT
<213> Artificial Sequence

<220>
<223> orf

<400> 582
Met Lys Asn Lys Ser Ile Gly Gly Ser Pro Pro Ile Arg Asp Gly Ser
1               5                   10                  15
Val Leu Thr Gly Glu Arg Lys Lys Gly Glu Leu Val Ile Arg Glu Arg
            20                  25                  30
Gly Ile Cys Asn Phe Tyr Ser Gly Val Val Thr Arg Arg His Ser Phe
        35                  40                  45
Gly Asn
    50

<210> 583
<211> 77
<212> PRT
<213> Artificial Sequence

<220>
<223> orf

<400> 583
Met Ile Arg Phe Leu Thr Leu Thr Leu Val Thr Gly Gly Asn Cys Gln
1               5                   10                  15
Cys Cys Phe Leu Arg Pro Leu Phe Val Arg Arg Ser Gly Ala Ser Pro
            20                  25                  30
Pro Pro Ala Ser Val Leu Lys Ser Ser Thr Leu Glu Pro Pro Ala Leu
        35                  40                  45
Tyr Leu Ser Ser Gly Val Val Ala Thr Pro Pro Asn Ser Ala Arg Ser
    50                  55                  60
Pro Ala Cys Gly Pro Thr Cys Leu Val Ile Ser Arg Leu
65                  70                  75

<210> 584
<211> 68
<212> PRT
<213> Artificial Sequence

<220>
<223> orf

<400> 584
Met Val Tyr Pro Ile Ile Gln Arg Ser Lys His Met Asn Val Arg Met
1               5                   10                  15
Pro Phe Phe Gly Leu Phe Ile Val Ser Ile Phe Val Arg Val His Arg
            20                  25                  30
Thr Ile Asn Phe Thr Lys Phe Val Lys Thr Thr Thr Phe Phe Ile Ile
        35                  40                  45
Val Leu Thr Ala Leu Asn Lys Arg Ser Lys Arg Phe Tyr Glu Val Cys
    50                  55                  60
Arg Pro Phe Thr
65

<210> 585

<211> 52
<212> PRT
<213> Artificial Sequence

<220>
<223> orf

<400> 585
Met Asn Thr Phe Cys Ile Phe Leu Asn Cys Phe Arg Cys Thr Asn Thr
1               5                   10                  15
Cys Thr Lys Trp Ala Lys Gln Met Leu Ile Thr Asp Met Lys Phe Cys
            20                  25                  30
Glu Leu Ser Leu Ile Tyr Cys Val Lys Ile Phe Glu Gln Gln Lys Ile
        35                  40                  45
Thr Ala Ala Val
    50

<210> 586
<211> 1158
<212> DNA
<213> Artificial Sequence

<220>
<223> orf

<400> 586
ataacacgtt ttggtatttt gaaacacagg tcgaagcaac aagaaaactt tttgtggacg      60

ctagcaaaat tcaaagaaaa ctacccaata gactcgccaa atgaagaaac tgtaaaggcc     120

ctaaaatctg cacagaagtt aaacggctgc ggtaattttt tgctgttcaa aaatctttac     180

acaatagatc aaactaagct cacaaaattt catgtctgta atcaacattt gctttgccca     240

ttttgtgcag gtattcgtgc atctaaagca attcaaaaat atacagaacg tgttgatgaa     300

gtattaaaga aaaatcgtaa gctcaagccc gttctaatca cgctcactgt gaaaaacggt     360

actgacctaa gtgaacggtc tgcacacctc ataaaatcgc ttagaacgct tattaagcgc     420

cgtcaggact ataataaaaa aggtcgtggt tttaacgaat tttgtaaaat taatggtgcg     480

atgtactcgt acgaaaatac ttacaatgaa caaaccaaag aatggcatcc gcacgttcat     540

atgtttgctc ctttggatga ttggatagac caggaactac tttctgaaac atggcatgaa     600

atcactggtg attcatttgt tgtggacatt cgaaaagtta aaaaacaaaa agacttaggc     660

tatgcaaaag ctgctgctga ggtgtgtaaa tacgctctta agtttgggga tctaacggtc     720

gaaaatacat gggaagcttt taaagtccta aaaggtaagc gcttaagcgg tgcatttggc     780

tctctttatg gtgtcaaaat tcctgaaaac cttgctgacg atatgccaga agaaaaagat     840

cttccgtatt tagagatgct ctataagttc gtctttggaa aacactctta ctacaatctc     900

gaaattacta gacacgtagg accgcaggcg ggagagcgcg cggagttagg aggagttgcg     960

acgacgcccg acgagagata tagcgcgggc ggttctagtg tagacgattt gagtacggag    1020

gcaggtggag ggctcgcacc actgcgacgt acgaaaagag gacgcaaaaa acagcactgg    1080

caattaccac cagtaacgag agttagagtc aaaaaacgaa tcaaaagatg ggatggttat 1140

ttgtgtgtta tacacatg 1158

<210> 587
<211> 237
<212> DNA
<213> Artificial Sequence

<220>
<223> orf

<400> 587
attgaggaga tcatcatggc tgtgccacat actacattgc ttggctatct aggcaaacac 60

atcacattca aagttccttg tccaattgag ttcaataact caggttttga tcagctttca 120

gggaaagtga tcgctgttaa tattgaatta aatgaaaaac attctttgtg tgttatgcat 180

gacgaatcaa cagattcggc tgattattac gaatttgaaa aaatgctaat acagccc 237

<210> 588
<211> 345
<212> DNA
<213> Artificial Sequence

<220>
<223> orf

<400> 588
atttgtgaat cacataatct tgttaatgaa tcaggtatga atatggaacc tacaaattta 60

gatatttttt taagtgaagt taaaaaaaca ggtgaaacga aattaactgg tgttttccac 120

caagttccat ttcgaatgtt ctctccacac tatcgacgtg ttgaaggtct tcgcaattat 180

atgaatgagc ctcgtaataa agttcttaat tctctaattg aaattgcttt agatcaggtt 240

tttgaaaatc tgtctaagga tccagaaatt tatgaagctg taatggccga agtttcaaaa 300

attgatattg gtacgtttga tggctctgga gacttagatc gtgat 345

<210> 589
<211> 291
<212> DNA
<213> Artificial Sequence

<220>
<223> orf

<400> 589
atcgtgatta aactagaggg aaccgtgata aatacatttc atcttgaagg tggaaaaaat 60

aaaaaggggg aagaatacga agccagtgac aaggtgcagt tacttggttc gttggagcta 120

cccaatgggc aaattaaaaa cgaattgatt gatcttaaag tcgaagatgc aagcatctac 180

gatgcattca aaaataagct tattagcatt agttgtggcg catttcccgc agggaaaaac 240

gtcgtgtttt atgttcgaaa aggtgcaaag cctgttttag ctgacggtct t 291

<210> 590
<211> 1257
<212> DNA
<213> Artificial Sequence

<220>
<223> orf

<400> 590
atgataaatg acaataaaaa acccctgtta tcggacagtt tggcgaccgg tgataacaag 60

ggtttgcaat ctgctaaggc agatcaacac agggatagaa taacacgttt tggtattttg 120

aaacacaggt cgaagcaaca agaaaacttt ttgtggacgc tagcaaaatt caaagaaaac 180

tacccaatag actcgccaaa tgaagaaact gtaaaggccc taaaatctgc acagaagtta 240

aacggctgcg gtaatttttt gctgttcaaa aatctttaca caatagatca aactaagctc 300

acaaaatttc atgtctgtaa tcaacatttg ctttgcccat tttgtgcagg tattcgtgca 360

tctaaagcaa ttcaaaaata tacagaacgt gttgatgaag tattaaagaa aaatcgtaag 420

ctcaagcccg ttctaatcac gctcactgtg aaaaacggta ctgacctaag tgaacggtct 480

gcacacctca taaaatcgct tagaacgctt attaagcgcc gtcaggacta taataaaaaa 540

ggtcgtggtt ttaacgaatt ttgtaaaatt aatggtgcga tgtactcgta cgaaaatact 600

tacaatgaac aaaccaaaga atggcatccg cacgttcata tgtttgctcc tttggatgat 660

tggatagacc aggaactact ttctgaaaca tggcatgaaa tcactggtga ttcatttgtt 720

gtggacattc gaaaagttaa aaaacaaaaa gacttaggct atgcaaaagc tgctgctgag 780

gtgtgtaaat acgctcttaa gtttggggat ctaacggtcg aaaatacatg ggaagctttt 840

aaagtcctaa aaggtaagcg cttaagcggt gcatttggct ctctttatgg tgtcaaaatt 900

cctgaaaacc ttgctgacga tatgccagaa gaaaaagatc ttccgtattt agagatgctc 960

tataagttcg tctttggaaa acactcttac tacaatctcg aaattactag acacgtagga 1020

ccgcaggcgg gagagcgcgc ggagttagga ggagttgcga cgacgcccga cgagagatat 1080

agcgcgggcg gttctagtgt agacgatttg agtacggagg caggtggagg gctcgcacca 1140

ctgcgacgta cgaaaagagg acgcaaaaaa cagcactggc aattaccacc agtaacgaga 1200

gttagagtca aaaaacgaat caaaagatgg gatggttatt tgtgtgttat acacatg 1257

<210> 591
<211> 255
<212> DNA
<213> Artificial Sequence

<220>
<223> orf

<400> 591
attcgaaatg gaacttggtg gaaaacacca gttaatttcg tttcacctgt ttttttaact     60

tcacttaaaa aaatatctaa atttgtaggt tccatattca tacctgattc attaacaaga    120

ttatgtgatt cacaaatgaa tcatacactg aatcaagaac aaatcaatag gggggtcgcc    180

acccattcgg gatggctctg ttcttaccgg agaacgaaaa aaaggggagc ttgtaatcag    240

agaaaggggg atttg                                                     255


<210> 592
<211> 150
<212> DNA
<213> Artificial Sequence


<220>
<223> orf

<400> 592
atcaagaaca aatcaatagg ggggtcgcca cccattcggg atggctctgt tcttaccgga     60

gaacgaaaaa aaggggagct tgtaatcaga gaaaggggga tttgtaattt ttattccgga    120

gtcgtcactc gtagacactc gttcgggaac                                     150


<210> 593
<211> 231
<212> DNA
<213> Artificial Sequence


<220>
<223> orf

<400> 593
   ttgattcgtt ttttgactct aactctcgtt actggtggta attgccagtg ctgttttttg     60
   cgtcctcttt tcgtacgtcg cagtggtgcg agccctccac ctgcctccgt actcaaatcg    120
   tctacactag aaccgcccgc gctatatctc tcgtcgggcg tcgtcgcaac tcctcctaac    180
   tccgcgcgct ctcccgcctg cggtcctacg tgtctagtaa tttcgagatt g             231


<210> 594
<211> 204
<212> DNA
<213> Artificial Sequence


<220>
<223> orf

<400> 594

ctggtctatc caatcatcca aaggagcaaa catatgaacg tgcggatgcc attctttggt 60

ttgttcattg taagtatttt cgtacgagta catcgcacca ttaattttac aaaattcgtt 120

aaaaccacga ccttttttat tatagtcctg acggcgctta ataagcgttc taagcgattt 180

tatgaggtgt gcagaccgtt cact 204

<210> 595
<211> 156
<212> DNA
<213> Artificial Sequence

<220>
<223> orf

<400> 595
atcaacacgt tctgtatatt tttgaattgc tttagatgca cgaatacctg cacaaaatgg 60

gcaaagcaaa tgttgattac agacatgaaa ttttgtgagc ttagtttgat ctattgtgta 120

aagatttttg aacagcaaaa aattaccgca gccgtt 156

<210> 596
<211> 2952
<212> DNA
<213> Artificial Sequence

<220>
<223> isolate

<400> 596
caacacaggg atagaataac acgttttggg attttgaaac atagagcgaa gcaacaagaa 60

aactttttgt ggactctggc aaaattcaaa gaaaactatc ctaaagatgc tccaagtgaa 120

gaaagcacaa gagcaattaa atctgcacag aaattaaaag agtgcggaaa tttcttacta 180

ttcaaaaatt tttacacaat agaccaaact atgctctcaa aatttcatgt atgtggacag 240

catttgttat gtcctttttg tgctggtatt cgtgcatcta aagcaattca aaaatatact 300

gaacgtgttg atgaagtgct taagaaaaat cgcaagctca agcctgtttt agtcacgttt 360

acggtgaaga atggaaccga cttaggggaa agatcatccc acctcataaa atcgtttaga 420

acgcttctga agcgtcgtag ggactatatt aaaaaaggcc gtgggtttaa tgagttctgt 480

aagatcaatg gggctatgta ttcttatgaa aacacgtata acgagcaaac aaatgaatgg 540

catccacact tacatatgtt tgcacttttg gatgattgga tagaccagga agagctttca 600

cagtattggc attccatcac tggtgattca atgattgtag atattcgccg ggtcaaaaaa 660

gaaaaaggct taggctattc aaaggctgct gcggaagtat gtaaatatgc tcttaaattc 720

ggtgatttgt ctgtccaaaa tacttgggaa gctttcaaag tcttaaaagg taagcgttta 780

agtggtgcat ttggttgttt atggggcgta aaaattcctg aaaatcttgc tgatgatatg 840

cctgatgaaa aagatcttcc ttacttagaa atgctctata aattcgtatt tggaaaaccg 900

tcttactatg acttaactat gacacgacat gtagagccac aaaataagga caacgccgac 960

gacgaggagc aggaacgtcg acgagaagga gaaggcggac gcaaattagt ggcgaatgag 1020

gacgatgtga ggagtgacga ggagcgtgcc gaccgacgga cacgaacgac acgaggacga 1080

aaaaaacaac actggcaagt acctccagta acgagggtac gagtgaaaaa acggattaaa 1140

agatgggatg gttatttgtg tgttatgcac atgtaaaatc ataaacctat gataaaattc 1200

tagtcgctga atagtcggat tgccgtccaa ctacccagct aaccacaacg atctaagaag 1260

gagatcatca tgactgtagc gaatactaat gctttaaatt atttaggcaa gtatgtttca 1320

tttaatcacg aagatatttt tgaaatttac ggagttatct ccaatgtaat tttcaacatg 1380

gacgggacca ttgaactgtc cattggatgg gatgattttt ataatttctc tgaaatgact 1440

aatttaaaag ttttaggaga aattagtctt tattgacttt agatttgaat ttaccaatgc 1500

ccgaaaatac atatttttgg gcattttttt gtctgtttct tgagaaacgc gtaagcgtgc 1560

attctgaaaa aataaacatt gagattggcg aagagattcg gggcgctggg cgtggggtcg 1620

ggatgtcact cgatgtcccg ccctgtgact ggcgacacga gatattcgac aatctcactt 1680

tagaaacaat atcagagggc acggagtgtg cccgaactga agaaataaaa attaaaaact 1740

acctttccct gattacaagc tcccttttga ttttctccgg gataaacaga gccatccgta 1800

tggtggcgat agattgacat tctaattaaa atctataata ttctataaac aatctagtag 1860

aatctaatcg gaatctaaaa tatgttattt aaatctcttg gaattgcttc cgctactaat 1920

aatgcttcta actctgagga tgctaagcct gtgactattc gtttaactga tggtgaggtt 1980

caactttatg atgctgtagc caaggctatg gggttaactc gtcaagattt tcttgcccat 2040

ttaattcgtt ctaattttaa acaagcatta accgattttt taataggtta cacggaatca 2100

tcaccatcta tctctttaaa agaattaatt tttttgggaa ctgacaatga agttgttcga 2160

aataaagttg attcaatgct tcgatctatt tctgcacaac taatggaaga tgatgaaaag 2220

gcaattcaag aacatcttca aaatgaaggt aattggtatg aatatgctac ccaacgtaaa 2280

ggtattttcc ctgaacctgt taaaccagtt gaggtaaaaa atgattaaac ctcaagtcat 2340

tagtaacact ccaatggaat ctcttgagga agcttgcgat tactttaagc aagttcttca 2400

ggaacatctc gataaaaaac gtgacatcac attatttgcc atcgctgttg taggtggtcg 2460

tttaaaagtc gtagagggat taccaaaatg atcaaattag aaggcacaat tattaatgtt 2520

tttacccaac aaggtggtaa agataaaaaa acaggcgaag cattcgcaga tcgggacaag 2580

attcaattat taggtgagtt tgaactcccc aatggtgata aaaaaaatga attaatagac 2640

cttactgttg aaaatggtaa ggaatatgag ggtatgaaaa acaagcgtgt gagcattccc 2700

tgtggtgctc ttgcttcagg tcgaaatgtg attttttatg tcgcaaaagg cgcaacacct 2760

aaacttttaa acacagtttg atactcgtta aaaaacagcg acttacgaag taagttagct 2820

gttttttctt ttacttgata ctattaatta aagtggggac aaaaattgac tgtaaaagac 2880

aataaaaaag cccatatttc tgatgctttg gcgggcgacg aaatacaggc tttgcaatct 2940

gctaaggcag at 2952

<210> 597
<211> 386
<212> PRT
<213> Artificial Sequence

<220>
<223> orf

<400> 597
Met Thr Arg Phe Gly Ile Leu Lys His Arg Ala Lys Gln Gln Glu Asn
1               5                   10                  15
Phe Leu Trp Thr Leu Ala Lys Phe Lys Glu Asn Tyr Pro Lys Asp Ala
            20                  25                  30
Pro Ser Glu Glu Ser Thr Arg Ala Ile Lys Ser Ala Gln Lys Leu Lys
        35                  40                  45
Glu Cys Gly Asn Phe Leu Leu Phe Lys Asn Phe Tyr Thr Ile Asp Gln
    50                  55                  60
Thr Met Leu Ser Lys Phe His Val Cys Gly Gln His Leu Leu Cys Pro
65                  70                  75                  80
Phe Cys Ala Gly Ile Arg Ala Ser Lys Ala Ile Gln Lys Tyr Thr Glu
                85                  90                  95
Arg Val Asp Glu Val Leu Lys Lys Asn Arg Lys Leu Lys Pro Val Leu
            100                 105                 110
Val Thr Phe Thr Val Lys Asn Gly Thr Asp Leu Gly Glu Arg Ser Ser
        115                 120                 125
His Leu Ile Lys Ser Phe Arg Thr Leu Leu Lys Arg Arg Arg Asp Tyr
    130                 135                 140
Ile Lys Lys Gly Arg Gly Phe Asn Glu Phe Cys Lys Ile Asn Gly Ala
145                 150                 155                 160
Met Tyr Ser Tyr Glu Asn Thr Tyr Asn Glu Gln Thr Asn Glu Trp His
                165                 170                 175
Pro His Leu His Met Phe Ala Leu Leu Asp Asp Trp Ile Asp Gln Glu
            180                 185                 190
Glu Leu Ser Gln Tyr Trp His Ser Ile Thr Gly Asp Ser Met Ile Val
        195                 200                 205
Asp Ile Arg Arg Val Lys Lys Glu Lys Gly Leu Gly Tyr Ser Lys Ala
    210                 215                 220
Ala Ala Glu Val Cys Lys Tyr Ala Leu Lys Phe Gly Asp Leu Ser Val
225                 230                 235                 240
Gln Asn Thr Trp Glu Ala Phe Lys Val Leu Lys Gly Lys Arg Leu Ser
                245                 250                 255
Gly Ala Phe Gly Cys Leu Trp Gly Val Lys Ile Pro Glu Asn Leu Ala
            260                 265                 270
Asp Asp Met Pro Asp Glu Lys Asp Leu Pro Tyr Leu Glu Met Leu Tyr
        275                 280                 285
Lys Phe Val Phe Gly Lys Pro Ser Tyr Tyr Asp Leu Thr Met Thr Arg
    290                 295                 300
His Val Glu Pro Gln Asn Lys Asp Asn Ala Asp Asp Glu Glu Gln Glu
305                 310                 315                 320
Arg Arg Arg Glu Gly Glu Gly Gly Arg Lys Leu Val Ala Asn Glu Asp
                325                 330                 335

Asp Val Arg Ser Asp Glu Glu Arg Ala Asp Arg Arg Thr Arg Thr Thr
            340                 345                 350
Arg Gly Arg Lys Lys Gln His Trp Gln Val Pro Pro Val Thr Arg Val
        355                 360                 365
Arg Val Lys Lys Arg Ile Lys Arg Trp Asp Gly Tyr Leu Cys Val Met
    370                 375                 380
His Met
385

<210> 598
<211> 70
<212> PRT
<213> Artificial Sequence

<220>
<223> orf

<400> 598
Met Ile Met Thr Val Ala Asn Thr Asn Ala Leu Asn Tyr Leu Gly Lys
1               5                   10                  15
Tyr Val Ser Phe Asn His Glu Asp Ile Phe Glu Ile Tyr Gly Val Ile
            20                  25                  30
Ser Asn Val Ile Phe Asn Met Asp Gly Thr Ile Glu Leu Ser Ile Gly
        35                  40                  45
Trp Asp Asp Phe Tyr Asn Phe Ser Glu Met Thr Asn Leu Lys Val Leu
    50                  55                  60
Gly Glu Ile Ser Leu Tyr
65                  70

<210> 599
<211> 114
<212> PRT
<213> Artificial Sequence

<220>
<223> orf

<400> 599
Met Thr Leu Asp Leu Asn Leu Pro Met Pro Glu Asn Thr Tyr Phe Trp
1               5                   10                  15
Ala Phe Phe Cys Leu Phe Leu Glu Lys Arg Val Ser Val His Ser Glu
            20                  25                  30
Lys Ile Asn Ile Glu Ile Gly Glu Glu Ile Arg Gly Ala Gly Arg Gly
        35                  40                  45
Val Gly Met Ser Leu Asp Val Pro Pro Cys Asp Trp Arg His Glu Ile
    50                  55                  60
Phe Asp Asn Leu Thr Leu Glu Thr Ile Ser Glu Gly Thr Glu Cys Ala
65                  70                  75                  80
Arg Thr Glu Glu Ile Lys Ile Lys Asn Tyr Leu Ser Leu Ile Thr Ser
                85                  90                  95
Ser Leu Leu Ile Phe Ser Gly Ile Asn Arg Ala Ile Arg Met Val Ala
            100                 105                 110
Ile Asp

<210> 600
<211> 148
<212> PRT
<213> Artificial Sequence

<220>
<223> orf

<400> 600
Met Leu Phe Lys Ser Leu Gly Ile Ala Ser Ala Thr Asn Asn Ala Ser
1               5                   10                  15
Asn Ser Glu Asp Ala Lys Pro Val Thr Ile Arg Leu Thr Asp Gly Glu
            20                  25                  30
Val Gln Leu Tyr Asp Ala Val Ala Lys Ala Met Gly Leu Thr Arg Gln
        35                  40                  45
Asp Phe Leu Ala His Leu Ile Arg Ser Asn Phe Lys Gln Ala Leu Thr
    50                  55                  60
Asp Phe Leu Ile Gly Tyr Thr Glu Ser Ser Pro Ser Ile Ser Leu Lys
65                  70                  75                  80
Glu Leu Ile Phe Leu Gly Thr Asp Asn Glu Val Val Arg Asn Lys Val
                85                  90                  95
Asp Ser Met Leu Arg Ser Ile Ser Ala Gln Leu Met Glu Asp Asp Glu
            100                 105                 110
Lys Ala Ile Gln Glu His Leu Gln Asn Glu Gly Asn Trp Tyr Glu Tyr
        115                 120                 125
Ala Thr Gln Arg Lys Gly Ile Phe Pro Glu Pro Val Lys Pro Val Glu
    130                 135                 140
Val Lys Asn Asp
145

<210> 601
<211> 56
<212> PRT
<213> Artificial Sequence


<220>
<223> orf

<400> 601
Met Ile Lys Pro Gln Val Ile Ser Asn Thr Pro Met Glu Ser Leu Glu
1               5                   10                  15
Glu Ala Cys Asp Tyr Phe Lys Gln Val Leu Gln Glu His Leu Asp Lys
            20                  25                  30
Lys Arg Asp Ile Thr Leu Phe Ala Ile Ala Val Val Gly Gly Arg Leu
        35                  40                  45
Lys Val Val Glu Gly Leu Pro Lys
    50                  55

<210> 602
<211> 116
<212> PRT
<213> Artificial Sequence


<220>
<223> orf

<400> 602
   Met Ile Cys His Arg Cys Cys Arg Trp Ser Phe Lys Ser Arg Arg Gly
   1               5                   10                  15
   Ile Thr Lys Met Ile Lys Leu Glu Gly Thr Ile Ile Asn Val Phe Thr
               20                  25                  30
   Gln Gln Gly Gly Lys Asp Lys Lys Thr Gly Glu Ala Phe Ala Asp Arg
           35                  40                  45
   Asp Lys Ile Gln Leu Leu Gly Glu Phe Glu Leu Pro Asn Gly Asp Lys
       50                  55                  60
   Lys Asn Glu Leu Ile Asp Leu Thr Val Glu Asn Gly Lys Glu Tyr Glu
   65                  70                  75                  80
   Gly Met Lys Asn Lys Arg Val Ser Ile Pro Cys Gly Ala Leu Ala Ser
                   85                  90                  95
   Gly Arg Asn Val Ile Phe Tyr Val Ala Lys Gly Ala Thr Pro Lys Leu
               100                 105                 110
   Leu Asn Thr Val
           115

<210> 603
<211> 420
<212> PRT
<213> Artificial Sequence

<220>
<223> orf

<400> 603
Met Thr Val Lys Asp Asn Lys Lys Ala His Ile Ser Asp Ala Leu Ala
1               5                   10                  15
Gly Asp Glu Ile Gln Ala Leu Gln Ser Ala Lys Ala Asp Gln His Arg
            20                  25                  30
Asp Arg Ile Thr Arg Phe Gly Ile Leu Lys His Arg Ala Lys Gln Gln
        35                  40                  45
Glu Asn Phe Leu Trp Thr Leu Ala Lys Phe Lys Glu Asn Tyr Pro Lys
    50                  55                  60
Asp Ala Pro Ser Glu Glu Ser Thr Arg Ala Ile Lys Ser Ala Gln Lys
65                  70                  75                  80
Leu Lys Glu Cys Gly Asn Phe Leu Leu Phe Lys Asn Phe Tyr Thr Ile
                85                  90                  95
Asp Gln Thr Met Leu Ser Lys Phe His Val Cys Gly Gln His Leu Leu
            100                 105                 110
Cys Pro Phe Cys Ala Gly Ile Arg Ala Ser Lys Ala Ile Gln Lys Tyr
        115                 120                 125
Thr Glu Arg Val Asp Glu Val Leu Lys Lys Asn Arg Lys Leu Lys Pro
    130                 135                 140
Val Leu Val Thr Phe Thr Val Lys Asn Gly Thr Asp Leu Gly Glu Arg
145                 150                 155                 160
Ser Ser His Leu Ile Lys Ser Phe Arg Thr Leu Leu Lys Arg Arg Arg
                165                 170                 175
Asp Tyr Ile Lys Lys Gly Arg Gly Phe Asn Glu Phe Cys Lys Ile Asn
            180                 185                 190
Gly Ala Met Tyr Ser Tyr Glu Asn Thr Tyr Asn Glu Gln Thr Asn Glu
        195                 200                 205
Trp His Pro His Leu His Met Phe Ala Leu Leu Asp Asp Trp Ile Asp
    210                 215                 220
Gln Glu Glu Leu Ser Gln Tyr Trp His Ser Ile Thr Gly Asp Ser Met
225                 230                 235                 240
Ile Val Asp Ile Arg Arg Val Lys Lys Glu Lys Gly Leu Gly Tyr Ser
                245                 250                 255
Lys Ala Ala Ala Glu Val Cys Lys Tyr Ala Leu Lys Phe Gly Asp Leu
            260                 265                 270
Ser Val Gln Asn Thr Trp Glu Ala Phe Lys Val Leu Lys Gly Lys Arg
        275                 280                 285
Leu Ser Gly Ala Phe Gly Cys Leu Trp Gly Val Lys Ile Pro Glu Asn
    290                 295                 300
Leu Ala Asp Asp Met Pro Asp Glu Lys Asp Leu Pro Tyr Leu Glu Met
305                 310                 315                 320
Leu Tyr Lys Phe Val Phe Gly Lys Pro Ser Tyr Tyr Asp Leu Thr Met
                325                 330                 335
Thr Arg His Val Glu Pro Gln Asn Lys Asp Asn Ala Asp Asp Glu Glu
            340                 345                 350
Gln Glu Arg Arg Arg Glu Gly Glu Gly Gly Arg Lys Leu Val Ala Asn

355                 360                 365
Glu Asp Asp Val Arg Ser Asp Glu Glu Arg Ala Asp Arg Arg Thr Arg
    370                 375                 380
Thr Thr Arg Gly Arg Lys Lys Gln His Trp Gln Val Pro Pro Val Thr
385                 390                 395                 400
Arg Val Arg Val Lys Lys Arg Ile Lys Arg Trp Asp Gly Tyr Leu Cys
                405                 410                 415
Val Met His Met
            420

<210> 604
<211> 51
<212> PRT
<213> Artificial Sequence

<220>
<223> orf

<400> 604
Met Leu Ser Arg Ser Ala Asn Ala Ser Pro Val Phe Leu Ser Leu Pro
1               5                   10                  15
Pro Cys Trp Val Lys Thr Leu Ile Ile Val Pro Ser Asn Leu Ile Ile
            20                  25                  30
Leu Val Ile Pro Leu Arg Leu Leu Asn Asp His Leu Gln Gln Arg Trp
        35                  40                  45
Gln Ile Met
    50

<210> 605
<211> 89
<212> PRT
<213> Artificial Sequence

<220>
<223> orf

<400> 605
Met Phe Ile Ser Ser Val Arg Ala His Ser Val Pro Ser Asp Ile Val
1               5                   10                  15
Ser Lys Val Arg Leu Ser Asn Ile Ser Cys Arg Gln Ser Gln Gly Gly
            20                  25                  30
Thr Ser Ser Asp Ile Pro Thr Pro Arg Pro Ala Pro Arg Ile Ser Ser
        35                  40                  45
Pro Ile Ser Met Phe Ile Phe Ser Glu Cys Thr Leu Thr Arg Phe Ser
    50                  55                  60
Arg Asn Arg Gln Lys Asn Ala Gln Lys Tyr Val Phe Ser Gly Ile Gly
65                  70                  75                  80
Lys Phe Lys Ser Lys Val Asn Lys Asp
                85

<210> 606
<211> 70
<212> PRT
<213> Artificial Sequence

<220>
<223> orf

<400> 606
Met Ser Arg Val Ala Ser His Arg Ala Gly His Arg Val Thr Ser Arg

1               5                   10                  15
Pro His Ala Gln Arg Pro Glu Ser Leu Arg Gln Ser Gln Cys Leu Phe
                20                  25                  30
Phe Gln Asn Ala Arg Leu Arg Val Ser Gln Glu Thr Asp Lys Lys Met
        35                  40                  45
Pro Lys Asn Met Tyr Phe Arg Ala Leu Val Asn Ser Asn Leu Lys Ser
    50                  55                  60
Ile Lys Thr Asn Phe Ser
65                  70

<210> 607
<211> 74
<212> PRT
<213> Artificial Sequence

<220>
<223> orf

<400> 607
Met Asp Gly Asn Pro Thr Ile Gln Arg Leu Glu Phe Tyr His Arg Phe
1               5                   10                  15
Met Ile Leu His Val His Asn Thr Gln Ile Thr Ile Pro Ser Phe Asn
                20                  25                  30
Pro Phe Phe His Ser Tyr Pro Arg Tyr Trp Arg Tyr Leu Pro Val Leu
        35                  40                  45
Phe Phe Ser Ser Ser Cys Arg Ser Cys Pro Ser Val Gly Thr Leu Leu
    50                  55                  60
Val Thr Pro His Ile Val Leu Ile Arg His
65                  70

<210> 608
<211> 63
<212> PRT
<213> Artificial Sequence

<220>
<223> orf

<400> 608
Met Glu Val Leu Ala Ser Val Val Phe Phe Val Leu Val Ser Phe Val
1               5                   10                  15
Ser Val Gly Arg His Ala Pro Arg His Ser Ser His Arg Pro His Ser
                20                  25                  30
Pro Leu Ile Cys Val Arg Leu Leu Leu Leu Val Asp Val Pro Ala Pro
        35                  40                  45
Arg Arg Arg Arg Cys Pro Tyr Phe Val Ala Leu His Val Val Ser
    50                  55                  60

<210> 609
<211> 76
<212> PRT
<213> Artificial Sequence

<220>
<223> orf

<400> 609
Met Arg Phe Phe Thr Arg Thr Leu Val Thr Gly Gly Thr Cys Gln Cys
1               5                   10                  15
Cys Phe Phe Arg Pro Arg Val Val Arg Val Arg Arg Ser Ala Arg Ser

20                  25                  30
Ser Ser Leu Leu Thr Ser Ser Ser Phe Ala Thr Asn Leu Arg Pro Pro
        35                  40                  45
Ser Pro Ser Arg Arg Arg Ser Cys Ser Ser Ser Ser Ala Leu Ser Leu
    50                  55                  60
Phe Cys Gly Ser Thr Cys Arg Val Ile Val Lys Ser
65                  70                  75

<210> 610
<211> 54
<212> PRT
<213> Artificial Sequence

<220>
<223> orf

<400> 610
Met Ser Ser Ala Arg Phe Ser Gly Ile Phe Thr Pro His Lys Gln Pro
1               5                   10                  15
Asn Ala Pro Leu Lys Arg Leu Pro Phe Lys Thr Leu Lys Ala Ser Gln
            20                  25                  30
Val Phe Trp Thr Asp Lys Ser Pro Asn Leu Arg Ala Tyr Leu His Thr
        35                  40                  45
Ser Ala Ala Ala Phe Glu
    50

<210> 611
<211> 50
<212> PRT
<213> Artificial Sequence

<220>
<223> orf

<400> 611
Met Thr Arg Arg Ile Ser Thr Ile Ile Glu Ser Pro Val Met Glu Cys
1               5                   10                  15
Gln Tyr Cys Glu Ser Ser Ser Trp Ser Ile Gln Ser Ser Lys Ser Ala
            20                  25                  30
Asn Ile Cys Lys Cys Gly Cys His Ser Phe Val Cys Ser Leu Tyr Val
        35                  40                  45
Phe Ser
    50

<210> 612
<211> 51
<212> PRT
<213> Artificial Sequence

<220>
<223> orf

<400> 612
Met Trp Met Pro Phe Ile Cys Leu Leu Val Ile Arg Val Phe Ile Arg
1               5                   10                  15
Ile His Ser Pro Ile Asp Leu Thr Glu Leu Ile Lys Pro Thr Ala Phe
            20                  25                  30
Phe Asn Ile Val Pro Thr Thr Leu Gln Lys Arg Ser Lys Arg Phe Tyr
        35                  40                  45
Glu Val Gly

    50

<210> 613
<211> 84
<212> PRT
<213> Artificial Sequence

<220>
<223> orf

<400> 613
Met Cys Arg Phe Asn Cys Ser Cys Ala Phe Phe Thr Trp Ser Ile Phe
1               5                   10                  15
Arg Ile Val Phe Phe Glu Phe Cys Gln Ser Pro Gln Lys Val Phe Leu
            20                  25                  30
Leu Leu Arg Ser Met Phe Gln Asn Pro Lys Thr Cys Tyr Ser Ile Pro
        35                  40                  45
Val Leu Ile Cys Leu Ser Arg Leu Gln Ser Leu Tyr Phe Val Ala Arg
    50                  55                  60
Gln Ser Ile Arg Asn Met Gly Phe Phe Ile Val Phe Tyr Ser Gln Phe
65                  70                  75                  80
Leu Ser Pro Leu

<210> 614
<211> 1158
<212> DNA
<213> Artificial Sequence

<220>
<223> orf

<400> 614
ataacacgtt ttgggatttt gaaacataga gcgaagcaac aagaaaactt tttgtggact      60
ctggcaaaat tcaaagaaaa ctatcctaaa gatgctccaa gtgaagaaag cacaagagca     120
attaaatctg cacagaaatt aaaagagtgc ggaaatttct tactattcaa aaatttttac     180
acaatagacc aaactatgct ctcaaaattt catgtatgtg gacagcattt gttatgtcct     240
ttttgtgctg gtattcgtgc atctaaagca attcaaaaat atactgaacg tgttgatgaa     300
gtgcttaaga aaaatcgcaa gctcaagcct gttttagtca cgtttacggt gaagaatgga     360
accgacttag gggaaagatc atcccacctc ataaaatcgt ttagaacgct tctgaagcgt     420
cgtagggact atattaaaaa aggccgtggg tttaatgagt tctgtaagat caatggggct     480
atgtattctt atgaaaacac gtataacgag caaacaaatg aatggcatcc acacttacat     540
atgtttgcac ttttggatga ttggatagac caggaagagc tttcacagta ttggcattcc     600
atcactggtg attcaatgat tgtagatatt cgccgggtca aaaaagaaaa aggcttaggc     660
tattcaaagg ctgctgcgga agtatgtaaa tatgctctta aattcggtga tttgtctgtc     720
caaaatactt gggaagcttt caaagtctta aaaggtaagc gtttaagtgg tgcatttggt     780
tgtttatggg gcgtaaaaat tcctgaaaat cttgctgatg atatgcctga tgaaaaagat     840

cttccttact tagaaatgct ctataaattc gtatttggaa aaccgtctta ctatgactta 900

actatgacac gacatgtaga gccacaaaat aaggacaacg ccgacgacga ggagcaggaa 960

cgtcgacgag aaggagaagg cggacgcaaa ttagtggcga atgaggacga tgtgaggagt 1020

gacgaggagc gtgccgaccg acggacacga acgacacgag gacgaaaaaa acaacactgg 1080

caagtacctc cagtaacgag ggtacgagtg aaaaaacgga ttaaaagatg ggatggttat 1140

ttgtgtgtta tgcacatg 1158

<210> 615
<211> 210
<212> DNA
<213> Artificial Sequence

<220>
<223> orf

<400> 615
atcatcatga ctgtagcgaa tactaatgct ttaaattatt taggcaagta tgtttcattt 60

aatcacgaag atatttttga aatttacgga gttatctcca atgtaatttt caacatggac 120

gggaccattg aactgtccat tggatgggat gatttttata atttctctga aatgactaat 180

ttaaaagttt taggagaaat tagtctttat 210

<210> 616
<211> 342
<212> DNA
<213> Artificial Sequence

<220>
<223> orf

<400> 616
ttgactttag atttgaattt accaatgccc gaaaatacat atttttgggc atttttttgt 60

ctgtttcttg agaaacgcgt aagcgtgcat tctgaaaaaa taaacattga gattggcgaa 120

gagattcggg gcgctgggcg tggggtcggg atgtcactcg atgtcccgcc ctgtgactgg 180

cgacacgaga tattcgacaa tctcacttta gaaacaatat cagagggcac ggagtgtgcc 240

cgaactgaag aaataaaaat taaaaactac ctttccctga ttacaagctc ccttttgatt 300

ttctccggga taaacagagc catccgtatg gtggcgatag at 342

<210> 617
<211> 444
<212> DNA
<213> Artificial Sequence

<220>
<223> orf

<400> 617
atgttattta aatctcttgg aattgcttcc gctactaata atgcttctaa ctctgaggat      60

gctaagcctg tgactattcg tttaactgat ggtgaggttc aactttatga tgctgtagcc     120

aaggctatgg ggttaactcg tcaagatttt cttgcccatt taattcgttc taattttaaa     180

caagcattaa ccgatttttt aataggttac acggaatcat caccatctat ctctttaaaa     240

gaattaattt ttttgggaac tgacaatgaa gttgttcgaa ataaagttga ttcaatgctt     300

cgatctattt ctgcacaact aatggaagat gatgaaaagg caattcaaga acatcttcaa     360

aatgaaggta attggtatga atatgctacc caacgtaaag gtattttccc tgaacctgtt     420

aaaccagttg aggtaaaaaa tgat                                            444


<210> 618
<211> 168
<212> DNA
<213> Artificial Sequence


<220>
<223> orf

<400> 618
atgattaaac ctcaagtcat tagtaacact ccaatggaat ctcttgagga agcttgcgat      60

tactttaagc aagttcttca ggaacatctc gataaaaaac gtgacatcac attatttgcc     120

atcgctgttg taggtggtcg tttaaaagtc gtagagggat taccaaaa                  168


<210> 619
<211> 348
<212> DNA
<213> Artificial Sequence


<220>
<223> orf

<400> 619
attatttgcc atcgctgttg taggtggtcg tttaaaagtc gtagagggat taccaaaatg      60

atcaaattag aaggcacaat tattaatgtt tttacccaac aaggtggtaa agataaaaaa     120

acaggcgaag cattcgcaga tcgggacaag attcaattat taggtgagtt tgaactcccc     180

aatggtgata aaaaaaatga attaatagac cttactgttg aaaatggtaa ggaatatgag     240

ggtatgaaaa acaagcgtgt gagcattccc tgtggtgctc ttgcttcagg tcgaaatgtg     300

attttttatg tcgcaaaagg cgcaacacct aaacttttaa acacagtt                  348


<210> 620
<211> 1260
<212> DNA
<213> Artificial Sequence

<220>
<223> orf

<400> 620
ttgactgtaa aagacaataa aaaagcccat atttctgatg ctttggcggg cgacgaaata      60

caggctttgc aatctgctaa ggcagatcaa cacagggata gaataacacg ttttgggatt     120

ttgaaacata gagcgaagca acaagaaaac tttttgtgga ctctggcaaa attcaaagaa     180

aactatccta aagatgctcc aagtgaagaa agcacaagag caattaaatc tgcacagaaa     240

ttaaaagagt gcggaaattt cttactattc aaaaattttt acacaataga ccaaactatg     300

ctctcaaaat ttcatgtatg tggacagcat ttgttatgtc ctttttgtgc tggtattcgt     360

gcatctaaag caattcaaaa atatactgaa cgtgttgatg aagtgcttaa gaaaaatcgc     420

aagctcaagc ctgttttagt cacgtttacg gtgaagaatg gaaccgactt aggggaaaga     480

tcatcccacc tcataaaatc gtttagaacg cttctgaagc gtcgtaggga ctatattaaa     540

aaaggccgtg ggtttaatga gttctgtaag atcaatgggg ctatgtattc ttatgaaaac     600

acgtataacg agcaaacaaa tgaatggcat ccacacttac atatgtttgc acttttggat     660

gattggatag accaggaaga gctttcacag tattggcatt ccatcactgg tgattcaatg     720

attgtagata ttcgccgggt caaaaaagaa aaaggcttag gctattcaaa ggctgctgcg     780

gaagtatgta aatatgctct taaattcggt gatttgtctg tccaaaatac ttgggaagct     840

ttcaaagtct taaaaggtaa gcgtttaagt ggtgcatttg gttgtttatg gggcgtaaaa      900

attcctgaaa atcttgctga tgatatgcct gatgaaaaag atcttcctta cttagaaatg     960

ctctataaat tcgtatttgg aaaaccgtct tactatgact taactatgac acgacatgta    1020

gagccacaaa ataaggacaa cgccgacgac gaggagcagg aacgtcgacg agaaggagaa    1080

ggcggacgca aattagtggc gaatgaggac gatgtgagga gtgacgagga gcgtgccgac    1140

cgacggacac gaacgacacg aggacgaaaa aaacaacact ggcaagtacc tccagtaacg    1200

agggtacgag tgaaaaaacg gattaaaaga tgggatggtt atttgtgtgt tatgcacatg    1260

<210> 621
<211> 153
<212> DNA
<213> Artificial Sequence

<220>
<223> orf

<400> 621
atcttgtccc gatctgcgaa tgcttcgcct gtttttttat ctttaccacc ttgttgggta      60

aaaacattaa taattgtgcc ttctaatttg atcattttgg taatccctct acgactttta     120

aacgaccacc tacaacagcg atggcaaata atg 153

<210> 622
<211> 267
<212> DNA
<213> Artificial Sequence

<220>
<223> orf

<400> 622
atttttattt cttcagttcg ggcacactcc gtgccctctg atattgtttc taaagtgaga 60

ttgtcgaata tctcgtgtcg ccagtcacag ggcgggacat cgagtgacat cccgacccca 120

cgcccagcgc cccgaatctc ttcgccaatc tcaatgttta ttttttcaga atgcacgctt 180

acgcgtttct caagaaacag acaaaaaaat gcccaaaaat atgtattttc gggcattggt 240

aaattcaaat ctaaagtcaa taaagac 267

<210> 623
<211> 210
<212> DNA
<213> Artificial Sequence

<220>
<223> orf

<400> 623
atatctcgtg tcgccagtca cagggcggga catcgagtga catcccgacc ccacgcccag 60

cgccccgaat ctcttcgcca atctcaatgt ttattttttc agaatgcacg cttacgcgtt 120

tctcaagaaa cagacaaaaa aatgcccaaa aatatgtatt ttcgggcatt ggtaaattca 180

aatctaaagt caataaagac taatttctcc 210

<210> 624
<211> 222
<212> DNA
<213> Artificial Sequence

<220>
<223> orf

<400> 624
ttggacggca atccgactat tcagcgacta gaattttatc ataggtttat gattttacat 60

gtgcataaca cacaaataac catcccatct tttaatccgt tttttcactc gtaccctcgt 120

tactggaggt acttgccagt gttgtttttt tcgtcctcgt gtcgttcgtg tccgtcggtc 180

ggcacgctcc tcgtcactcc tcacatcgtc ctcattcgcc ac 222

<210> 625

<211> 189
<212> DNA
<213> Artificial Sequence

<220>
<223> orf

<400> 625
ctggaggtac ttgccagtgt tgtttttttc gtcctcgtgt cgttcgtgtc cgtcggtcgg 60

cacgctcctc gtcactcctc acatcgtcct cattcgccac taatttgcgt ccgccttctc 120

cttctcgtcg acgttcctgc tcctcgtcgt cggcgttgtc cttattttgt ggctctacat 180

gtcgtgtca 189

<210> 626
<211> 228
<212> DNA
<213> Artificial Sequence

<220>
<223> orf

<400> 626
   atccgttttt tcactcgtac cctcgttact ggaggtactt gccagtgttg tttttttcgt 60
   cctcgtgtcg ttcgtgtccg tcggtcggca cgctcctcgt cactcctcac atcgtcctca 120
   ttcgccacta atttgcgtcc gccttctcct tctcgtcgac gttcctgctc ctcgtcgtcg 180
   gcgttgtcct tattttgtgg ctctacatgt cgtgtcatag ttaagtca 228

<210> 627
<211> 162
<212> DNA
<213> Artificial Sequence

<220>
<223> orf

<400> 627
atatcatcag caagattttc aggaattttt acgccccata aacaaccaaa tgcaccactt 60

aaacgcttac cttttaagac tttgaaagct tcccaagtat tttggacaga caaatcaccg 120

aatttaagag catatttaca tacttccgca gcagcctttg aa 162

<210> 628
<211> 150
<212> DNA
<213> Artificial Sequence

<220>
<223> orf

<400> 628
ttgacccggc gaatatctac aatcattgaa tcaccagtga tggaatgcca atactgtgaa      60

agctcttcct ggtctatcca atcatccaaa agtgcaaaca tatgtaagtg tggatgccat     120

tcatttgttt gctcgttata cgtgttttca                                      150


<210> 629
<211> 153
<212> DNA
<213> Artificial Sequence


<220>
<223> orf

<400> 629
gtgtggatgc cattcatttg tttgctcgtt atacgtgttt tcataagaat acatagcccc      60

attgatctta cagaactcat taaacccacg gcctttttta atatagtccc tacgacgctt     120

cagaagcgtt ctaaacgatt ttatgaggtg gga                                  153


<210> 630
<211> 252
<212> DNA
<213> Artificial Sequence


<220>
<223> orf

<400> 630
ctgtgcagat ttaattgctc ttgtgctttc ttcacttgga gcatctttag gatagttttc      60

tttgaatttt gccagagtcc acaaaaagtt ttcttgttgc ttcgctctat gtttcaaaat     120

cccaaaacgt gttattctat ccctgtgttg atctgcctta gcagattgca aagcctgtat     180

ttcgtcgccc gccaaagcat cagaaatatg ggctttttta ttgtctttta cagtcaattt     240

ttgtccccac tt                                                         252


<210> 631
<211> 2234
<212> DNA
<213> Artificial Sequence


<220>
<223> isolate

<400> 631
caacacaggg atagaataac acgttttggg attttgaaac atagagcgaa gctacaagaa      60

caatacttgt ggacgcaggt tgatttcaaa tcagaaggtg aaaatgatct atccaataag     120

gctcttaagg ctgcaaccaa attaaagggg tgcggtcaat ttctgctatt ccataattac     180

tacacaatag atcaagttaa gctggctaaa gctcattatt gttctcagca tttactatgc     240

cctatgtgtg ctggtgtaag ggcagctaag tcaatgagtc gatatatcca acgtattgaa 300

gaattgatgc gtcagaatcg ccatttaaag cccgtattga tcactttgac ggttaagaac 360

ggtgaagacc tacaggaacg ctttaagcat cttagaagct catttagaac gcttttagac 420

cgctataacg attataagaa aaaaggccgt ggctttaatc agttttgtaa aattgatagt 480

gctttctatt ccactgaata tacctacaac ccaaaaacta aagaatggca tcctcatatt 540

catatttttg ctttactcaa tgaatggata gaccaggaag aattagccga aacttggcac 600

gacattaccc tggattctta tatcgtcgat attcgaagag ttaagaaaac taaagaacac 660

ggctatagca aagctgttgc agaggtatgt aagtatgccc ttaagtttag tgatctctca 720

ctggagaata cttgggaagc atatctttct ttgaaaggta ataggctgac tggctgtttt 780

ggttctatgt atggtgtcaa gctacctgaa aaacttactg atgatctacc tcttgatgat 840

cttccatatt tggagctgct ctatcgtttc gtatttgata aaaaatccta ctacaaccta 900

gaaataacaa aagacgtaaa gccaaacaaa aggaatgagg aaggatgaac gaggagcttc 960

gacgaggccc gacgcccgaa tgacttgtga aggcgacacg tcaaatatta gcaaggatac 1020

acaaaggtac ctttatgcct tgtaaggggt tcgacccctt aaaccccaaa gggcgcactt 1080

taccattgag caaagctcaa cggaaattgc tcccaaaaat aagaggagct aaaaatgaat 1140

tatttcactt tgtatattgt taattgcaat aggtcacatc cgaataggga taatgctttt 1200

cattgttttc atgtggctca atattataga tatcttgagt atctccatta ttaacagagg 1260

gggcggagtg cccccgaact gactcactcg aatttcaaaa cttcctttcc ctgattacaa 1320

agctcccctt agatttctaa gcgggagctt ttagccatcc cgaatgggtg gcgtcacaaa 1380

actatttgat tttttcatga ttcaaggtac gattcattgt gaatcacatt cctatatatg 1440

aaaaaatcag gtgagaatat gcaaattgat gaaccaacaa acttccaagt attcatggca 1500

gaagtaaaca agacagcaaa gacagaaagt attggtgttt atcatcaagt acctttccgt 1560

atggctcgat ggaactttgc acgactggaa ggcctccgta atcacatggg agaacctcga 1620

aacaaagttc ttaactcact aatcgaaatt gctttagatc aagtatttga acaacttgag 1680

catggcagta aagagatccg tcgttctgtt ctggaggaag tttccaaagt tctcgaatca 1740

attgaacatg atggttctgg gagcttagac aatgattaat ctacaaggaa ctttgataaa 1800

tgctttccgt gtggatggtg gtaaaggtaa agacggcaaa gaatatgaag cgcgtggcaa 1860

ggtacaaatt cttggttcgt tggaactccc aaacggggaa attaaacacg aacttgttga 1920

cctaacagtt gaggattctc gactgtttga accattcaaa aatcaggtta ttagcatctc 1980

atgtggtgct atggctatcg gtcgtaatgt tgttttttat gttcgcaaag gcgcgaaacc 2040

agttttagca gaacaaatgt gaaacacgaa aaaaaacagt gagttagcct tggcttctcg 2100

ctgttttttc tttctcttga tactattgat taactagggg ataaaaattg actaaaaaag 2160

acaataaaaa agcccataat tcggatgctt tggcgggcga tgaattacag gctttgcaat 2220

ctgctaaggc agat 2234

<210> 632
<211> 51
<212> PRT
<213> Artificial Sequence

<220>
<223> orf

<400> 632
Met Thr Leu Pro Trp Ile Leu Ile Ser Ser Ile Phe Glu Glu Leu Arg
1               5                   10                  15
Lys Leu Lys Asn Thr Ala Ile Ala Lys Leu Leu Gln Arg Tyr Val Ser
            20                  25                  30
Met Pro Leu Ser Leu Val Ile Ser His Trp Arg Ile Leu Gly Lys His
        35                  40                  45
Ile Phe Leu
    50

<210> 633
<211> 310
<212> PRT
<213> Artificial Sequence

<220>
<223> orf

<400> 633
Met Thr Arg Phe Gly Ile Leu Lys His Arg Ala Lys Leu Gln Glu Gln
1               5                   10                  15
Tyr Leu Trp Thr Gln Val Asp Phe Lys Ser Glu Gly Glu Asn Asp Leu
            20                  25                  30
Ser Asn Lys Ala Leu Lys Ala Ala Thr Lys Leu Lys Gly Cys Gly Gln
        35                  40                  45
Phe Leu Leu Phe His Asn Tyr Tyr Thr Ile Asp Gln Val Lys Leu Ala
    50                  55                  60
Lys Ala His Tyr Cys Ser Gln His Leu Leu Cys Pro Met Cys Ala Gly
65                  70                  75                  80
Val Arg Ala Ala Lys Ser Met Ser Arg Tyr Ile Gln Arg Ile Glu Glu
                85                  90                  95
Leu Met Arg Gln Asn Arg His Leu Lys Pro Val Leu Ile Thr Leu Thr
            100                 105                 110
Val Lys Asn Gly Glu Asp Leu Gln Glu Arg Phe Lys His Leu Arg Ser
        115                 120                 125
Ser Phe Arg Thr Leu Leu Asp Arg Tyr Asn Asp Tyr Lys Lys Lys Gly
    130                 135                 140
Arg Gly Phe Asn Gln Phe Cys Lys Ile Asp Ser Ala Phe Tyr Ser Thr
145                 150                 155                 160
Glu Tyr Thr Tyr Asn Pro Lys Thr Lys Glu Trp His Pro His Ile His
                165                 170                 175
Ile Phe Ala Leu Leu Asn Glu Trp Ile Asp Gln Glu Glu Leu Ala Glu
            180                 185                 190
Thr Trp His Asp Ile Thr Leu Asp Ser Tyr Ile Val Asp Ile Arg Arg
        195                 200                 205
Val Lys Lys Thr Lys Glu His Gly Tyr Ser Lys Ala Val Ala Glu Val

210                 215                 220
Cys Lys Tyr Ala Leu Lys Phe Ser Asp Leu Ser Leu Glu Asn Thr Trp
225                 230                 235                 240
Glu Ala Tyr Leu Ser Leu Lys Gly Asn Arg Leu Thr Gly Cys Phe Gly
                245                 250                 255
Ser Met Tyr Gly Val Lys Leu Pro Glu Lys Leu Thr Asp Asp Leu Pro
            260                 265                 270
Leu Asp Asp Leu Pro Tyr Leu Glu Leu Leu Tyr Arg Phe Val Phe Asp
        275                 280                 285
Lys Lys Ser Tyr Tyr Asn Leu Glu Ile Thr Lys Asp Val Lys Pro Asn
    290                 295                 300
Lys Arg Asn Glu Glu Gly
305                 310

<210> 634
<211> 58
<212> PRT
<213> Artificial Sequence

<220>
<223> orf

<400> 634
Met Lys Ala Thr Arg Gln Ile Leu Ala Arg Ile His Lys Gly Thr Phe
1               5                   10                  15
Met Pro Cys Lys Gly Phe Asp Pro Leu Asn Pro Lys Gly Arg Thr Leu
            20                  25                  30
Pro Leu Ser Lys Ala Gln Arg Lys Leu Leu Pro Lys Ile Arg Gly Ala
        35                  40                  45
Lys Asn Glu Leu Phe His Phe Val Tyr Cys
    50                  55

<210> 635
<211> 118
<212> PRT
<213> Artificial Sequence

<220>
<223> orf

<400> 635
Met Thr Phe Leu Tyr Met Lys Lys Ser Gly Glu Asn Met Gln Ile Asp
1               5                   10                  15
Glu Pro Thr Asn Phe Gln Val Phe Met Ala Glu Val Asn Lys Thr Ala
            20                  25                  30
Lys Thr Glu Ser Ile Gly Val Tyr His Gln Val Pro Phe Arg Met Ala
        35                  40                  45
Arg Trp Asn Phe Ala Arg Leu Glu Gly Leu Arg Asn His Met Gly Glu
    50                  55                  60
Pro Arg Asn Lys Val Leu Asn Ser Leu Ile Glu Ile Ala Leu Asp Gln
65                  70                  75                  80
Val Phe Glu Gln Leu Glu His Gly Ser Lys Glu Ile Arg Arg Ser Val
                85                  90                  95
Leu Glu Glu Val Ser Lys Val Leu Glu Ser Ile Glu His Asp Gly Ser
            100                 105                 110
Gly Ser Leu Asp Asn Asp
        115

<210> 636
<211> 96
<212> PRT

<213> Artificial Sequence

<220>
<223> orf

<400> 636
Met Ile Asn Leu Gln Gly Thr Leu Ile Asn Ala Phe Arg Val Asp Gly
1               5                   10                  15
Gly Lys Gly Lys Asp Gly Lys Glu Tyr Glu Ala Arg Gly Lys Val Gln
            20                  25                  30
Ile Leu Gly Ser Leu Glu Leu Pro Asn Gly Glu Ile Lys His Glu Leu
        35                  40                  45
Val Asp Leu Thr Val Glu Asp Ser Arg Leu Phe Glu Pro Phe Lys Asn
    50                  55                  60
Gln Val Ile Ser Ile Ser Cys Gly Ala Met Ala Ile Gly Arg Asn Val
65                  70                  75                  80
Val Phe Tyr Val Arg Lys Gly Ala Lys Pro Val Leu Ala Glu Gln Met
                85                  90                  95

<210> 637
<211> 344
<212> PRT
<213> Artificial Sequence

<220>
<223> orf

<400> 637
Met Thr Lys Lys Asp Asn Lys Lys Ala His Asn Ser Asp Ala Leu Ala
1               5                   10                  15
Gly Asp Glu Leu Gln Ala Leu Gln Ser Ala Lys Ala Asp Gln His Arg
            20                  25                  30
Asp Arg Ile Thr Arg Phe Gly Ile Leu Lys His Arg Ala Lys Leu Gln
        35                  40                  45
Glu Gln Tyr Leu Trp Thr Gln Val Asp Phe Lys Ser Glu Gly Glu Asn
    50                  55                  60
Asp Leu Ser Asn Lys Ala Leu Lys Ala Ala Thr Lys Leu Lys Gly Cys
65                  70                  75                  80
Gly Gln Phe Leu Leu Phe His Asn Tyr Tyr Thr Ile Asp Gln Val Lys
                85                  90                  95
Leu Ala Lys Ala His Tyr Cys Ser Gln His Leu Leu Cys Pro Met Cys
            100                 105                 110
Ala Gly Val Arg Ala Ala Lys Ser Met Ser Arg Tyr Ile Gln Arg Ile
        115                 120                 125
Glu Glu Leu Met Arg Gln Asn Arg His Leu Lys Pro Val Leu Ile Thr
    130                 135                 140
Leu Thr Val Lys Asn Gly Glu Asp Leu Gln Glu Arg Phe Lys His Leu
145                 150                 155                 160
Arg Ser Ser Phe Arg Thr Leu Leu Asp Arg Tyr Asn Asp Tyr Lys Lys
                165                 170                 175
Lys Gly Arg Gly Phe Asn Gln Phe Cys Lys Ile Asp Ser Ala Phe Tyr
            180                 185                 190
Ser Thr Glu Tyr Thr Tyr Asn Pro Lys Thr Lys Glu Trp His Pro His
        195                 200                 205
Ile His Ile Phe Ala Leu Leu Asn Glu Trp Ile Asp Gln Glu Glu Leu
    210                 215                 220
Ala Glu Thr Trp His Asp Ile Thr Leu Asp Ser Tyr Ile Val Asp Ile
225                 230                 235                 240
Arg Arg Val Lys Lys Thr Lys Glu His Gly Tyr Ser Lys Ala Val Ala

                    245                             250                             255
Glu Val Cys Lys Tyr Ala Leu Lys Phe Ser Asp Leu Ser Leu Glu Asn
                260                             265                             270
Thr Trp Glu Ala Tyr Leu Ser Leu Lys Gly Asn Arg Leu Thr Gly Cys
            275                             280                             285
Phe Gly Ser Met Tyr Gly Val Lys Leu Pro Glu Lys Leu Thr Asp Asp
    290                             295                             300
Leu Pro Leu Asp Asp Leu Pro Tyr Leu Glu Leu Leu Tyr Arg Phe Val
305                             310                             315                             320
Phe Asp Lys Lys Ser Tyr Tyr Asn Leu Glu Ile Thr Lys Asp Val Lys
                325                             330                             335
Pro Asn Lys Arg Asn Glu Glu Gly
                340

<210> 638
<211> 64
<212> PRT
<213> Artificial Sequence

<220>
<223> orf

<400> 638
Met Asn Gly Ser Asn Ser Arg Glu Ser Ser Thr Val Arg Ser Thr Ser
1                   5                   10                  15
Ser Cys Leu Ile Ser Pro Phe Gly Ser Ser Asn Glu Pro Arg Ile Cys
                20                  25                  30
Thr Leu Pro Arg Ala Ser Tyr Ser Leu Pro Ser Leu Pro Leu Pro Pro
            35                  40                  45
Ser Thr Arg Lys Ala Phe Ile Lys Val Pro Cys Arg Leu Ile Ile Val
    50                  55                  60

<210> 639
<211> 52
<212> PRT
<213> Artificial Sequence

<220>
<223> orf

<400> 639
Met Ile Thr Glu Ala Phe Gln Ser Cys Lys Val Pro Ser Ser His Thr
1                   5                   10                  15
Glu Arg Tyr Leu Met Ile Asn Thr Asn Thr Phe Cys Leu Cys Cys Leu
                20                  25                  30
Val Tyr Phe Cys His Glu Tyr Leu Glu Val Cys Trp Phe Ile Asn Leu
            35                  40                  45
His Ile Leu Thr
    50

<210> 640
<211> 153
<212> DNA
<213> Artificial Sequence

<220>
<223> orf

<400> 640
acgacattac cctggattct tatatcgtcg atattcgaag agttaagaaa actaaagaac 60

acggctatag caaagctgtt gcagaggtat gtaagtatgc ccttaagttt agtgatctct 120

cactggagaa tacttgggaa gcatatcttt ctt 153

<210> 641
<211> 930
<212> DNA
<213> Artificial Sequence

<220>
<223> orf

<400> 641
ataacacgtt ttgggatttt gaaacataga gcgaagctac aagaacaata cttgtggacg 60

caggttgatt tcaaatcaga aggtgaaaat gatctatcca ataaggctct taaggctgca 120

accaaattaa aggggtgcgg tcaatttctg ctattccata attactacac aatagatcaa 180

gttaagctgg ctaaagctca ttattgttct cagcatttac tatgccctat gtgtgctggt 240

gtaagggcag ctaagtcaat gagtcgatat atccaacgta ttgaagaatt gatgcgtcag 300

aatcgccatt taaagcccgt attgatcact ttgacggtta agaacggtga agacctacag 360

gaacgcttta agcatcttag aagctcattt agaacgcttt tagaccgcta taacgattat 420

aagaaaaaag gccgtggctt taatcagttt tgtaaaattg atagtgcttt ctattccact 480

gaatatacct acaacccaaa aactaaagaa tggcatcctc atattcatat ttttgcttta 540

ctcaatgaat ggatagacca ggaagaatta gccgaaactt ggcacgacat taccctggat 600

tcttatatcg tcgatattcg aagagttaag aaaactaaag aacacggcta tagcaaagct 660

gttgcagagg tatgtaagta tgcccttaag tttagtgatc tctcactgga gaatacttgg 720

gaagcatatc tttctttgaa aggtaatagg ctgactggct gttttggttc tatgtatggt 780

gtcaagctac ctgaaaaact tactgatgat ctacctcttg atgatcttcc atatttggag 840

ctgctctatc gtttcgtatt tgataaaaaa tcctactaca acctagaaat aacaaaagac 900

gtaaagccaa acaaaaggaa tgaggaagga 930

<210> 642
<211> 174
<212> DNA
<213> Artificial Sequence

<220>
<223> orf

<400> 642
gtgaaggcga cacgtcaaat attagcaagg atacacaaag gtacctttat gccttgtaag 60

gggttcgacc ccttaaaccc caaagggcgc actttaccat tgagcaaagc tcaacggaaa 120

ttgctcccaa aaataagagg agctaaaaat gaattatttc actttgtata ttgt 174

<210> 643
<211> 354
<212> DNA
<213> Artificial Sequence

<220>
<223> orf

<400> 643
atcacattcc tatatatgaa aaaatcaggt gagaatatgc aaattgatga accaacaaac 60

ttccaagtat tcatggcaga agtaaacaag acagcaaaga cagaaagtat tggtgtttat 120

catcaagtac ctttccgtat ggctcgatgg aactttgcac gactggaagg cctccgtaat 180

cacatgggag aacctcgaaa caaagttctt aactcactaa tcgaaattgc tttagatcaa 240

gtatttgaac aacttgagca tggcagtaaa gagatccgtc gttctgttct ggaggaagtt 300

tccaaagttc tcgaatcaat tgaacatgat ggttctggga gcttagacaa tgat 354

<210> 644
<211> 288
<212> DNA
<213> Artificial Sequence

<220>
<223> orf

<400> 644
atgattaatc tacaaggaac tttgataaat gctttccgtg tggatggtgg taaaggtaaa 60

gacggcaaag aatatgaagc gcgtggcaag gtacaaattc ttggttcgtt ggaactccca 120

aacggggaaa ttaaacacga acttgttgac ctaacagttg aggattctcg actgtttgaa 180

ccattcaaaa atcaggttat tagcatctca tgtggtgcta tggctatcgg tcgtaatgtt 240

gttttttatg ttcgcaaagg cgcgaaacca gttttagcag aacaaatg 288

<210> 645
<211> 1032
<212> DNA
<213> Artificial Sequence

<220>
<223> orf

<400> 645
ttgactaaaa aagacaataa aaaagcccat aattcggatg ctttggcggg cgatgaatta 60

caggctttgc aatctgctaa ggcagatcaa cacagggata gaataacacg ttttgggatt 120

ttgaaacata gagcgaagct acaagaacaa tacttgtgga cgcaggttga tttcaaatca 180

gaaggtgaaa atgatctatc caataaggct cttaaggctg caaccaaatt aaaggggtgc 240

ggtcaatttc tgctattcca taattactac acaatagatc aagttaagct ggctaaagct 300

cattattgtt ctcagcattt actatgccct atgtgtgctg gtgtaagggc agctaagtca 360

atgagtcgat atatccaacg tattgaagaa ttgatgcgtc agaatcgcca tttaaagccc 420

gtattgatca ctttgacggt taagaacggt gaagacctac aggaacgctt taagcatctt 480

agaagctcat ttagaacgct tttagaccgc tataacgatt ataagaaaaa aggccgtggc 540

tttaatcagt tttgtaaaat tgatagtgct ttctattcca ctgaatatac ctacaaccca 600

aaaactaaag aatggcatcc tcatattcat atttttgctt tactcaatga atggatagac 660

caggaagaat tagccgaaac ttggcacgac attaccctgg attcttatat cgtcgatatt 720

cgaagagtta agaaaactaa agaacacggc tatagcaaag ctgttgcaga ggtatgtaag 780

tatgccctta agtttagtga tctctcactg gagaatactt gggaagcata tctttctttg 840

aaaggtaata ggctgactgg ctgttttggt tctatgtatg gtgtcaagct acctgaaaaa 900

cttactgatg atctacctct tgatgatctt ccatatttgg agctgctcta tcgtttcgta 960

tttgataaaa aatcctacta caacctagaa ataacaaaag acgtaaagcc aaacaaaagg 1020

aatgaggaag ga 1032

<210> 646
<211> 192
<212> DNA
<213> Artificial Sequence

<220>
<223> orf

<400> 646
ttgaatggtt caaacagtcg agaatcctca actgttaggt caacaagttc gtgtttaatt 60

tccccgtttg ggagttccaa cgaaccaaga atttgtacct tgccacgcgc ttcatattct 120

ttgccgtctt tacctttacc accatccaca cggaaagcat ttatcaaagt tccttgtaga 180

ttaatcattg tc 192

<210> 647
<211> 156
<212> DNA
<213> Artificial Sequence

<220>
<223> orf

<400> 647
gtgattacgg aggccttcca gtcgtgcaaa gttccatcga gccatacgga aaggtacttg 60

atgataaaca ccaatacttt ctgtctttgc tgtcttgttt acttctgcca tgaatacttg     120

gaagtttgtt ggttcatcaa tttgcatatt ctcacc                               156

## Description

The present invention relates to CMI (Cow Milk Isolates) nucleotide sequences as well as probes and primers comprising part of said nucleotide sequences and antibodies against polypeptides encoded by said nucleotide sequences. Finally, the present invention relates to the use of said compounds as an early marker for the future development of diseases such as cancer, atherosclerosis, diabetes and diseases of the CNS and as a target for treatment and prevention of these diseases.

### Background

A large set of novel viral and phage genomes has been identified during the past several years. These were identified through analyses of the human microbiome (Kowarsky et al., 2017; Fernandes et al., 2018) or of samples from other sources. Metagenomic analyses are commonly applied (Rosario et al., 2012; Gronenborn et al., 2018; Wang et al., 2018; Kazlauskas et al., 2018; Kraberger et al., 2015; Varsani et al., 2018), although the application of more conservative techniques also led to the identification of a number of new infectious agents (de Villiers et al., 2009; Peretti et al., 2015; zur Hausen and de Villiers, 2015). Analyses mostly relied on comparisons to other known agents or the identification of a replication gene in the assembled genome. Unfortunately, several studies concentrated only on the presence of known infectious entities, while paying less attention to identifying yet unknown or putative novel infectious agents (Moustafa et al., 2017; Sadeghi et al., 2017; Legoff et al., 2017). The discovery of chimeric genomes has weakened the strict demarcation between certain types or groups of infectious agents resulting in studies investigating their phylogenetic origin (Kurpovic, 2013; Krupovic et al., 2015; Roux et al., 2013; Wawrzyniak et al., 2017; Kazlauskas et al., 2017).

Published data on the epidemiology of colon and breast cancer suggested milk and meat factors (BMMFs) derived from dairy cattle as presumably species-specific risk for these cancers after consumption of products from these animals (zur Hausen, 2012; 2015; zur Hausen and de Villiers, 2015; zur Hausen et al., 2017; 2018). Putative Rep genes were identified as part of the BMMF's DNA sequences obtained by *in silico* comparisons to available sequences. Amplification using abutting primers in the rep gene led to the isolation of full and partial circular DNA genomes from bovine sera (Funk et al., 2014). This was extended to samples from commercially available milk products for the presence of specific circular single-stranded DNA genomes. Full-length circular single-stranded DNA molecules of 14 different isolates of (∼1100 to 3000 nucleotides) were cloned and sequenced (Whitley et al., 2014; Gunst et al., 2014; Funk et al., 2014; Lamberto et al., 2014). Four additional isolates were obtained from human brain and serum (all from patients with multiple sclerosis) (Whitley et al., 2014; Gunst et al., 2014; Lamberto et al., 2014). The 18 isolates were divided into four different groups BMMF1 through BMMF4, according to their molecular characteristics (zur Hausen et al., 2017). Three of these groups revealed a remarkable degree of similarity to *Acinetobacter baumannii* and Psychrobacter plasmids. The fourth group comprised 3 isolates being representatives of *Gemycirularviridae.* Representatives of all four BMMF groups have been analyzed for expression of genetic activity and replication in human cells (zur Hausen et al., 2017; Eilebrecht et al., 2018).

Thus, the technical problem underlying the present invention is to identify specific nucleotide sequences that might be associated with diseases such as cancer, atherosclerosis, diabetes or diseases of the CNS and, thus, to provide means for diagnosis and therapy.

### Brief Description of the Present Invention

The solution to said technical problem is achieved by providing the embodiments characterized in the claims.

Recently, circular DNA genomes (Healthy Cattle Blood Isolate: HCBI1, HCBI2 and HCBI7) were isolated from serum from healthy cattle (Funk et al., 2014). These isolates were grouped as BMMF2 based on their nucleotide identity to the previously described Sphinx2.36 (acc. no HQ444405, Manuelidis et al., 2011, zur Hausen et al., 2017). The DNA sequences and open reading frames of several of these components showed a recognizable relationship to two sequences which were already described in transmissible spongiform encephalopathies (TSE) for TSE-diseases of sheep and humans.

The TSE isolates have also been hypothesized to play a role in cancer induction (Manuelidis, 2011). The inventors of the present invention postulated a role of infections in specifically colon, breast and prostate cancers but also Hodgkin's disease and others, and diseases of the CNS, e.g. Multiple sclerosis MS, amyotrophic lateral sclerosis, Parkinson's disease, Alzheimer disease (zur Hausen, 2012, zur Hausen et al., 2017, 2018).

The inventors subsequently used two sets of abutting PCR primers overlapping in the highly conserved region of their Rep genes to amplify full-length circular DNA molecules. The inventors analyzed 16 cow milk products, mainly milk samples for the presence of additional members of this group. The full-length circular genomes of 97 isolates (16 cow milk products, 81 cow milk) belonging to the BMMF2 group were generated ranging from 2102 to 3090 nucleotides in size and the 23 sequences (all from cow milk samples and all belonging to the BMMF2 group; C2MI) claimed in the present application are representative members thereof. BLASTN comparisons to all available DNA databanks, revealed similarity mainly to plasmids of *Acinetobacter baumanii,* (*Acinetobacter baumannii* str. AYE plasmid p4ABAYE, acc. no CU459139, *Acinetobacter baumannii* strain A85 plasmid pA85-1, acc. no CP021783 and *Acinetobacter baumannii* strain DS002 plasmid pTS236 acc. no JN872565, Longkumar et al., 2013), but also to ssDNA isolates from Rat gut (pRGRH0103 acc. no LN852793 and pRGRH0636 acc. no. LN853262). The nucleotide sequence of p4ABAYE differs from pA85-1 in 66 positions (Hamidian et al., 2014).

*In silico* analyses of putative open reading frames (ORF, >50aa) were performed on each genome by using alternative start codons (Kearse and Wilusz, 2017). This resulted in the identification of a replication gene in each isolate with mainly 2 additional major ORFs on the positive DNA strand. The encoded putative proteins ranged from 101 to 138 amino acids and 96 to 148 amino acids in size, respectively. Additional smaller ORFs were randomly present. Multiple ORFs (average number 2 to 10, up to 125 amino acids in size) were identified on the reverse strand. BLASTP analyses of the latter putative proteins revealed no or very weak similarity to known proteins.

### Characterization of the BMMF2 Rep Proteins

Bacterial plasmids are classified based on nucleotide homology of their replicase genes (Bertini et al., 2010). The inventors compared the putative Rep proteins of all BMMF2, as well as BMMF1 isolates. A core conserved region (329aa) was used in the phylogenetic analyses. BMMF2 rep proteins were grouped into 2 clades (A and B), whereas BMMF1 rep proteins formed a third clade (C). Clades A and B shared 60 to 65% similarity. Rep proteins in clade A shared 69-90% similarity within the clade and 70-82% to the p4ABAYE rep. Similarly clade B rep proteins shared 80-90% within the clade and 59-62% to p4ABAYE rep. The rep proteins of BMMF1 (clade C) are more diverse and shared almost no similarity (10-12%) to clades A and B.

The putative rep proteins of several BMMF2 isolates did not result only from one ORF, but were compiled of more than one truncated ORF. In order to ascertain whether potential introns were present in these interrupted BMMF2 genomes, the inventors performed GENSCAN analyses (Burge and Karlin, 1997). This tool has been developed to identify genes in prokaryotes, eukaryotes and plants. The inventors aligned these genes to the respective full-length genome. Very notable in the GENSCAN products are the presence of a polyadenosine tails (polyA) in almost every isolate, including the plasmid p4ABAYE. PolyA plays an important role in the maturation of RNA transcripts of almost all eukaryotes by stabilizing the mRNA for transport to the cytoplasm and subsequent translation (Curinha et al., 2014).

### Brief description of the drawings

### Abbreviations:

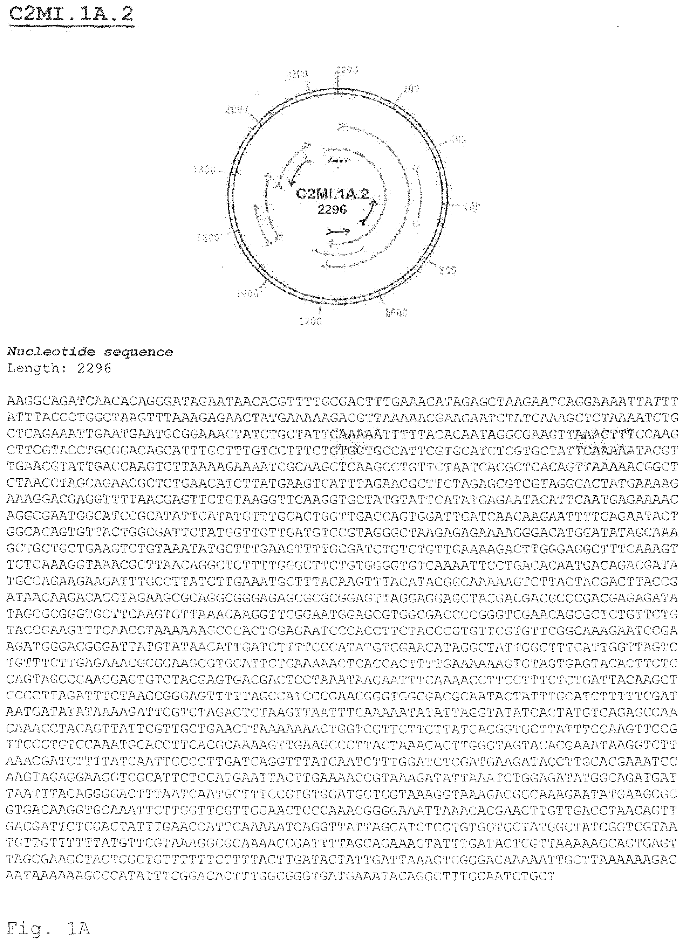

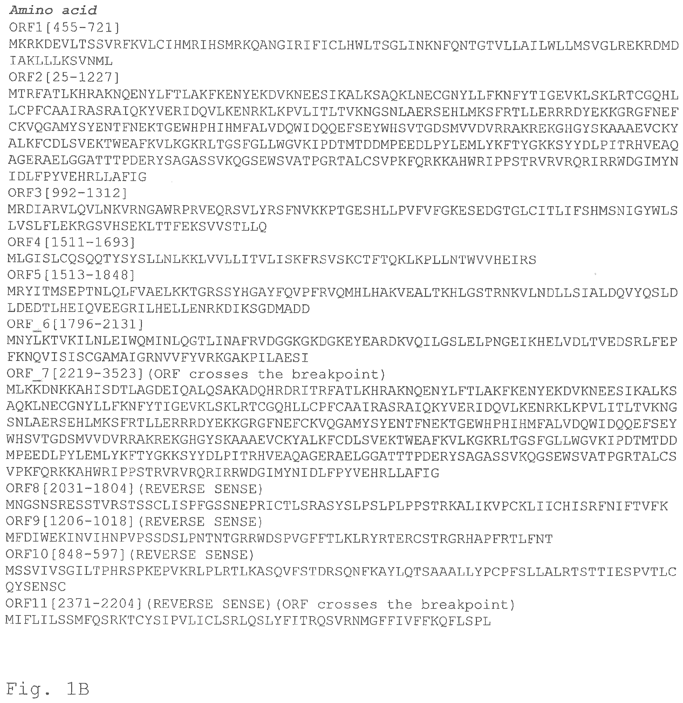

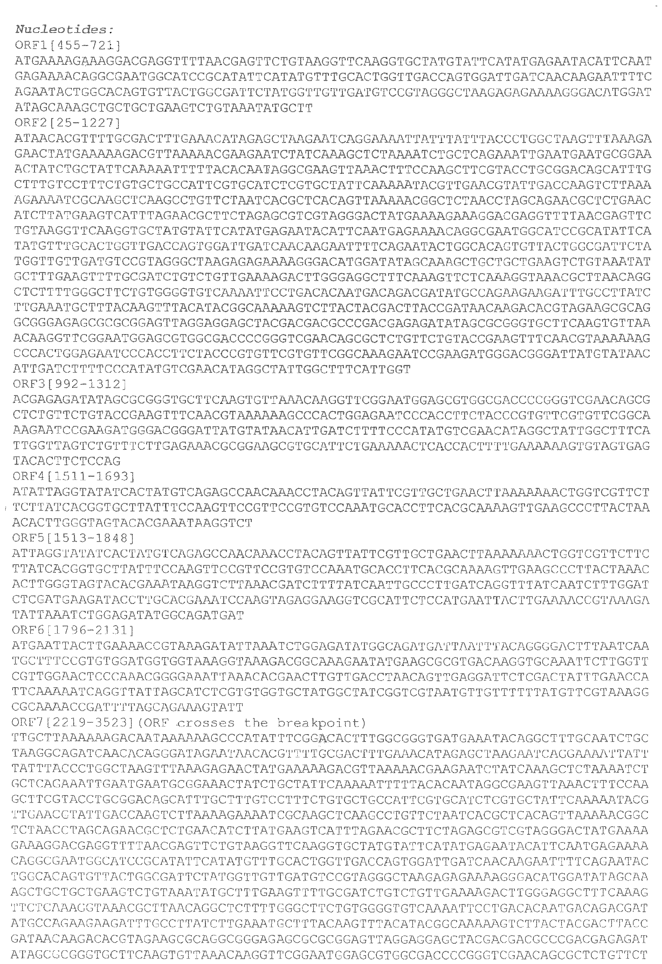

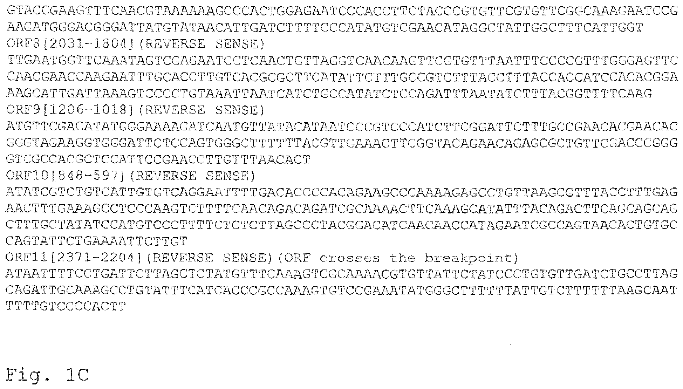

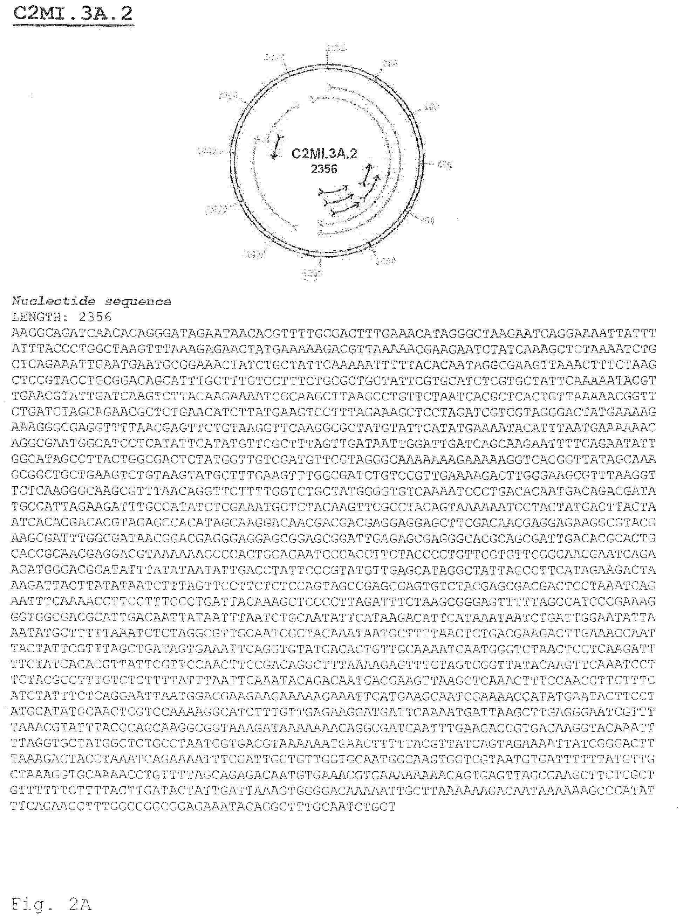

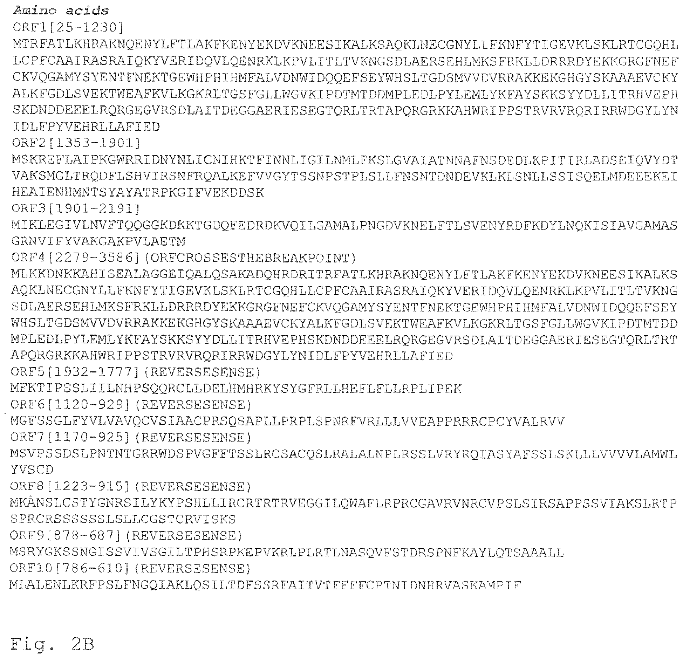

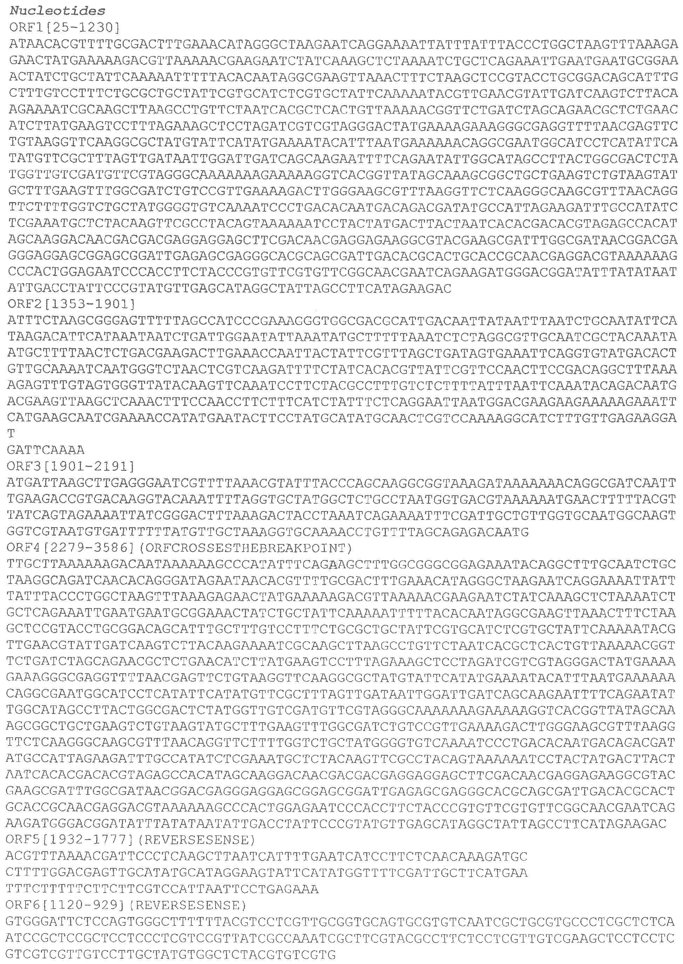

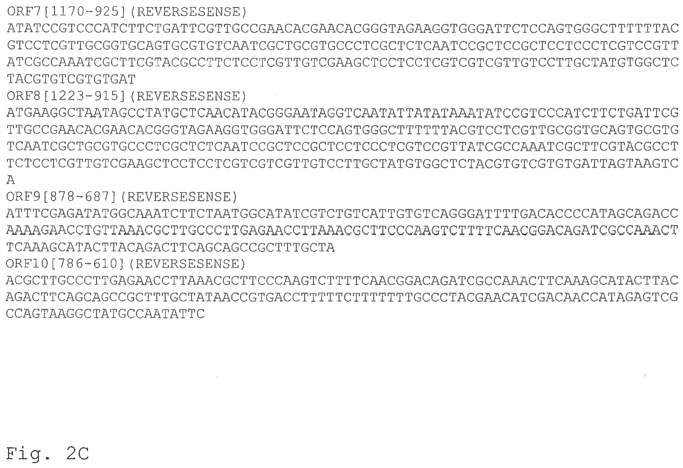

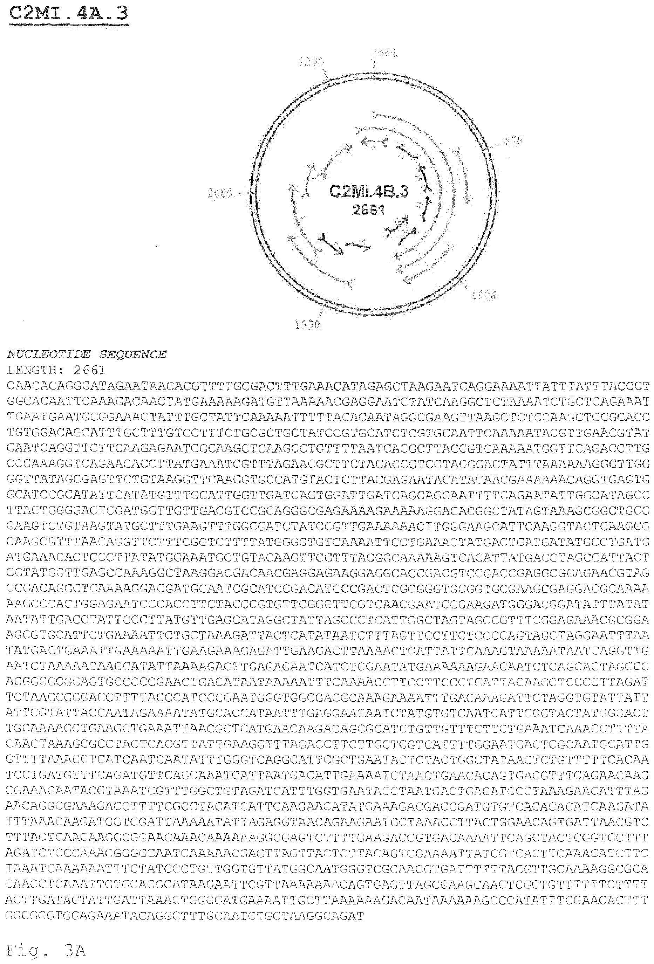

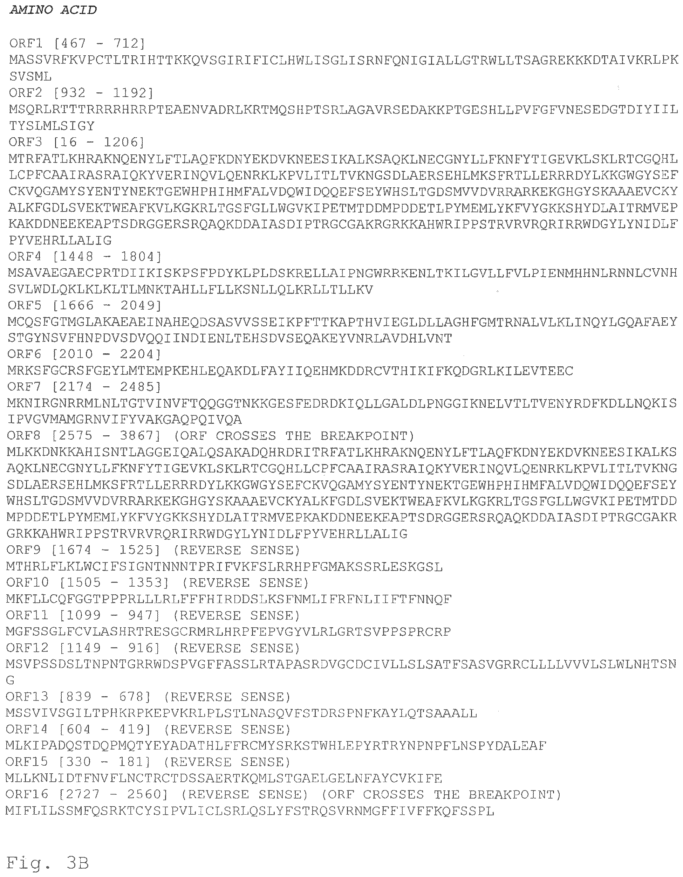

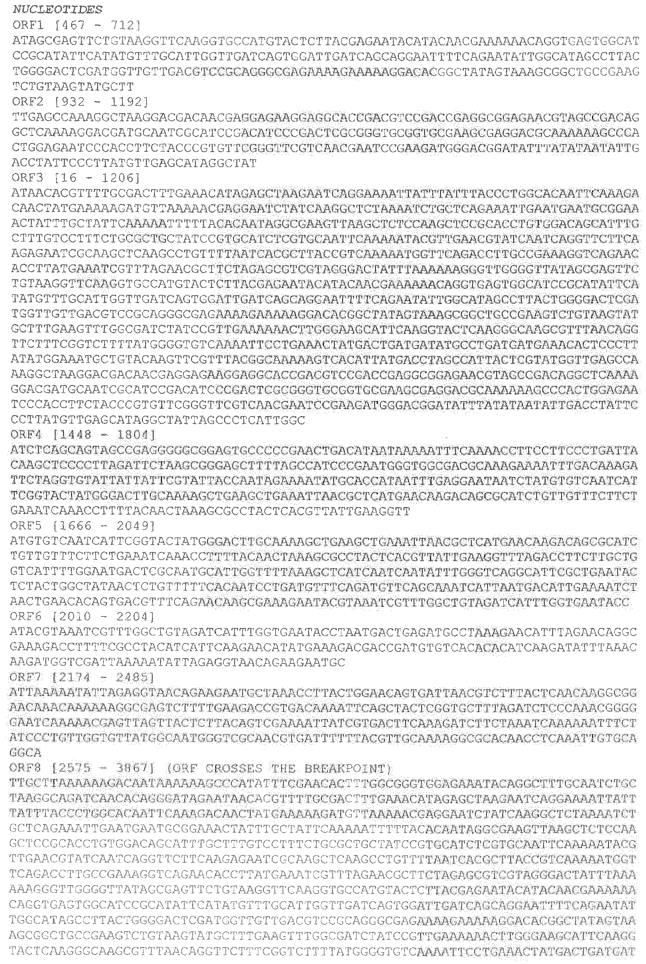

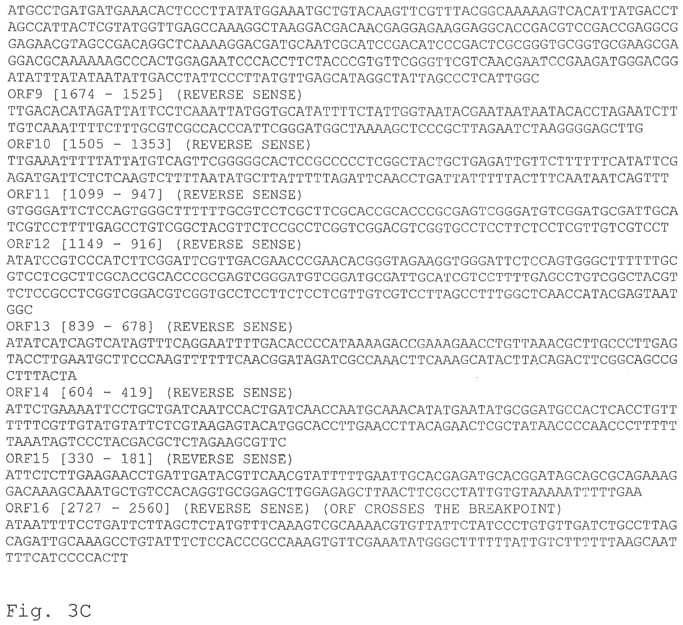

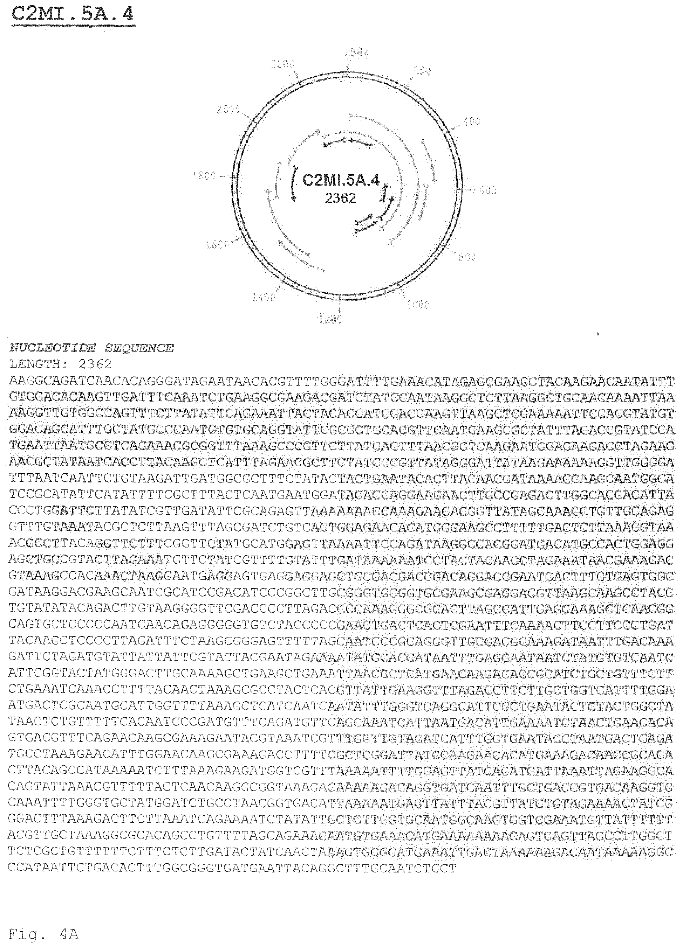

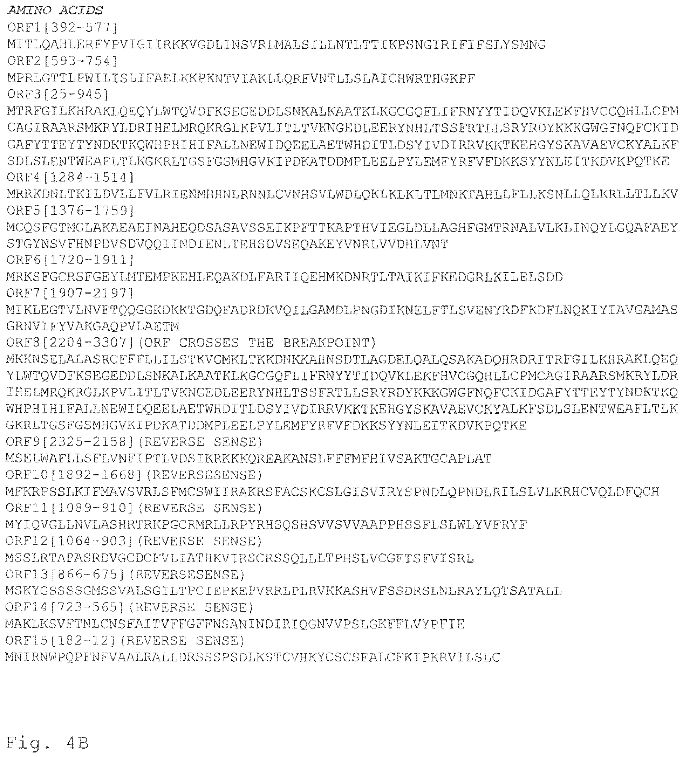

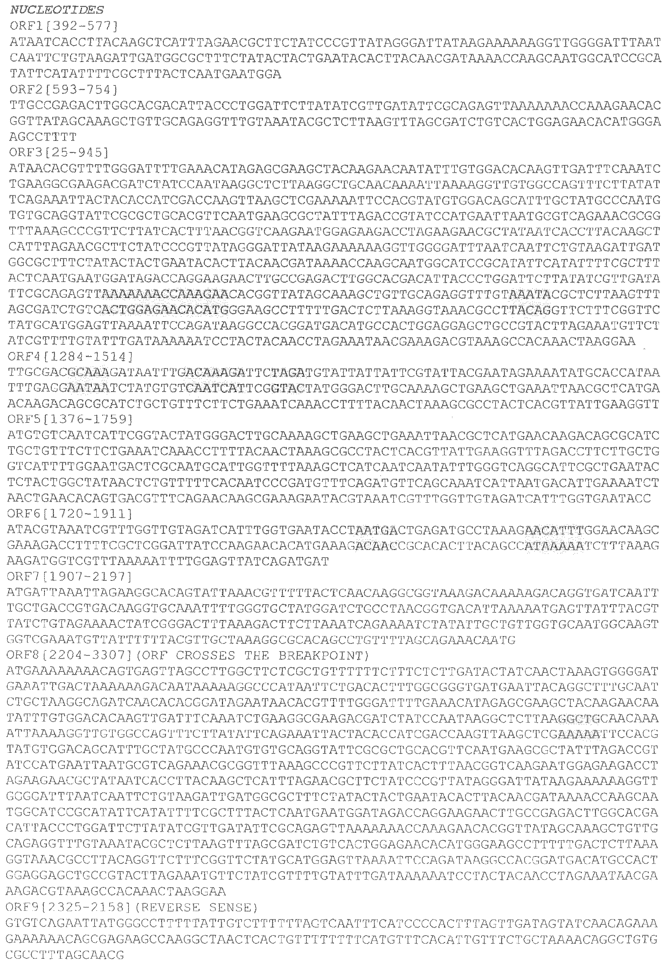

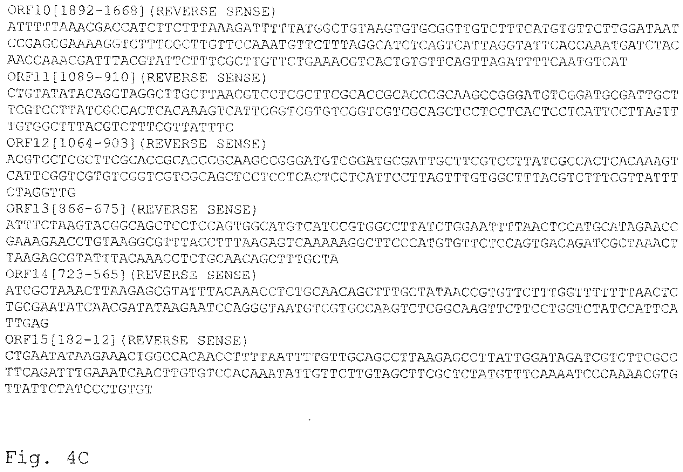

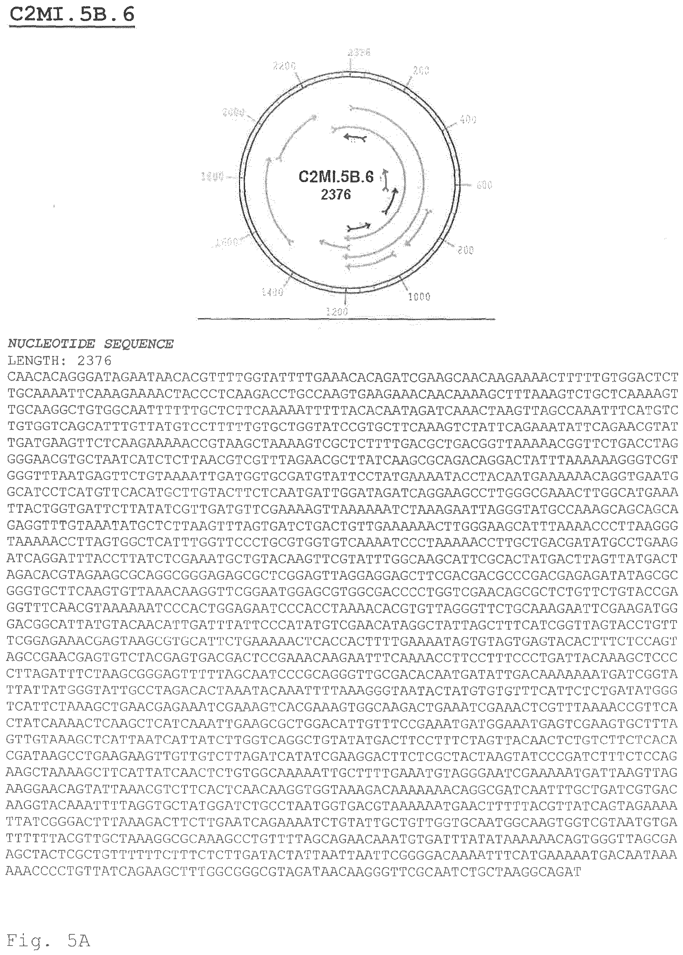

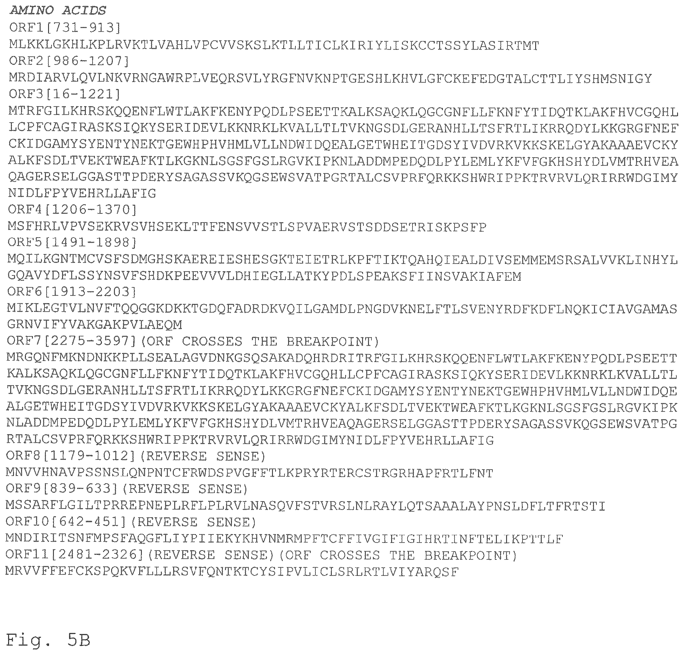

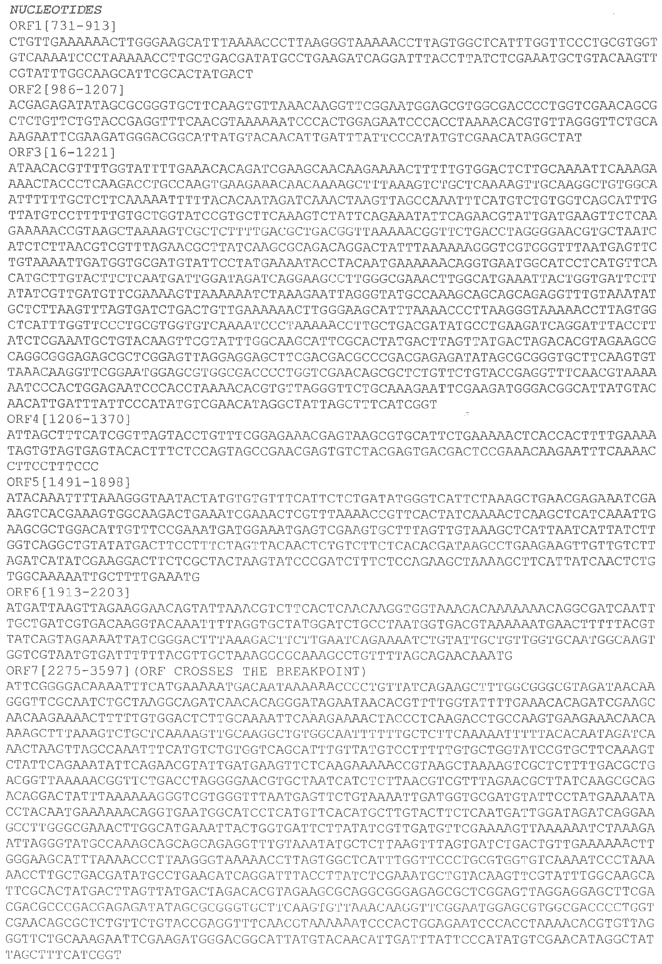

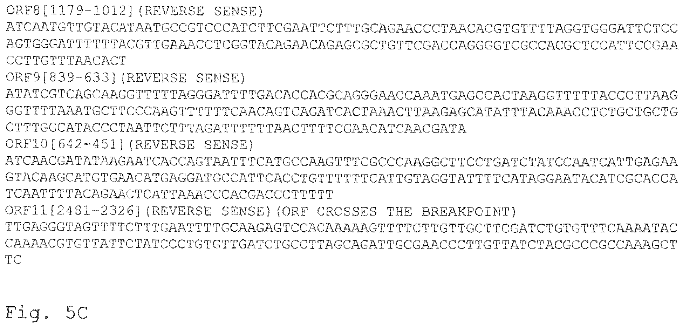

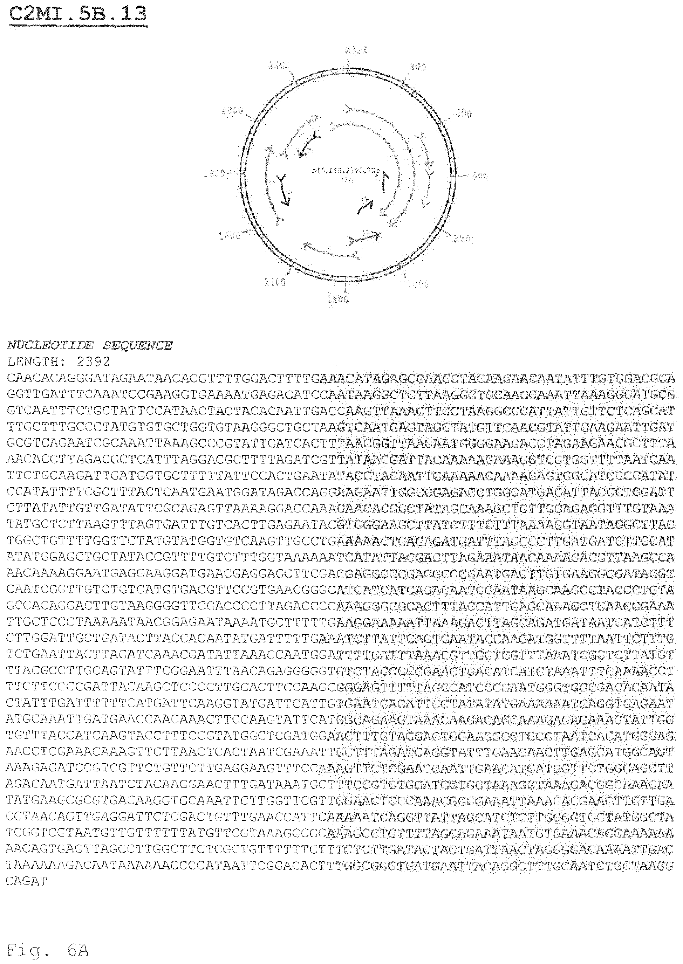

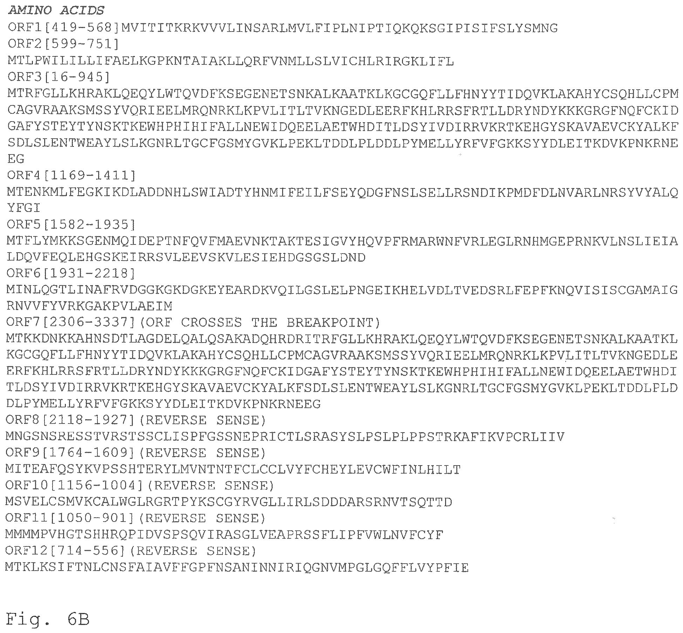

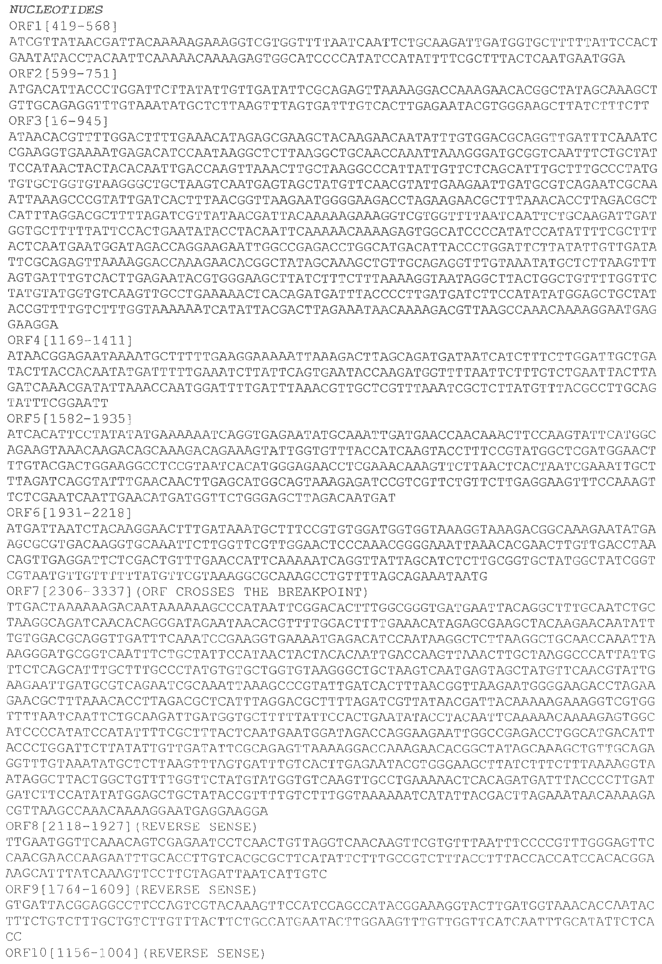

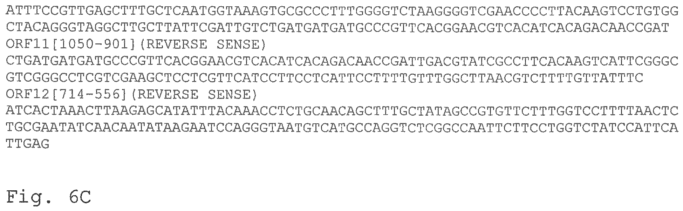

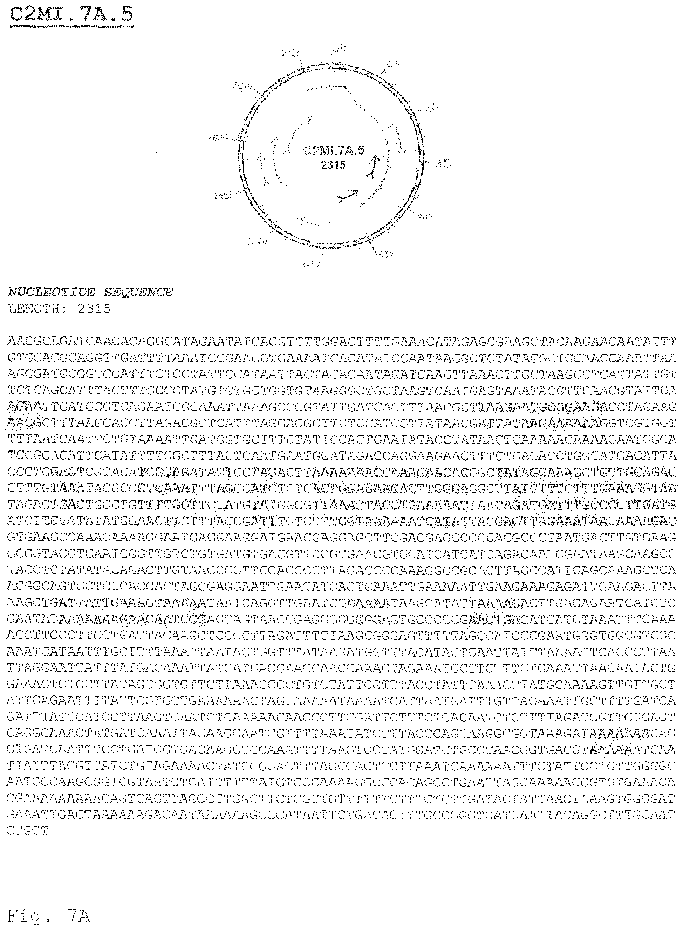

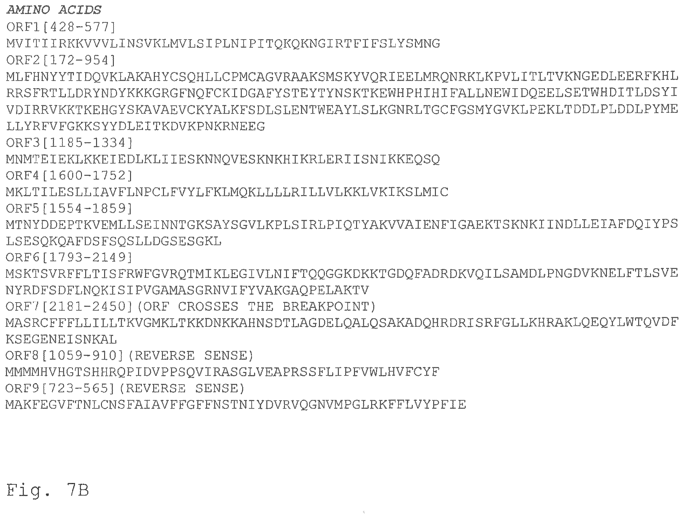

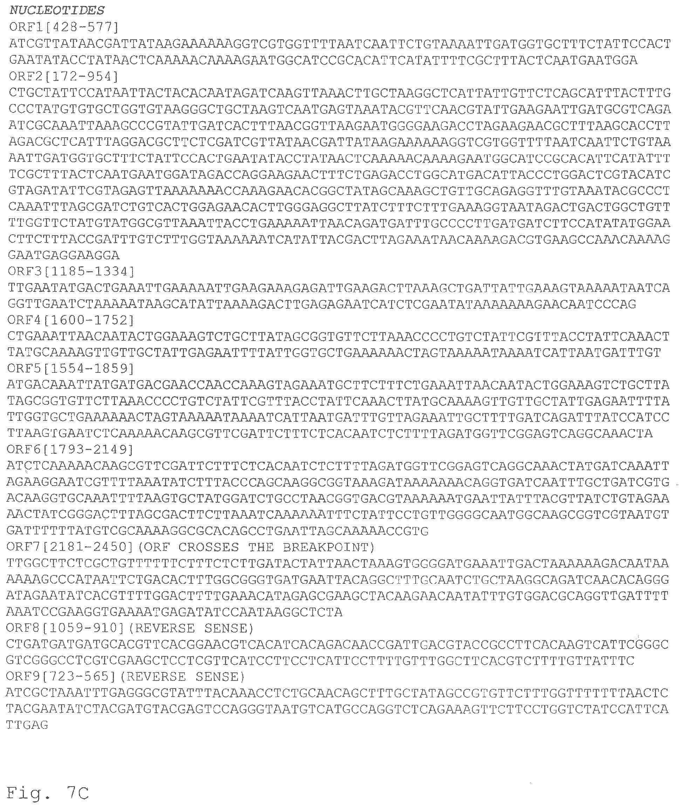

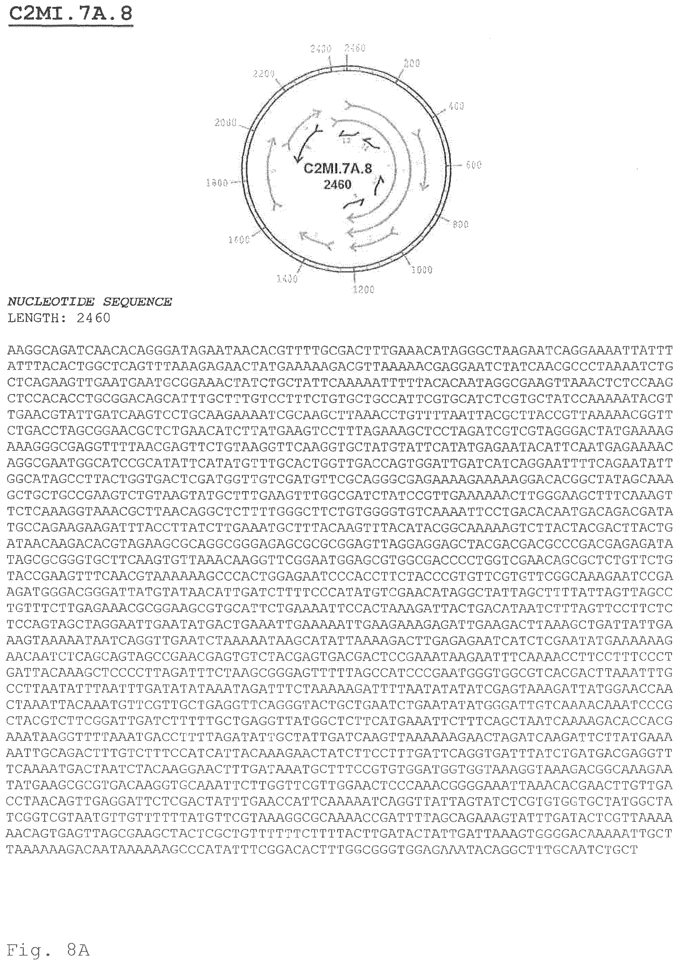

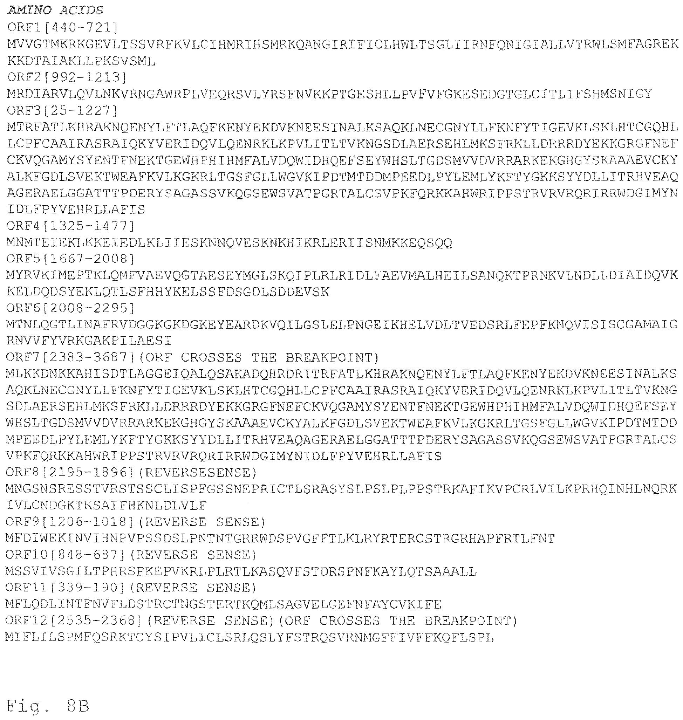

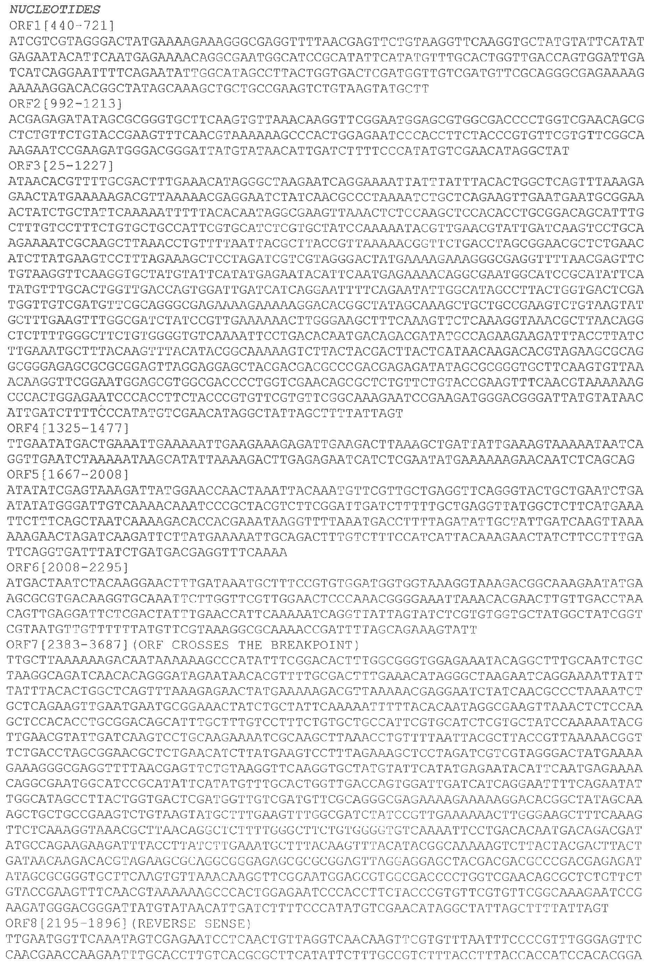

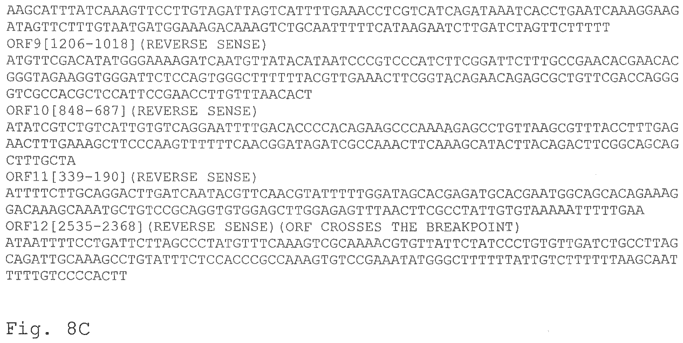

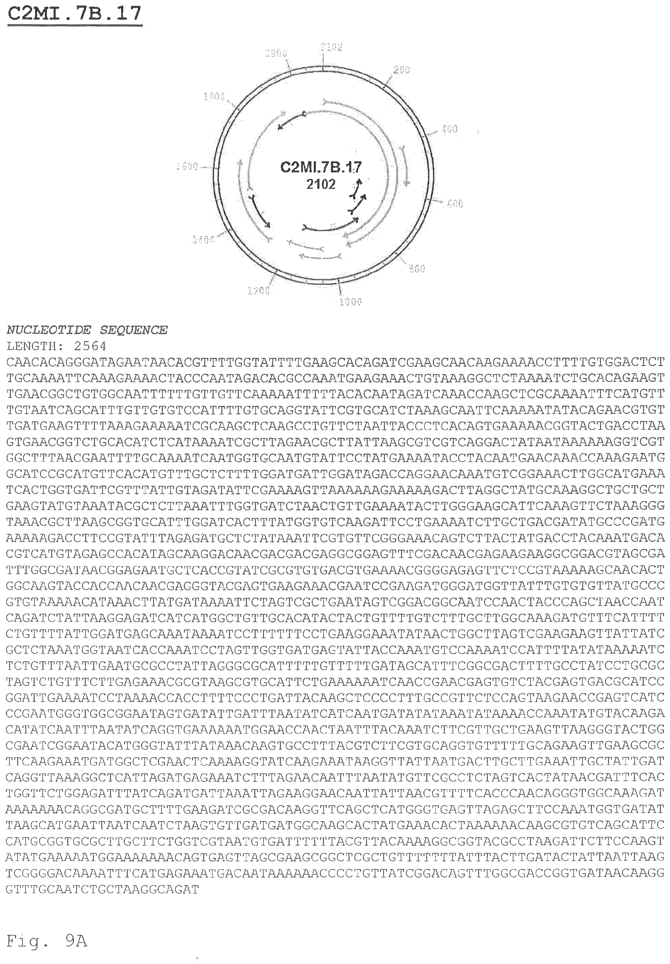

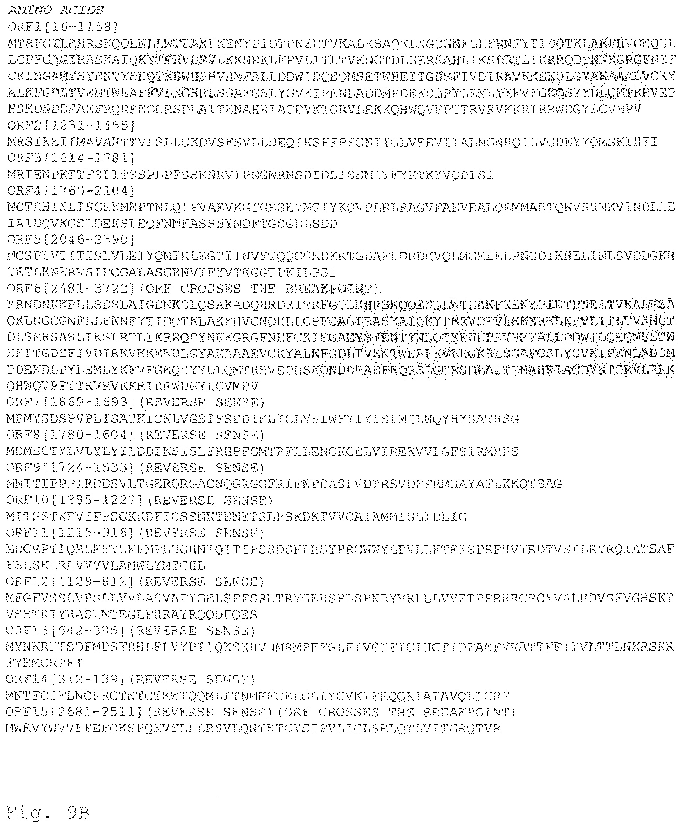

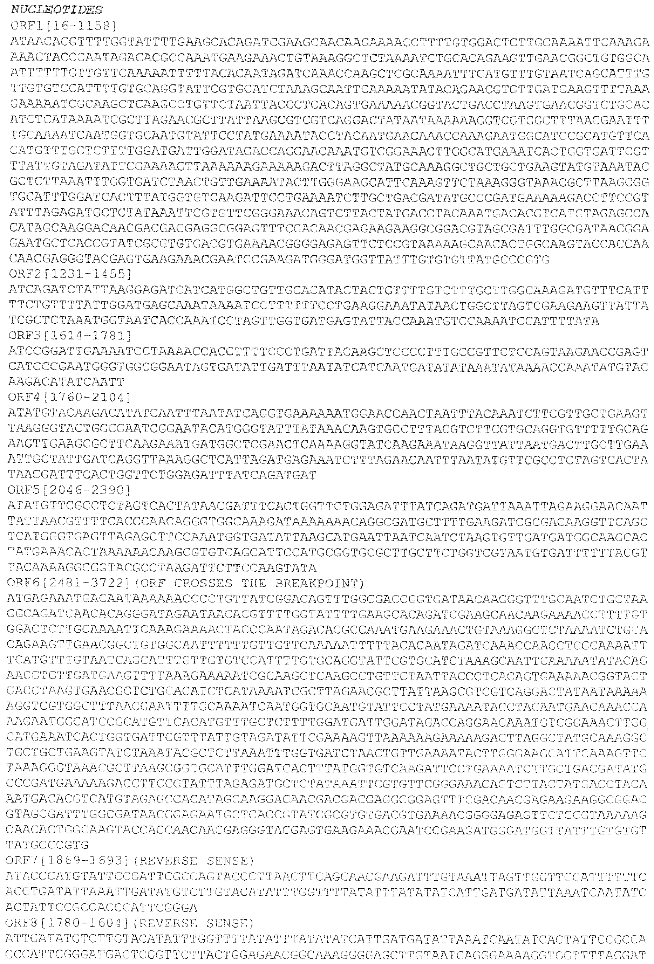

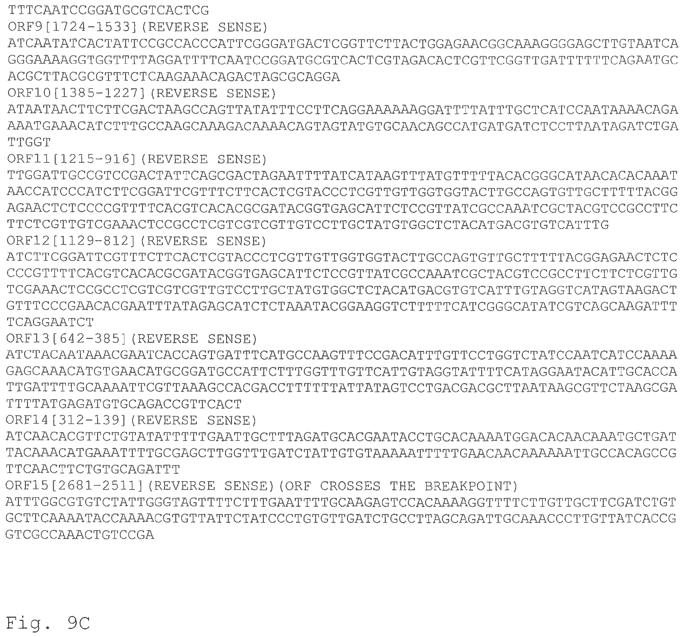

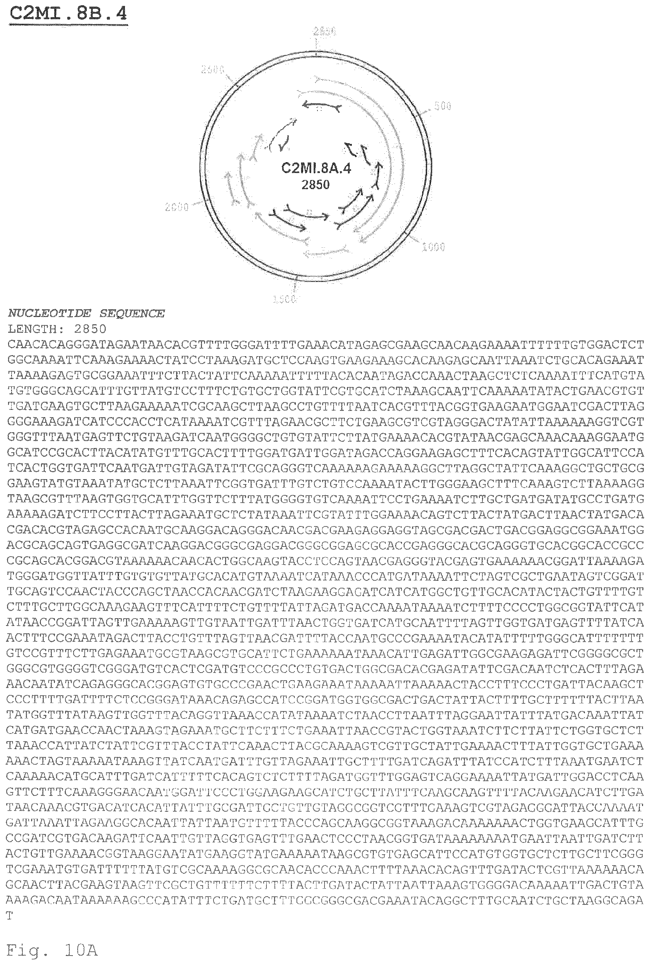

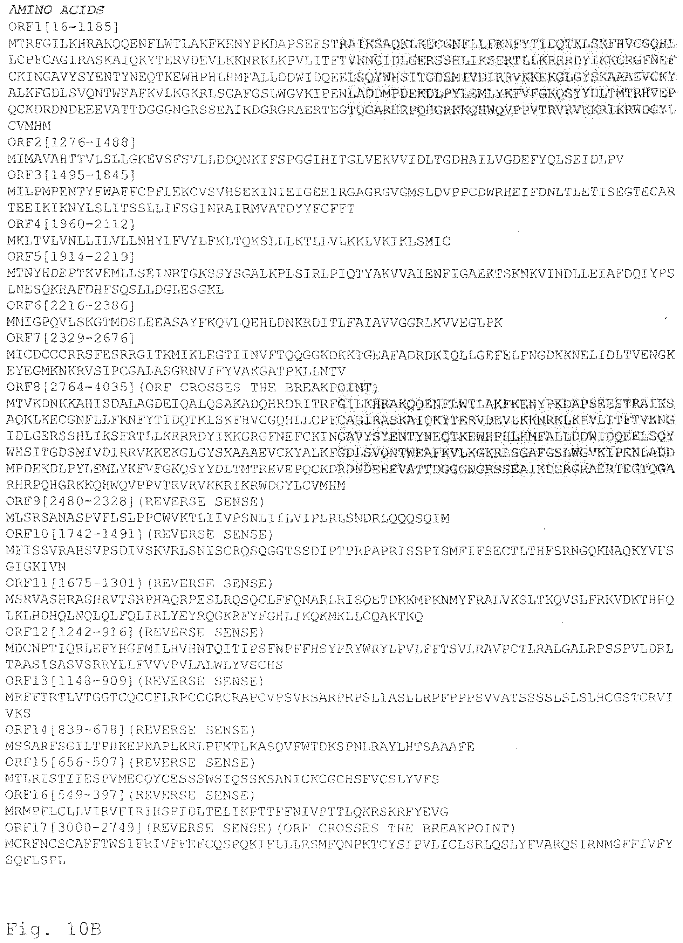

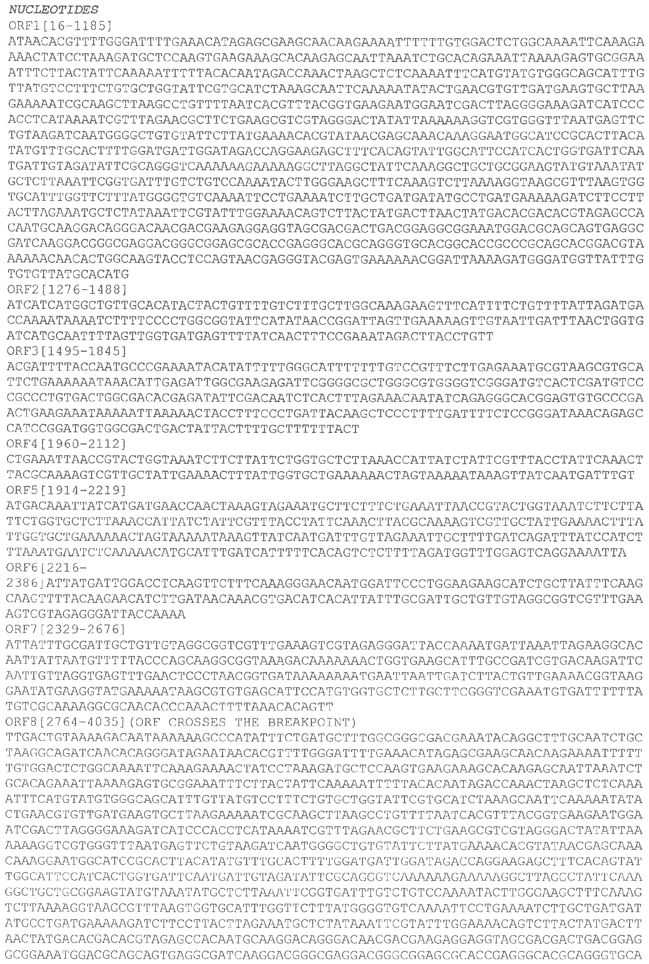

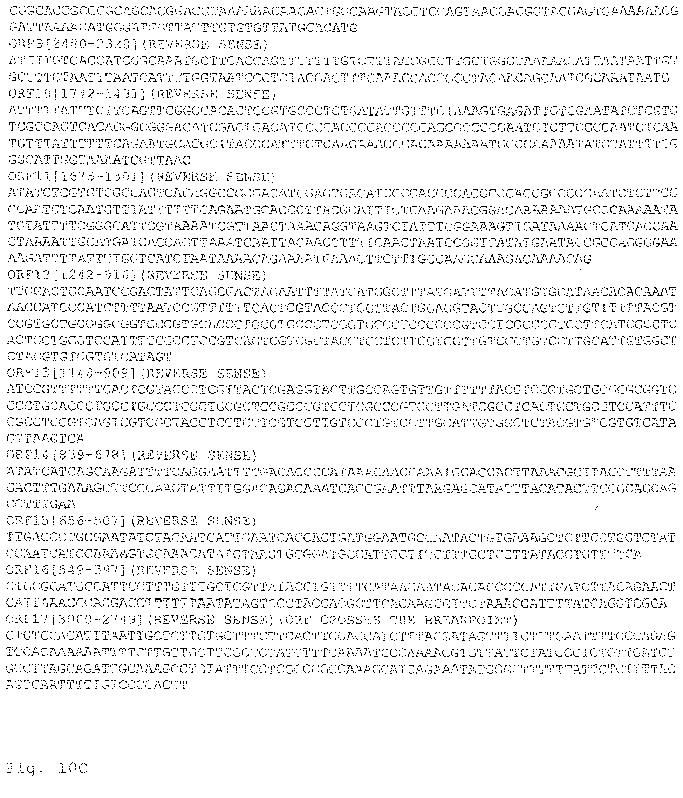

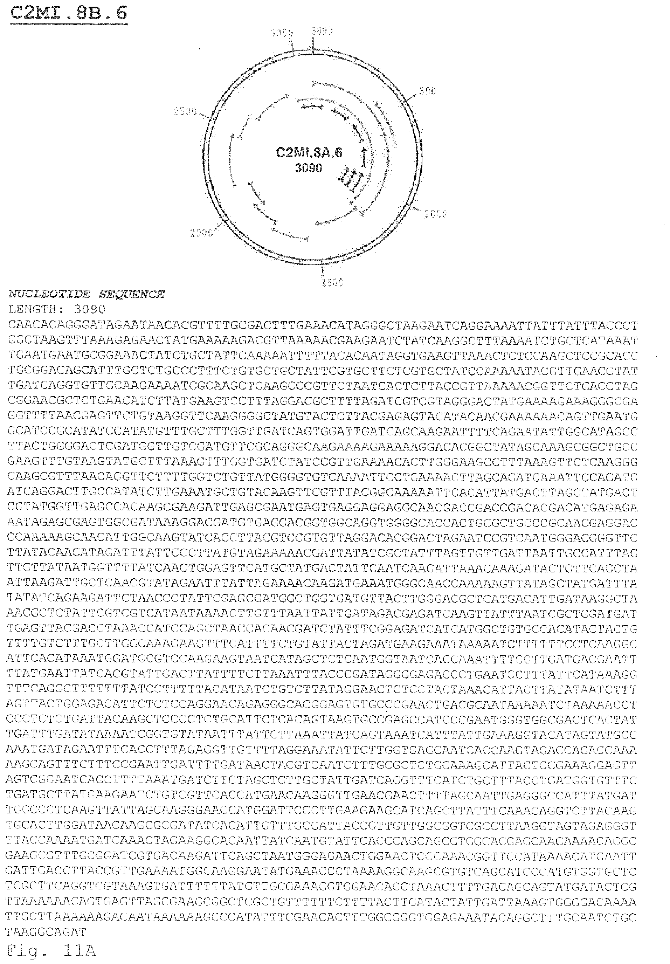

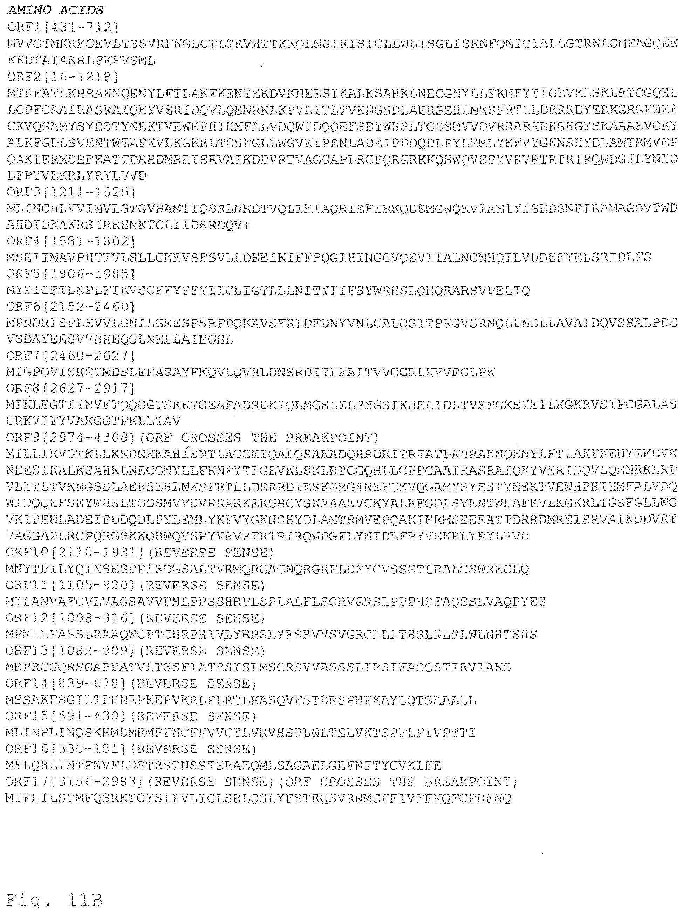

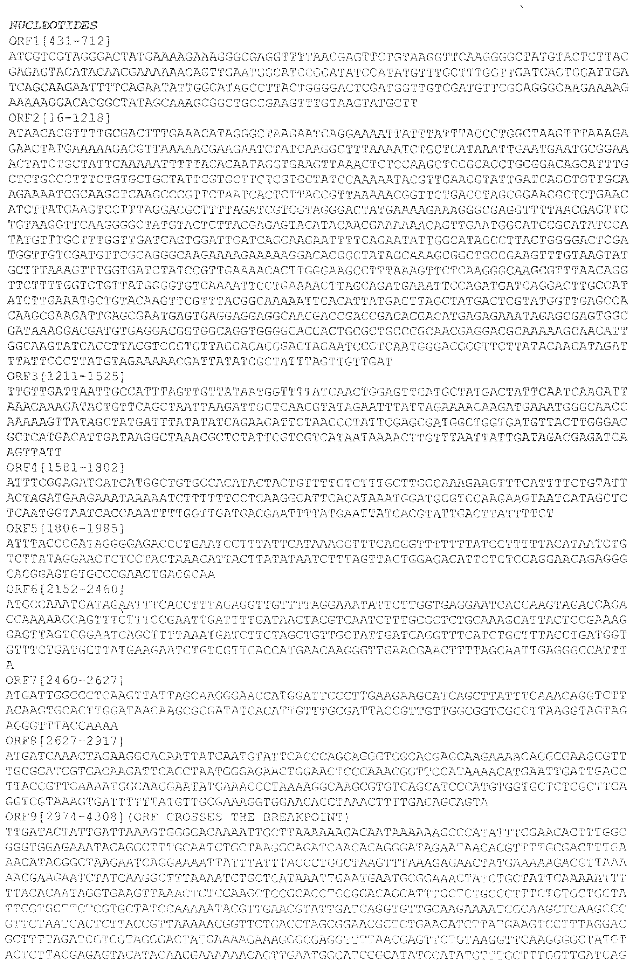

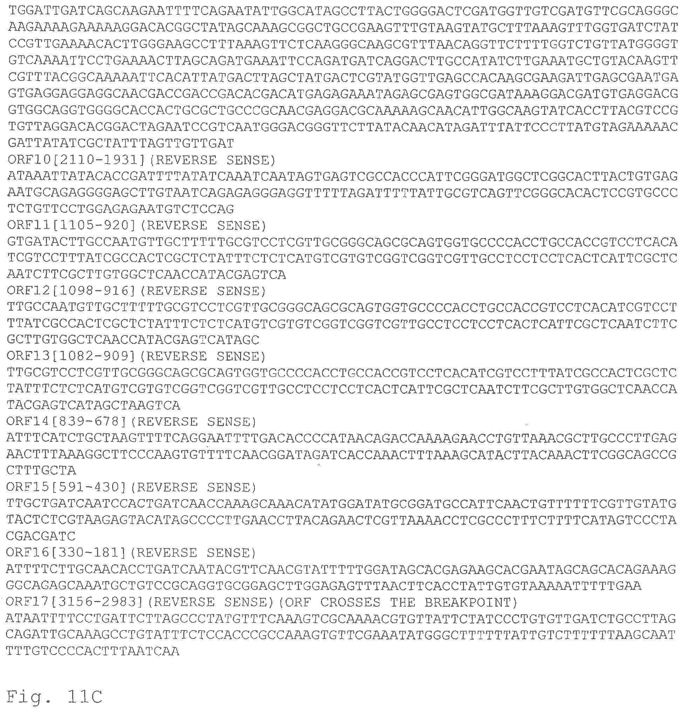

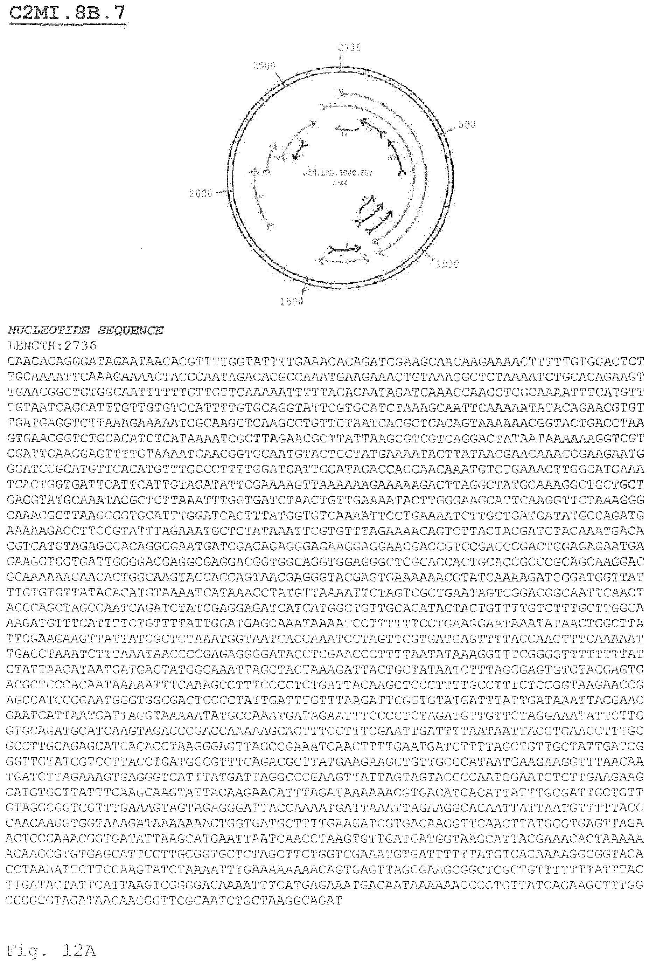

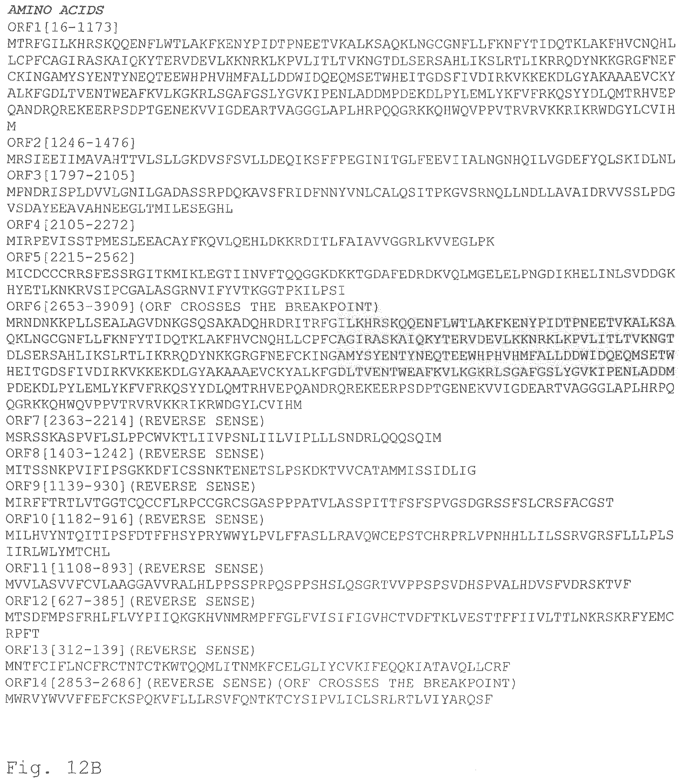

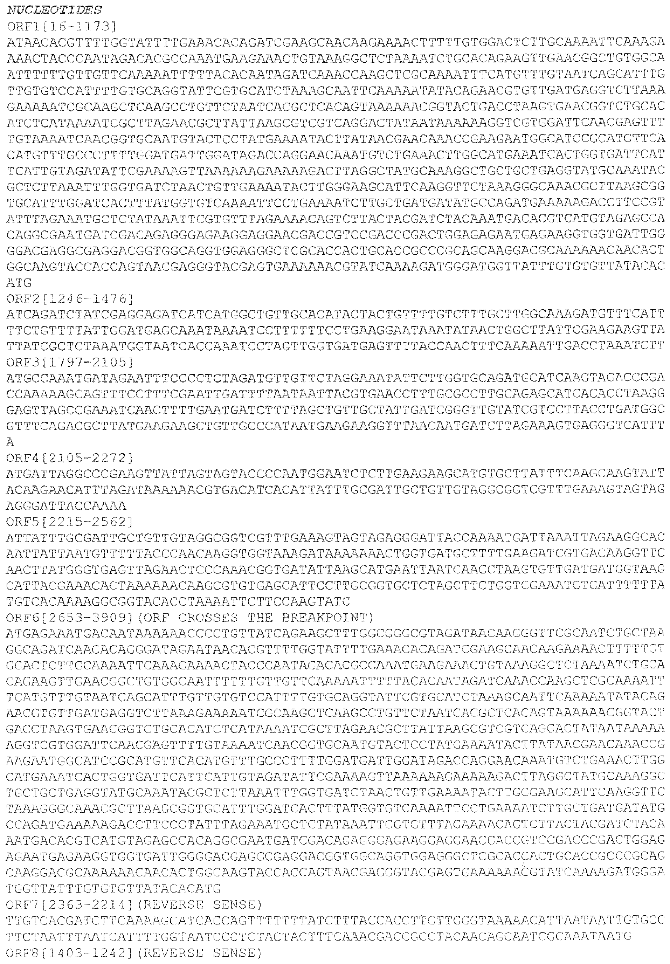

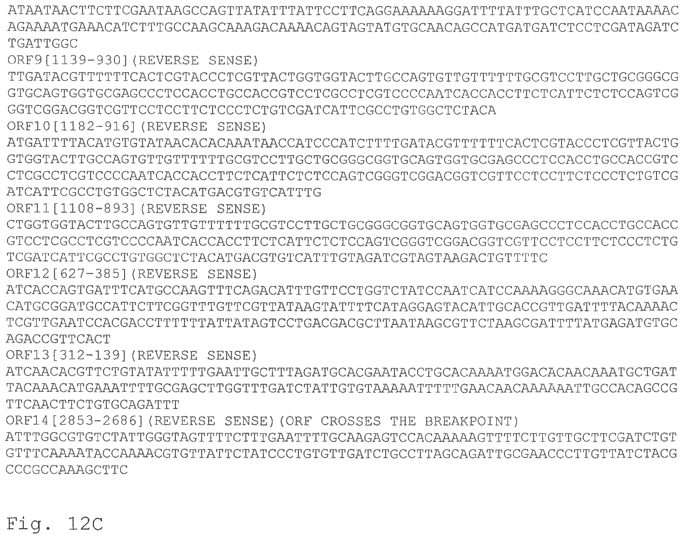

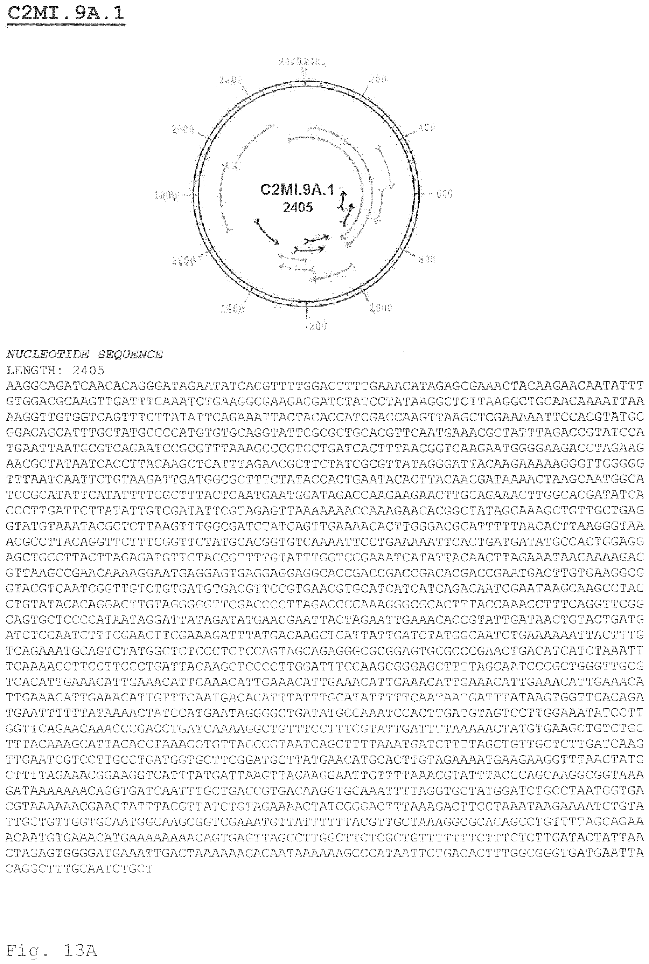

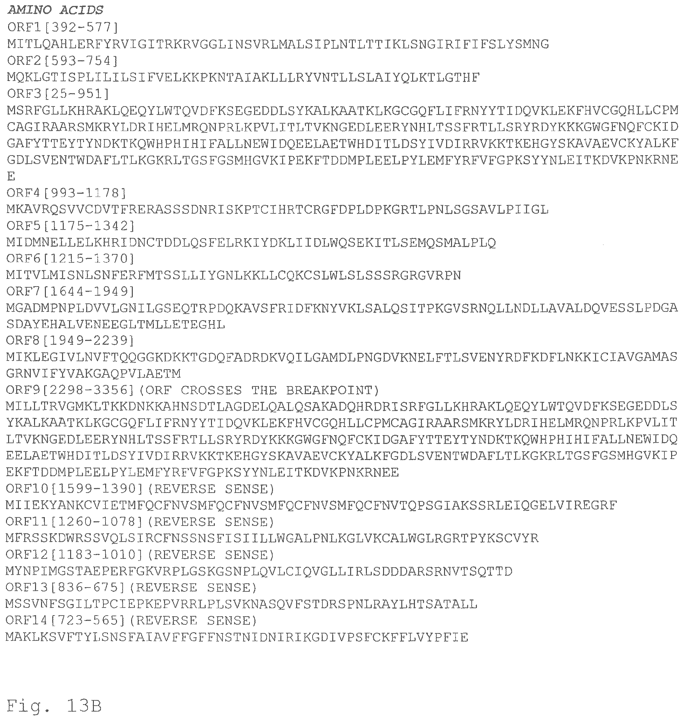

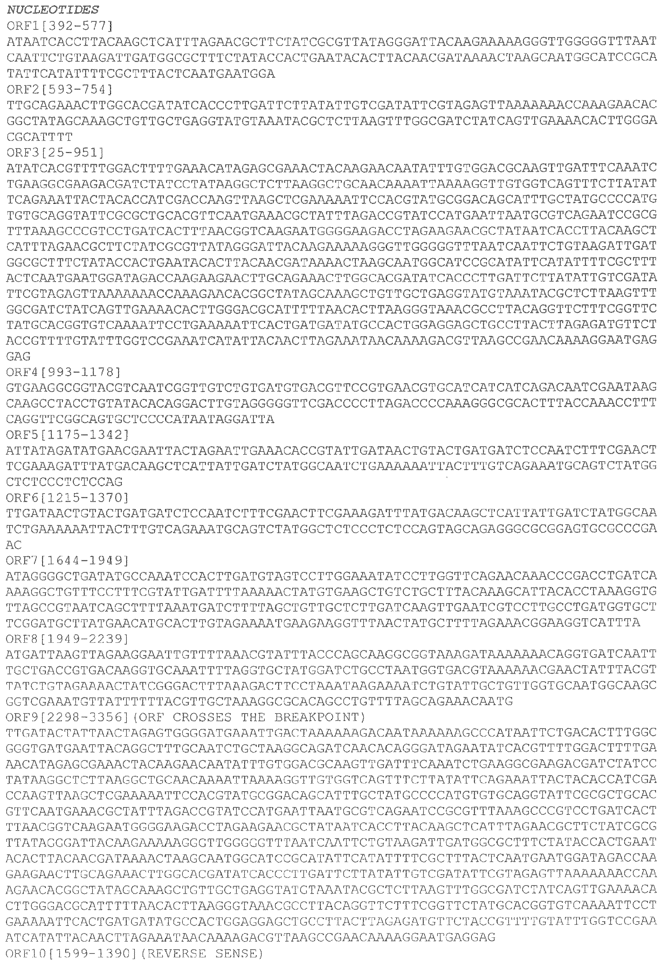

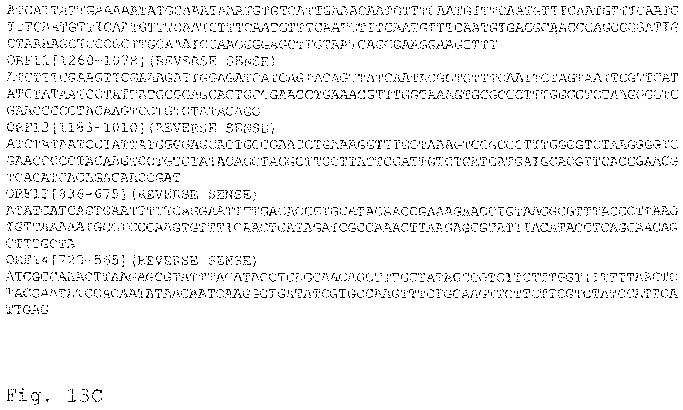

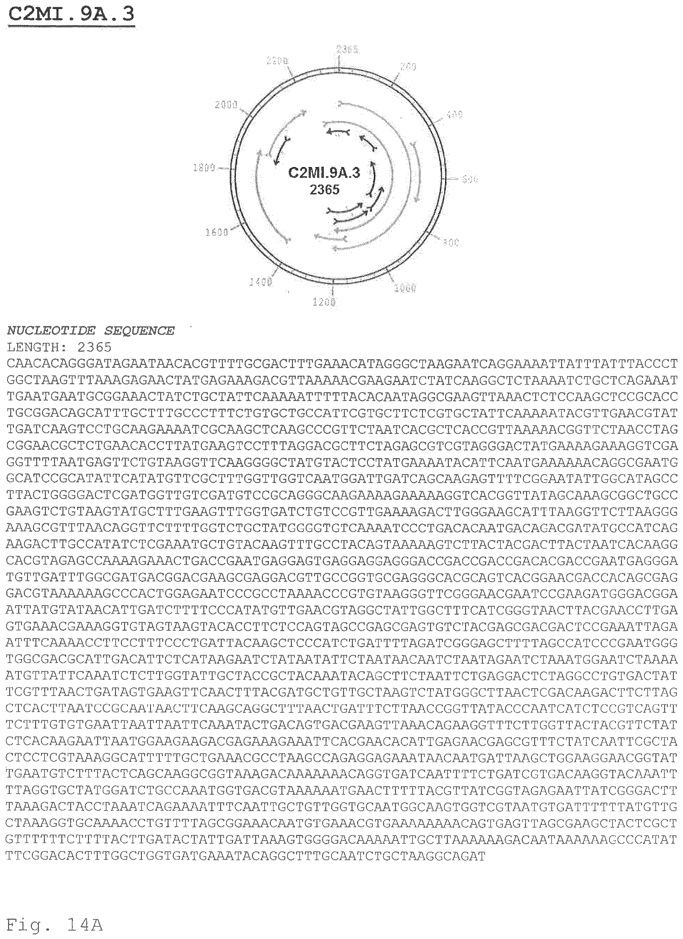

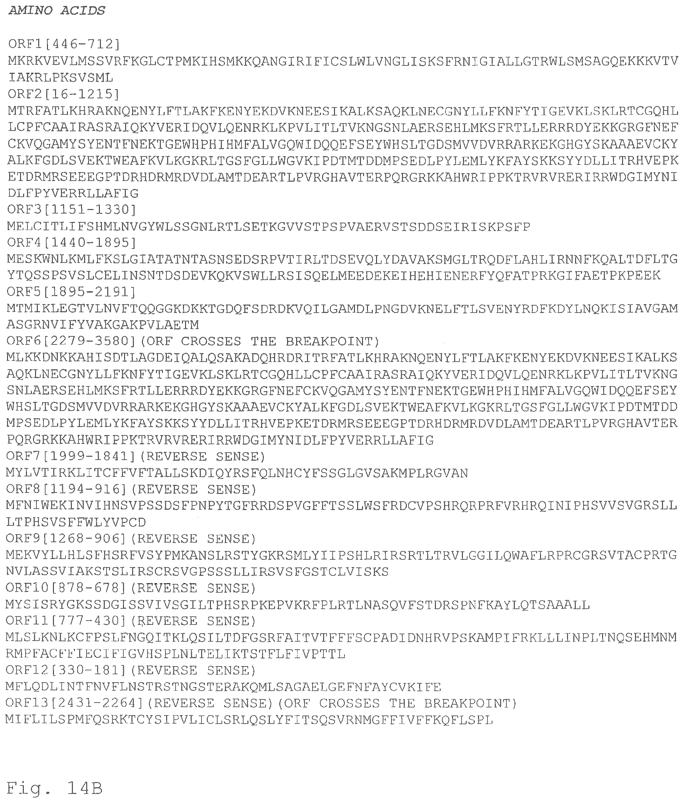

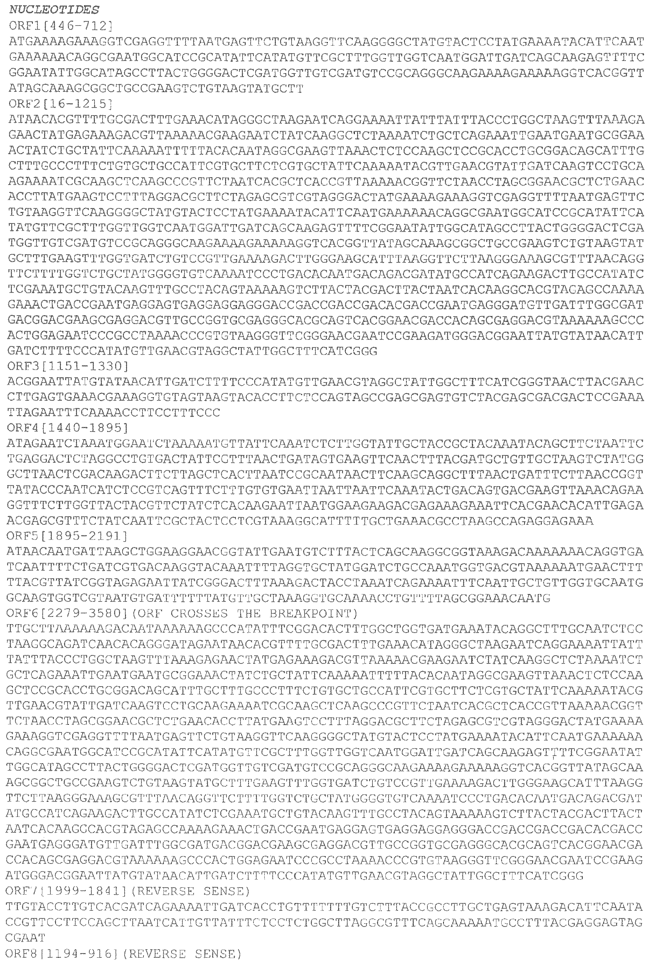

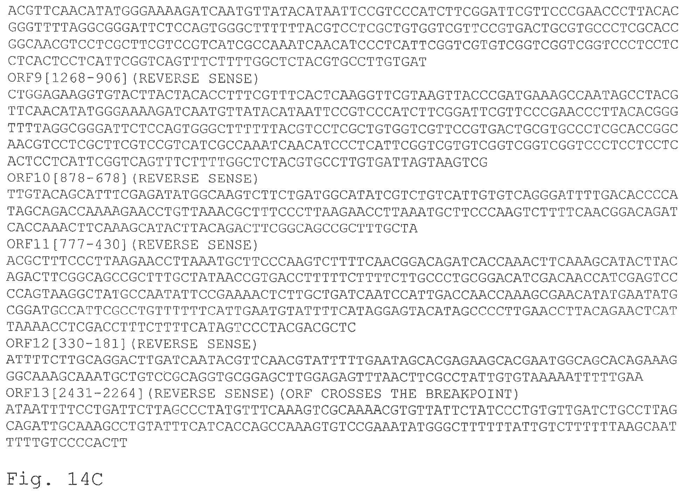

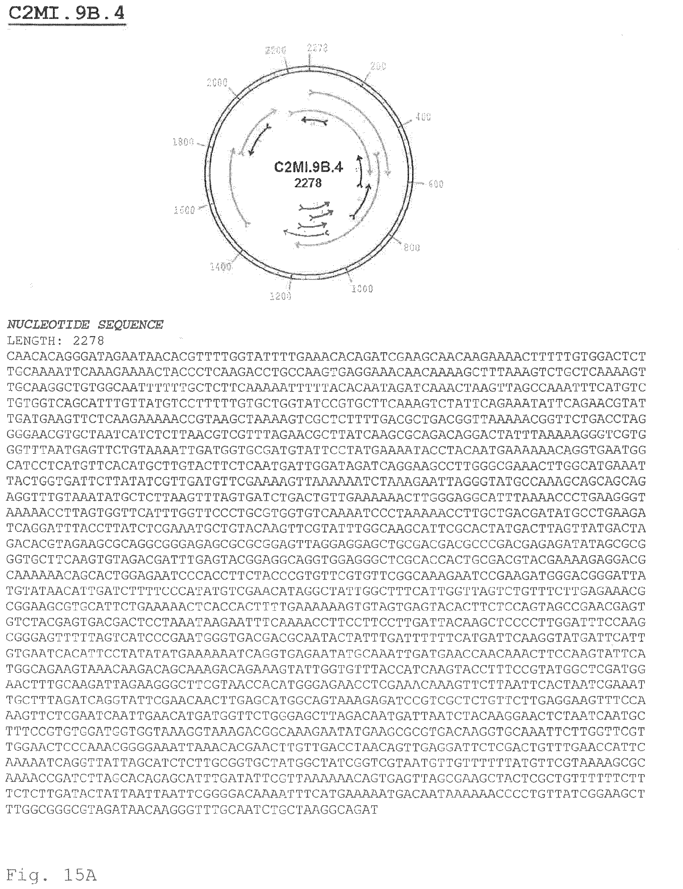

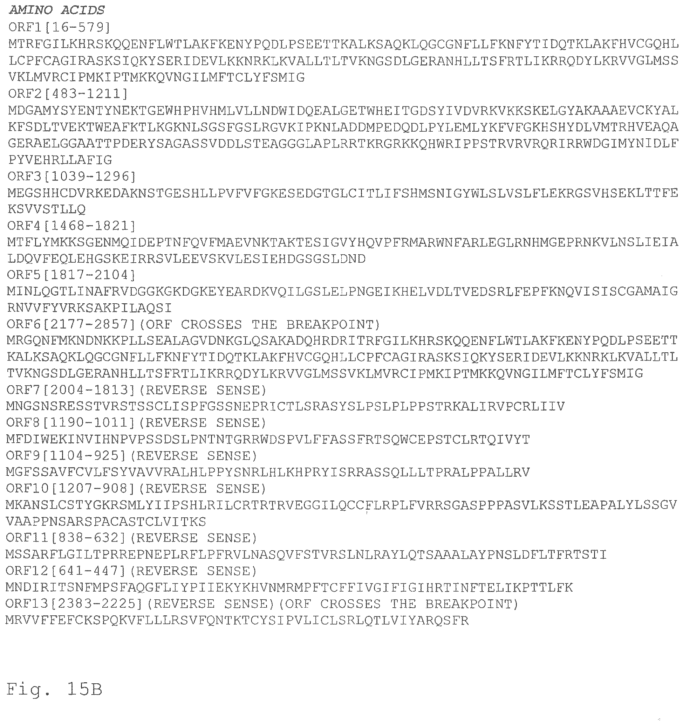

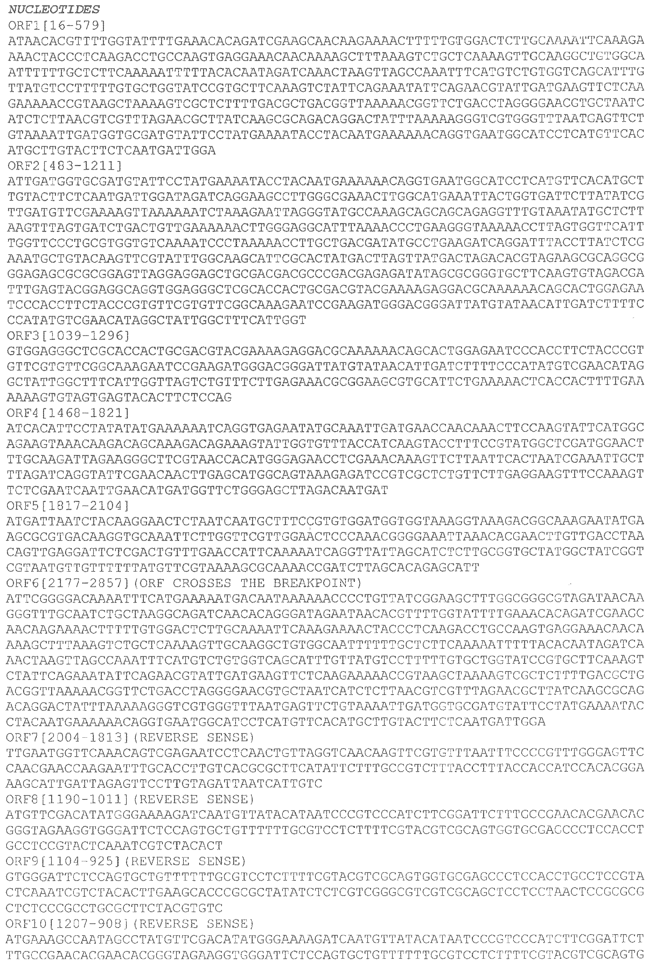

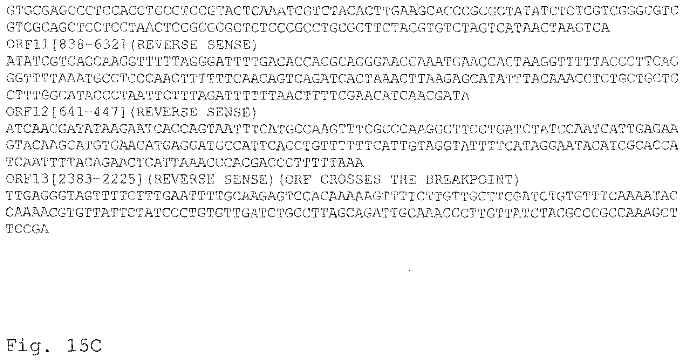

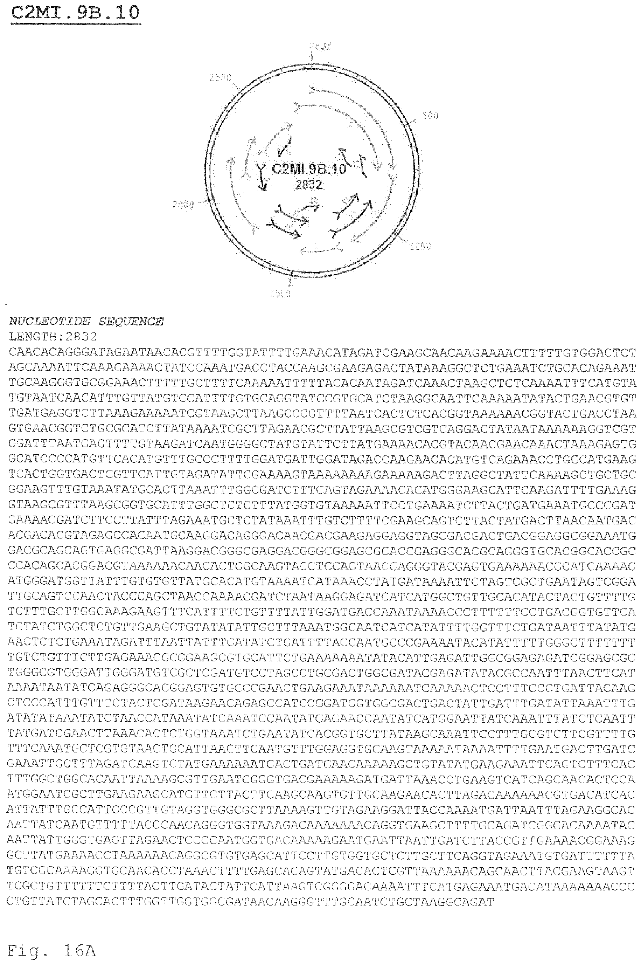

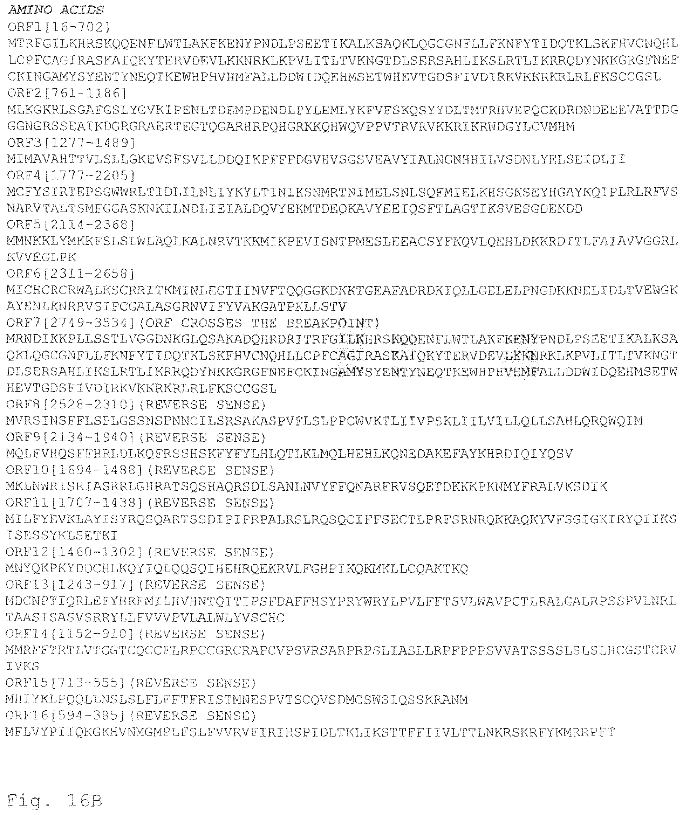

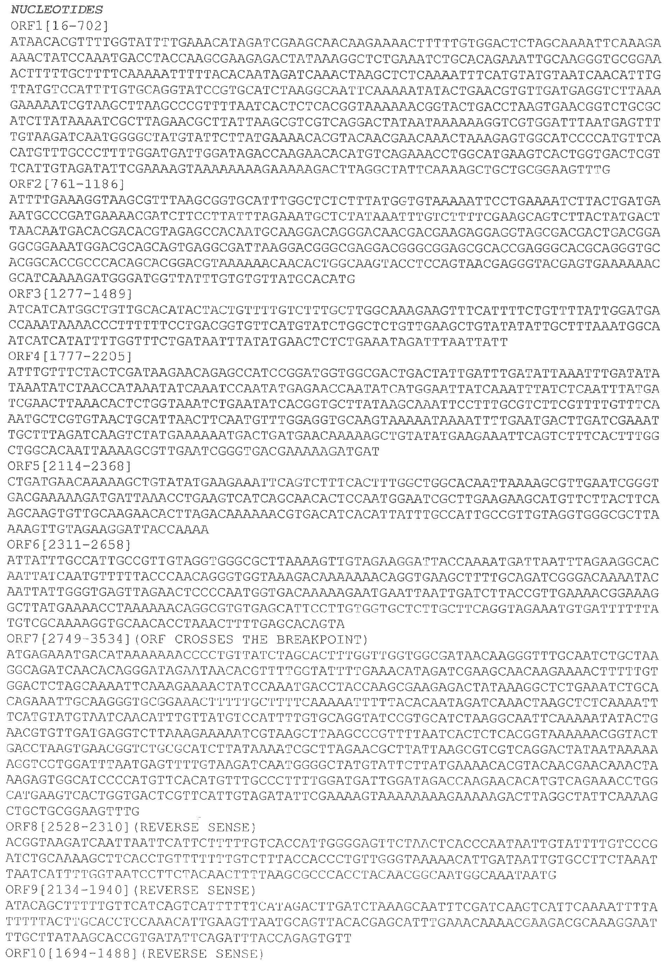

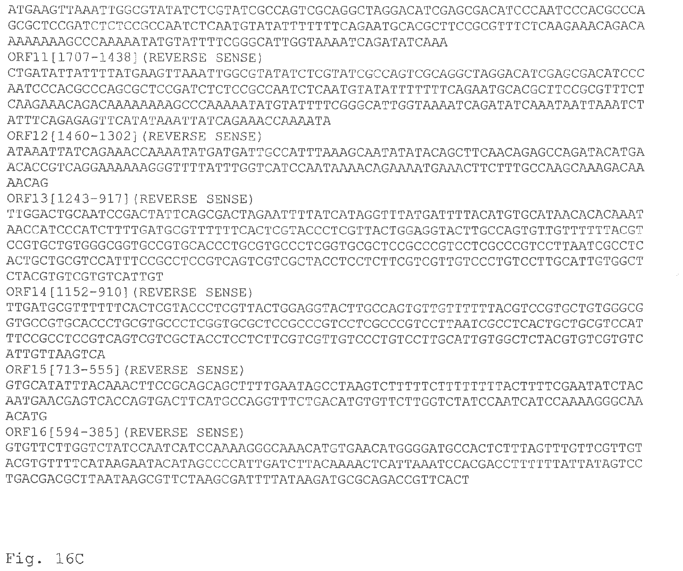

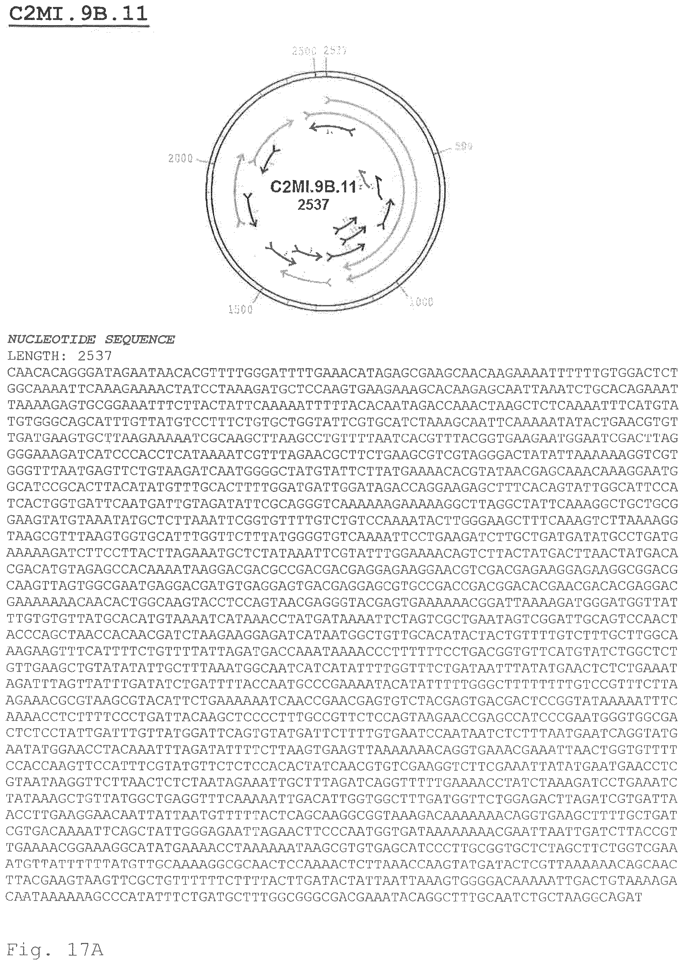

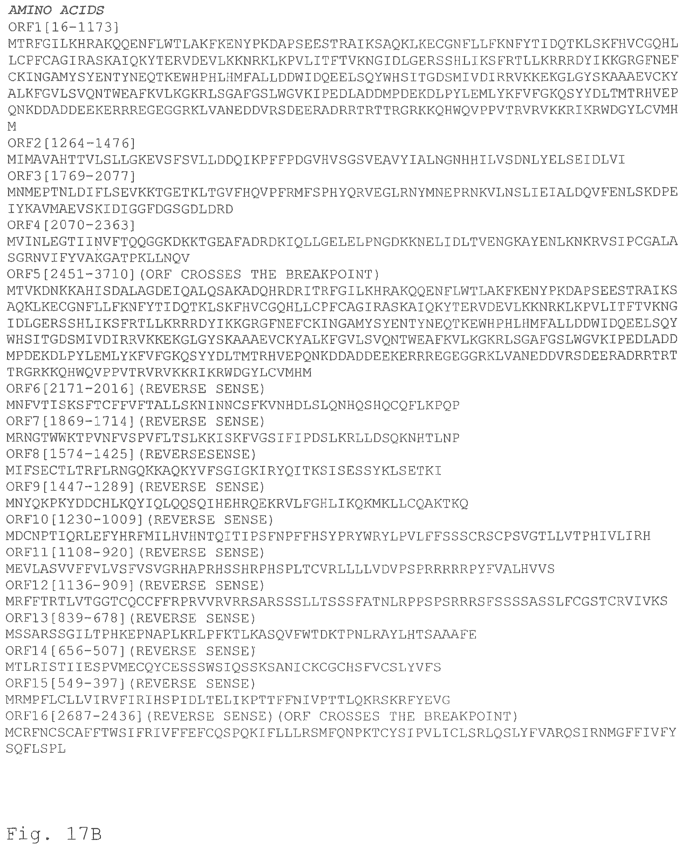

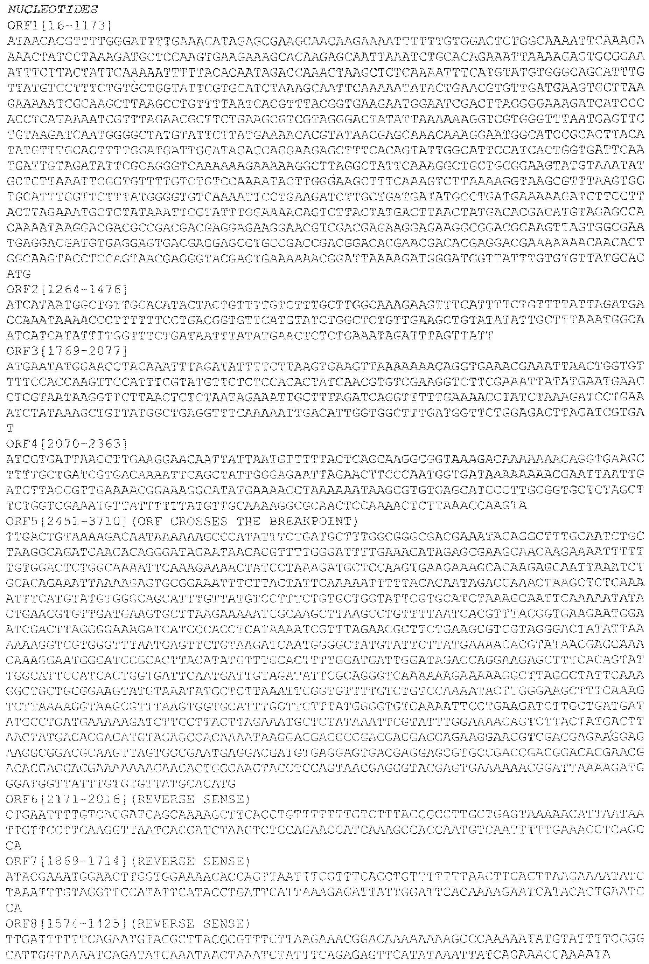

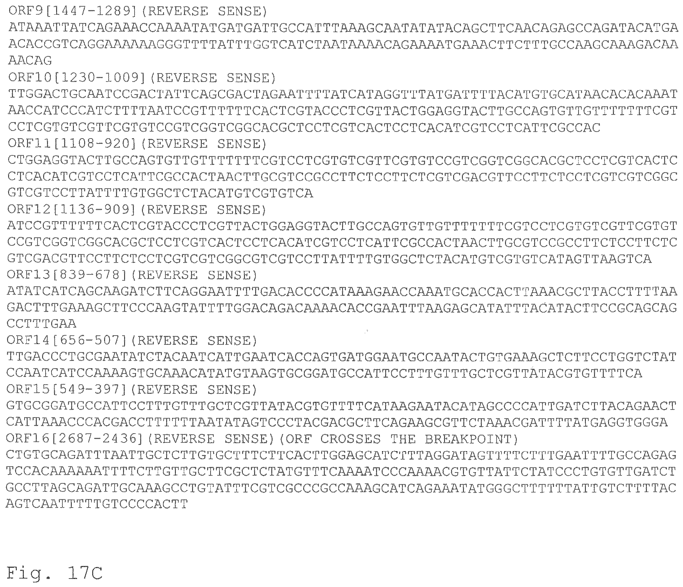

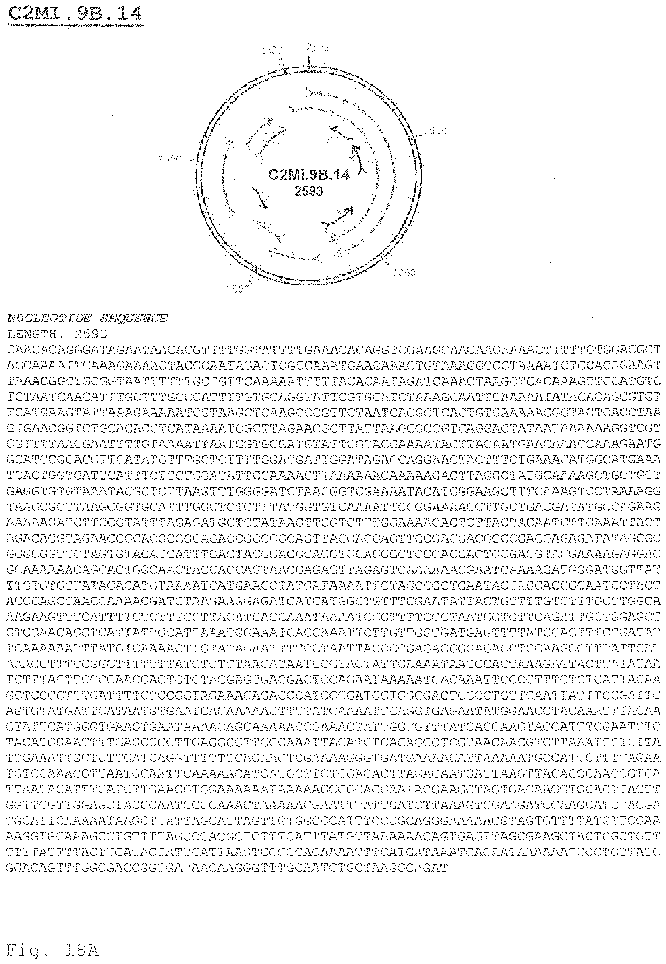

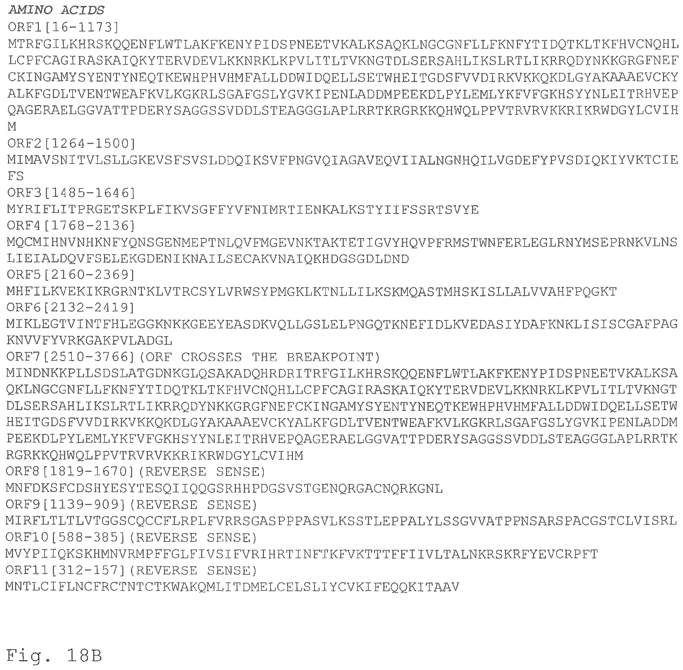

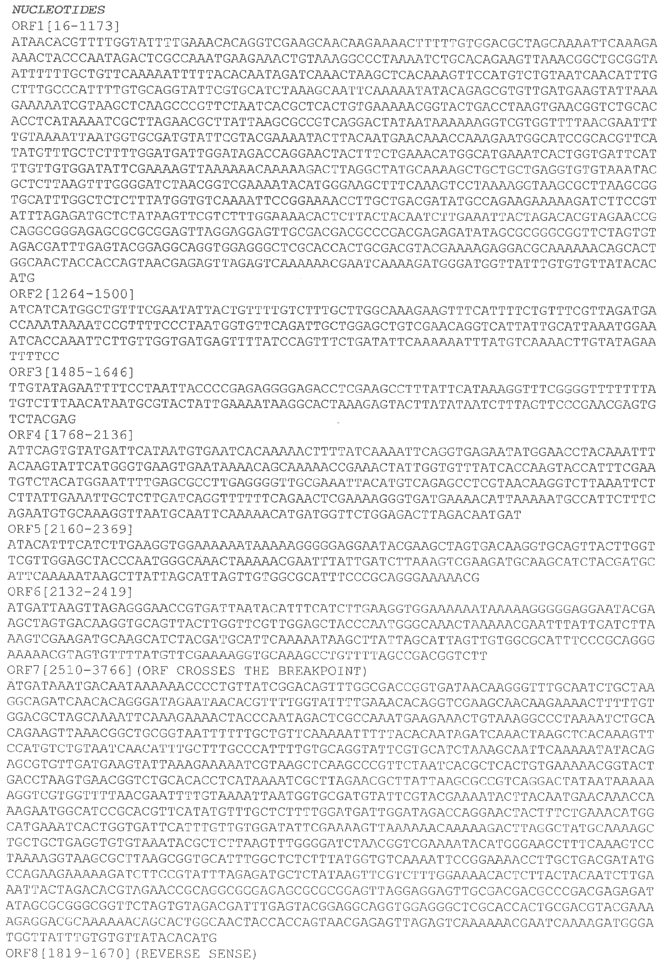

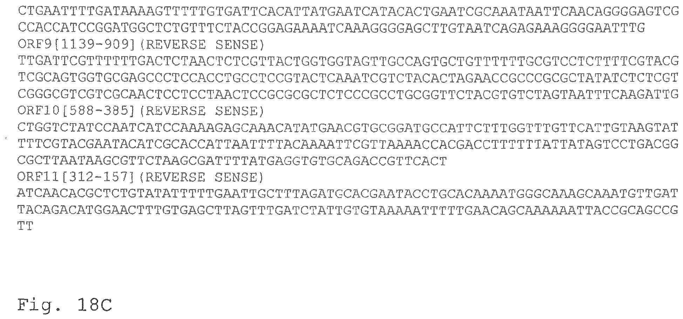

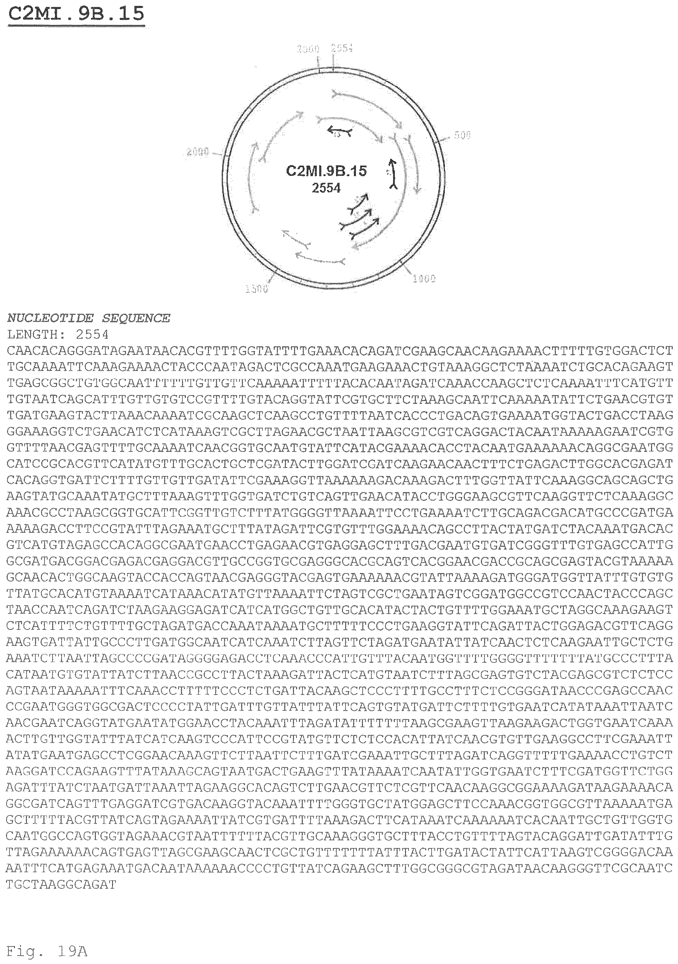

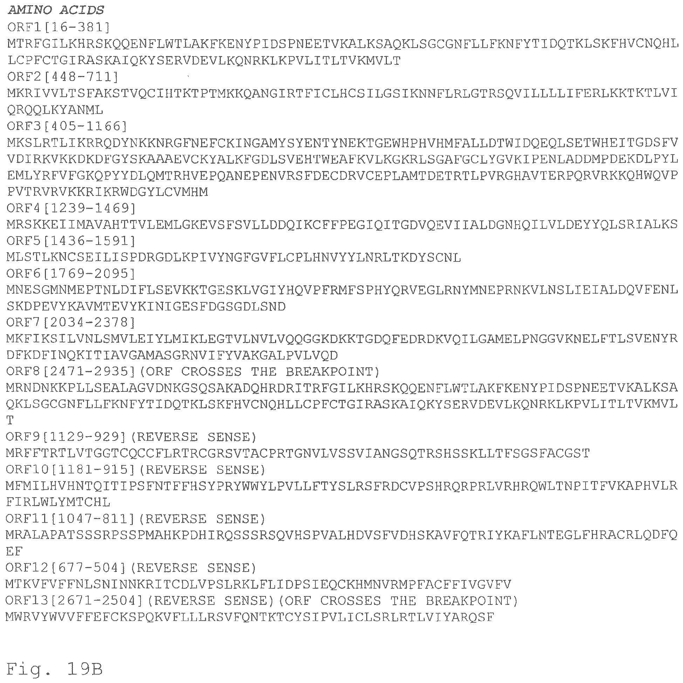

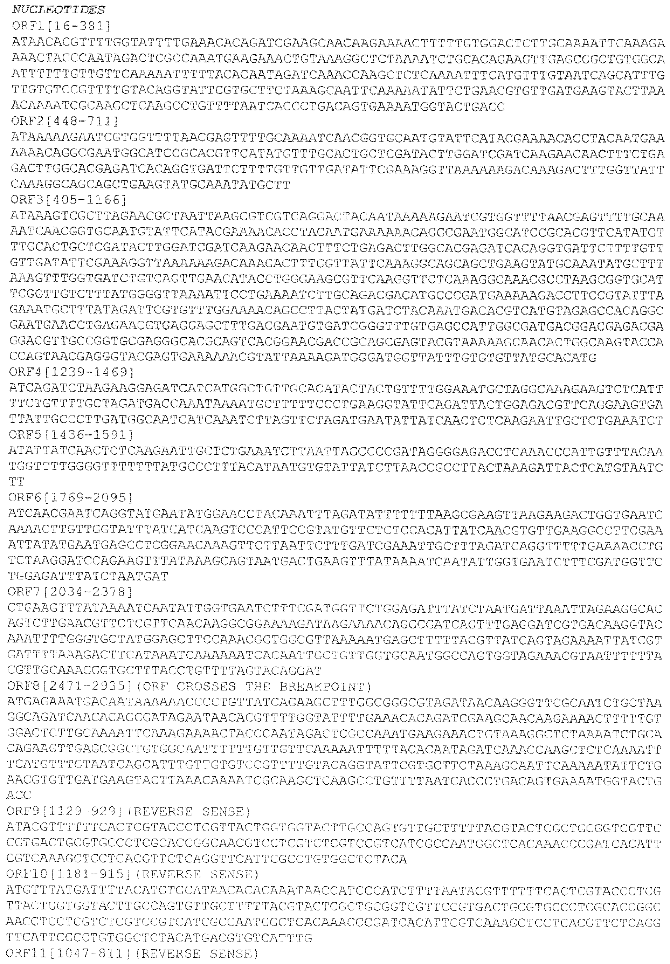

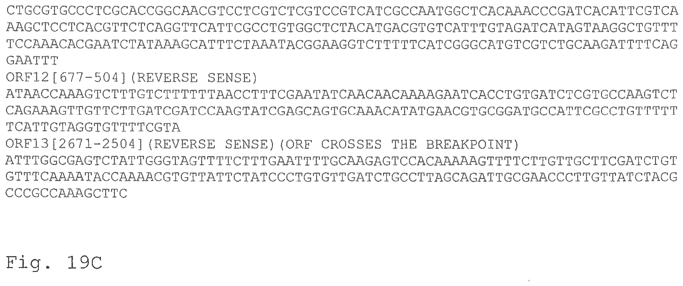

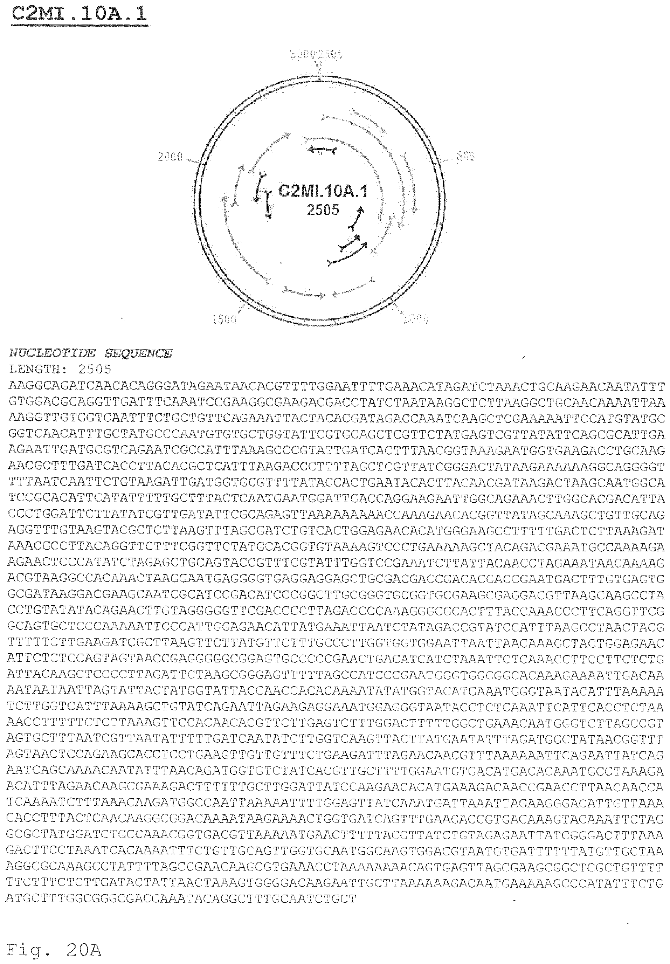

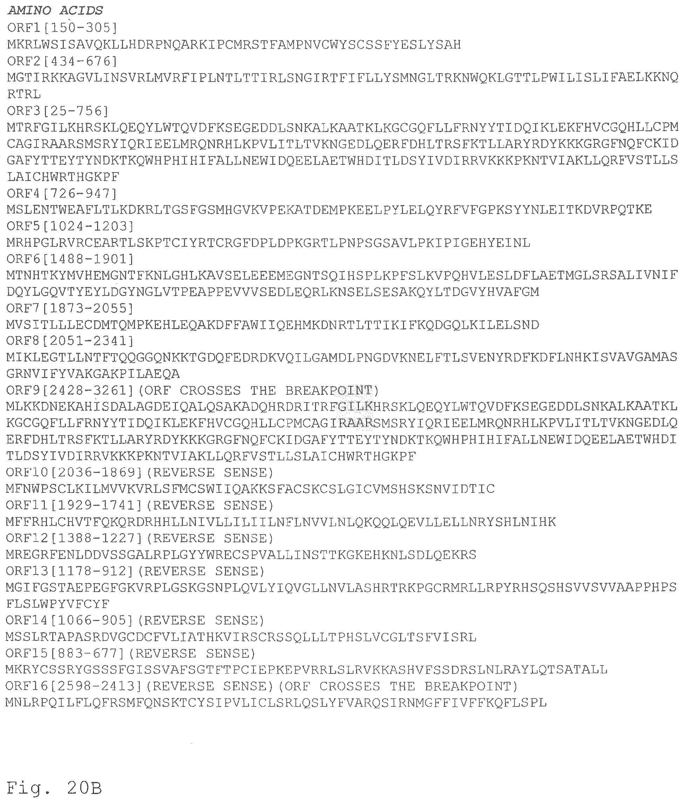

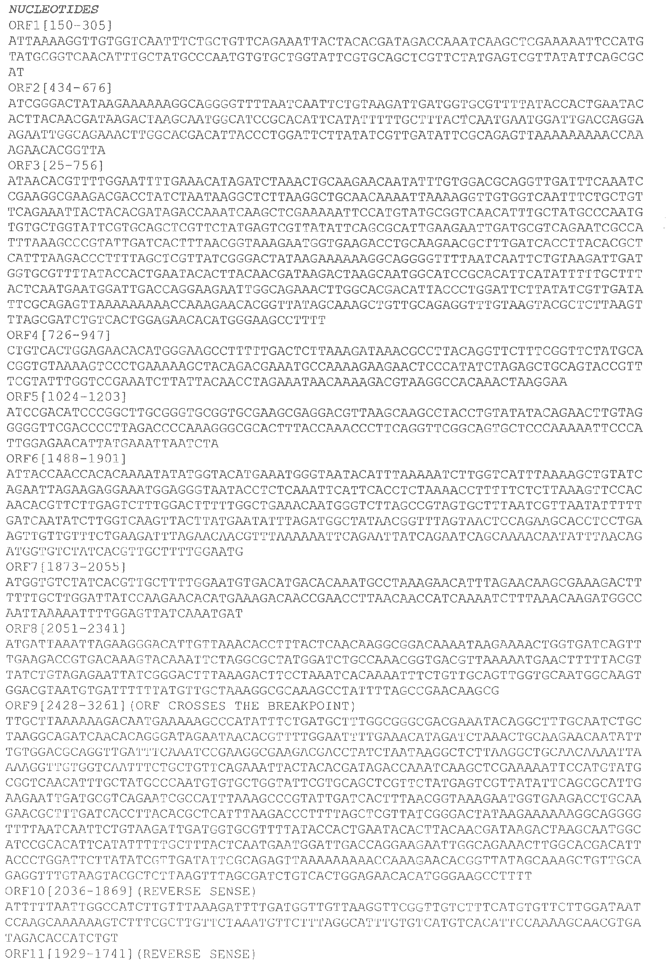

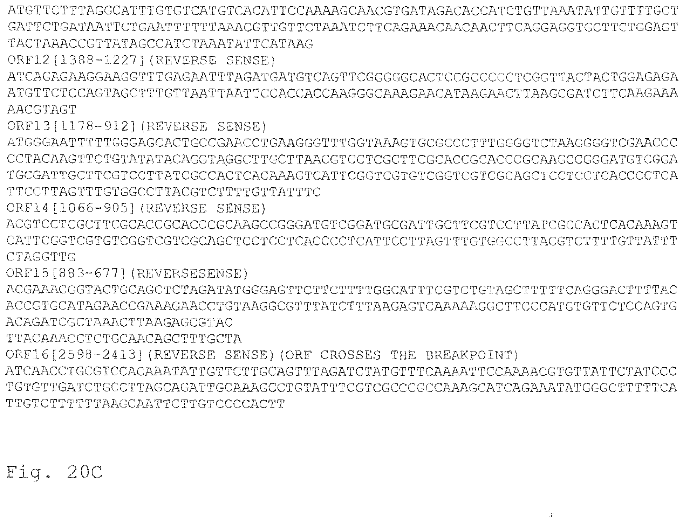

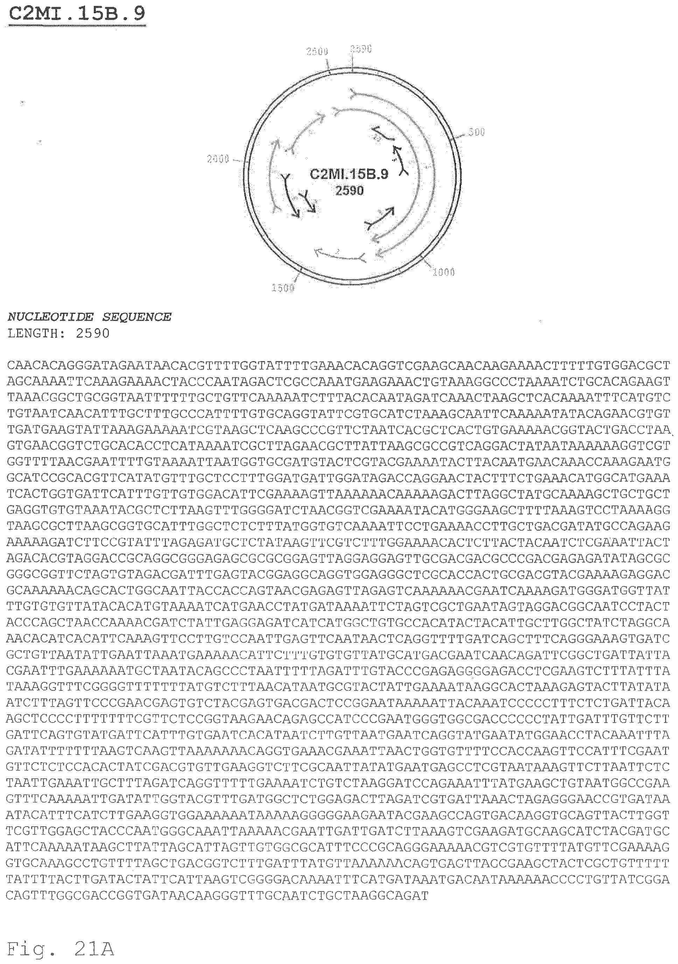

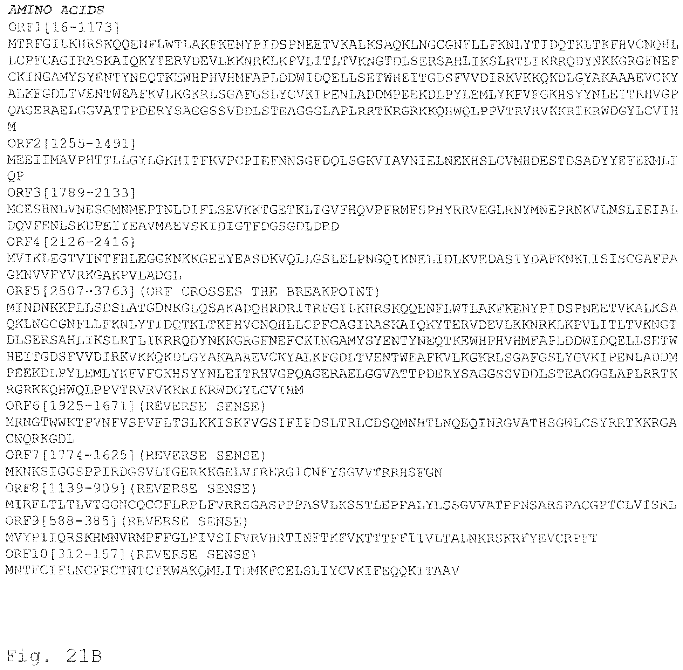

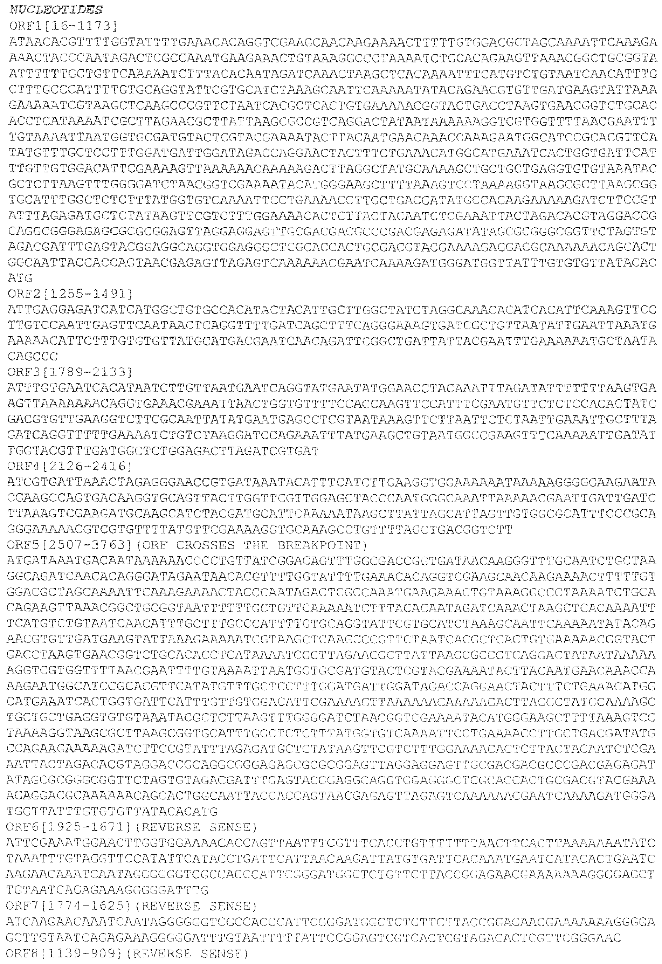

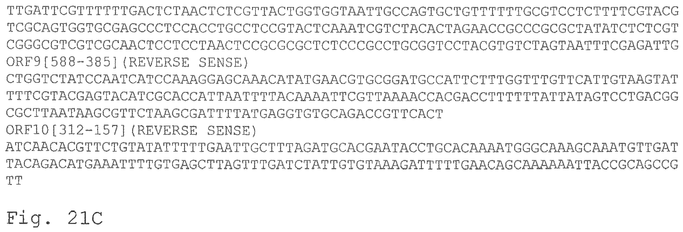

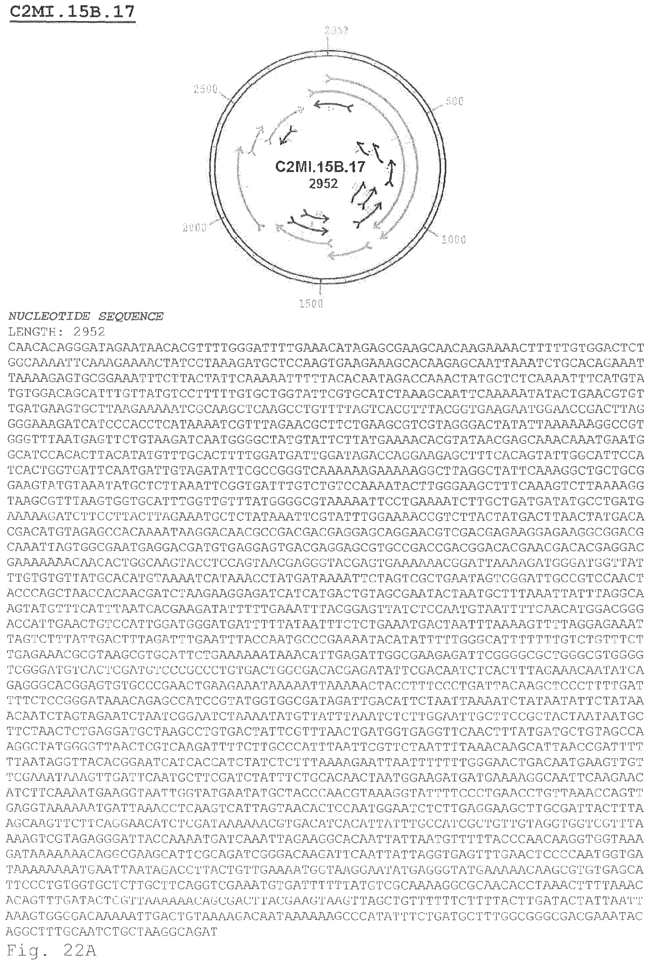

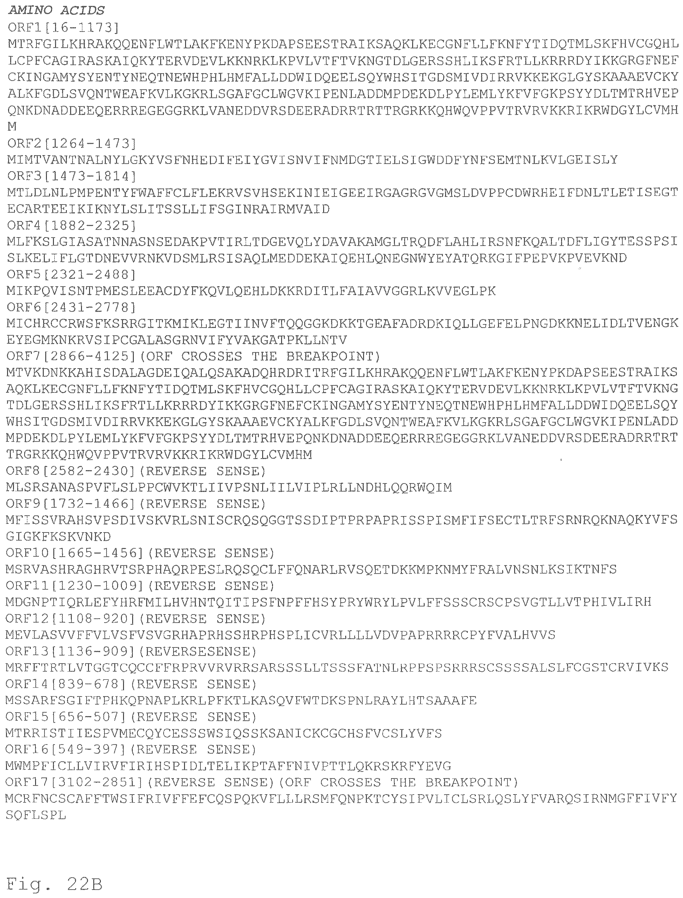

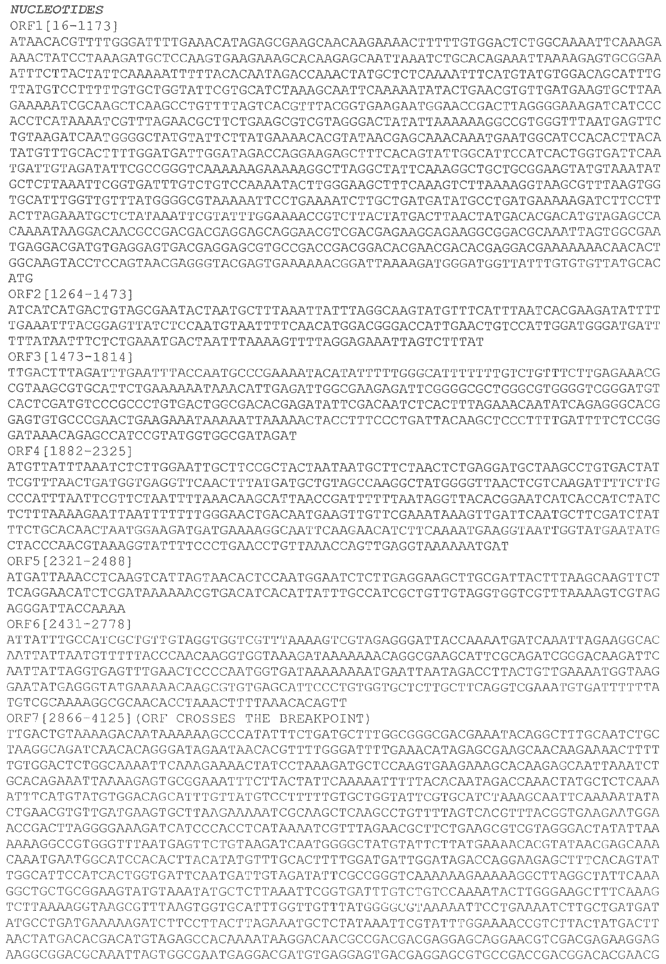

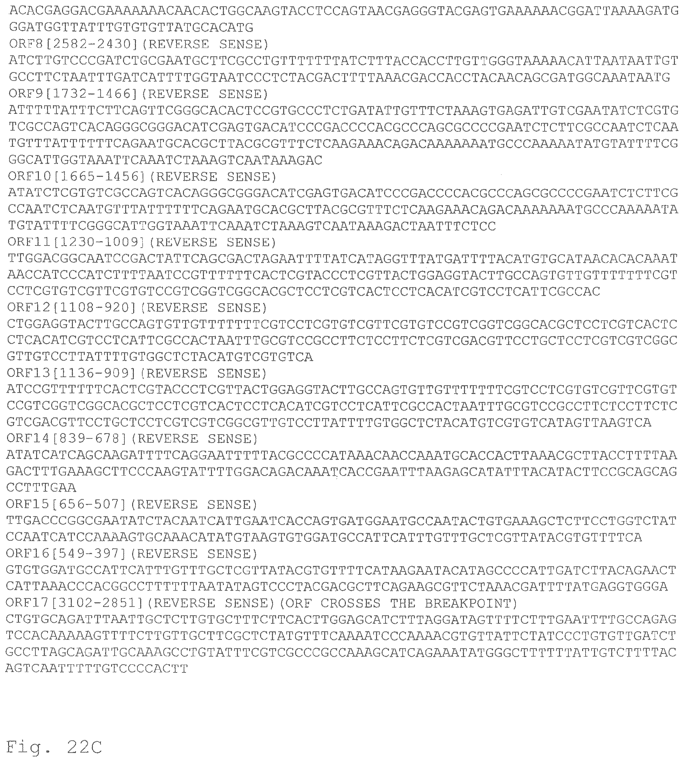

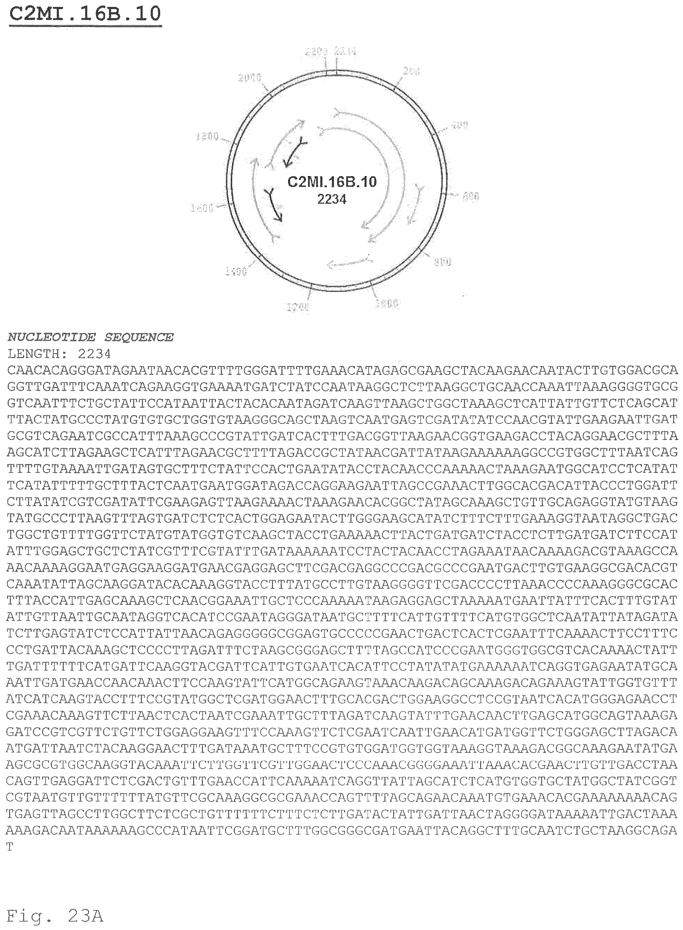

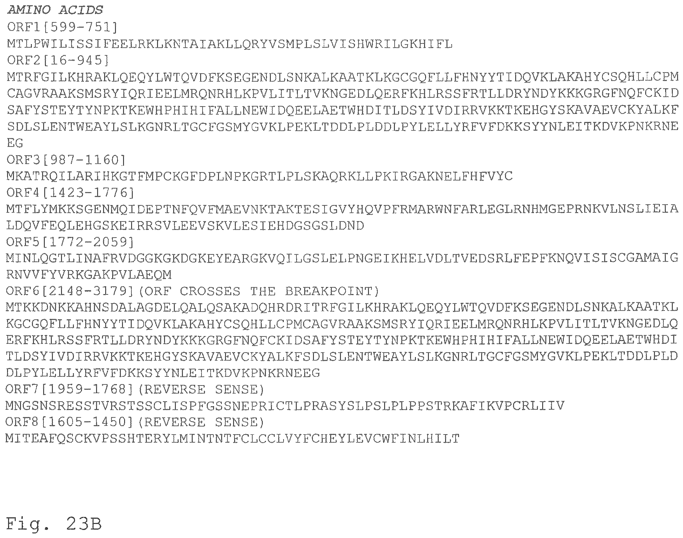

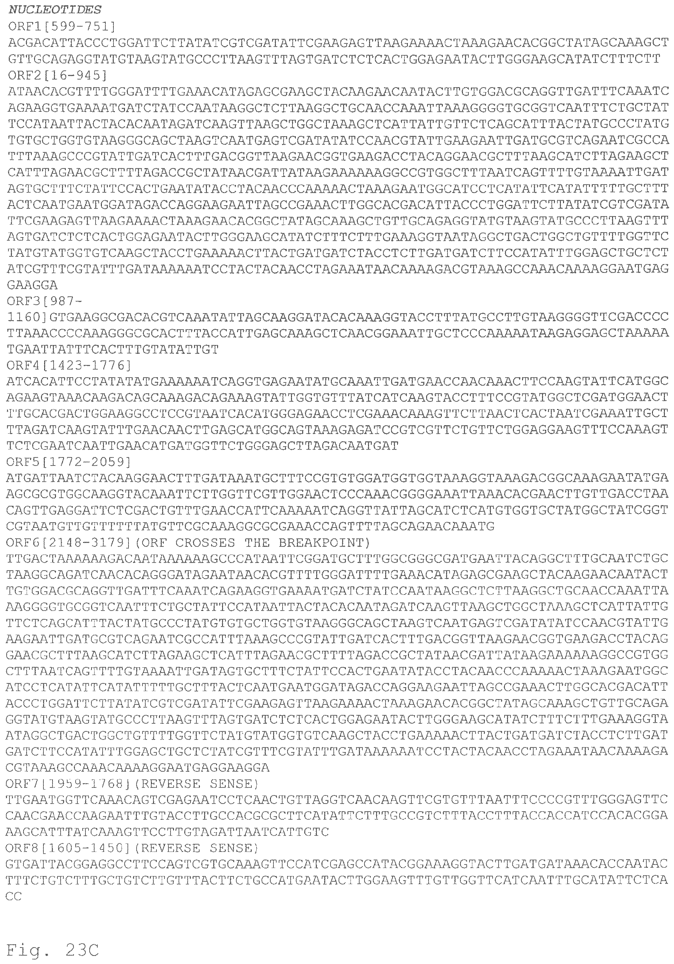

Rep = replication protein;
CMI:cattle milk isolate
   C2MI: cattle milk isolate belonging to the BMMF2 group
   HCBI: healthy cattle blood isolate
   Sphinx: slow progressive hidden infection of variable (X) latency
Figures 1 - 23: Nucleotide and amino acid sequences of
   - C2MI.1A.2 (Fig. 1)
   - C2MI.3A.2 (Fig. 2)
   - C2MI.4A.3 (Fig. 3)
   - C2MI.5A.4 (Fig. 4)
   - C2MI.5A.6 (Fig. 5)
   - C2MI.5B.13 (Fig. 6)
   - C2MI.7A.5 (Fig. 7)
   - C2MI.7B.8 (Fig. 8)
   - C2MI.7B.17 (Fig. 9)
   - C2MI.8B.4 (Fig. 10)
   - C2MI.8B.6 (Fig. 11)
   - C2MI.8B.7 (Fig. 12)
   - C2MI.9A.1 (Fig. 13)
   - C2MI.9A.3 (Fig. 14)
   - C2MI.9B.4 (Fig. 15)
   - C2MI.9B.10 (Fig. 16)
   - C2MI.9B.11 (Fig. 17)
   - C2MI.9B.14 (Fig. 18)
   - C2MI.9B.15 (Fig. 19)
   - C2MI.10A.1 (Fig. 20)
   - C2MI.15B.9 (Fig. 21)
   - C2MI.15B.17 (Fig. 22)
   - C2MI.16B.10 (Fig. 23)

### Detailed description of the present invention

In the present application a new concept for the pathogenesis of diseases of the CNS (e.g. Multiple sclerosis MS, amyotrophic lateral sclerosis, transmissible spongiforme encephalopathies/ Prion-linked diseases, Parkinson's disease, Alzheimer disease), diabetes, atherosclerosis and colon, breast and prostate cancer is presented.

For diseases of the CNS the interaction of an amplifying virus and the amplified DNA of a Bovine Meat and Milk Factor (BMMF) plays a role for their development (zur Hausen et al., 2017)

Two risk factors seem to deserve special attention: the relatively consistent results pointing to a possible role of different, predominantly herpes-group viruses, and the consumption of fresh cow milk, potentially including other dairy products. In addition, Vitamin D deficiency plays a role as a risk factor.

### - Viral Risk Factors

Apparently all Herpes virus types share two properties which seem to be relevant for the subsequent discussion:
During their persistence in a latent stage, spontaneous reactivation may occur, in part regulated by specific gene functions, partly also by epigenetic mechanisms. Reactivation may also be triggered by interaction with extracellular cytokines, such as transforming growth factor β.

Spontaneous induction of a lytic cycle has been observed for virtually every human pathogenic Herpes virus type. The high antibody titers against Epstein-Barr virus structural proteins in EBV-positive Burkitt's lymphomas and nasopharyngeal cancers may serve here as one example. Reactivations of human Herpes virus type 6, Varicella-Zoster virus, Herpes simplex virus and others are not rare events and may affect a number of different cell types.

A second remarkable property of herpes virus infection represents the amplification of various double- or single-stranded small DNA virus genomes upon infection of cells containing such DNAs in a latent state. This has been noted for human and monkey Polyoma viruses, JC and SV40, for human and bovine Papilloma viruses, as well as for single-stranded Adeno-associated (AAV) and Anello-/TT-viruses.

Spontaneous induction of Herpes-group viruses and the amplification of latent small viral DNA form the basis for the subsequent postulation of the mechanism underlying diseases of the CNS.

### - Bovine meat and milk factor (BMMF)

A correlation between consumption of non-pasteurized cow milk and MS development has been observed, in particular when non-pasteurized cow milk was consumed in the early phase of life (zur Hausen, 2017; zur Hausen, 2018).

### - Vitamin-D deficiency

A role of vitamin-D deficiency has repeatedly been implicated for the initiation of MS.

A convincing relationship between vitamin D deficiency and Epstein Barr Virus reactivation originates from early studies on EBV reactivation by transforming growth factor beta (TGF-β). A serum factor, purified and labeled as Epstein-Barr virus-inducing factor (EIF) have been reported to be identical to the subsequently described TGF-β molecule. TGF-β in turn is negatively regulated by activated vitamin D receptors. This could very well explain the season-related preferential onset of MS and of exacerbations.

Thus, at least two factors have been implicated as potential etiological contributors for both diseases of the CNS (e.g. MS) and cancer (e.g. colon and breast cancer): vitamin D deficiency and the reactivation of various herpes group viruses, mainly Epstein-Barr virus (EBV), human herpes virus type 6, and varicella-zoster virus.

Thus, it is considered by the, inventors that diseases of the CNS, in particular Multiple Sclerosis (MS), Parkinson, ALS or Alzheimer, result from
- Latent infections of the same cell with two different infectious agents, one of them most likely a herpes-type virus(in particular EBV, HHV-6, VZV, but also HSV, HHV-7), the other one acquired by bovine milk consumption (bovine milk factor - BMF) as the first event. Each of them latently infects individual cells, but occasionally genomes of both agents occur within the same cell.
- Reactivation of the herpes-type virus most frequently, but not only, Epstein Barr virus (EBV) to a lytic cycle as a second precondition. For EBV this is probably linked to increased levels of transforming growth factor β (TGF β) which is negatively regulated by activated vitamin D receptors;
- As third event, amplification of BMMF, resulting in partial suppression of Herpes-type DNA synthesis and formation of BMF particles or spreading of its nucleic acid to neighbouring cells via neuronal interconnections;
- This is followed by an infection of neighbouring cells with expression of BMMF protein;
- Finally, T-cell response against BMMF leads to the destruction of affected cells and in case of MS to plaque formation. This supports the clinical observation of the focal appearance of lesions, commonly starting from a central vein and the intensive localized immune response in early lesions.

The risk for insulin-dependent diabetes mellitus or atherosclerosis has been repeatedly also linked to cow milk consumption and vitamin D deficiency. The latter system seems to come particularly close to the MS situation.

This concept also may apply to other presumably autoimmune diseases and certain cancers occurring at increased frequency under conditions of vitamin D deficiency. As far as cancers are concerned, this specifically accounts for colon- and breast cancer, and possibly for ovarian, prostate, pancreatic cancer and lung cancers.

As regards colon cancer the inventors found that the uptake of BMMF (Bovine Meat and Milk Factor) agents within the first months of life either by substitution of breast-feeding during weaning by cow milk products or by the uptake of cow milk products, in general, leads to the early infection of newborns with BMMF antigens in the colon. Based on the decline of maternal antibodies and the frequently observed weakness of the immune system often coupled with induction of immunotolerances of the newborn during this very early period of life, these agents might either directly escape colonic immune response or a situation of immunotolerance against these agents might be induced. Within the next years or decades - depending on the immune system of the host - more and more BMMF antigens accumulate within the mesenchymal region of the lamina propria. This accumulation may be triggered also by the uptake of specific molecules that may represent receptors for BMMFs. These molecules are also taken up by consumption of cow products and are metabolized into receptors on the surface of the host cells. When a certain level of antigen is reached by continuous uptake of BMMFs in combination with focal spreading of infection, the host immune response induces a state of chronic and local inflammation producing a stable increase of reactive oxygen species (ROS) and cyclooxygenase-2 (Cox-2) which dramatically increases the probability deregulated cell proliferation with concomitant fixation of random mutations in surrounding cells induced by ROS.

Available results based on Ki67 (Ki67: proliferation marker) IHC staining show that especially the basal epithelial cells derived from epithelial stem cells located close to the endpoint of the crypts and migrating towards the distal luminal part of the crypts feature an intrinsically high proliferation enabling stochastic manifestation of mutations as a basic requirement for tumorgenesis and development of colon cancer. Thus, BMMFs represent a specific and local trigger for induction of chronic inflammation within mesenchymal tissue leading to an increase of ROS inducing proliferation and mutation in surrounding epithelial cells eventually leading to the formation of polyps as source for colon cancer.

Accordingly, the present invention relates to a C2MI polynucleic acid comprising:
(a) a nucleotide sequence depicted in any one of Figures 1(A) to 23(A);
(b) a nucleotide sequence having at least 90% identity to a nucleotide sequence of (a);
(c) a fragment of a nucleotide sequence of (a) or (b);
(d) a nucleotide sequence being complementary to a nucleotide sequence of (a), (b) or (c); or
(e) a nucleotide sequence which is redundant as a result of the degeneracy of the genetic code compared to any of the above-given nucleotide sequences.

The term "polynucleic acid" refers to a single-stranded or double-stranded nucleic acid sequence. A polynucleic acid may consist of deoxyribonucleotides or ribonucleotides, nucleotide analogues or modified nucleotides or may have been adapted for diagnostic or therapeutic purposes. A polynucleic acid may also comprise a double stranded cDNA clone which can be used, for example, for cloning purposes.

The C2MI polynucleic acids of the invention can be prepared according to well-known routine methods, for example, by (a) isolating the entire DNA or RNA from a sample, (b) detecting the CMI sequence by hybridization or PCR and (c) cloning of the C2MI sequence into a vector.

Also included within the present invention are sequence variants and recombinants of the polynucleic acid of the invention containing either deletions and/or insertions of one or more nucleotides, especially insertions or deletions of one or more codons, mainly at the terminal ends of oligonucleotides (either 3' or 5') but also within and which show at least 90%, 91%, 92%, 93%, 94%, 95%, 96%, 97%, 98% or 99% identity as well as recombination to said polynucleic acid sequences of the invention. Polynucleic acid sequences according to the present invention which are similar to the sequences as shown in Figures 1 to 23 can be characterized and isolated according to any of the techniques known in the art, such as amplification by means of sequence-specific primers, hybridization with sequence-specific probes under more or less stringent conditions, sequence determination of the genetic information of C2MI.

The present invention also provides functional fragments of the nucleotide sequences of the present invention described above that harbour a replication gene which codes for a replication protein. An autonomous replicating nucleotide sequence comprises a nucleotide sequence of the replication gene or a fragment thereof which is capable of inducing autonomous replication. Replication protein represents an endonuclease which binds single-stranded DNA inducing a single-stranded cut at or near the origin of replication (Wolds, 1997). The skilled person can derive at such fragments capable of inducing autonomous replication without undue experimentation. Such fragments may have a length of at least 45, at least 55, or at least 65 nt.

The person skilled in the art can easily determine by using standard assays which nucleic acid sequences are related to a nucleotide sequence of Figures 1 to 23 and have still the same function as the full length sequences.

The present invention also provides polynucleic acid sequences which are redundant as a result of the degeneracy of the genetic code compared to any of the above-given nucleotide sequences. These variant polynucleic acid sequences will thus encode the same amino acid sequence as the polynucleic acids they are derived from.

The C2MI polynucleic acids of the invention might be present as an extrachromosomal episome, might be integrated into the host's genome and/or might be linked to a host cell DNA.

The present invention also relates to an oligonucleotide primer comprising or consisting of part of a polynucleic acid as defined above, with said primer being able to act as primer for sequencing or specifically amplifying C2MI polynucleic acid of the invention.

The term "primer" refers to a single stranded DNA oligonucleotide sequence capable of acting as a point of initiation for synthesis of a primer extension product which is complementary to the nucleic acid strand to be copied. The length and the sequence of the primer must be such that they allow priming the synthesis of the extension products. Preferably the primer is about 5-50 nucleotides. Specific length and sequence will depend on the complexity of the required DNA or RNA targets, as well as on the conditions of primer use, such as temperature and ionic strength.

The fact that amplification primers do not have to match exactly with a corresponding template sequence to warrant proper amplification is an accepted principle. The amplification method used can be, for example, polymerase chain reaction (PCR), ligase chain reaction (LCR), nucleic acid sequence-based amplification (NASBA), transcription-based amplification system (TAS), strand displacement amplification (SDA) or amplification by means of Qb replicase or any other suitable method to amplify nucleic acid molecules using primer extension. During amplification, the amplified products can be labelled either using labelled primers or by incorporating labelled nucleotides. In a particular preferred embodiment the C2MI sequences of the present invention have been obtained by using two sets of abutting PCR primers overlapping in the highly conserved region of their Rep genes to amplify full-length circular DNA molecules. All C2MI isolates of the present invention were isolated by using a back-to-back primer pair A or B (5p->3p).

The "A" or "B" in the sequence name denotes these two primer pairs.
>LSconAfwd
   aaggcagatcaacacagg
>LSconArev
   agcagattgcaaagcctg
>LSconBfwd
   caacacagggatagaataac
>LSconBrev
   atctgccttagcagattgc

Labels may be isotopic (32P, 35S, etc.) or non-isotopic (biotin, digoxigenin, etc.). The amplification reaction is repeated between 20 and 70 times, advantageously between 25 and 45 times.

Any of a variety of sequencing reactions known in the art can be used to directly sequence the genetic information and determine the ORF by translating the sequence of the sample into the corresponding amino acid sequence. Exemplary sequencing reactions include those based on techniques developed by Sanger or Maxam and Gilbert. It is also contemplated that a variety of automated sequencing procedures may be utilized when performing the subject assays including sequencing by mass spectrometry (see, for example: PCT publication WO 94/16101). It will be evident to one skilled in the art that, for example the occurrence of only two or three nucleic bases needs to be determined in the sequencing reaction.

Preferably, these primers are about 5 to 50 nucleotides long, more preferably from about 10 to 25 nucleotides. Most preferred are primers having a length of at least 13 bases.

The present invention also relates to an oligonucleotide probe comprising or consisting of part of a C2MI polynucleic acid as defined above, with said probe being able to act as a hybridization probe for specific detection of a C2MI polynucleic acid according to the invention.

The probe can be labelled or attached to a solid support.

The term "probe" refers to single stranded sequence-specific oligonucleotides which have a sequence which is complementary to the target sequence of a C2MI polynucleic acid to be detected.

Preferably, these probes are about 5 to 50 nucleotides long, more preferably from about 10 to 25 nucleotides. Most preferred are probes having a length of at least 13 bases.

The term "solid support" can refer to any substrate to which an oligonucleotide probe can be coupled, provided that it retains its hybridization characteristics and provided that the background level of hybridization remains low. Usually the solid substrate will be a microtiter plate, a membrane (e.g. nylon or nitrocellulose) or a microsphere (bead). Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, NH₂ groups, SH groups, carboxylic groups, or coupling with biotin or haptens.

The oligonucleotides according to the present invention, used as primers or probes may also contain or consist of nucleotide analogues such as phosphorothioates, alkylphosphoriates or peptide nucleic acids or may contain intercalating agents. These modifications will necessitate adaptions with respect to the conditions under which the oligonucleotide should be used to obtain the required specificity and sensitivity. However, the eventual results will be essentially the same as those obtained with the unmodified oligonucleotides.

The introduction of these modifications may be advantageous in order to positively influence characteristics such as hybridization kinetics, reversibility of the hybrid-formation, biological stability of the oligonucleotide molecules, etc.

The polynucleic acids of the invention may be comprised in a composition of any kind. Said composition may be for diagnostic, therapeutic or prophylactic use.

The present invention also relates to a recombinant expression vector comprising a C2MI polynucleic acid of the invention as defined above operably linked to prokaryotic, eukaryotic or viral transcription and translation control elements as well as host cells containing such vector.

The term "vector" may comprise a plasmid, a cosmid, an artificial chromosome, a phage, or a virus or a transgenic non-human animal. Examples are adenovirus, adeno-associated virus, herpes simplex virus, vaccinia, or, an RNA virus such as a retrovirus.

The term "recombinant expression" used within the context of the present invention refers to the fact that the polypeptides of the present invention are produced by recombinant expression methods be it in prokaryotes, or lower or higher eukaryotes as discussed in detail below.

The term "host cell" refers to cells which can be or have been, used as recipients for a recombinant vector or other transfer polynucleotide, and include the progeny of the original cell which has been transfected.

It is understood that the progeny of a single parental cell may not necessarily be completely identical in morphology or in genomic or total DNA complement as the original parent, due to natural, accidental, or deliberate mutation or recombination.

The term "lower eukaryote" refers to host cells such as yeast, fungi and the like. Lower eukaryotes are generally (but not necessarily) unicellular. Preferred lower eukaryotes are yeasts, particularly species within Saccharomyces, Schizosaccharomyces, Kluiveromyces, Pichia (e. g. Pichia pastoris), Hansenula (e. g. Hansenula polymorph), Schwaniomyces, Schizosaccharomyces, Yarowia, Zygosaccharomyces and the like. Saccharomyces cerevisiae, S. carlsbergensis and K. lactis are the most commonly used yeast hosts, and are convenient fungal hosts.

The term "higher eukaryote" refers to host cells derived from higher animals, such as mammals, reptiles, insects, and the like. Presently preferred higher eukaryote host cells are derived from Chinese hamster (e. g. CHO), monkey (e. g. COS and Vero cells), baby hamster kidney (BHK), pig kidney (PK15), rabbit kidney 13 cells (RK13), the human osteosarcoma cell line 143 B, the human cell line HeLa and human hepatoma cell lines like Hep G2, the 293TT cell line (Buck et al., 2004) and insectcell lines (e.g. Spodoptera frugiperda). The host cells may be provided in suspension or flask cultures, tissue cultures, organ cultures and the like. Alternatively the host cells may also be transgenic non-human animals.

The term "prokaryotes" refers to hosts such as E. coli, Lactobacillus, Lactococcus, Salmonella, Streptococcus, Bacillus subtilis, Acinetobacter or Streptomyces. Also these hosts are contemplated within the present invention.

The segment of the C2MI DNA encoding the desired sequence inserted into the vector sequence may be attached to a signal sequence. Said signal sequence may be that from a non-C2MI source, but particularly preferred constructs according to the present invention contain signal sequences appearing in the C2MI genome before the respective start points of the proteins.

Higher eukaryotes may be transformed with vectors, or may be infected with a recombinant virus, for example a recombinant vaccinia virus. Techniques and vectors for the insertion of foreign DNA into vaccinia virus are well known in the art, and utilize, for example homologous recombination. A wide variety of viral promoter sequences, possibly terminator sequences and poly(A)-addition sequences, possibly enhancer sequences and possibly amplification sequences, all required for the mammalian expression, are available in the art. Vaccinia is particularly preferred since vaccinia halts the expression of host cell proteins. For vaccination of humans the avipox and Ankara Modified Virus (AMV) are particularly useful vectors.

Also known are insect expression transfer vectors derived from baculovirus Autographa californica nuclear polyhedrosis virus (AcNPV), which is a helper-independent viral expression vector. Expression vectors derived from this system usually use the strong viral polyhedrin gene promoter to drive the expression of heterologous genes. Different vectors as well as methods for the introduction of heterologous DNA into the desired site of baculovirus are available to the person skilled in the art for baculovirus expression. Also different signals for posttranslational modification recognized by insect cells are known in the art.

The present invention also relates to a polypeptide having an amino acid sequence encoded by a C2MI polynucleic acid as defined above, or a part or an analogue thereof being substantially similar and biologically equivalent.

The term "polypeptide" refers to a polymer of amino acids and does not refer to a specific length of the product. Thus, peptides, oligopeptides, and proteins are included within the definition of polypeptide. This term also does not refer to or exclude post-expression modifications of the polypeptide, for example, glycosylations, acetylations, phosphorylations and the like. Included within the definition are, for example, polypeptides containing one or more analogues of an amino acid (including, for example, unnatural amino acids, peptide nucleic acid (PNA), etc.), polypeptides with substituted linkages, as well as other modifications known in the art, both naturally occurring and non-naturally occurring.

The polypeptides according to the present invention contain preferably at least 3, preferably 4 or 5 contiguous C2MI amino acids, 6 or 7 preferably however at least 8 contiguous C2MI amino acids, at least 10 or at least 15.

The polypeptides of the invention, and particularly the fragments, can be prepared by classical chemical synthesis. The synthesis can be carried out in homogeneous solution or in solid phase. The polypeptides according to this invention can also be prepared by means of recombinant DNA techniques. The present invention also relates to a method for production of a recombinant polypeptide as defined above, comprising: (a) transformation of an appropriate cellular host with a recombinant vector, in which a polynucleic acid or a part thereof as defined above has been inserted under the control of the appropriate regulatory elements, (b) culturing said transformed cellular host under conditions enabling the expression of said insert, and (c) harvesting said polypeptide.

The present invention also relates to an antibody raised upon immunization with at least one polypeptide as defined above, with said antibody being specifically reactive with any of said polypeptides, and with said antibody being preferably a monoclonal antibody. The term "antibody", preferably, relates to antibodies which consist essentially of pooled monoclonal antibodies with different epitopic specificities, as well as distinct monoclonal antibody preparations. Monoclonal antibodies are made from an antigen containing, e.g., a polypeptide encoded by a C2MI polynucleic acid of the invention or a fragment thereof by methods well known to those skilled in the art. As used herein, the term "antibody"(Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. Thus, these fragments are preferred, as well as the products of a Fab or other immunoglobulin expression library. Moreover, antibodies useful for the purposes of the present invention include chimerical, single chain, and humanized antibodies.

The present invention also relates to diagnostic and therapeutic approaches using cell-mediated immune responses.

Preferably, the antibody or antigen binding fragment thereof carries a detectable label. The antibody/fragment can be directly or indirectly detectably labeled, for example, with a radioisotope, a fluorescent compound, a bioluminescent compound, a chemiluminescent compound, a metal chelator or an enzyme. Those of ordinary skill in the art will know of other suitable labels for binding to the antibody, or will be able to ascertain such, using routine experimentation.

The present invention also relates to a diagnostic kit for use in determining the presence of a C2MI polynucleic acid or polypeptide of the invention, said kit comprising a primer, a probe, and/or an antibody of the invention. Said kit may have any format well known to the person skilled in the art, e.g. can be an ELISA-based kit.

The present invention also relates to a method for the detection of a C2MI polynucleic acid according to the invention present in a biological sample, comprising: (a) optionally extracting sample polynucleic acid, (b) amplifying the polynucleic acid as described above with at least one primer as defined above, optionally a labelled primer, and (c) detecting the amplified polynucleic acids.

The term "polynucleic acid" can also be referred to as analyte strand and corresponds to a single- or double-stranded polynucleic acid molecule.

The term "labelled" refers to the use of labelled nucleic acids. This may include the use of labelled nucleotides incorporated during the polymerase step of the amplification or labelled primers, or by any other method known to the person skilled in the art.

The present invention also relates to a method for the detection of a C2MI polynucleic acid according to the invention present in a biological sample, comprising: (a) optionally extracting sample polynucleic acid, (b) hybridizing the polynucleic acid as described above with at least one probe as defined above, and (c) detecting the hybridized polynucleic acids.

The hybridization and washing conditions are to be understood as stringent and are generally known in the art. However, according to the hybridization solution (SSC, SSPE, etc.), these probes should be hybridized at their appropriate temperature in order to attain sufficient specificity.

According to the hybridization solution (SSC, SSPE, etc.), these probes should be stringently hybridized at their appropriate temperature in order to attain sufficient specificity. However, by slightly modifying the DNA probes, either by adding or deleting one or a few nucleotides at their extremities (either 3' or 5'), or substituting some non-essential nucleotides (i.e. nucleotides not essential to discriminate between types) by others (including modified nucleotides or inosine) these probes or variants thereof can be caused to hybridize specifically at the same hybridization conditions (i.e. the same temperature and the same hybridization solution). Also changing the amount (concentration) of probe used may be beneficial to obtain more specific hybridization results. It should be noted in this context, that probes of the same length, regardless of their GC content, will hybridize specifically at approximately the same temperature in TMACI solutions.

Suitable assay methods for purposes of the present invention to detect hybrids formed between the oligonucleotide probes and the C2MI polynucleic acid sequences in a sample may comprise any of the assay formats known in the art, such as the conventional dot-blot format, sandwich hybridization or reverse hybridization. For example, the detection can be accomplished using a dot blot format, the unlabelled amplified sample being bound to a membrane, the membrane being incorporated with at least one labelled probe under suitable hybridization and wash conditions, and the presence of bound probe being monitored.

An alternative and preferred method is a "reverse" dot-blot format, in which the amplified sequence contains a label. In this format, the unlabelled oligonucleotide probes are bound to a solid support and exposed to the labelled sample under appropriate stringent hybridization and subsequent washing conditions. It is to be understood that also any other assay method which relies on the formation of a hybrid between the polynucleic acids of the sample and the oligonucleotide probes according to the present invention may be used.

The present invention also relates to a method for detecting a polypeptide encoded by a C2MI polynucleic acid of the present invention or an antibody against said polypeptide present in a biological sample, comprising: (a) contacting the biological sample for the presence of such polypeptide or antibody as defined above, and (b) detecting the immunological complex formed between said antibody and said polypeptide.

The immunoassay methods according to the present invention may utilize antigens from different domains of the new and unique polypeptide sequences of the present invention. It is within the scope of the invention to use for instance single or specific oligomeric antigens, dimeric antigens, as well as combinations of single or specific oligomeric antigens. The C2MI antigens of the present invention may be employed in virtually any assay format that employs a known antigen to detect antibodies or cell-mediated immune responses. Thus, the present invention also encompasses the detection of cell-mediated immune responses against C2MI antigens and the application of therapeutic interferences based on cell-mediated immune responses against C2MI antigens.

Of course, an assay format that denatures the C2MI conformational epitope should be avoided or adapted. A common feature of all of these assays is that: the antigen is contacted with the body component suspected of containing C2MI antibodies under conditions that permit the antigen to bind to any such antibody present in the component. Such conditions will typically be physiologic temperature, pH and ionic strength using an excess of antigen. The incubation of the antigen with the specimen is followed by detection of immune complexes comprised of the antigen.

Design of the immunoassays is subject to a great deal of variation, and many formats are known in the art. Protocols may, for example, use solid supports, or immunoprecipitation. Most assays involve the use of labeled antibody or polypeptide; the labels may be, for example, enzymatic, fluorescent, chemiluminescent, radioactive, or dye molecules. Assays which amplify the signals from the immune complex are also known; examples of which are assays which utilize biotin and avidin or streptavidin, and enzyme-labeled and mediated immunoassays, such as ELISA assays.

The immunoassay may be in a heterogeneous or in a homogeneous format, and of a standard or competitive type. In a heterogeneous format, the polypeptide is typically bound to a solid matrix or support to facilitate separation of the sample from the polypeptide after incubation. Examples of solid supports that can be used are nitrocellulose (e. g., in membrane or microtiter well form), polyvinyl chloride (e. g., in sheets or microtiter wells), polystyrene latex (e. g., in beads or microtiter plates, polyvinylidine fluoride (known as Immunolon), diazotized paper, nylon membranes, activated beads, and Protein A beads. The solid support containing the antigenic polypeptides is typically washed after separating it from the test sample, and prior to detection of bound antibodies. Both standard and competitive formats are known in the art.

In a homogeneous format, the test sample is incubated with the combination of antigens in solution. For example, it may be under conditions that will precipitate any antigen-antibody complexes which are formed. Both standard and competitive formats for these assays are known in the art.

In a standard format, the amount of C2MI antibodies in the antibody-antigen complexes is directly monitored. This may be accomplished by determining whether (labelled) anti-xenogeneic (e. g. anti-human) antibodies which recognize an epitope on anti-C2MI antibodies will bind due to complex formation. In a competitive format, the amount of C2MI antibodies in the sample is deduced by monitoring the competitive effect on the binding of a known amount of labelled antibody (or other competing ligand) in the complex.

Complexes formed comprising anti-C2MI antibody (or in the case of competitive assays, the amount of competing antibody) are detected by any of a number of known, depending on the format. For example, unlabeled C2MI antibodies in the complex may be detected using a conjugate of anti-xenogeneic Ig complexed with a label (e. g. an enzyme label).

In an immunoprecipitation or agglutination assay format the reaction between the C2MI antigens and the antibody forms a network that precipitates from the solution or suspension and forms a visible layer or film of precipitate. If no anti-C2MI antibody is present in the test specimen, no visible precipitate is formed.

There currently exist three specific types of particle agglutination (PA) assays. These assays are used for the detection of antibodies to various antigens when coated tο a support. One type of this assay is the hemagglutination assay using red blood cells (RBCs) that are sensitized by passively adsorbing antigen (or antibody) to the RBC. The addition of specific antigen/antibodies present in the body component, if any, causes the RBCs coated with the purified antigen to agglutinate.

To eliminate potential non-specific reactions in the hemagglutination assay, two artificial carriers may be used instead of RBC in the PA. The most common of these are latex particles.

The solid phase selected can include polymeric or glass beads, nitrocellulose, microparticles, microwells of a reaction tray, test tubes and magnetic beads. The signal generating compound can include an enzyme, a luminescent compound, a chromogen, a radioactive element and a chemiluminescent compound. Examples of enzymes include alkaline phosphatase, horseradish peroxidase and beta-galactosidase. Examples of enhancer compounds include biotin, anti-biotin and avidin. Examples of enhancer compounds binding members include biotin, anti-biotin and avidin.

The above methods are useful for evaluating the risk of developing diseases e.g. cancer or a disease of the CNS, due to the deleterious effects of the presence of a subgenomic C2MI polynucleotide sequence by itself or linked to a particular host gene or gene fragment within the patient's cells and allow taking appropriate counter measures.

Thus, the present invention also relates to an antisense oligonucleotide or iRNA specific for the C2MI virus polynucleic acid of the invention.

The generation of suitable antisense oligonucleotides or iRNAs includes determination of a site or sites within the C2MI polynucleic acid for the antisense interaction to occur such that the desired effect, e.g., inhibition of expression of the polypeptide, will result. A preferred intragenic site is (a) the region encompassing the translation initiation or termination codon of the open reading frame (ORF) of the gene or (b) a region of the mRNA which is a "loop" or "bulge", i.e., not part of a secondary structure. Once one or more target sites have been identified, oligonucleotides are chosen which are sufficiently complementary to the target, i.e., hybridize sufficiently well and with sufficient specificity, to give the desired effect. In the context of this invention, "hybridization" means hydrogen bonding, which may be Watson-Crick, Hoogsteen or reversed Hoogsteen hydrogen bonding, between complementary nucleoside or nucleotide bases. "Complementary" as used herein, refers to the capacity for precise pairing between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can hydrogen bond with each other. Thus, "specifically hybridizable" and "complementary" are terms which are used to indicate a sufficient degree of complementarity or precise pairing such that stable and specific binding occurs between the oligonucleotide and the DNA or RNA target. It is understood in the art that the sequence of an antisense compound does not need to be 100% complementary to that of its target nucleic acid to be specifically hybridizable. An antisense compound is specifically hybridizable when binding of the compound to the target DNA or RNA molecule interferes with the normal function of the target DNA or RNA to cause a loss of utility, and there is a sufficient degree of complementarity to avoid non-specific binding of the antisense compound to non-target sequences under conditions in which specific binding is desired, i.e., in the case of therapeutic treatment.

"Oligonucleotide" (in particular in the context of antisense compounds) refers to an oligomer or polymer of ribonucleic acid (RNA) or deoxyribonucleic acid (DNA) or mimetics thereof. This term includes oligonucleotides composed of naturally-occurring nucleobases, sugars and covalent internucleoside (backbone) linkages as well as oligonucleotides having non-naturally-occurring portions which function similarly. Such modified or substituted oligonucleotides are often preferred over native forms because of desirable properties such as, for example, enhanced cellular uptake, enhanced affinity for nucleic acid target and increased stability in the presence of nucleases. While antisense oligonucleotides are a preferred form of the antisense compound, the present invention comprehends other oligomeric antisense compounds, including but not limited to oligonucleotide mimetics such as are described below. The antisense compounds in accordance with this invention comprise from about 6 to about 50 nucleobases (i.e. from about 6 to about 50 linked nucleosides). Particularly preferred antisense compounds are antisense oligonucleotides, even more preferably those comprising from about 15 to about 25 nucleobases. Antisense compounds include ribozymes, external guide sequences (EGS), oligonucleotides (oligozymes), and other short catalytic RNAs or catalytic oligonucleotides which hybridize to the target nucleic acid and inhibit its expression. The antisense compounds also include an iRNA comprising a sense sequence and an antisense sequence, wherein the sense and antisense sequences form an RNA duplex and wherein the antisense sequence comprises a nucleotide sequence sufficiently complementary to the nucleotide sequence of a C2MI polynucleic acid of the present invention.

Alternatively, the invention provides a vector allowing to transcribe an antisense oligonucleotide of the invention, e.g., in a mammalian host. Preferably, such a vector is a vector useful for gene therapy. Preferred vectors useful for gene therapy are viral vectors, e.g. adenovirus, adeno-associated virus, herpes simplex virus, vaccinia, or, an RNA virus such as a retrovirus. Preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957. The C2MI polynucleotide sequences of the invention may also serve as a suitable vector itself, either composed solely of rearranged C2MI sequences or of chimeric C2MI host cell DNA sequences. In addition, the nucleotide sequences of the invention may be used for the construction of artificial chromosomes.

In order to achieve expression only in the target organ, the DNA sequences for transcription of the antisense oligonucleotides can be linked to a tissue specific promoter and used for gene therapy. Such promoters are well known to those skilled in the art.

Within an oligonucleotide structure, the phosphate groups are commonly referred to as forming the internucleoside backbone of the oligonucleotide. The normal linkage or backbone of RNA and DNA is a 3' to 5' phosphodiester linkage. Specific examples of preferred antisense compounds useful in the present invention include oligonucleotides containing modified backbones or non-natural internucleoside linkages. Oligonucleotides having modified backbones include those that retain a phosphorus atom in the backbone and those that do not have a phosphorus atom in the backbone. Modified oligonucleotide backbones which can result in increased stability are known to the person skilled in the art, preferably such modification is a phosphorothioate linkage.

A preferred oligonucleotide mimetic is an oligonucleotide mimetic that has been shown to have excellent hybridization properties, and is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone.

Modified oligonucleotides may also contain one or more substituted or modified sugar moieties. Preferred oligonucleotides comprise one of the following at the 2' position: OH; F; 0-, S-, or N-alkyl; 0-, S-, or N-alkenyl; 0-, S- or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl. A particularly preferred modified sugar moiety is a 2'-O-methoxyethyl sugar moiety.

Antisense-oligonucleotides of the invention may also include nucleobase modifications or substitutions. Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine etc., with 5-methylcytosine substitutions being preferred since these modifications have been shown to increase nucleic acid duplex stability.

Another modification of the oligonucleotides of the invention involves chemically linking to the oligonucleotide one or more moieties or conjugates which enhance the activity, cellular distribution or cellular uptake of the oligonucleotide. Such moieties include lipid moieties such as a cholesterol moiety, cholic acid, a thioether, a thiocholesterol, an aliphatic chain, e.g., dodecandiol or undecyl residues, a phospholipid, a polyamine or a polyethylene glycol chain, or adamantane acetic acid, a palmityl moiety, or an octadecylamine or hexylamino-carbonyl-oxycholesterol moiety.

The present invention also includes antisense compounds which are chimeric compounds. "Chimeric" antisense compounds or "chimeras," in the context of this invention, are antisense compounds, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Chimeric antisense compounds of the invention may be formed as composite structures of two or more oligonucleotides, modified oligonucleotides, oligonucleosides and/or oligonucleotide mimetics as described above. Such compounds have also been referred to in the art as hybrids or gapmers.

The present invention also relates to a pharmaceutical composition comprising an antibody or antisense oligonucleotide of the invention and a suitable excipient, diluent or carrier. Preferably, in a pharmaceutical composition, such compound as described above is combined with a pharmaceutically acceptable carrier. "Pharmaceutically acceptable" is meant to encompass any carrier, which does not interfere with the effectiveness of the biological activity of the active ingredient and that is not toxic to the host to which it is administered. Examples of suitable pharmaceutical carriers are well known in the art and include phosphate buffered saline solutions, water, emulsions, such as oil/water emulsions, various types of wetting agents, sterile solutions etc. Such carriers can be formulated by conventional methods and the active compound can be administered to the subject at an effective dose.

An "effective dose" refers to an amount: of the active ingredient that is sufficient to prevent the disease or to affect the course and the severity of the disease, leading to the reduction or remission of such pathology. An "effective dose" useful for treating and/or preventing these diseases or disorders may be determined using methods known to one skilled in the art.

Administration of the suitable compositions may be effected by different ways, e.g. by intravenous, intraperitoneal, oral, subcutaneous, intramuscular, topical or intradermal administration. The route of administration, of course, depends on the kind of therapy and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of therapy, general health and other drugs being administered concurrently.

In a preferred embodiment of the present invention, the disease that can be prevented/treated is cancer, preferably breast cancer, ovarian cancer, lung cancer, prostate cancer, pancreatic cancer, Hodgkin's disease, colorectal cancer or colon cancer or a disease of the CNS, preferably Alzheimer's disease or multiple sclerosis (MS), amyotrophic lateral sclerosis, Parkinson's disease, or transmissible spongiforme encephalopathies/Prion-linked diseases. In addition, due to a similarity of risk factors between MS and diabetes mellitus, the latter condition is also included. Furthermore, atherosclerosis is included. The terms "cancer" and "disease of the CNS" also comprise early stages of said diseases.

The present invention also relates to a vaccine for immunizing a mammal against a C2MI infection, comprising at least one C2MI polypeptide or C2MI polynucleic acid as defined above or corresponding VLP (virus-like particle) or peptide/protein/DNA complexes, in a pharmaceutically acceptable carrier. It also involves molecular and immunological tests in animals (in particular cattle) and within their products (e.g. milk and dairy products).

It may also include small chemicals for targeted therapy derived from the analysis of structural components of these agents.

A "vaccine" is an immunogenic composition capable of eliciting protection against C2MI, whether partial or complete. A vaccine may also be useful for treatment of an already infected individual, in which case it is called a therapeutic vaccine.

The term "therapeutic" refers to a composition capable of treating C2MI infection or diseases linked to this infection. The term "effective amount" refers to an amount of epitope-bearing polypeptide sufficient to induce an immunogenic response in the individual to which it is administered, or to otherwise detectably immunoreact in its intended system (e. g., immunoassay). Preferably, the effective amount is sufficient to effect treatment, as defined above. The exact amount necessary will vary according to the application. For vaccine applications or for the generation of polyclonal antiserum/antibodies, for example, the effective amount may vary depending on the species, age, and general condition of the individual, the severity of the condition being treated, the particular polypeptide selected and its mode of administration, etc. Effective amounts will be found within a relatively large, non-critical range. An appropriate effective amount can be readily determined using routine experimentation. Preferred ranges of proteins for prophylaxis of C2MI caused diseases are 0.01 to 100 µg/dose, preferably 0.1 to 50 µg/dose. Several doses may be needed per individual in order to achieve a sufficient immune response and subsequent protection- against a C2MI infection and a C2MI linked disease, respectively.

Pharmaceutically acceptable carriers include any carrier that does not itself induce the production of antibodies harmful to the individual receiving the vaccine. Suitable carriers are typically large, slowly metabolized macromolecules such as proteins, polysaccharides, polylactic acids, polyglycolic acids, polymeric amino acids, and amino acid copolymers. Such carriers are well known to those of ordinary skill in the art.

Preferred adjuvants to enhance effectiveness of the composition include, but are not limited to: aluminium hydroxide (alum), N-acetyl-muramyl-L-threonyl-D-isoglutamine (thr-MDP) as found in U.S. Patent No. 4,606,918, N-acetyl-normuramyl-L-alanyl-D-isoglutamine (nor-MDP), N-acetylmuramyl-L-alanyl-D-isoglutaminyl-L-alanine-2-(1'-2'-dipalmitoyl sn-glycero-3-hydroxy-phosphoryloxy)-ethylamine (MTP-PE) and RIBI, which contains three components extracted from bacteria, monophosphoryl lipid A, trehalose dimycolate, and cell wall Skeleton (MPL +TDM + CWS) in a 2% squalene/Tween 80 emulsion. Any of the 3 components MPL, TDM or CWS may also be used alone or combined 2 by 2. Additionally, adjuvants such as Stimulon (Cambridge Bioscience, Worcester, MA) or SAF-1 (Syntex) may be used. Further, Complete Freund's Adjuvant (CFA) and Incomplete Freund's Adjuvant (IFA) may be used for non-human applications and research purposes.

The immunogenic compositions typically will contain pharmaceutically acceptable vehicles, such as water, saline, glycerol, ethanol, etc. Additionally, auxiliary substances, such as wetting or emulsifying agents, pH buffering substances, preservatives, and the like, may be included in such vehicles.

Typically, the immunogenic compositions are prepared as injectables, either as liquid solutions or suspensions. Solid forms suitable for solution in, or suspension in, liquid vehicles prior to injection may also be prepared. The preparation also may be emulsified or encapsulated in liposomes for enhanced adjuvant effect. The proteins may also be incorporated into Immune Stimulating Complexes together with saponins, for example Quil A (ISCOMS).

Immunogenic compositions used as vaccines comprise a "sufficient amount" or "an immunologically effective amount" of the proteins of the present invention, as well as any other of the above mentioned components, as needed. "Immunologically effective amount" means that the administration of that amount to an individual, either in a single dose or as part of a series, is effective for treatment, as defined above. This amount varies depending upon the health and physical condition of the individual to be treated, the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials. Usually the amount will vary from 0.01 to 1000 µg/dose, more particularly from 0.1 -100 µg/dose.

The following examples are intended to illustrate, but not to limit the invention. While such examples are typical of those that might be used, other methods known to those skilled in the art may alternatively be utilized.

### Examples

### Material

### Commercial Assays

- NucleoSpin® Gel and PCR Clean up Macherey-Nagel
- Invitrogen™ TA Cloning™ Kits with pCR™ 2.1 Vector, without Competent Cells Invitrogen
- Invitrogen™ TA Cloning™ Kit, with pCR™2.1 Vector and One Shot™ TOP10F' Chemically Competent E. coli Invitrogen
- Invitrogen™ One Shot™ TOP10F' Chemically Competent E. coli Invitrogen
- AccuPrep Plasmid Mini Extraction Kit Bioneer
- JeneJet Plasmid Miniprep Kit Thermo Scientific
- Plasmid maxi Kit Qiagen

### Chemicals:

- TaKaRa LA Taq® DNA Polymerase with GC Buffer TaKaRa
- Sodium chloride Sigma-Aldrich
- Yeast Extract Gerbu
- Trypton / Pepton from Casein Roth
- phi29 DNA Polymerase New England Biolabs M02695
- dNTPs Takara '4030
- Exo-resistant Random primer Thermo Fisher Scientific S0181

### Example 1: DNA extraction

DNA (16 samples from cow milk and cow milk products) was extracted with phenol/chloroform as previously described (Whitley et al., 2014). Samples were diluted 1:1 with proteinase K buffer (0.2M Tris-HCL, pH7.5, 25mM EDTA, 0.3M NaCl, 2%SDS) and digested overnight with proteinase K (final concentration 1mg/ml, Sigma-Aldrich P6556) at 37°C. Ethanol precipitation followed after the phenol/chloroform extraction. Samples 5-16 were subsequently subjected to an additional purification using a Nucleospin Gel and PCR Clean up Kit according to the manufacturer's protocol (Machery and Nagel, 740.609.250). DNA was finally eluted in 5mM Tris-HCl, pH 8.0.

### Example 2: Rolling circle amplification

Rolling circle amplification was performed as described by Funk et al, 2014. Template DNA (50ng) was incubated in a total volume of 10µl (1x phi29 DNA polymerase buffer) with 25µM Exo-resistant Random primer at 95°C for 3 min followed by cooling on ice. This sample was diluted to 20µl by adding BSA (0.4mg/ml), 0.75mM dNTPs each and 10U phi29 DNA Polymerase in 1x phi29 DNA polymerase buffer. Incubation followed at 30°C for 18h and 10 minutes at 65°C before stopping the reaction at 15°C.

### Example 3: Polymerase chain reaction

A 3µl template from the RCA amplified DNA was used in the polymerase chain reaction (PCR) using TAKARA Taq enzyme and the accompanying solutions according to the manufacturer's protocol (LA TAKARA RR02AG). The respective abutting primers were added at a final concentraton of 0.2mM each. Primers were designed based on the conserved regions after aligning HCBI1, HCBI2 and HCBI7 for the BMMF2 group and for the BMMF1 group based on the conserved region of the replication gene. PCR was performed by using touchdown protocols specific for the respective primer pairs:
MBB2 primers for the BMMF1 group:
Mbb2 (MSBI1.176bbF296 forward) -5'-TGCAGAAATTGCCCCTCGACT-3' and
Mbb2 (MSBI1.176bbR295 reverse) -5'-AACAATGGGGAAGAAGTCAAAGG-3'.

The first round of 5 cycles amplification was performed at 30 seconds melting at 94°C, 1 minute annealing at 64°C and 2 minutes elongation at 72°C, followed by a second round of 5 cycles using 62°C annealing and a third round of 30 cycles using 60°C for annealing. The final round of 10 minutes at 72°C followed.

Amplification for the BMMF2 group was performed as described for the BMMF1 group using the MBB2 primers, except that the touchdown temperatures were 58°C, 56°C and 54°C for the LSconA primer and 56°C, 54°C and 52°C for the LSconB primer and the elongation time was prolonged to 3 minutes at 72°C per cycle. The abutting primer pairs used for the BMMF2 group were:
LSConA5p (forward) -5'- AAGGCAGATCAACACAGG-3' and LSconA3p (reverse) - 5'-AGCAGATTGCAAAGCCTG-3'.
LSconB5p (forward) - 5'-CAACACAGGGATAGAATAAC-3' and LSconB3p (reverse) - 5'- ATCTGCCTTAGCAGATTG C-3'.

PCR products were separated by gel electrophoresis, visible fragments excised and DNA extracted using the Nucleospin Gel and PCR Clean up Kit according to the manufacturer's protocol (Machery and Nagel, 740.609.250). DNA was finally eluted in 5mM Tris-HCl, pH 8.0.

Eluted DNA was ligated and cloned into pCR2.1 using the TA-Cloning Kit and transformed into E.coli - all according to the manufacturer's protocol (Invitrogen™ TA Cloning™ Kit, with pCR™2.1 Vector and One Shot™ TOP10F' Chemically Competent E. coli (Invitrogen K203040).
Sequencing of all clones was performed by GATC.

### Example 4: In silico analyses

All sequences obtained were compared to sequences available in Genbank and the putative protein sequences to swissprot and swissprot_splicevar.

Predicted genes were obtained with the gene identification program GENSCAN which was developed Chris Burge from Stanford University (Burge and Karlin, 1997). It analyzes genomic DNA sequences from human, other vertebrates, invertebrates and plants.

Phylogenetic trees were developed using maximum likelihood based on aligned (MUSCLE algorithm) sequences - both full-length nucleotide genomes as well as the core region of Rep.

### References

Bertini A, Poirel L, Mugnier PD, Villa L, Nordmann P, Carattoli A. Characterization and PCR-based replicon typing of resistance plasmids in Acinetobacter baumannii. Antimicrob Agents Chemother. 2010; 54:4168-4177. doi: 10.1128/AAC.00542-10.
Biagini P. Bacterial plasmid-related DNA sequences isolated from plasma of patients in maintenance hemodialysis (unpublished).
Boer DR, Ruiz-Masó JA, Rueda M, Petoukhov MV, Machón C, Svergun DI, Orozco M1, del Solar G, Coll M. Conformational plasticity of RepB, the replication initiator protein of promiscuous streptococcal plasmid pMV158. Sci Rep. 2016; 6:20915. doi: 10.1038/srep20915.
Boer DR, Ruíz-Masó JA, López-Blanco JR, Blanco AG, Vives-Llàcer M, Chacón P, Usón I, Gomis-Rüth FX, Espinosa M, Llorca O, del Solar G, Coll M. Plasmid replication initiator RepB forms a hexamer reminiscent of ring helicases and has mobile nuclease domains. EMBO J. 2009; 28:1666-78. doi: 10.1038/emboj.2009.125
Briddon RW, Martin DP, Roumagnac P, Navas-Castillo J, Fiallo-Olivé E, Moriones E, Lett J-M, Zerbini FM, Varsani A. Alphasatellitidae: a new family with two subfamilies for the classification of geminivirus- and nanovirus-associated alphasatelllites. Arch Virol. 2018; doi.org/10.1007/s00705-018-3854-2.
Burge C, Karlin S. Prediction of complete gene structures in human genomic DNA. J Mol Biol 1997; 268:78-94. (Genscan)
Curinha A1, Oliveira Braz S, Pereira-Castro I, Cruz A, Moreira A. Implications of polyadenylation in health and disease. Nucleus. 2014; 5(6):508-19. doi: 10.4161/nucl.36360.
Denoeud F1, Henriet S, Mungpakdee S, Aury JM, Da Silva C, Brinkmann H, Mikhaleva J, Olsen LC, Jubin C, Cañestro C, Bouquet JM, Danks G, Poulain J, Campsteijn C, Adamski M, Cross I, Yadetie F, Muffato M, Louis A, Butcher S, Tsagkogeorga G, Konrad A, Singh S, Jensen MF, Huynh Cong E, Eikeseth-Otteraa H, Noel B, Anthouard V, Porcel BM, Kachouri-Lafond R, Nishino A, Ugolini M, Chourrout P, Nishida H, Aasland R, Huzurbazar S, Westhof E, Delsuc F, Lehrach H, Reinhardt R, Weissenbach J, Roy SW, Artiguenave F, Postlethwait JH, Manak JR, Thompson EM, Jaillon O, Du Pasquier L, Boudinot P, Liberles DA, Volff JN, Philippe H, Lenhard B, Roest Crollius H, Wincker P, Chourrout D. Plasticity of animal genome architecture unmasked by rapid evolution of a pelagic tunicate. Science. 2010 Dec 3;330(6009):1381-5
de Villiers EM, Kimmel R, Leppik: L, Gunst K. Intragenomic rearrangement in TT viruses: a possible role in the pathogenesis of disease. Curr Top Microbiol Immunol 2009; 331:91-107.Review.
de Villiers EM, Borkosky SS, Kimmel R, Gunst K, Fei JW. The diversity of torque teno viruses: in vitro replication leads to the formation of additional replication-competent subviral molecules. J Virol. 2011; 85(14):7284-7295. doi: 10.1128/JVI.02472-10. Epub 2011 May 18.
Eilebrecht S, Hotz-Wagenblatt A, Sarachaga V, Burk A, Falida K, Chakraborty D, Nikitina E, Tessmer C, Whitley C, Sauerland C, Gunst K, Grewe I, Bund T. Expression and replication of virus-like circular DNA in human cells. Sci Rep. 2018; 8:2851.
Falida K, Eilebrecht S, Gunst K, Zur Hausen H, de Villiers EM. Isolation of Two Virus-Like Circular DNAs from Commercially Available Milk Samples. Genome Announc. 2017; 5(17). pii: e00266-17. doi: 10.1128/genomeA.00266-17.
Fernandes MA, Verstraete SG, Phan TG, Deng X, Stekol E, LaMere B, Lynch SV, Heyman MB, Delwart E. Enteric Virome and Bacterial Microbiota in Children with Ulcerative Colitis and Crohn's Disease. J Pediatr Gastroenterol Nutr. 2018 Aug 30. doi: 10.1097/MPG.0000000000002140. [Epub ahead of print]
Fondi M, Bacci G, Brilli M, Papaleo MC, Mengoni A, Vaneechoutte M, Dijkshoorn L, Fani R. Exploring the evolutionary dynamics of plasmids: the Acinetobacter pan-plasmidome. BMC Evol Biol. 2010; 10:59. doi: 10.1186/1471-2148-10-59.
Fournier PE1, Vallenet D, Barbe V, Audic S, Ogata H, Poirel L, Richet H, Robert C, Mangenot S, Abergel C, Nordmann P, Weissenbach J, Raoult D, Claverie JM. Comparative genomics of multidrug resistance in Acinetobacter baumannii. PLoS Genet. 2006; 2(1):e7. Epub 2006 Jan 13.
Funk M, Gunst K, Lucansky V, Müller H, zur Hausen H, de Villiers EM. Isolation of protein-associated circular DNA from healthy cattle serum. Genome Announc 2014;2:e00846-14.
Gomis-Rüth FX, Solá M, Acebo P, Párraga A, Guasch A, Eritja R, González A, Espinosa M, del Solar G, Coll M. The structure of plasmid-encoded transcriptional repressor CopG unliganded and bound to its operator. EMBO J. 1998 Dec 15;17(24):7404-15.
Gronenborn B, Randles JW, Knierim D, Barrière Q, Vetten HJ, Warthmann N, Cornu D, Sileye T, Winter S, Timchenko T. Analysis of DNAs associated with coconut foliar decay disease implicates a unique single-stranded DNA virus representing a new taxon. Sci Rep. 2018; 8(1):5698. doi: 10.1038/s41598-018-23739-y.
Gunst K, Zur Hausen H, de Villiers EM. Isolation of bacterial plasmid-related replication-associated circular DNA from a serum sample of a multiple sclerosis patient. Genome Announc. 2014; 2(4). pii: e00847-14. doi: 10.1128/genomeA.00847-14.
Hamidian,M., Kenyon,J.J., Holt,K.E., Pickard,D., Hall,R.M. A conjugative plasmid carrying the carbapenem resistance gene blaOXA-23 in AbaR4 in an extensively resistant GC1 Acinetobacterbaumannii isolate. J. Antimicrob. Chemother. 2014; 69: 2625-2628.
Harding CM, Hennon SW, Feldman MF. Uncovering the mechanisms of Acinetobacter baumannii virulence. Nat Rev Microbiol. 2018; 16:91-102. doi: 10.1038/nrmicro.2017.148. Epub 2017 Dec 18.
Hassan I, Orillio AF, Fiallo-Olivé E, Briddon RW, Navas-Castillo J. Infectiviy, effects on helper viruses and whitefly transmission of the deltasatellites associated with sweepoviruses (genus Begomovirus, family Geminiviridae). 2016; Sci Rep. 6: 30204.
Jelcic I, Hotz-Wagenblatt A, Hunziker A, zur Hausen H, de Villiers EM. Isolation of multiple TT virus genotypes from spleen biopsy tissue from a Hodgkin's disease patient: genome reorganization and diversity in the hypervariable region. J Virol 2004; 78:7498-74507.
Jones D.T., Taylor W.R., and Thornton J.M. (1992). The rapid generation of mutation data matrices from protein sequences. Computer Applications in the Biosciences 8: 275-282.
Jørgensen TS, Xu Z, Hansen MA, Sørensen SJ, Hansen LH. Hundreds of circular novel plasmids and DNA elements identified in a rat cecum metamobilome. PLoS One. 2014; 9e87924.
Jorgensen TJ, Hansen MA, Xu Z, Tabak MA, Sorensen, SJ, Hansen, LH. Plasmids, circular viruses and viroids from rat gut. (unpublished).
Kazlauskas D, Dayaram A, Kraberger S, Goldstein S, Varsani A, Krupovic M. Evolutionary history of ssDNA bacilldnaviruses features horizontal acquisition of the capsid gene from ssRNA nodaviruses. Virology 2017; 504: 114-121.
Kazlauskas D, Varsani A, Krupovic M. Pervasive chimerism in the replication-associated proteins of uncultured single-stranded DNA viruses. Viruses 2018; 10: 187; doi:10.3390/v10040187.
Kowarsky M, Camunas-Soler J, Kertesz M, De Vlaminck I, Koh W, Pan W, Martin L, Neff NF, Okamoto J, Wong RJ, Kharbanda S, El-Sayed Y, Blumenfeld Y, Stevenson DK, Shaw GM, Wolfe ND, Quake SR. Numerous uncharacterized and highly divergent microbes which colonize humans are revealed by circulating cell-free DNA. PNAS USA 2017; 114:9623-9628. doi/10.1073/pnas.1707009114
Kraberger S, Argüello-Astorga GR, Greenfield LG, Galilee C, Law D, Martin DP, Varsani A. Characterisation of a diverse range of circular replication-associated protein encoding DNA viruses recovered from a sewage treatment oxidation pond. Infect Genet Evol. 2015; 31:73-86.
Krupovic M. Networks of evolutionary interactions underlying the polyphyletic origin of ssDNA viruses. Curr Opin Virol. 2013; 3: 578-586.
Krupovic M, Zhi N, LiJ, Hu G, Koonin EV, WEong S, Shevchenko S, Zhao K, Young NS. Multiple layers of chimerism in a single-stranded DNA discovered by deep sequencing. Genome Biol Evol. 2015; 7:993-1001.
Lamberto I, Gunst K, Müller H, Zur Hausen H, de Villiers EM. Mycovirus-like DNA virus sequences from cattle serum and human brain and serum samples from multiple sclerosis patients. Genome Announc. 2014; 2(4). pii: e00848-14. doi: 10.1128/genomeA.00848-14.
Lean SS, Yeo CC. Small, Enigmatic Plasmids of the Nosocomial Pathogen, Acinetobacter baumannii: Good, Bad, Who Knows? Front Microbiol. 2017; 8:1547. doi: 10.3389/fmicb.2017.01547. eCollection 2017.
Legoff J, Resche-Rigon M, Bouquet J, Robin M, Naccache SN, Mercier-Delarue S, Federman S, Samayoa E, Rousseau C, Piron P, Kapel N, Simon F, Socié G, Chiu CY. The eukaryotic gut virome in hematopoietic stem cell transplantation: new clues in enteric graft-versus-host disease. Nat Med. 2017; 23: 1080-1085.
Leppik L, Gunst K, Lehtinen M, Dillner J, Streker K, de Villiers EM. In vivo and in vitro intragenomic rearrangement of TT viruses. J Virol. 2007; 81(17):9346-9356. Epub 2007 Jun 27.
Longkumer T, Kamireddy S, Muthyala VR, Akbarpasha S, Pitchika GK, Kodetham G, Ayaluru M, Siddavattam D. Acinetobacter phage genome is similar to Sphinx 2.36, the circular DNA copurified with TSE infected particles. Sci Rep. 2013; 3:2240. doi: 10.1038/srep02240.
Manuelidis L. Nuclease resistant circular DNAs copurify with infectivity in scrapie and CJD. J Neurovirol. 2011; 17(2):131-45.
Miras M, Miller WA, Truniger V, Aranda MA. Non-canonical translation in plant RNA viruses. Front. Plant Sci. 8:494 doi: 10.3389/fpls.2017.00494. eCollection 2017. Review.
Moustafa A, Xie C, Kirkness E, Biggs W, Wong E, Turpaz Y, Bloom K, Delwart E, Nelson KE, Venter JC, Telenti A. The blood DNA virome in 8,000 humans. PLoS Pathog. 2017; 13:e1006292. doi: 10.1371/journal.ppat.1006292. eCollection 2017 Mar.
Peretti A, FitzGerald PC, Bliskovsky V, Buck CB. Hamburger polyomaviruses. J Gen Virol. 2015; 96: 833-839.
Priebe SD, Lacks SA. Region of the Streptococcal plasmid pMV158 required for conjugative mobilization. J Bateriol 1989; 171: 4778-4784.
Rosario K, Duffy S, Breitbart M. A field guide to eukaryotic circular single-stranded DNA viruses: Insights gained from metagenomics. Arch Virol. 2012; 157: 1851-1871.
Rosario K, Schenck RO, Harbeitner RC, Lawler SN, Breitbart M. Novel circular single-stranded DNA viruses identified in marine invertebrates reveal high sequence diversity and consistent predicted intrinsic disorder patterns within putative structural proteins. Front Microbiol. 2015; 6:696. doi: 10.3389/fmicb.2015.00696. eCollection 2015.
Roux S, Enault F, Bronner G, Vaulot D, Forterre P, Krupovic M. Chimeric viruses blur the borders between the major groups of eukaryotic single-stranded DNA viruses. Nature Commun. 2013; 4:2700.
Ruiz-Maso JA, Leungo LM, Moreno-Cordoba I, Diaz-Orejas R, del Solar G. Successful establishment of plasmids R1 and pMV158 in a new host requires the relief of the transcriptional repression of their essential rep genes. Frontiers in Microbiol 2017; 8: 2367. Doi: 10.3389/fmicb.2017.02367.
Sadeghi M, Kaupusinsky B, Yugo DM, Phan TG, Deng X, Kanevsky I, Opriessnig T, Woolums AR, Hurley DJ, Meng XJ, Delwart E. Virome of US bovine calf serum. Biologicals 2017; 46: 64-67.
Salto IP, Torres Tejerizo G, Wibberg D, Pühler A, Schlüter A, Pistorio M.Comparative genomic analysis of Acinetobacter spp. plasmids originating from clinical settings and environmental habitats. Sci Rep. 2018 May 17; 8:7783. doi: 10.1038/s41598-018-26180-3.
Stedman KM. Deep Recombination: RNA and ssDNA Virus Genes in DNA Virus and Host Genomes. Annu Rev Virol. 2015 Nov;2(1):203-17. doi: 10.1146/annurev-virology-100114-055127. Review.
Tamura K., Stecher G., Peterson D., Filipski A., and Kumar S. (2013). MEGA6: Molecular Evolutionary Genetics Analysis version 6.0. Molecular Biology and Evolution30: 2725-2729.
Varsani A, Krupovic M. Smacoviridae: a new family of animal-associated single-stranded DNA viruses. Arch Virol. 2018; doi.org/10.1007/s00705-018-3820-z.
Wang H, Li S, Mahmood A, Yang S, Wang X, Shen Q, Shan T, Deng X, Li J, Hua X, Cui L, Delwart E, Zhang W. Plasma virome of cattle from forest region revealed diverse small circular ssDNA viral genomes. Virology Journal 2018; 15:11.
Wawrzyniak P, Plucienniczak G, Bartosik D. The different faces of rolling-circle replication and its multifunctional initiator proteins. Frontiers in Microbiol. 2017; 8: 2353. doi: 10.3389/fmicb.2017.02353.
Whitley C, Gunst K, Müller H, Funk M, zur Hausen H, de Villiers E-M. Novel replication-compotent circular DNA molecules from healthy cattle serum and milk and multiple sclerosis-affected human brain tissue. Genome Announc. 2014; 2(4). pii: e00849-14.
zur Hausen H. Red meat consumption and cancer: Reasons to suspect involvement of bovine infectious factors in colorectal cancer. Int J Cancer 2012;130:2475-83.
zur Hausen H. What do breast and CRC cancers and MS have in common? Nature Rev Clinical Oncology 2015); 12:569-570.
zur Hausen H, de Villiers EM. Cancer "causation" by infectionsindividual contributions and synergistic networks. Semin Oncol. 2014; 41(6):860-875. doi: 10.1053/j.seminoncol.2014.10.003. Epub 2014 Oct 29.
zur Hausen H, de Villiers EM. Dairy cattle serum and milk factors contributing to the risk of colon and breast cancers. Int J Cancer 2015; 137:959-967
zur Hausen H, Bund T, de Villiers EM. Infectious agents in bovine red meat and milk and their potential role in cancer and other chronic diseases. Curr Topics Microbiol Immunol 2017; Mar28. doi: 10.1007/82_2017_3. [Epub ahead of print]
zur Hausen H, Bund T, de Villiers EM. Specific Nutritional Infections Early in Life as Risk Factors for Human Colon and Breast Cancers Several Decades Later. Int J Cancer. 2018 Sep 24. doi: 10.1002/ijc.31882. [Epub ahead of print]

## Claims

1. A method for isolating DNA from cow milk, comprising the steps of
(a) phenol-chloroform extraction of cow milk followed by ethanol precipitation,
(b) performing rolling circle amplification (RCA),
(c) inverted PCR using 2 abutting primer pairs A and B selected from
>LSconAfwd:
aaggcagatcaacacagg
>LSconArev:
agcagattgcaaagcctg
>LSconBfwd:
caacacagggatagaataac
>LSconBrev:
atctgccttagcagattgc

2. A C2MI polynucleic acid obtainable by the method of claim 1 comprising:
(a) a nucleotide sequence depicted in any one of Figures 1(A) to 23(A);
(b) a nucleotide sequence having at least 90% identity to a nucleotide sequence of (a);
(c) a fragment of a nucleotide sequence of (a) or (b);
(d) a nucleotide sequence being complementary to a nucleotide sequence of (a), (b) or (c); or
(e) a nucleotide sequence which is redundant as a result of the degeneracy of the genetic code compared to any of the above-given nucleotide sequences.

3. An oligonucleotide probe comprising part of a C2MI polynucleic acid of claim 2, said probe being capable of acting as a hybridization probe for specific detection of the nucleic acid of a certain C2MI isolate containing a nucleotide sequence of claim 2.

4. An expression vector comprising a C2MI polynucleic acid of claim 2 or 3 operably linked to prokaryotic, eukaryotic or viral transcription and translation control elements.

5. A host cell transformed or modified with an expression vector according to claim 4.

6. A polypeptide being encoded by a C2MI polynucleic acid of claim 2.

7. An antibody or antigen binding fragment thereof specifically binding to a polypeptide of claim 6.

8. Use of a probe according to claim 3, a polypeptide of claim 6, or an antibody or fragment thereof according to claim 7 for the preparation of a diagnostic composition for the diagnosis of a predisposition or an early stage of cancer, a disease of the CNS, atherosclerosis or diabetes.

9. Use according to claim 8, wherein the cancer is breast cancer, ovarian cancer, lung cancer, prostate cancer, colorectal cancer or colon cancer, and the disease of the CNS is Multiple sclerosis MS, amyotrophic lateral sclerosis, transmissible spongiforme encephalopathies/Prion-linked diseases, Parkinson's disease or Alzheimer disease.

10. A method for the detection of a C2MI polynucleic acid according to claim 2 in a biological sample, comprising: (a) optionally extracting sample polynucleic acid, (b) hybridizing the polynucleic acid as described above with at least one probe according to claim 3, optionally a labelled probe, and (c) detecting the hybridized polynucleic acid.

11. A method for detecting a polypeptide of claim 6 or an antibody of claim 7 present in a biological sample, comprising: (a) contacting the biological sample for the presence and/or concentration of such polypeptide or antibody as defined above, and (b) detecting the immunological complex formed between said antibody and/or said polypeptide.

12. An antisense oligonucleotide reducing or inhibiting the expression of a C2MI polynucleic acid of claim 2 or a vector containing said antisense oligonucleotide.

13. A pharmaceutical composition comprising the antibody or antigen binding fragment thereof of claim 7 or the antisense oligonucleotide of claim 12 and a suitable pharmaceutical carrier.

14. A vaccine comprising a C2MI polynucleic acid of claim 2 or a polypeptide according to claim 6, preferably wherein the vaccine comprises a VLP or protein/DNA or polypeptide/DNA complex or specific proteins or attenuated infectious agents.

15. Use of a C2MI polynucleic acid of claim 2 as a lead component for the development of a medicament for prevention or treatment of cancer, a disease of the CNS, atherosclerosis or diabetes, preferably wherein the cancer is breast cancer, ovarian cancer, lung cancer, prostate cancer, colorectal cancer or colon cancer and the disease of the CNS is Multiple sclerosis MS, amyotrophic lateral sclerosis, transmissible spongiforme encephalopathies/Prion-linked diseases, Parkinson's disease or Alzheimer disease.
